# EUROPEAN PATENT APPLICATION

(11) **EP 1 864 971 A1**
(43) Date of publication of application: **12.12.2007**
(21) Application number: 06731350.2
(22) Date of filing: 31.03.2006
(51) Int. Cl.: C07D 207/26, A61K 31/4015, A61K 31/4025, A61K 31/4174, A61K 31/4178, A61K 31/4184, A61K 31/422, A61K 31/4439, A61K 31/45, A61K 31/451, A61K 31/454, A61K 31/496, A61K 31/505, A61K 31/513, A61K 31/5377, A61K 31/55, A61K 31/695, A61P 1/04, A61P 1/06, A61P 1/12

(54) **PROPHYLACTIC/THERAPEUTIC AGENT FOR DIABETES**

(30) Priority: 31.03.2005 JP 2005102913; 20.10.2005 JP 2005306397
(71) Applicant: Takeda Pharmaceutical Company Limited, Osaka-shi, Osaka 541-0045 (JP)
(72) Inventor: CHO, Nobuo c/o TAKEDA PHARMACEUTICAL CO. Ltd., Osaka-shi, Osaka, 5328686 (JP); KASAI, Shizuo c/o TAKEDA PHARMACEUTICAL CO. Ltd., Osaka-shi, Osaka, 5328686 (JP); YAMASHITA, Toshiro c/o TAKEDA PHARMACEUT.CO. Ltd., Osaka-shi, Osaka, 5328686 (JP)
(74) Representative: Weber, Thomas
(86) International application number: PCT/JP2006/307402
(87) International publication number: WO 2006/104280

(57) **Abstract**

The present invention relates to a 11β-hydroxysteroid dehydrogenase 1 inhibitor comprising a compound represented by the formula (1): 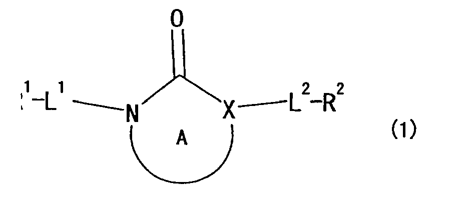 wherein each symbol is as defined in the description, or a salt thereof, or a prodrug thereof. The 11β-hydroxysteroid dehydrogenase 1 inhibitor of the present invention has a superior activity, and is useful as a pharmaceutical agent such as agents for the prophylaxis or treatment of diabetes, insulin resistance, obesity, abnormal lipid metabolism, hypertension and the like, and the like.

SEQUENCE LISTING
<110> Takeda Pharmaceutical Company Limited
<120> Agent for Preventing or Treating Diabetes
<130> 09899
<150> JP 2005-102913
<151> 2005-03-31
<150> JP 2005-306397
<151> 2005-10-20
<160> 2
<210> 1
<211> 41
<212> DNA
<213> Artificial Sequence
<220>
<223> Designed oligonucleotide primer to amplify DNA encoding human 11βHSD1
<400> 1
aaagaattcg ccatggcttt tatgaaaaaa tatctcctcc c          41
<210> 2
<211> 34
<212> DNA
<213> Artificial Sequence
<220>
<223> Designed oligonucleotide primer to amplify DNA encoding human 11βHSD1
<400> 2
aaaactgcag ctacttgttt atgaatctgt ccat          34

## Description

### Technical Field

The present invention relates to a 11β-hydroxysteroid dehydrogenase 1 inhibitor useful as a pharmaceutical agent such as agents for the prophylaxis or treatment of diabetes, insulin resistance, obesity, abnormal lipid metabolism, hypertension and the like, and the like.

### Background Art

11β-Hydroxysteroid dehydrogenase (sometimes to be abbreviated as 11β-HSD1 in the present specification) is an enzyme that converts inactive glucocorticoids (cortisone or other 11-keto steroid) to active glucocorticoid (cortisol or other 11β-hydroxysteroid), and responsible for regeneration of glucocorticoids in the tissue (Endocrinol 142:1371-1376, 2001). In recent years, it has been reported that a mouse that highly expresses 11β-HSD1 fat-specifically has diabetes, impaired glucose tolerance, insulin resistance, obesity, abnormal lipid metabolism (increase in serum triglyceride and free fatty acid), hypertension and the like (Science 294:2166-2170, 2001; J Clin Invest 112:83-90, 2003), and suggested that excess active glucocorticoids produced by 11β-HSD1 causes the onset of the aforementioned pathologies.

In addition, a report has documented that glucocorticoid not only promotes gluconeogenesis but also suppresses glycolysis in the liver; suppresses sugar uptake in fat, promotes lipolysis to increase free fatty acid; suppresses sugar uptake in muscle; and the like (Nippon Rinsho 60:280-285, 2002).

Moreover, since 11β-HSD1 deficient mouse shows degraded gluconeogenesis and glycogenolysis in the liver during fasting, resistance to blood glucose increase during breeding on a high fat feed (Proc Natl Acad Sci USA 94:14924-14929, 1997), and decreased plasma triglyceride and increased HDL cholesterol (J Biol Chem 276:41293-41300, 2001), 11β-HSD1 inhibitor is expected to improve hyperglycemia, obesity, abnormal lipid metabolism, hypertension, metabolic syndrome (state concurrently associated with at least one of type 2 diabetes, impaired glucose tolerance and insulin resistance, and at least two from obesity, abnormal lipid metabolism, hypertension and microalbuminuria) and the like.

As a 11β-hydroxysteroid dehydrogenase 1 inhibitor, the following compound has been reported.
(1) A compound represented by the formula: 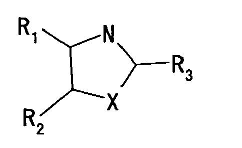 wherein
   R₁ and R₂: the one of them is R₅-SO₂-N(R₄)-L- (R₄: H or a hydrocarbon group; R₅: a hydrocarbon group; and L: an optionally substituted linker group), or they in combination form a ring substituted by R₅-SO₂-N (R₄)-L-;
   R₃: H or a substituent (an oxo group etc.); and
   X : S, O, NR₆ or C(R₇) (R₈) (R₆, R₇ and R₈ : H or a hydrocarbon group (see, for example, W02004/037251).
   As a compound having pyrrolidinone ring, the following compound has been reported.
(2) A compound represented by the formula: 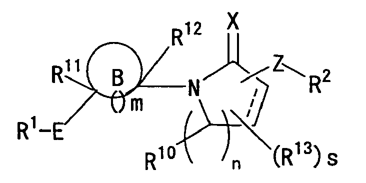 wherein
   ring B: a saturated or partially saturated C₃₋₈ cycloalkyl, or a saturated or partially saturated 3- to 7-membered heterocycle, each of which is optionally substituted by R⁵ in the number of 0 to 2 (R⁵: H, O, C₁₋₆ alkyl etc.);
   X: O or S;
   Z: a bond or (CR¹⁵R¹⁵)₁ (R¹⁵: H, C₁₋₄ alkyl, OH etc.; and 1: 1-3); R²: a C₆₋₁₀ aryl optionally substituted by R⁷ in the number of 0 to 5 (R⁷: a C₁₋₈ alkyl, Cl, OH etc.), and the like;
   s: 0 or 1;
   n: 0-3;
   R¹⁰: H and the like;
   R¹¹: H and the like;
   R¹²: H and the like;
   R¹: a C₆₋₁₀ aryl substituted by R⁶ in the number of 0 to 5, or a 5- to 10-membered aromatic heterocyclic group substituted by R⁶ in the number of 0 to 5 (R⁶: a C₁₋₈ alkyl etc.); and
   E: -S(O)p-CHRe-, -CHReNRe-, -C(O)-NRe-, -NRe-C(O)NRe-, -SO₂-NRe- or -NRe-SO₂NRe- (p: 0-2; and Re: H or a C₁₋₃ alkyl),
   which is useful as a chemokine receptor modulator (see, for example, US 2004/0186140).
(3) A compound represented by the formula: 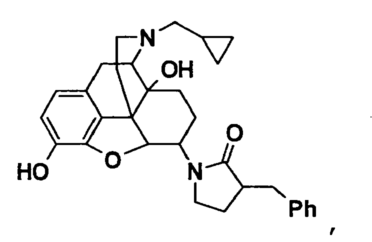 which is useful as an agent for the prophylaxis or treatment of frequent urination or incontinence (see, for example, W02004/033457).
(4) A compound represented by the formula: 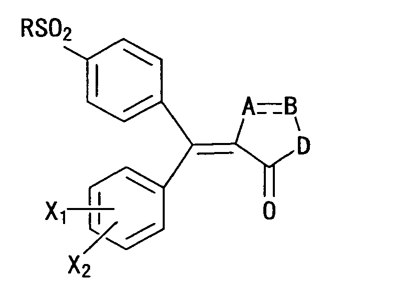 wherein
   A: CH₂ and the like;
   B: CH₂ and the like;
   D: N-R" (R": H, a C₃₋₇ cycloalkyl etc.) and the like; and
   X₁ and X₂: H and the like,
   which is useful as a cyclooxygenase (COX) inhibitor (see, for example, USP 6004994).
(5) A compound represented by the formula: 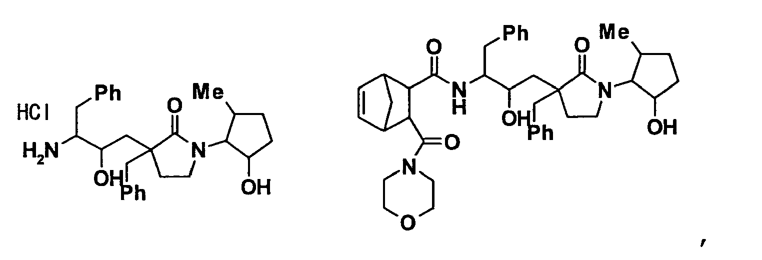 which is useful as a HIV protease inhibitor (see, for example, USP 5811462).
(6) A compound represented by the formula: 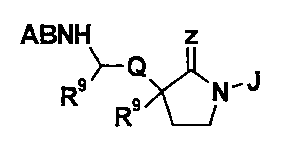 wherein
   R⁹: H or -(C(R¹⁰)(R¹⁰))n-R¹¹ (R¹⁰: H etc.; R¹¹: H, an optionally substituted C₆₋₁₀ aryl etc.; and n: 0-5) and the like;
   Q: -CH(OH)-CH₂- or -CH(NHR¹³)-CH₂-;
   Z: O, S and NH; and
   J: R¹⁴ (R¹⁴: H, an optionally substituted C₁₋₆ alkyl etc.), or -(C(R¹⁴) (R¹⁷))n-R¹⁷ (R¹⁷: H, an optionally substituted aryl, an optionally substituted heterocycle, an optionally substituted saturated or unsaturated 5- to 7-membered carbon ring etc.), which is useful as a HIV protease inhibitor (see, for example, EP 550924).
(7) A compound represented by formula: 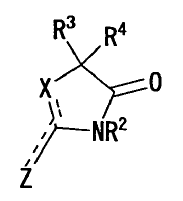 wherein
   Z: O, S, OH, a C₁₋₁₀ alkylthio, (H,H) and the like;
   X: CH₂CH₂, NR¹, CHR¹ (R¹: H, a C₁₋₆ alkylphenyl etc.) and the like; R²: naphthyl, phenyl, a C₁₋₆ alkylphenyl, 1-adamantyl, a C₃₋₁₀ cycloalkyl and the like; and
   R³ and R⁴: benzyl, H, Ph, a CN-substituted C₁₋₆ alkyl, Het-CH₂ and the like,
   which is useful as a neurotransmitter release enhancer (see, for example, WO95/29909).
(8) The following compound (see, for example, Synthesis (1996),(8), pages 941-948). 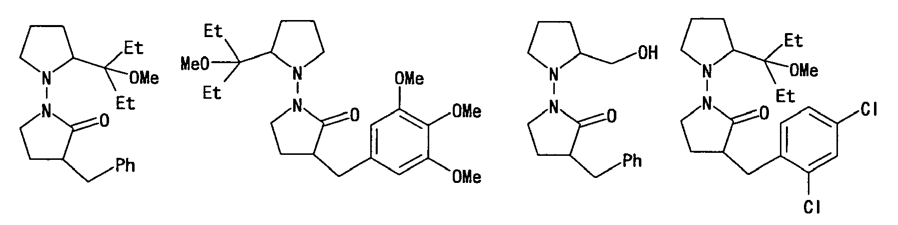
(9) A compound represented by the formula: 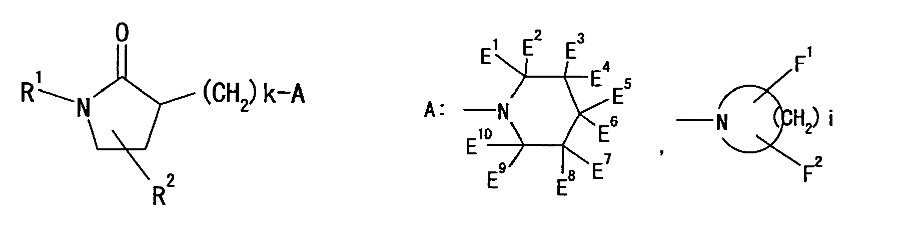 wherein
   R¹: a C₁₋₁₂ alkyl, a hydrogenated fused polycyclic C₉₋₁₅ hydrocarbon, or a hydrogenated phenyl which is optionally substituted;
   R²: H or a C₁₋₁₂ alkyl;
   k: 1-3;
   E¹-E¹⁰: H, OH and the like;
   F¹and F²: H, OH and the like; and
   i: 4, 6-9,
   which is a sigma receptor ligand and useful as a agent for the prophylaxis or treatment of mental disease, ischemic brain diseases and the like (see, for example, EP 0668275).

However, none of the above-mentioned prior art reports on the compound of the present invention.

### Disclosure of the Invention

The purpose of the present invention is to provide a 11β-hydroxysteroid dehydrogenase 1 inhibitor which is useful as a pharmaceutical agent such as agents for the prophylaxis or treatment of diabetes, insulin resistance, obesity, abnormal lipid metabolism, hypertension and the like, and the like.

The present invention relates to
[1] a 11β-hydroxysteroid dehydrogenase 1 inhibitor comprising a compound represented by the formula (1): 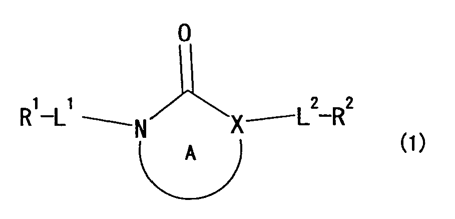 wherein
   R¹ is an optionally substituted cyclic group;
   R² is a hydrogen atom or an optionally substituted cyclic group; X is N or CR³ (R³ is a hydrogen atom or a substituent);
   L¹ and L² are the same or different and each is a bond, an optionally substituted divalent aliphatic hydrocarbon group, or a group represented by the formula: -(akn¹)ₘ-Y-(akn²)ₙ- (akn¹ and akn² are the same or different and each is an optionally substituted C₁₋₆ alkylene group; m and n are the same or different and each is 0 or 1; and Y is -O-, -S-, -SO-, -SO₂-, -NR⁴-, -SO₂NR⁴- or -NR⁴SO₂- (R⁴ is a hydrogen atom or an optionally substituted C₁₋₆ alkyl group); and ring A is an optionally substituted 4- to 7-membered nitrogen-containing non-aromatic heterocycle wherein the non-aromatic heterocycle is optionally condensed with an optionally substituted ring,
   or a salt thereof (hereinafter to be abbreviated as compound (1)), or a prodrug thereof;
[2] the agent of the above-mentioned [1], wherein the cyclic group for R¹ is a C₃₋₆ cycloalkyl group;
[3] use of compound (1) or a prodrug thereof for the production of a 11β-hydroxysteroid dehydrogenase 1 inhibitor;
[4] a method of inhibiting 11β-hydroxysteroid dehydrogenase 1 in a mammal, which comprises administering compound (1) or a prodrug thereof to the mammal;
[5] a compound represented by the formula (2a): 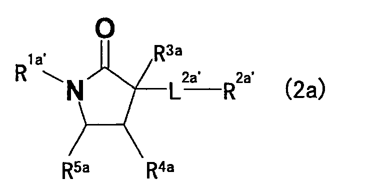 wherein
   R^{1a'} is an optionally substituted non-aromatic cyclic group provided that the non-aromatic cyclic group should be selected from a C₃₋₆ cycloalkyl group optionally condensed with a benzene ring, a 6-membered non-aromatic heterocyclic group, a 5- or 6-membered non-aromatic heterocyclic group condensed with a benzene ring, and a C₇₋₁₀ cross-linked hydrocarbon group; and
   the non-aromatic cyclic group should not have, as a substituent, a group represented by R'-E- (E is -S(O)ₜ-CHR^{e}-, -CHR^{e}NR^{e}-, -C(O)-NR^{e}-, -NR^{e}-C (O) NR^{e}-, -SO₂-NR^{e}- or -NR^{e}-SO₂NR^{e}-(t is an integer of 0 to 2; and R^{e} is a hydrogen atom or a C₁₋₃ alkyl group); and R' is an optionally substituted C₆₋₁₀ aryl group or an optionally substituted 5- to 10-membered aromatic heterocyclic group);
   R^{2a*'*} is an optionally substituted cyclic group (provided that the cyclic group should not be a non-aromatic heterocyclic group); L^{2a'} is a C₁₋₆ alkylene group;
   R^{3a} is a hydrogen atom, an optionally substituted hydroxy group, an optionally substituted mercapto group, an optionally substituted amino group or an acyl group;
   R^{4a} and R^{5a} are the same or different and each is a hydrogen atom or a substituent, or a salt thereof (hereinafter to be abbreviated as compound (2a));
[6] compound (2a) wherein the non-aromatic cyclic group for R^{1a'} is a C₃₋₆ cycloalkyl group;
[7] compound (2a) wherein R^{1a*'*} is a C₃₋₆ cycloalkyl group optionally condensed with a benzene ring, a 6-membered non-aromatic heterocyclic group, a 5- or 6-membered non-aromatic heterocyclic group condensed with a benzene ring, or a C₇₋₁₀ cross-linked hydrocarbon group, each of which is optionally substituted by 1 to 5 substituents selected from
   (1) a halogen atom;
   (2) a hydroxy group;
   (3) a cyano group;
   (4) a nitro group;
   (5) a carboxyl group;
   (6) a carbamoyl group;
   (7) an oxo group;
   (8) a C₁₋₃ alkylenedioxy group;
   (9) a C₁₋₆ alkyl group optionally substituted by 1 to 3 substituents selected from the following (i) to (vii)
      (i) a halogen atom,
      (ii) a carboxyl group,
      (iii) a hydroxy group,
      (iv) a C₁₋₆ alkoxy-carbonyl group,
      (v) a carbamoyl group optionally mono- or di-substituted by substituent (s) selected from a C₆₋₁₄ aryl group and a C₇₋₁₃ aralkyl group,
      (vi) an aromatic heterocyclic group optionally substituted by C₆₋₁₄ aryl group(s) optionally substituted by 1 to 3 halogen atoms, and
      (vii) a non-aromatic heterocyclyl-carbonyl group;
   (10) a C₂₋₆ alkenyl group optionally substituted by 1 to 3 substituents selected from a carboxyl group and a C₁₋₆ alkoxy-carbonyl group;
   (11) a C₂₋₆ alkynyl group;
   (12) a C₆₋₁₄ aryl group optionally substituted by 1 to 3 halogen atoms;
   (13) a C₇₋₁₃ aralkyl group;
   (14) a C₁₋₆ alkoxy group optionally substituted by 1 to 3 halogen atoms;
   (15) an amino group optionally mono- or di-substituted by C₁₋₆ alkyl group(s);
   (16) a C₁₋₆ alkyl-carbonyl group;
   (17) a C₁₋₆ alkoxy-carbonyl group;
   (18) a C₇₋₁₃ aralkyloxy-carbonyl group;
   (19) a C₁₋₆ alkylsulfinyl group;
   (20) a C₁₋₆ alkylsulfonyl group;
   (21) a non-aromatic heterocyclic group; and
   (22) a formyl group;
[8] compound (2a) wherein the cyclic group for R^{2a'} is a C₆₋₁₄ aryl group;
[9] compound (2a) wherein the non-aromatic cyclic group for R^{1a'} is a C₇₋₁₀ cross-linked hydrocarbon group, and R^{2a'} is a phenyl group having a substituent at the para-position;
[10] compound (2a) of the above-mentioned [9], wherein the substituent which the phenyl group has at the para-position,
   (1) a nitro group;
   (2) a carboxyl group;
   (3) a C₁₋₃ alkylenedioxy group;
   (4) a C₁₋₆ alkyl group substituted by 1 to 3 substituents selected from the following (i) to (viii)
      (i) a carboxyl group,
      (ii) a hydroxy group,
      (iii) a C₁₋₆ alkoxy-carbonyl group,
      (iv) a carbamoyl group optionally mono- or di-substituted by substituent (s) selected from a C₆₋₁₄ aryl group and a C₇₋₁₃ aralkyl group,
      (v) an amino group optionally mono- or di-substituted by C₁₋₆ alkyl-carbonyl group(s),
      (vi) an aromatic heterocyclic group optionally substituted by C₆₋₁₄ aryl group(s) optionally substituted by 1 to 3 halogen atoms,
      (vii) a non-aromatic heterocyclic group, and
      (viii) a non-aromatic heterocyclyl-carbonyl group optionally substituted by 1 to 3 C₁₋₆ alkoxy-carbonyl groups;
   (5) a C₂₋₆ alkenyl group optionally substituted by 1 to 3 substituents selected from a carboxyl group and a C₁₋₆ alkoxy-carbonyl group;
   (6) a C₂₋₆ alkynyl group optionally substituted by 1 to 3 hydroxy groups;
   (7) a C₆₋₁₄ aryl group optionally substituted by 1 to 3 substituents selected from the following (i) to (ix)
      (i) a C₁₋₆ alkyl group optionally substituted by 1 to 3 substituents selected from a hydroxy group and a halogen atom,
      (ii) a formyl group,
      (iii) a cyano group,
      (iv) a carboxyl group,
      (v) a carbamoyl group,
      (vi) a halogen atom,
      (vii) a C₁₋₆ alkyl-carbonyl group,
      (viii) a C₁₋₆ alkylsulfonyl group, and
      (ix) an amino group optionally mono- or di-substituted by substituent(s) selected from a C₁₋₆ alkyl-carbonyl group and a C₁₋₆ alkylsulfonyl group;
   (8) a C₇₋₁₃ aralkyl group;
   (9) an aromatic heterocyclic group optionally substituted by 1 to 3 substituents selected from the following (i) to (v)
      (i) a C₁₋₆ alkyl group optionally substituted by 1 to 3 hydroxy groups,
      (ii) a carboxyl group,
      (iii) a C₃₋₁₀ cycloalkyl group,
      (iv) a halogen atom, and
      (v) a formyl group;
   (10) a non-aromatic heterocyclic group optionally substituted by 1 to 3 substituents selected from the following (i) to (iii)
      (i) a C₁₋₆ alkyl group optionally substituted by 1 to 3 substituents selected from
         (a) a hydroxy group,
         (b) a C₆₋₁₄ aryl group optionally substituted by 1 to 3 substituents selected from a C₁₋₆ alkyl group optionally substituted by 1 to 3 halogen atoms and a halogen atom, and
         (c) an aromatic heterocyclic group,
      (ii) an oxo group, and
      (iii) a C₁₋₆ alkyl-carbonyl group;
   (11) a C₁₋₆ alkoxy group substituted by 1 to 3 substituents selected from the following (i) to (xii)
      (i) a cyano group,
      (ii) a carboxyl group,
      (iii) an amino group optionally mono- or di-substituted by substituent(s) selected from
         (a) a C₁₋₆ alkyl group,
         (b) a C₁₋₆ alkoxy-carbonyl group,
         (c) a C₆₋₁₄ aryl-carbonyl group optionally substituted by C₁₋₆ alkyl group(s) optionally substituted by 1 to 3 halogen atoms, and
         (d) a C₆₋₁₄ arylsulfonyl group,
      (iv) a C₆₋₁₄ aryloxy group,
      (v) a C₇₋₁₃ aralkyloxy group optionally substituted by 1 to 3 halogen atoms,
      (vi) a C₁₋₆ alkyl-carbonyl group,
      (vii) a C₁₋₆ alkoxy-carbonyl group,
      (viii) a carbamoyl group optionally mono- or di-substituted by substituent(s) selected from
         (a) a C₁₋₆ alkyl group optionally substituted by 1 to 3 substituents selected from
            (a-1) a C₆₋₁₄ aryl group optionally substituted by 1 to 3 substituents selected from a C₁₋₆ alkyl group optionally substituted by 1 to 3 halogen atoms, a halogen atom and a C₁₋₆ alkoxy group,
            (a-2) an aromatic heterocyclic group, and
            (a-3) a C₃₋₁₀ cycloalkyl group,
         (b) a C₃₋₁₀ cycloalkyl group,
         (c) a C₆₋₁₄ aryl group optionally substituted by 1 to 3 substituents selected from
            (c-1) a halogen atom, and
            (c-2) a C₁₋₆ alkyl group optionally substituted by 1 to 3 halogen atoms, and
         (d) a C₆₋₁₄ arylsulfonyl group optionally substituted by C₁₋₆ alkyl group(s) optionally substituted by 1 to 3 halogen atoms,
      (ix) an aromatic heterocyclic group optionally substituted by 1 to 3 substituents selected from
         (a) a C₁₋₆ alkyl group optionally substituted by 1 to 3 substituents selected from a hydroxy group and a halogen atom,
         (b) a C₆₋₁₄ aryl group optionally substituted by 1 to 3 halogen atoms,
         (c) a carboxyl group, and
         (d) a C₁₋₆ alkoxy-carbonyl group,
      (x) an aromatic heterocyclyl-oxy group optionally substituted by C₁₋₆ alkyl group(s) optionally substituted by 1 to 3 halogen atoms,
      (xi) a non-aromatic heterocyclic group optionally substituted by 1 to 3 substituents selected from
         (a) a C₃₋₁₀ cycloalkyl group, and
         (b) an oxo group, and
      (xii) a C₁₋₆ alkoxy group;
   (12) a C₂₋₆ alkynyloxy group;
   (13) a C₃₋₁₀ cycloalkyl-C₁₋₆ alkyloxy group;
   (14) a C₁₋₆ alkylthio group;
   (15) an amino group optionally mono- or di-substituted by substituent(s) selected from the following (i) to (xvii)
      (i) a C₁₋₆ alkyl group optionally substituted by 1 to 3 substituents selected from a hydroxy group and a C₁₋₆ alkoxy-carbonyl group,
      (ii) a C₆₋₁₄ aryl group,
      (iii) a C₇₋₁₃ aralkyl group,
      (iv) a C₁₋₆ alkyl-carbonyl group optionally substituted by 1 to 3 substituents selected from
         (a) a hydroxy group,
         (b) a carboxyl group,
         (c) a C₆₋₁₄ aryl group,
         (d) a C₁₋₆ alkoxy-carbonyl group,
         (e) a C₁₋₆ alkyl-carbonyloxy group,
         (f) a C₆₋₁₄ arylsulfonyl group,
         (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
         (h) a halogen atom, and
         (i) an aromatic heterocyclic group,
      (v) a C₁₋₆ alkoxy-carbonyl group,
      (vi) a C₆₋₁₄ aryl-carbonyl group optionally substituted by 1 to 3 substituents selected from
         (a) a C₁₋₆ alkyl group optionally substituted by 1 to 3 halogen atoms, and
         (b) a sulfamoyl group,
      (vii) a C₇₋₁₃ aralkyl-carbonyl group,
      (viii) a C₃₋₁₀ cycloalkyl-carbonyl group optionally substituted by 1 to 3 substituents selected from
         (a) a hydroxy group,
         (b) a carboxyl group,
         (c) a C₁₋₆ alkoxy-carbonyl group,
         (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
         (e) an oxo group, and
         (f) a C₁₋₆ alkyl group optionally substituted by 1 to 3 hydroxy groups,
      (ix) an aromatic heterocyclyl-carbonyl group optionally substituted by C₁₋₆ alkyl group(s) optionally substituted by 1 to 3 hydroxy groups,
      (x) a C₁₋₆ alkylsulfonyl group optionally substituted by 1 to 3 substituents selected from a non-aromatic heterocyclic group optionally substituted by oxo group(s) and a halogen atom,
      (xi) a C₆₋₁₄ arylsulfonyl group optionally substituted by 1 to 3 substituents selected from a C₁₋₆ alkyl group optionally substituted by 1 to 3 halogen atoms and a halogen atom,
      (xii) a C₇₋₁₃ aralkylsulfonyl group,
      (xiii) a C₃₋₁₀ cycloalkylsulfonyl group,
      (xiv) an aromatic heterocyclyl-sulfonyl group optionally substituted by 1 to 3 C₁₋₆ alkyl group(s),
      (xv) -CONR⁶R⁷
         wherein
         R⁶ and R⁷ are the same or different and each is selected from the following (a) to (f),
         (a) a hydrogen atom,
         (b) a C₁₋₆ alkyl group optionally substituted by 1 to 3 substituents selected from the following (b-1) to (b-4)
            (b-1) a hydroxy group,
            (b-2) a carboxyl group,
            (b-3) a C₁₋₆ alkoxy-carbonyl group, and
            (b-4) a carbamoyl group optionally mono- or di-substituted by C₁₋₆ alkyl group (s),
         (c) a C₆₋₁₄ aryl group optionally substituted by 1 to 3 substituents selected from the following (c-1) to (c-5)
            (c-1) a C₁₋₆ alkyl group optionally substituted by 1 to 3 substituents selected from a hydroxy group and a halogen atom,
            (c-2) a carboxyl group,
            (c-3) a C₁₋₆ alkoxy-carbonyl group,
            (c-4) a carbamoyl group, and
            (c-5) a C₁₋₆ alkoxy group optionally substituted by 1 to 3 halogen atoms,
         (d) a C₇₋₁₃ aralkyl group,
         (e) a C₁₋₆ alkoxy group, and
         (f) a C₇₋₁₃ aralkyloxy group, or R⁶ and R⁷ form, together with the adjacent nitrogen atom, an optionally substituted nitrogen-containing heterocycle,
      (xvi) a sulfamoyl group optionally mono- or di-substituted by C₁₋₆ alkyl group(s) optionally substituted by 1 to 3 C₃₋₁₀ cycloalkyl groups, and
      (xvii) a non-aromatic heterocyclyl-carbonyl group optionally substituted by 1 to 3 substituents selected from
         (a) a carboxyl group,
         (b) a C₁₋₆ alkoxy-carbonyl group, and
         (c) a C₇₋₁₃ aralkyl group optionally substituted by C₁₋₆ alkyl group(s) optionally substituted by 1 to 3 halogen atoms;
   (16) a C₁₋₆ alkyl-carbonyl group;
   (17) a C₁₋₆ alkoxy-carbonyl group;
   (18) a C₇₋₁₃ aralkyloxy-carbonyl group;
   (19) a C₁₋₆ alkylsulfinyl group;
   (20) a C₁₋₆ alkylsulfonyl group;
   (21) a carbamoyl group optionally mono- or di-substituted by substituent(s) selected from a C₁₋₆ alkyl group, a C₆₋₁₄ aryl group, a C₇₋₁₃ aralkyl group and an aromatic heterocyclyl-C₁₋₆ alkyl group;
   (22) a C₆₋₁₄ aryloxy group;
   (23) a C₇₋₁₃ aralkyloxy group substituted by 1 to 3 substituents selected from a C₁₋₆ alkyl group substituted by 1 to 3 hydroxy groups, a carboxyl group and a C₁₋₆ alkoxy-carbonyl group;
   (24) an aromatic heterocyclyl-oxy group optionally substituted by 1 to 3 substituents selected from a C₁₋₆ alkyl group optionally substituted by 1 to 3 halogen atoms and a halogen atom;
   (25) a tri-C₁₋₆ alkyl-silyloxy group;
   (26) a formyl group; and
   (27) a C₁₋₆ alkylsulfonyloxy group optionally substituted by 1 to 3 halogen atoms;
[11] compound (2a) wherein R^{3a} is a hydrogen atom;
[12] compound (2a) wherein R^{4a} is a hydrogen atom;
[13] compound (2a) wherein R^{5a} is a hydrogen atom;
[14] compound (2a) which is
   3-(2,4-dichlorobenzyl)-1-(4-hydroxy-4-methylcyclohexyl)pyrrolidin-2-one;
   N-(3-chloro-4-{[1-(5-hydroxy-2-adamantyl)-2-oxopyrrolidin-3-yl]methyl}phenyl)-N'-[4-(hydroxymethyl)phenyl]urea;
   N-(3'-chloro-4'-{[1-(5-hydroxy-2-adamantyl)-2-oxopyrrolidin-3-yl,]methyl}biphenyl-3-yl)methanesulfonamide; 1-(3-chloro-4-{[1-(5-hydroxy-2-adamantyl)-2-oxopyrrolidin-3-yl]methyl}phenyl)-3-[4-(trifluoromethyl)benzyl]imidazolidin-2-one;
   2-(3-chloro-4-([1-(4-hydroxy-4-methylcyclohexyl)-2-oxopyrrolidin-3-yl]methyl}phenoxy)-N-[4-(trifluoromethyl)benzyl]acetamide;
   3-(2-chloro-4-([3-(4-fluorophenyl)-1,2,4-oxadiazol-5-yl]methoxy}benzyl)-1-(4-hydroxy-4-methylcyclohexyl)pyrrolidin-2-one;
   3-{[3-chloro-3'-(hydroxymethyl)biphenyl-4-yl]methyl}-1-(4-hydroxy-4-methylcyclohexyl)pyrrolidin-2-one;
   or a salt thereof;
[15] a pharmaceutical agent comprising compound (2a);
[16] the pharmaceutical agent of the above-mentioned [15], which is an agent for the prophylaxis or treatment of diabetes, obesity or arteriosclerosis;
and the like.

The 11β-hydroxysteroid dehydrogenase 1 inhibitor of the present invention has a superior activity, and useful as a pharmaceutical agent such as agent for the prophylaxis or treatment of diabetes, insulin resistance, obesity, abnormal lipid metabolism, hypertension and the like, and the like.

### Best Mode for Embodying the Invention

In the present specification, unless otherwise specified, the "halogen atom" means fluorine atom, chlorine atom, bromine atom or iodine atom.

In the present specification, unless otherwise specified, the "C₁₋₃ alkylenedioxy group" means methylenedioxy, ethylenedioxy, trimethylenedioxy or the like.

In the present specification, unless otherwise specified, the "C₁₋₆ alkyl group" means methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.

In the present specification, unless otherwise specified, the "C₁₋₆ alkoxy group" means methoxy, ethoxy, propoxy, isopropoxy, butoxy, isobutoxy, sec-butoxy, tert-butoxy or the like.

In the present specification, unless otherwise specified, the "C₂₋₆ alkenyloxy group" means ethenyloxy, 1-propenyloxy, 2-propenyloxy, 1-butenyloxy, 2-butenyloxy, 3-butenyloxy, 1-pentenyloxy, 2-pentenyloxy, 3-pentenyloxy, 4-pentenyloxy, 1-hexenyloxy, 3-hexenyloxy, 5-hexenyloxy or the like.

In the present specification, unless otherwise specified, the "C₁₋₆ alkoxy-carbonyl group" means methoxycarbonyl, ethoxycarbonyl, propoxycarbonyl, tert-butoxycarbonyl or the like.

In the present specification, unless otherwise specified, the "C₁₋₆ alkyl-carbonyl group" means acetyl, propanoyl, butanoyl, 2-methylpropanoyl, pentanoyl, 3-methylbutanoyl or the like.

In the present specification, unless otherwise specified, the "C₁₋₆ alkylsulfonyl group" means methylsulfonyl, ethylsulfonyl, propylsulfonyl, isopropylsulfonyl, butylsulfonyl or the like.

Each symbol in the formula (1) is described in detail in the following.

X is N or CR³ (R³ is a hydrogen atom or a substituent). X is preferably CR³ (R³ is a hydrogen atom or a substituent).

As the substituent for R³, an "optionally substituted hydrocarbon group", an "optionally substituted heterocyclic group", an "optionally substituted hydroxy group", an "optionally substituted mercapto group", an "optionally substituted amino group", a "cyano group", a "nitro group", an "acyl group", a "halogen atom" and the like can be mentioned.

As the "hydrocarbon group" of the aforementioned "optionally substituted hydrocarbon group", for example, a C₁₋₁₀ alkyl group, a C₂₋₁₀ alkenyl group, a C₂₋₁₀ alkynyl group, a C₃₋₁₀ cycloalkyl group, a C₃₋₁₀ cycloalkenyl group, a C₄₋₁₀ cycloalkadienyl group, a C₆₋₁₄ aryl group, a C₇₋₁₃ aralkyl group, a C₈₋₁₃ arylalkenyl group, a C₃₋₁₀ cycloalkyl-C₁₋₆ alkyl group and the like can be mentioned.

As used herein, as the C₁₋₁₀ alkyl group, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like can be mentioned.

As the C₂₋₁₀ alkenyl group, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like can be mentioned.

As the C₂₋₁₀ alkynyl group, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like can be mentioned.

As the C₃₋₁₀ cycloalkyl group, for example, cyclopropyl, cyclobutyl, cyclopentyl, cyclohexyl, cycloheptyl, cyclooctyl and the like can be mentioned.

As the C₃₋₁₀ cycloalkenyl group, for example, 2-cyclopenten-1-yl, 3-cyclopenten-1-yl, 1-cyclohexen-1-yl, 2-cyclohexen-1-yl, 3-cyclohexen-1-yl and the like can be mentioned.

As the C₄₋₁₀ cycloalkadienyl group, for example, 2,4-cyclopentadien-1-yl, 2,4-cyclohexadien-1-yl, 2,5-cyclohexadien-1-yl and the like can be mentioned.

The above-mentioned C₃₋₁₀ cycloalkyl group, C₃₋₁₀ cycloalkenyl group and C₄₋₁₀ cycloalkadienyl group are each optionally condensed with a benzene ring, and as such fused ring groups, for example, indanyl, dihydronaphthyl, tetrahydronaphthyl, fluorenyl and the like can be mentioned. In addition, as the aforementioned hydrocarbon group, a C₇₋₁₀ cross-linked hydrocarbon group such as bicyclo[2.2.1]heptyl(norbornanyl), bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like, and the like can be mentioned.

As the C₆₋₁₄ aryl group, for example, phenyl, naphthyl, anthryl, phenanthryl, acenaphthyl, biphenylyl and the like can be mentioned. Of these, phenyl, 1-naphthyl, 2-naphthyl and the like are preferable.

As the C₇₋₁₃ aralkyl group, for example, benzyl, phenethyl, naphthylmethyl, biphenylylmethyl and the like can be mentioned.

As the C₈₋₁₃ arylalkenyl group, for example, styryl and the like can be mentioned.

As the C₃₋₁₀ cycloalkyl-C₁₋₆ alkyl group, for example, cyclohexylmethyl and the like can be mentioned.

The C₁₋₁₀ alkyl group, C₂₋₁₀ alkenyl group and C₂₋₁₀ alkynyl group exemplified as the aforementioned "hydrocarbon group" optionally have 1 to 3 substituents at substitutable positions.

As such substituents, for example,
(1) a C₃₋₁₀ cycloalkyl group (e.g., cyclopropyl, cyclohexyl);
(2) a C₆₋₁₄ aryl group (e.g., phenyl, naphthyl) optionally substituted by 1 to 3 substituents selected from the following (i) to (xi)
   (i) a C₁₋₆ alkyl group optionally substituted by 1 to 3 substituents selected from a hydroxy group and a halogen atom,
   (ii) a hydroxy group,
   (iii) a formyl group,
   (iv) a cyano group,
   (v) a carboxyl group,
   (vi) a carbamoyl group,
   (vii) a halogen atom,
   (viii) a C₁₋₆ alkoxy group,
   (ix) a C₁₋₆ alkyl-carbonyl group,
   (x) a C₁₋₆ alkylsulfonyl group, and
   (xi) an amino group optionally mono- or di-substituted by substituent(s) selected from a C₁₋₆ alkyl-carbonyl group and a C₁₋₆ alkylsulfonyl group;
(3) an aromatic heterocyclic group (e.g., thienyl, furyl, pyridyl, oxazolyl, thiazolyl, tetrazolyl, oxadiazolyl, pyrazinyl, pyrimidinyl, quinolyl, indolyl) optionally substituted by 1 to 3 substituents selected from the following (i) to (ix)
   (i) a C₁₋₆ alkyl group optionally substituted by 1 to 3 substituents selected from a halogen atom and a hydroxy group,
   (ii) a hydroxy group,
   (iii) a carboxyl group,
   (iv) a C₁₋₆ alkoxy group,
   (v) a C₃₋₁₀ cycloalkyl group (e.g., cyclopropyl),
   (vi) a halogen atom,
   (vii) a C₁₋₆ alkylsulfonyl group,
   (viii) a C₁₋₆ alkyl-carbonyl group, and
   (ix) a formyl group;
(4) a non-aromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl, oxazolidinyl, imidazolidinyl) optionally substituted by 1 to 3 substituents selected from the following (i) to (vii)
   (i) a C₁₋₆ alkyl group optionally substituted by 1 to 3 substituents selected from
      (a) a halogen atom,
      (b) a hydroxy group,
      (c) a C₆₋₁₄ aryl group optionally substituted by 1 to 3 substituents selected from a C₁₋₆ alkyl group optionally substituted by 1 to 3 halogen atoms and a halogen atom, and
      (d) an aromatic heterocyclic group (e.g., pyridyl),
   (ii) a hydroxy group,
   (iii) a C₁₋₆ alkoxy group,
   (iv) an oxo group,
   (v) a halogen atom,
   (vi) a C₁₋₆ alkylsulfonyl group, and
   (vii) a C₁₋₆ alkyl-carbonyl group;
(5) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl(s) group optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group (s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(6) an amidino group;
(7) a C₁₋₆ alkyl-carbonyl group optionally substituted by 1 to 3 halogen atoms;
(8) a C₁₋₆ alkoxy-carbonyl group optionally substituted by 1 to 3 halogen atoms;
(9) a C₇₋₁₃ aralkyloxy-carbonyl group (e.g., benzyloxycarbonyl);
(10) a C₁₋₆ alkylsulfonyl group optionally substituted by 1 to 3 halogen atoms;
(11) a carbamoyl group optionally mono- or di-substituted by substituent(s) selected from the following (i) to (iv)
   (i) a C₁₋₆ alkyl group optionally substituted by 1 to 3 halogen atoms,
   (ii) a C₆₋₁₄ aryl group (e.g., phenyl),
   (iii) a C₇₋₁₃ aralkyl group (e.g., benzyl), and
   (iv) an aromatic heterocyclyl-C₁₋₆ alkyl group (e.g., furfuryl);
(12) a thiocarbamoyl group optionally mono- or di-substituted by C₁₋₆ alkyl group(s) optionally substituted by 1 to 3 halogen atoms;
(13) a sulfamoyl group optionally mono- or di-substituted by C₁₋₆ alkyl group(s) optionally substituted by 1 to 3 halogen atoms;
(14) a carboxyl group;
(15) a hydroxy group;
(16) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(17) a C₂₋₆ alkenyloxy group optionally substituted by 1 to 3 halogen atoms;
(18) a C₂₋₆ alkynyloxy group (e.g., ethynyloxy, 2-propynyloxy) optionally substituted by 1 to 3 halogen atoms;
(19) a C₃₋₁₀ cycloalkyloxy group (e.g., cyclohexyloxy);
(20) a C₇₋₁₃ aralkyloxy group (e.g., benzyloxy) optionally substituted by 1 to 3 substituents selected from the following (i) to (iii)
   (i) a C₁₋₆ alkyl group optionally substituted by 1 to 3 hydroxy groups,
   (ii) a carboxyl group, and
   (iii) a C₁₋₆ alkoxy-carbonyl group;
(21) a C₆₋₁₄ aryloxy group (e.g., phenyloxy, naphthyloxy);
(22) an aromatic heterocyclyl-oxy group (e.g., pyridyloxy) optionally substituted by 1 to 3 substituents selected from the following (i) to (ii)
   (i) a C₁₋₆ alkyl group optionally substituted by 1 to 3 halogen atoms, and
   (ii) a halogen atom;
(23) a C₁₋₆ alkyl-carbonyloxy group (e.g., acetyloxy, tert-butylcarbonyloxy);
(24) a mercapto group;
(25) a C₁₋₆ alkylthio group (e.g., methylthio, ethylthio) optionally substituted by 1 to 3 halogen atoms;
(26) a C₇₋₁₃ aralkylthio group (e.g., benzylthio);
(27) a C₆₋₁₄ arylthio group (e.g., phenylthio, naphthylthio);
(28) a sulfo group;
(29) a cyano group;
(30) an azido group;
(31) a nitro group;
(32) a nitroso group;
(33) a halogen atom;
(34) a C₁₋₆ alkylsulfinyl group (e.g., methylsulfinyl);
(35) an oxo group;
(36) a C₃₋₁₀ cycloalkyl-C₁₋₆ alkyloxy group (e.g., cyclopropylmethyloxy);
(37) a C₁₋₃ alkylenedioxy group;
(38) a hydroxyimino group optionally substituted by a C₁₋₆ alkyl group;
(39) a tri-C₁₋₆ alkyl-silyloxy group (e.g., trimethylsilyloxy, triethylsilyloxy, tert-butyldimethylsilyloxy);
(40) a formyl group;
(41) a C₁₋₆ alkylsulfonyloxy group optionally substituted by 1 to 3 halogen atoms;
and the like can be mentioned.

As the "nitrogen-containing heterocycle" of the "optionally substituted nitrogen-containing heterocycle" formed by R⁶ and R⁷ together with the adjacent nitrogen atom, for example, a 5- to 7-membered nitrogen-containing heterocycle containing, as a ring-constituting atom besides carbon atoms, at least one nitrogen atom and optionally further containing one or two heteroatoms selected from an oxygen atom, a sulfur atom and a nitrogen atom can be mentioned. As preferable examples of the nitrogen-containing heterocycle, pyrrolidine, imidazolidine, pyrazolidine, piperidine, piperazine, morpholine, thiomorpholine and the like can be mentioned.

The nitrogen-containing heterocycle optionally has 1 to 3 (preferably 1 or 2) substituents at substitutable positions. As such substituents,
(i) a C₁₋₆ alkyl group optionally substituted by 1 to 3 halogen atoms,
(ii) a hydroxy group,
(iii) a C₁₋₆ alkoxy group,
(iv) an oxo group,
(v) a halogen atom
and the like can be mentioned.

The C₃₋₁₀ cycloalkyl group, C₃₋₁₀ cycloalkenyl group, C₄₋₁₀ cycloalkadienyl group, C₆₋₁₄ aryl group, C₇₋₁₃ aralkyl group, C₈₋₁₃ arylalkenyl group, C₃₋₁₀ cycloalkyl-C₁₋₆ alkyl group and C₇₋₁₀ cross-linked hydrocarbon group exemplified as the aforementioned "hydrocarbon group" optionally have 1 to 5, preferably 1 to 3, substituents at substitutable positions.

As such substituents, for example,
(1) those exemplified as the substituents of the aforementioned C₁₋₁₀ alkyl group and the like;
(2) a C₁₋₆ alkyl group optionally substituted by 1 to 3 substituents selected from the following (i) to (xii)
   (i) a halogen atom,
   (ii) a carboxyl group,
   (iii) a hydroxy group,
   (iv) a C₁₋₆ alkoxy-carbonyl group,
   (v) a C₁₋₆ alkyl-carbonyloxy group (e.g., acetyloxy, tert-butylcarbonyloxy),
   (vi) a C₆₋₁₄ aryloxy group (e.g., phenoxy),
   (vii) a C₁₋₆ alkyl-carbamoyloxy group (e.g., ethylcarbamoyloxy),
   (viii) a carbamoyl group optionally mono- or di-substituted by substituent(s) selected from a C₆₋₁₄ aryl group (e.g., phenyl) and a C₇₋₁₃ aralkyl group (e.g., benzyl),
   (ix) an amino group optionally mono- or di-substituted by C₁₋₆ alkyl-carbonyl group(s),
   (x) an aromatic heterocyclic group (e.g., oxadiazolyl) optionally substituted by C₆₋₁₄ aryl group(s) (e.g., phenyl) optionally substituted by 1 to 3 halogen atoms,
   (xi) a non-aromatic heterocyclic group (e.g., piperidino), and
   (xii) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(3) a C₂₋₆ alkenyl group (e.g., ethenyl, 1-propenyl) optionally substituted by 1 to 3 substituents selected from the following (i) to (v)
   (i) a halogen atom,
   (ii) a carboxyl group,
   (iii) a hydroxy group,
   (iv) a C₁₋₆ alkoxy-carbonyl group, and
   (v) a carbamoyl group;
(4) a C₂₋₆ alkynyl group (e.g., ethynyl, 1-propynyl) optionally substituted by 1 to 3 substituents selected from the following (i) to (v)
   (i) a halogen atom,
   (ii) a carboxyl group,
   (iii) a hydroxy group,
   (iv) a C₁₋₆ alkoxy-carbonyl group, and
   (v) a carbamoyl group;
(5) a C₇₋₁₃ aralkyl group (e.g., benzyl) optionally substituted by 1 to 3 substituents selected from the following (i) to (vi)
   (i) a C₁₋₆ alkyl group optionally substituted by 1 to 3 substituents selected from a halogen atom and a hydroxy group,
   (ii) a hydroxy group,
   (iii) a C₁₋₆ alkoxy group,
   (iv) a halogen atom,
   (v) a C₁₋₆ alkylsulfonyl group, and
   (vi) a C₁₋₆ alkyl-carbonyl group;
and the like can be mentioned.

As the "heterocyclic group" of the aforementioned "optionally substituted heterocyclic group", an aromatic heterocyclic group and a non-aromatic heterocyclic group can be mentioned.

As used herein, as the aromatic heterocyclic group, for example, a 4- to 7-membered (preferably 5- or 6-membered) monocyclic aromatic heterocyclic group containing, as a ring-constituting atom besides carbon atoms, 1 to 4 heteroatoms selected from an oxygen atom, a sulfur atom and a nitrogen atom, and a fused aromatic heterocyclic group can be mentioned. As the fused aromatic heterocyclic group, for example, a group derived from a fused ring wherein a ring constituting the 4- to 7- membered monocyclic aromatic heterocyclic group, and 1 or 2 rings selected from a 5- or 6-membered ring containing 1 or 2 nitrogen atoms, a 5-membered ring containing one sulfur atom, a benzene ring and the like are fused, and the like can be mentioned.

As preferable examples of the aromatic heterocyclic group, monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 4-isothiazolyl), oxazolyl (e.g., 2-oxazolyl, 4-oxazolyl, 5-oxazolyl), isoxazolyl, oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-3-yl, 1,2,4-triazin-5-yl, 1,2,4-triazin-6-yl) and the like; fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo [1,2-a] pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c] [1,2,4] triazin-3-yl) and the like;
and the like can be mentioned.

As the non-aromatic heterocyclic group, for example, a 4-to 7-membered (preferably 5- or 6-membered) monocyclic non-aromatic heterocyclic group containing, as a ring-constituting atom besides carbon atoms, 1 to 4 heteroatoms selected from an oxygen atom, a sulfur atom and a nitrogen atom, and a fused non-aromatic heterocyclic group can be mentioned. As the fused non-aromatic heterocyclic group, for example, a group derived from a fused ring wherein a ring constituting the 4- to 7- membered monocyclic non-aromatic heterocyclic group, and 1 or 2 rings selected from a 5- or 6-membered ring containing 1 or 2 nitrogen atoms, a 5-membered ring containing one sulfur atom, a benzene ring and the like are fused, and the like can be mentioned.

As preferable examples of the non-aromatic heterocyclic group, pyrrolidinyl (e.g., 1-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethylenimin-1-yl), oxazolidinyl (e.g., oxazolidin-3-yl), oxazolinyl (e.g., oxazolin-3-yl), thiazolidinyl (e.g., thiazolidin-3-yl), thiazolinyl (e.g., thiazolin-3-yl), imidazolidinyl (e.g., imidazolidin-3-yl), imidazolinyl (e.g., imidazolin-3-yl), dihydroindolyl (e.g., 2,3-dihydro-1H-indol-1-yl), dihydroisoindolyl (e.g., 1,3-dihydro-2H-isoindol-2-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, tetrahydropyranyl (e.g., 4-tetrahydropyranyl), tetrahydrothiopyranyl (e.g., 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), dihydrobenzofuranyl (e.g., 2,3-dihydro-1-benzofuran-5-yl), dihydrobenzodioxine (e.g., 2,3-dihydro-1,4-benzodioxine), dihydrobenzoxazepine (e.g., 3,4-dihydro-2H-1,5-benzoxazepine), 4,5,6,7-tetrahydro-1-benzofuranyl (e.g., 4,5,6,7-tetrahydro-1-benzofuran-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl), pyrazolidinyl (e.g., pyrazolidin-1-yl), tetrahydropyrimidinyl and the like can be mentioned.

The "heterocyclic group" of the aforementioned "optionally substituted heterocyclic group" optionally has 1 to 3 substituents at substitutable positions. As such substituents, for example, those exemplified as the substituents which the C₃₋₁₀ cycloalkyl and the like exemplified as the "hydrocarbon group" of the aforementioned "optionally substituted hydrocarbon group" optionally has, can be mentioned.

As the aforementioned "optionally substituted hydroxy group", for example, a hydroxy group optionally substituted by a substituent selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group, a C₁₋₆ alkyl-carbonyl group, a 5- or 6-membered aromatic heterocyclic group, a fused aromatic heterocyclic group and the like, each of which is optionally substituted, can be mentioned.

As used herein, as the C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group, those exemplified as the "hydrocarbon group" of the aforementioned "optionally substituted hydrocarbon group" can be mentioned.

As the 5 or 6-membered aromatic heterocyclic group, a 5 or 6-membered cyclic group, from among the "aromatic heterocyclic groups" exemplified as the "heterocyclic group" of the aforementioned "optionally substituted heterocyclic group", can be mentioned.

As the fused aromatic heterocyclic group, a fused cyclic group, from among the "aromatic heterocyclic groups" exemplified as the "heterocyclic group" of the aforementioned "optionally substituted heterocyclic group", can be mentioned.

The aforementioned C₁₋₁₀ alkyl group, C₂₋₁₀ alkenyl group, C₃₋₁₀ cycloalkyl group, C₃₋₁₀ cycloalkenyl group, C₆₋₁₄ aryl group, C_{7-13 13} aralkyl group, C₈₋₁₃ arylalkenyl group, C₁₋₆ alkyl-carbonyl group, 5 or 6-membered aromatic heterocyclic group and fused aromatic heterocyclic group optionally have 1 to 3 substituents at substitutable positions.

As the substituents of the C₁₋₁₀ alkyl group, C₂₋₁₀ alkenyl group and C₁₋₆ alkyl-carbonyl group, those exemplified as the substituents which the C₁₋₁₀ alkyl and the like exemplified as the "hydrocarbon group" of the aforementioned "optionally substituted hydrocarbon group" optionally has, can be mentioned.

As the substituents of the C₃₋₁₀ cycloalkyl group, C₃₋₁₀ cycloalkenyl group, C₆₋₁₄ aryl group, C₇₋₁₃ aralkyl group, C₈₋₁₃ arylalkenyl group, 5 or 6-membered aromatic heterocyclic group and fused aromatic heterocyclic group, those exemplified as the substituents which the C₃₋₁₀ cycloalkyl and the like exemplified as the "hydrocarbon group" of the aforementioned "optionally substituted hydrocarbon group" optionally has, can be mentioned.

As the aforementioned "optionally substituted mercapto group", for example, a mercapto group optionally substituted by a substituent selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₈₋₁₃ arylalkenyl group, a C₁₋₆ alkyl-carbonyl group, a 5 or 6-membered aromatic heterocyclic group, a fused aromatic heterocyclic group and the like, each of which is optionally substituted, can be mentioned.

As the substituents, those exemplified as the substituents of the aforementioned "optionally substituted hydroxy group" can be mentioned.

As the aforementioned "optionally substituted amino group", for example, an amino group optionally mono- or di-substituted by substituent(s) selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group and a C₈₋₁₃ arylalkenyl group, each of which is optionally substituted; an acyl group and the like, can be mentioned.

As used herein, as the C₁₋₁₀ alkyl group, C₂₋₁₀ alkenyl group, C₃₋₁₀ cycloalkyl group, C₃₋₁₀ cycloalkenyl group, C₆₋₁₄ aryl group,

C₇₋₁₃ aralkyl group and C₈₋₁₃ arylalkenyl group, those exemplified as the "hydrocarbon group" of the aforementioned "optionally substituted hydrocarbon group" can be mentioned.

The aforementioned C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group and C₈₋₁₃ arylalkenyl group optionally have 1 to 3 substituents at substitutable positions.

As the substituents of the C₁₋₁₀ alkyl group and C₂₋₁₀ alkenyl group, those exemplified as the substituents which the C₁₋₁₀ alkyl and the like exemplified as the "hydrocarbon group" of the aforementioned "optionally substituted hydrocarbon group" optionally has, can be mentioned.

As the substituents of the C₃₋₁₀ cycloalkyl group, C₃₋₁₀ cycloalkenyl group, C₆₋₁₄ aryl group, C₇₋₁₃ aralkyl group and C₈₋₁₃ arylalkenyl group, those exemplified as the substituents which the C₃₋₁₀ cycloalkyl and the like exemplified as the "hydrocarbon group" of the aforementioned "optionally substituted hydrocarbon group" optionally has, can be mentioned.

As the "acyl group" exemplified as the substituent of the "optionally substituted amino group", those similar to the "acyl group" below, which is exemplified as the "substituent" for R³ can be mentioned.

As the "acyl group" which is exemplified as the "substituent" for R³, for example, a group represented by the formula: -COR^{a}, -CO-OR^{a}, -SO₂R^{a}, -SOR^{a}, -CO-NR^{a'} R^{b'} or -CS-NR^{a'}R^{b'} wherein R^{a} is a hydrogen atom, an optionally substituted hydrocarbon group or an optionally substituted heterocyclic group, and R^{a'} and R^{b'} are the same or different and each is a hydrogen atom, an optionally substituted hydrocarbon group, an optionally substituted heterocyclic group or an optionally substituted hydroxy group, or R^{a'} and R^{b'} optionally form, together with the adjacent nitrogen atom, an optionally substituted nitrogen-containing heterocycle, and the like can be mentioned.

As the "optionally substituted hydrocarbon group" and "optionally substituted heterocyclic group" for R^{a}, R^{a'} or R^{b,}, those similar to the "optionally substituted hydrocarbon group" and "optionally substituted heterocyclic group", which are exemplified as the "substituent" for R³, can be mentioned. As the "optionally substituted hydroxy group" for R^{a'} or R^{b'}, those similar to the "optionally substituted hydroxy group" exemplified as the "substituent" for R³ can be mentioned.

As the "nitrogen-containing heterocycle" of the "optionally substituted nitrogen-containing heterocycle" formed by R^{a'} and R^{b'} together with the adjacent nitrogen atom, for example, a 5- to 7-membered nitrogen-containing heterocycle containing, as a ring-constituting atom besides carbon atoms, at least one nitrogen atom and optionally further containing one or two heteroatoms selected from an oxygen atom, a sulfur atom and a nitrogen atom can be mentioned. As preferable examples of the nitrogen-containing heterocycle, pyrrolidine, imidazolidine, pyrazolidine, piperidine, piperazine, morpholine, thiomorpholine, oxopiperazine and the like can be mentioned.

The nitrogen-containing heterocycle optionally has 1 to 3 (preferably 1 or 2) substituents at substitutable positions. As such substituents, those exemplified as the substituents which the C₃₋₁₀ cycloalkyl group and the like exemplified as the "hydrocarbon group" of the aforementioned "optionally substituted hydrocarbon group" optionally has, can be mentioned.

As preferable examples of the "acyl group",
(1) a formyl group;
(2) a carboxyl group;
(3) a carbamoyl group;
(4) a C₁₋₆ alkyl-carbonyl group;
(5) a C₁₋₆ alkoxy-carbonyl group optionally substituted by 1 to 3 substituents selected from a carboxyl group, a carbamoyl group, a thiocarbamoyl group, a C₁₋₆ alkoxy-carbonyl group and a C₁₋₆ alkyl-carbonyloxy group (e.g., methoxycarbonyl, ethoxycarbonyl, propoxycarbonyl, tert-butoxycarbonyl; carboxymethoxycarbonyl, carboxyethoxycarbonyl, carboxybutoxycarbonyl; carbamoylmethoxycarbonyl; thiocarbamoylmethoxycarbonyl; ethoxycarbonylmethoxycarbonyl, ethoxycarbonylethoxycarbonyl, methoxycarbonylbutoxycarbonyl, ethoxycarbonylbutoxycarbonyl; tert-butylcarbonyloxymethoxycarbonyl);
(6) a C₃₋₁₀ cycloalkyl-carbonyl group (e.g., cyclopentylcarbonyl, cyclohexylcarbonyl);
(7) a C₆₋₁₄ aryl-carbonyl group (e.g., benzoyl, 1-naphthoyl, 2-naphthoyl) optionally substituted by 1 to 3 substituents selected from a halogen atom, a cyano group, a C₁₋₆ alkyl group optionally substituted by 1 to 3 halogen atoms, a C₁₋₆ alkoxy group, a carboxyl group, a C₁₋₆ alkoxy-carbonyl group, an aromatic heterocyclic group (e.g., tetrazolyl, oxadiazolyl), a non-aromatic heterocyclic group (e.g., oxooxadiazolyl) and a carbamoyl group;
(8) a C₆₋₁₄ aryloxy-carbonyl group (e.g., phenyloxycarbonyl, naphthyloxycarbonyl) optionally substituted by 1 to 3 substituents selected from a carboxyl group, a C₁₋₆ alkoxy-carbonyl group and a carbamoyl group;
(9) a C₇₋₁₃ aralkyloxy-carbonyl group optionally substituted by 1 to 3 substituents selected from a carboxyl group, a carbamoyl group, a thiocarbamoyl group, a C₁₋₆ alkoxy-carbonyl group, a halogen atom, a cyano group, a nitro group, a C₁₋₆ alkoxy group, a C₁₋₆ alkylsulfonyl group and a C₁₋₆ alkyl group (e.g., benzyloxycarbonyl, phenethyloxycarbonyl; carboxybenzyloxycarbonyl; methoxycarbonylbenzyloxycarbonyl; biphenylylmethoxycarbonyl);
(10) a carbamoyl group mono- or di-substituted by substituent(s) selected from a C₁₋₆ alkyl group optionally substituted by 1 to 3 substituents selected from a halogen atom and a C₁₋₆ alkoxy group, a C₇₋₁₃ aralkyl group and a C₂₋₆ alkenyloxy group (e.g., methylcarbamoyl, ethylcarbamoyl, dimethylcarbamoyl, diethylcarbamoyl, ethylmethylcarbamoyl, propylcarbamoyl, isopropylcarbamoyl, butylcarbamoyl, isobutylcarbamoyl, trifluoroethylcarbamoyl, N-methoxyethyl-N-methylcarbamoyl, benzylcarbamoyl, 2-propenyloxycarbamoyl);
(11) a C₁₋₆ alkylsulfonyl group optionally substituted by 1 to 3 substituents selected from a carboxyl group, a carbamoyl group and a C₁₋₆ alkoxy-carbonyl group (e.g., methylsulfonyl, carboxymethylsulfonyl);
(12) a C₁₋₆ alkylsulfinyl group (e.g., methylsulfinyl);
(13) a thiocarbamoyl group;
(14) a C₇₋₁₃ aralkyl-carbonyl group (e.g., benzylcarbonyl, phenethylcarbonyl);
(15) an aromatic heterocycle (e.g., furyl, thienyl, oxazolyl, thiazolyl, isoxazolyl, isothiazolyl, pyrazolyl, pyridyl, pyrazinyl, benzofuryl, benzothienyl, quinoxalinyl)-carbonyl group optionally substituted by 1 to 3 substituents selected from a C₁₋₆ alkyl group, a C₆₋₁₄ aryl group, a C₇₋₁₃ aralkyl group, a C₁₋₆ alkoxy group, a carboxyl group, a C₁₋₆ alkoxy-carbonyl group and a carbamoyl group (e.g., furylcarbonyl, thienylcarbonyl, thiazolylcarbonyl, pyrazolylcarbonyl, pyridylcarbonyl, pyrazinylcarbonyl, benzofurylcarbonyl, benzothienylcarbonyl, quinoxalinylcarbonyl);
and the like can be mentioned.

R³ is preferably
a hydrogen atom;
a C₁₋₆ alkyl group;
a C₆₋₁₄ aryl group;
a C₇₋₁₃ aralkyl group optionally substituted by 1 to 3 substituents selected from a halogen atom, a C₁₋₆ alkyl group and a C₁₋₆ alkoxy group;
a carboxyl group;
a carbamoyl group;
a C₁₋₆ alkoxy-carbonyl group; or
a carbamoyl group mono- or di-substituted by substituent(s) selected from a C₁₋₆ alkyl group, a C₇₋₁₃ aralkyl group and a C₂₋₆ alkenyloxy group,
more preferably
a hydrogen atom;
a C₁₋₆ alkyl group;
a C₆₋₁₄ aryl group; or
a C₇₋₁₃ aralkyl group optionally substituted by 1 to 3 substituents selected from a halogen atom, a C₁₋₆ alkyl group and a C₁₋₆ alkoxy group,
particularly preferably a hydrogen atom.

X is preferably CH.

R¹ is an optionally substituted cyclic group, and R² is a hydrogen atom or an optionally substituted cyclic group.

As the "cyclic group" of the "optionally substituted cyclic group" for R¹ or R², for example, an aromatic group, a non-aromatic cyclic group and the like can be mentioned.

As the aromatic group, for example, an aromatic hydrocarbon group, an aromatic heterocyclic group and the like can be mentioned.

As the aromatic hydrocarbon group, for example, a C₆₋₁₄ aryl group and the like can be mentioned. As the C₆₋₁₄ aryl group, those exemplified as the "substituent" for R³ can be mentioned. As the aromatic heterocyclic group, those exemplified as the "substituent" for R³ can be mentioned.

As the non-aromatic cyclic group, for example, a non-aromatic cyclic hydrocarbon group, a non-aromatic heterocyclic group and the like can be mentioned.

As the non-aromatic cyclic hydrocarbon group, for example, a C₃₋₁₀ cycloalkyl group, a C₃₋₁₀ cycloalkenyl group, a C₄₋₁₀ cycloalkadienyl group, a C₇₋₁₀ cross-linked hydrocarbon group and the like, each of which is optionally condensed with a benzene ring, can be mentioned. As these, those exemplified as the "substituent" for R³ can be mentioned.

As the non-aromatic heterocyclic group, those exemplified as the "substituent" for R³ can be mentioned.

The "cyclic group" of the "optionally substituted cyclic group" for R¹ or R² is preferably a C₆₋₁₄ aryl group, a C₃₋₁₀ cycloalkyl group optionally condensed with a benzene ring, an aromatic heterocyclic group, a non-aromatic heterocyclic group, a C₇₋₁₀ cross-linked hydrocarbon group (preferably norbornanyl, adamantyl) or the like.

The "cyclic group" of the "optionally substituted cyclic group" for R¹ is more preferably a C₆₋₁₄ aryl group (preferably phenyl), a C₃₋₆ cycloalkyl group optionally condensed with a benzene ring (preferably cyclopropyl, cyclohexyl, indanyl, tetrahydronaphthyl), a 6-membered aromatic heterocyclic group (preferably pyridyl, oxadiazolyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, particularly preferably a C₃₋₆ cycloalkyl group (preferably cyclopropyl, cyclohexyl), a C₇₋₁₀ cross-linked hydrocarbon group (preferably norbornanyl, adamantyl) or the like.

The "cyclic group" of the "optionally substituted cyclic group" for R² is more preferably a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), a 6-membered non-aromatic heterocyclic group (preferably piperidinyl), a 5- or 6-membered non-aromatic heterocyclic group condensed with a benzene ring (preferably tetrahydroquinolinyl, tetrahydroisoquinolinyl) or the like, further more preferably a C₆₋₁₄ aryl group (preferably phenyl, naphthyl), particularly preferably a phenyl group.

When the "cyclic group" of the "optionally substituted cyclic group" for R¹ is a C₇₋₁₀ cross-linked hydrocarbon group, R² is preferably a phenyl group having a substituent at the para-position. The phenyl group of the "phenyl group having a substituent at the para-position" optionally further has 1 to 3 substituents, besides the substituent at the para-position.

The "cyclic group" of the "optionally substituted cyclic group" for R¹ or R² optionally has 1 to 5, preferably 1 to 3, substituents at substitutable positions. As such substituents, those exemplified as the substituents which the C₃₋₁₀ cycloalkyl group and the like exemplified as the aforementioned "substituent" for R³ optionally has, can be mentioned.

As preferable substituents,
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄-aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group(s) optionally substituted by 1 to 3 C₃₋₁₀. cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group;
(31) a C₁₋₆ alkylsulfonyloxy group optionally substituted by 1 to 3 halogen atoms;
and the like can be mentioned.

As more preferable substituents,
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group, an amino group, a C₁₋₆ alkyl-carbonylamino group (e.g., acetylamino) and a non-aromatic heterocyclic group (e.g., piperidino);
(9) a C₂₋₆ alkenyl group (e.g., ethenyl, 1-propenyl) optionally substituted by 1 to 3 C₁₋₆ alkoxy-carbonyl groups;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from a C₁₋₆ alkyl group and a C₁₋₆ alkylsulfonyl group;
(12) a C₇₋₁₃ aralkyl group (e.g., benzyl);
(13) an aromatic heterocyclic group (e.g., furyl, pyridyl, pyrimidinyl);
(14) a non-aromatic heterocyclic group (e.g., piperazinyl, oxazolidinyl) optionally substituted by 1 to 3 substituents selected from a C₁₋₆ alkyl group optionally substituted by 1 to 3 hydroxy groups, an oxo group and a C₁₋₆ alkyl-carbonyl group;
(15) a C₁₋₆ alkoxy group optionally substituted by 1 to 3 substituents selected from the following (i) to (x)
   (i) a halogen atom,
   (ii) a hydroxy group,
   (iii) a cyano group,
   (iv) a carboxyl group,
   (v) a C₁₋₆ alkoxy group,
   (vi) a C₇₋₁₃ aralkyloxy group (e.g., benzyloxy),
   (vii) a C₁₋₆ alkyl-carbonyl group,
   (viii) a C₁₋₆ alkoxy-carbonyl group,
   (ix) a carbamoyl group optionally mono- or di-substituted by C₁₋₆ alkyl group(s) (e.g., methylcarbamoyl, dimethylcarbamoyl, diethylcarbamoyl), and
   (x) a non-aromatic heterocyclic group (e.g., pyrrolidinyl);
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xi)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from a hydroxy group and a C₁₋₆ alkyl-carbonyloxy group (e.g., acetyloxy, tert-butylcarbonyloxy),
   (v) a C₁₋₆ alkoxy-carbonyl group,
   (vi) a C₆₋₁₄ aryl-carbonyl group (e.g., benzoyl),
   (vii) a C₇₋₁₃ aralkyl-carbonyl group (e.g., benzylcarbonyl, phenethylcarbonyl),
   (viii) a C₁₋₆ alkylsulfonyl group optionally substituted by 1,3-dioxo-1,3-dihydro-2H-isoindol-2-yl group,
   (ix) a C₆₋₁₄ arylsulfonyl group (e.g., benzenesulfonyl, toluenesulfonyl, 1-naphthalenesulfonyl, 2-naphthalenesulfonyl),
   (x) a C₇₋₁₃ aralkylsulfonyl group (e.g., benzylsulfonyl), and
   (xi) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl),
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(27) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(28) a tri-C₁₋₆ alkyl-silyloxy group (e.g., trimethylsilyloxy, triethylsilyloxy, tert-butyldimethylsilyloxy);
and the like can be mentioned.

As particularly preferable substituents,
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group and a non-aromatic heterocyclic group (e.g., piperidino);
(9) a C₆₋₁₄ aryl group (e.g., phenyl, naphthyl) optionally substituted by 1 to 3 C₁₋₆ alkyl groups;
(10) a C₁₋₆ alkoxy group optionally substituted by 1 to 3 halogen atoms;
(11) a C₃₋₁₀ cycloalkyl-C₁₋₆ alkyloxy group (e.g., cyclopropylmethyloxy);
(12) a C₁₋₆ alkylthio group (e.g., methylthio, ethylthio);
(13) an amino group optionally substituted by 1 or 2 substituents selected from a C₁₋₆ alkyl group, a C₁₋₆ alkyl-carbonyl group, a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkyl-aminocarbonyl group (e.g., methylaminocarbonyl, ethylaminocarbonyl), a C₆₋₁₄ aryl-aminocarbonyl group (e.g., phenylaminocarbonyl, 1-naphthylaminocarbonyl, 2-naphthylaminocarbonyl), a C₇₋₁₃ aralkyl-aminocarbonyl group (e.g., benzylaminocarbonyl), a C₁₋₆ alkylsulfonyl group, a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(14) a C₁₋₆ alkyl-carbonyl group;
(15) a C₁₋₆ alkoxy-carbonyl group;
(16) a C₁₋₆ alkylsulfinyl group (e.g., methylsulfinyl);
(17) a C₁₋₆ alkylsulfonyl group;
(18) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(19) a C₇₋₁₃ aralkyloxy group (e.g., benzyloxy) ;
and the like can be mentioned.

R¹ is preferably a C₆₋₁₄ aryl group (preferably phenyl), a C₃₋₆ cycloalkyl group optionally condensed with a benzene ring (preferably cyclopropyl, cyclohexyl, indanyl, tetrahydronaphthyl), a 6-membered aromatic heterocyclic group (preferably pyridyl, oxadiazolyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group;
(31) a C₁₋₆ alkylsulfonyloxy group optionally substituted by 1 to 3 halogen atoms;
   and the like.

R¹ is more preferably a C₆₋₁₄ aryl group (preferably phenyl), a C₃₋₆ cycloalkyl group optionally condensed with a benzene ring (preferably cyclohexyl, cyclopropyl, indanyl, tetrahydronaphthyl), a 6-membered aromatic heterocyclic group (preferably pyridyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group, an amino group, a C₁₋₆ alkyl-carbonylamino group (e.g., acetylamino) and a non-aromatic heterocyclic group (e.g., piperidino);
(9) a C₂₋₆ alkenyl group (e.g., ethenyl, 1-propenyl) optionally substituted by 1 to 3 C₁₋₆ alkoxy-carbonyl groups;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from a C₁₋₆ alkyl group and a C₁₋₆ alkylsulfonyl group;
(12) a C₇₋₁₃ aralkyl group (e.g., benzyl);
(13) an aromatic heterocyclic group (e.g., furyl, pyridyl, pyrimidinyl);
(14) a non-aromatic heterocyclic group (e.g., piperazinyl, oxazolidinyl) optionally substituted by 1 to 3 substituents selected from a C₁₋₆ alkyl group optionally substituted by 1 to 3 hydroxy groups, an oxo group and a C₁₋₆ alkyl-carbonyl group;
(15) a C₁₋₆ alkoxy group optionally substituted by 1 to 3 substituents selected from the following (i) to (x)
   (i) a halogen atom,
   (ii) a hydroxy group,
   (iii) a cyano group,
   (iv) a carboxyl group,
   (v) a C₁₋₆ alkoxy group,
   (vi) a C₇₋₁₃ aralkyloxy group (e.g., benzyloxy),
   (vii) a C₁₋₆ alkyl-carbonyl group,
   (viii) a C₁₋₆ alkoxy-carbonyl group,
   (ix) a carbamoyl group optionally mono- or di-substituted by C₁₋₆ alkyl group(s) (e.g., methylcarbamoyl, dimethylcarbamoyl, diethylcarbamoyl), and
   (x) a non-aromatic heterocyclic group (e.g., pyrrolidinyl);
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xi)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from a hydroxy group and a C₁₋₆ alkyl-carbonyloxy group (e.g., acetyloxy, tert-butylcarbonyloxy),
   (v) a C₁₋₆ alkoxy-carbonyl group,
   (vi) a C₆₋₁₄ aryl-carbonyl group (e.g., benzoyl),
   (vii) a C₇₋₁₃ aralkyl-carbonyl group (e.g., benzylcarbonyl, phenethylcarbonyl),
   (viii) a C₁₋₆ alkylsulfonyl group optionally substituted by 1,3-dioxo-1,3-dihydro-2H-isoindol-2-yl group,
   (ix) a C₆₋₁₄ arylsulfonyl group (e.g., benzenesulfonyl, toluenesulfonyl, 1-naphthalenesulfonyl, 2-naphthalenesulfonyl),
   (x) a C₇₋₁₃ aralkylsulfonyl group (e.g., benzylsulfonyl), and
   (xi) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl),
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(27) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(28) a tri-C₁₋₆ alkyl-silyloxy group (e.g., trimethylsilyloxy, triethylsilyloxy, tert-butyldimethylsilyloxy);
and the like.

R¹ is particularly preferably a C₆₋₁₄ aryl group (preferably phenyl), a C₃₋₆ cycloalkyl group optionally condensed with a benzene ring (preferably cyclohexyl, cyclopropyl, indanyl, tetrahydronaphthyl), a 6-membered aromatic heterocyclic group (preferably pyridyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl), norbornanyl, adamantly or the like, each of which is optionally substituted by 1 to 3 substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group and a non-aromatic heterocyclic group (e.g., piperidino);
(9) a C₆₋₁₄ aryl group (e.g., phenyl, naphthyl) optionally substituted by 1 to 3 C₁₋₆ alkyl groups;
(10) a C₁₋₆ alkoxy group optionally substituted by 1 to 3 halogen atoms;
(11) a C₃₋₁₀ cycloalkyl-C₁₋₆ alkyloxy group (e.g., cyclopropylmethyloxy);
(12) a C₁₋₆ alkylthio group (e.g., methylthio, ethylthio);
(13) an amino group optionally mono- or di-substituted by substituent(s) selected from a C₁₋₆ alkyl group, a C₁₋₆ alkyl-carbonyl group, a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkyl-aminocarbonyl group (e.g., methylaminocarbonyl, ethylaminocarbonyl), a C₆₋₁₄ aryl-aminocarbonyl group (e.g., phenylaminocarbonyl, 1-naphthylaminocarbonyl, 2-naphthylaminocarbonyl), a C₇₋₁₃ aralkyl-aminocarbonyl group (e.g., benzylaminocarbonyl), a C₁₋₆ alkylsulfonyl group, a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(14) a C₁₋₆ alkyl-carbonyl group;
(15) a C₁₋₆ alkoxy-carbonyl group;
(16) a C₁₋₆ alkylsulfinyl group (e.g., methylsulfinyl);
(17) a C₁₋₆ alkylsulfonyl group;
(18) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(19) a C₇₋₁₃ aralkyloxy group (e.g., benzyloxy) ;
and the like.

R² is a hydrogen atom or an optionally substituted cyclic group, preferably an optionally substituted cyclic group, more preferably a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), a 6-membered non-aromatic heterocyclic group (preferably piperidinyl), a 5- or 6-membered non-aromatic heterocyclic group condensed with a benzene ring (preferably tetrahydroquinolinyl, tetrahydroisoquinolinyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy) ;
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group;
(31) a C₁₋₆ alkylsulfonyloxy group optionally substituted by 1 to 3 halogen atoms;
and the like.

R² is more preferably a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), a 6-membered non-aromatic heterocyclic group (preferably piperidinyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group, an amino group, a C₁₋₆ alkyl-carbonylamino group (e.g., acetylamino) and a non-aromatic heterocyclic group (e.g., piperidino);
(9) a C₂₋₆ alkenyl group (e.g., ethenyl, 1-propenyl) optionally substituted by 1 to 3 C₁₋₆ alkoxy-carbonyl groups;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from a C₁₋₆ alkyl group and a C₁₋₆ alkylsulfonyl group;
(12) a C₇₋₁₃ aralkyl group (e.g., benzyl);
(13) an aromatic heterocyclic group (e.g., furyl, pyridyl, pyrimidinyl);
(14) a non-aromatic heterocyclic group (e.g., piperazinyl, oxazolidinyl) optionally substituted by 1 to 3 substituents selected from a C₁₋₆ alkyl group optionally substituted by 1 to 3 hydroxy groups, an oxo group and a C₁₋₆ alkyl-carbonyl group;
(15) a C₁₋₆ alkoxy group optionally substituted by 1 to 3 substituents selected from the following (i) to (x)
   (i) a halogen atom,
   (ii) a hydroxy group,
   (iii) a cyano group,
   (iv) a carboxyl group,
   (v) a C₁₋₆ alkoxy group,
   (vi) a C₇₋₁₃ aralkyloxy group (e.g., benzyloxy),
   (vii) a C₁₋₆ alkyl-carbonyl group,
   (viii) a C₁₋₆ alkoxy-carbonyl group,
   (ix) a carbamoyl group optionally mono- or di-substituted by C₁₋₆ alkyl group(s) (e.g., methylcarbamoyl, dimethylcarbamoyl, diethylcarbamoyl), and
   (x) a non-aromatic heterocyclic group (e.g., pyrrolidinyl);
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xi)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from a hydroxy group and a C₁₋₆ alkyl-carbonyloxy group (e.g., acetyloxy, tert-butylcarbonyloxy),
   (v) a C₁₋₆ alkoxy-carbonyl group,
   (vi) a C₆₋₁₄ aryl-carbonyl group (e.g., benzoyl),
   (vii) a C₇₋₁₃ aralkyl-carbonyl group (e.g., benzylcarbonyl, phenethylcarbonyl),
   (viii) a C₁₋₆ alkylsulfonyl group optionally substituted by 1, 3-dioxo-1, 3-dihydro-2H-isoindol-2-yl group,
   (ix) a C₆₋₁₄ arylsulfonyl group (e.g., benzenesulfonyl, toluenesulfonyl, 1-naphthalenesulfonyl, 2-naphthalenesulfonyl),
   (x) a C₇₋₁₃ aralkylsulfonyl group (e.g., benzylsulfonyl), and
   (xi) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl),
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(27) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(28) a tri-C₁₋₆ alkyl-silyloxy group (e.g., trimethylsilyloxy, triethylsilyloxy, tert-butyldimethylsilyloxy);
and the like.

R² is particularly preferably a C₆₋₁₄ aryl group (preferably phenyl, naphthyl), a C₃₋₆ cycloalkyl group (preferably cyclopropyl, cyclohexyl), a 5- or 6-membered aromatic heterocyclic group (preferably pyridyl, imidazolyl), a 6-membered non-aromatic heterocyclic group (preferably piperidinyl) or the like, each of which is optionally substituted by 1 to 3 substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group and a non-aromatic heterocyclic group (e.g., piperidino);
(9) a C₆₋₁₄ aryl group (e.g., phenyl, naphthyl) optionally substituted by 1 to 3 C₁₋₆ alkyl groups;
(10) a C₁₋₆ alkoxy group optionally substituted by 1 to 3 halogen atoms;
(11) a C₃₋₁₀ cycloalkyl-C₁₋₆ alkyloxy group (e.g., cyclopropylmethyloxy);
(12) a C₁₋₆ alkylthio group (e.g., methylthio, ethylthio);
(13) an amino group optionally mono- or di-substituted by substituent(s) selected from a C₁₋₆ alkyl group, a C₁₋₆ alkyl-carbonyl group, a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkyl-aminocarbonyl group (e.g., methylaminocarbonyl, ethylaminocarbonyl), a C₆₋₁₄ aryl-aminocarbonyl group (e.g., phenylaminocarbonyl, 1-naphthylaminocarbonyl, 2-naphthylaminocarbonyl), a C₇₋₁₃ aralkyl-aminocarbonyl group (e.g., benzylaminocarbonyl), a C₁₋₆ alkylsulfonyl group (e.g., methylsulfonyl, ethylsulfonyl, isopropylsulfonyl), a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(14) a C₁₋₆ alkyl-carbonyl group;
(15) a C₁₋₆ alkoxy-carbonyl group;
(16) a C₁₋₆ alkylsulfinyl group (e.g. , methylsulfinyl);
(17) a C₁₋₆ alkylsulfonyl group;
(18) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(19) a C₇₋₁₃ aralkyloxy group (e.g., benzyloxy);
and the like.

L¹ and L² are the same or different and each is (1) a bond, (2) an optionally substituted divalent aliphatic hydrocarbon group, or (3) a group represented by the formula: -(akn¹)ₘ-Y-(akn²)ₙ- (akn¹ and akn² are the same or different and each is an optionally substituted C₁₋₆ alkylene group; m and n are the same or different and each is 0 or 1; and Y is -O-, -S-, -SO-, -SO₂-, -NR⁴-, -SO₂NR⁴- or -NR⁴SO₂- (R⁴ is a hydrogen atom or optionally substituted C₁₋₆ alkyl group).

As the "divalent aliphatic hydrocarbon group" of the "optionally substituted divalent aliphatic hydrocarbon group" for L¹ or L², for example, a divalent hydrocarbon group having 1 to 10 carbon atoms can be mentioned, and specifically,
(1) a C₁₋₁₀ alkylene group (e.g., -CH₂-, - (CH₂)₂-, -(CH₂)₃-, -CH₂)₄-, -(CH₂)₅-, -(CH₂)₆-, -CH(CH₃)-, -C(CH₃)₂-, -CH(C₂H₅)-, -CH(CH3))2-, -(CH₂)₂C(CH₃)₂-, -(CH₂)₃C(CH₃)₂-, -C(C₂H₅)₂-, -CH₂-C(CH₃)(C₂H₅)-);
(2) a C₂₋₁₀ alkenylene group (e.g., -CH=CH-, -CH₂-CH=CH-, -CH=CH-CH₂-, -CH=CH-CH₂-CH₂-, -C(CH₃)₂-CH=CH-,-CH₂-CH=CH-CH₂-, -CH₂-CH₂-CH=CH-, -CH=CH-CH=CH-, -CH=CH-CH₂-CH₂-CH₂-);
(3) a C₂₋₁₀ alkynylene group (e.g., -C≡C-, -CH₂-C≡C-, -CH₂-C≡C-CH₂-CH₂-);
and the like can be mentioned.

The divalent aliphatic hydrocarbon group is preferably a C₁₋₁₀ alkylene group or the like, more preferably a C₁₋₆ alkylene group or the like, particularly preferably -CH₂-, -CH(CH₃)-, -C(CH₃)₂-, -(CH₂)₂C(CH₃)₂- or the like.

The "divalent aliphatic hydrocarbon group" of the "optionally substituted divalent aliphatic hydrocarbon group" for L¹ or L² optionally has 1 to 3 substituents at substitutable positions. As such substituents, those exemplified as the substituents which the C₁₋₁₀ alkyl group and the like exemplified as the aforementioned "substituent" for R³ optionally has, can be mentioned. As preferable substituents, an oxo group, a hydroxy group, a C₃₋₁₀ cycloalkyl group (preferably cyclopropyl), a hydroxyimino group optionally substituted by a C₁₋₆ alkyl group (preferably hydroxyimino, methoxyimino) and the like can be mentioned.

As the "C₁₋₆ alkylene group" of the "optionally substituted C₁₋₆ alkylene group" for akn¹ or akn², an alkylene group having 1 to 6 carbon atoms, from among the C₁₋₁₀ alkylene groups exemplified as the "divalent aliphatic hydrocarbon group" of the aforementioned "optionally substituted divalent aliphatic hydrocarbon group", can be mentioned. Akn¹ and akn² are each preferably a C₁₋₃ alkylene group.

The "C₁₋₆ alkylene group" of the "optionally substituted C₁₋₆ alkylene group" for akn¹ or akn² optionally has 1 to 3 substituents at substitutable positions. As such substituents, those exemplified as the substituents which the C₁₋₁₀ alkyl group and the like exemplified as the aforementioned "substituent" for R³ optionally has, can be mentioned. As preferable substituents, an oxo group and the like can be mentioned.

The "C₁₋₆ alkyl group" of the "optionally substituted C₁₋₆ alkyl group" for R⁴ is preferably methyl.

The "C₁₋₆ alkyl group" of the "optionally substituted C₁₋₆ alkyl group" for R⁴ optionally has 1 to 3 substituents at substitutable positions. As such substituents, those exemplified as the substituents which the C₁₋₁₀ alkyl group and the like exemplified as the aforementioned "substituent" for R³ optionally has, can be mentioned.

R⁴ is preferably a hydrogen atom, or a C₁₋₆ alkyl group optionally substituted by 1 or 2 substituents selected from an oxo group, a C₁₋₆ alkoxy group and the like, more preferably a hydrogen atom or a C₁₋₆ alkyl group.

Y is preferably -O-, -NR⁴- (R⁴ is preferably a hydrogen atom or a C₁₋₆ alkyl group), -S-, -SO-, -SO₂- or the like.

L¹ and L² are the same or different and each is preferably a bond;
a C₁₋₆ alkylene group optionally substituted by substituent(s) selected from an oxo group, a hydroxy group, a C₃₋₁₀ cycloalkyl group (preferably cyclopropyl), a hydroxyimino group optionally substituted by a C₁₋₆ alkyl group (preferably hydroxyimino, methoxyimino) and the like;
-(akn¹)ₘ-O-(akn²)ₙ- (akn¹ and akn² are each a C₁₋₃ alkylene group optionally substituted by an oxo group and the like, and m and n are the same or different and each is 0 or 1);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by an oxo group and the like, and m and n are the same or different and each is 0 or 1);
-(akn¹)ₘ-S-(akn²)n- (akn¹ and akn² are each a C₁₋₃ alkylene group optionally substituted by an oxo group and the like, and m and n are the same or different and each is 0 or 1);
-(akn¹)ₘ-SO-(akn²)ₙ- (akn¹ and akn² are each a C₁₋₃ alkylene group optionally substituted by an oxo group and the like, and m and n are the same or different and each is 0 or 1);
-(akn¹)ₘ-SO₂-(akn²)ₙ- (akn¹ and akn² are each a C₁₋₃ alkylene group optionally substituted by an oxo group and the like, and m and n are the same or different and each is 0 or 1);
or the like.

L¹ and L² are the same or different and each is more preferably
a bond;
a C₁₋₆ alkylene group optionally substituted by substituent(s) selected from an oxo group, a hydroxy group, a C₃₋₁₀ cycloalkyl group (preferably cyclopropyl), a hydroxyimino group optionally substituted by a C₁₋₆ alkyl group (preferably hydroxyimino, methoxyimino) and the like;
-(akn¹)ₘ-O-(akn²)ₙ- (akn¹ and akn² are each a C₁₋₃ alkylene group optionally substituted by an oxo group and the like, and m and n are the same or different and each is 0 or 1);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by an oxo group and the like, and m and n are the same or different and each is 0 or 1);
or the like.

L¹ is more preferably
a bond;
a C₁₋₆ alkylene group (preferably -CH₂-, -CH(CH₃)-, -C(CH₃)₂-, - (CH₂)₂C (CH₃)₂-) optionally substituted by C₃₋₁₀ cycloalkyl group (s) (preferably cyclopropyl);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by an oxo group and the like, and m and n are the same or different and each is 0 or 1);
or the like.

L¹ is further more preferably
a bond;
a C₁₋₆ alkylene group (preferably -CH₂-, -CH(CH₃)-, -C(CH₃)₂-, -(CH₂)₂C(CH₃)₂-) ;
or the like.

L¹ is particularly preferably a bond.

L² is more preferably
a bond;
a C₁₋₆ alkylene group optionally substituted by substituent(s) selected from an oxo group, a hydroxy group, a hydroxyimino group optionally substituted by a C₁₋₆ alkyl group (preferably hydroxyimino, methoxyimino) and the like;
-(akn¹)ₘ-O-(akn²)ₙ-(akn¹ and akn² are each a C₁₋₃ alkylene group optionally substituted by an oxo group and the like, and m and n are the same or different and each is 0 or 1);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by an oxo group and the like, and m and n are the same or different and each is 0 or 1);
-(akn¹)ₘ-S-(akn²)ₙ- (akn¹ and akn² are each a C₁₋₃ alkylene group optionally substituted by an oxo group and the like, and m and n are the same or different and each is 0 or 1);
-(akn¹)ₘ-SO-(akn²)ₙ- (akn¹ and akn² are each a C₁₋₃ alkylene group optionally substituted by an oxo group and the like, and m and n are the same or different and each is 0 or 1);
-(akn¹)ₘ-SO₂-(akn²)ₙ- (akn¹ and akn² are each a C₁₋₃ alkylene group optionally substituted by an oxo group and the like, and m and n are the same or different and each is 0 or 1);
or the like.

L² is further more preferably
a bond;
a C₁₋₆ alkylene group optionally substituted by substituent(s) selected from an oxo group, a hydroxy group, a hydroxyimino group optionally substituted by a C₁₋₆ alkyl group (preferably hydroxyimino, methoxyimino) and the like;
-(akn¹)ₘ-O-(akn²)ₙ- (akn¹ and akn² are each a C₁₋₃ alkylene group optionally substituted by an oxo group and the like, and m and n are the same or different and each is 0 or 1);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by an oxo group and the like, and m and n are the same or different and each is 0 or 1);
or the like.

L² is particularly preferably a C₁₋₆ alkylene group.

Ring A is an optionally substituted 4- to 7-membered nitrogen-containing non-aromatic heterocycle wherein the non-aromatic heterocycle is optionally condensed with an optionally substituted ring.

As the "4- to 7-membered nitrogen-containing non-aromatic heterocycle" of the "optionally substituted 4- to 7-membered nitrogen-containing non-aromatic heterocycle" for ring A, for example, a 4- to 7-membered nitrogen-containing (preferably 5- or 6-membered) non-aromatic heterocycle containing, as a ring-constituting atom besides carbon atoms, at least one nitrogen atom and optionally further containing one or two heteroatoms selected from an oxygen atom, a sulfur atom and a nitrogen atom can be mentioned. As the non-aromatic heterocycle, a ring containing at least one nitrogen atom, from among the rings corresponding to "the non-aromatic heterocyclic groups" exemplified as the aforementioned "substituent" for R³, can be mentioned.

As preferable examples of the 4- to 7-membered nitrogen-containing non-aromatic heterocycle, pyrrolidine, pyrazolidine, piperidine, morpholine, thiomorpholine, piperazine, oxazolidine, thiazolidine, imidazolidine, oxadiazolidine, thiadiazolidine, tetrahydropyrimidine, hexahydropyrimidine, hexamethylenimine and the like can be mentioned. Of these, pyrrolidine, imidazolidine, piperidine, hexahydropyrimidine and the like are preferable, and pyrrolidine is particularly preferable.

The 4- to 7-membered nitrogen-containing non-aromatic heterocycle is optionally condensed with an optionally substituted ring, and as the ring, benzene, a C₃₋₁₀ cycloalkane, a 5- to 7-membered aromatic heterocycle and a 5- to 7-membered non-aromatic heterocycle can be mentioned.

As the "C₃₋₁₀ cycloalkane", for example, cyclopentane, cyclohexane, cycloheptane and the like can be mentioned.

As the "5- to 7-membered aromatic heterocycle", for example, a 5- to 7-membered aromatic heterocycle containing, as a ring-constituting atom besides carbon atoms, 1 to 4 heteroatoms selected from an oxygen atom, a sulfur atom and a nitrogen atom can be mentioned.

Specifically, for example, furan, thiophene, pyridine, pyrimidine, pyridazine, pyrazine, pyrrole, imidazole, pyrazole, thiazole, isothiazole, oxazole, isoxazole, oxadiazole (e.g., 1,2,4-oxadiazole, 1,3,4-oxadiazole), thiadiazole (e.g., 1,2,4-thiadiazole, 1,3,4-thiadiazole), triazole (e.g., 1,2,4-triazole, 1,2,3-triazole), tetrazole, triazine (e.g., 1,2,4-triazine) and the like can be mentioned.

As the "5- to 7-membered non-aromatic heterocycle", for example, a 5- to 7-membered non-aromatic heterocycle containing, as a ring-constituting atom besides carbon atoms, 1 to 4 heteroatoms selected from an oxygen atom, a sulfur atom and a nitrogen atom can be mentioned.

Specifically, for example, pyrrolidine, imidazoline, pyrazolidine, pyrazoline, piperidine, piperazine, hexamethylenimine, imidazolidine, dioxole, dioxolan, dihydrooxadiazole (e.g., 4,5-dihydro-1,2,4-oxadiazole), oxazolidine (e.g., 1,3-oxazolidine), tetrahydropyran, morpholine, thiomorpholine, dioxazole, oxazolidine, oxadiazoline, thiazolidine, thiadiazoline, triazoline, dithiazole and the like can be mentioned.

As such fused ring, for example, dihydroindole, dihydroisoindole, dihydroquinoline, tetrahydroquinoline, dihydroisoquinoline, tetrahydroisoquinoline and the like can be mentioned.

The 4- to 7-membered nitrogen-containing non-aromatic heterocycle for ring A is substituted by an oxo group on the carbon atom between the ring-constituting nitrogen atom and X (X is N or CR³). The 4- to 7-membered nitrogen-containing non-aromatic heterocycle optionally has, besides the oxo group, -L¹-R¹ and -L²-R² (the symbols in the formula are as defined above), 1 to 3 substituents at substitutable positions. As such substituents those exemplified as the aforementioned "substituent" for R³ and oxo group can be mentioned.

As the substituents of the "optionally substituted ring" with which the 4- to 7-membered nitrogen-containing non-aromatic heterocycleoptionally is condensed, those exemplified as the substituents which the C₃₋₁₀ cycloalkyl group and the like exemplified as the aforementioned "substituent" for R³ optionally has, can be mentioned.

Ring A is preferably a 5- or 6-membered non-aromatic heterocycle (preferably pyrrolidine, imidazolidine, piperidine, hexahydropyrimidine, hexamethylenimine) optionally substituted by 1 to 3 substituents selected from
a C₁₋₆ alkyl group optionally substituted by 1 to 3 substituents selected from a hydroxy group, a C₆₋₁₄ aryloxy group and a C₁₋₆ alkyl-carbamoyloxy group;
a hydroxy group;
an oxo group;
a carboxyl group;
an amino group optionally mono- or di-substituted by C₁₋₆ alkoxy-carbonyl group(s);
a C₁₋₆ alkoxy-carbonyl group;
and the like,
more preferably a 5- or 6-membered non-aromatic heterocycle (preferably pyrrolidine, imidazolidine, piperidine, hexahydropyrimidine) optionally substituted by 1 to 3 substituents selected from a C₁₋₆ alkyl group, a hydroxy group, an oxo group and the like,
particularly preferably a unsubstituted 5- or 6-membered non-aromatic heterocycle (preferably pyrrolidine, imidazolidine, piperidine, hexahydropyrimidine; more preferably pyrrolidine).

As preferable examples of compound (I), the following compounds can be mentioned.

### [Compound A]

A compound wherein
R¹ is a C₆₋₁₄ aryl group (preferably phenyl), a C₃₋₆ cycloalkyl group optionally condensed with a benzene ring (preferably cyclopropyl, cyclohexyl, indanyl, tetrahydronaphthyl), a 6-membered aromatic heterocyclic group (preferably pyridyl, oxadiazolyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), or a C₇₋₁₀ cross-linked hydrocarbon group (preferably norbornanyl, adamantyl), each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group(s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group; and
(31) a C₁₋₆ alkylsulfonyloxy group optionally substituted by 1 to 3 halogen atoms;

R² is a hydrogen atom; or a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), a 6-membered non-aromatic heterocyclic group (preferably piperidinyl), or a 5- or 6-membered non-aromatic heterocyclic group condensed with a benzene ring (preferably tetrahydroquinolinyl, tetrahydroisoquinolinyl), each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         -(a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group; and
(31) a C₁₋₆ alkylsulfonyloxy group optionally substituted by 1 to 3 halogen atoms;

X is N or CR³ (R³ is
a hydrogen atom;
a C₁₋₆ alkyl group;
a C₆₋₁₄ aryl group;
a C₇₋₁₃ aralkyl group optionally substituted by 1 to 3 substituents selected from a halogen atom, a C₁₋₆ alkyl group and a C₁₋₆ alkoxy group;
a carboxyl group;
a carbamoyl group;
a C₁₋₆ alkoxy-carbonyl group; or
a carbamoyl group mono- or di-substituted by substituent(s) selected from a C₁₋₆ alkyl group, a C₇₋₁₃ aralkyl group and a C₂₋₆
alkenyloxy group);

L¹ is
a bond;
a C₁₋₆ alkylene group (preferably -CH₂-, -CH(CH₃)-, -C(CH₃)₂-, -(CH₂)₂C(CH₃)₂-) optionally substituted by C₃₋₁₀ cycloalkyl group (s) (preferably cyclopropyl); or -(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by oxo group(s), and m and n are the same or different and each is 0 or 1);

L² is
a bond;
a C₁₋₆ alkylene group optionally substituted by substituent(s) selected from an oxo group, a hydroxy group and a hydroxyimino group optionally substituted by a C₁₋₆ alkyl group (preferably hydroxyimino, methoxyimino);
-(akn¹)ₘ-O- (akn²)ₙ- (akn¹ and akn² are each a C₁₋₃ alkylene group optionally substituted by oxo group(s), and m and n are the same or different and each is 0 or 1);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by oxo group(s), and m and n are the same or different and each is 0 or 1);
-(akn¹)ₘ-S-(akn²)ₙ- (akn¹ and akn² are each a C₁₋₃ alkylene group optionally substituted by oxo group(s), and m and n are the same or different and each is 0 or 1);
-(akn¹)ₘ-SO-(akn²)ₙ- (akn¹ and akn² are each a C₁₋₃ alkylene group optionally substituted by oxo group(s), and m and n are the same or different and each is 0 or 1); or
-(akn¹)ₘ-SO₂-(akn²)ₙ- (akn¹ and akn² are each a C₁₋₃ alkylene group optionally substituted by a oxo group(s), and m and n are the same or different and each is 0 or 1); and

ring A is a 5- or 6-membered non-aromatic heterocycle (preferably pyrrolidine, imidazolidine, piperidine, hexahydropyrimidine, hexamethylenimine) optionally substituted by 1 to 3 substituents selected from
a C₁₋₆ alkyl group optionally substituted by 1 to 3 substituents selected from a hydroxy group, a C₆₋₁₄ aryloxy group and a C₁₋₆ alkyl-carbamoyloxy group;
a hydroxy group;
an oxo group;
a carboxyl group;
an amino group optionally mono- or di-substituted by C₁₋₆ alkoxy-carbonyl group(s); and
a C₁₋₆ alkoxy-carbonyl group.

### [Compound B]

A compound wherein
R¹ is a C₆₋₁₄ aryl group (preferably phenyl), a C₃₋₆ cycloalkyl group optionally condensed with a benzene ring (preferably cyclohexyl, cyclopropyl, indanyl, tetrahydronaphthyl), a 6-membered aromatic heterocyclic group (preferably pyridyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), or a C₇₋₁₀ cross-linked hydrocarbon group (preferably norbornanyl, adamantyl), each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group, an amino group, a C₁₋₆ alkyl-carbonylamino group (e.g., acetylamino) and a non-aromatic heterocyclic group (e.g., piperidino);
(9) a C₂₋₆ alkenyl group (e.g., ethenyl, 1-propenyl) optionally substituted by 1 to 3 C₁₋₆ alkoxy-carbonyl groups;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from a C₁₋₆ alkyl group and a C₁₋₆ alkylsulfonyl group;
(12) a C₇₋₁₃ aralkyl group (e.g., benzyl);
(13) an aromatic heterocyclic group (e.g., furyl, pyridyl, pyrimidinyl);
(14) a non-aromatic heterocyclic group (e.g., piperazinyl, oxazolidinyl) optionally substituted by 1 to 3 substituents selected from a C₁₋₆ alkyl group optionally substituted by 1 to 3 hydroxy groups, an oxo group and a C₁₋₆ alkyl-carbonyl group;
(15) a C₁₋₆ alkoxy group optionally substituted by 1 to 3 substituents selected from the following (i) to (x)
   (i) a halogen atom,
   (ii) a hydroxy group,
   (iii) a cyano group,
   (iv) a carboxyl group,
   (v) a C₁₋₆ alkoxy group,
   (vi) a C₇₋₁₃ aralkyloxy group (e.g., benzyloxy),
   (vii) a C₁₋₆ alkyl-carbonyl group,
   (viii) a C₁₋₆ alkoxy-carbonyl group,
   (ix) a carbamoyl group optionally mono- or di-substituted by C₁₋₆ alkyl group(s) (e.g., methylcarbamoyl, dimethylcarbamoyl, diethylcarbamoyl), and
   (x) a non-aromatic heterocyclic group (e.g., pyrrolidinyl);
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xi)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from a hydroxy group and a C₁₋₆ alkyl-carbonyloxy group (e.g., acetyloxy, tert-butylcarbonyloxy),
   (v) a C₁₋₆ alkoxy-carbonyl group,
   (vi) a C₆₋₁₄ aryl-carbonyl group (e.g., benzoyl),
   (vii) a C₇₋₁₃ aralkyl-carbonyl group (e.g., benzylcarbonyl, phenethylcarbonyl),
   (viii) a C₁₋₆ alkylsulfonyl group optionally substituted by 1,3-dioxo-1,3-dihydro-2H-isoindol-2-yl group,
   (ix) a C₆₋₁₄ arylsulfonyl group (e.g., benzenesulfonyl, toluenesulfonyl, 1-naphthalenesulfonyl, 2-naphthalenesulfonyl),
   (x) a C₇₋₁₃ aralkylsulfonyl group (e.g., benzylsulfonyl), and
   (xi) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (n-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl),
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(27) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom; and
(28) a tri-C₁₋₆ alkyl-silyloxy group (e.g., trimethylsilyloxy, triethylsilyloxy, tert-butyldimethylsilyloxy);

R² is a hydrogen atom; or a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), or a 6-membered non-aromatic heterocyclic group (preferably piperidinyl), each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group, an amino group, a C₁₋₆ alkyl-carbonylamino group (e.g., acetylamino) and a non-aromatic heterocyclic group (e.g., piperidino);
(9) a C₂₋₆ alkenyl group (e.g., ethenyl, 1-propenyl) optionally substituted by 1 to 3 C₁₋₆ alkoxy-carbonyl groups;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from a C₁₋₆ alkyl group and a C₁₋₆ alkylsulfonyl group;
(12) a C₇₋₁₃ aralkyl group (e.g., benzyl);
(13) an aromatic heterocyclic group (e.g., furyl, pyridyl, pyrimidinyl);
(14) a non-aromatic heterocyclic group (e.g., piperazinyl, oxazolidinyl) optionally substituted by 1 to 3 substituents selected from a C₁₋₆ alkyl group optionally substituted by 1 to 3 hydroxy groups, an oxo group and a C₁₋₆ alkyl-carbonyl group;
(15) a C₁₋₆ alkoxy group optionally substituted by 1 to 3 substituents selected from the following (i) to (x)
   (i) a halogen atom,
   (ii) a hydroxy group,
   (iii) a cyano group,
   (iv) a carboxyl group,
   (v) a C₁₋₆ alkoxy group,
   (vi) a C₇₋₁₃ aralkyloxy group (e.g., benzyloxy),
   (vii) a C₁₋₆ alkyl-carbonyl group,
   (viii) a C₁₋₆ alkoxy-carbonyl group,
   (ix) a carbamoyl group optionally mono- or di-substituted by C₁₋₆ alkyl group(s) (e.g., methylcarbamoyl; dimethylcarbamoyl, diethylcarbamoyl), and
   (x) a non-aromatic heterocyclic group (e.g., pyrrolidinyl);
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xi)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from a hydroxy group and a C₁₋₆ alkyl-carbonyloxy group (e.g., acetyloxy, tert-butylcarbonyloxy),
   (v) a C₁₋₆ alkoxy-carbonyl group,
   (vi) a C₆₋₁₄ aryl-carbonyl group (e.g., benzoyl),
   (vii) a C₇₋₁₃ aralkyl-carbonyl group (e.g., benzylcarbonyl, phenethylcarbonyl),
   (viii) a C₁₋₆ alkylsulfonyl group optionally substituted by 1,3-dioxo-1,3-dihydro-2H-isoindol-2-yl group,
   (ix) a C₆₋₁₄ arylsulfonyl group (e.g., benzenesulfonyl, toluenesulfonyl, 1-naphthalenesulfonyl, 2-naphthalenesulfonyl),
   (x) a C₇₋₁₃ aralkylsulfonyl group (e.g., benzylsulfonyl), and
   (xi) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl),
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(27) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom; and
(28) a tri-C₁₋₆ alkyl-silyloxy group (e.g., trimethylsilyloxy, triethylsilyloxy, tert-butyldimethylsilyloxy);

X is N or CR³ (R³ is
a hydrogen atom;
a C₁₋₆ alkyl group;
a C₆₋₁₄ aryl group;
a C₇₋₁₃ aralkyl group optionally substituted by 1 to 3 substituents selected from a halogen atom, a C₁₋₆ alkyl group and a C₁₋₆ alkoxy group;
a carboxyl group;
a carbamoyl group;
a C₁₋₆ alkoxy-carbonyl group; or
a carbamoyl group mono- or di-substituted by substituent(s) selected from a C₁₋₆ alkyl group, a C₇₋₁₃ aralkyl group and a C₂₋₆ alkenyloxy group);

L¹ is
a bond;
a C₁₋₆ alkylene group (preferably -CH₂-, -CH(CH₃)-, -C(CH₃)₂-) optionally substituted by C₃₋₁₀ cycloalkyl group(s) (preferably cyclopropyl); or -(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by oxo group(s), and m and n are the same or different and each is 0 or 1);

L² is
a bond;
a C₁₋₆ alkylene group optionally substituted by substituent(s) selected from an oxo group, a hydroxy group and a hydroxyimino group optionally substituted by a C₁₋₆ alkyl group (preferably hydroxyimino, methoxyimino);
-(akn¹)ₘ-O-(akn²)ₙ- (akn¹ and akn² are each a C₁₋₃ alkylene group optionally substituted by oxo group(s), and m and n are the same or different and each is 0 or 1); or
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by oxo group(s), and m and n are the same or different and each is 0 or 1); and

ring A is a 5- or 6-membered non-aromatic heterocycle (preferably pyrrolidine, imidazolidine, piperidine, hexahydropyrimidine, hexamethylenimine) optionally substituted by 1 to 3 substituents selected from
a C₁₋₆ alkyl group optionally substituted by 1 to 3 substituents selected from a hydroxy group, a C₆₋₁₄ aryloxy group and a C₁₋₆ alkyl-carbamoyloxy group;
a hydroxy group;
an oxo group;
a carboxyl group;
an amino group optionally mono- or di-substituted by C₁₋₆ alkoxy-carbonyl group(s); and
a C₁₋₆ alkoxy-carbonyl group.

### [Compound C]

A compound wherein
R¹ is a C₆₋₁₄ aryl group (preferably phenyl), a C₃₋₆ cycloalkyl group optionally condensed with a benzene ring (preferably cyclohexyl, cyclopropyl, indanyl, tetrahydronaphthyl), a 6-membered aromatic heterocyclic group (preferably pyridyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl), norbornanyl or adamantly, each of which is optionally substituted by 1 to 3 substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group and a non-aromatic heterocyclic group (e.g., piperidino);
(9) a C₆₋₁₄ aryl group (e.g., phenyl, naphthyl) optionally substituted by 1 to 3 C₁₋₆ alkyl groups;
(10) a C₁₋₆ alkoxy group optionally substituted by 1 to 3 halogen atoms;
(11) a C₃₋₁₀ cycloalkyl-C₁₋₆ alkyloxy group (e.g., cyclopropylmethyloxy);
(12) a C₁₋₆ alkylthio group (e.g., methylthio, ethylthio);
(13) an amino group optionally mono- or di-substituted by substituent (s) selected from a C₁₋₆ alkyl group, a C₁₋₆ alkyl-carbonyl group (e.g., acetyl, 2-methylpropanoyl, 3-methylbutanoly), a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkyl-aminocarbonyl group (e.g., methylaminocarbonyl, ethylaminocarbonyl), a C₆₋₁₄ aryl-aminocarbonyl group (e.g., phenylaminocarbonyl, 1-naphthylaminocarbonyl, 2-naphthylaminocarbonyl), a C₇₋₁₃ aralkyl-aminocarbonyl group (e.g., benzylaminocarbonyl), a C₁₋₆ alkylsulfonyl group, a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(14) a C₁₋₆ alkyl-carbonyl group (e.g., acetyl, 2-methylpropanoyl, 3-methylbutanoly);
(15) a C₁₋₆ alkoxy-carbonyl group;
(16) a C₁₋₆ alkylsulfinyl group (e.g., methylsulfinyl);
(17) a C₁₋₆ alkylsulfonyl group (e.g., methylsulfonyl);
(18) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl); and
(19) a C₇₋₁₃ aralkyloxy group (e.g., benzyloxy) ;

R² is a hydrogen atom; or a C₆₋₁₄ aryl group (preferably phenyl, naphthyl), a C₃₋₆ cycloalkyl group (preferably cyclopropyl, cyclohexyl), a 5- or 6-membered aromatic heterocyclic group (preferably pyridyl, imidazolyl), or a 6-membered non-aromatic heterocyclic group (preferably piperidinyl), each of which is optionally substituted by 1 to 3 substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group and a non-aromatic heterocyclic group (e.g., piperidino);
(9) a C₆₋₁₄ aryl group (e.g., phenyl, naphthyl) optionally substituted by 1 to 3 C₁₋₆ alkyl groups;
(10) a C₁₋₆ alkoxy group optionally substituted by 1 to 3 halogen atoms;
(11) a C₃₋₁₀ cycloalkyl-C₁₋₆ alkyloxy group (e.g., cyclopropylmethyloxy);
(12) a C₁₋₆ alkylthio group (e.g., methylthio, ethylthio);
(13) an amino group optionally mono- or di-substituted by substituent(s) selected from a C₁₋₆ alkyl group, a C₁₋₆ alkyl-carbonyl group (e.g., acetyl, 2-methylpropanoyl, 3-methylbutanoly), a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkyl-aminocarbonyl group (e.g., methylaminocarbonyl, ethylaminocarbonyl), a C₆₋₁₄ aryl-aminocarbonyl group (e.g., phenylaminocarbonyl, 1-naphthylaminocarbonyl, 2-naphthylaminocarbonyl), a C₇₋₁₃ aralkyl-aminocarbonyl group (e.g., benzylaminocarbonyl), a C₁₋₆ alkylsulfonyl group (e.g., methylsulfonyl, ethylsulfonyl, isopropylsulfonyl), a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(14) a C₁₋₆ alkyl-carbonyl group (e.g., acetyl, 2-methylpropanoyl, 3-methylbutanoly);
(15) a C₁₋₆ alkoxy-carbonyl group;
(16) a C₁₋₆ alkylsulfinyl group (e.g., methylsulfinyl);
(17) a C₁₋₆ alkylsulfonyl group (e.g., methylsulfonyl);
(18) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl); and
(19) a C₇₋₁₃ aralkyloxy group (e.g., benzyloxy);

X is N or CR³ (R³ is
a hydrogen atom;
a C₁₋₆ alkyl group;
a C₆₋₁₄ aryl group; or
a C₇₋₁₃ aralkyl group optionally substituted by 1 to 3 substituents selected from a halogen atom, a C₁₋₆ alkyl group and a C₁₋₆ alkoxy group);

L¹ is
a bond;
a C₁₋₆ alkylene group (preferably -CH₂-, -CH(CH₃)-, -C(CH₃)₂-) optionally substituted by C₃₋₁₀ cycloalkyl group(s) (preferably cyclopropyl); or -(akn¹)ₘ-NR⁴-(akn²)ₙ- (R⁴ is a hydrogen atom or a C₁₋₆ alkyl group, akn¹ and akn² are each a Cₗ-₃ alkylene group optionally substituted by oxo group(s), and m and n are the same or different and each is 0 or 1);

L² is
a bond;
a C₁₋₆ alkylene group optionally substituted by substituent(s) selected from an oxo group, a hydroxy group and a hydroxyimino group optionally substituted by a C₁₋₆ alkyl group (preferably hydroxyimino, methoxyimino);
-(akn¹)ₘO-(akn²)n- (akn¹ and akn² are each a C₁₋₃ alkylene group optionally substituted by oxo group(s), and m and n are the same or different and each is 0 or 1); or
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by oxo group(s), and m and n are the same or different and each is 0 or 1); and
ring A is a 5- or 6-membered non-aromatic heterocycle (preferably pyrrolidine, imidazolidine, piperidine, hexahydropyrimidine) optionally substituted by 1 to 3 substituents selected from a C₁₋₆ alkyl group, a hydroxy group and an oxo group.

Of compound (1), a compound represented by the following the formula (2): 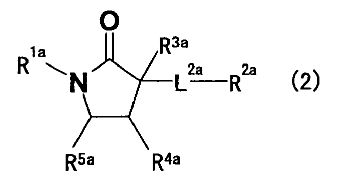 wherein
R^{1a} is an optionally substituted non-aromatic cyclic group provided that the non-aromatic cyclic group should not be 4,5α-epoxymorphinanyl group, and the non-aromatic cyclic group should not have, as a substituent, a group represented by R*'*-E- (E is -S (O)ₜ-CHR^{e}-, -CHR^{e}NR^{e}-, -C(O)-NR^{e}-, -NR^{e}-C(O)NR^{e}-, -SO₂-NR^{e}- or -NR^{e}-SO₂NR^{e}- (t is an integer of 0 to 2; and R^{e} is a hydrogen atom or a C₁₋₃ alkyl group); and R' is an optionally substituted C₆₋₁₀ aryl group or an optionally substituted 5- to 10-membered aromatic heterocyclic group);
R^{2a} is an optionally substituted cyclic group;
L^{2a} is an optionally substituted divalent aliphatic hydrocarbon group (provided that the divalent aliphatic hydrocarbon group should not have a hydroxy group as a substituent), or a group represented by -(akn¹)ₘ-Y-(akn²)ₙ- (akn¹ and akn² are the same or different and each is an optionally substituted C₁₋₆ alkylene group; m and n are the same or different and each is 0 or 1; and Y is -O-, -S-, -SO-, -SO₂-, -NR⁴-, -SO₂NR⁴- or -NR⁴SO₂- (R⁴ is a hydrogen atom or an optionally substituted C₁₋₆ alkyl group));
R^{3a} is a hydrogen atom, an optionally substituted hydroxy group, an optionally substituted mercapto group, an optionally substituted amino group or an acyl group; and
R^{4a} and R^{5a} are the same or different and each is a hydrogen atom or a substituent,
or a salt thereof (hereinafter to be abbreviated as compound (2)) is one of preferable embodiments of the present invention.

Each symbol in the formula (2) is described in detail in the following.

As the "non-aromatic cyclic group" of the "optionally substituted non-aromatic cyclic group" for R^{1a}, those similar to the "non-aromatic cyclic group" exemplified as the "cyclic group" of the aforementioned "optionally substituted cyclic group" for R¹ can be mentioned. However, the non-aromatic cyclic group should not contain 4,5a-epoxymorphinanyl group.

The "non-aromatic cyclic group" of the "optionally substituted non-aromatic cyclic group" for R^{1a} is preferably a C₃₋₁₀ cycloalkyl group optionally condensed with a benzene ring, a non-aromatic heterocyclic group, a C₇₋₁₀ cross-linked hydrocarbon group (preferably norbornanyl, adamantyl) or the like, more preferably a C₃₋₆ cycloalkyl group optionally condensed with a benzene ring (preferably cyclopropyl, cyclohexyl, indanyl, tetrahydronaphthyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, particularly preferably a C₃₋₆ cycloalkyl group (preferably cyclohexyl), a C₇₋₁₀ cross-linked hydrocarbon group (preferably adamantyl) or the like.

As the "cyclic group" of the "optionally substituted cyclic group" for R^{2a}, those similar to the "cyclic group" of the aforementioned "optionally substituted cyclic group" for R² can be mentioned.

The "cyclic group" of the "optionally substituted cyclic group" for R^{2a} is preferably a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), a 6-membered non-aromatic heterocyclic group (preferably piperidinyl), a 5- or 6-membered non-aromatic heterocyclic group condensed with a benzene ring (preferably tetrahydroquinolinyl, tetrahydroisoquinolinyl) or the like, more preferably a C₆₋₁₄ aryl group (preferably phenyl, naphthyl), particularly preferably a phenyl group.

The "non-aromatic cyclic group" of the "optionally substituted non-aromatic cyclic group" for R^{1a} and the "cyclic group" of the "optionally substituted cyclic group" for R^{2a} optionally have 1 to 5, preferably 1 to 3, substituents at substitutable positions. As such substituents, those exemplified as preferable substituents of the "cyclic group" of the aforementioned "optionally substituted cyclic group" for R¹ or R² can be mentioned.

As more preferable substituents,
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group, an amino group, a C₁₋₆ alkyl-carbonylamino group (e.g., acetylamino) and a non-aromatic heterocyclic group (e.g., piperidino);
(9) a C₂₋₆ alkenyl group (e.g., ethenyl, 1-propenyl) optionally substituted by 1 to 3 C₁₋₆ alkoxy-carbonyl groups;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from a C₁₋₆ alkyl group and a C₁₋₆ alkylsulfonyl group;
(12) a C₇₋₁₃ aralkyl group (e.g., benzyl);
(13) an aromatic heterocyclic group (e.g., furyl, pyridyl, pyrimidinyl);
(14) a non-aromatic heterocyclic group (e.g., piperazinyl, oxazolidinyl) optionally substituted by 1 to 3 substituents selected from a C₁₋₆ alkyl group optionally substituted by 1 to 3 hydroxy groups, an oxo group and a C₁₋₆ alkyl-carbonyl group;
(15) a C₁₋₆ alkoxy group optionally substituted by 1 to 3 substituents selected from the following (i) to (x)
   (i) a halogen atom,
   (ii) a hydroxy group,
   (iii) a cyano group,
   (iv) a carboxyl group,
   (v) a C₁₋₆ alkoxy group,
   (vi) a C₇₋₁₃ aralkyloxy group (e.g., benzyloxy),
   (vii) a C₁₋₆ alkyl-carbonyl group,
   (viii) a C₁₋₆ alkoxy-carbonyl group,
   (ix) a carbamoyl group optionally mono- or di-substituted by C₁₋₆ alkyl group(s) (e.g., methylcarbamoyl, dimethylcarbamoyl, diethylcarbamoyl), and
   (x) a non-aromatic heterocyclic group (e.g., pyrrolidinyl);
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xi)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from a hydroxy group and a C₁₋₆ alkyl-carbonyloxy group (e.g., acetyloxy, tert-butylcarbonyloxy),
   (v) a C₁₋₆ alkoxy-carbonyl group,
   (vi) a C₆₋₁₄ aryl-carbonyl group (e.g., benzoyl),
   (vii) a C₇₋₁₃ aralkyl-carbonyl group (e.g., benzylcarbonyl, phenethylcarbonyl),
   (viii) a C₁₋₆ alkylsulfonyl group optionally substituted by 1,3-dioxo-1,3-dihydro-2H-isoindol-2-yl group,
   (ix) a C₆₋₁₄ arylsulfonyl group (e.g., benzenesulfonyl, toluenesulfonyl, 1-naphthalenesulfonyl, 2-naphthalenesulfonyl),
   (x) a C₇₋₁₃ aralkylsulfonyl group (e.g., benzylsulfonyl), and
   (xi) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group (s) (e.g., methylcarbamoyl),
      (c) a C₆₋₁₄ aryl group (e.g., phenyl, 1-naphthyl, 2-nathpthyl),
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(27) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(28) a tri-C₁₋₆ alkyl-silyloxy group (e.g., trimethylsilyloxy, triethylsilyloxy, tert-butyldimethylsilyloxy);
and the like can be mentioned.

As particularly preferable substituents,
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom, a hydroxy group and a non-aromatic heterocyclic group (e.g., piperidino);
(9) a C₆₋₁₄ aryl group (e.g., phenyl, naphthyl) optionally substituted by 1 to 3 C₁₋₆ alkyl groups;
(10) a C₁₋₆ alkoxy group optionally substituted by 1 to 3 halogen atoms;
(11) a C₃₋₁₀ cycloalkyl-C₁₋₆ alkyloxy group (e.g., cyclopropylmethyloxy);
(12) a C₁₋₆ alkylthio group (e.g., methylthio, ethylthio);
(13) an amino group optionally mono- or di-substituted by substituent(s) selected from a C₁₋₆ alkyl group, a C₁₋₆ alkyl-carbonyl group, a C₁₋₆ alkoxy-carbonyl group, a C₆₋₁₄ aryl-carbonyl group (e.g., benzoyl), a C₇₋₁₃ aralkyl-carbonyl group (e.g., benzylcarbonyl, phenethylcarbonyl), a C₁₋₆ alkyl-aminocarbonyl group (e.g., methylaminocarbonyl, ethylaminocarbonyl), a C₆₋₁₄ aryl-aminocarbonyl group (e.g., phenylaminocarbonyl, 1-naphthylaminocarbonyl, 2-naphthylaminocarbonyl), a C₇₋₁₃ aralkyl-aminocarbonyl group (e.g., benzylaminocarbonyl), a C₁₋₆ alkylsulfonyl group, a C₆₋₁₄ arylsulfonyl group (e.g., benzenesulfonyl, toluenesulfonyl, 1-naphthalenesulfonyl, 2-naphthalenesulfonyl) and a C₇₋₁₃ aralkylsulfonyl group (e.g., benzylsulfonyl);
(14) a C₁₋₆ alkyl-carbonyl group;
(15) a C₁₋₆ alkoxy-carbonyl group;
(16) a C₁₋₆ alkylsulfinyl group (e.g., methylsulfinyl);
(17) a C₁₋₆ alkylsulfonyl group;
(18) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(19) a C₇₋₁₃ aralkyloxy group (e.g., benzyloxy);
and the like can be mentioned.

The "non-aromatic cyclic group" of the "optionally substituted non-aromatic cyclic group" for R^{1a} should not have, as a substituent, a group represented by R'-E- (E is -S(O)ₜ-CHR^{e}-, -CHR^{e}NR^{e}-, -C(O)-NR^{e}-, -NR^{e}-C(O)NR^{e}-, -SO₂-NR^{e}- or -NR^{e}-SO₂NR^{e}- (t is an integer of 0 to 2; and R^{e} is a hydrogen atom or a C₁₋₃ alkyl group); and R' is an optionally substituted C₆₋₁₀ aryl group or an optionally substituted 5- to 10-membered aromatic heterocyclic group).

R^{1a} is preferably a C₃₋₆ cycloalkyl group optionally condensed with a benzene ring (preferably cyclopropyl, cyclohexyl, indanyl, tetrahydronaphthyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) a carbamoyl group;
(7) an oxo group;
(8) a C₁₋₃ alkylenedioxy group;
(9) a C₁₋₆ alkyl group optionally substituted by 1 to 3 substituents selected from the following (i) to (vii)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an aromatic heterocyclic group (e.g., oxadiazolyl) optionally substituted by C₆₋₁₄ aryl group(s) (e.g., phenyl) optionally substituted by 1 to 3 halogen atoms, and
   (vii) a non-aromatic heterocyclyl-carbonyl group (e.g., pyrrolidinylcarbonyl);
(10) a C₂₋₆ alkenyl group (e.g., ethenyl, 1-propenyl) optionally substituted by 1 to 3 substituents selected from a carboxyl group and a C₁₋₆ alkoxy-carbonyl group;
(11) a C₂₋₆ alkynyl group (e.g., ethynyl, 1-propynyl);
(12) a C₆₋₁₄ aryl group (e.g., phenyl) optionally substituted by 1 to 3 halogen atoms;
(13) a C₇₋₁₃ aralkyl group (e.g., benzyl);
(14) a C₁₋₆ alkoxy group optionally substituted by 1 to 3 halogen atoms;
(15) an amino group optionally mono- or di-substituted by C₁₋₆ alkyl group(s);
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a non-aromatic heterocyclic group (e.g., piperidinyl);
(22) a formyl group;
and the like.

R^{1a} is more preferably a C₃₋₆ cycloalkyl group optionally condensed with a benzene ring (preferably cyclopropyl, cyclohexyl, indanyl, tetrahydronaphthyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from a halogen atom and a hydroxy group;
(9) a C₂₋₆ alkynyl group (e.g., ethynyl, 1-propynyl);
(10) a C₇₋₁₃ aralkyl group (e.g., benzyl);
(11) a C₁₋₆ alkoxy group optionally substituted by 1 to 3 halogen atoms;
(12) an amino group optionally mono- or di-substituted by C₁₋₆ alkyl group(s);
(13) a C₁₋₆ alkyl-carbonyl group;
(14) a C₁₋₆ alkoxy-carbonyl group;
(15) a C₇₋₁₃ aralkyloxy-carbonyl group (e.g., benzyloxycarbonyl);
(16) a C₁₋₆ alkylsulfinyl group (e.g., methylsulfinyl);
(17) a C₁₋₆ alkylsulfonyl group;
and the like.

R^{1a} is particularly preferably a C₃₋₆ cycloalkyl group (preferably cyclohexyl) or a C₇₋₁₀ cross-linked hydrocarbon group (preferably adamantyl), each of which is optionally substituted by 1 to 5 (preferably 1 to 3) substituents selected from
(1) a hydroxy group;
(2) an oxo group;
(3) a C₁₋₃ alkylenedioxy group;
(4) a C₁₋₆ alkyl group optionally substituted by 1 to 3 substituents selected from a halogen atom and a hydroxy group;
(5) a C₂₋₆ alkynyl group (e.g., ethynyl, 1-propynyl);
(6) a C₁₋₆ alkoxy group optionally substituted by 1 to 3 halogen atoms;
(7) an amino group optionally mono- or di-substituted by C₁₋₆ alkyl group(s);
and the like.

R^{2a} is preferably a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl), a 6-membered non-aromatic heterocyclic group (preferably piperidinyl), a 5- or 6-membered non-aromatic heterocyclic group condensed with a benzene ring (preferably tetrahydroquinolinyl, tetrahydroisoquinolinyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group(s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy) ;
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group;
(31) a C₁₋₆ alkylsulfonyloxy group optionally substituted by 1 to 3 halogen atoms;
   and the like.

When the "non-aromatic cyclic group" of the "optionally substituted non-aromatic cyclic group" for R^{1a} is a C₇₋₁₀ cross-linked hydrocarbon group (preferably norbornanyl, adamantyl), R^{2a} is preferably a phenyl group having a substituent at the para-position. The phenyl group of the "phenyl group having a substituent at the para-position" optionally further has 1 to 3 substituents, besides the substituent at the para-position. As the substituents other than the substituent at the para-position, those exemplified as preferable substituents of the "cyclic group" of the aforementioned "optionally substituted cyclic group" for R¹ or R² can be mentioned. The substituent at the para-position is selected from the following.
(1) a nitro group;
(2) a carboxyl group;
(3) a C₁₋₃ alkylenedioxy group;
(4) a C₁₋₆ alkyl group substituted by 1 to 3 substituents selected from the following (i) to (viii)
   (i) a carboxyl group,
   (ii) a hydroxy group,
   (iii) a C₁₋₆ alkoxy-carbonyl group,
   (iv) a carbamoyl group optionally mono- or di-substituted by substituent(s) selected from a C₆₋₁₄ aryl group (e.g., phenyl) and a C₇₋₁₃ aralkyl group (e.g., benzyl),
   (v) an amino group optionally mono- or di-substituted by C₁₋₆ alkyl-carbonyl group(s),
   (vi) an aromatic heterocyclic group (e.g., oxadiazolyl) optionally substituted by C₆₋₁₄ aryl group(s) (e.g., phenyl) optionally substituted by 1 to 3 halogen atoms,
   (vii) a non-aromatic heterocyclic group (e.g., piperidino), and
   (viii) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(5) a C₂₋₆ alkenyl group (e.g., ethenyl, 1-propenyl) optionally substituted by 1 to 3 substituents selected from a carboxyl group and a C₁₋₆ alkoxy-carbonyl group;
(6) a C₂₋₆ alkynyl group (e.g., ethynyl, 1-propynyl) optionally substituted by 1 to 3 hydroxy groups;
(7) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(8) a C₇₋₁₃ aralkyl group (e.g., benzyl);
(9) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(10) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(11) a C₁₋₆ alkoxy group substituted by 1 to 3 substituents selected from the following (i) to (xii)
   (i) a cyano group,
   (ii) a carboxyl group,
   (iii) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (iv) a C₆₋₁₄ aryloxy group (e.g., phenoxy),
   (v) a C₇₋₁₃ aralkyloxy group (e.g., benzyloxy) optionally substituted by 1 to 3 halogen atoms,
   (vi) a C₁₋₆ alkyl-carbonyl group,
   (vii) a C₁₋₆ alkoxy-carbonyl group,
   (viii) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (ix) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (x) an aromatic heterocyclyl-oxy group (e.g., pyridyloxy) optionally substituted by C₁₋₆ alkyl group(s) optionally substituted by 1 to 3 halogen atoms,
   (xi) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xii) a C₁₋₆ alkoxy group;
(12) a C₂₋₆ alkynyloxy group (e.g., ethynyloxy, 2-propynyloxy);
(13) a C₃₋₁₀ cycloalkyl-C₁₋₆ alkyloxy group (e.g., cyclopropylmethyloxy);
(14) a C₁₋₆ alkylthio group (e.g., methylthio, ethylthio);
(15) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(22) a C₆₋₁₄ aryloxy group (e.g., phenoxy);
(23) a C₇₋₁₃ aralkyloxy group (e.g., benzyloxy) substituted by 1 to 3 substituents selected from a C₁₋₆ alkyl group substituted by 1 to 3 hydroxy groups, a carboxyl group and a C₁₋₆ alkoxy-carbonyl group;
(24) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(25) a tri-C₁₋₆ alkyl-silyloxy group (e.g., trimethylsilyloxy, triethylsilyloxy, tert-butyldimethylsilyloxy);
(26) a formyl group;
(27) a C₁₋₆ alkylsulfonyloxy group optionally substituted by 1 to 3 halogen atoms;
and the like.

The "optionally substituted divalent aliphatic hydrocarbon group" and "-(akn¹)ₘ-Y-(akn²)ₙ-" for L^{2a}, those similar to the "optionally substituted divalent aliphatic hydrocarbon group" and "-(akn¹)ₘ-Y-(akn²)ₙ-" for L¹ or L² can be mentioned. Provided that when L^{2a} is a divalent aliphatic hydrocarbon group, the divalent aliphatic hydrocarbon group should not have a hydroxy group as a substituent.

L^{2a} is preferably
a C₁₋₆ alkylene group optionally substituted by substituent(s) selected from an oxo group, a hydroxyimino group optionally substituted by a C₁₋₆ alkyl group (preferably hydroxyimino, methoxyimino) and the like;
-(akn¹)ₘ-O-(akn²)ₙ- (akn¹ and akn² are each a C₁₋₃ alkylene group optionally substituted by an oxo group and the like, and m and n are the same or different and each is 0 or 1);
-(akn¹)ₘ-NR⁴-(akn²)ₙ-(R⁴is a hydrogen atom or a C₁₋₆ alkyl group, akn¹ and akn² are each a C₁₋₃ alkylene group optionally substituted by an oxo group and the like, and m and n are the same or different and each is 0 or 1);
-(akn¹)ₘ-S-(akn²)ₙ- (akn¹ and akn² are each a C₁₋₃ alkylene group optionally substituted by an oxo group and the like, and m and n are the same or different and each is 0 or 1);
-(akn¹)ₘ-SO-(akn²)ₙ- (akn¹ and akn² are each a C₁₋₃ alkylene group optionally substituted by an oxo group and the like, and m and n are the same or different and each is 0 or 1);
-(akn¹)ₘ-SO₂-(akn²)ₙ- (akn¹ and akn² are each a C₁₋₃ alkylene group optionally substituted by an oxo group and the like, and m and n are the same or different and each is 0 or 1);
or the like.

L^{2a} is more preferably
a C₁₋₆ alkylene group optionally substituted by substituent(s) selected from an oxo group, a hydroxyimino group optionally substituted by a C₁₋₆ alkyl group (preferably hydroxyimino, methoxyimino) and the like;
-(akn¹)ₘ-O-(akn²)ₙ- (akn¹ and akn² are each a C₁₋₃ alkylene group optionally substituted by an oxo group and the like, and m and n are the same or different and each is 0 or 1);
-(akn¹)ₘ-NR⁴-(akn²)ₙ- (R⁴ is a hydrogen atom or a C₁₋₆ alkyl group, akn¹ and akn² are each a C₁₋₃ alkylene group optionally substituted by an oxo group and the like, and m and n are the same or different and each is 0 or 1);
or the like.

L^{2a} is particularly preferably a C₁₋₆ alkylene group.

As the "optionally substituted hydroxy group", "optionally substituted mercapto group", "optionally substituted amino group" and "acyl group" for R^{3a}, those similar to the "optionally substituted hydroxy group", "optionally substituted mercapto group", "optionally substituted amino group" and "acyl group" exemplified as the aforementioned "substituent" for R³ can be mentioned.

R^{3a} is preferably
a hydrogen atom;
a carboxyl group;
a carbamoyl group;
a C₁₋₆ alkoxy-carbonyl group;
a carbamoyl group mono- or di-substituted substituent(s) selected from a C₁₋₆ alkyl group, a C₇₋₁₃ aralkyl group and a C₂₋₆ alkenyloxy group
or the like, more preferably a hydrogen atom.

As the "substituent" for R^{4a} or R^{5a}, those similar to the aforementioned "substituent" for R³ can be mentioned.

R^{4a} and R^{5a} are the same or different and each is
preferably
a hydrogen atom;
a C₁₋₆ alkyl group optionally substituted by 1 to 3 substituents selected from a hydroxy group, a C₆₋₁₄ aryloxy group (e.g., phenoxy) and a C₁₋₆ alkyl-carbamoyloxy group (e.g.,
ethylcarbamoyloxy);
a hydroxy group;
a carboxyl group;
an amino group optionally mono- or di-substituted by C₁₋₆ alkoxycarbonyl group(s);
a C₁₋₆ alkoxy-carbonyl group
or the like.

R^{4a} is more preferably
a hydrogen atom;
a C₁₋₆ alkyl group optionally substituted by 1 to 3 substituents selected from a hydroxy group, a C₆₋₁₄ aryloxy group (e.g., phenoxy) and a C₁₋₆ alkyl-carbamoyloxy group (e.g.,
ethylcarbamoyloxy);
a hydroxy group;
a carboxyl group;
an amino group optionally mono- or di-substituted by C₁₋₆ alkoxy-carbonyl group(s);
a C₁₋₆ alkoxy-carbonyl group
or the like, particularly preferably a hydrogen atom.

R^{5a} is more preferably
a hydrogen atom;
a carboxyl group;
a C₁₋₆ alkoxy-carbonyl group
or the like, particularly preferably a hydrogen atom.

Of compound (2), compound (2a) is preferable.

Each symbol in the formula (2a) is described in detail in the following.

The "non-aromatic cyclic group" of the "optionally substituted non-aromatic cyclic group" for R^{1a'} is selected from a C₃₋₆ cycloalkyl group optionally condensed with a benzene ring, a 6-membered non-aromatic heterocyclic group, a 5- or 6-membered non-aromatic heterocyclic group condensed with a benzene ring, and a C₇₋₁₀ cross-linked hydrocarbon group, and as these, those similar to the "non-aromatic cyclic group" exemplified as the "cyclic group" of the aforementioned "optionally substituted cyclic group" for R¹ can be mentioned.

The C₃₋₆ cycloalkyl group optionally condensed with a benzene ring is preferably cyclopropyl, cyclohexyl, indanyl, tetrahydronaphthyl or the like, the 6-membered non-aromatic heterocyclic group is preferably piperidinyl, tetrahydropyranyl, tetrahydrothiopyranyl, 1-oxidotetrahydrothiopyranyl, 1,1-dioxidotetrahydrothiopyranyl, morpholinyl or the like, the 5- or 6-membered non-aromatic heterocyclic group condensed with a benzene ring is preferably dihydroindolyl or the like, and the C₇₋₁₀ cross-linked hydrocarbon group is preferably norbornanyl, adamantly or the like.

The "non-aromatic cyclic group" of the "optionally substituted non-aromatic cyclic group" for R^{1a'} is particularly preferably a C₃₋₆ cycloalkyl group (preferably cyclohexyl), a C₇₋₁₀ cross-linked hydrocarbon group (preferably adamantyl) or the like.

The "non-aromatic cyclic group" of the "optionally substituted non-aromatic cyclic group" for R^{1a'} optionally has 1 to 5, preferably 1 to 3, substituents at substitutable positions. As such substituents, those exemplified as the substituent of the aforementioned "optionally substituted cyclic group" for R¹ or R² can be mentioned. However, the "non-aromatic cyclic group" of the "optionally substituted non-aromatic cyclic group" for R^{1a'} should not have, as a substituent, a group represented by R'-E- (E is -S(O)ₜ-CHR^{e}-, -CHR^{e}NR^{e}-, -C(O)-NR^{e}-, -NR^{e}-C(O)NR^{e}-, -SO₂-NR^{e}- or -NR^{e}-SO₂NR^{e}- (t is an integer of 0 to 2; and R^{e} is a hydrogen atom or a C₁₋₃ alkyl group); and R' is an optionally substituted C₆₋₁₀ aryl group or an optionally substituted 5- to 10-membered aromatic heterocyclic group).

As preferable substituents,
(1) a halogen atom;
(2) a hydroxy group;
(3) a cyano group;
(4) a nitro group;
(5) a carboxyl group;
(6) a carbamoyl group;
(7) an oxo group;
(8) a C₁₋₃ alkylenedioxy group;
(9) a C₁₋₆ alkyl group optionally substituted by 1 to 3 substituents selected from the following (i) to (vii)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent (s) selected from a C₆₋₁₄ aryl group (e.g., phenyl) and a C₇₋₁₃ aralkyl group (e.g., benzyl),
   (vi) an aromatic heterocyclic group (e.g., oxadiazolyl) optionally substituted by C₆₋₁₄ aryl group(s) (e.g., phenyl) optionally substituted by 1 to 3 halogen atoms, and
   (vii) a non-aromatic heterocyclyl-carbonyl group (e.g., pyrrolidinylcarbonyl);
(10) a C₂₋₆ alkenyl group (e.g., ethenyl, 1-propenyl) optionally substituted by 1 to 3 substituents selected from a carboxyl group and a C₁₋₆ alkoxy-carbonyl group;
(11) a C₂₋₆ alkynyl group (e.g., ethynyl, 1-propynyl);
(12) a C₆₋₁₄ aryl group (e.g., phenyl) optionally substituted by 1 to 3 halogen atoms;
(13) a C₇₋₁₃ aralkyl group (e.g., benzyl);
(14) a C₁₋₆ alkoxy group optionally substituted by 1 to 3 halogen atoms;
(15) an amino group optionally mono- or di-substituted by C₁₋₆ alkyl group(s);
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a non-aromatic heterocyclic group (e.g., piperidinyl);
(22) a formyl group;
and the like can be mentioned.

R^{1a'} is preferably a C₃₋₆ cycloalkyl group optionally condensed with a benzene ring (preferably cyclopropyl, cyclohexyl, indanyl, tetrahydronaphthyl), a 6-membered non-aromatic heterocyclic group (preferably piperidinyl, tetrahydropyranyl, tetrahydrothiopyranyl, 1-oxidotetrahydrothiopyranyl, 1,1-dioxidotetrahydrothiopyranyl, morpholinyl), a 5- or 6-membered non-aromatic heterocyclic group condensed with a benzene ring (preferably dihydroindolyl), a C₇₋₁₀ cross-linked hydrocarbon group (preferably norbornanyl, adamantyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) a carbamoyl group;
(7) an oxo group;
(8) a C₁₋₃ alkylenedioxy group;
(9) a C₁₋₆ alkyl group optionally substituted by 1 to 3 substituents selected from the following (i) to (vii)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an aromatic heterocyclic group (e.g., oxadiazolyl) optionally substituted by C₆₋₁₄ aryl group(s) (e.g., phenyl) optionally substituted by 1 to 3 halogen atoms, and
   (vii) a non-aromatic heterocyclyl-carbonyl group (e.g., pyrrolidinylcarbonyl);
(10) a C₂₋₆ alkenyl group (e.g., ethenyl, 1-propenyl) optionally substituted by 1 to 3 substituents selected from a carboxyl group and a C₁₋₆ alkoxy-carbonyl group;
(11) a C₂₋₆ alkynyl group (e.g., ethynyl, 1-propynyl) ;
(12) a C₆₋₁₄ aryl group (e.g., phenyl) optionally substituted by 1 to 3 halogen atoms;
(13) a C₇₋₁₃ aralkyl group (e.g., benzyl);
(14) a C₁₋₆ alkoxy group optionally substituted by 1 to 3 halogen atoms;
(15) an amino group optionally mono- or di-substituted by C₁₋₆ alkyl group(s);
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a non-aromatic heterocyclic group (e.g., piperidinyl);
(22) a formyl group;
and the like.

R^{1a'} is particularly preferably a C₃₋₆ cycloalkyl group (preferably cyclohexyl), a C₇₋₁₀ cross-linked hydrocarbon group (preferably adamantyl) or the like, each of which is optionally substituted by 1 or 2 substituents selected from
(1) a hydroxy group;
(2) a C₁₋₆ alkyl group optionally substituted by hydroxy group(s) (preferably methyl, hydroxymethyl);
and the like.

As particularly preferable specific examples of R^{1a'}, cyclohexyl having, at the 4-position,
(1) a hydroxy group, and/or
(2) a C₁₋₆ alkyl group optionally substituted by hydroxy group(s) (preferably methyl, hydroxymethyl);
2-adamantyl having, at the 5-position,
(1) a hydroxy group, and/or
(2) a C₁₋₆ alkyl group optionally substituted by hydroxy group(s) (preferably methyl, hydroxymethyl); and
1-adamantyl having, at the 3-position,
(1) a hydroxy group, and/or
(2) a C₁₋₆ alkyl group optionally substituted by hydroxy group(s) (preferably methyl, hydroxymethyl);
can be mentioned.

As the "optionally substituted cyclic group (provided that the cyclic group should not be a non-aromatic heterocyclic group)" for R^{2a'}, a group wherein the "cyclic group" is not a non-aromatic heterocyclic group, from among those exemplified as the aforementioned "optionally substituted cyclic groups" for R^{2a}, can be mentioned.

R^{2a'} is preferably a C₆₋₁₄ aryl group (preferably phenyl, naphthyl), a C₃₋₆ cycloalkyl group (preferably cyclopropyl, cyclohexyl), a C₃₋₆ cycloalkenyl group (preferably cyclohexenyl), a 5- or 6-membered aromatic heterocyclic group (preferably pyridyl, imidazolyl, pyrazolyl) or the like, each of which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group;
(31) a C₁₋₆ alkylsulfonyloxy group optionally substituted by 1 to 3 halogen atoms;
and the like.

When the "non-aromatic cyclic group" of the "optionally substituted non-aromatic cyclic group" for R^{1a'} is a C₇₋₁₀ cross-linked hydrocarbon group (preferably norbornanyl, adamantyl), R^{2a'} is preferably a phenyl group having a substituent at the para-position. The substituent is selected from the following.
(1) a nitro group;
(2) a carboxyl group;
(3) a C₁₋₃ alkylenedioxy group;
(4) a C₁₋₆ alkyl group substituted by 1 to 3 substituents selected from the following (i) to (viii)
   (i) a carboxyl group,
   (ii) a hydroxy group,
   (iii) a C₁₋₆ alkoxy-carbonyl group,
   (iv) a carbamoyl group optionally mono- or di-substituted by substituent(s) selected from a C₆₋₁₄ aryl group (e.g., phenyl) and a C₇₋₁₃ aralkyl group (e.g., benzyl),
   (v) an amino group optionally mono- or di-substituted by C₁₋₆ alkyl-carbonyl group(s),
   (vi) an aromatic heterocyclic group (e.g., oxadiazolyl) optionally substituted by C₆₋₁₄ aryl group(s) (e.g., phenyl) optionally substituted by 1 to 3 halogen atoms,
   (vii) a non-aromatic heterocyclic group (e.g., piperidino), and
   (viii) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(5) a C₂₋₆ alkenyl group (e.g., ethenyl, 1-propenyl) optionally substituted by 1 to 3 substituents selected from a carboxyl group and a C₁₋₆ alkoxy-carbonyl group;
(6) a C₂₋₆ alkynyl group (e.g., ethynyl, 1-propynyl) optionally substituted by 1 to 3 hydroxy groups;
(7) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(8) a C₇₋₁₃ aralkyl group (e.g., benzyl);
(9) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(10) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(11) a C₁₋₆ alkoxy group substituted by 1 to 3 substituents selected from the following (i) to (xii)
   (i) a cyano group,
   (ii) a carboxyl group,
   (iii) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (iv) a C₆₋₁₄ aryloxy group (e.g., phenoxy),
   (v) a C₇₋₁₃ aralkyloxy group (e.g., benzyloxy) optionally substituted by 1 to 3 halogen atoms,
   (vi) a C₁₋₆ alkyl-carbonyl group,
   (vii) a C₁₋₆ alkoxy-carbonyl group,
   (viii) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (ix) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (x) an aromatic heterocyclyl-oxy group (e.g., pyridyloxy) optionally substituted by C₁₋₆ alkyl group(s) optionally substituted by 1 to 3 halogen atoms,
   (xi) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xii) a C₁₋₆ alkoxy group;
(12) a C₂₋₆ alkynyloxy group (e.g., ethynyloxy, 2-propynyloxy);
(13) a C₃₋₁₀ cycloalkyl-C₁₋₆ alkyloxy group (e.g., cyclopropylmethyloxy);
(14) a C₁₋₆ alkylthio group (e.g., methylthio, ethylthio);
(15) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group(s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(22) a C₆₋₁₄ aryloxy group (e.g., phenoxy);
(23) a C₇₋₁₃ aralkyloxy group (e.g., benzyloxy) substituted by 1 to 3 substituents selected from a C₁₋₆ alkyl group substituted by 1 to 3 hydroxy groups, a carboxyl group and a C₁₋₆ alkoxy-carbonyl group;
(24) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(25) a tri-C₁₋₆ alkyl-silyloxy group (e.g., trimethylsilyloxy, triethylsilyloxy, tert-butyldimethylsilyloxy);
(26) a formyl group;
(27) a C₁₋₆ alkylsulfonyloxy group optionally substituted by 1 to 3 halogen atoms;
and the like.

As the aforementioned substituent at the para-position, the following substituents are particularly preferable.
(1) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(2) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(3) a C₁₋₆ alkoxy group substituted by 1 to 3 substituents selected from the following (i) to (xii)
   (i) a cyano group,
   (ii) a carboxyl group,
   (iii) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (iv) a C₆₋₁₄ aryloxy group (e.g., phenoxy),
   (v) a C₇₋₁₃ aralkyloxy group (e.g., benzyloxy) optionally substituted by 1 to 3 halogen atoms,
   (vi) a C₁₋₆ alkyl-carbonyl group,
   (vii) a C₁₋₆ alkoxy-carbonyl group,
   (viii) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g. , cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (ix) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (x) an aromatic heterocyclyl-oxy group (e.g., pyridyloxy) optionally substituted by C₁₋₆ alkyl group(s) optionally substituted by 1 to 3 halogen atoms,
   (xi) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xii) a C₁₋₆ alkoxy group; and
(4) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms.

The phenyl group of the "phenyl group having a substituent at the para-position" optionally further has 1 to 3 substituents, besides the substituent at the para-position. As the substituents other than the substituent at the para-position, those exemplified as preferable substituents of the "cyclic group" of the aforementioned "optionally substituted cyclic group" for R¹ or R² can be mentioned. The substituents other than the substituent at the para-position" is preferably 1 to 3 substituents selected from
(1) a halogen atom;
(2) a hydroxy group; and
(3) a C₁₋₆ alkoxy group.

As the "C₁₋₆ alkylene group" for L^{2a'} , those exemplified as the "aliphatic hydrocarbon group" of the "optionally substituted divalent aliphatic hydrocarbon group" for L¹ or L² can be mentioned.

L^{2a'} is preferably -CH₂-, -CH(CH₃)-, -C(CH₃)₂-, -(CH₂)₂C(CH₃)₂- or the like, more preferably -CH₂-.

As preferable specific examples of compound (2a), the following compounds can be mentioned.

### [Compound D]

Compound (2a) wherein
R^{1a'} is a C₃₋₆ cycloalkyl group optionally condensed with a benzene ring (particularly preferably cyclohexyl) and optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) a carbamoyl group;
(7) an oxo group;
(8) a C₁₋₃ alkylenedioxy group;
(9) a C₁₋₆ alkyl group optionally substituted by 1 to 3 substituents selected from the following (i) to (vii)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an aromatic heterocyclic group (e.g., oxadiazolyl) optionally substituted by C₆₋₁₄ aryl group(s) (e.g., phenyl) optionally substituted by 1 to 3 halogen atoms, and
   (vii) a non-aromatic heterocyclyl-carbonyl group (e.g., pyrrolidinylcarbonyl);
(10) a C₂₋₆ alkenyl group (e.g., ethenyl, 1-propenyl) optionally substituted by 1 to 3 substituents selected from a carboxyl group and a C₁₋₆ alkoxy-carbonyl group;
(11) a C₂₋₆ alkynyl group (e.g., ethynyl, 1-propynyl);
(12) a C₆₋₁₄ aryl group (e.g., phenyl) optionally substituted by 1 to 3 halogen atoms;
(13) a C₇₋₁₃ aralkyl group (e.g., benzyl);
(14) a C₁₋₆ alkoxy group optionally substituted by 1 to 3 halogen atoms;
(15) an amino group optionally mono- or di-substituted by C₁₋₆ alkyl group(s);
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a non-aromatic heterocyclic group (e.g., piperidinyl); and
(22) a formyl group;

R^{2a}' is a cyclic group [provided that the cyclic group is selected from a C₆₋₁₄ aryl group, (preferably phenyl, naphthyl), a C₃₋₆ cycloalkyl group (preferably cyclopropyl, cyclohexyl), a C₃₋₆ cycloalkenyl group (preferably cyclohexenyl) and a 5- or 6-membered aromatic heterocyclic group (preferably pyridyl, imidazolyl, pyrazolyl), preferably a C₆₋₁₄ aryl group (preferably phenyl)], which is optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group(s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆. alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group; and
(31) a C₁₋₆ alkylsulfonyloxy group optionally substituted by 1 to 3 halogen atoms; and
R^{3a}, R^{4a} and R^{5a} are each a hydrogen atom.

### [Compound E]

Compound (2a) wherein
R^{1a'} is a C₇₋₁₀ cross-linked hydrocarbon group (particularly preferably adamantyl) optionally substituted by 1 to 5 (preferably 1 to 3) substituents selected from;
(1) a halogen atom;
(2) a hydroxy group;
(3) a cyano group;
(4) a nitro group;
(5) a carboxyl group;
(6) a carbamoyl group;
(7) an oxo group;
(8) a C₁₋₃ alkylenedioxy group;
(9) a C₁₋₆ alkyl group optionally substituted by 1 to 3 substituents selected from the following (i) to (vii)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an aromatic heterocyclic group (e.g., oxadiazolyl) optionally substituted by C₆₋₁₄ aryl group(s) (e.g., phenyl) optionally substituted by 1 to 3 halogen atoms, and
   (vii) a non-aromatic heterocyclyl-carbonyl group (e.g., pyrrolidinylcarbonyl);
(10) a C₂₋₆ alkenyl group (e.g., ethenyl, 1-propenyl) optionally substituted by 1 to 3 substituents selected from a carboxyl group and a C₁₋₆ alkoxy-carbonyl group;
(11) a C₂₋₆ alkynyl group (e.g., ethynyl, 1-propynyl);
(12) a C₆₋₁₄ aryl group (e.g., phenyl) optionally substituted by 1 to 3 halogen atoms;
(13) a C₇₋₁₃ aralkyl group (e.g., benzyl);
(14) a C₁₋₆ alkoxy group optionally substituted by 1 to 3 halogen atoms;
(15) an amino group optionally mono- or di-substituted by C₁₋₆ alkyl group(s);
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a non-aromatic heterocyclic group (e.g., piperidinyl); and
(22) a formyl group;

R^{2a'} is a C₆₋₁₄ aryl group (preferably phenyl) optionally substituted by 1 to 5 (preferably 1 to 3) substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group (s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group (s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group; and
(31) a C₁₋₆ alkylsulfonyloxy group optionally substituted by 1 to 3 halogen atoms; and
R^{3a}, R^{4a} and R^{5a} are each a hydrogen atom.

### [Compound F]

Compound E wherein
R^{2a'} is a phenyl group having, at the para-position, a substituent selected from
(1) a nitro group;
(2) a carboxyl group;
(3) a C₁₋₃ alkylenedioxy group;
(4) a C₁₋₆ alkyl group substituted by 1 to 3 substituents selected from the following (i) to (viii)
   (i) a carboxyl group,
   (ii) a hydroxy group,
   (iii) a C₁₋₆ alkoxy-carbonyl group,
   (iv) a carbamoyl group optionally mono- or di-substituted by substituent(s) selected from a C₆₋₁₄ aryl group (e.g., phenyl) and a C₇₋₁₃ aralkyl group (e.g., benzyl),
   (v) an amino group optionally mono- or di-substituted by C₁₋₆ alkyl-carbonyl group(s),
   (vi) an aromatic heterocyclic group (e.g., oxadiazolyl) optionally substituted by C₆₋₁₄ aryl group (s) (e.g., phenyl) optionally substituted by 1 to 3 halogen atoms,
   (vii) a non-aromatic heterocyclic group (e.g., piperidino), and
   (viii) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(5) a C₂₋₆ alkenyl group (e.g., ethenyl, 1-propenyl) optionally substituted by 1 to 3 substituents selected from a carboxyl group and a C₁₋₆ alkoxy-carbonyl group;
(6) a C₂₋₆ alkynyl group (e.g., ethynyl, 1-propynyl) optionally substituted by 1 to 3 hydroxy groups;
(7) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(8) a C₇₋₁₃ aralkyl group (e.g., benzyl);
(9) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(10) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(11) a C₁₋₆ alkoxy group substituted by 1 to 3 substituents selected from the following (i) to (xii)
   (i) a cyano group,
   (ii) a carboxyl group,
   (iii) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (iv) a C₆₋₁₄ aryloxy group (e.g., phenoxy),
   (v) a C₇₋₁₃ aralkyloxy group (e.g., benzyloxy) optionally substituted by 1 to 3 halogen atoms,
   (vi) a C₁₋₆ alkyl-carbonyl group,
   (vii) a C₁₋₆ alkoxy-carbonyl group,
   (viii) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (ix) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (x) an aromatic heterocyclyl-oxy group (e.g., pyridyloxy) optionally substituted by C₁₋₆ alkyl group(s) optionally substituted by 1 to 3 halogen atoms,
   (xi) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xii) a C₁₋₆ alkoxy group;
(12) a C₂₋₆ alkynyloxy group (e.g., ethynyloxy, 2-propynyloxy);
(13) a C₃₋₁₀ cycloalkyl-C₁₋₆ alkyloxy group (e.g., cyclopropylmethyloxy);
(14) a C₁₋₆ alkylthio group (e.g., methylthio, ethylthio);
(15) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(16) a C₁₋₆ alkyl-carbonyl group;
(17) a C₁₋₆ alkoxy-carbonyl group;
(18) a C₇₋₁₃ aralkyloxy-carbonyl group (e.g., benzyloxycarbonyl);
(19) a C₁₋₆ alkylsulfinyl group (e.g., methylsulfinyl);
(20) a C₁₋₆ alkylsulfonyl group;
(21) a carbamoyl group optionally mono- or di-substituted by substituent(s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(22) a C₆₋₁₄ aryloxy group (e.g., phenoxy);
(23) a C₇₋₁₃ aralkyloxy group (e.g., benzyloxy) substituted by 1 to 3 substituents selected from a C₁₋₆ alkyl group substituted by 1 to 3 hydroxy groups, a carboxyl group and a C₁₋₆ alkoxy-carbonyl group;
(24) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(25) a tri-C₁₋₆ alkyl-silyloxy group (e.g., trimethylsilyloxy, triethylsilyloxy, tert-butyldimethylsilyloxy);
(26) a formyl group; and
(27) a C₁₋₆ alkylsulfonyloxy group optionally substituted by 1 to 3 halogen atoms; and
optionally further having, besides the substituent at the para-position, 1 to 3 substituents selected from
(1) a halogen atom;
(2) a hydroxy group;
(3) a cyano group;
(4) a nitro group;
(5) a carboxyl group;
(6) an oxo group;
(7) a C₁₋₃ alkylenedioxy group;
(8) a C₁₋₆ alkyl group optionally substituted by 1 to 3 substituents selected from the following (i) to (ix)
   (i) a halogen atom,
   (ii) a carboxyl group,
   (iii) a hydroxy group,
   (iv) a C₁₋₆ alkoxy-carbonyl group,
   (v) a carbamoyl group optionally mono- or di-substituted by substituent(s) selected from a C₆₋₁₄ aryl group (e.g., phenyl) and a C₇₋₁₃ aralkyl group (e.g., benzyl),
   (vi) an amino group optionally mono- or di-substituted by C₁₋₆ alkyl-carbonyl group(s),
   (vii) an aromatic heterocyclic group (e.g., oxadiazolyl) optionally substituted by C₆₋₁₄ aryl group (s) (e.g., phenyl) optionally substituted by 1 to 3 halogen atoms,
   (viii) a non-aromatic heterocyclic group (e.g., piperidino), and
   (ix) a non-aromatic heterocyclyl-carbonyl group (e.g., pyrrolidinylcarbonyl, piperazinylcarbonyl) optionally substituted by 1 to 3 C₁₋₆ alkoxy-carbonyl groups;
(9) a C₂₋₆ alkenyl group (e.g., ethenyl, 1-propenyl) optionally substituted by 1 to 3 substituents selected from a carboxyl group and a C₁₋₆ alkoxy-carbonyl group;
(10) a C₂₋₆ alkynyl group (e.g., ethynyl, 1-propynyl) optionally substituted by 1 to 3 hydroxy groups;
(11) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(12) a C₇₋₁₃ aralkyl group (e.g., benzyl);
(13) an aromatic heterocyclic group (e.g., furyl, thienyl, oxadiazolyl, pyridyl, pyrimidinyl) optionally substituted by 1 to 3 substituents selected from the following (i) to (v)
   (i) a C₁₋₆ alkyl group optionally substituted by 1 to 3 hydroxy groups,
   (ii) a carboxyl group,
   (iii) a C₃₋₁₀ cycloalkyl group (e.g., cyclopropyl),
   (iv) a halogen atom, and
   (v) a formyl group;
(14) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(15) a C₁₋₆ alkoxy group optionally substituted by 1 to 3 substituents selected from the following (i) to (xiv)
   (i) a halogen atom,
   (ii) a hydroxy group,
   (iii) a cyano group,
   (iv) a carboxyl group,
   (v) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (vi) a C₆₋₁₄ aryloxy group (e.g., phenoxy),
   (vii) a C₇₋₁₃ aralkyloxy group (e.g., benzyloxy) optionally substituted by 1 to 3 halogen atoms,
   (viii) a C₁₋₆ alkyl-carbonyl group,
   (ix) a C₁₋₆ alkoxy-carbonyl group,
   (x) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (xi) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (xii) an aromatic heterocyclyl-oxy group (e.g., pyridyloxy) optionally substituted by C₁₋₆ alkyl group(s) optionally substituted by 1 to 3 halogen atoms,
   (xiii) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xiv) a C₁₋₆ alkoxy group;
(16) a C₂₋₆ alkynyloxy group (e.g., ethynyloxy, 2-propynyloxy);
(17) a C₃₋₁₀ cycloalkyl-C₁₋₆ alkyloxy group (e.g., cyclopropylmethyloxy);
(18) a C₁₋₆ alkylthio group (e.g., methylthio, ethylthio);
(19) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms;
(20) a C₁₋₆ alkyl-carbonyl group;
(21) a C₁₋₆ alkoxy-carbonyl group;
(22) a C₇₋₁₃ aralkyloxy-carbonyl group (e.g., benzyloxycarbonyl);
(23) a C₁₋₆ alkylsulfinyl group (e.g., methylsulfinyl);
(24) a C₁₋₆ alkylsulfonyl group;
(25) a carbamoyl group optionally mono- or di-substituted by substituent (s) selected from a C₁₋₆ alkyl group, a C₆₋₁₄ aryl group (e.g., phenyl), a C₇₋₁₃ aralkyl group (e.g., benzyl) and an aromatic heterocyclyl-C₁₋₆ alkyl group (e.g., furfuryl);
(26) a C₆₋₁₄ aryloxy group (e.g., phenoxy);
(27) a C₇₋₁₃ aralkyloxy group (e.g., benzyloxy) optionally substituted by 1 to 3 substituents selected from a C₁₋₆ alkyl group optionally substituted by 1 to 3 hydroxy groups, a carboxyl group and a C₁₋₆ alkoxy-carbonyl group;
(28) an aromatic heterocyclyl-oxy group (e.g., pyridyloxy) optionally substituted by 1 to 3 substituents selected from a C₁₋₆ alkyl group optionally substituted by 1 to 3 halogen atoms and a halogen atom;
(29) a tri-C₁₋₆ alkyl-silyloxy group (e.g., trimethylsilyloxy, triethylsilyloxy, tert-butyldimethylsilyloxy);
(30) a formyl group; and
(31) a C₁₋₆ alkylsulfonyloxy group optionally substituted by 1 to 3 halogen atoms.

### [Compound G]

Compound F wherein
R^{2a'} is a phenyl group having, at the para-position, a substituent selected from
(1) a C₆₋₁₄ aryl group (e.g., phenyl, naphthyl) optionally substituted by 1 to 3 substituents selected from the following (i) to (ix)
   (i) a C₁₋₆ alkyl group optionally substituted by 1 to 3 substituents selected from a hydroxy group and a halogen atom,
   (ii) a formyl group,
   (iii) a cyano group,
   (iv) a carboxyl group,
   (v) a carbamoyl group,
   (vi) a halogen atom,
   (vii) a C₁₋₆ alkyl-carbonyl group,
   (viii) a C₁₋₆ alkylsulfonyl group, and
   (ix) an amino group optionally mono- or di-substituted by substituent(s) selected from a C₁₋₆ alkyl-carbonyl group and a C₁₋₆ alkylsulfonyl group;
(2) a non-aromatic heterocyclic group (e.g., piperazinyl, oxazolidinyl, pyrrolidinyl, imidazolidinyl) optionally substituted by 1 to 3 substituents selected from the following (i) to (iii)
   (i) a C₁₋₆ alkyl group optionally substituted by 1 to 3 substituents selected from
      (a) a hydroxy group,
      (b) a C₆₋₁₄ aryl group optionally substituted by 1 to 3 substituents selected from a C₁₋₆ alkyl group optionally substituted by 1 to 3 halogen atoms and a halogen atom, and
      (c) an aromatic heterocyclic group (e.g., pyridyl),
   (ii) an oxo group, and
   (iii) a C₁₋₆ alkyl-carbonyl group;
(3) a C₁₋₆ alkoxy group substituted by 1 to 3 substituents selected from the following (i) to (xii)
   (i) a cyano group,
   (ii) a carboxyl group,
   (iii) an amino group optionally mono- or di-substituted by substituent(s) selected from
      (a) a C₁₋₆ alkyl group,
      (b) a C₁₋₆ alkoxy-carbonyl group,
      (c) a C₆₋₁₄ aryl-carbonyl group optionally substituted by C₁₋₆ alkyl group(s) optionally substituted by 1 to 3 halogen atoms, and
      (d) a C₆₋₁₄ arylsulfonyl group,
   (iv) a C₆₋₁₄ aryloxy group (e.g., phenoxy),
   (v) a C₇₋₁₃ aralkyloxy group (e.g., benzyloxy) optionally substituted by 1 to 3 halogen atoms,
   (vi) a C₁₋₆ alkyl-carbonyl group,
   (vii) a C₁₋₆ alkoxy-carbonyl group,
   (viii) a carbamoyl group optionally mono- or di-substituted by substituent(s) selected from
      (a) a C₁₋₆ alkyl group optionally substituted by 1 to 3 substituents selected from
         (a-1) a C₆₋₁₄ aryl group (e.g., phenyl) optionally substituted by 1 to 3 substituents selected from a C₁₋₆ alkyl group optionally substituted by 1 to 3 halogen atoms, a halogen atom and a C₁₋₆ alkoxy group,
         (a-2) an aromatic heterocyclic group (e.g., pyridyl, furyl), and
         (a-3) a C₃₋₁₀ cycloalkyl group (e.g., cyclopropyl),
      (b) a C₃₋₁₀ cycloalkyl group (e.g., cyclopropyl),
      (c) a C₆₋₁₄ aryl group (e.g., phenyl) optionally substituted by 1 to 3 substituents selected from
         (c-1) a halogen atom, and
         (c-2) a C₁₋₆ alkyl group optionally substituted by 1 to 3 halogen atoms, and
      (d) a C₆₋₁₄ arylsulfonyl group (e.g., phenylsulfonyl) optionally substituted by C₁₋₆ alkyl group(s) optionally substituted by 1 to 3 halogen atoms,
   (ix) an aromatic heterocyclic group (e.g., oxazolyl, thiazolyl, imidazolyl, benzothiazolyl, oxadiazolyl) optionally substituted by 1 to 3 substituents selected from
      (a) a C₁₋₆ alkyl group optionally substituted by 1 to 3 substituents selected from a hydroxy group and a halogen atom,
      (b) a C₆₋₁₄ aryl group (e.g., phenyl) optionally substituted by 1 to 3 halogen atoms,
      (c) a carboxyl group, and
      (d) a C₁₋₆ alkoxy-carbonyl group,
   (x) an aromatic heterocyclyl-oxy group (e.g., pyridyloxy) optionally substituted by C₁₋₆ alkyl group(s) optionally substituted by 1 to 3 halogen atoms,
   (xi) a non-aromatic heterocyclic group (e.g., pyrrolidinyl, morpholinyl, dihydroisoindolyl) optionally substituted by 1 to 3 substituents selected from
      (a) a C₃₋₁₀ cycloalkyl group (e.g., cyclopropyl), and
      (b) an oxo group, and
   (xii) a C₁₋₆ alkoxy group; and
(4) an amino group optionally mono- or di-substituted by substituent(s) selected from the following (i) to (xvii)
   (i) a C₁₋₆ alkyl group optionally substituted by 1 to 3 substituents selected from a hydroxy group and a C₁₋₆ alkoxy-carbonyl group,
   (ii) a C₆₋₁₄ aryl group (e.g., phenyl),
   (iii) a C₇₋₁₃ aralkyl group (e.g., benzyl),
   (iv) a C₁₋₆ alkyl-carbonyl group optionally substituted by 1 to 3 substituents selected from
      (a) a hydroxy group,
      (b) a carboxyl group,
      (c) a C₆₋₁₄ aryl group (e.g., phenyl),
      (d) a C₁₋₆ alkoxy-carbonyl group,
      (e) a C₁₋₆ alkyl-carbonyloxy group (e.g., acetyloxy, tert-butylcarbonyloxy),
      (f) a C₆₋₁₄ arylsulfonyl group (e.g., phenylsulfonyl),
      (g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (h) a halogen atom, and
      (i) an aromatic heterocyclic group (e.g., pyridyl),
   (v) a C₁₋₆ alkoxy-carbonyl group,
   (vi) a C₆₋₁₄ aryl-carbonyl group (e.g., benzoyl) optionally substituted by 1 to 3 substituents selected from
      (a) a C₁₋₆ alkyl group optionally substituted by 1 to 3 halogen atoms, and
      (b) a sulfamoyl group,
   (vii) a C₇₋₁₃ aralkyl-carbonyl group (e.g., benzylcarbonyl, phenethylcarbonyl),
   (viii) a C₃₋₁₀ cycloalkyl-carbonyl group (e.g., cyclopropylcarbonyl, cyclohexylcarbonyl) optionally substituted by 1 to 3 substituents selected from
      (a) a hydroxy group,
      (b) a carboxyl group,
      (c) a C₁₋₆ alkoxy-carbonyl group,
      (d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
      (e) an oxo group, and
      (f) a C₁₋₆ alkyl group optionally substituted by 1 to 3 hydroxy groups,
   (ix) an aromatic heterocyclyl-carbonyl group (e.g., furoyl) optionally substituted by C₁₋₆ alkyl group(s) optionally substituted by 1 to 3 hydroxy groups,
   (x) a C₁₋₆ alkylsulfonyl group optionally substituted by 1 to 3 substituents selected from a non-aromatic heterocyclic group (e.g., 1,3-dihydro-2H-isoindol-2-yl group) optionally substituted by oxo group(s) and a halogen atom,
   (xi) a C₆₋₁₄ arylsulfonyl group (e.g., benzenesulfonyl, toluenesulfonyl, 1-naphthalenesulfonyl, 2-naphthalenesulfonyl) optionally substituted by 1 to 3 substituents selected from a C₁₋₆ alkyl group optionally substituted by 1 to 3 halogen atoms and a halogen atom,
   (xii) a C₇₋₁₃ aralkylsulfonyl group (e.g., benzylsulfonyl),
   (xiii) a C₃₋₁₀ cycloalkylsulfonyl group (e.g., cyclopropylsulfonyl),
   (xiv) an aromatic heterocyclyl-sulfonyl group (e.g., isoxazolylsulfonyl) optionally substituted by 1 to 3 C₁₋₆ alkyl groups,
   (xv) -CONR⁶R⁷
      wherein
      R⁶ and R⁷ are the same or different and each is selected from the following (a) to (f),
      (a) a hydrogen atom,
      (b) a C₁₋₆ alkyl group optionally substituted by 1 to 3 substituents selected from the following (b-1) to (b-4)
         (b-1) a hydroxy group,
         (b-2) a carboxyl group,
         (b-3) a C₁₋₆ alkoxy-carbonyl group, and
         (b-4) a carbamoyl group optionally mono- or di-substituted by C₁₋₆ alkyl group(s) (e.g., methylcarbamoyl),
      (c) a C₆₋₁₄ aryl group (e.g., phenyl, 1-naphthyl, 2-nathpthyl) optionally substituted by 1 to 3 substituents selected from the following (c-1) to (c-5)
         (c-1) a C₁₋₆ alkyl group optionally substituted by 1 to 3 substituents selected from a hydroxy group and a halogen atom,
         (c-2) a carboxyl group,
         (c-3) a C₁₋₆ alkoxy-carbonyl group,
         (c-4) a carbamoyl group, and
         (c-5) a C₁₋₆ alkoxy group optionally substituted by 1 to 3 halogen atoms,
      (d) a C₇₋₁₃ aralkyl group (e.g., benzyl),
      (e) a C₁₋₆ alkoxy group, and
      (f) a C₇₋₁₃ aralkyloxy group (e.g., benzyloxy), or R⁶ and R⁷ form, together with the adjacent nitrogen atom, an optionally substituted nitrogen-containing heterocycle (e.g., pyrrolidine),
   (xvi) a sulfamoyl group optionally mono- or di-substituted by C₁₋₆ alkyl group (s) optionally substituted by 1 to 3 C₃₋₁₀ cycloalkyl groups, and
   (xvii) a non-aromatic heterocyclyl-carbonyl group (e.g., piperazinylcarbonyl) optionally substituted by 1 to 3 substituents selected from
      (a) a carboxyl group,
      (b) a C₁₋₆ alkoxy-carbonyl group, and
      (c) a C₇₋₁₃ aralkyl group optionally substituted by C₁₋₆ alkyl group(s) optionally substituted by 1 to 3 halogen atoms; and
optionally further having, besides the substituent at the para-position, 1 to 3 substituents selected from
(1) a halogen atom;
(2) a hydroxy group; and
(3) a C₁₋₆ alkoxy group.

### [Compound H]

3-(2,4-dichlorobenzyl)-1-(4-hydroxy-4-methylcyclohexyl)pyrrolidin-2-one (Example 412A, Example 413A);
N-(3-chloro-4-{[1-(5-hydroxy-2-adamantyl)-2-oxopyrrolidin-3-yl]methyl}phenyl)-N'-[4-(hydroxymethyl)phenyl]urea (Example 468A);
N-(3'-chloro-4'-{[1-(5-hydroxy-2-adamantyl)-2-oxopyrrolidin-3-yl]methyl}biphenyl-3-yl)methanesulfonamide (Example 470A);
1-(3-chloro-4-{[1-(5-hydroxy-2-adamantyl)-2-oxopyrrolidin-3-yl]methyl}phenyl)-3-[4-(trifluoromethyl)benzyl]imidazolidin-2-one (Example 510A);
2-(3-chloro-4-{[1-(4-hydroxy-4-methylcyclohexyl)-2-oxopyrrolidin-3-yl]methyl}phenoxy)-N-[4-(trifluoromethyl)benzyl]acetamide (Example 602A, Example 603A);
3-(2-chloro-4-{[3-(4-fluorophenyl)-1,2,4-oxadiazol-5-yl]methoxy}benzyl)-1-(4-hydroxy-4-methylcyclohexyl)pyrrolidin-2-one (Example 619A);
3-{[3-chloro-3'-(hydroxymethyl)biphenyl-4-yl]methyl}-1-(4-hydroxy-4-methylcyclohexyl)pyrrolidin-2-one (Example 654A);
or a salt thereof.

As salts of compound (1), compound (2) and compound (2a) (hereinafter to be collectively abbreviated as the compound of the present invention), a pharmacologically acceptable salt is preferable. As such salts, for example, a salt with inorganic base, a salt with organic base, a salt with inorganic acid, a salt with organic acid, a salt with basic or acidic amino acid and the like can be mentioned.

Preferable examples of salts with inorganic base include alkali metal salts such as sodium salt, potassium salt and the like; alkaline earth metal salts such as calcium salt, magnesium salt and the like; and aluminum salts; ammonium salts and the like.

As preferable examples of the salts with organic bases, salts with trimethylamine, triethylamine, pyridine, picoline, ethanolamine, diethanolamine, triethanolamine, dicyclohexylamine, N,N-dibenzylethylenediamine and the like can be mentioned.

As preferable examples of the salts with inorganic acids, salts with hydrochloric acid, hydrobromic acid, nitric acid, sulfuric acid, phosphoric acid and the like can be mentioned.

As preferable examples of the salts with organic acids, salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like can be mentioned.

As preferable examples of the salts with basic amino acid, salts with arginine, lysin, ornithine and the like.

As preferable examples of the salts with acidic amino acids, salts with aspartic acid, glutamic acid and the like can be mentioned.

A prodrug of the compound of the present invention means a compound which is converted to the present invention with a reaction due to an enzyme, an gastric acid, etc. under the physiological condition in the living body, that is, a compound which is converted to the compound of the present invention with oxidation, reduction, hydrolysis, etc. according to an enzyme; a compound which is converted to the compound of the present invention by hydrolysis etc. due to gastric acid, etc. A prodrug of the compound of the present invention may be a compound obtained by subjecting an amino group in the compound of the present invention to an acylation, alkylation or phosphorylation (e.g., a compound obtained by subjecting an amino group in the compound of the present invention to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation, etc.); a compound obtained by subjecting a hydroxy group in the compound of the present invention to an acylation, alkylation, phosphorylation or boration (e.g., a compound obtained by subjecting an hydroxy group in the compound of the present invention to an acetylation, palmitoylation, propanoylation, pivaloylation, succinylation, fumarylation, alanylation or dimethylaminomethylcarbonylation, etc.); a compound obtained by subjecting a carboxyl group in the compound of the present invention to an esterification or amidation (e.g., a compound obtained by subjecting a carboxyl group in the compound of the present invention to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, etc.) and the like. Any of these compounds can be produced from the compound of the present invention by a method known *per se*.

A prodrug of the compound of the present invention may also be one which is converted into the present invention under a physiological condition, such as those described in IYAKUHIN no KAIHATSU (Development of Pharmaceuticals), Vol. 7, Design of Molecules, p.163-198, Published by HIROKAWA SHOTEN (1990).

The compound of the present invention may be labeled with an isotope (e.g., ³H, ¹⁴C, ³⁵S, ¹²⁵I etc.) and the like.

Furthermore, the compound of the present invention may be a non-hydrate or hydrate.

The compound of the present invention or a prodrug thereof (hereinafter sometimes to be abbreviated as the compound of the present invention) shows low toxicity and can be used as an agent for the prophylaxis or treatment of various diseases to be mentioned later for mammals (e.g., humans, mice, rats, rabbits, dogs, cats, bovines, horses, bovines, pigs, monkeys etc.) as they are or by admixing with a pharmacologically acceptable carrier and the like to give a pharmaceutical composition.

Here, various organic or inorganic carriers conventionally used as materials for pharmaceutical preparations are used as a pharmacologically acceptable carrier, which are added as excipient, lubricant, binder and disintegrant for solid preparations; or solvent, solubilizing agent, suspending agent, isotonicity agent, buffer and soothing agent for liquid preparations, and the like. Where necessary, an additive for pharmaceutical preparations such as preservative, antioxidant, coloring agent, sweetening agent and the like can be used.

Preferable examples of the excipient include lactose, sucrose, D-mannitol, D-sorbitol, starch, α-starch, dextrin, crystalline cellulose, low-substituted hydroxypropylcellulose, sodium carboxymethylcellulose, gum acacia, pullulan, light anhydrous silicic acid, synthetic aluminum silicate, magnesium aluminate metasilicate and the like.

Preferred examples of the lubricant include magnesium stearate, calcium stearate, talc, colloidal silica and the like.

Preferable examples of the binder include α-starch, saccharose, gelatin, gum acacia, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.

Preferable examples of the disintegrant include lactose, sucrose, starch, carboxymethylcellulose, calcium carboxymethylcellulose, sodium croscarmellose, sodium carboxymethylstarch, light anhydrous silicic acid, low-substituted hydroxypropylcellulose and the like.

Preferable examples of the solvent include water for injection, physiological brine, Ringer's solution, alcohol, propylene glycol, polyethylene glycol, sesame oil, corn oil, olive oil, cottonseed oil and the like.

Preferred examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferred examples of the suspending agent include surfactants such as stearyltriethanolamine, sodium lauryl sulfate, lauryl aminopropionate, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil and the like.

Preferred examples of the isotonicity agent include sodium chloride, glycerol, D-mannitol, D-sorbitol, glucose and the like.

Preferred examples of the buffer include buffers such as phosphate, acetate, carbonate, citrate and the like.

Preferred examples of the soothing agent include benzyl alcohol and the like.

Preferred examples of the preservative include p-oxybenzoates, chlorobutanol, benzyl alcohol, phenethyl alcohol, dehydroacetic acid, sorbic acid and the like.

Preferred examples of the antioxidant include sulfite, ascorbate and the like.

Preferable examples of the coloring agent include water-soluble edible tar pigments (e.g., foodcolors such as Food Color Red Nos. 2 and 3, Food Color Yellow Nos. 4 and 5, Food Color Blue Nos. 1 and 2 and the like), water insoluble lake pigments (e.g., aluminum salt of the aforementioned water-soluble edible tar pigment), natural pigments (e.g., beta carotene, chlorophil, red iron oxide) and the like.

Preferable examples of the sweetening agent include saccharin sodium, dipotassium glycyrrhizinate, aspartame, stevia and the like.

The dosage form of the aforementioned pharmaceutical composition is, for example, an oral agent such as tablets (inclusive of sublingual tablets and orally disintegrable tablets), capsules (inclusive of soft capsules and microcapsules), granules, powders, troches, syrups, emulsions, suspensions and the like; or a parenteral agent such as injections (e.g., subcutaneous injections, intravenous injections, intramuscular injections, intraperitoneal injections, drip infusions), external agents (e.g., transdermal preparations, ointments), suppositories (e.g., rectal suppositories, vaginal suppositories), pellets, nasal preparations, pulmonary preparations (inhalations), ophthalmic preparations and the like. These may be administered safely via an oral or parenteral route.

These agents may be controlled-release preparations such as rapid-release preparations and sustained-release preparations (e.g., sustained-release microcapsules).

The pharmaceutical composition can be produced according to a method conventionally used in the field of pharmaceutical preparation, such as the method described in Japan Pharmacopoeia and the like.

While the content of the compound of the present invention in the pharmaceutical composition varies depending on the dosage form, dose of the compound of the present invention and the like, it is, for example, about 0.1 to 100 wt%.

The compound of the present invention has a superior 11β-HSD1 inhibitory action, and can be used as an agent for the prophylaxis or treatment of various diseases for mammals (e.g., human, bovine, horse, dog, cat, monkey, mouse, rat, specifically human). In addition, as the compound of the present invention has a selective 11β-HSD1 inhibitory action, it shows low toxicity (e.g., acute toxicity, chronic toxicity, cardiotoxicity, carcinogenic, genetic toxicity), which causes fewer side effects.

The compound of the present invention can be used as an agent for the prophylaxis or treatment of diabetes (e.g., type-1 diabetes, type-2 diabetes, gestational diabetes etc.); an agent for the prophylaxis or treatment of hyperlipidemia (e.g., hypertriglyceridemia, hypercholesterolemia, hypo-HDL-emia, postprandial hyperlipidemia etc.); an agent for the prophylaxis or treatment of arteriosclerosis; an agent for the prophylaxis or treatment of impaired glucose tolerance (IGT); and an agent for preventing progression of impaired glucose tolerance into diabetes.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) reported new diagnostic criteria of diabetes in 1997 and WHO in 1998.

According to these reports, diabetes is a condition showing a fasting blood glucose level (glucose concentration of intravenous plasma) of not less than 126 mg/dl and a 75 g oral glucose tolerance test 2 h level (glucose concentration of intravenous plasma) of not less than 200 mg/dl.

According to the above-mentioned reports, impaired glucose tolerance is a condition showing a fasting blood glucose level (glucose concentration of intravenous plasma) of less than 126 mg/dl and a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75 g oral glucose tolerance test 2 h level (glucose concentration of intravenous plasma) of less than 140 mg/dl is called IFG (Impaired Fasting Glycemia).

The compound of the present invention can also be used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

The compound of the present invention can also be used as an agent for the prophylaxis or treatment of, for example, diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infections, inferior limb infection etc.), diabetic gangrene, xerostomia, hypacusis, cerebrovascular disorder, peripheral blood circulation disorder etc.], obesity, osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, hypertension, polycystic ovary syndrome, kidney disease (e.g., diabetic nephropathy, glomerular nephritis, glomerulosclerosis, nephrotic syndrome, hypertensive nephrosclerosis, end stage kidney disease etc.), muscular dystrophy, myocardial infarction, angina pectoris, cerebrovascular accident (e.g., cerebral infarction, cerebral apoplexy), abnormal sugar metabolism, abnormal lipid metabolism, insulin resistance syndrome, Syndrome X, metabolic syndrome (state concurrently associated with at least one of type 2 diabetes, impaired glucose tolerance and insulin resistance, and at least two from obesity, abnormal lipid metabolism, hypertension and trace albumin urine), Cushing's syndrome, hyperinsulinemia, hyperinsulinemia-induced sensory disorder, tumor (e.g., leukemia, breast cancer, prostate cancer, skin cancer), irritable bowel syndrome, acute or chronic diarrhea, inflammatory diseases (e.g., chronic rheumatoid arthritis, spondylitis deformans, osteoarthritis, lumbago, gout, postoperative or traumatic inflammation, swelling, neuralgia, pharyngolaryngitis, cystitis, hepatitis (inclusive of non-alcoholic steatohepatitis), pneumonia, pancreatitis, inflammatory bowel disease, ulcerative colitis, stomach mucous membrane injury (including stomach mucous membrane injury caused by aspirin)), visceral obesity syndrome and the like.

The compound of the present invention can also be used for improvement of insulin resistance, decrease of visceral fat, suppression of accumulation of visceral fat, improvement of sugar metabolism, improvement of lipid metabolism, suppression of oxidative LDL production, improvement of lipoprotein metabolism, improvement of coronary metabolism, prophylaxis or treatment of cardiovascular complication, prophylaxis or treatment of heart failure complication, lowering of blood remnant, prophylaxis or treatment of anovulation, prophylaxis or treatment of hirsutism, prophylaxis or treatment of hyperandrogenism, improvement of pancreatic (β cell) function, regeneration of pancreas (β cell), promotion of regeneration of pancreas (β cell) and the like.

The compound of the present invention can also be used for the secondary prevention and suppression of progression of various diseases mentioned above (e.g., cardiovascular event such as myocardial infarction etc.).

While the dose of the compound of the present invention varies depending on the administration subject, administration route, target disease, condition and the like, the compound of the present invention is generally given in a single dose of about 0.01-100 mg/kg body weight, preferably 0.05-30 mg/kg body weight, more preferably 0.1-10 mg/kg body weight, in the case of, for example, oral administration to adult diabetic patients. This dose is desirably given 1 to 3 times a day.

The compound of the present invention can be used in combination with drugs such as a therapeutic agent for diabetes, a therapeutic agent for diabetic complications, a therapeutic agent for hyperlipidemia, an antihypertensive agent, an antiobestic agent, a diuretic, a chemotherapeutic agent, an immunotherapeutic agent, an antithrombotic agent, a therapeutic agent for osteoporosis, a antidementia agent, an erectile dysfunction improver, a therapeutic agent for pollakiuria or urinary incontinence, a therapeutic agent for dysuria and the like (hereinafter to be referred to as a combination drug). In this case, the timing of administration of the compound of the present invention and a combination drug is not limited. These may be simultaneously administered to an administration subject or administered in a staggered manner. Moreover, the compound of the present invention and a combination drug may be administered as two kinds of preparations each containing an active ingredient, or may be administered as a single preparation containing both active ingredients.

The dose of the combination drug can be determined as appropriate based on the dose clinically employed. The proportion of the compound of the present invention and the combination drug can be appropriately determined depending on the administration subject, administration route, target disease, condition, combination and the like. When, for example, the administration subject is human, the combination drug is used in an amount of 0.01-100 parts by weight per 1 part by weight of the compound of the present invention.

Examples of the therapeutic agents for diabetes include insulin preparations (e.g., animal insulin preparations extracted from pancreas of bovine and swine; human insulin preparations genetically synthesized using *Escherichia coli,* yeast; zinc insulin; protamine zinc insulin; fragment or derivative of insulin (e.g., INS-1 etc.), oral insulin preparation and the like), insulin sensitizers (e.g., pioglitazone or a salt thereof (preferably hydrochloride), rosiglitazone or a salt thereof (preferably maleate), Reglixane (JTT-501), Netoglitazone (MCC-555), DRF-2593, KRP-297, R-119702, Rivoglitazone (CS-011), FK-614, compounds described in WO99/58510 (e.g., (E)-4-[4-(5-methyl-2-phenyl-4-oxazolylmethoxy)benzyloxyimino]-4-phenylbutyric acid), compounds described in WO01/38325, Tesaglitazar (AZ-242), Ragaglitazar (NN-622), Muraglitazar (BMS-298585), ONO-5816, Edaglitazone (BM-13-1258), Metaglidasen (MBX-102), Naveglitazar (LY-519818), MX-6054, LY-510929, Balaglitazone (NN-2344), T-131 or a salt thereof, THR-0921), PPARγ agonists, PPARγ antagonists, PPARγ/α dual agonists, α-glucosidase inhibitors (e.g., voglibose, acarbose, miglitol, emiglitate etc.), biguanides (e.g., phenformin, metformin, buformin or a salt thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride, glipizide, glybuzole), repaglinide, senaglinide, nateglinide, mitiglinide or calcium salt hydrate thereof], GPR40 agonists, GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), dipeptidyl-peptidase IV inhibitors (e.g., NVP-DPP-278, PT-100, P32/98, Vildagliptin (LAF-237), P93/01, TS-021, Sitagliptin phosphate (MK-431), Saxagliptin (BMS-477118), E-3024, T-6666 (TA-6666), 823093, 825964, 815541), β3 agonists (e.g., AJ-9677), gluconeogenesis inhibitors (e.g., glycogen phosphorylase inhibitors, glucose-6-phosphatase inhibitors, glucagon antagonists), SGLT (sodium-glucose cotransporter) inhibitors (e.g., T-1095), 11β-HSD1 inhibitors (e.g., BVT-3498), adiponectin or agonists thereof, IKK inhibitors (e.g., AS-2868), leptin resistance improving drugs, somatostatin receptor agonists (compounds described in WO01/25228, WO03/42204, WO98/44921, WO98/45285 and WO99/22735), glucokinase activators (e.g., Ro-28-1675) and the like.

Examples of the therapeutic agents for diabetic complications include aldose reductase inhibitors (e.g., Tolrestat, Epalrestat, Zenarestat, Zopolrestat, Minalrestat, Fidarestat (SNK-860), ranirestst (AS-3201) and CT-112), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), stimulators (e.g., Y-128), PKC inhibitors (e.g., ruboxistaurin mesylate; LY-333531), AGE inhibitors (e.g., ALT-946, pimagedine, N-phenacylthiazolium bromide (ALT-766), ALT-711, EXO-226, Pyridorin, Pyridoxamine), active oxygen scavengers (e.g., thioctic acid), cerebral vasodilators (e.g., tiapuride, mexiletine, somatostatin receptor agonists (BIM23190), apoptosis signal regulating kinase-1 (ASK-1) inhibitors and the like.

Examples of the therapeutic agents for hyperlipidemia include HMG-CoA reductase inhibitors (e.g., pravastatin, simvastatin, lovastatin, atorvastatin, fluvastatin, pitavastatin, rosuvastatin and salts thereof (e.g., sodium salt, calcium salt)), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, phytosterols (e.g., soysterol, γ-oryzanol) and the like.

Examples of the antihypertensive agents include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121), clonidine and the like.

Examples of the antiobesity agents include antiobesity agents acting on the central nervous system (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonists; pancreatic lipase inhibitors (e.g., orlistat, ATL-962), β3 agonists (e.g., AJ-9677), peptide anorexiants (e.g., leptin, CNTF (Ciliary Neurotropic Factor)), cholecystokinin agonists (e.g., lintitript, FPL-15849), feeding deterrents (e.g., P-57) and the like.

Examples of the diuretics include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the chemotherapeutic agents include alkylating agents (e.g., cyclophosphamide, ifosfamide), metabolic antagonists (e.g., methotrexate, 5-fluorouracil and derivatives thereof), antitumor antibiotics (e.g., mitomycin, adriamycin), plant-derived antitumor agents (e.g., vincristine, vindesine, Taxol), cisplatin, carboplatin, etoposide and the like. Of these, Furtulon or NeoFurtulon, which are 5-fluorouracil derivatives, and the like are preferable.

Examples of the immunotherapeutic agents include microorganism or bacterial components (e.g., muramyl dipeptide derivatives, Picibanil), polysaccharides having immunity potentiating activity (e.g., lentinan, schizophyllan, krestin), cytokines obtained by genetic engineering techniques (e.g., interferon, interleukin (IL)), colony stimulating factors (e.g., granulocyte colony stimulating factor, erythropoietin) and the like, with preference given to interleukins such as IL-1, IL-2, IL-12 and the like.

Examples of the antithrombotic agents include heparin (e.g., heparin sodium, heparin calcium, dalteparin sodium), warfarins (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

Examples of the therapeutic agents for osteoporosis include alfacalcidol, calcitriol, elcatonin, calcitonin salmon, estriol, ipriflavone, pamidronate disodium, alendronate sodium hydrate, incadronate disodium, risedronate disodium and the like.

Examples of the antidementia agents include tacrine, donepezil, rivastigmine, galanthamine and the like.

Examples of the erectile dysfunction improvers include apomorphine, sildenafil citrate and the like.

Examples of the therapeutic agents for pollakiuria or urinary incontinence include flavoxate hydrochloride, oxybutynin hydrochloride, propiverine hydrochloride and the like.

Examples of the therapeutic agents for dysuria include acetylcholine esterase inhibitors (e.g., distigmine) and the like.

Furthermore, drugs having a cachexia-improving action established in animal models and clinical situations, such as cyclooxygenase inhibitors (e.g., indomethacin), progesterone derivatives (e.g., megestrol acetate), glucosteroids (e.g., dexamethasone), metoclopramide agents, tetrahydrocannabinol agents, fat metabolism improving agents (e.g., eicosapentanoic acid), growth hormones, IGF-1, or antibodies to a cachexia-inducing factor such as TNF-α, LIF, IL-6, oncostatin M and the like, can be used in combination with the compound of the present invention.

The combination drug is preferably insulin preparation, insulin sensitizer, α-glucosidase inhibitor, biguanide, insulin secretagogue (preferably sulfonylurea) and the like.

Two or more kinds of the above-mentioned combination drugs may be used in an appropriate combination.

When the compound of the present invention is used in combination with a combination drug, the amount thereof can be reduced within a safe range in consideration of counteraction of these agents. Particularly, the dose of an insulin sensitizer, an insulin secretagogue (preferably a sulfonylurea) and a biguanide can be reduced as compared with the normal dose. Therefore, an adverse effect which may be caused by these agents can be prevented safely. In addition, the dose of the therapeutic agent for diabetic complications, therapeutic agent for hyperlipemia and antihypertensive agent can be reduced whereby an adverse effect which may be caused by these agents can be prevented effectively.

Hereinafter the production methods of the compound of the present invention are explained.

The compound of the present invention can be produced according to a method known per se, for example, the method shown by the following production methods 1 to 5 or a method analogous thereto.

Each symbol of the compounds in the schematic drawings of the reaction schemes is as defined above unless particularly described. Z¹, Z², Z³, M, R^{A1} and R^{A2} are as defined in the following.

Z¹ is a leaving group, and specifically, a halogen atom (preferably chlorine, bromine, iodine), a hydroxy group, an optionally halogenated C₁₋₆ alkylsulfonyloxy group (e.g., methanesulfonyloxy, ethanesulfonyloxy, trifluoromethanesulfonyloxy), a C₁₋₆ alkylthio group (e.g., methylthio, ethylthio) and the like can be mentioned.

Z² is a leaving group, and specifically, a halogen atom (preferably chlorine, bromine), a hydroxy group; a group capable of forming an active ester group, such as a phenyloxy group (e.g., phenyloxy, 2,3,4,5,6-pentachlorophenyloxy, 4-nitrophenyloxy), a (succinimido-1-yl)oxy group, a (benzothiazol-2-yl)thio group and the like, each of which optionally has electron attractive group(s) (e.g., a halogen, a nitro group), and the like can be mentioned.

Z³ is a leaving group, and specifically, a halogen atom (preferably chlorine, bromine, iodine), a hydroxy group, an optionally halogenated C₁₋₆ alkylsulfonyloxy group (e.g., methanesulfonyloxy, ethanesulfonyloxy, trifluoromethanesulfonyloxy), a C₁₋₆ alkylthio group (e.g., methylthio, ethylthio) and the like can be mentioned. When L² is a carbonyl group, as Z³, a hydrogen atom, a C₁₋₆ alkyl group (in the case, L² is optionally bonded to R² to form a ring), a C₁₋₆ alkoxy group and the like can be mentioned.

M is a metal, and specifically, magnesium, lithium, zinc and the like can be mentioned.

R^{A1} and R^{A2} are each a hydrogen atom or a substituent. As the substituent, those similar to the aforementioned "substituent" for R³, and those similar to the substituents which the "4- to 7-membered nitrogen-containing non-aromatic heterocycle" of the "optionally substituted 4- to 7-membered nitrogen-containing non-aromatic heterocycle" for ring A optionally has, can be mentioned.

R^{c} is those similar to the optionally substituted hydrocarbon group exemplified as the aforementioned R^{a}.

P is an integer of 1 to 4.

q is an integer of 0 to 3.

r is an integer of 0 to 2.

s is an integer of 1 to 3.

### [Production method 1a]

Of the compound of the present invention, compound (V) can be produced, for example, according to the following method.

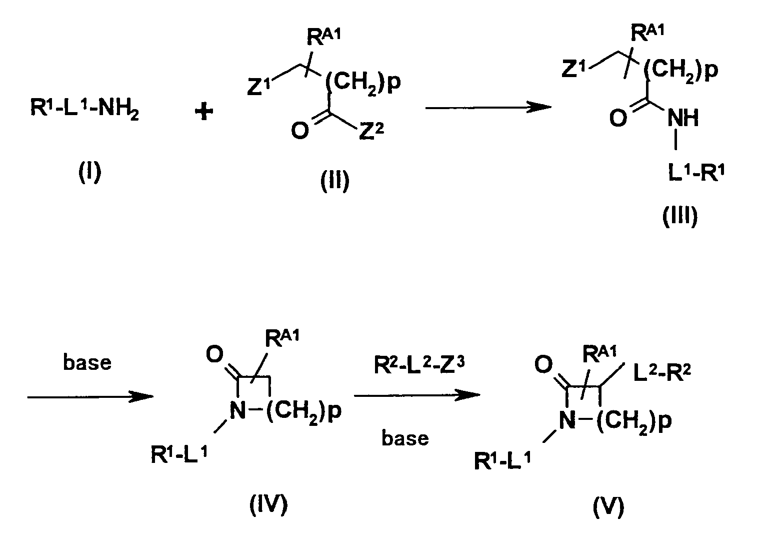

In this production method, compound (V) can be produced by reacting compound (I) with compound (II) to give compound (III), subjecting compound (III) to cyclization with a base to give compound (IV), and reacting compound (IV) with a compound represented by R²-L²-Z³ in the presence of a base.

Compound (I) is a primary amine, a substituted hydrazine or the like, and it is commercially available. Alternatively, compound (I) can be also produced according to a method known per se such as reductive amination and the like (e.g., the methods described in Tetrahedron Letters, 42, 4257 (2001); Journal of the Organic Chemistry, 46, 4376 (1981); Jikken Kagaku Kouza, the fourth edition, vol. 20, page 279 (1992) (Maruzen Press); and the like).

Compound (II) is, for example, commercially available as a carboxylic acid, a carboxylic halide and the like. Alternatively, compound (II) can be also produced according to a method known per se such as an activation of a carboxylic acid, and the like (the methods described in Jikken Kagaku Kouza, the fourth edition, vol. 22, pages 1, 115 (1992) (Maruzen Press); and the like).

In the production of compound (III), when Z² is a halogen atom, the reaction can be carried out in the presence of a base. As the base, for example, amines (e.g., triethylamine, N,N-diisopropylethylamine, pyridine, 4-dimethylaminopyridine), alkali metal carbonates (e.g., potassium carbonate, sodium carbonate, cesium carbonate) and the like can be mentioned. Of these, triethylamine, N,N-diisopropylethylamine, pyridine, 4-dimethylaminopyridine and the like are preferable.

The amount of the base to be used is generally 0.1 to 100 equivalents, preferably 1 to 10 equivalents, per 1 equivalent of compound (I).

This reaction is carried out, for example, in a solvent such as an ether solvent, a halogenated hydrocarbon solvent, a nitrile solvent, pyridine, an aromatic solvent, an ester solvent, an amide solvent and the like. These solvents may be used in a mixture at an appropriate ratio. Of these, tetrahydrofuran, dichloromethane, pyridine and the like are preferable.

The reaction temperature is generally about 0°C to 100°C, preferably 0 to 40°C.

The reaction time is, for example, 0.5 hr to 1 day.

In the production of compound (III), when Z² is a hydroxy group, the reaction can be carried out with a condensing agent (e.g., 1-ethyl-3-(3'-dimethylaminopropyl)carbodiimide (EDCI), 1,3-dicyclohexylcarbodiimide (DCC), bis(2-oxo-3-oxazolidinyl)phosphinic chloride (BOP-Cl), (benzotriazol-1-yloxy)tripyrrolidinophosphonium hexafluorophosphate (PyBOP)).

Of these, EDCI, BOP-Cl, PyBOP and the like are preferable.

The amount of the condensing agent to be used is generally 1 to 10 equivalents, preferably 1 to 3 equivalents, per 1 equivalent of compound (I).

In this reaction, an additive such as N-hydroxysuccinimide (HOSu), N-hydroxybenzotriazole (HOBt), amines (e.g., triethylamine, N,N-diisopropylethylamine, pyridine, 4-dimethylaminopyridine) and the like can be used together with a condensing agent to improve the reaction efficiency.

The amount of the additive to be used is generally 0.1 to 100 equivalents, preferably 0.1 to 10 equivalents, per 1 equivalent of compound (I).

This reaction is generally carried out in an inert solvent (e.g., an ether solvent, a halogenated hydrocarbon solvent, a nitrile solvent, an amide solvent). These solvents may be used in a mixture at an appropriate ratio. Of these, tetrahydrofuran, dichloromethane, acetonitrile and the like are preferable.

The reaction temperature is generally about 0°C to 100°C, preferably 0°C to 40°C.

The reaction time is, for example, 0.5 hr to 3 days.

In the production of compound (III), when Z² is a group capable of forming an active ester group, such as a phenyloxy group, a (succinimido-1-yl)oxy group, a (benzothiazol-2-yl)thio group and the like, the reaction can be carried out in the presence of a base. As the base, for example, amines (e.g., triethylamine, N,N-diisopropylethylamine, pyridine, 4-dimethylaminopyridine), alkali metal carbonates (e.g., potassium carbonate, sodium carbonate, cesium carbonate) and the like can be mentioned. Of these, triethylamine, N,N-diisopropylethylamine, pyridine, 4-dimethylaminopyridine and the like are preferable.

The amount of the base to be used is generally 0.1 to 100 equivalents, preferably 1 to 10 equivalents, per 1 equivalent of compound (I).

This reaction is carried out, for example, in a solvent such as an ether solvent, a halogenated hydrocarbon solvent, a nitrile solvent, pyridine, an aromatic solvent, an ester solvent, an amide solvent and the like. These solvents may be used in a mixture at an appropriate ratio. Of these, tetrahydrofuran, dichloromethane, pyridine and the like are preferable.

The reaction temperature is generally about 0°C to 100°C, preferably 0 to 40°C.

The reaction time is, for example, 0.5 hr to 1 day.

In the production of compound (IV), when Z¹ of compound (III) is a halogen atom or an optionally halogenated C₁₋₆ alkylsulfonyloxy group, the reaction can be carried out with a base.

As the base, for example, alkali metal hydrides (e.g., sodium hydride, potassium hydride), organic lithium amides (e.g., lithium diisopropylamide, lithium dicyclohexylamide, lithium bis(trimethylsilyl)amide), amines (e.g., triethylamine, pyridine, 4-dimethylaminopyridine, N,N-diisopropylethylamine, 1,8-diazabicyclo[5.4.0]undec-7-ene (DBU)), alkali metal hydroxides (e.g., sodium hydroxide, potassium hydroxide), alkali metal carbonates (e.g., potassium carbonate, sodium carbonate), alkali metal C₁₋₆ alkoxides (e.g., sodium methoxide, sodium ethoxide, potassium tert-butoxide) and the like can be mentioned. Of these, alkali metal hydrides such as sodium hydride and the like, and organic lithium amides such as lithium bis(trimethylsilyl)amide and the like are preferable.

The amount of the base to be used is generally 1 to 10 equivalents, preferably 1 to 3 equivalents, per 1 equivalent of compound (III).

In addition, an additive such as sodium iodide, potassium iodide and the like can be used together with a base. The amount of the additive to be used is generally 0.1 to 3 equivalents, preferably 0.1 to 1 equivalent, per 1 equivalent of compound (III).

This reaction is carried out, for example, in a solvent such as an amide solvent, an ether solvent, a halogenated hydrocarbon solvent, a nitrile solvent, an aromatic solvent and the like. These solvents may be used in a mixture at an appropriate ratio. Of these, N,N-dimethylformamide, tetrahydrofuran, xylene and the like are preferable.

The reaction temperature is generally -78°C to 100°C, preferably 0°C to 80°C.

The reaction time is, for example, 0.5 hr to 3 days.

In the production of compound (IV), when Z¹ of compound (III) is a hydroxy group, compound (IV) can be also produced by converting the hydroxy group to a halogen atom with a halogenating reagent, and reacting the resulting compound according to the aforementioned method.

As the halogenating reagent, for example, thionyl chloride, thionyl bromide, phosphorus trichloride, phosphorus pentachloride, phosphorus oxychloride, carbon tetrabromide and the like can be mentioned.

The amount of the halogenating reagent to be used is generally 1 to 100 equivalents, preferably 1 to 10 equivalents, per 1 equivalent of compound (III).

This reaction is generally carried out in an inert solvent (e.g., an ether solvent, a halogenated hydrocarbon solvent, an aromatic solvent etc.) or without a solvent. These solvents may be used in a mixture at an appropriate ratio. Of these, tetrahydrofuran, toluene, carbon tetrachloride and the like are preferable.

The reaction temperature is generally -20°C to 200°C, preferably 0°C to 100°C.

The reaction time is, for example, 0.5 hr to 16 hr.

When Z¹ of compound (III) is a hydroxy group, compound (IV) can be also produced by converting the hydroxy group to an optionally halogenated C₁₋₆ alkylsulfonyloxy group and the like with an optionally halogenated C₁₋₆ alkylsulfonyl chloride (e.g., methanesulfonyl chloride, ethanesulfonyl chloride, trifluoromethanesulfonyl chloride) and the like, and reacting the resulting compound according to the aforementioned method.

The amount of the optionally halogenated C₁₋₆ alkylsulfonyl chloride to be used is generally 1 to 100 equivalents, preferably 1 to 10 equivalents, per 1 equivalent of compound (III).

This reaction is generally carried out in an inert solvent (e.g., an ether solvent, a halogenated hydrocarbon solvent, an aromatic solvent etc.) or without a solvent. These solvents may be used in a mixture at an appropriate ratio. Of these, tetrahydrofuran, toluene, carbon tetrachloride and the like are preferable.

In the reaction of compound (III) with an optionally halogenated C₁₋₆ alkylsulfonyl chloride, a base can be used as necessary.

As the base, for example, amines (e.g., triethylamine, N,N-diisopropylethylamine, pyridine, 4-dimethylaminopyridine), alkali metal carbonates (e.g., potassium carbonate, sodium carbonate, cesium carbonate) and the like can be mentioned. Of these, triethylamine, N,N-diisopropylethylamine, pyridine, 4-dimethylaminopyridine and the like are preferable.

The reaction temperature is generally -20°C to 200°C, preferably 0°C to 100°C.

The reaction time is, for example, 0.5 hr to 16 hr.

When Z¹ of compound (III) is a hydroxy group, compound (IV) can be also produced according to the Mitsunobu reaction (e.g., the methods described in Tetrahedron Letters, 34, 1639 (1993), Tetrahedron Letters, 35, 5081 (1994), Tetrahedron Letters, 36, 2531 (1995), Tetrahedron Letters, 37, 2549 (1996), Journal of the Medicinal Chemistry, 44, 2933 (2001); and the like).

When Z¹ of compound (III) is a C₁₋₆ alkylthio group, compound (IV) can be produced by converting compound (III) to a sulfonium salt with an alkylating agent such as methyl iodide and the like, according to a method known per se (e.g., the methods described in Journal of the Medicinal Chemistry, 44, 2933 (2001); and the like), and subjecting the resulting compound to cyclization with a base.

As the base, for example, organic lithium amides (e.g., lithium diisopropylamide, lithium dicyclohexylamide, lithium bis(trimethylsilyl)amide), alkali metal hydrides (e.g., sodium hydride, potassium hydride), amines (e.g., pyridine, 4-dimethylaminopyridine, N,N-diisopropylethylamine, 1,8-diazabicyclo[5.4.0]undec-7-ene (DBU)), alkali metal hydroxides (e.g., sodium hydroxide, potassium hydroxide), alkali metal carbonates (e.g., potassium carbonate, sodium carbonate), alkali metal C₁₋₆ alkoxides (e.g., sodium methoxide, sodium ethoxide, potassium tert-butoxide) and the like can be mentioned. Of these, organic lithium amides such as lithium bis(trimethylsilyl)amide and the like, and alkali metal hydrides such as sodium hydride and the like are preferable.

The amount of the base to be used is generally 1 to 10 equivalents, preferably 1 to 3 equivalents, per 1 equivalent of compound (III).

This reaction is carried out, for example, in a solvent such as an amide solvent, an ether solvent, a halogenated hydrocarbon solvent, a nitrile solvent, pyridine, hexamethylphosphorotriamide and the like. These solvents may be used in a mixture at an appropriate ratio. Of these, N,N-dimethylformamide, tetrahydrofuran, hexamethylphosphorotriamide and the like are preferable.

The reaction temperature is generally -78°C to 150°C, preferably 0°C to 80°C.

The reaction time is, for example, 0.5 hr to 3 days.

compound (V) can be produced by reacting compound (IV) with a compound represented by R²-L²-Z³ in the presence of a base.

R²-L²-Z³ is, for example, an electrophilic reagent such as a halogenated alkyl, an aldehyde, a ketone, an ester, an acid halide, an acid anhydride and the like. These are commercially available. Alternatively, it can be also produced according to method known per se (e.g., the methods described in Jikken Kagaku Kouza, the fourth edition, vol. 19, pages 416, 460 (1992) (Maruzen Press); Jikken Kagaku Kouza, the fourth edition, vol. 21, pages 1, 149 (1992) (Maruzen Press); Jikken Kagaku Kouza, the fourth edition, vol. 22, pages 43, 115, 127 (1992) (Maruzen Press); and the like).

The amount of the compound R²-L²-Z to be used is generally 1 to 10 equivalents, preferably 1 to 3 equivalents, per 1 equivalent of compound (IV).

As the base, for example, organic lithium amides (e.g., lithium diisopropylamide, lithium dicyclohexylamide, lithium bis(trimethylsilyl)amide), alkali metal hydrides (e.g., sodium hydride, potassium hydride), alkali metal hydroxides (e.g., sodium hydroxide, potassium hydroxide), alkali metal C₁₋₆ alkoxides (e.g., sodium methoxide, sodium ethoxide, potassium tert-butoxide) and the like can be mentioned. Of these, organic lithium amides such as lithium diisopropylamide, lithium bis(trimethylsilyl)amide and the like are preferable.

The amount of the base to be used is generally 1 to 10 equivalents, preferably 1 to 3 equivalents, per 1 equivalent of compound (IV).

This reaction is generally carried out in an inert solvent (e.g., an ether solvent, an aromatic solvent, hexamethylphosphorotriamide etc.). These solvents may be used in a mixture at an appropriate ratio. Of these, tetrahydrofuran, toluene, hexamethylphosphorotriamide and the like are preferable.

The reaction temperature is generally -78°C to 100°C, preferably -78°C to 30°C.

The reaction time is, for example, 0.5 hr to 16 hr.

### [Production method 1b]

Of the compound of the present invention, compound (VIII) can be produced, for example, according to the following method.

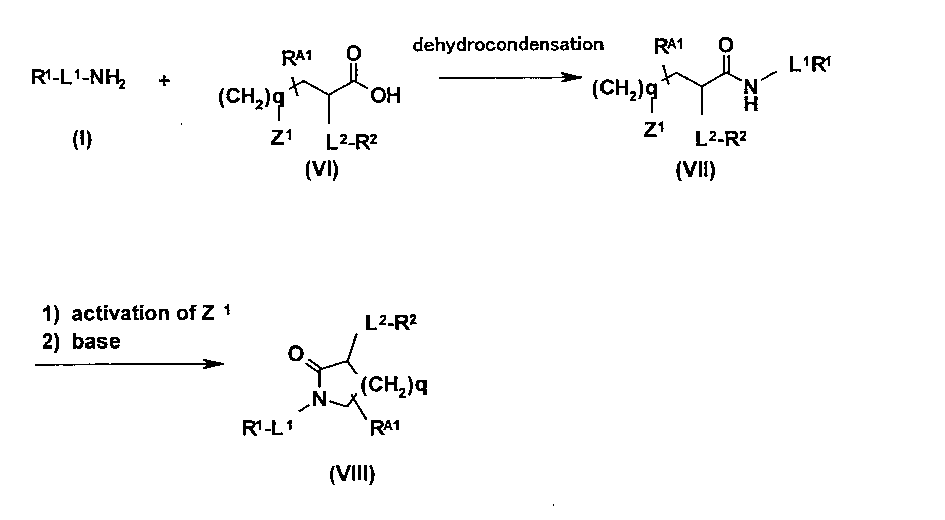

In this production method, compound (VIII) can be produced by reacting compound (I) with compound (VI) to give compound (VII), and subjecting compound (VII) to cyclization with a base.

Compound (VII) can be produced, for example, in the same manner as in the production of compound (III) wherein Z² is a hydroxy group, in the aforementioned Production method 1a.

In the production of compound (VIII), when Z¹ of compound (VII) is a hydroxy group, compound (VIII) can be produced, in the same manner as in the production of compound (IV) in the aforementioned Production method 1a, by converting the hydroxy group to a halogen atom or an optionally halogenated C₁₋₆ alkylsulfonyloxy group, and subjecting the resulting compound to cyclization with a base. Alternatively, compound (VIII) can be also produced by subjecting compound (VII) to the aforementioned Mitsunobu reaction.

When Z¹ of compound (VII) is a C₁₋₆ alkylthio group, compound (VIII) can be produced in the same manner as in the production of compound (IV) wherein Z¹ is a C₁₋₆ alkylthio group, in the aforementioned Production method 1a.

### [Production method 2]

Compound (VIII) can be also produced, for example, according to the following method.

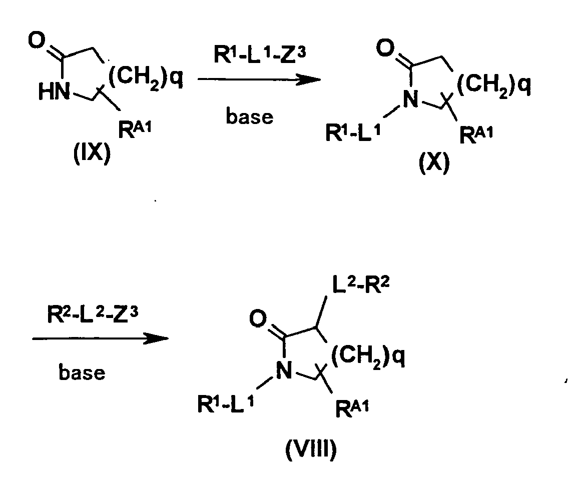

In this production method, compound (VIII) can be produced by reacting compound (IX) with a compound represented by R¹-L¹-Z³ in the presence of a base to give compound (X), and reacting the obtained compound (X) with a compound represented by R²-L²-Z³ in the presence of a base.

Compound (X) can be produced by reacting compound (IX) with a compound represented by R¹-L¹-Z³ in the presence of a base.

Compound (IX) is commercially available, or can be also produced according to a method known per se (e.g., the methods described in Jikken Kagaku Kouza, the fourth edition, vol.22, page 182 (1992) (Maruzen Press); and the like).

R¹-L¹-Z³ may be, for example, a halogenated alkyl and the like, and it is commercially available, or can be also produced according to a method known per se (e.g., the methods described in Jikken Kagaku Kouza, the fourth edition, vol. 19, pages 416, 460 (1992) (Maruzen Press); and the like).

The amount of the compound R¹-L¹-Z³ to be used is generally 1 to 10 equivalents, preferably 1 to 3 equivalents, per 1 equivalent of compound (IX).

As the base, for example, alkali metal hydrides (e.g., sodium hydride, potassium hydride), amines (e.g., pyridine, 4-dimethylaminopyridine, N,N-diisopropylethylamine, 1,8-diazabicyclo[5.4.0]undec-7-ene (DBU)), alkali metal hydroxides (e.g., sodium hydroxide, potassium hydroxide), alkali metal carbonates (e.g., potassium carbonate, sodium carbonate), alkali metal C₁₋₆ alkoxides (e.g., sodium methoxide, sodium ethoxide, potassium tert-butoxide) and the like can be mentioned. Of these, alkali metal hydrides such as sodium hydride and the like, and alkali metal carbonates such as potassium carbonate and the like are preferable.

The amount of the base to be used is generally 1 to 10 equivalents, preferably 1 to 3 equivalents, per 1 equivalent of compound (IX).

This reaction is carried out, for example, in a solvent such as an amide solvent, an ether solvent, a halogenated hydrocarbon solvent, a nitrile solvent, pyridine, an aromatic solvent, water-containing acetone and the like. These solvents may be used in a mixture at an appropriate ratio. Of these, N,N-dimethylformamide, tetrahydrofuran and the like are preferable.

The reaction temperature is generally 0°C to 150°C, preferably 0°C to 100°C.

The reaction time is, for example, 0.5 hr to 3 days.

When R¹-L¹-Z³ is a tertiary alkyl halide such as 1-bromoadamantane and the like, compound (X) can be also produced according to a method known per se such as an alkylation reaction by activation of a tertiary alkyl halide with a silver salt (e.g., silver sulfate), and the like (e.g., the methods described in Journal of the Medicinal Chemistry, 14, 535 (1971); and the like).

Compound (VIII) can be produced using compound (X) and in the same manner as in the production of compound (V) in the aforementioned Production method 1a.

### [Production method 3]

Compound (VIII) can be also produced, for example, according to the following method.

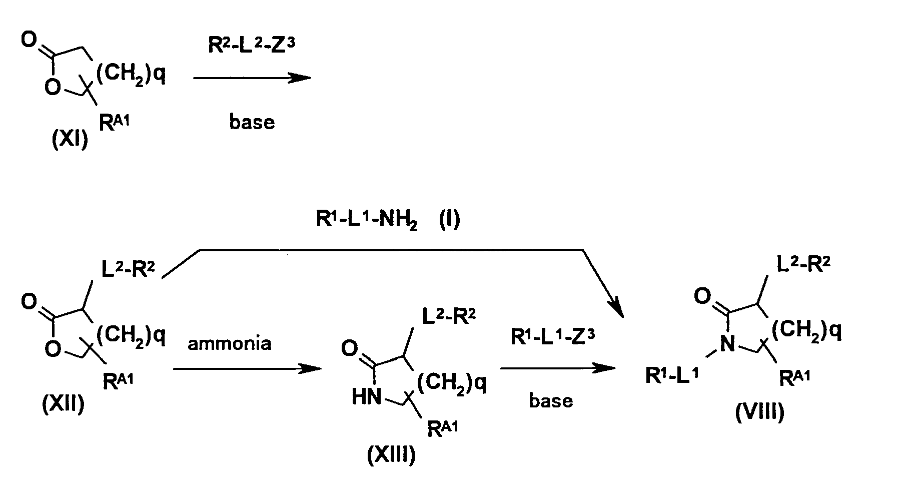

In this production method, compound (VIII) can be produced by reacting compound (XI) with a compound represented by R²-L²-Z³ in the presence of a base to give compound (XII), and reacting compound (XII) with compound (I). Alternatively, compound (VIII) can be produced by reacting compound (XII) with ammonia to give compound (XIII), and reacting the obtained compound (XIII) with a compound represented by R¹-L¹-Z³ in the presence of a base.

Compound (XI) is commercially available, or can be also produced according to a method known per se (e.g., the methods described in Jikken Kagaku Kouza, the fourth edition, vol. 22, page 83, (1992) (Maruzen Press); and the like).

Compound (XII) is commercially available, or can be produced using compound (XI) and in the same manner as in the production of compound (V) in the aforementioned Production method 1a. Alternatively, compound (XII) can be also produced according to a method known per se (e.g., the methods described in Journal of the Medicinal Chemistry, 35, 285 (1992); Jikken Kagaku Kouza, the fourth edition, vol. 22, page 83, (1992) (Maruzen Press); and the like).

Compound (VIII) can be produced according to a method known per se such as ring opening-cyclization reaction of compound (XII) using compound (I), and the like (e.g., the methods described in Tetrahedron Asymmetry, 10, 3877 (1999); and the like).

Alternatively, compound (VIII) can be also produced using compound (XIII) and in the same manner as in the production of compound (X) in the aforementioned Production method 2.

Compound (XIII) can be produced according to a method known per se such as ring opening-cyclization reaction of compound (XII) using ammonia, and the like (e.g., the methods described in Journal of the Medicinal Chemistry, 35, 285 (1992); and the like).

### [Production method 4a]

Of the compound of the present invention, compound (XVII) can be produced, for example, according to the following method.

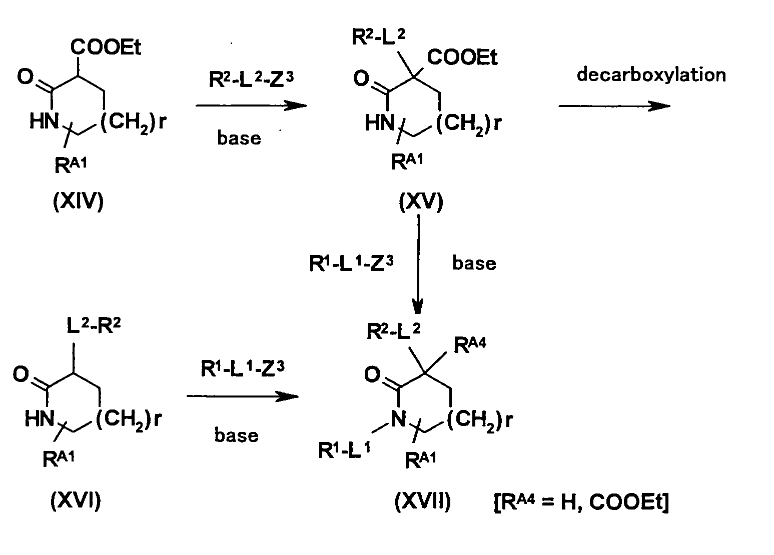

In this production method, compound (XVII) can be produced by reacting compound (XIV) with a compound represented by R²-L²-Z³ in the presence of a base to give compound (XV); and converting compound (XV) to compound (XVI) by decarboxylation, then reacting compound (XVI) with a compound represented by R¹-L¹-Z³ in the presence of a base, or directly reacting compound (XV) with a compound represented by R¹-L¹-Z³ in the presence of a base.

Compound (XIV) is commercially available, or can be produced according to a method known per se (e.g., the methods described in Jikken Kagaku Kouza, the fourth edition, vol. 22, page 182, (1992) (Maruzen Press); and the like).

Compound (XV) can be produced by reacting compound (XIV) with a compound represented by R²-L²-Z³ in the presence of a base.

R²-L²-Z³ is, for example, an electrophilic reagent such as a halogenated alkyl and the like, and it is commercially available, or can be produced according to a method known per se (e.g., the methods described in Jikken Kagaku Kouza, the fourth edition, vol. 19, pages 416, 460, (1992) (Maruzen Press); and the like).

As the base, for example, organic lithium amides (e.g., lithium diisopropylamide, lithium dicyclohexylamide, lithium bis(trimethylsilyl)amide), alkali metal hydrides (e.g., sodium hydride, potassium hydride), alkali metal hydroxides (e.g., sodium hydroxide, potassium hydroxide), alkali metal C₁₋₆ alkoxides (e.g., sodium methoxide, sodium ethoxide, potassium tert-butoxide) and the like can be mentioned. Of these, alkali metal C₁₋₆ alkoxides such as sodium methoxide and the like are preferable.

The amount of the base to be used is generally 1 to 10 equivalents, preferably 1 to 3 equivalents, per 1 equivalent of compound (XIV).

This reaction is generally carried out in an inert solvent (e.g., an ether solvent, an aromatic solvent etc.). These solvents may be used in a mixture at an appropriate ratio. Of these, tetrahydrofuran, 1,2-dimethoxyethane and the like are preferable.

The amount of the R²-L²-Z³ to be used is generally 1 to 10 equivalents, preferably 1 to 3 equivalents, per 1 equivalent of compound (XIV).

The reaction temperature is generally 0°C to 100°C, preferably 30°C to 80°C.

The reaction time is, for example, 0.5 hr to 16 hr.

Compound (XVI) can be produced by subjecting compound (XV) to decarboxylation under an acidic or basic condition, according to a method known per se (e.g., the methods described in Tetrahedron, 48, 2761 (1992); and the like).

Compound (XVII) can be produced using compound (XV) or compound (XVI) and in the same manner as in the production of compound (X) in the aforementioned Production method 2.

### [Production method 4b]

Of the compound of the present invention, compound (XX) and compound (XXI) can be produced, for example, according to the following method.

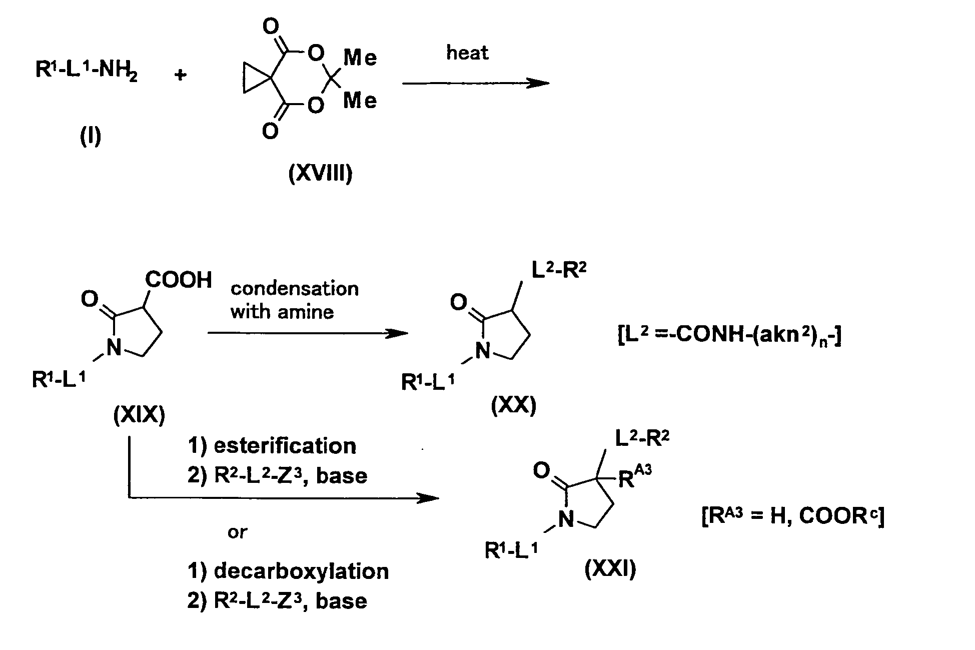

In this production method, compound (XX) can be produced by reacting compound (I) with compound (XVIII), i.e., 6,6-dimethyl-5,7-dioxaspiro[2.5]octane-4,8-dione to give compound (XIX), and subjecting compound (XIX) to dehydration condensation with an amine. Compound (XXI) can be produced by subjecting compound (XIX) to esterification, and reacting the resulting compound with a compound represented by R²-L²-Z³ in the presence of a base; or by subjecting compound (XIX) to decarboxylation, and reacting the resulting compound with a compound represented by R²-L²-Z³ in the presence of a base.

Compound (XIX) can be produced using compound (I) and compound (XVIII) according to a method known per se (e.g., the methods described in Journal of the Medicinal Chemistry, 47, 530 (2004); and the like).

Compound (XX) can be produced using compound (XIX) and a primary or secondary amine and in the same manner as in the production of compound (III) in the aforementioned Production method 1a (L² is -CONH-(akn²)n-).

For example, compound (XXI) wherein R^{A3} is COOR^{c} can be produced by subjecting compound (XIX) to esterification according to a method known per se (e.g., the methods described in Jikken Kagaku Kouza, the fourth edition, vol. 22, page 43 (1992) (Maruzen Press); and the like), and reacting the resulting compound in the same manner as in the production of compound (XV) in the aforementioned Production method 4a. Compound (XXI) wherein R^{A3} is a hydrogen atom can be also produced by subjecting compound (XIX) to decarboxylation under an acidic or basic condition, according to a method known per se (e.g., the methods described in Tetrahedron, 48, 2761 (1992); and the like), and reacting the resulting compound in the same manner as in the production of compound (V) in the aforementioned Production method 1a.

### [Production method 5a]

Of the compound of the present invention, compound (XXIV) can be produced, for example, according to the following method.

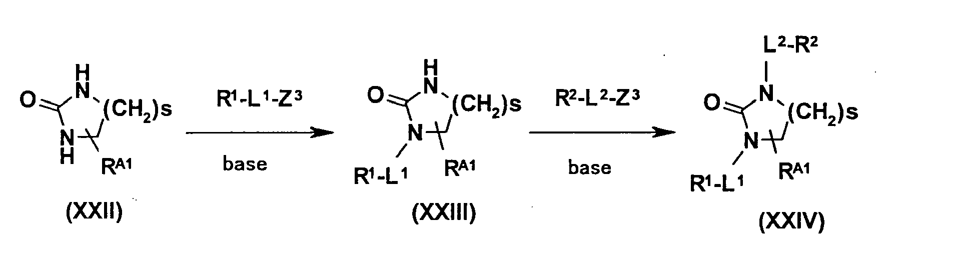

In this production method, compound (XXIV) can be produced by reacting compound (XXII) with a compound represented by R¹-L¹-Z³ in the presence of a base to give compound (XXIII), and reacting compound (XXIII) with a compound represented by R²-L²-Z³ in the presence of a base.

Compound (XXII) is commercially available, or can be also produced according to a method known per se (e.g., the methods described in Journal of the Medicinal Chemistry, 42, 2870 (1999); and the like).

Compound (XXIII) can be produced using compound (XXII) and in the same manner as in the production of compound (X) in the aforementioned Production method 2. Alternatively, compound (XXIII) is commercially available, or can be produced according to a method known per se (e.g., the methods described in Journal of the Organic Chemistry, 64, 2941 (1999); and the like).

Compound (XXIV) can be produced using compound (XXIII) and in the same manner as in the production of compound (X) in the aforementioned Production method 2.

### [Production method 5b]

Of the compound of the present invention, compound (XXVIII) can be produced, for example, according to the following method.

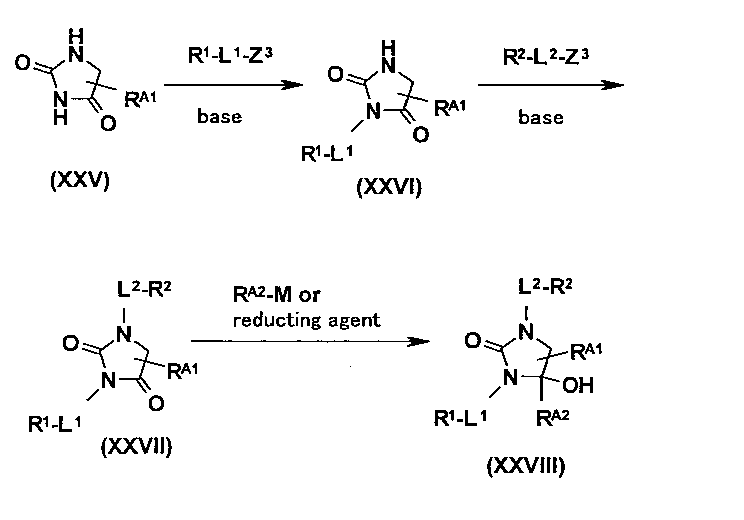

In this production method, compound (XXVIII) can be produced by reacting compound (XXV) with a compound represented by R¹-L¹-Z³ in the presence of a base to give compound (XXVI); reacting compound (XXVI) with a compound represented by R²-L²-Z³ in the presence of a base to give compound (XXVII); and reacting compound (XXVII) with an organic metal reagent represented by R^{A2}-M, or subjecting compound (XXVII) to a reduction reaction.

Compound (XXV) is commercially available, or can be also produced according to a method known per se (e.g., the methods described in Journal of the Organic Chemistry, 30, 3414 (1965); and the like).

Compound (XXVI) can be produced using compound (XXV) and in the same manner as in the production of compound (X) in the aforementioned Production method 2. Alternatively, compound (XXVI) is commercially available, or can be also produced according to a method known per se (e.g., the methods described in Journal of the Organic Chemistry, 30, 3414 (1965); and the like).

Compound (XXVII) can be produced using compound (XXVI) and in the same manner as in the production of compound (X) in the aforementioned Production method 2.

Compound (XXVIII) can be produced by reacting compound (XXVII) with an organic metal reagent represented by R^{A2}-M, or subjecting compound (XXVII) to a reduction reaction.

For example, compound (XXVIII) wherein R^{A2} is an alkyl group can be produced by reacting compound (XXVII) with an organic metal reagent (R^{A2}-M).

As the organic metal reagent, for example, Grignard reagents (e.g., methylmagnesium bromide, ethylmagnesium bromide, isopropylmagnesium bromide), organic lithium reagents (e.g., methyllithium, butyllithium), organic zinc reagents (e.g., zinc dimethyl, zinc diethyl) and the like can be used, and Grignard reagents such as methylmagnesium bromide and the like, and organic lithium reagents such as methyllithium and the like are preferable.

The amount of the organic metal reagent to be used is generally 1 to 10 equivalents, preferably 1 to 5 equivalents, per 1 equivalent of compound (XXVII).

This reaction is carried out, for example, in a solvent such as an ether solvent, an aromatic solvent and the like.

These solvents may be used in a mixture at an appropriate ratio. Of these, tetrahydrofuran, toluene and the like are preferable.

The reaction temperature is generally -78°C to 100°C, preferably 0°C to 50°C.

The reaction time is, for example, 0.5 hr to 1 day.

Compound (XXVIII) wherein R^{A2} is a hydrogen atom can be produced by subjecting compound (XXVII) to a reduction reaction.

The reduction reaction is generally carried out with a reducing agent. As the reducing agent, for example, metal hydrogen complex compounds (e.g., sodium borohydride, lithium borohydride, lithium aluminum hydride), metal hydrides (e.g., diisobutylaluminum hydride, tri-n-butyltin hydride) and the like can be mentioned. Lithium aluminum hydride is preferable.

The amount of the reducing agent to be used is generally 1 to 10 equivalents, preferably 1 to 5 equivalents, per 1 of equivalent compound (XXVII).

This reaction is carried out, for example, in a solvent such as an ether solvent, an aromatic solvent, an alcohol solvent and the like. These solvents may be used in a mixture at an appropriate ratio. Tetrahydrofuran and the like are preferable.

The reaction temperature is generally -78°C to 100°C, preferably 0°C to 50°C.

The reaction time is, for example, 0.5 hr to 7 days.

In the above-mentioned production method, the "ether solvent", "halogenated hydrocarbon solvent", "aromatic solvent", "nitrile solvent", "ester solvent", "amide solvent", "ketone solvent", "sulfoxide solvent" and "alcohol solvent" mean the following.

As the aforementioned "ether solvent", for example, diethyl ether, tetrahydrofuran (THF), 1,4-dioxane, 1,2-dimethoxyethane and the like can be mentioned.

As the aforementioned "halogenated hydrocarbon solvent", for example, dichloromethane, chloroform, 1,2-dichloroethane, carbon tetrachloride and the like can be mentioned.

As the aforementioned "aromatic solvent", for example, benzene, toluene, xylene, pyridine and the like can be mentioned.

As the aforementioned "nitrile solvent", for example, acetonitrile, propionitrile and the like can be mentioned.

As the aforementioned "ester solvent", for example, ethyl acetate, methyl acetate and the like can be mentioned.

As the aforementioned "amide solvent", for example, N,N-dimethylformamide (DMF), N,N-dimethylacetamide, N-methylpyrrolidone and the like can be mentioned.

As the aforementioned "ketone solvent", for example, acetone, methyl ethyl ketone and the like can be mentioned.

As the aforementioned "sulfoxide solvent", for example, dimethyl sulfoxide (DMSO) and the like can be mentioned.

As the aforementioned "alcohol solvent", for example, methanol, ethanol, isopropanol, tert-butanol and the like can be mentioned.

In the production of the compound of the present invention, when the starting compound has an amino group, a carboxyl group, a hydroxy group or a carbonyl group as a substituent, a protecting group generally used in peptide chemistry and the like may be introduced into these groups. By removing the protecting group as necessary after the reaction, the objective compound can be obtained.

Examples of the amino-protecting group include a formyl group; a C₁₋₆ alkyl-carbonyl group, a C₁₋₆ alkoxy-carbonyl group, a benzoyl group, a C₇₋₁₃ aralkyl-carbonyl group (e.g., benzylcarbonyl), a C₇₋₁₃ aralkyloxy-carbonyl group (e.g., benzyloxycarbonyl, 9-fluorenylmethoxycarbonyl), a trityl group, a phthaloyl group, an N,N-dimethylaminomethylene group, a tri-substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like can be mentioned. These groups are optionally substituted by 1 to 3 substituents selected from a halogen atom, a C₁₋₆ alkoxy group, a nitro group and the like.

Examples of the carboxyl-protecting group include a C₁₋₆ alkyl group, a C₇₋₂₀ aralkyl group (e.g., benzyl, trityl), a phenyl group, a tri-substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like. These groups are optionally substituted by 1 to 3 substituents selected from a halogen atom, a C₁₋₆ alkoxy group, a nitro group and the like.

Examples of the hydroxy-protecting group include a C₁₋₆ alkyl group, a phenyl group, a trityl group, a C₇₋₁₃ aralkyl group (e.g., benzyl), a formyl group, a C₁₋₆ alkyl-carbonyl group, a benzoyl group, a C₇₋₁₃ aralkyl-carbonyl group (e.g., benzylcarbonyl), a 2-tetrahydropyranyl group, a 2-tetrahydrofuranyl group, a tri-substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like can be mentioned. These groups are optionally substituted by 1 to 3 substituents selected from a halogen atom, a C₁₋₆ alkyl group, a C₁₋₆ alkoxy group, a nitro group and the like.

Examples of the carbonyl-protecting group include a cyclic acetal (e.g., 1,3-dioxane), a non-cyclic acetal (e.g., a di-C₁₋₆ alkylacetal) and the like can be mentioned.

For introduction or elimination of the above-mentioned protecting group, a method known per se, for example, a method described in Protective Groups in Organic Synthesis, John Wiley and Sons (1980) and the like can be mentioned. For example, employed is a method using acid, base, UV light, hydrazine, phenyl hydrazine, sodium N-methyldithiocarbamate, tetrabutylammonium fluoride, palladium acetate, trialkylsilyl halide (e.g., trimethylsilyl iodide, trimethylsilyl bromide and the like) and the like, reduction and the like.

The compound of the present invention obtained according to the above-mentioned production method can be isolated and purified by a known means, for example, solvent extraction, liquid conversion, phase transfer, crystallization, recrystallization, chromatography and the like. In addition, various starting material compounds used in each of the above-mentioned production methods can be isolated and purified by a known means such as those mentioned above and the like. Alternatively, the starting material compounds may be directly used in the form of a reaction mixture without isolation as the starting materials of the next step.

For the production of the compound of the present invention, when the starting material compound can form a salt, the compound may also be used in the form of a salt. As such salt, those similar to the salts of the aforementioned compound of the present invention can be mentioned.

When the compound of the present invention contains an optical isomer, a stereoisomer, a positional isomer or a rotational isomer, these are encompassed in the compound of the present invention, and obtained as a single product according to a synthetic method and separation method known per se. For example, an optical isomer and an optical isomer resolved from this compound are also encompassed in the compound of the present invention.

The compound of the present invention may be in the form of a crystal.

The crystal of the compound of the present invention (hereinafter sometimes to be abbreviated as the crystal of the present invention) can be produced by crystallization of the compound of the present invention according to a crystallization method known *per se*.

In the present specification, the melting point refers to that measured using, for example, micromelting point measuring apparatus (Yanako, MP-500D or Buchi, B-545) or DSC (differential scanning calorimetry) device (SEIKO, EXSTAR6000) and the like.

In general, melting points vary depending on measurement apparatuses, measurement conditions and the like. The crystal in the present specification may show a different melting point described in the present specification, as long as it is within general error range.

The crystal of the present invention is superior in physicochemical properties (e.g., melting point, solubility, stability and the like) and biological properties (e.g., pharmacokinetics (absorption, distribution, metabolism, excretion), efficacy expression and the like), and is extremely useful as a pharmaceutical agent.

### Examples

The present invention is explained in more detail by way of the following Examples, Formulation Examples and Experimental Examples, which do not limit the present invention and may be changed without departing from the scope of the present invention.

Abbreviations in the Reference Example and Examples have the following meanings:
s: singlet, d: doublet, t: triplet, q: quartet, m: multiplet, brs: broad singlet, J: coupling constant

In the Reference Examples and Examples, room temperature means a temperature of from 1 to 30°C. Unless otherwise specified, % means wt%.

In the following Examples, the "less polar product" and "more polar product" mean the following. When four steric isomers are present as the reaction products and the reaction mixture is purified by silica gel column chromatography, a mixture of two isomers eluted earlier using a solvent system having low polarity is referred to as the "less polar product" and a mixture of two isomers eluted later using a solvent system having high polarity is referred to as the "more polar product".

Moreover, genetic manipulation methods described in Reference Examples below are based on the methods described in Maniatis et al., Molecular Cloning, Cold Spring Harbor Laboratory, 1989, and the appended protocol of reagents. SEQ ID NOs in the sequence listing in the present specification show the following sequences.

### SEQ ID NO: 1

The base sequence of the primer used for PCR reaction in the following Reference Example 1a.

### SEQ ID NO: 2

The base sequence of the primer used for PCR reaction in the following Reference Example 1a.

### Reference Example 1a

### Cloning of human 11βHSD1 gene and preparation of recombinant Baculovirus

Human 11βHSD1 gene was cloned by PCR reaction using human liver cDNA (Clontech, QUICK-Clone cDNA) as a template and the following primer sets prepared in reference to the base sequence of human 11βHSD1 gene reported by Tannin, G.M. et al. (J. Biol. Chem., 266(25), 16653-16658 (1991)).

### hhsd1-EcoS:

5'-AAAGAATTCGCCATGGCTTTTATGAAAAAATATCTCCTCCC-3' (SEQ ID NO: 1)

### hhsd1-PstA:

5'-AAAACTGCAGCTACTTGTTTATGAATCTGTCCAT-3' (SEQ ID NO: 2)

PCR reaction was conducted according to the protocol attached to Pyrobest polymerase (Takara). The obtained PCR product was electrophoresed on agarose gel (1%). A 0.9 kb DNA fragment containing 11βHSD1 gene was recovered from the gel, and then digested with restriction enzymes EcoRI and PstI. The DNA treated with the restriction enzymes was electrophoresed on agarose gel (1%), and the obtained two fragments were recovered and ligated to plasmid pFASTBAC1 (Invitrogen) digested with restriction enzymes EcoRI and PstI to give expression plasmid pFB-hHSD1. The base sequence of the insertion fragment was confirmed and found to be identical with the object sequence. Using BAC-TO-BAC Baculovirus Expression System (Invitrogen), virus stock BAC-HHSD1 of recombinant Baculovirus was prepared. Reference Example 2a

### Preparation of human 11βHSD1 enzyme

Sf-21 cells (Invitrogen) were inoculated at 1x10⁶ cells/ml to Sf-900 II SFM medium (Invitrogen) (150 mL) containing 10% fetal bovine serum and cultured at 27°C for 24 hr. Recombinant Baculovirus BAC-HHSD1 (1.0 ml) obtained in Reference Example 1a was added to the culture and the cells were further cultured for 72 hr. The cells were separated from the culture medium by centrifugation (2000 rpm, 10 min) and washed twice with PBS.

The cells were suspended in a buffer (100 mM sucrose, 50 mM potassium chloride, 40 mM potassium dihydrogen phosphate, 30 mM ethylenediamine tetraacetic acid (EDTA), 1X Protease inhibitor complete EDTA Free (Roche) pH 7.2, 15 ml) and disrupted by 3 times of treatment in a homogenizer (POLYTRON) (20000 rpm, 30 sec). The microsome fraction obtained by centrifugation (35000 rpm, 60 min) was suspended in 10 mL of a buffer (PBS containing 5% glycerol). The obtained suspension was subjected to a treatment in a homogenizer (POLYTRON) (10000 rpm, 10 sec) and preserved at -80°C. The obtained microsome fraction was used as the human 11βHSD1 enzyme in the below-mentioned Experimental Example.

### Reference Example 1A 1-(2-methylbenzyl)pyrrolidin-2-one

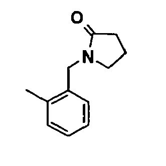

To a mixture of 2-pyrrolidone (5.0 g), α-bromo-o-xylene (9.9 g) and N,N-dimethylformamide (100 mL) was added 60% sodium hydride (2.35 g), and the mixture was stirred at room temperature for 1 hr. Water was added to the reaction mixture, and the mixture was extracted with ethyl acetate. The extract was washed successively with water and saturated brine, and dried over anhydrous magnesium sulfate. The solvent was evaporated under reduced pressure, and the residue was subjected to silica gel column chromatography (hexane-ethyl acetate 4:1-1:1) to give the title compound (6.90 g, 68%) as a colorless solid.
1H NMR (300 MHz, CDCl₃) δ ppm 1.80-2.13 (m, 2 H), 2.30 (s, 3 H), 2.45 (t, J=8.1 Hz, 2 H), 3.09-3.35 (m, 2 H), 4.47 (s, 2 H), 7.05-7.23 (m, 4 H).

### Reference Example 2A 1-(2, 6-dichlorobenzyl)pyrrolidin-2-one

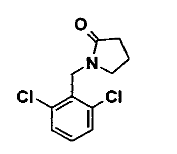

In the same manner as in Reference Example 1A, the title compound was obtained from 2-pyrrolidone and α,2,6-trichlorotoluene.
1H NMR (300 MHz, CDCl₃) δ ppm 1.86-2.02 (m, 2 H), 2.41 (t, J=8.1 Hz, 2 H), 3.02-3.23 (m, 2 H), 4.81 (s, 2 H), 7.15-7.25 (m, 1 H), 7.29-7.41 (m, 2 H).

### Reference Example 3A 1-(1-methyl-1-phenylethyl)pyrrolidin-2-one

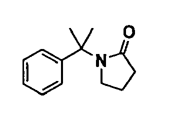

To a mixture of cumylamine (5.0 g), triethylamine (5.6 mL) and tetrahydrofuran (200 mL) was added a solution of 4-chlorobutyryl chloride (5.74 g) in tetrahydrofuran (50 mL) at 0°C, and the mixture was stirred at room temperature for 3 hr. The precipitate was removed by filtration, the filtrate was concentrated under reduced pressure, and the obtained residue was partitioned between ethyl acetate and water. The ethyl acetate layer was washed successively with 10% aqueous sodium bicarbonate and saturated brine, and dried over anhydrous magnesium sulfate, and the solvent was evaporated under reduced pressure to give a colorless solid (6.68 g). To a mixture of this solid (4.89 g) and N,N-dimethylformamide (50 mL) was added 60% sodium hydride (1.6 g), and the mixture was stirred at room temperature for 1 hr. Water was added to the reaction solution, and the mixture was extracted with ethyl acetate. The extract was washed successively with water and saturated brine, and dried over anhydrous magnesium sulfate. The solvent was evaporated under reduced pressure, and the residue was subjected to silica gel column chromatography (hexane-ethyl acetate 4:1-1:1) to give the title compound (3.0 g, 74%) as a colorless oil. 1H NMR (300 MHz, CDCl₃)δ ppm 1.75 (s, 6 H), 1.89-2.03 (m, 2 H), 2.38 (t, J=8.1 Hz, 2 H), 3.35-3.46 (m, 2 H), 7.16-7.28 (m, 1 H), 7.32 (d, J=4.5 Hz, 4 H).

### Reference Example 4A 1-(1-phenylcyclopropyl)pyrrolidin-2-one

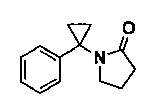

In the same manner as in Reference Example 3A, the title compound was obtained from 1-phenylcyclopropanamine and 4-chlorobutyryl chloride.
1H NMR (300 MHz, CDCl₃) δ ppm 1.38-1.49 (m, 4 H), 1.62-2.06 (m, 2 H), 2.41 (t, J=8.1 Hz, 2 H), 3.40-3.44 (m, 2 H), 7.19-7.33 (m, 5 H).

### Reference Example 5A 1-(2,3-dihydro-1H-inden-1-yl)pyrrolidin-2-one

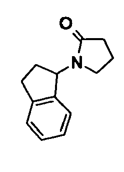

In the same manner as in Reference Example 3A, the title compound was obtained from 1-aminoindane and 4-chlorobutyryl chloride.
1H NMR (300 MHz, CDCl₃)δ ppm 1.81-2.12 (m, 3 H), 2.27-2.64 (m, 3 H), 2.74 -3.14 (m, 3 H), 3.11-3.32 (m, 1 H), 5.80 (t, J=7.7 Hz, 1 H), 7.02-7.43 (m, 4H).

### Reference Example 6A 1-(1,2,3,4-tetrahydronaphthalen-1-yl)pyrrolidin-2-one

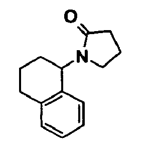

In the same manner as in Reference Example 3A, the title compound was obtained from 1,2,3,4-tetrahydro-1-naphthylamine and 4-chlorobutyryl chloride.
1H NMR (300 MHz, CDCl₃)δ ppm 1.71-2.11 (m, 6 H), 2.43-2.56 (m, 2 H), 2.69 -2.86 (m, 2 H), 2.96-3.12 (m, 1 H), 3.18-3.34 (m, 1 H), 5.34-5.53 (m, 1 H), 6.95-7.06 (m, 1 H), 7.06-7.22 (m, 3 H). Reference Example 7A ethyl 4-(2-oxopyrrolidin-1-yl)piperidine-1-carboxylate

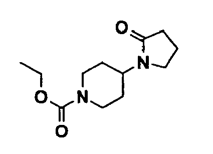

In the same manner as in Reference Example 3A, the title compound was obtained from ethyl 4-aminopiperidine-1-carboxylate and 4-chlorobutyryl chloride.
1H NMR (300 MHz, CDCl₃)δ ppm 1.26 (t, J=7.1 Hz, 3 H), 1.48-1.76 (m, 4 H), 1.92-2.11 (m, 2 H), 2.35-2.47 (m, 2 H), 2.73-2.93 (m, 2 H), 3.27-3.37 (m, 2H), 4.02-4.37 (m, 5 H).

### Reference Example 8A 1-(tetrahydro-2H-pyran-4-yl)pyrrolidin-2-one

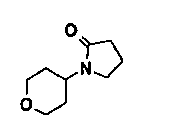

In the same manner as in Reference Example 3A, the title compound was obtained from tetrahydro-2H-pyran-4-amine and 4-chlorobutyryl chloride.
1H NMR (300 MHz, CDCl₃) δ ppm 1.54-1.66 (m, 2 H), 1.67-1.85 (m, 2 H), 1.93 -2.10 (m, 2 H), 2.33-2.47 (m, 2 H), 3.30-3.42 (m, 2 H), 3.43-3.56 (m, 2 H), 4.02 (dd, J=11.6, 4.6 Hz, 2 H), 4.15-4.30 (m, 1 H).

### Reference Example 9A 1-(tetrahydro-2H-thiopyran-4-yl)pyrrolidin-2-one

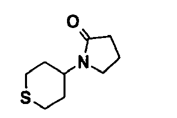

In the same manner as in Reference Example 3A, the title compound was obtained from tetrahydro-2H-thiopyran-4-amine and 4-chlorobutyryl chloride.
1H NMR (300 MHz, CDCl₃) δ ppm 1.71-1.89 (m, 2 H), 1.90-2.09 (m, 4 H), 2.29 -2.47 (m, 2 H), 2.59-2.73 (m, 2 H), 2.73-2.92 (m, 2 H), 3.28-3.41 (m, 2 H), 3.86-4.05 (m, 1 H).

### Reference Example 10A 1-cyclohexyl-2-oxopyrrolidine-3-carboxylic acid

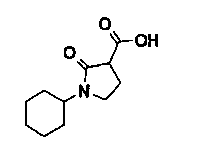

A mixture of cyclohexylamine (9.52 g), 6,6-dimethyl-5,7-dioxaspiro[2.5]octane-4,8-dione (10.0 g) and acetonitrile (150 mL) was stirred at 65°C for 2 hr under nitrogen atmosphere. Cyclohexylamine (0.5 mL) was added to the solution, and the mixture was stirred at 65°C for 3 hr, and then at room temperature for 48 hr. The precipitate was collected by filtration, and dissolved in water. This solution was adjusted with hydrochloric acid to pH 2, and extracted with ethyl acetate. The extract was washed with saturated brine, and dried over anhydrous magnesium sulfate, and the solvent was evaporated under reduced pressure to give the title compound (9.36 g, 75%) as a colorless solid.
1H NMR (300 MHz, CDCl₃) δ ppm 1.09-1.18 (m, 1 H), 1.31-1.49 (m, 4 H), 1.70-1.85 (m, 4 H), 2.21-2.35 (m, 1 H), 2.40-2.51 (m, 1 H), 3.32-3.89 (m, 4 H), 3.92 -3.93 (m, 1 H).

### Reference Example 11A tert-butyl (1-cyclohexyl-2-oxopyrrolidin-3-yl)carbamate

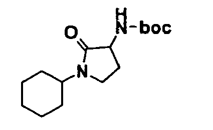

A mixture of cyclohexylamine (4.3 g), Boc-DL-methionine (12.0 g), 1-ethyl-3-(3'-dimethylaminopropyl)carbodiimide (9.2 g), N-hydroxybenzotriazole (6.5 g) and acetonitrile (200 mL) was stirred at room temperature for 16 hr. Water was added to the reaction solution, and the mixture was extracted with ethyl acetate. The ethyl acetate layer was washed successively with water and saturated brine, and dried over anhydrous magnesium sulfate, and the solvent was evaporated under reduced pressure. The obtained residue was subjected to silica gel column chromatography (hexane-ethyl acetate 1:1) to give a colorless solid (13.2 g). A mixture of this solid and methyl iodide (100 mL) was stirred at room temperature for 48 hr under nitrogen atmosphere. The reaction solution was concentrated under reduced pressure, and the obtained solid was dissolved in tetrahydrofuran (500 mL). Lithium bis(trimethylsilyl)amide (1.1 mol/L, a tetrahydrofuran solution, 36.2 mL) was added to the solution at 0°C under nitrogen atmosphere, and the mixture was stirred for 3 hr. Saturated aqueous ammonium chloride (30 mL) was added to the reaction solution, and the mixture was concentrated under reduced pressure. The residue was partitioned between ethyl acetate and water, and the ethyl acetate layer was washed with saturated brine, and dried over anhydrous magnesium sulfate. The solvent was evaporated under reduced pressure, and the residue was subjected to silica gel column chromatography (hexane-ethyl acetate 9:1-1:1) to give the title compound (2.79 g, 25%) as a colorless solid.
1H NMR (300 MHz, CDCl₃) δ ppm 1.00-1.52 (m, 5 H), 1.45 (s, 9 H), 1.61-1.88 (m, 6 H), 2.53-2.73 (m, 1 H), 3.14-3.41 (m, 2 H), 3.84-4.03 (m, 1 H), 4.06-4.29 (m, 1 H), 4.97-5.25 (m, 1 H).

### Reference Example 12A tert-butyl [4-(bromomethyl)phenyl](methyl)carbamate

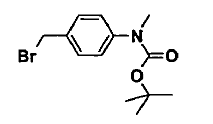

A mixture of p-toluidine (10.7 g), di-tert-butyl dicarbonate (24.0 g) and tetrahydrofuran (300 mL) was heated under reflux for 2 hr. The reaction solution was concentrated under reduced pressure, and the residue was dissolved in ethyl acetate. This solution was washed successively with 1 M aqueous citric acid solution and saturated brine, and dried over anhydrous magnesium sulfate. The solvent was evaporated under reduced pressure, and the residue was subjected to silica gel column chromatography to give a colorless solid from a fraction eluted with hexane-ethyl acetate (4:1). To a mixture of this compound, methyl iodide (21.3 g) and N,N-dimethylformamide (300 mL) was added 60% sodium hydride (6.0 g), and the mixture was stirred at room temperature for 2 hr. Water was added to the reaction solution, and the mixture was extracted with ethyl acetate. The ethyl acetate layer was washed successively with water and saturated brine, and dried over anhydrous magnesium sulfate. The solvent was evaporated under reduced pressure to give a yellow oil. A mixture of this oil (5.0 g), N-bromosuccinimide (4.42 g), azobisisobutyronitrile (0.4 g) and carbon tetrachloride (90 mL) was heated under reflux for 20 min. The reaction solution was filtrated, and the filtrate was concentrated under reduced pressure. The obtained residue was subjected to silica gel column chromatography (hexane-ethyl acetate 95:1-7:3) to give the title compound (4.71 g, 71%) as a yellow solid.
1H NMR (300 MHz, CDCl₃) δ ppm 1.46 (s, 9 H), 3.26 (s, 3 H), 4.49 (s, 2 H), 7.18-7.25 (m, 2 H), 7.31-7.37 (m, 2 H).

### Reference Example 13A methyl 1-cyclohexyl-2-oxopyrrolidine-3-carboxylate

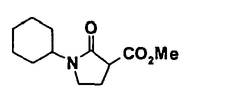

A mixture of cyclohexylamine (31 g), 6,6-dimethyl-5,7-dioxaspiro[2.5]octane-4,8-dione (31.3 g) and acetonitrile (450 mL) was stirred at 65°C for 2 hr. Cyclohexylamine (5.5 g) was added, and the mixture was stirred at 65°C for 1.5 hr, and then at room temperature for 14 hr. The white precipitate in the reaction mixture was collected by filtration, and washed with acetonitrile-diisopropyl ether. The obtained white substance was suspended in brine containing 1N hydrochloric acid (250 mL), and the mixture was extracted with ethyl acetate-tetrahydrofuran (3:1, v/v). The aqueous layer was extracted again with ethyl acetate, and the organic layers were combined, washed with saturated brine, and dried over anhydrous magnesium sulfate.

The solvent was evaporated under reduced pressure, and the residue was washed with diisopropyl ether to give 1-cyclohexyl-2-oxopyrrolidine-3-carboxylic acid (25.6 g, 66%) as white crystals. A mixture of the obtained carboxylic acid (15 g), concentrated sulfuric acid (0.7 mL) and methanol (500 mL) was heated under reflux for 18 hr. After cooling, the reaction mixture was concentrated under reduced pressure, saturated aqueous sodium hydrogencarbonate was added to the residue, and the mixture extracted with ethyl acetate. The organic layer was washed with saturated brine, and dried over anhydrous magnesium sulfate. The solvent was evaporated under reduced pressure, and the residue was crystallized from diisopropyl ether-hexane to give the title compound (13.4 g, 84%) as colorless crystals.
1H NMR (300 MHz, CDCl₃) δ ppm 1.06-1.16 (m, 1H), 1.29-1.44 (m, 4H), 1.64-1.80 (m, 5H), 2.16-2.28 (m, 1H), 2.31-2.42 (m, 1H), 3.28-3.35 (m, 1 H), 3.42-3.52 (m, 2H), 3.77 (s, 3H), 3.89-3.96 (m, 1H).

### Reference Example 14A methyl 1-cyclohexyl-5-oxopyrrolidine-3-carboxylate

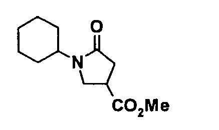

A mixture of cyclohexylamine (12 g), dimethyl itaconate (21.1 g) and methanol (80 mL) was heated under reflux for 16 hr. After cooling, the reaction mixture was concentrated under reduced pressure, diluted hydrochloric acid was added to the residue, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 2:3-1:4) to give the title compound (22.3 g, 82%) as a pale-yellow oil.
1H NMR (300 MHz, CDCl₃)δppm 1.05-1.15 (m, 1H), 1.29-1.46 (m, 4H), 1.64-1.72 (m, 3H), 1.78-1.82 (m, 2H), 2.60-2.76 (m, 2H), 3.14-3.25 (m, 1H), 3.49- 3.59 (m, 2H), 3.74 (s, 3H), 3.89-4.00 (m, 1H).

### Reference Example 15A 1-(1-adamantyl)-5-oxoproline ethyl ester

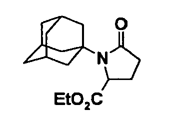

A mixture of 1-bromoadamantane (7.50 g), ethyl 2-oxopyrrolidine-5-carboxylate (16.4 g), silver sulfate (10.9 g) and N-methylpyrrolidone (5 mL) was stirred at 60°C for 1.5 hr, and then at 95°C for 6 hr. After cooling, ethyl acetate was added to the reaction mixture, the pale-green insoluble substance was filtrated through celite, and washed with ethyl acetate. Water was added to the filtrate, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 3:2-2:3) to give the title compound (4.14 g, 41%) as a pale-yellow oil.
1H NMR (300 MHz, CDCl₃) δ ppm 1.31 (t, J=7.2 Hz, 3H), 1.63-1.72 (m, 6H), 1.88-1.97 (m, 1H), 2.05-2.08 (m, 6H), 2.13-2.31 (m, 5H), 2.48-2.62 (m, 1H), 4.24 (q, J=7.2 Hz, 2H), 4.35-4.39 (m, 1H).

### Reference Example 16A (4S)-1-(1-adamantyl)-4-hydroxypyrrolidin-2-one

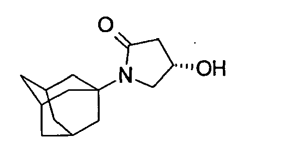

In the same manner as in Reference Example 15A, the title compound was obtained from 1-bromoadamantane and (4S)-4-hydroxypyrrolidin-2-one.
1H NMR (300 MHz, CDCl₃) δ ppm 1.57-1.64 (m, 6 H), 1.68-1.76 (m, 6 H), 2.16 (m, 3 H), 2.27-2.35 (dd, J=17.1, 6.0 Hz, 1 H), 2.49-2.58 (dd, J=17.1, 9.0 Hz, 1 H), 3.22-3.26 (dd, J=9.6, 4.8 Hz, 1 H), 3.54-3.60 (dd, J=9.9, 7.2 Hz, 1 H), 4.57 (quint, J=6.0 Hz, 1 H), 5.72 (br, 1 H).

### Reference Example 17A 1-cyclohexyl-4-(hydroxymethyl)pyrrolidin-2-one

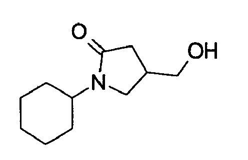

A mixture of methyl 1-cyclohexyl-5-oxopyrrolidine-3-carboxylate obtained in Reference Example 14A (1.58 g) and ethanol (30 mL) was added to sodium borohydride (2.66 g) over 2 hr at 0°C, and the mixture was stirred at room temperature for 14 hr. Water was added to the reaction solution, and the mixture was extracted with ethyl acetate. The ethyl acetate layer was washed with saturated brine, and dried over anhydrous magnesium sulfate. The solvent was evaporated under reduced pressure, and the residue was crystallized from hexane-ethyl acetate to give the title compound (1.30 g, 94%) as a colorless solid.
1H NMR (300 MHz, CDCl₃) δ ppm 1.10 (m, 1 H), 1.29-1.39 (m, 4 H), 1.68-1.76 (m, 4 H), 2.03 (m, 1 H), 2.18-2.24 (t, J=9.0 Hz, 1 H), 2.47-2.53 (m, 2 H), 2.74 (m, 1 H), 3.18-3.22 (m, 1 H), 3.43-3.48 (t, J=7.5 Hz, 1 H), 3.52-3.61 (m, 2 H), 3.91 (m, 1 H).

### Reference Example 18A methyl 3-(2-oxopyrrolidin-1-yl)adamantane-1-carboxylate

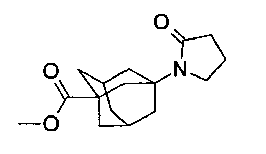

In the same manner as in Reference Example 15A, the title compound was obtained from methyl 3-bromoadamantane-1-carboxylate and 2-pyrrolidinone.
1H NMR (300 MHz, CDCl₃) δ ppm 1.64-1.68 (m, 2 H), 1.80-1.96 (m, 6 H), 2.07-2.37 (m, 10 H), 3.39-3.48 (m, 2 H), 3.65 (s, 3 H).

Reference Example 19A 1-[3-(hydroxymethyl)-1-adamantyl]pyrrolidin-2-one

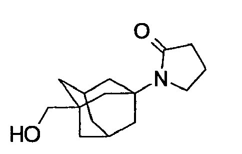

To a mixture of methyl 3-(2-oxopyrrolidin-1-yl)adamantane-1-carboxylate obtained in Reference Example 18A (2.7 g) and tetrahydrofuran (40 mL) was added lithium borohydride (1.63 g) at 0°C, and the mixture was stirred at room temperature for 3 hr. Water was added to the reaction mixture, and the mixture was extracted with ethyl acetate. The ethyl acetate layer was washed with saturated brine, and dried over anhydrous magnesium sulfate. The solvent was evaporated under reduced pressure, and the residue was crystallized from hexane-ethyl acetate to give the title compound (2.2 g, 91%) as a colorless solid.
1H NMR (300 MHz, CDCl₃)δ ppm 1.43-1.60 (m, 6 H), 1.67-1.75 (m, 2 H), 1.84-1.94 (m, 3 H), 2.09 (m, 4 H), 2.18 (m, 2 H), 2.30-2.36 (m, 2 H), 3.26-3.28 (m, 2 H), 3.43-3.48 (m, 2 H).

Reference Example 20A 1-(2,3-dihydro-1H-indol-1-yl)pyrrolidin-2-one

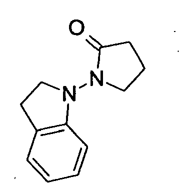

In the same manner as in Reference Example 3A, the title compound was obtained from indolin-1-amine and 4-chlorobutyryl chloride.
1H NMR (300 MHz, CDCl₃) δ ppm 2.09-2.16 (m, 2 H), 2.43-2.51 (m, 2 H), 3.05-3.07 (m, 2 H), 3.39-3.46 (m, 2 H), 3.57 (m, 2 H), 6.48-6.52 (m, 1 H), 6.80-6.87 (m, 2 H), 7.06-7.12 (m, 2 H).

### Reference Example 21A 1-(6-methoxy-2,3-dihydro-1H-inden-1-yl)pyrrolidin-2-one

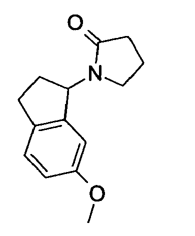

In the same manner as in Reference Example 3A, the title compound was obtained from 6-methoxyindan-1-amine and 4-chlorobutyryl chloride.
1H NMR (300 MHz, CDCl₃) δ ppm 1.87-2.05 (m, 3 H), 2.31-2.51 (m, 3 H), 2.82-2.94 (m, 2 H), 2.98-3.10 (m, 1 H), 3.17-3.29 (m, 1 H), 3.78 (s, 3 H), 5.72-5. 80 (t, J=7.8 Hz, 1 H), 6. 56 (d, J=2.2 Hz, 1 H), 6. 77-6. 83 (dd, J=8.8, 2.2 Hz, 1 H), 7.12-7.16 (d, J=8.8 Hz, 1 H).

Reference Example 22A 1-(piperidin-1-yl)pyrrolidin-2-one

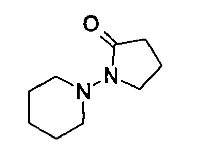

In the same manner as in Reference Example 3A, the title compound was obtained from piperidin-1-amine and 4-chlorobutyryl chloride.
1H NMR (300 MHz, CDCl₃) δ ppm 1.37-1.44 (m, 2 H), 1.67-1.75 (m, 4 H), 1.96-2.09 (m, 2 H), 2.30-2.36 (m, 2 H), 2.88-2.92 (m, 4 H), 3.42-3.47 (m, 2 H).

### Reference Example 23A 1-(morpholin-4-yl)pyrrolidin-2-one

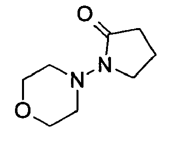

In the same manner as in Reference Example 3A, the title compound was obtained from morpholin-4-amine and 4-chlorobutyryl chloride.
1H NMR (300 MHz, CDCl₃) δ ppm 1.98-2.07 (m, 2 H), 2.33-2.38 (m, 2 H), 2.96-2.99 (m, 4 H), 3.44-3.49 (m, 2 H), 3.81-3.84 (m, 4 H). Reference Example 24A 2-chloro-4-(methoxymethoxy)benzyl bromide

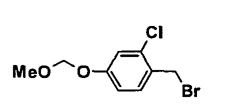

To a solution of 3-chloro-4-methylphenol (25 g) in tetrahydrofuran (200 mL) was added sodium hydride (60% oil; 10.5 g) by small portions under ice-cooling. The mixture was stirred at 0°C for 2.5 hr, and chloromethyl methyl ether (20 mL) was gradually added dropwise. The reaction mixture was stirred at 0°C for 30 min, and then at room temperature 1.5 hr. Saturated aqueous ammonium chloride was added to the reaction mixture under ice-cooling, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 95:5-9:1) to give 2-chloro-4-methoxymethoxytoluene (30.2 g, 93%) as a colorless oil. A mixture of the obtained 2-chloro-4-methoxymethoxytoluene (30.2 g), N-bromosuccinimide (30.2 g), α,α'-azobisisobutyronitrile (2.65 g) and carbon tetrachloride (120 mL) was stirred at 100°C for 1.5 hr. After cooling, the white insoluble substance was filtrated, and washed with a small amount of dichloromethane. The filtrate was concentrated under reduced pressure, and the residue was dissolved in ethyl acetate. The organic layer was washed with saturated aqueous sodium hydrogencarbonate. The aqueous layer was extracted again with ethyl acetate, and the organic layers were combined, washed with saturated brine, and dried over anhydrous magnesium sulfate. The solvent was evaporated under reduced pressure, and the residue was subjected to silica gel column chromatography (hexane-ethyl acetate 98:2-9:1) to give the title compound (11.3 g, 26%) as a pale-brown oil.
1H NMR (300 MHz, CDCl₃) δ ppm 3.47 (s, 3H), 4.58 (s, 2H), 5.16 (s, 2H), 6.92 (dd, J=8.4, 2.4 Hz, 1H), 7.10 (d, J=2.4 Hz, 1H), 7.34 (d, J=8.4 Hz, 1H) .

### Reference Example 25A 1-[(1S)-2,3-dihydro-1H-inden-1-yl]pyrrolidin-2-one

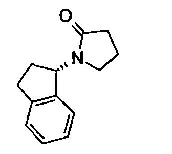

In the same manner as in Reference Example 3A, the title compound was obtained from (1S)-indan-1-amine and 4-chlorobutyryl chloride.
1H NMR (300 MHz, CDCl₃)δ ppm 1.84-2.07 (m, 3 H), 2.31-2.54 (m, 3 H), 2.82-3.10 (m, 3 H), 3.14-3.28 (m, 1 H), 5.80 (t, J=7.6 Hz, 1 H), 7.05-7.33 (m, 4 H).

### Reference Example 26A 1-[(1R)-2,3-dihydro-1H-inden-1-yl]pyrrolidin-2-one

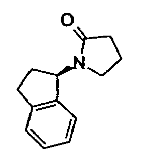

In the same manner as in Reference Example 3A, the title compound was obtained from (1R)-indan-1-amine and 4-chlorobutyryl chloride.
1H NMR (300 MHz, CDCl₃) δ ppm 1.86-2.07 (m, 3 H), 2.32-2.53 (m, 3 H), 2.83-3.09 (m, 3 H), 3.15-3.27 (m, 1 H), 5.80 (t, J=7.6 Hz, 1 H), 7.04-7.35 (m, 4 H).

Reference Example 27A 1-[(1S, 2R)-2-hydroxy-2, 3-dihydro-1H-inden-1-yl]pyrrolidin-2-one

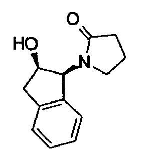

In the same manner as in Reference Example 3A, the title compound was obtained from (1S,2R)-1-aminoindan-2-ol and 4-chlorobutyryl chloride.
1H NMR (300 MHz, CDCl₃) δ ppm 1.80-2.11 (m, 2 H), 2.39-2.60 (m, 2 H), 2.84-3.12 (m, 2 H), 3.14-3.41 (m, 3 H), 4.73-4.88 (m, 1 H), 5.48 (d, J=6.8 Hz, 1 H), 7.16-7.35 (m, 4H).

### Reference Example 28A 1-((1S,2R)-2-{[tert-butyl(dimethyl)silyl]oxy}-2,3-dihydro-1H-inden-1-yl)pyrrolidin-2-one

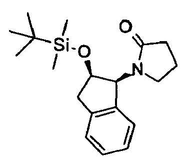

To a solution of 1-[(1S,2R)-2-hydroxy-2,3-dihydro-1H-inden-1-yl]pyrrolidin-2-one obtained in Reference Example 27A (724 mg) and imidazole (295 mg) in N,N-dimethylformamide (1 mL) was added tert-butyldimethylchlorosilane (603 mg), and the mixture was stirred at 50°C for 1 hr. Water was added to the reaction mixture, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 95:5-85:15) to give the title compound (1.03 g, 93%) as a colorless oil.
1H NMR (300 MHz, CDCl₃) δ ppm 0.00 (s, 3 H), 0.04 (s, 3 H), 0.80 (s, 9 H), 1.65-1.99 (m, 2 H), 2.28-2.41 (m, 1 H), 2.73-2.96 (m, 2 H), 3.11 (dd, J=16.2, 7.5 Hz, 1 H), 3.25-3.41 (m, 1 H), 4.56-4.69 (m, 1H), 5.49 (d, J=7.2 Hz, 1 H), 7.07-7.23 (m, 4 H).

### Reference Example 29A tert-butyl [3-chloro-4-(chloromethyl)phenyl](methyl)carbamate

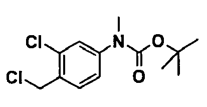

A mixture of tert-butyl [3-chloro-4-(hydroxymethyl)phenyl]carbamate (0.79 g), acetyl chloride (0.26 g), triethylamine (0.48 mL) and tetrahydrofuran (10 mL) was stirred at room temperature for 1 hr. The reaction mixture was partitioned between ethyl acetate (40 mL) and water (40 mL), the ethyl acetate layer was washed with saturated brine, and dried over anhydrous magnesium sulfate, and the solvent was evaporated under reduced pressure. The obtained residue was dissolved in N,N-dimethylformamide (10 mL), methyl iodide (0.42 g) and sodium hydride (0.12 g) were added successively under ice-cooling, and the mixture was stirred at room temperature for 30 min. The reaction mixture was poured into water (40 mL), and the mixture was extracted with ethyl acetate (40 mL). The ethyl acetate layer was washed with water and saturated brine, and dried over anhydrous magnesium sulfate. The solvent was evaporated under reduced pressure. The obtained residue was dissolved in ethanol (10 mL), 2N sodium hydroxide (2 mL) was added thereto, and the mixture was stirred at room temperature for 24 hr. The reaction mixture was concentrated under reduced pressure, and the obtained residue was partitioned between ethyl acetate (30 mL) and water (30 mL). The ethyl acetate layer was washed with saturated brine, and dried over anhydrous magnesium sulfate. The solvent was evaporated under reduced pressure, and the obtained residue was dissolved in tetrahydrofuran (5 mL). Thionyl chloride (0.49 g) was added dropwise to the solution under ice-cooling, and the mixture was stirred at 0°C for 10 min, and then at room temperature for 30 min. The reaction mixture was evaporated under reduced pressure to give the title compound (0.54 g, 61%) as a pale-yellow oil.
1H NMR (300 MHz, CDCl₃)δppm 1.47 (s, 9 H), 3.26 (s, 3 H), 4.68 (s, 2 H), 7.18 (dd, J=8.3, 2.3 Hz, 1 H), 7.34 (d, J=2.3 Hz, 1 H), 7.40 (d, J=8.3 Hz, 1 H).

### Reference Example 30A 1-(trans-4-hydroxycyclohexyl)pyrrolidin-2-one

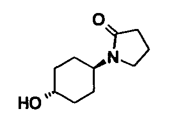

In the same manner as in Reference Example 3A, the title compound was obtained from trans-4-aminocyclohexanol and 4-chlorobutyryl chloride.
1H NMR (300 MHz, CDCl₃) δ ppm 1.45-1.60 (m, 2 H), 1.70-1.86 (m, 2 H), 1.90-2.20 (m, 7 H), 2.39 (t, J=8.1 Hz, 2 H), 3.25-3.40 (m, 2 H), 3.88-4.15 (m, 1 H), 4.54-4.80 (m, 1 H).

Reference Example 31A 1-(4-methoxycyclohexyl)pyrrolidin-2-one (a mixture of cis and trans)

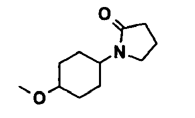

In the same manner as in Reference Example 3A, the title compound was obtained from 4-methoxycyclohexanamine and 4-chlorobutyryl chloride.
1H NMR (300 MHz, CDCl₃) δ ppm 1.25-1.58 (m, 4 H), 1.63-1.83 (m, 2 H), 1.89-2.19 (m, 4 H), 2.32-2.44 (m, 2 H), 3.31 and 3.34 (s, 3 H), 3.32-3.40 (m, 2 H), 3.41-3.47 (m, 1 H), 3.88-4.09 (m, 1 H). Reference Example 32A benzyl 3-(2-oxopyrrolidin-1-yl)piperidine-1-carboxylate

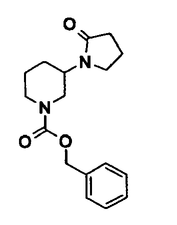

In the same manner as in Reference Example 3A, the title compound was obtained from benzyl 3-aminopiperidine-1-carboxylate and 4-chlorobutyryl chloride.
1H NMR (300 MHz, CDCl₃) δ ppm 1.43-1.92 (m, 5 H), 1.92-2.12 (m, 2 H), 2.29-2.47 (m, 2 H), 2.54-2.96 (m, 2 H), 3.22-3.50 (m, 2 H), 3.87-4.23 (m, 2 H), 4.98-5.34 (m, 2 H), 7.28-7.46 (m, 5 H).

Reference Example 33A 1-[(1R,2R,4S)-bicyclo[2.2.1]hept-2-yl]pyrrolidin-2-one

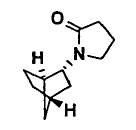

In the same manner as in Reference Example 3A, the title compound was obtained from (1R,2R,4S)-bicyclo[2.2.1]heptan-2-amine and 4-chlorobutyryl chloride.
1H NMR (300 MHz, CDCl₃) δ ppm 1.04-1.61 (m, 7 H), 1.66-1.82 (m, 1 H), 1.84-2.09 (m, 2 H), 2.18 (s, 1 H), 2.23-2.45 (m, 3 H), 3.24-3.53 (m, 2 H), 4.05 (dd, J=8.4, 5.2 Hz, 1 H).

Reference Example 34A 1-[4-(hydroxymethyl)cyclohexyl]pyrrolidin-2-one (a mixture of cis-form and trans-form)

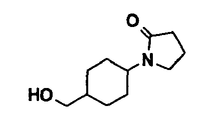

In the same manner as in Reference Example 3A, the title compound was obtained from (4-aminocyclohexyl)methanol and 4-chlorobutyryl chloride.

LC-MS (ESI+); m/z 198 (M+H)⁺

### Reference Example 35A 2-chloro-4-methoxy-3-(methoxymethoxy)benzaldehyde

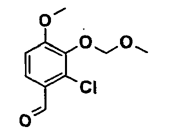

A mixture of 2-chloro-3-hydroxy-4-methoxybenzaldehyde (8.5 g), chloromethyl methyl ether (4.0 g), sodium hydride (60% oil; 2.0 g) and N,N-dimethylformamide (200 mL) was stirred at 0°C for 1 hr. The reaction mixture was poured into water (500 mL), and the mixture was extracted with ethyl acetate (500 mL). The ethyl acetate layer was washed with water, 1N sodium hydroxide and saturated brine, and dried over anhydrous magnesium sulfate. The solvent was evaporated under reduced pressure, and the residue was purified by silica gel column chromatography (hexane-ethyl acetate 4:1) to give the title compound (10.4 g, 99%) as a colorless solid.
1H NMR (300 MHz, CDCl₃) δ ppm 3.67 (s, 3 H), 3.95 (s, 3 H), 5.19 (s, 2 H), 6. 93 (d, J=8. 9 Hz, 1 H), 7.75 (d, J=8. 9 Hz, 1 H), 10.36 (s, 1 H).

### Reference Example 36A 2,6-dichloro-4-methoxy-3-(methoxymethoxy)benzaldehyde

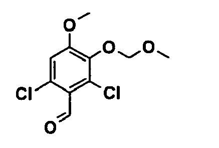

In the same manner as in Reference Example 35A, the title compound was obtained from 2,6-dichloro-3-hydroxy-4-methoxybenzaldehyde.
1H NMR (300 MHz, CDCl₃) δ ppm 3.65 (s, 3 H), 3.94 (s, 3 H), 5.17 (s, 2 H), 6.92 (s, 1 H), 10.41 (s, 1H).

### Reference Example 37A 1-(5-methoxy-1,2,3,4-tetrahydronaphthalen-1-yl)pyrrolidin-2-one

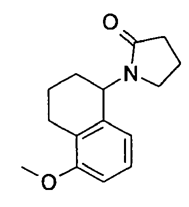

In the same manner as in Reference Example 3A, the title compound was obtained from 5-methoxy-1,2,3,4-tetrahydronaphthalen-1-amine.
1H NMR (300 MHz, CDCl₃) δ ppm 1.73-1.80 (m, 2 H), 1.91-2.08 (m, 4 H), 2.47-2.56 (m, 3 H), 2.75-2.82 (m, 1 H), 3.02-3.09 (m, 1 H), 3.20-3.29 (m, 1 H), 3.82 (s, 3 H), 5.37-5.41 (m, 1 H), 6.63-6.66 (d, J=7.8 Hz, 1 H), 6.70-6.73 (d, J=8.1 Hz, 1 H), 7.09-7.15 (d, J=7.8 Hz, 1 H).

Reference Example 38A 1-(5-hydroxy-2-adamantyl)pyrrolidin-2-one

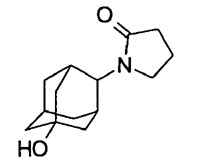

In the same manner as in Reference Example 3A, the title compound was obtained from 4-aminoadamantan-1-ol hydrochloride.
1H NMR (300 MHz, CDCl₃)δ ppm 1.46-1.59 (m, 2 H), 1.59-2.12 (m, 11 H), 2.18 (d, J=3.0 Hz, 1 H), 2.32-2.51 (m, 4 H), 3.64 (t, J=6.9 Hz, 2 H), 3.92 (s, 1 H).
LC/MS (ESI+); m/z 236 (M+H)⁺

### Reference Example 39A (2,6-dichloro-4-methoxyphenyl)methanol and

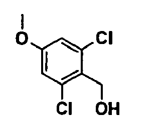

### Reference Example 40A (2,4-dichloro-6-methoxyphenyl)methanol

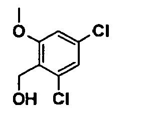

A mixture of 3,5-dichloroanisole (5.0 g), paraformaldehyde (1.1 g), concentrated hydrochloric acid (50 mL) and concentrated sulfuric acid (0.5 mL) was stirred at 60°C for 16 hr. The reaction mixture was poured into ice, and the mixture was extracted with diethyl ether (100 mL). The extract was washed successively with water, saturated sodium hydrogencarbonate solution and saturated brine, and dried over anhydrous magnesium sulfate. The solvent was evaporated under reduced pressure, and a mixture of the obtained pale-yellow oil (6.57 g), 1N sodium hydroxide (56 mL) and dioxane (28 mL) was heated under reflux for 3 hr. The reaction mixture was extracted with dichloromethane (50 mL), and the extract was washed with water and saturated brine, and dried over anhydrous magnesium sulfate. The solvent was evaporated under reduced pressure, and the obtained residue was subjected to silica gel column chromatography (hexane-ethyl acetate 9:1) to give a mixture (3:1) of (2,6-dichloro-4-methoxyphenyl)methanol and (2,4-dichloro-6-methoxyphenyl)methanol.

### Reference Example 39A

1H NMR (300 MHz, CDCl₃) δ ppm 1.95 (t, J=6.8 Hz, 1 H), 3.80 (s, 3H), 4.89 (d, J=6.8 Hz, 2 H), 6.88 (s, 2 H).

### Reference Example 40A

1H NMR (300 MHz, CDCl₃) δ ppm 2.20 (t, J=6.8 Hz, 1 H), 3.88 (s, 3H), 4.81 (d, J=6.8 Hz, 2 H), 6.81 (s, 1 H), 7.04 (s, 1 H). Reference Example 41A 1,3-dichloro-2-(chloromethyl)-5-methoxybenzene and

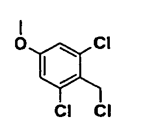

### Reference Example 42A 1,5-dichloro-2-(chloromethyl)-3-methoxybenzene

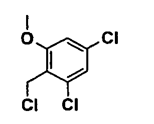

To a solution of a mixture (1.50 g, 3:1) of (2,6-dichloro-4-methoxyphenyl)methanol obtained in Reference Example 39A and (2,4-dichloro-6-methoxyphenyl)methanol obtained in Reference Example 40A in tetrahydrofuran (30 mL) was added thionyl chloride (1.06 g) under ice-cooling, and the mixture was stirred at room temperature for 1 hr. The reaction solution was poured into saturated sodium hydrogencarbonate solution (60 mL), and the mixture was extracted with ethyl acetate (50 mL). The extract was washed with saturated brine, and dried over anhydrous magnesium sulfate, and the solvent was evaporated under reduced pressure to give a mixture (1.57 g, 96%, 3:1) of 1,3-dichloro-2-(chloromethyl)-5-methoxybenzene and 1,5-dichloro-2-(chloromethyl)-3-methoxybenzene, as a colorless solid.

### Reference Example 41A

1H NMR (300 MHz, CDCl₃) δ ppm 3.80 (s, 3 H), 4.82 (s, 2 H), 4.89 (d, J=6.8 Hz, 2 H), 6.90 (s, 2 H).

### Reference Example 42A

1H NMR (300 MHz, CDCl₃)δ ppm 3.89 (s, 3 H), 4.75 (s, 2 H), 4.89 (d, J=6.8 Hz, 2 H), 6.81 (s, 1 H), 7.04 (s, 1 H).

### Reference Example 43A 3-(2,6-dichlorobenzyl)-1-(2-hydroxy-1,1-dimethylethyl)pyrrolidin-2-one

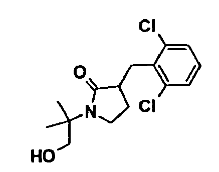

In the same manner as in Example 255A, the title compound was obtained from 1-(2-hydroxy-1,1-dimethylethyl)pyrrolidin-2-one and α,2,6-trichlorotoluene.
1H NMR (300 MHz, CDCl₃) δ ppm 1.27 (s, 3 H), 1.30 (s, 3 H), 1.77-2.08 (m, 2 H), 2.84-3.16 (m, 2 H), 3.23-3.39 (m, 1 H), 3.40-3.57 (m, 2 H), 3.71-3.84 (m, 2 H), 5.16 (t, J=7.0 Hz, 1 H), 7.00-7.20 (m, 1 H), 7.30 (d, J=8.3 Hz, 2 H).

### Reference Example 44A 2-[3-(2,6-dichlorobenzyl)-2-oxopyrrolidin-1-yl]-2-methylpropanal

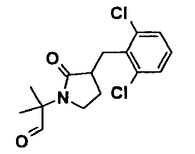

To a mixture of oxalyl chloride (1.96 mL) and dichloromethane (100 mL) was added dimethyl sulfoxide (1.54 mL) at -78°C, and the mixture was stirred for 5 min. A solution of 3-(2,6-dichlorobenzyl)-1-(2-hydroxy-1,1-dimethylethyl)pyrrolidin-2-one obtained in Reference Example 43A (6.20 g) in dichloromethane (20 mL) was added dropwise to the mixture, and the mixture was stirred at -78°C for 20 min. Triethylamine (13.8 mL) was added to the reaction solution, and the mixture was allowed to warm to room temperature, washed successively with water and saturated brine, and dried over magnesium sulfate. The solvent was evaporated under reduced pressure, and the residue was subjected to silica gel column chromatography (hexane-ethyl acetate 4:1) to give the title compound (5.40 g) as colorless plate crystals.
1H NMR (300 MHz, CDCl₃) δ ppm 1.31 (s, 3 H), 1.33 (s, 3 H), 1.94-2.14 (m, 2 H), 2.87-3.16 (m, 2 H), 3.24-3.37 (m, 1 H), 3.39-3.51 (m, 2 H), 7.04-7.17 (m, 1 H), 7.28-7.35 (m, 2 H), 9.55 (s, 1 H).

### Reference Example 45A ethyl (2E)-4-[3-(2,6-dichlorobenzyl)-2-oxopyrrolidin-1-yl]-4-methylpenta-2-enoate

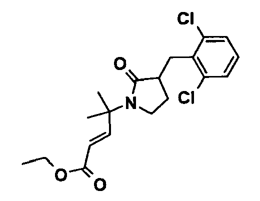

In the same manner as in Example 516A, the title compound was obtained from 2-[3-(2,6-dichlorobenzyl)-2-oxopyrrolidin-1-yl]-2-methylpropanal obtained in Reference Example 44A and (carboethoxymethyl)triphenylphosphine bromide.
1H NMR (300 MHz, CDCl₃) δ ppm 1.30 (t, J=7.1 Hz, 3 H), 1.53 (d, J=2.1 Hz, 6 H), 1.77-1.99 (m, 2 H), 2.78-2.97 (m, 1 H), 2.97-3.12 (m, 1 H), 3.21-3.35 (m, 1 H), 3.36-3.54 (m, 2 H), 4.20 (q, J=7.2 Hz, 2 H), 5.83 (d, J=15.8 Hz, 1 H), 7.02-7.16 (m, 2 H), 7.29 (d, J=7.9 Hz, 2 H).

### Reference Example 46A ethyl 4-[3-(2,6-dichlorobenzyl)-2-oxopyrrolidin-1-yl]-4-methylpentanoate

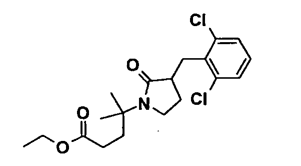

In the same manner as in Example 527A, the title compound was obtained from ethyl (2E)-4-[3-(2,6-dichlorobenzyl)-2-oxopyrrolidin-1-yl]-4-methylpenta-2-enoate obtained in Reference Example 45A.
1H NMR (300 MHz, CDCl₃) δ ppm 1.26 (t, J=7.2 Hz, 3 H), 1.36-1.41 (m, 6 H), 1.75-1.95 (m, 2 H), 2.15-2.38 (m, 4 H), 2.77-2.93 (m, 1 H), 2.95-3.08 (m, 1 H), 3.17-3.32 (m, 1 H), 3.36-3.51 (m, 2 H), 4.13 (q, J=7.2 Hz, 2 H), 6.97-7.15 (m, 1 H), 7.27-7.33 (m, 2 H).

### Reference Example 47A 4-[3-(2,6-dichlorobenzyl)-2-oxopyrrolidin-1-yl]-4-methylpentanoic acid

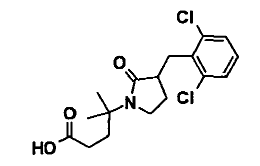

In the same manner as in Example 155A, the title compound was obtained from ethyl 4-[3-(2,6-dichlorobenzyl)-2-oxopyrrolidin-1-yl]-4-methylpentanoate obtained in Reference Example 46A.
1H NMR (300 MHz, CDCl₃) δ ppm 1.38 (s, 6 H), 1.77-1.97 (m, 2 H), 2.20-2.44 (m, 4 H), 2.81-2.96 (m, 1 H), 2.97-3.10 (m, 1 H), 3.20-3.35 (m, 1 H), 3.38-3.52 (m, 2 H), 7.04-7.15 (m, 1 H), 7.27-7.32 (m, 2 H).

### Reference Example 48A 3-(4-bromomethyl-3-chlorophenyl)-5-cyclopropyl-1,2,4-oxadiazol

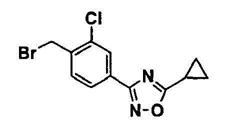

To a solution of cyclopropanecarboxylic acid (1.17 g) in dichloromethane (60 mL) was added 1,1'-carbonyldiimidazole (2.24 g), and the mixture was stirred at room temperature for 5 hr. 3-Chloro-4-methylbenzamidoxime (2.51 g) was added to the mixture, and the reaction mixture was stirred at room temperature for 18 hr, and concentrated under reduced pressure. The residue was suspended in toluene (100 mL), and the suspension was heated under reflux for 21 hr. After cooling, water was added to the reaction mixture, and the mixture was extracted with ethyl acetate. The organic layer was washed with saturated brine, and dried over anhydrous magnesium sulfate.

The solvent was evaporated under reduced pressure, the residue was subjected to silica gel column chromatography (hexane-ethyl acetate 95:5-9:1), and the obtained crystals were recrystallized from hexane to give 3-(3-chloro-4-methylphenyl)-5-cyclopropyl-1,2,4-oxadiazol (2.12 g, 66%) as colorless crystals.
1H NMR (300 MHz, CDCl₃) δ ppm 1.22-1.34 (m, 4 H), 2.21-2.29 (m, 1 H), 2.42 (s, 3 H), 7.31 (d, J=7.8 Hz, 1 H), 7.81 (dd, J=7.8, 1.8 Hz, 1 H), 8.02 (d, J=1.8 Hz, 1 H).

A mixture of the obtained 3-(3-chloro-4-methylphenyl)-5-cyclopropyl-1,2,4-oxadiazol (1.50 g), N-bromosuccinimide (1.15 g), α,α'-azobisisobutyronitrile (0.12 g) and carbon tetrachloride (10 mL) was heated under reflux for 1.5 hr. α,α'-Azobisisobutyronitrile (0.12 g) was added, and the mixture was further heated under reflux for 1.5 hr. After cooling, the reaction mixture was concentrated under reduced pressure, and residue was partitioned between ethyl acetate and water. The aqueous layer was extracted again with ethyl acetate, and the organic layers were combined, washed with saturated brine, and dried over anhydrous magnesium sulfate. The solvent was evaporated under reduced pressure, and the residue was subjected to silica gel column chromatography (hexane-ethyl acetate 97:3-9:1) to give the title compound (1.34 g, 66%) as a white solid.
1H NMR (300 MHz, CDCl₃) δ ppm 1.23-1.35 (m, 4 H), 2.21-2.30 (m, 1 H), 4.61 (s, 2 H), 7.53 (d, J=7.8 Hz, 1 H), 7.92 (dd, J=7.8, 1.5 Hz, 1 H), 8.08 (d, J=1.5 Hz, 1 H).

### Reference Example 1B 1-(2-methylbenzyl)imidazolidin-2-one

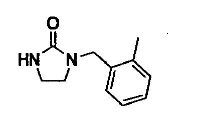

To a mixture of ethyleneurea (10 g), α-bromo-o-xylene (10.7 g) and N,N-dimethylformamide (150 mL) was added 60% sodium hydride (4.65 g), and the mixture was stirred at room temperature for 1 hr. Water was added to the reaction solution, and the mixture was extracted with ethyl acetate. The ethyl acetate layer was washed successively with water and saturated brine, and dried over anhydrous magnesium sulfate, and the solvent was evaporated under reduced pressure. The residue was subjected to silica gel column chromatography to give the title compound (3.43 g, 32%) as a colorless solid from a fraction eluted with ethyl acetate.
1H NMR (300 MHz, CDCl₃) δ ppm 2.34 (s, 3 H), 3.17-3.33 (m, 2 H), 3.35-3.49 (m, 2 H), 4.38 (s, 2 H), 4.81 (s, 1 H), 7.09-7.24 (m, 4 H).

### Reference Example 2B 3-(2-methylbenzyl)imidazolidine-2,4-dione

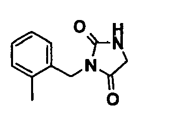

A mixture of hydantoin (5.0 g), α-bromo-o-xylene (9.25 g), potassium carbonate (6.91 g) and acetone-water (5:2) was heated under reflux for 5 hr. The reaction solution was concentrated under reduced pressure, and the residue was partitioned between ethyl acetate-water. The ethyl acetate layer was washed with saturated brine, and dried over anhydrous magnesium sulfate. The solvent was evaporated under reduced pressure, and the obtained colorless solid was recrystallized from ethyl acetate-hexane to give the title compound (6.95 g, 68%) as a colorless solid.
LC/MS (ESI+); m/z 205 (M+H)⁺
Reference Example 3B 1-(morpholin-4-yl)imidazolidin-2-one

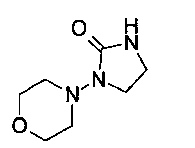

To a solution (240 mL) of morpholin-4-amine (6.12 g) in tetrahydrofuran was added dropwise 1-chloro-2-isocyanatoethane (6.65 g) at room temperature, and the mixture was stirred at room temperature 30 min. Then, potassium tert-butoxide (8.75 g) was added to the reaction mixture, and the mixture was stirred at room temperature for 30 min. Water was added to the reaction mixture, and the mixture was extracted with ethyl acetate. The ethyl acetate layer was washed successively with water and saturated brine, and dried over anhydrous magnesium sulfate, and the solvent was evaporated under reduced pressure. The residue was recrystallized from ethyl acetate-hexane solvent to give the title compound (3.2 g, 31%) as a colorless solid.
1H NMR (300 MHz, CDCl₃) δ ppm 2.29-2.95 (m, 4 H), 3.37-3.42 (m, 2 H), 3.47-3.52 (m, 2 H), 3.81 -3.84 (m, 4 H), 5.53 (m, 1 H). Reference Example 4B 1-(2,3-dihydro-1H-inden-1-yl)imidazolidin-2-one

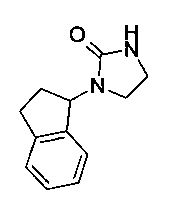

In the same manner as in Reference Example 3B, the title compound was obtained from indan-1-amine and 1-chloro-2-isocyanatoethane.
1H NMR (300 MHz, CDCl₃) δ ppm 1.94-2.03 (m, 1 H), 2.33-2.44 (m, 1 H), 2.86-3.02 (m, 2 H), 3.06-3.14 (m, 1 H), 3.24-3.32 (m, 1 H), 3.41-3.44 (m, 2 H), 4.79 (m, 1 H), 5.57-5.62 (t, J=7.5 Hz, 1 H), 7.21-7.26 (m, 4 H).

### Reference Example 5B 1-[(1R,2R,4R)-bicyclo[2.2.1]hept-2-yl]imidazolidin-2-one

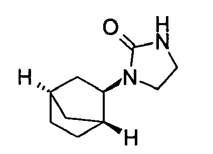

In the same manner as in Reference Example 3B, the title compound was obtained from [(1R,2R,4R)-bicyclo[2.2.1]heptan-2-amine and 1-chloro-2-isocyanatoethane.
1H NMR (300 MHz, CDCl₃) δ ppm 1.12-1.21 (m, 3 H), 1.32-1.49 (m, 5 H), 2.22 (m, 1 H), 2.30 (m, 1 H), 3.33-3.49 (m, 4 H), 3.81-3.86 (m, 1 H), 4.34 (m, 1 H).

### Reference Example 6B 1-(2,3-dihydro-1H-indol-1-yl)imidazolidin-2-one

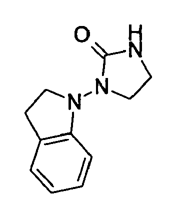

In the same manner as in Reference Example 3B, the title compound was obtained from indolin-1-amine and 1-chloro-2-isocyanatoethane.
1H NMR (300 MHz, CDCl₃) δ ppm 3.02-3.08 (t, J=8.1 Hz, 2 H), 3.50 (m, 4 H), 3.57 (m, 2 H), 5.57 (br, 1H), 6.63-6.66 (m, 1 H), 6.80 -6.86 (m, 1 H), 7.09-7.14 (m, 2 H).

Reference Example 7B 1-[1-(1-adamantyl)ethyl]imidazolidin-2-one

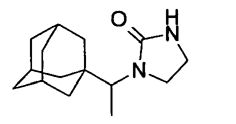

In the same manner as in Reference Example 3B, the title compound was obtained from 1-(1-adamantyl)ethanamine and 1-chloro-2-isocyanatoethane.
1H NMR (300 MHz, CDCl₃) δ ppm 1.06-1.09 (d, J=7.5 Hz, 3 H), 1.54-1. 72 (m, 12 H), 1.98 (m, 3 H), 3.33 -3.53 (m, 4 H), 3.57-3.62 (m, 1 H), 4.41 (br, 1 H).

### Reference Example 1C ethyl 3-(2,6-dichlorobenzyl)-2-oxopiperidine-3-carboxylate

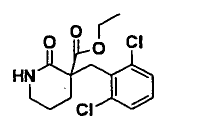

A mixture of ethyl 2-oxo-3-piperidinecarboxylate (3.42 g, 0.02 mol), 20% ethanol solution (6.81 mL, 0.02 mol) of sodium ethylate and ethanol (20 mL) was stirred at room temperature for 5 min. 2,6-Dichlorobenzyl bromide (4.80 g, 0.02 mol) was added, and the mixture was stirred at 70°C for 2 hr. The reaction mixture was poured into water, and the mixture was extracted with ethyl acetate. The ethyl acetate layer was washed with saturated brine, and dried over anhydrous magnesium sulfate.

The solvent was evaporated under reduced pressure, and isopropyl ether-hexane solution was added to the residue. The resulting crystals were collected by filtration, and washed with hexane to give the title compound (4.60 g, 70%) as a white powder.
1H NMR (300 MHz, CDCl₃) δ ppm 1.24 (t, J=7.2Hz, 3 H), 1.60-1.75 (m, 2 H), 1.85-1.94 (m, 1 H), 2.16-2.24 (m, 1 H), 3.25-3.33 (m, 2 H), 3.60 (d, J=14.8 Hz, 1 H), 4.21 (d, J=14.8 Hz, 1 H), 4.15-4.28 (m, 2 H), 5.91 (brs, 1 H), 7.11 (t, J=8.0 Hz, 1 H), 7.29 (d, J=8.0 Hz, 2 H).

### Reference Example 2C 3-(2,6-dichlorobenzyl)piperidin-2-one

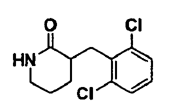

A mixture of ethyl 3-(2,6-dichlorobenzyl)-2-oxopiperidine-3-carboxylate obtained in Reference Example 1C (4.50 g, 0.014 mol), 1N aqueous sodium hydroxide solution (20 mL) and ethanol (20 mL) was stirred at room temperature for 10 hr. The reaction mixture was concentrated, and 1N hydrochloric acid (22 mL) was added to the residue. The resulting precipitate was collected by filtration, wash successively with water and hexane, and dried. The obtained white powder was heated at 130°C for 30 min, and isopropyl ether-hexane solution was added, and the resulting precipitate was collected by filtration, washed with hexane, and dried to give the title compound (1.52 g, 76%) as a white powder.
1H NMR (300 MHz, CDCl₃) δ ppm 1.55-1.70 (m, 3 H), 1.85-1.95 (m, 1 H), 2. 75-2. 85 (m, 1 H), 3. 10 (dd, J=13.8, 11.6 Hz, 1 H), 3.25-3.35 (m, 2 H), 3.71 (dd, J=13.8, 4.4 Hz, 1H), 5.75 (brs, 1H), 7.10 (t, J=8.0 Hz, 1 H), 7.30 (d, J=8.0 Hz, 2 H).

### Reference Example 1D 1-(2-methylbenzyl)tetrahydropyrimidin-2(1H)-one

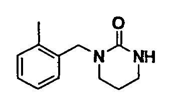

To a mixture of tetrahydro-2-pyrimidinone (11.6 g), α-bromo-o-xylene (10.7 g) and N,N-dimethylformamide (150 mL) was added sodium hydride (4.65 g), and the mixture was stirred at room temperature for 1 hr. Water was added to the reaction solution, and the mixture was extracted with ethyl acetate. The ethyl acetate layer was washed successively with water and saturated brine, and dried over anhydrous magnesium sulfate, and the solvent was evaporated under reduced pressure. The residue was subjected to silica gel column chromatography (ethyl acetate-ethyl acetate-methanol 20:1) to give the title compound (1.58 g, 13%) as a colorless solid.
1H NMR (300 MHz, CDCl₃) δ ppm 1.84-1.97 (m, 2 H), 2.31 (s, 3 H), 3.05-3.18 (m, 2 H), 3.25-3.47 (m, 2 H), 4.58 (s, 2 H), 5.12 (s, 1 H), 7.09-7.22 (m, 4 H).

Example 1A 3-(2,6-dichlorobenzyl)-1-(2-methylbenzyl)pyrrolidin-2-one

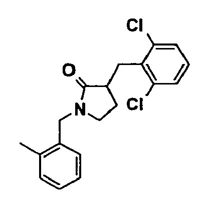

To a mixture of 1-(2-methylbenzyl)pyrrolidin-2-one obtained in Reference Example 1A (0.50 g) and tetrahydrofuran (10 mL) was added lithium diisopropylamide (tetrahydrofuran solution, 2 M, 1.32 mL) at -78°C under nitrogen atmosphere, and the mixture was stirred for 10 min. A solution of α,2,6-trichlorotoluene (0.52 g) in tetrahydrofuran (5 mL) was added to the obtained solution, and the mixture was further stirred at -78°C for 10 min, and allowed to warm to room temperature. 10% Aqueous ammonium chloride was added to the reaction solution, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 9:1-4:1) to give the title compound (0.74 g, 80%) as a colorless solid.
1H NMR (300 MHz, CDCl₃) δ ppm 1.83-1.98 (m, 2 H), 2.33 (s, 3 H), 2.91-3.12 (m, 3 H), 3.13-3.30 (m, 1 H), 3.43-3.63 (m, 1 H), 4.51 (s, 2 H), 7.04-7.14 (m, 1 H), 7.14-7.23 (m, 4 H), 7.27-7.35 (m, 2 H).

### Example 2A 1-benzyl-3-(2,6-dichlorobenzyl)pyrrolidin-2-one

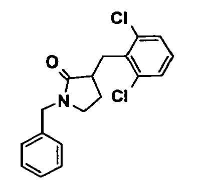

In the same manner as in Example 1A, the title compound was obtained from 1-benzylpyrrolidin-2-one and α,2,6-trichlorotoluene.
1H NMR (300 MHz, CDCl₃) δ ppm 1.83-2.03 (m, 2 H), 2.91-3.33 (m, 4 H), 3.54 (dd, J=13.0, 4.1 Hz, 1 H), 4.38-4.60 (m, 2 H), 7.02-7.18 (m, 1 H), 7.20-7.46 (m, 7 H).

### Example 3A 3,3-bis(2,6-dichlorobenzyl)-1-[(1R)-1-phenylethyl]pyrrolidin-2-one

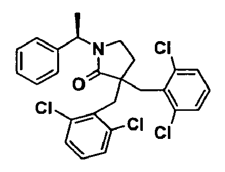

In the same manner as in Example 1A, the title compound was obtained from 1-[(1R)-1-phenylethyl]pyrrolidin-2-one and α,2,6-trichlorotoluene.
LC-MS (ESI+);m/z 508 (M+H)⁺

### Example 4A 3-(2-methylbenzyl)-1-(1-methyl-1-phenylethyl)pyrrolidin-2-one

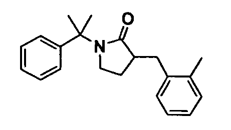

In the same manner as in Example 1A, the title compound was obtained from 1-(1-methyl-1-phenylethyl)pyrrolidin-2-one obtained in Reference Example 3A and α-bromo-o-xylene.
1H NMR (300 MHz, CDCl₃) δ ppm 1.60-1.72 (m, 1 H), 1.75 (s, 3 H), 1.78 (s, 3 H), 1.91-2.04 (m, 1 H), 2.32 (s, 3 H), 2.47-2.60 (m, 1 H), 2.61-2.75 (m, 1 H), 3.23-3.37 (m, 3 H), 7.06-7.17 (m, 4 H), 7.19-7.27 (m, 1 H), 7.28-7.41 (m, 4 H).

Example 5A 3-(2,6-dichlorobenzyl)-1-(1-methyl-1-phenylethyl)pyrrolidin-2-one

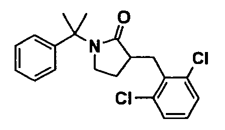

In the same manner as in Example 1A, the title compound was obtained from 1-(1-methyl-1-phenylethyl)pyrrolidin-2-one obtained in Reference Example 3A and α,2,6-trichlorotoluene.
1H NMR (300 MHz, CDCl₃) δ ppm 1.77 (s, 3 H), 1.80 (s, 3 H), 1.82-1.93 (m, 2 H), 2.82-2.97 (m, 1 H), 2.99-3.12 (m, 1 H), 3.18-3.29 (m, 1 H), 3.30-3.40 (m, 1 H), 3.45 (dd, J=13.4, 4.1 Hz, 1 H), 7.03-7.14 (m, 1 H), 7.19-7.28 (m, 2 H), 7.28-7.41 (m, 5 H).

Example 6A 1-(cyclohexylmethyl)-3-(2-methylbenzyl)pyrrolidin-2-one

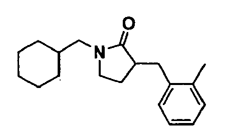

In the same manner as in Example 1A, the title compound was obtained from 1-(cyclohexylmethyl)pyrrolidin-2-one and α-bromo-o-xylene.
1H NMR (300 MHz, CDCl₃) δ ppm 0.76-1.08 (m, 2 H), 1.07-1.29 (m, 2 H), 1.54-1.82 (m, 4 H), 1.79-2.03 (m, 2 H), 2.25-2.32 (m, 3 H), 2.39-2.70 (m, 2 H), 2.99-3.37 (m, 3 H), 3.59-3.84 (m, 2 H), 4.57 (d like, 2 H), 6.91-7.09 (m, 2 H), 7.09-7.25 (m, 3 H).

### Example 7A 1-(cyclohexylmethyl)-3-(2,6-dichlorobenzyl)pyrrolidin-2-one

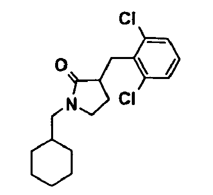

In the same manner as in Example 1A, the title compound was obtained from 1-(cyclohexylmethyl)pyrrolidin-2-one and α,2,6-trichlorotoluene.
1H NMR (300 MHz, CDCl₃) δ ppm 0.73-0.99 (m, 2 H), 1.03-1.31 (m, 2 H), 1.43-1.82 (m, 6 H), 1.86-2.04 (m, 2 H), 2.44-2.82 (m, 2 H), 2.90-3.09 (m, 2 H), 3.70 (s, 2 H), 4.74-5.07 (m, 2 H), 7.11-7.24 (m, 1 H), 7.29-7.43 (m, 2 H).

Example 8A 1-cyclohexyl-3-(2-methylbenzyl)pyrrolidin-2-one

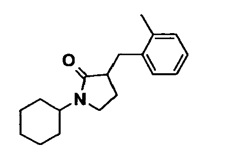

In the same manner as in Example 1A, the title compound was obtained from 1-cyclohexylpyrrolidin-2-one and α-bromo-o-xylene.
1H NMR (300 MHz, CDCl₃) δ ppm 0.97-1.19 (m, 1 H), 1.28-1.52 (m, 4 H), 1.56-1.89 (m, 6 H), 1.90-2.12 (m, 1 H), 2.34 (s, 3 H), 2.44-2.60 (m, 1 H), 2.62-2.82 (m, 1 H), 3.09-3.29 (m, 2 H), 3.27-3.43 (m, 1 H), 3.86-4.05 (m, 1 H), 7.02-7.21 (m, 4 H).

Example 9A 1-cyclohexyl-3-(2,6-dichlorobenzyl)pyrrolidin-2-one

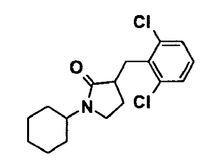

In the same manner as in Example 1A, the title compound was obtained from 1-cyclohexylpyrrolidin-2-one and α,2,6-trichlorotoluene.
1H NMR (300 MHz, CDCl₃) δ ppm 0.92-1.23 (m, 1 H), 1.24-1.52 (m, 4 H), 1.61-2.01 (m, 7 H), 2.84-2.98 (m, 1 H), 2.98-3.11 (m, 1 H), 3.11-3.27 (m, 1 H), 3.26-3.40 (m, 1 H), 3.49 (dd, J=13.2, 4.3 Hz, 1 H), 3.80-4.13 (m, 1 H), 7.00-7.19 (m, 1 H), 7.27-7.36 (m, 2 H).

### Example 10A 1-(2,6-dichlorobenzyl)-3-(2-methylbenzyl)pyrrolidin-2-one

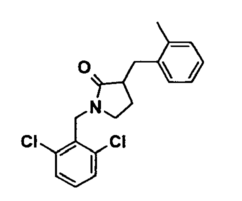

In the same manner as in Example 1A, the title compound was obtained from 1-(2,6-dichlorobenzyl)pyrrolidin-2-one obtained in Reference Example 2A and α-bromo-o-xylene.
1H NMR (300 MHz, CDCl₃) δ ppm 1.55-1.76 (m, 1 H), 1.89-2.03 (m, 1 H), 2.34 (s, 3 H), 2.49-2.66 (m, 1 H), 2.67-2.81 (m, 1 H), 2.96-3.11 (m, 2 H), 3.38 (dd, J=14.1, 3.8 Hz, 1 H), 4.73-4.97 (m, 2 H), 7.05-7.24 (m, 5 H), 7.30-7.39 (m, 2 H).

### Example 11A 3-(2-methylbenzyl)-1-[(1R)-1-phenylethyl]pyrrolidin-2-one (less polar product)

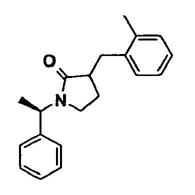

In the same manner as in Example 1A, the title compound was obtained from 1-[(1R)-1-phenylethyl]pyrrolidin-2-one and α-bromo-o-xylene.
1H NMR (300 MHz, CDCl₃) δ ppm 1.53 (d, J=7.2 Hz, 3 H), 1.59-1.80 (m, 1 H), 1.85-2.05 (m, 1 H), 2.35 (s, 3 H), 2.51-2.65 (m, 1 H), 2.65-2.76 (m, 1 H), 2.76-2.90 (m, 1 H), 3.10-3.28 (m, 1 H), 3.37 (dd, J=13.9, 3.6 Hz, 1 H), 5.54 (q, J=7.2 Hz, 1 H), 7.03-7.20 (m, 4 H), 7.21-7.44 (m, 5 H).

### Example 12A 3-(2-methylbenzyl)-1-[(IR)-1-phenylethyl]pyrrolidin-2-one (more polar product)

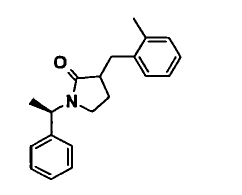

In the same manner as in Example 1A, the title compound was obtained from 1-[(1R)-1-phenylethyl]pyrrolidin-2-one and α-bromo-o-xylene.
1H NMR (300 MHz, CDCl₃) δ ppm 1.53 (d, J=7.2 Hz, 3 H), 1.55-1.62 (m, 1 H), 1.90-2.11 (m, 1 H), 2.33 (s, 3 H), 2.54 (dd, J=14.1, 10.4 Hz, 1 H), 2.66-2.82 (m, 1 H), 2.86-2.98 (m, 1 H), 3.11-3.27 (m, 1 H), 3.38 (dd, J=14.1, 3.8 Hz, 1 H), 5.52 (q, J=7.2 Hz, 1 H), 7.05-7.20 (m, 4 H), 7.22-7.42 (m, 5 H).

### Example 13A 3-(2,6-dichlorobenzyl)-1-[(1S)-1-phenylethyl]pyrrolidin-2-one (less polar product)

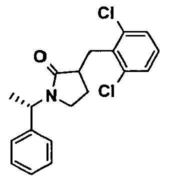

In the same manner as in Example 1A, the title compound was obtained from 1-[(1S)-1-phenylethyl]pyrrolidin-2-one and α,2,6-trichlorotoluene.
1H NMR (300 MHz, CDCl₃) δ ppm 1. 57 (d, J=7.2 Hz, 3 H), 1.81-1.94 (m, 2 H), 2.78-2.90 (m, 1 H), 2.89-3.01 (m, 1 H), 3.02-3.15 (m, 1 H), 3. 25-3. 38 (m, 1 H), 3. 51 (dd, J=13. 4, 4.7 Hz, 1 H), 5. 54 (q, J=7.2 Hz, 1 H), 6.98-7.16 (m, 1 H), 7.19-7.41 (m, 7 H).

### Example 14A 3-(2,6-dichlorobenzyl)-1-[(1S)-1-phenylethyl]pyrrolidin-2-one (more polar product)

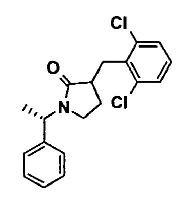

In the same manner as in Example 1A, the title compound was obtained from 1-[(1S)-1-phenylethyl]pyrrolidin-2-one and α,2,6-trichlorotoluene.
1H NMR (300 MHz, CDCl₃)δ ppm 1.52 (d, J=7.2 Hz, 3 H), 1.70-2.00 (m, 2 H), 2.90-3.11 (m, 3 H), 3.12-3.24 (m, 1 H), 3.50-3.59 (m, 1 H), 5.51 (q, J=7.2 Hz, 1 H), 7.05-7.13 (m, 1 H), 7.22-7.41 (m, 7 H).

### Example 15A 3-[(2,6-dichlorophenyl) (hydroxy)methyl]-1-[(IR)-1-phenylethyl]pyrrolidin-2-one

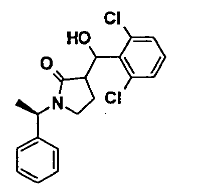

In the same manner as in Example 1A, the title compound was obtained from 1-[(1R)-1-phenylethyl]pyrrolidin-2-one and 2,6-dichlorobenzaldehyde.
1H NMR (300 MHz, CDCl₃) δ ppm 1.52-1.63 (m, 3 H), 1.62-1.87 (m, 2 H), 2.79-3.08 (m, 1 H), 3.19-3.41 (m, 1 H), 3.56-3.78 (m, 1 H), 5.28-5.45 (m, 1 H), 5.46-5.61 (m, 1 H), 5.60-5.78 (m, 1 H), 7.09-7.20 (m, 1 H), 7.27-7.44 (m, 7 H).

### Example 16A 3-(2,6-dichlorobenzyl)-1-[(1R)-1-phenylethyl]pyrrolidin-2-one (less polar product)

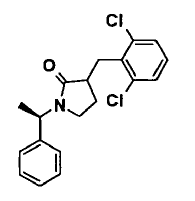

In the same manner as in Example 1A, the title compound was obtained from 1-[(1R)-1-phenylethyl]pyrrolidin-2-one and α,2,6-trichlorotoluene.
1H NMR (300 MHz, CDCl₃) δ ppm 1.57 (d, J=7.2 Hz, 3 H), 1.80-1.93 (m, 2 H), 2.76-2.89 (m, 1 H), 2.89-3.01 (m, 1 H), 3.03-3.16 (m, 1 H), 3.27-3.37 (m, 1 H), 3.51 (dd, J=13.4, 4.7 Hz, 1 H), 5.54 (q, J=7.2 Hz, 1 H), 7.05-7.15 (m, 1 H), 7.21-7.39 (m, 7 H).

### Example 17A 3-(2,6-dichlorobenzyl)-1-[(1R)-1-phenylethyl]pyrrolidin-2-one (more polar product)

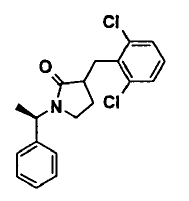

In the same manner as in Example 1A, the title compound was obtained from 1-[(1R)-1-phenylethyl]pyrrolidin-2-one and α,2,6-trichlorotoluene.
1H NMR (300 MHz, CDCl₃) δ ppm 1.52 (d, J=7.2 Hz, 3 H), 1.73-1.86 (m, 1 H), 1.85-1.99 (m, 1 H), 2.89-3.11 (m, 3 H), 3.11-3.26 (m, 1 H), 3.48-3.60 (m, 1 H), 5.52 (q, J=7.2 Hz, 1 H), 7.04-7.14 (m, 1 H), 7.23-7.44 (m, 7 H).

### Example 18A tert-butyl (methyl)[4-({2-oxo-1-[(1R)-1-phenylethyl]pyrrolidin-3-yl}methyl)phenyl]carbamate (less polar product)

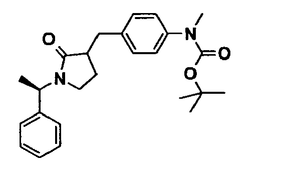

In the same manner as in Example 1A, the title compound was obtained from 1-[(1R)-1-phenylethyl]pyrrolidin-2-one and tert-butyl [4-(bromomethyl)phenyl](methyl)carbamate obtained in Reference Example 12A.
1H NMR (300 MHz, CDCl₃) δ ppm 1.44 (s, 9 H), 1.43-1.49 (m, 3 H), 1.70 (d, J=7.9 Hz, 1 H), 1.83-2.02 (m, 1 H), 2.63-2.87 (m, 3 H), 2.97-3.13 (m, 1 H), 3.13-3.24 (m, 1 H), 3.24 (s, 3 H), 5.51 (q, J=6.8 Hz, 1 H), 7.08-7.23 (m, 4 H), 7.21-7.40 (m, 5 H).

### Example 19A tert-butyl (methyl)[4-({2-oxo-1-[(1R)-1-phenylethyl]pyrrolidin-3-yl}methyl)phenyl]carbamate (more polar product)

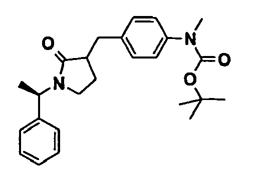

In the same manner as in Example 1A, the title compound was obtained from 1-[(1R)-1-phenylethyl]pyrrolidin-2-one and tert-butyl [4-(bromomethyl)phenyl](methyl)carbamate obtained in Reference Example 12A.
1H NMR (300 MHz, CDCl₃) δ ppm 1.44 (s, 9 H), 1.47-1.69 (m, 6 H), 1.89-2.11 (m, 1 H), 2.55-2.89 (m, 2 H), 3.08-3.27 (m, 1 H), 3.23 (s, 3 H), 5.50 (q, J=6.7 Hz, 1 H), 7.01-7.20 (m, 3 H), 7.17-7.43 (m, 6 H).

### Example 20A 3-[4-(methylamino)benzyl]-1-[(1R)-1-phenylethyl]pyrrolidin-2-one (less polar product)

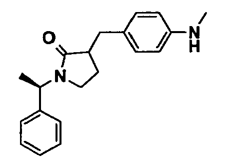

A mixture of tert-butyl (methyl) [4-({2-oxo-1-[(1R)-1-phenylethyl]pyrrolidin-3-yl}methyl)phenyl]carbamate obtained in Example 18A (0.47 g), 4N hydrogen chloride-ethyl acetate (2 mL) and ethyl acetate (3 mL) was stirred at room temperature for 3 hr. Saturated aqueous sodium hydrogencarbonate was added to the reaction solution, and the mixture was extracted with ethyl acetate. The extract was dried over anhydrous magnesium sulfate, and concentrated under reduced pressure. The residue was subjected to silica gel column chromatography (hexane-ethyl acetate 9:1-2:3) to give the title compound (0.28 g, 79%) as a pale-yellow oil.
1H NMR (300 MHz, CDCl₃) δ ppm 1.45 (d, J=7.2 Hz, 3 H), 1.64-1.77 (m, 1 H), 1.84-2.02 (m, 1 H), 2.60-2.71 (m, 2 H), 2.72-2.80 (m, 1 H), 2.82 (s, 3 H), 2.97-3.14 (m, 2 H), 3.63 (s, 1H), 5.51 (q, J=7.2 Hz, 1 H), 6.49-6.60 (m, 2 H), 6.99-7.10 (m, 2 H), 7.21-7.39 (m, 5 H).

Example 21A 3-[4-(methylamino)benzyl]-1-[(1R)-1-phenylethyl]pyrrolidin-2-one (more polar product)

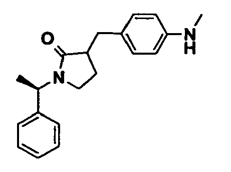

In the same manner as in Example 20A, the title compound was obtained from tert-butyl (methyl)[4-({2-oxo-1-[(1R)-1-phenylethyl]pyrrolidin-3-yl}methyl)phenyl]carbamate obtained in Example 19A.
1H NMR (300 MHz, CDCl₃) δ ppm 1.50 (d, J=7.2 Hz, 3 H), 1.54-1.72 (m, 1 H), 1.89-2.10 (m, 1 H), 2.52-2.65 (m, 1 H), 2.65-2.87 (m, 2 H), 2.82 (s, 3 H), 3.04-3.21 (m, 2 H), 3.61 (s, 1 H), 5.49 (q, J=7.2 Hz, 1 H), 6.46-6.58 (m, 2 H), 6.97-7.05(m, 2 H), 7.18-7.37 (m, 5 H).

### Example 22A N-methyl-N-[4-({2-oxo-1-[(1R)-1-phenylethyl]pyrrolidin-3-yl}methyl)phenyl]acetamide (less polar product)

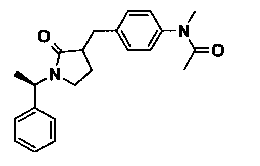

A mixture of 3-(4-(methylamino)benzyl)-1-[(1R)-1-phenylethyl]pyrrolidin-2-one obtained in Example 20A (0.25 g), acetyl chloride (0.07 g), triethylamine (0.12 mL) and tetrahydrofuran (8 mL) was stirred at room temperature for 1 hr. Saturated aqueous sodium hydrogencarbonate was added to the reaction solution, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 1:1-ethyl acetate) to give the title compound (0.20 g, 70%) as a pale-yellow amorphous form.
1H NMR (300 MHz, CDCl₃)δ ppm 1.47 (d, J=7.2 Hz, 3 H), 1.60-1.75 (m, 1 H), 1.86 (s, 3 H), 1.93-2.13 (m, 1 H), 2.65-2.90 (m, 3 H), 2.96-3.16 (m, 1 H), 3.16-3.32 (m, 1 H), 3.24 (s, 3 H), 5.51 (q, J=7.2 Hz, 1 H), 7.10 (d, J=8.3 Hz, 2H), 7.20-7.43 (m, 7 H). Example 23A N-methyl-N-[4-({2-oxo-1-[(1R)-1-phenylethyl]pyrrolidin-3-yl}methyl)phenyl]acetamide (more polar product)

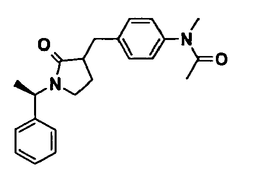

In the same manner as in Example 22A, the title compound was obtained from 3-[4-(methylamino)benzyl]-1-[(1R)-1-phenylethyl]pyrrolidin-2-one obtained in Example 21A.
1H NMR (300 MHz, CDCl₃) δ ppm 1.52 (d, J=7 .2 Hz, 3 H), 1. 53-1. 66 (m, 1 H), 1. 83 (s, 3 H), 1. 96-2. 12 (m, 1 H), 2. 69-2. 87 (m, 3 H), 3.12-3.29 (m, 2 H), 3.23 (s, 3 H), 5. 50 (q, J=7 . 2 Hz, 1 H), 7.05 (d, J=8.1 Hz, 2 H), 7.18-7.37 (m, 7 H).

### Example 24A 3-(2,6-dichlorobenzyl)-3-methyl-1-[(1R)-1-phenylethyl]pyrrolidin-2-one

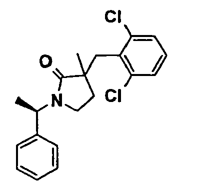

In the same manner as in Example 1A, the title compound was obtained from 3-(2,6-dichlorobenzyl)-1-[(1R)-1-phenylethyl]pyrrolidin-2-one and methyl iodide obtained in Example 16A.
LC-MS (ESI+); m/z 362 (M+H)⁺

### Example 25A 1-(4-methoxyphenyl)-3-(2-methylbenzyl)pyrrolidin-2-one

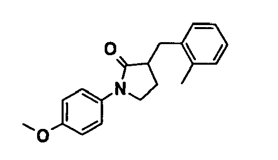

In the same manner as in Example 1A, the title compound was obtained from 1-(4-methoxyphenyl)pyrrolidin-2-one and α-bromo-o-xylene.
1H NMR (300 MHz, CDCl₃) δ ppm 1.77-1.94 (m, 1 H), 2.09-2.27 (m, 1 H), 2.37 (s, 3 H), 2.67 (dd, J=14.1, 10.4 Hz, 1 H), 2.83-2.99 (m, 1 H), 3.43 (dd, J=14.1, 3.8 Hz, 1 H), 3.66-3.75 (m, 2 H), 3.81 (s, 3 H), 6.87-6.96 (m, 2 H), 7.11-7.22 (m, 4 H), 7.49-7.59 (m, 2 H).

### Example 26A 3-(2,6-dichlorobenzyl)-1-(4-methoxyphenyl)pyrrolidin-2-one

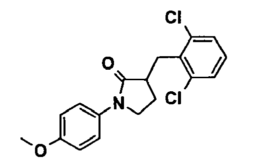

In the same manner as in Example 1A, the title compound was obtained from 1-(4-methoxyphenyl)pyrrolidin-2-one and α,2,6-trichlorotoluene.
1H NMR (300 MHz, CDCl₃) δ ppm 1.93-2.19 (m, 2 H), 3.00-3.28 (m, 2 H), 3.50-3.64 (m, 1 H), 3.64-3.93 (m, 2 H), 3.81 (s, 3 H), 6.84-6.99 (m, 2 H), 7.04-7.18 (m, 1 H), 7.32 (d, J=7.9 Hz, 2 H), 7.49-7.74 (m, 2 H).

### Example 27A 3-(2,6-dichlorobenzyl)-1-(1,4-dioxaspiro[4.5]dec-8-yl)pyrrolidin-2-one

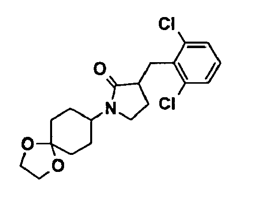

In the same manner as in Example 1A, the title compound was obtained from 1-(1,4-dioxaspiro[4.5]dec-8-yl)pyrrolidin-2-one and α,2,6-trichlorotoluene.
1H NMR (300 MHz, CDCl₃) δ ppm 1.64-2.02 (m, 10 H), 2.84-2.98 (m, 1 H), 2.98-3.10 (m, 1 H), 3.12-3.25 (m, 1 H), 3.27-3.42 (m, 1 H), 3.49 (dd, J=13.2, 4.3 Hz, 1 H), 3.95 (s, 4 H), 4.00-4.18 (m, 1 H), 7.04-7.16 (m, 1 H), 7.27-7.33 (m, 2 H).

### Example 28A 3-(2,6-dichlorobenzyl)-1-(4-oxocyclohexyl)pyrrolidin-2-one

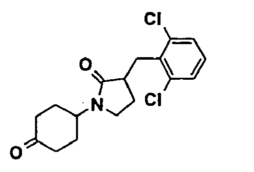

A mixture of 3-(2,6-dichlorobenzyl)-1-(1,4-dioxaspiro[4.5]dec-8-yl)pyrrolidin-2-one obtained in Example 27A (0.67 g), 2N hydrochloric acid (1.74 mL) and tetrahydrofuran (15 mL) was stirred at 60°C for 3 hr. Saturated aqueous sodium hydrogencarbonate was added to the reaction solution, and the mixture was extracted with ethyl acetate. The ethyl acetate layer was washed with saturated brine, and dried over anhydrous magnesium sulfate. The solvent was evaporated under reduced pressure. The residue was triturated with hexane-ethyl acetate to give the title compound (0.53 g, 90%) as a colorless powder.
1H NMR (300 MHz, CDCl₃) δ ppm 1.77-2.19 (m, 6 H), 2.37-2.66 (m, 4 H), 2.88-3.12 (m, 2 H), 3.12-3.26 (m, 1 H), 3.28-3.41 (m, 1 H), 3.50 (dd, J=13.0, 4.3 Hz, 1 H), 4.37-4.59 (m, 1 H), 7.05-7.16 (m, 1 H), 7.31 (d, J=7.9 Hz, 2 H).

### Example 29A 3-(2,6-dichlorobenzyl)-1-(4-hydroxycyclohexyl)pyrrolidin-2-one

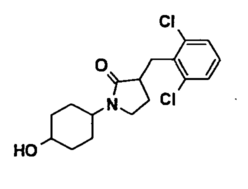

A mixture of 3-(2,6-dichlorobenzyl)-1-(4-oxocyclohexyl)pyrrolidin-2-one obtained in Example 28A (0.18 g), sodium borohydride (0.04 g) and methanol (5 mL) was stirred at room temperature for 1 hr. The reaction solution was concentrated under reduced pressure, and the residue was partitioned between water-ethyl acetate. The ethyl acetate layer was washed with saturated brine, and dried over anhydrous magnesium sulfate. The solvent was evaporated under reduced pressure, and the residue was subjected to silica gel column chromatography (ethyl acetate) to give the title compound (0.14 g, 77%) as a colorless powder.
LC-MS (ESI+); m/z 342 (M)⁺

### Example 30A 3-(2,6-dichlorobenzyl)-1-(4-hydroxy-4-methylcyclohexyl)pyrrolidin-2-one

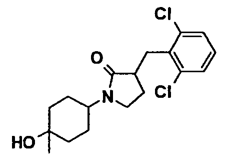

A mixture of 3-(2,6-dichlorobenzyl)-1-(4-oxocyclohexyl)pyrrolidin-2-one obtained in Example 28A (0.17 g), methylmagnesium bromide (3 M tetrahydrofuran solution, 0.33 mL) and tetrahydrofuran (10 mL) was stirred at room temperature for 1 hr. Saturated aqueous ammonium chloride was added to the reaction solution, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (ethyl acetate) to give the title compound (0.17 g, 95%) as a colorless powder.
LC-MS (ESI+); m/z 356 (M)⁺

### Example 31A 3-(2,6-dichlorobenzyl)-1-(4-hydroxycyclohexyl)pyrrolidin-2-one (retention time 21 min)

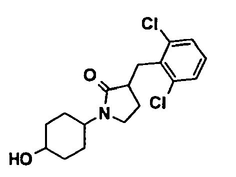

3-(2,6-Dichlorobenzyl)-1-(4-hydroxycyclohexyl)pyrrolidin-2-one obtained in Example 29A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.22-1.27 (m, 2 H), 1.52-1.97 (m, 8 H), 2.91- 3.09 (m, 2 H), 3.18-3.26 (m, 1 H), 3.35-3.52 (m, 2 H), 3.98-4.08 (m, 2 H), 7.07-7.12 (m, 1 H), 7.29 (d, J=7.8 Hz, 2 H).

### Example 32A 3-(2,6-dichlorobenzyl)-1-(4-hydroxycyclohexyl)pyrrolidin-2-one (retention time 27 min)

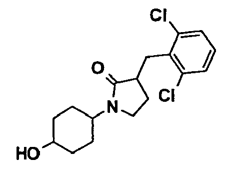

3-(2,6-Dichlorobenzyl)-1-(4-hydroxycyclohexyl)pyrrolidin-2-one obtained in Example 29A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.22-1.27 (m, 2 H), 1.37-2.07 (m, 8 H), 2.86-3.07 (m, 2 H), 3.13-3.21 (m, 1 H), 3.28-3.35 (m, 1 H), 3.45-3.60 (m, 2 H), 3.95-4.02 (m, 1 H), 7.07-7.12 (m, 1 H), 7.29 (d, J=7.8 Hz, 2 H).

### Example 33A 3-(2,6-dichlorobenzyl)-1-(4-hydroxycyclohexyl)pyrrolidin-2-one (retention time 38 min)

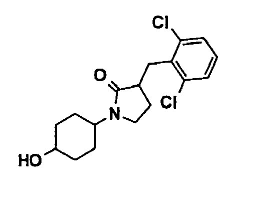

3-(2,6-Dichlorobenzyl)-1-(4-hydroxycyclohexyl)pyrrolidin-2-one obtained in Example 29A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.22-1.30 (m, 2 H), 1.40-2.07 (m, 8 H), 2.90-3.08 (m, 2 H), 3.13-3.21 (m, 1 H), 3.28-3.35 (m, 1 H), 3.45-3.59 (m, 2 H), 3.95-4.03 (m, 1 H), 7.07-7.13 (m, 1 H), 7.30 (d, J=8.1 Hz, 2 H).

### Example 34A 3-(2,6-dichlorobenzyl)-1-(4-hydroxycyclohexyl)pyrrolidin-2-one (retention time 45 min)

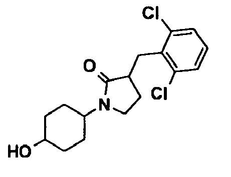

3-(2,6-Dichlorobenzyl)-1-(4-hydroxycyclohexyl)pyrrolidin-2-one obtained in Example 29A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.22-1.25 (m, 2 H), 1.43-1.97 (m, 8 H), 2.91-3.08 (m, 2 H), 3.18-3.26 (m, 1 H), 3.35-3.52 (m, 2 H), 3.97-4.08 (m, 2 H), 7.07-7.12 (m, 1 H), 7.29 (d, J=8.1 Hz, 2 H).

### Example 35A 3-(2,6-dichlorobenzyl)-1-(4-hydroxy-4-methylcyclohexyl)pyrrolidin-2-one (retention time 18 min)

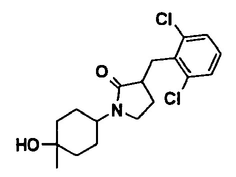

3-(2,6-Dichlorobenzyl)-1-(4-hydroxy-4-methylcyclohexyl)pyrrolidin-2-one obtained in Example 30A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.03 (s, 1 H), 1.25 (s, 3 H), 1.44-1.64 (m, 4 H), 1.64-2.11 (m, 6 H), 2.78-3.14 (m, 2 H), 3.13-3.29 (m, 1 H), 3.32-3.44 (m, 1 H), 3.49 (dd, J=13.2, 4.1 Hz, 1 H), 3.90-4.05 (m, 1 H), 7.00-7.18 (m, 1 H), 7.23-7.37 (m, 2 H).

### Example 36A 3-(2,6-dichlorobenzyl)-1-(4-hydroxy-4-methylcyclohexyl)pyrrolidin-2-one (retention time 31 min)

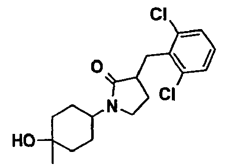

3-(2,6-Dichlorobenzyl)-1-(4-hydroxy-4-methylcyclohexyl)pyrrolidin-2-one obtained in Example 30A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.29 (s, 3 H), 1.44 (s, 1 H), 1.49-2.09 (m, 10 H), 2.80-3.12 (m, 2 H), 3.13-3.27 (m, 1 H), 3.28-3.41 (m, 1 H), 3.49 (dd, J=13.2, 4.3 Hz, 1 H), 3.86-4.10 (m, 1 H), 7.02-7.16 (m, 1 H), 7.30 (d, J=8.3 Hz, 2 H).

### Example 37A 3-(2,6-dichlorobenzyl)-1-(4-hydroxy-4-methylcyclohexyl)pyrrolidin-2-one (retention time 35 min)

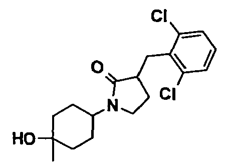

3-(2,6-Dichlorobenzyl)-1-(4-hydroxy-4-methylcyclohexyl)pyrrolidin-2-one obtained in Example 30A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.06 (s, 1 H), 1.21-1.28 (m, 1 H), 1.25 (s, 3 H), 1.46-2.03 (m, 9 H), 2.85-2.99 (m, 1 H), 2.99-3.11 (m, 1 H), 3.15-3.29 (m, 1 H), 3.31-3.44 (m, 1 H), 3.49 (dd, J=13.2, 4.3 Hz, 1 H), 3.89-4.05 (m, 1 H), 7.04-7.15 (m, 1 H), 7.27-7.34 (m, 2 H).

### Example 38A 3-(2,6-dichlorobenzyl)-1-(4-hydroxy-4-methylcyclohexyl)pyrrolidin-2-one (retention time 53 min)

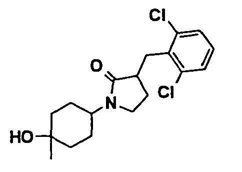

3-(2,6-Dichlorobenzyl)-1-(4-hydroxy-4-methylcyclohexyl)pyrrolidin-2-one obtained in Example 30A was subjected to resolution with normal phase chiral HPLC (manufactured by Daicel Chemical Industries, Ltd., chiralpak AD (trade name), 50 mmID X 500 mmL, hexane-ethanol 85:15) to give the title compound.
1H NMR (300 MHz, CDCl₃) δ ppm 1.29 (s, 3 H), 1.38 (s, 1 H), 1.45-2.03 (m, 10 H), 2.84-3.09 (m, 2 H), 3.14-3.27 (m, 1 H), 3.29-3.41 (m, 1 H), 3.49 (dd, J=13.2, 4.3 Hz, 1 H), 3.83-4.08 (m, 1 H), 7.04-7.17 (m, 1 H), 7.27-7.33 (m, 2 H).

### Example 39A ethyl 4-[3-(2, 6-dichlorobenzyl)-2-oxopyrrolidin-1-yl]piperidine-1-carboxylate

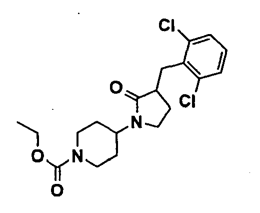

In the same manner as in Example 1A, the title compound was obtained from ethyl 4-(2-oxopyrrolidin-1-yl)piperidine-1-carboxylate obtained in Reference Example 7A and α,2,6-trichlorotoluene.
1H NMR (300 MHz, CDCl₃) δ ppm 1.26 (t, J=7.1 Hz, 3 H), 1.43-1.79 (m, 4 H), 1.79-2.13 (m, 2 H), 2.74-3.10 (m, 4 H), 3.10-3.23 (m, 1 H), 3.24-3.38 (m, 1 H), 3.49 (dd, J=13.2, 4.3 Hz, 1 H), 4.07-4.40 (m, 5 H), 7.03-7.16 (m, 1 H), 7.30 (d, J=8.1 Hz, 2 H). Example 40A 3-(2,6-dichlorobenzyl)-1-(piperidin-4-yl)pyrrolidin-2-one

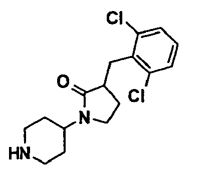

A mixture of ethyl 4-[3-(2,6-dichlorobenzyl)-2-oxopyrrolidin-1-yl]piperidine-1-carboxylate obtained in Example 39A (2.0 g), trimethylsilyl iodide (5.0 g) and chloroform (20 mL) was stirred at 60°C for 16 hr. Methanol (2 mL), 10% sodium thiosulfate solution (20 mL) and 1N sodium hydroxide solution (10 mL) were added successively to the reaction solution, and the mixture was extracted with ethyl acetate. The ethyl acetate layer was washed with saturated aqueous sodium hydrogencarbonate and saturated brine, and dried over anhydrous magnesium sulfate. The solvent was evaporated under reduced pressure to give the title compound (1.1 g, 67%) as a yellow solid.
1H NMR (300 MHz, CDCl₃) δ ppm 1.77-2.09 (m, 6 H), 2.82-3.12 (m, 4 H), 3.17-3.29 (m, 1 H), 3.33-3.56 (m, 4 H), 4.09-4.29 (m, 2 H), 7.05-7.15 (m, 1 H), 7.30 (d, J=8.1 Hz, 2 H).

### Example 41A 1-(1-acetylpiperidin-4-yl)-3-(2,6-dichlorobenzyl)pyrrolidin-2-one

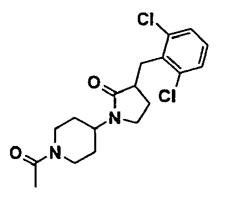

In the same manner as in Example 22A, the title compound was obtained from 3-(2,6-dichlorobenzyl)-1-(piperidin-4-yl)pyrrolidin-2-one obtained in Example 40A and acetyl chloride.
1H NMR (300 MHz, CDCl₃) δ ppm 1.48-1.68 (m, 2 H), 1.68-2.03 (m, 4 H), 2.11 (d like, 3 H), 2.51-2.69 (m, 1 H), 2.87-3.24 (m, 4 H), 3.25-3.38 (m, 1 H), 3.49 (dd, J=13.2, 4.3 Hz, 1 H), 3.82-3.96 (m, 1 H), 4.12-4.32 (m, 1 H), 4.67-4.86 (m, 1 H), 7.05-7.15 (m, 1 H), 7.30 (d, J=8.1 Hz, 2 H).

### Example 42A 3-(2,6-dichlorobenzyl)-1-[1-(methylsulfonyl)piperidin-4-yl]pyrrolidin-2-one

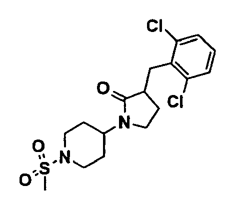

In the same manner as in Example 22A, the title compound was obtained from 3-(2,6-dichlorobenzyl)-1-(piperidin-4-yl)pyrrolidin-2-one obtained in Example 40A and methanesulfonyl chloride.
1H NMR (300 MHz, CDCl₃) δ ppm 1.72-2.09 (m, 6 H), 2.68-2.86 (m, 2 H), 2.80 (s, 3 H), 2.87-3.12 (m, 2 H), 3.12-3.27 (m, 1 H), 3.26-3.41 (m, 1 H), 3.48 (dd, J=13.0, 4.3 Hz, 1 H), 3.81-4.02 (m, 2 H), 4.03-4.24 (m, 1 H), 7.04-7.16 (m, 1 H), 7.30 (d, J=7.9 Hz, 2 H).

### Example 43A 1-(2,3-dihydro-1H-inden-1-yl)-3-(2-methylbenzyl)pyrrolidin-2-one

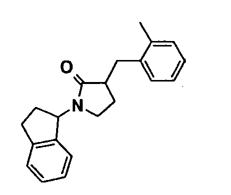

In the same manner as in Example 1A, the title compound was obtained from 1-(2,3-dihydro-1H-inden-1-yl)pyrrolidin-2-one 1-(1,2,3,4-tetrahydronaphthalen-1-yl)pyrrolidin-2-one obtained in Reference Example 5A and α-bromo-o-xylene.
1H NMR (300 MHz, CDCl₃) δ ppm 1.59-1.77 (m, 1 H), 1.86-2.09 (m, 2 H), 2.30-2.50 (m, 1 H), 2.37 (s, 3 H), 2.52-2.69 (m, 1 H), 2.70-3.19 (m, 5 H), 3.31-3.54 (m, 1 H), 5.75-5.92 (m, 1 H), 6.98-7.32 (m, 8 H).

Example 44A 3-(2,6-dichlorobenzyl)-1-(2,3-dihydro-1H-inden-1-yl)pyrrolidin-2-one

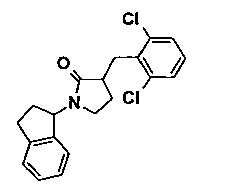

In the same manner as in Example 1A, the title compound was obtained from 1-(2,3-dihydro-1H-inden-1-yl)pyrrolidin-2-one obtained in Reference Example 5A and α,2,6-trichlorotoluene.
1H NMR (300 MHz, CDCl₃) δ ppm 1.75-2.11 (m, 3 H), 2.30-2.58 (m, 1 H), 2.83-3.27 (m, 6 H), 3.47-3.70 (m, 1 H), 5.76-5.91 (m, 1 H), 7.06-7.15 (m, 1 H), 7.14-7.28 (m, 4 H), 7.28-7.37 (m, 2 H).

Example 45A 3-(2-methylbenzyl)-1-(1,2,3,4-tetrahydronaphthalen-1-yl)pyrrolidin-2-one

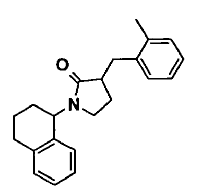

In the same manner as in Example 1A, the title compound was obtained from 1-(1,2,3,4-tetrahydronaphthalen-1-yl)pyrrolidin-2-one obtained in Reference Example 6A and α-bromo-o-xylene.
1H NMR (300 MHz, CDCl₃) δ ppm 1.61-2.15 (m, 6 H), 2.38 (s, 3 H), 2.53-2.71 (m, 1 H), 2.72-3.01 (m, 4 H), 3.02-3.19 (m, 1 H), 3.39 (dd, J=14.1, 3.8 Hz, 1 H), 5.35-5.57 (m, 1 H), 6.91-7.03 (m, 1 H), 7.03-7.23 (m, 7 H).

### Example 46A 3-(2,6-dichlorobenzyl)-1-(1,2,3,4-tetrahydronaphthalen-1-yl)pyrrolidin-2-one

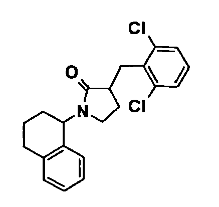

In the same manner as in Example 1A, the title compound was obtained from 1-(1,2,3,4-tetrahydronaphthalen-1-yl)pyrrolidin-2-one obtained in Reference Example 6A and α, 2, 6-trichlorotoluene.
1H NMR (300 MHz, CDCl₃) δ ppm 1.75-2.20 (m, 6 H), 2.72-2.87 (m, 2 H), 2.86-3.02 (m, 1 H), 2.99-3.16 (m, 2 H), 3.18-3.33 (m, 1 H), 3.45-3.69 (m, 1 H), 5.36-5.55 (m, 1 H), 6.93-7.03 (m, 1 H), 7.05-7.20 (m, 4 H), 7.31 (d, J=8.1 Hz, 2 H).

### Example 47A 1-(2,6-dichlorobenzyl)-3-(2-methylbenzoyl)pyrrolidin-2-one

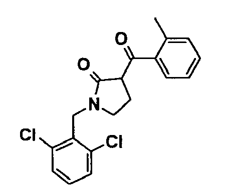

In the same manner as in Example 1A, the title compound was obtained from 1-(2,6-dichlorobenzyl)pyrrolidin-2-one obtained in Reference Example 2A and methyl 2-methylbenzoate. 1H NMR (300 MHz, CDCl₃) δ ppm 2.07-2.26 (m, 1 H), 2.44-2.59 (m, 1 H), 2.51 (s, 3 H), 3.10-3.24 (m, 1 H), 3.32-3.46 (m, 1 H), 4.42 (dd, J=9.2, 5.3 Hz, 1 H), 4.75-4.94 (m, 2 H), 7.14-7.29 (m, 2 H), 7.28-7.49 (m, 4 H), 8.02 (dd, J=7.5, 0.9 Hz, 1 H).

### Example 48A 1-(2,6-dichlorobenzyl)-3-[(hydroxyimino)(2-methylphenyl)methyl]pyrrolidin-2-one

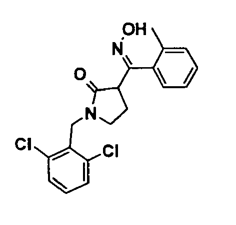

A mixture of 1-(2,6-dichlorobenzyl)-3-(2-methylbenzoyl)pyrrolidin-2-one obtained in Example 47A (0.38 g), hydroxylamine hydrochloride (0.24 g), sodium acetate (0.26 g) and ethanol-water (4:1, 15 mL) was stirred at 80°C for 16 hr under nitrogen atmosphere. The reaction solution was concentrated under reduced pressure, and the residue was partitioned between ethyl acetate-water. The ethyl acetate layer was washed with saturated brine, and dried over anhydrous magnesium sulfate, and the solvent was evaporated under reduced pressure. The residue was subjected to silica gel column chromatography (hexane-ethyl acetate 95:5-2:3) to give the title compound (0.33 g, 83%) as a colorless solid.
LC-MS (ESI+); m/z 377 (M)⁺

### Example 49A 1-(2,6-dichlorobenzyl)-3-[(methoxyimino)(2-methylphenyl)methyl]pyrrolidin-2-one

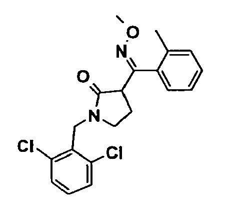

In the same manner as in Example 48A, the title compound was obtained from 1-(2,6-dichlorobenzyl)-3-(2-methylbenzoyl)pyrrolidin-2-one obtained in Example 47A and O-methylhydroxylamine hydrochloride.
1H NMR (300 MHz, CDCl₃) δ ppm 2.07-2.32 (m, 2 H), 2.40 (s, 3 H), 3.15 (dd, J=8.1, 6.0 Hz, 2 H), 3.93-4.07 (m, 1 H), 3.95 (s, 3 H), 4.67-4.75 (d like, 1 H), 4.85-4.94 (d like, 1 H), 7.11-7.40 (m, 7 H).

### Example 50A 3-(2,6-dichlorobenzoyl)-1-(2-methylbenzyl)pyrrolidin-2-one

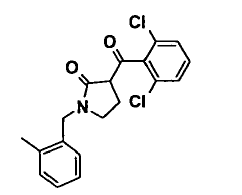

In the same manner as in Example 1A, the title compound was obtained from 1-(2-methylbenzyl)pyrrolidin-2-one obtained in Reference Example 1A and methyl 2,6-dichlorobenzoate.
LC-MS (ESI+); m/z 362 (M)⁺

### Example 51A 1-(1-adamantyl)-3-(2,6-dichlorobenzyl)pyrrolidin-2-one

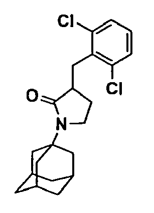

In the same manner as in Example 1A, the title compound was synthesized from 1-(1-adamantyl)pyrrolidin-2-one and α,2,6-trichlorotoluene.
1H NMR (300 MHz, CDCl₃) δ ppm 1.62-1.95 (m, 8 H), 2.04-2.24 (m, 9 H), 2.75-2.90 (m, 1 H), 2.95-3.07 (m, 1 H), 3.18-3.33 (m, 1 H), 3.40-3.53 (m, 2 H), 7.03-7.13 (m, 1 H), 7.26-7.32 (m, 2 H).

Example 52A 1-(1-adamantyl)-3-(2-methylbenzyl)pyrrolidin-2-one

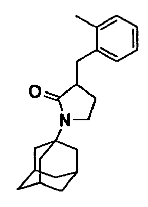

In the same manner as in Example 1A, the title compound was synthesized from 1-(1-adamantyl)pyrrolidin-2-one and α-bromo-o-xylene.
1H NMR (300 MHz, CDCl₃) δ ppm 1.49-1.79 (m, 7 H), 1.85-2.04 (m, 1 H), 2.04-2.26 (m, 9 H), 2.33 (s, 3 H), 2.42-2.79 (m, 2 H), 3.19-3.47 (m, 3 H), 7.03-7.20 (m, 4 H).

Example 53A 1-(1-adamantyl)-3,3-bis(2-methylbenzyl)pyrrolidin-2-one

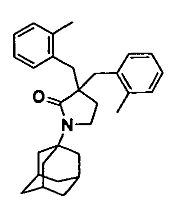

In the same manner as in Example 1A, the title compound was synthesized from 1-(1-adamantyl)pyrrolidin-2-one and α-bromo-o-xylene.
1H NMR (300 MHz, CDCl₃) δ ppm 1.61-1.74 (m, 8 H), 1.92-2.07 (m, 9 H), 2.28 (s, 6 H), 2.58 (t, J=7.0 Hz, 2 H), 2.80 (d, J=13.6 Hz, 2 H), 3.10 (d, J=13.6 Hz, 2 H), 7.03-7.18 (m, 6 H), 7.25-7.34 (m, 2 H).

### Example 54A 3-(2-methoxybenzyl)-1-(tetrahydro-2H-pyran-4-yl)pyrrolidin-2-one

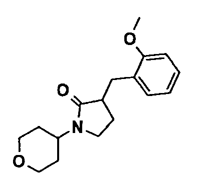

In the same manner as in Example 1A, the title compound was obtained from 1-(tetrahydro-2H-pyran-4-yl)pyrrolidin-2-one obtained in Reference Example 8A and 2-methoxybenzyl bromide.
1H NMR (300 MHz, CDCl₃) δ ppm 1.51-1.81 (m, 5 H), 1.89-2.03 (m, 1 H), 2.58 (dd, J=13.4, 10.0 Hz, 1 H), 2.74-2.87 (m, 1 H), 3.12-3.21 (m, 2 H), 3.31 (dd, J=13.4, 4.1 Hz, 1 H), 3.44-3.55 (m, 2 H), 3.82 (s, 3 H), 3.98-4.03 (m, 2 H), 4.16-4.30 (m, 1 H), 6.82-6.92 (m, 2 H), 7.12-7.24 (m, 2 H).

### Example 55A 3-(2,6-dichlorobenzyl)-1-(tetrahydro-2H-pyran-4-yl)pyrrolidin-2-one

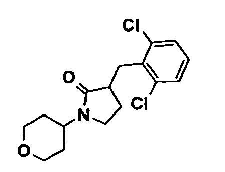

In the same manner as in Example 1A, the title compound was obtained from 1-(tetrahydro-2H-pyran-4-yl)pyrrolidin-2-one obtained in Reference Example 8A and α,2,6-trichlorotoluene.
1H NMR (300 MHz, CDCl₃) δ ppm 1.59-2.11 (m, 6 H), 2.84-3.13 (m, 2 H), 3.12-3.29 (m, 1 H), 3.30-3.41 (m, 1 H), 3.42-3.60 (m, 3 H), 3.94-4.11 (m, 2 H), 4.15-4.36 (m, 1 H), 7.08-7.16 (m, 1 H), 7.28-7.36 (m, 2 H).

### Example 56A 1-(cyclopropylmethyl)-3-phenylpyrrolidin-2-one

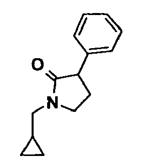

To a mixture of 3-phenylpyrrolidin-2-one (0.25 g), cyclopropylmethyl bromide (0.23 g) and N,N-dimethylformamide (8 mL) was added 60% sodium hydride (74 mg), and the mixture was stirred at room temperature for 1 hr. Water was added to the reaction solution, and the mixture was extracted with ethyl acetate. The extract was washed successively with water and saturated brine, and dried over anhydrous magnesium sulfate. The solvent was evaporated under reduced pressure, and the residue was subjected to silica gel column chromatography (hexane-ethyl acetate 9:1-3:7) to give the title compound (0.10 g, 30%) as a pale-yellow oil.
1H NMR (300 MHz, CDCl₃) δ ppm 0.22-0.30 (m, 2 H), 0.50-0.60 (m, 2 H), 0.90-1.06 (m, 1 H), 2.02-2.22 (m, 1 H), 2.46-2.62 (m, 1 H), 3.19-3.28 (m, 2 H), 3.45-3.74 (m, 3 H), 7.20-7.39 (m, 5 H).

Example 57A 1-(2-methylbenzyl)-3-phenylpyrrolidin-2-one

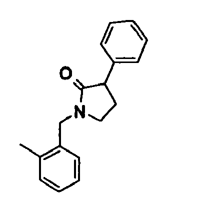

In the same manner as in Reference Example 1A, the title compound was obtained from 3-phenylpyrrolidin-2-one and α-bromo-o-xylene.
1H NMR (300 MHz, CDCl₃) δ ppm 2.00-2.20 (m, 1 H), 2.33 (s, 3 H), 2.41-2.57 (m, 1 H), 3.18-3.38 (m, 2 H), 3.73 (t, J=8.9 Hz, 1 H), 4.44 (d, J=14.7 Hz, 1 H), 4.63-4.73 (m, 1 H), 7.16-7.39 (m, 9 H).

### Example 58A 1-(2,6-dichlorobenzyl)-3-phenylpyrrolidin-2-one

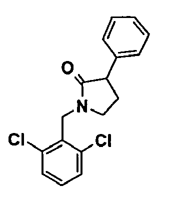

In the same manner as in Reference Example 1A, the title compound was obtained from 3-phenylpyrrolidin-2-one and α,2,6-trichlorotoluene.
1H NMR (300 MHz, CDCl₃) δ ppm 2.22-2.50 (m, 2 H), 2.98-3.18 (m, 2 H), 3.25-3.40 (m, 1 H), 4.80-4.95 (m, 1 H), 4.96-5.16 (m, 1 H), 7.19-7.56 (m, 8 H).

### Example 59A 1,3-bis(2-methylbenzyl)-3-phenylpyrrolidin-2-one

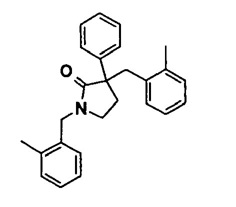

In the same manner as in Reference Example 1A, the title compound was obtained from 3-phenylpyrrolidin-2-one and α-bromo-o-xylene.
1H NMR (300 MHz, CDCl₃)δ ppm 2.03-2.14 (m, 1 H), 2.17 (s, 3 H x2), 2.29-2.45 (m, 1 H), 2.71-2.82 (m, 1 H), 2.86-3.00 (m, 1 H), 3.20 (d, J=13.9 Hz, 1 H), 3.36-3.52 (m, 1 H), 4.31-4.45 (m, 1 H), 4.48-4.63 (m, 1 H), 6.90 (d, J=7.5 Hz, 1 H), 6.99-7.21 (m, 6 H), 7.20-7.41 (m, 4 H), 7.45-7.57 (m, 2 H).

Example 60A 1,3-bis(2,6-dichlorobenzyl)-3-phenylpyrrolidin-2-one

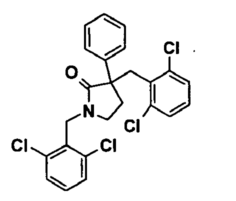

In the same manner as in Reference Example 1A, the title compound was obtained from 3-phenylpyrrolidin-2-one and α,2,6-trichlorotoluene.
1H NMR (300 MHz, CDCl₃) δ ppm 2.27-2.53 (m, 2 H), 2.72-3.01 (m, 2 H), 3.63-3.78 (m, 2 H), 4.78-4.94 (m, 2 H), 6.98-7.35 (m, 9 H), 7.37-7.51 (m, 2 H).

Example 61A 3-(2,6-dichlorobenzyl)-1-(tetrahydro-2H-thiopyran-4-yl)pyrrolidin-2-one

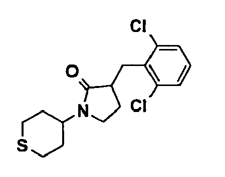

In the same manner as in Example 1A, the title compound was obtained from 1-(tetrahydro-2H-thiopyran-4-yl)pyrrolidin-2-one obtained in Reference Example 9A and α,2,6-trichlorotoluene. 1H NMR (300 MHz, CDCl₃) δ ppm 1.68-2.14 (m, 6 H), 2.57-2.75 (m, 2 H), 2.76-3.11 (m, 4 H), 3.14-3.26 (m, 1 H), 3.28-3.42 (m, 1 H), 3.48 (dd, J=13.2, 4.3 Hz, 1 H), 3.91-4.07 (m, 1 H), 7.04-7.18 (m, 1 H), 7.27-7.35 (m, 2 H).

### Example 62A 3-(2,6-dichlorobenzyl)-1-(1-oxidetetrahydro-2H-thiopyran-4-yl)pyrrolidin-2-one

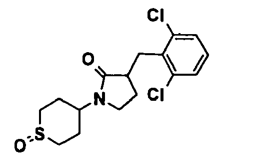

3-(2,6-dichlorobenzyl)-1-(tetrahydro-2H-thiopyran-4-yl)pyrrolidin-2-one obtained in Example 61A (0.30 g), m-chloroperbenzoic acid (hydroscopic, 0.28 g) and dichloromethane (15 mL) were stirred at 0°C for 30 min. 10% Sodium thiosulfate was added to the reaction solution, and the mixture was stirred for 20 min, and extracted with dichloromethane. The dichloromethane layer was washed successively with 10% aqueous sodium bicarbonate and saturated brine, and dried over anhydrous magnesium sulfate. The solvent was evaporated under reduced pressure, and the residue was subjected to silica gel column chromatography (ethyl acetate-methanol 10:1) to give the title compound (0.13 g, 42%) as a colorless solid.
1H NMR (300 MHz, CDCl₃) δ ppm 1.65-1.83 (m, 1 H), 1.85-2.22 (m, 3 H), 2.45-2.72 (m, 3 H), 2.75-3.60 (m, 8 H), 4.04-4.34 (m, 1 H), 7.03-7.16 (m, 1 H), 7.30 (d, J=7.9 Hz, 2 H).

Example 63A 3-(2,6-dichlorobenzyl)-1-(1,1-dioxidotetrahydro-2H-thiopyran-4-yl)pyrrolidin-2-one

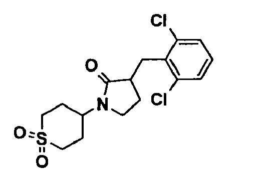

In the same manner as in Example 62A, to give the title compound (0.27 g, 82%) was obtained as a colorless solid from 3-(2,6-dichlorobenzyl)-1-(tetrahydro-2H-thiopyran-4-yl)pyrrolidin-2-one obtained in Example 61A (0.30 g) and m-chloroperbenzoic acid (hydroscopic, 0.56 g).
1H NMR (300 MHz, CDCl₃) δ ppm 1.80-2.20 (m, 4 H), 2.22-2.52 (m, 2 H), 2.85-3.30 (m, 7 H), 3.32-3.53 (m, 2 H), 4.17-4.40 (m, 1 H), 7.06-7.18 (m, 1 H), 7.28-7.37 (m, 2 H).

### Example 64A 3-(2, 6-dichlorobenzyl)-1-(1-ethylpiperidin-4-yl)pyrrolidin-2-one

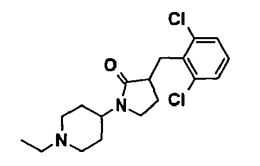

A mixture of 3-(2,6-dichlorobenzyl)-1-(piperidin-4-yl)pyrrolidin-2-one obtained in Example 40A (0.25 g), ethyl iodide (0.12 g), triethylamine (0.11 mL) and N,N-dimethylformamide (3 mL) was stirred at room temperature for 1 day. Water was added to the reaction solution, and the mixture was extracted with ethyl acetate. The extract was washed successively with water and saturated brine, and dried over anhydrous magnesium sulfate. The solvent was evaporated under reduced pressure, and the residue was subjected to silica gel column chromatography (hexane-ethyl acetate 1:1) to give the title compound (0.11 g, 41%) as a pale-brown solid.
1H NMR (300 MHz, CDCl₃) δ ppm 1.09 (t, J=7.2 Hz, 3 H), 1.65-2.12 (m, 8 H), 2.42 (q, J=7.2 Hz, 2 H), 2.85-3.08 (m, 4H), 3.12-3.26 (m, 1 H), 3.29-3.42 (m, 1 H), 3.49 (dd, J=13.2, 4.3 Hz, 1 H), 3.96-4.12 (m, 1 H), 7.05-7.13 (m, 1 H), 7.27-7.35 (m, 2 H). Example 65A 1-(2,3-dihydro-1H-inden-1-yl)-3-(2-methoxybenzyl)pyrrolidin-2-one (less polar product)

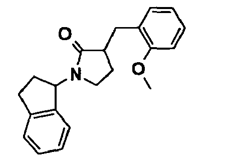

In the same manner as in Example 1A, the title compound was obtained from 1-(2, 3-dihydro-1H-inden-1-yl)pyrrolidin-2-one obtained in Reference Example 5A and 2-methoxybenzyl chloride.
1H NMR (300 MHz, CDCl₃) δ ppm 1.62-1.79 (m, 1 H), 1.82-2.01 (m, 2 H), 2. 30-2. 48 (m, 1 H), 2.64 (dd, J=13. 4, 10.0 Hz, 1 H), 2.79-3.08 (m, 5 H), 3. 37 (dd, J=13. 4, 4. 1 Hz, 1 H), 3. 82 (s, 3 H), 5. 83 (t, J=7.6 Hz, 1 H), 6.82-6.92 (m, 2 H), 7.08-7.25 (m, 6 H).

### Example 66A 1-(2, 3-dihydro-1H-inden-1-yl)-3-(2-methoxybenzyl)pyrrolidin-2-one (more polar product)

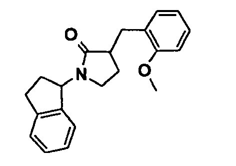

In the same manner as in Example 1A, the title compound was obtained from 1-(2,3-dihydro-1H-inden-1-yl)pyrrolidin-2-one obtained in Reference Example 5A and 2-methoxybenzyl chloride.
1H NMR (300 MHz, CDCl₃) δ ppm 1.57-1.74 (m, 1 H), 1.85-2.02 (m, 2 H), 2.30-2.46 (m, 1 H), 2.64 (dd, J=13.6, 10.2 Hz, 1 H), 2.81-3.12 (m, 5 H), 3.42 (dd, J=13.6, 4. 1 Hz, 1 H), 3.82 (s, 3 H), 5.81 (t, J=7.8 Hz, 1 H), 6.81-6.94 (m, 2 H), 7.05 (d, J=7.2 Hz, 1 H), 7.14-7.25 (m, 5 H) .

Example 67A 3-(2-methoxybenzyl)-1-(1,2,3,4-tetrahydronaphthalen-1-yl)pyrrolidin-2-one

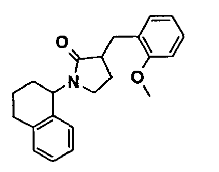

In the same manner as in Example 1A, the title compound was obtained from 1-(1,2,3,4-tetrahydronaphthalen-1-yl)pyrrolidin-2-one obtained in Reference Example 6A and 2-methoxybenzyl chloride.
1H NMR (300 MHz, CDCl₃) δ ppm 1.61-2.03 (m, 6 H), 2.59-3.16 (m, 6 H), 3.37 (dd, J=13.5, 4.2 Hz, 1 H), 3.83 (s, 3 H), 5.45 (dd, J=9.1, 5.4 Hz, 1 H), 6.81-7.03 (m, 3 H), 7.04-7.25 (m, 5 H). Example 68A N-(2-chlorophenyl)-1-cyclohexyl-2-oxopyrrolidine-3-carboxamide

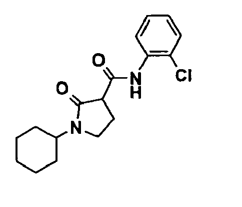

A mixture of 1-cyclohexyl-2-oxopyrrolidine-3-carboxylic acid obtained in Reference Example 10A (0.70 g), 2-chloroaniline (0.47 g), bis(2-oxo-3-oxazolidinyl)phosphinic chloride (0.93 g), triethylamine (1.0 mL) and dichloromethane (20 mL) was stirred at room temperature for 16 hr. The reaction solution was concentrated, and the residue was partitioned between ethyl acetate-water. The ethyl acetate layer was washed successively with 2N hydrochloric acid and saturated brine, and dried over anhydrous magnesium sulfate, and the solvent was evaporated under reduced pressure to give the title compound (0.94 g, 89%) as a colorless solid.
1H NMR (300 MHz, CDCl₃) δ ppm 1.00-1.23 (m, 1 H), 1.27-1.50 (m, 4 H), 1.61-1.96 (m, 5 H), 2.33-2.58 (m, 2 H), 3.28-3.45 (m, 2 H), 3.52 (t, J=9.6 Hz, 1 H), 3.92-4.12 (m, 1 H), 6.95-7.07 (m, 1 H), 7.18-7.32 (m, 1 H), 7.38 (dd, J=8.3, 1.5 Hz, 1 H), 8.41 (dd, J=8.3, 1.5 Hz, 1 H), 10.40 (s, 1 H).

### Example 69A 1-cyclohexyl-N-(2-methoxyphenyl)-N-methyl-2-oxopyrrolidine-3-carboxamide

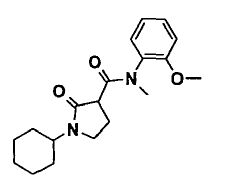

In the same manner as in Example 68A, the title compound was obtained from 1-cyclohexyl-2-oxopyrrolidine-3-carboxylic acid obtained in Reference Example 10A and 2-methoxy-N-methylaniline.
1H NMR (300 MHz, CDCl₃) δ ppm 1.08 (t, J=11.7 Hz, 1 H), 1.20-1.45 (m, 5 H), 1.53-1.83 (m, 4 H), 1.83-2.00 (m, 1 H), 2.32-2.51 (m, 1 H), 3.08-3.27 (m, 1 H), 3.22 (s, 3 H), 3.32 (dd, J=9.2, 6.6 Hz, 1 H), 3.42-3.53 (m, 1 H), 3.76-3.96 (m, 1 H), 3.82 (s, 3 H), 6.87-7.12 (m, 2 H), 7.28-7.41 (m, 1 H), 7.61 (dd, J=7.7, 1.7 Hz, 1 H).

Example 70A 1-cyclohexyl-N-(2,6-dichlorobenzyl)-2-oxopyrrolidine-3-carboxamide

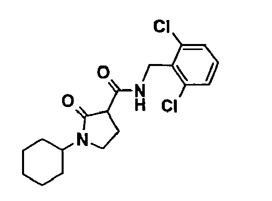

A mixture of 1-cyclohexyl-2-oxopyrrolidine-3-carboxylic acid obtained in Reference Example 10A (0.70 g), 2,6-dichlorobenzylamine (0.64 g), 1-ethyl-3-(3'-dimethylaminopropyl)carbodiimide (0.70 g), N-hydroxybenzotriazole (0.49 g) and acetonitrile (20 mL) was stirred at room temperature for 16 hr. Water was added to the reaction solution, and the mixture was extracted with ethyl acetate. The ethyl acetate layer was washed successively with 2N hydrochloric acid, 10% aqueous sodium bicarbonate and saturated brine, was dried over anhydrous magnesium sulfate, and the solvent was evaporated under reduced pressure to give the title compound (1.09 g, 89%) as a colorless solid.
1H NMR (300 MHz, CDCl₃) δ ppm 0.99-1.21 (m, 1 H), 1.25-1.46 (m, 4 H), 1.61-1.89 (m, 5 H), 2.22-2.53 (m, 2 H), 3.20-3.44 (m, 3 H), 3.81-4.00 (m, 1 H), 4.64 (dd, J=13.9, 4.9 Hz, 1 H), 4.93 (dd, J=13.9, 6.4 Hz, 1 H), 7.10-7.22 (m, 1 H), 7.28-7.38 (m, 2 H), 8.05-8.18 (m, 1 H).

### Example 71A N-benzyl-l-cyclohexyl-N-methyl-2-oxopyrrolidine-3-carboxamide

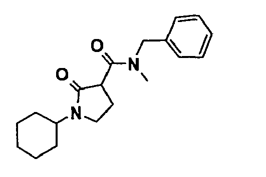

In the same manner as in Example 68A, the title compound was obtained from 1-cyclohexyl-2-oxopyrrolidine-3-carboxylic acid obtained in Reference Example 10A and N-methylbenzylamine.
1H NMR (300 MHz, CDCl₃) δ ppm 0.95-1.24 (m, 1 H), 1.23-1.48 (m, 4 H), 1.60-1.91 (m, 5 H), 1.94-2.24 (m, 1 H), 2.53-2.74 (m, 1 H), 2.99 (s, 1 H), 3.18 (s, 2 H), 3.21-3.41 (m, 1 H), 3.46-3.62 (m, 1 H), 3.64-4.04 (m, 2 H), 4.44 and 5.41 (d, J=17.3 Hz, 1 H), 4.65 (s, 1 H), 7.14-7.47 (m, 5 H).

### Example 72A N-[2-(2-chlorophenyl)ethyl]-1-cyclohexyl-2-oxopyrrolidine-3-carboxamide

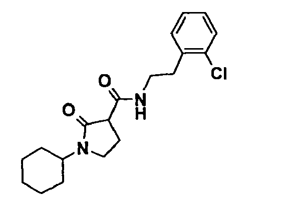

In the same manner as in Example 70A, the title compound was obtained from 1-cyclohexyl-2-oxopyrrolidine-3-carboxylic acid obtained in Reference Example 10A and 2-(2-chlorophenyl)ethylamine.
1H NMR (300 MHz, CDCl₃) δ ppm 0.98-1.21 (m, 1 H), 1.28-1.50 (m, 4 H), 1.56-1.95 (m, 5 H), 2.19-2.50 (m, 2 H), 2.98 (t, J=7.3 Hz, 2 H), 3.17-3.30 (m, 2 H), 3.30-3.42 (m, 1 H), 3.42-3.69 (m, 2 H), 3.77-3.96 (m, 1 H), 7.10-7.29 (m, 3 H), 7.29-7.40 (m, 1 H), 7.73 (s, 1 H).

### Example 73A 1-cyclohexyl-N-methyl-2-oxo-N-(2-phenylethyl)pyrrolidine-3-carboxamide

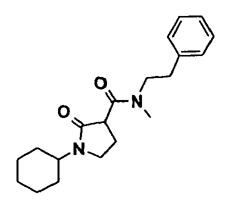

In the same manner as in Example 68A, the title compound was obtained from 1-cyclohexyl-2-oxopyrrolidine-3-carboxylic acid obtained in Reference Example 10A and N-methylphenethylamine.
1H NMR (300 MHz, CDCl₃) δ ppm 0.97-1.20 (m, 1 H), 1.20-1.47 (m, 4 H), 1.57-1.89 (m, 5 H), 1.98-2.40 (m, 1 H), 2.45-2.65 (m, 1 H), 2.76-2.96 (m, 2 H), 2.99 (s, 1 H), 3.10-3.23 (m, 2 H), 3.23-3.37 (m, 1 H), 3.36-3.97 (m, 4 H), 4.06-4.30 (m, 1 H), 7.10-7.38 (m, 5 H).

### Example 74A 1-cyclohexyl-3-{[4-(2-methylphenyl)piperidin-1-yl]carbonyl}pyrrolidin-2-one

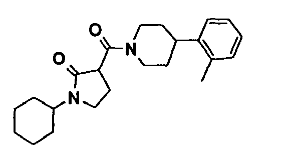

In the same manner as in Example 68A, the title compound was obtained from 1-cyclohexyl-2-oxopyrrolidine-3-carboxylic acid obtained in Reference Example 10A and 4-(2-methylphenyl)piperidine.
1H NMR (300 MHz, CDCl₃) δ ppm 1.00-1.23 (m, 1 H), 1.37 (t like, 4 H), 1.59-1.97 (m, 8 H), 2.00-2.22 (m, 2 H), 2.36 (d, J=1.3 Hz, 3 H), 2.57-2.84 (m, 2 H), 2.88-3.08 (m, 1 H), 3.08-3.43 (m, 2 H), 3.47-3.62 (m, 1 H), 3.74-3.98 (m, 2 H), 4.38-4.57 (m, 1 H), 4.73-4.94 (m, 1 H), 7.05-7.21 (m, 3 H), 7.22-7.30 (m, 1 H).

### Example 75A 1-(2,3-dihydro-1H-inden-1-yl)-3-[(1-methyl-1H-imidazol-2-yl)carbonyl]pyrrolidin-2-one

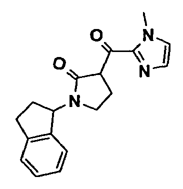

In the same manner as in Example 1A, the title compound was obtained from 1-(2,3-dihydro-1H-inden-1-yl)pyrrolidin-2-one obtained in Reference Example 5A and methyl 1-methyl-1H-imidazole-2-carboxylate.
LC-MS (ESI+); m/z 310 (M+H)⁺

### Example 76A 1-(2,3-dihydro-1H-inden-1-yl)-3-[(1-methyl-4-phenyl-1H-imidazol-2-yl)carbonyl]pyrrolidin-2-one

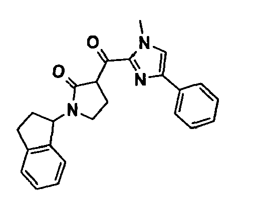

In the same manner as in Example 1A, the title compound was synthesized from 1-(2,3-dihydro-1H-inden-1-yl)pyrrolidin-2-one obtained in Reference Example 5A and methyl 1-methyl-4-phenyl-1H-imidazole-2-carboxylate.
1H NMR (300 MHz, CDCl₃) δ ppm 1.94-2.14 (m, 1 H), 2.29-2.55 (m, 3 H), 2.83-3.11 (m, 2 H), 3.17-3.37 (m, 2 H), 4.09 (s, 3 H), 5.17 (dd, J=9.2, 7.9 Hz, 1 H), 5.82 (t, J=7.9 Hz, 1 H), 7.11-7.35 (m, 5 H), 7.34-7.50 (m, 3 H), 7.75-7.87 (m, 2 H).

### Example 77A 1-(1-adamantyl)-3-[(1-methyl-1H-imidazol-2-yl)carbonyl]pyrrolidin-2-one

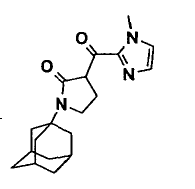

In the same manner as in Example 1A, the title compound was obtained from 1-(1-adamantyl)pyrrolidin-2-one and methyl 1-methyl-1H-imidazole-2-carboxylate.
1H NMR (300 MHz, CDCl₃) δ ppm 1.60-1.82 (m, 8 H), 2.01-2.27 (m, 10 H), 2.28-2.48 (m, 1 H), 3.39-3.54 (m, 1 H), 3.56-3.69 (m, 1 H), 4.00 (s, 1 H), 4.99 (dd, J=9.3, 8.0 Hz, 1 H), 7.04 (s, 1 H), 7.20 (s, 1 H).

### Example 78A 1-(1-adamantyl)-3-[(1-methyl-4-phenyl-1H-imidazol-2-yl)carbonyl]pyrrolidin-2-one

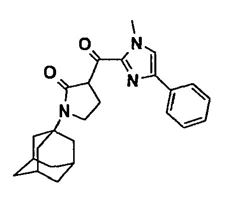

In the same manner as in Example 1A, the title compound was obtained from 1-(1-adamantyl)pyrrolidin-2-one and methyl 1-methyl-4-phenyl-1H-imidazole-2-carboxylate.
1H NMR (300 MHz, CDCl₃) δ ppm 1.60-1.82 (m, 7 H), 2.04-2.47 (m, 10 H), 3.44-3.59 (m, 1 H), 3.62-3.75 (m, 1 H), 4.04 (s, 3 H), 5.07 (dd, J=9.4, 7.7 Hz, 1 H), 7.23-7.45 (m, 4 H), 7.73-7.87 (m, 2 H).

### Example 79A 3-(2-chloro-4-fluorobenzyl)-1-(2,3-dihydro-1H-inden-1-yl)pyrrolidin-2-one (less polar product)

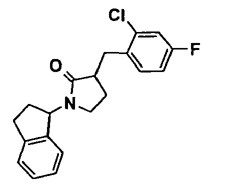

In the same manner as in Example 1A, the title compound was obtained from 1-(2,3-dihydro-1H-inden-1-yl)pyrrolidin-2-one obtained in Reference Example 5A and 2-chloro-4-fluorobenzyl bromide.
1H NMR (300 MHz, CDCl₃) δ ppm 1.62-1.79 (m, 1 H), 1.81-2.14 (m, 2 H), 2.28-2.54 (m, 1 H), 2.72-3.10 (m, 6 H), 3.28-3.55 (m, 1 H), 5.82 (t, J=7.6 Hz, 1 H), 6.87-6.98 (m, 1 H), 7.05-7.36 (m, 6 H).

### Example 80A 3-(2-chloro-4-fluorobenzyl)-1-(2,3-dihydro-1H-inden-1-yl)pyrrolidin-2-one (more polar product)

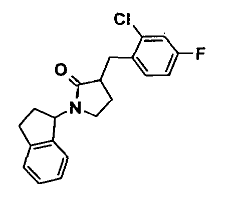

In the same manner as in Example 1A, the title compound was obtained from 1-(2,3-dihydro-1H-inden-1-yl)pyrrolidin-2-one obtained in Reference Example 5A and 2-chloro-4-fluorobenzyl bromide.
1H NMR (300 MHz, CDCl₃) δ ppm 1.57-1.78 (m, 1 H), 1.84-2.10 (m, 2 H), 2.29-2.51 (m, 1 H), 2.76-3.03 (m, 5 H), 3.03-3.19 (m, 1 H), 3.32-3.51 (m, 1 H), 5.80 (t, J=7.7 Hz, 1 H), 6.87-6.99 (m, 1 H), 7.03 (d, J=7.2 Hz, 1 H), 7.07-7.28 (m, 4 H), 7.31 (dd, J=8.6, 6.1 Hz, 1 H).

Example 81A 1-(1-adamantyl)-3-(2-methoxybenzyl)pyrrolidin-2-one

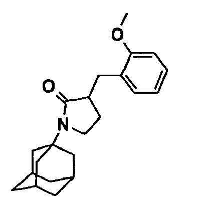

In the same manner as in Example 1A, the title compound was obtained from 1-(1-adamantyl)pyrrolidin-2-one and 2-methoxybenzyl bromide.
1H NMR (300 MHz, CDCl₃) δ ppm 1.52-1.98 (m, 8 H), 2.14 (s, 9 H), 2.44-2.60 (m, 1 H), 2.60-2.80 (m, 1 H), 3.12-3.42 (m, 3 H), 3.81 (s, 3 H), 6.79-6.95 (m, 2 H), 7.07-7.25 (m, 2 H).

### Example 82A 1-(1-adamantyl)-3-(2-chloro-4-fluorobenzyl)pyrrolidin-2-one

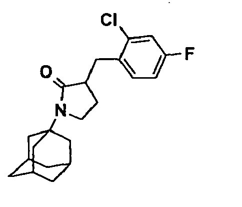

In the same manner as in Example 1A, the title compound was obtained from 1-(1-adamantyl)pyrrolidin-2-one and 2-chloro-4-fluorobenzyl bromide.
1H NMR (300 MHz, CDCl₃) δ ppm 1.50-1.77 (m, 7 H), 1.84-2.01 (m, 1 H), 2.03-2.26 (m, 8 H), 2.59-2.83 (m, 2 H), 3.19-3.44 (m, 3 H), 6.82-6.99 (m, 1 H), 7.10 (dd, J=8.5, 2.6 Hz, 1 H), 7.18-7.31 (m, 2 H).

### Example 83A 3-(2-chloro-4-fluorobenzyl)-1-(1,4-dioxaspiro[4.5]dec-8-yl)pyrrolidin-2-one

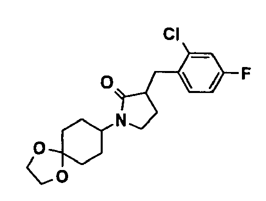

In the same manner as in Example 1A, the title compound was obtained from 1-(1,4-dioxaspiro[4.5]dec-8-yl)pyrrolidin-2-one and 2-chloro-4-fluorobenzyl bromide.
1H NMR (300 MHz, CDCl₃) δ ppm 1.60-1.90 (m, 9 H), 1.93-2.12 (m, 1 H), 2.69-2.86 (m, 2 H), 3.12-3.40 (m, 3 H), 3.94 (s, 4 H), 4.05 (dd, J=10.8, 3.1 Hz, 1 H), 6.84-6.97 (m, 1 H), 7.10 (dd, J=8.7, 2.6 Hz, 1 H), 7.19-7.32 (m, 1 H).

### Example 84A 3-(2-chloro-4-fluorobenzyl)-1-(4-oxocyclohexyl)pyrrolidin-2-one

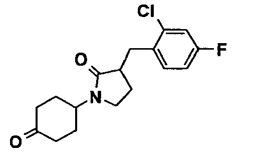

In the same manner as in Example 28A, the title compound was obtained from 3-(2-chloro-4-fluorobenzyl)-1-(1,4-dioxaspiro[4.5]dec-8-yl)pyrrolidin-2-one obtained in Example 83A.
1H NMR (300 MHz, CDCl₃) δ ppm 1.63-2.19 (m, 6 H), 2.36-2.63 (m, 4 H), 2.70-2.91 (m, 2 H), 3.15-3.28 (m, 2 H), 3.27-3.43 (m, 1 H), 4.39-4.61 (m, 1 H), 6.85-7.00 (m, 1 H), 7.12 (dd, J=8.5, 2.6 Hz, 1 H), 7.20-7.36 (m, 1 H).

### Example 85A 3-(2-chloro-4-fluorobenzyl)-1-(4-hydroxycyclohexyl)pyrrolidin-2-one

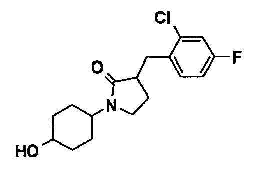

In the same manner as in Example 29A, the title compound was obtained from 3-(2-chloro-4-fluorobenzyl)-1-(4-oxocyclohexyl)pyrrolidin-2-one obtained in Example 84A.
LC-MS (ESI+); m/z 326 (M+H)⁺

### Example 86A 3-(2-chloro-4-fluorobenzyl)-1-(4-hydroxy-4-methylcyclohexyl)pyrrolidin-2-one (less polar product)

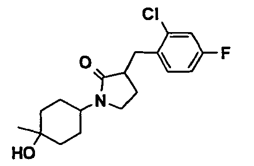

In the same manner as in Example 30A, the title compound was obtained from 3-(2-chloro-4-fluorobenzyl)-1-(4-oxocyclohexyl)pyrrolidin-2-one obtained in Example 84A.
LC-MS (ESI+); m/z 340 (M+H)⁺

### Example 87A 3-(2-chloro-4-fluorobenzyl)-1-(4-hydroxy-4-methylcyclohexyl)pyrrolidin-2-one (more polar product)

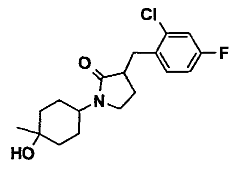

In the same manner as in Example 30A, the title compound was obtained from 3-(2-chloro-4-fluorobenzyl)-1-(4-oxocyclohexyl)pyrrolidin-2-one obtained in Example 84A.
LC-MS (ESI+); m/z 340 (M+H)⁺

### Example 88A tert-butyl (benzyl)(1-cyclohexyl-2-oxopyrrolidin-3-yl)carbamate

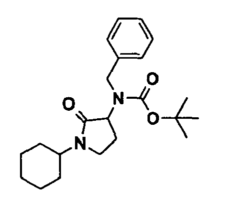

In the same manner as in Example 56A, the title compound was obtained from tert-butyl (1-cyclohexyl-2-oxopyrrolidin-3-yl)carbamate obtained in Reference Example 11A and benzyl bromide.
1H NMR (300 MHz, CDCl₃)δ ppm 0.96-1.19 (m, 1 H), 1.18-1.56 (m, 13 H), 1.56-2.27 (m, 7 H), 2.96-3.36 (m, 2 H), 3.82-4.06 (m, 1 H), 4.34 (s, 1 H), 4.42-4.83 (m, 2 H), 7.16-7.40 (m, 5 H).

### Example 89A 3-(benzylamino)-1-cyclohexylpyrrolidin-2-one hydrochloride

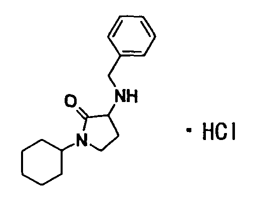

In the same manner as in Example 20A, the title compound was obtained from tert-butyl (benzyl)(1-cyclohexyl-2-oxopyrrolidin-3-yl)carbamate obtained in Example 88A.
1H NMR (300 MHz, CDCl₃) δ ppm 0.92-1.21 (m, 1 H), 1.21-1.56 (m, 4 H), 1.64 (s, 2 H), 1.81 (d like, 3 H), 2.27 (d, J=4.9 Hz, 1 H), 2.44 (d, J=7.5 Hz, 1 H), 3.10-3.29 (m, 1 H), 3.46-3.73 (m, 2 H), 3.79-4.00 (m, 1 H), 4.30-4.54 (m, 2 H), 7.29-7.50 (m, 3 H), 7.67-7.80 (m, 2 H), 10.22 (s, 1 H).

### Example 90A tert-butyl (1-cyclohexyl-2-oxopyrrolidin-3-yl)(2,6-dichlorobenzyl)carbamate

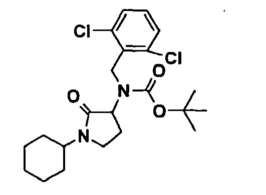

In the same manner as in Example 56A, the title compound was obtained from tert-butyl (1-cyclohexyl-2-oxopyrrolidin-3-yl)carbamate obtained in Reference Example 11A and α,2,6-trichlorotoluene.
1H NMR (300 MHz, CDCl₃) δ ppm 1.29-1.53 (m, 12 H), 1.54-1.90 (m, 7 H), 2.06-2.28 (m, 1 H), 3.02 (q, J=8.6 Hz, 1 H), 3.14-3.42 (m, 1 H), 3.43-3.73 (m, 1 H), 3.91 (t, J=10.5 Hz, 1 H), 4.79-4.94 (m, 2 H), 5.13 (d, J=13.9 Hz, 1 H), 7.12-7.23 (m, 1 H), 7.30-7.40 (m, 2 H) .

### Example 91A 1-cyclohexyl-3-[(2,6-dichlorobenzyl)amino]pyrrolidin-2-one hydrochloride

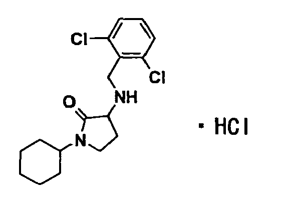

In the same manner as in Example 20A, the title compound was obtained from tert-butyl (1-cyclohexyl-2-oxopyrrolidin-3-yl)(2,6-dichlorobenzyl)carbamate obtained in Example 90A.
1H NMR (300 MHz, CDCl₃) δ ppm 1.21-1.53 (m, 4 H), 1.58-1.97 (m, 6 H), 2.32-2.56 (m, 1 H), 2.76-3.03 (m, 1 H), 3.19-3.38 (m, 1 H), 3.62 (t, J=8.6 Hz, 2 H), 3.77-3.97 (m, 1 H), 4.59-4.72 (m, 1 H), 4.76-4.90 (m, 1 H), 7.30-7.39 (m, 1 H), 7.39-7.47 (m, 2 H). Example 92A tert-butyl (1-cyclohexyl-2-oxopyrrolidin-3-yl)(methyl)carbamate

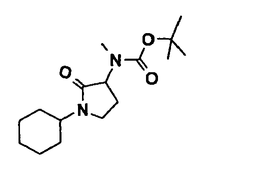

In the same manner as in Example 56A, the title compound was obtained from tert-butyl (1-cyclohexyl-2-oxopyrrolidin-3-yl)carbamate obtained in Reference Example 11A and methyl iodide.
1H NMR (300 MHz, CDCl₃) δ ppm 0.96-1.22 (m, 1 H), 1.30-1.53 (m, 4 H), 1.44-1.50 (m, 9 H), 1.59-1.86 (m, 6 H), 2.27 (d, J=7.2 Hz, 1 H), 2.78 (d like, 3 H), 3.12-3.42 (m, 2 H), 3.85-4.05 (m, 1 H), 4.45-5.01 (m, 1 H).

### Example 93A 1-cyclohexyl-3-(methylamino)pyrrolidin-2-one hydrochloride

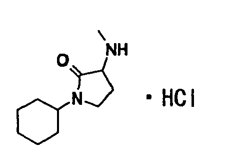

In the same manner as in Example 20A, the title compound was obtained from tert-butyl (l-cyclohexyl-2-oxopyrrolidin-3-yl)(methyl)carbamate obtained in Example 92A.
1H NMR (300 MHz, CDCl₃) δ ppm 1.01-1.56 (m, 5 H), 1.59-1.92 (m, 5 H), 2.50 (d, J=6.4 Hz, 2 H), 2.87 (s, 3 H), 3.25-3.40 (m, 1 H), 3.53-3.66 (m, 1 H), 3.79-3.98 (m, 1 H), 4.10 (t, J=8.0 Hz, 1 H), 9.78 (s, 1 H).

### Example 94A 1-cyclohexyl-3-[N-(2,6-dichlorobenzyl)-N-methylamino]pyrrolidin-2-one

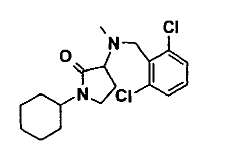

A mixture of 1-cyclohexyl-3-(methylamino)pyrrolidin-2-one hydrochloride obtained in Example 93A (0.23 g), α, 2, 6-trichlorotoluene (0.22 g), potassium carbonate (0.29 g), potassium iodide (0.18 g) and N,N-dimethylformamide (10 mL) was stirred at room temperature for 16 hr. Water was added to the reaction solution, and the mixture was extracted with ethyl acetate. The ethyl acetate layer was washed with water and saturated brine, and dried over anhydrous magnesium sulfate, and the solvent was evaporated under reduced pressure. The residue was subjected to NH-silica gel column chromatography (hexane-ethyl acetate 9:1) to give the title compound (0.32 g, 90%) as a colorless solid.
1H NMR (300 MHz, CDCl₃) δ ppm 0.97-1.21 (m, 1 H), 1.25-1.51 (m, 4 H), 1.62-1.88 (m, 5 H), 1.98-2.28 (m, 2 H), 2. 33 (s, 3 H), 3.18-3.41 (m, 2 H), 3.70 (dd, J=9.0, 7.9 Hz, 1 H), 3.90-4.05 (m, 1 H), 4.05-4.13 (m, 1 H), 4.17-4.25 (m, 1 H), 7.08-7.18 (m, 1 H), 7.27-7.33 (m, 2 H).

Example 95A 2,6-dichloro-N-(1-cyclohexyl-2-oxopyrrolidin-3-yl)-N-methylbenzamide

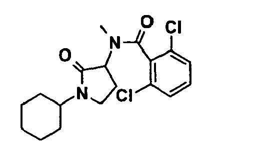

In the same manner as in Example 68A, the title compound was obtained from 1-cyclohexyl-3-(methylamino)pyrrolidin-2-one hydrochloride obtained in Example 93A and 2,6-dichlorobenzoic acid.
1H NMR (300 MHz, CDCl₃) δ ppm 1.00-1.25 (m, 1 H), 1.28-1.52 (m, 4 H), 1.62-1.89 (m, 5 H), 1.96-2.14 (m, 1 H), 2.41-2.55 (m, 1 H), 2.80 (s, 3 H), 3.27-3.50 (m, 2 H), 3.90-4.05 (m, 1 H), 5.34 (t, J=9.4 Hz, 1 H), 7.20-7.29 (m, 1 H), 7.29-7.39 (m, 2 H).

### Example 96A 3-(2-methylbenzyl)-1-[methyl(phenyl)amino]pyrrolidin-2-one

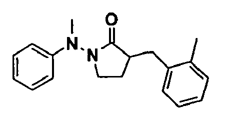

In the same manner as in Example 1A, the title compound was synthesized from 1-[methyl(phenyl)amino]pyrrolidin-2-one and α-bromo-o-xylene.
1H NMR (300 MHz, CDCl₃) δ ppm 1.72-1.94 (m, 1 H), 2.08-2.25 (m, 1 H), 2.37 (s, 3 H), 2.64-2.87 (m, 2 H), 3.15 (s, 3 H), 3.26-3.51 (m, 3 H), 6.63-6.76 (m, 2 H), 6.87 (t, J=7.3 Hz, 1 H), 7.06-7.33 (m, 6 H).

### Example 97A 3-(2,6-dichlorobenzyl)-1-[methyl(phenyl)amino]pyrrolidin-2-one

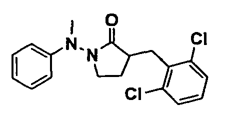

In the same manner as in Example 1A, the title compound was synthesized from 1-[methyl(phenyl)amino]pyrrolidin-2-one and α,2,6-trichlorotoluene.
1H NMR (300 MHz, CDCl₃) δ ppm 1.93-2.14 (m, 2 H), 2.92-3.11 (m, 1 H), 3.11-3.28 (m, 1 H), 3.17 (s, 3 H), 3.31-3.49 (m, 2 H), 3.56 (dd, J=13.6, 4.7 Hz, 1 H), 6.70-6.80 (m, 2 H), 6.88 (t, J=7.3 Hz, 1 H), 7.06-7.19 (m, 1 H), 7.20-7.38 (m, 4 H).

### Example 98A 3-benzyl-1-[(1R)-1-phenylethyl]pyrrolidin-2-one (less polar product)

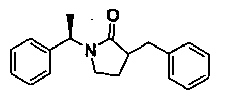

In the same manner as in Example 1A, the title compound was obtained from 1-[(1R)-1-phenylethyl] pyrrolidin-2-one and benzyl bromide.
1H NMR (300 MHz, CDCl₃) δ ppm 1.45 (d, J=7.2 Hz, 3 H), 1.61-1.77 (m, 1 H), 1.86-2.03 (m, 1 H), 2.63-2.86 (m, 3 H), 2.97-3.11 (m, 1 H), 3.15-3.30 (m, 1 H), 5.51 (q, J=7.2 Hz, 1 H), 7.02-7.55 (m, 10 H).

### Example 99A 3-benzyl-1-[(1R)-1-phenylethyl]pyrrolidin-2-one (more polar product)

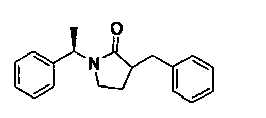

In the same manner as in Example 1A, the title compound was obtained from 1-[(1R)-1-phenylethyl]pyrrolidin-2-one and benzyl bromide.
1H NMR (300 MHz, CDCl₃) δ ppm 1.51 (d, J=7.1 Hz, 3 H), 1.55-1.70 (m, 1 H), 1.91-2.04 (m, 1 H), 2.58-2.94 (m, 3 H), 3.07-3.38 (m, 2 H), 5.50 (q, J=7.1 Hz, 1 H), 6.87-7.54 (m, 10 H).

### Example 100A 3-(2-methoxybenzyl)-1-[(1R)-1-phenylethyl]pyrrolidin-2-one (less polar product)

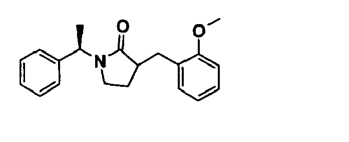

In the same manner as in Example 1A, the title compound was obtained from 1-[(1R)-1-phenylethyl]pyrrolidin-2-one and 2-methoxybenzyl chloride.
1H NMR (300 MHz, CDCl₃) δ ppm 1.50 (d, J=7.1 Hz, 3 H), 1.59-1.75 (m, 1 H), 1.76-1.96 (m, 1 H), 2.51-2.69 (m, 1 H), 2.68-2.92 (m, 2 H), 3.02-3.21 (m, 1 H), 3.26-3.43 (m, 1 H), 3.81 (s, 3 H), 5.53 (q, J=7.1 Hz, 1 H), 6.77-6.98 (m, 2 H), 7.11-7.41 (m, 7 H).

### Example 101A 3-(2-methoxybenzyl)-1-[(1R)-1-phenylethyl]pyrrolidin-2-one (more polar product)

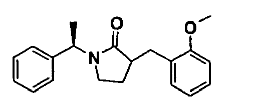

In the same manner as in Example 1A, the title compound was obtained from 1-[(1R)-1-phenylethyl]pyrrolidin-2-one and 2-methoxybenzyl chloride.
1H NMR (300 MHz, CDCl₃) δ ppm 1.51 (d, J=7.0 Hz, 3 H), 1.56-1.70 (m, 1 H), 1.77-2.06 (m, 1 H), 2.57 (m, 1 H), 2.74-2.98 (m, 2 H), 3.05-3.23 (m, 1 H), 3.29-3.48 (m, 1 H), 3.80 (s, 3 H), 5.51 (q, J=7.0 Hz, 1 H), 6.68-6.95 (m, 2 H), 7.03-7.50 (m, 7 H).

### Example 102A 3-(2, 6-difluorobenzyl)-1-[(1R)-1-phenylethyl]pyrrolidin-2-one (less polar product)

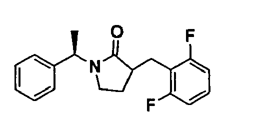

In the same manner as in Example 1A, the title compound was obtained from 1-[(1R)-1-phenylethyl]pyrrolidin-2-one and 2,6-difluorobenzyl chloride.
1H NMR (300 MHz, CDCl₃) δ ppm 1.53 (d, J=7.3 Hz, 3 H), 1.66-1.83 (m, 1 H), 1.83-2.01 (m, 1 H), 2.63-2.90 (m, 3 H), 3.16-3.39 (m, 2 H), 5.53 (q, J=7.3 Hz, 1 H), 6.75-6.96 (m, 2 H), 7.09-7.23 (m, 1 H), 7.22-7.39 (m, 5 H).

### Example 103A 3-(2,6-difluorobenzyl)-1-[(1R)-1-phenylethyl]pyrrolidin-2-one (more polar product)

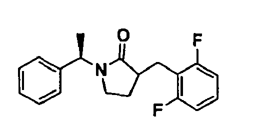

In the same manner as in Example 1A, the title compound was obtained from 1-[(1R)-1-phenylethyl]pyrrolidin-2-one and 2,6-difluorobenzyl chloride.
1H NMR (300 MHz, CDCl₃) δ ppm 1.52 (d, J=7.1 Hz, 3 H), 1.56-1.73 (m, 1 H), 1.85-2.06 (m, 1 H), 2.59-2.86 (m, 2 H), 2.86-3.00 (m, 1 H), 3.08-3.25 (m, 1 H), 3.27-3.41 (m, 1 H), 5.50 (q, J=7.1 Hz, 1 H), 6.73-6.95 (m, 2 H), 7.07-7.21 (m, 1 H), 7.22-7.43 (m, 5 H).

### Example 104A 1-[(1R)-1-phenylethyl]-3-[2-(trifluoromethyl)benzyl]pyrrolidin-2-one (less polar product)

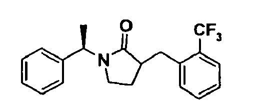

In the same manner as in Example 1A, the title compound was obtained from 1-[(1R)-1-phenylethyl]pyrrolidin-2-one and 2-trifluoromethylbenzyl chloride.
1H NMR (300 MHz, CDCl₃) δ ppm 1.47-1.70 (m, 4 H), 1.85-2.02 (m, 1 H), 2.69-2.98 (m, 3 H), 3.14-3.28 (m, 1 H), 3.39-3.54 (m, 1 H), 5.54 (q, J=7.1 Hz, 1 H), 7.20-7.38 (m, 6 H), 7.40-7.55 (m, 2 H), 7.63 (d, J=7.7 Hz, 1 H) .

### Example 105A 1-[(1R)-1-phenylethyl]-3-[2-(trifluoromethyl)benzyl]pyrrolidin-2-one (more polar product)

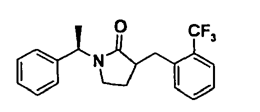

In the same manner as in Example 1A, the title compound was obtained from 1-[(1R)-1-phenylethyl]pyrrolidin-2-one and 2-trifluoromethylbenzyl chloride.
1H NMR (300 MHz, CDCl₃) δ ppm 1.42-1.57 (m, 5 H), 1.91-2.04 (m, 1 H), 2.76-2.99 (m, 3 H), 3.09-3.26 (m, 1 H), 3.41-3.56 (m, 1 H), 7.20-7.39 (m, 6 H), 7.40-7.48 (m, 2 H), 7.62 (d, J=7.9 Hz, 1 H).

### Example 106A 3-(2,6-difluorobenzyl)-1-(1-methyl-1-phenylethyl)pyrrolidin-2-one

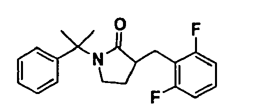

In the same manner as in Example 1A, the title compound was obtained from 1-(1-methyl-1-phenylethyl)pyrrolidin-2-one obtained in Reference Example 3A and 2,6-difluorobenzyl chloride.
1H NMR (300 MHz, CDCl₃) δ ppm 1.60-1.83 (m, 7 H), 1.87-2.03 (m, 1 H), 2.58-2.81 (m, 2 H), 3.15-3.40 (m, 3 H), 6.76-6.95 (m, 2 H), 7.05-7.38 (m, 6 H).

### Example 107A 1-(1-methyl-1-phenylethyl)-3-[2-(trifluoromethyl)benzyl]pyrrolidin-2-one

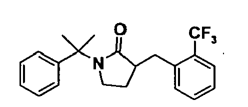

In the same manner as in Example 1A, the title compound was obtained from 1-(1-methyl-1-phenylethyl)pyrrolidin-2-one obtained in Reference Example 3A and 2-trifluoromethylbenzyl chloride.
1H NMR (300 MHz, CDCl₃) δ ppm 1.49-1.69 (m, 1 H), 1.74 (s, 3 H), 1.78 (s, 3 H), 1.87-2.04 (m, 1 H), 2.68-2.82 (m, 1 H), 2.82-2.97 (m, 1 H), 3.22-3.32 (m, 2 H), 3.32-3.44 (m, 1 H), 7.18-7.28 (m, 2 H), 7.28-7.35 (m, 4 H), 7.36-7.50 (m, 2 H), 7.57-7.67 (m, 1 H).

### Example 108A 1-cyclohexyl-3-(2,6-difluorobenzyl)pyrrolidin-2-one

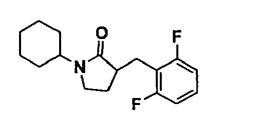

In the same manner as in Example 1A, the title compound was obtained from 1-cyclohexylpyrrolidin-2-one and 2,6-difluorobenzyl chloride.
1H NMR (300 MHz, CDCl₃) δ ppm 0.97-1.18 (m, 1 H), 1.23-1.51 (m, 4 H), 1.60-1.86 (m, 6 H), 1.90-2.08 (m, 1 H), 2.59-2.84 (m, 2 H), 3.07-3.35 (m, 3 H), 3.85-4.07 (m, 1 H), 6.77-6.96 (m, 2 H), 7.07-7.23 (m, 1 H).

### Example 109A 1-cyclohexyl-3-[2-(trifluoromethyl)benzyl]pyrrolidin-2-one

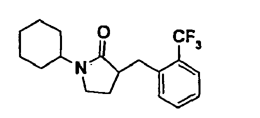

In the same manner as in Example 1A, the title compound was obtained from 1-cyclohexylpyrrolidin-2-one and 2-trifluoromethylbenzyl chloride.
1H NMR (300 MHz, CDCl₃) δ ppm 0.95-1.19 (m, 1 H), 1.23-1.47 (m, 4 H), 1.56-1.86 (m, 6 H), 1.92-2.08 (m, 1 H), 2.69-2.94 (m, 2 H), 3.10-3.28 (m, 2 H), 3.36-3.48 (m, 1 H), 3.88-4.04 (m, 1 H), 7.28-7.35 (m, 1 H), 7.40-7.51 (m, 2 H), 7.59-7.66 (m, 1 H). Example 110A 3-[4-(chloromethyl)benzoyl]-1-cyclohexylpyrrolidin-2-one

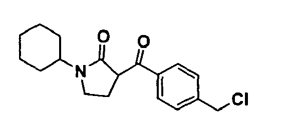

In the same manner as in Example 1A, the title compound was obtained from 1-cyclohexylpyrrolidin-2-one and methyl 4-(chloromethyl)benzoate,
1H NMR (300 MHz, CDCl₃) δ ppm 1.04-1.47 (m, 6 H), 1.62-1.87 (m, 6 H), 2.13-2.30 (m, 1 H), 2.54-2.68 (m, 1 H), 3.33-3.44 (m, 1 H), 3.50-3.62 (m, 1 H), 3.80-3.94 (m, 1 H), 4.45 (dd, J=9.2, 4.9 Hz, 1 H), 4.62 (s, 2 H), 7.50 (d, J=8.5 Hz, 1 H), 8.14 (d, J=8.5 Hz, 1 H).

### Example 111A 1-cyclohexyl-3-(2-methoxybenzyl)pyrrolidin-2-one

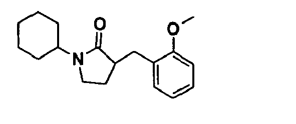

In the same manner as in Example 1A, the title compound was obtained from 1-cyclohexylpyrrolidin-2-one and 2-methoxybenzyl chloride.
1H NMR (300 MHz, CDCl₃) δ ppm 0.97-1.17 (m, 1 H), 1.27-1.49 (m, 4 H), 1.58-1.84 (m, 6 H), 1.86-2.00 (m, 1 H), 2.57 (dd, J=13.6, 10.1 Hz, 1 H), 2.70-2.86 (m, 1 H), 3.11-3.21 (m, 2 H), 3.31 (dd, J=13.6, 4.1 Hz, 1 H), 3.81 (s, 3 H), 3.87-4.04 (m, 1 H), 6.77-6.93 (m, 2 H), 7.11-7.23 (m, 2 H).

### Example 112A 1-cyclohexyl-3-(3-methoxybenzyl)pyrrolidin-2-one

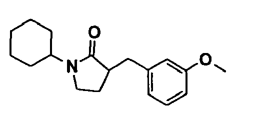

In the same manner as in Example 1A, the title compound was obtained from 1-cyclohexylpyrrolidin-2-one and 3-methoxybenzyl chloride.
1H NMR (300 MHz, CDCl₃) δ ppm 0.96-1.20 (m, 1 H), 1.21-1.50 (m, 4 H), 1.59-1.87 (m, 6 H), 1.93-2.13 (m, 1 H), 2.53-2.84 (m, 2 H), 3.04-3.27 (m, 3 H), 3.79 (s, 3 H), 3.88-4.05 (m, 1 H), 6.68-6.84 (m, 3 H), 7.12-7.25 (m, 1 H).

### Example 113A 1-cyclohexyl-3-(4-methoxybenzyl)pyrrolidin-2-one

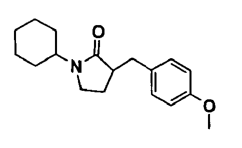

In the same manner as in Example 1A, the title compound was obtained from 1-cyclohexylpyrrolidin-2-one and 4-methoxybenzyl chloride.
1H NMR (300 MHz, CDCl₃) δ ppm 0.96-1.49 (m, 5 H), 1.54-1.86 (m, 6 H), 1.89-2.13 (m, 1 H), 2.58-2.78 (m, 2 H), 3.00-3.22 (m, 3 H), 3.78 (s, 3 H), 3.85-4.04 (m, 1 H), 6.82 (d, J=8.7 Hz, 2 H), 7.12 (d, J=8.7 Hz, 2 H).

### Example 114A 3-(4-bromobenzyl)-1-cyclohexylpyrrolidin-2-one

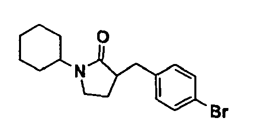

In the same manner as in Example 1A, the title compound was obtained from 1-cyclohexylpyrrolidin-2-one and 4-bromobenzyl bromide.
1H NMR (300 MHz, CDCl₃) δ ppm 0.99-1.18 (m, 1 H), 1.18-1.50 (m, 4 H), 1.50-1.87 (m, 6 H), 1.89-2.20 (m, 1 H), 2.53-2.83 (m, 2 H), 2.99-3.36 (m, 3 H), 3.75-4.10 (m, 1 H), 7.08 (d, J=8.3 Hz, 2 H), 7.39 (d, J=8.3 Hz, 2 H).

### Example 115A methyl 4-[(1-cyclohexyl-2-oxopyrrolidin-3-yl)methyl]benzoate

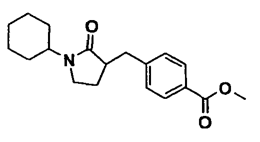

A mixture of 3-(4-bromobenzyl)-1-cyclohexylpyrrolidin-2-one obtained in Example 114A (1.37 g), palladium acetate (45 mg), 1,1'-bis(diphenylphosphino)ferrocene (0.11 g), triethylamine (0.62 mL), methanol (32 mL) and tetrahydrofuran (32 mL) was stirred at 100°C for 8 hr under carbon monoxide atmosphere (0.2 Mpa). The insoluble substance was removed, water was added to the filtrate, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 9:1-3:2) to give the title compound (0.03 g, 2%) as a brown oil.
1H NMR (300 MHz, CDCl₃) δ ppm 0.91-1.51 (m, 5 H), 1.53-1.87 (m, 6 H), 1.88-2.19 (m, 1 H), 2.63-2.89 (m, 2 H), 2.98-3.39 (m, 3 H), 3.74-4.10 (m, 4 H), 7.28 (d, J=7.8 Hz, 2 H), 7.95 (d, J=7.8 Hz, 2 H).

Example 116A 1-cyclohexyl-3-[4-(piperidin-1-ylmethyl)benzoyl]pyrrolidin-2-one

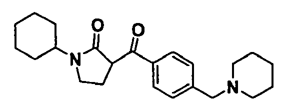

A mixture of 3-[4-(chloromethyl)benzoyl]-1-cyclohexylpyrrolidin-2-one obtained in Example 110A (0.17 g), piperidine (0.18 mL), potassium iodide (88 mg) and ethyl acetate (7 mL) was stirred at room temperature for 16 hr. The reaction mixture was extracted with water and ethyl acetate, and the ethyl acetate layer was washed with saturated brine, and dried over anhydrous magnesium sulfate. The solvent was evaporated under reduced pressure, and the residue was subjected to NH-silica gel column chromatography (hexane-ethyl acetate 9:1-3:2) to give the title compound (0.14 g, 72%) as an orange powder.
1H NMR (300 MHz, CDCl₃)δ ppm 1.03-1.50 (m, 8 H), 1.50-1.89 (m, 8 H), 2.12-2.28 (m, 1 H), 2.30-2.43 (m, 4 H), 2.50-2.69 (m, 1 H), 3.29-3.46 (m, 1 H), 3.47-3.63 (m, 3 H), 3.81-3.96 (m, 1 H), 4.46 (dd, J=9.1, 4.8 Hz, 1 H), 7.44 (d, J=8.3 Hz, 2 H), 8.07 (d, J=8.3 Hz, 2 H).

### Example 117A 1-(2,6-dichlorobenzyl)-3-(2-methoxybenzyl)pyrrolidin-2-one

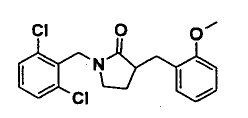

In the same manner as in Example 1A, the title compound was obtained from 1-(2,6-dichlorobenzyl)pyrrolidin-2-one obtained in Reference Example 2A and 2-methoxybenzyl chloride.
1H NMR (300 MHz, CDCl₃) δ ppm 1.55-1.75 (m, 1 H), 1.79-1.95 (m, 1 H), 2.53-2.66 (m, 1 H), 2.74-2.90 (m, 1 H), 2.92-3.07 (m, 2 H), 3.30-3.42 (m, 1 H), 3.80 (s, 3 H), 4.75-4.90 (m, 2 H), 6.80-6.93 (m, 2 H), 7.09-7.40 (m, 5 H).

### Example 118A 1-(2,6-dichlorobenzyl)-3-(1-hydroxycyclohexyl)pyrrolidin-2-one

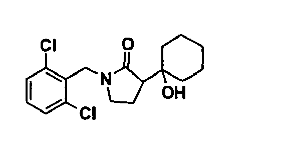

To a mixture of 1-(2,6-dichlorobenzyl)pyrrolidin-2-one obtained in Reference Example 2A (0.72 g) and tetrahydrofuran (20 mL) was added lithium bis(trimethylsilyl)amide (1.1 M tetrahydrofuran solution, 2.95 mL) at -78°C under nitrogen atmosphere, and the mixture was stirred at -78°C for 1 hr. Boron trifluoride ether complex (0.37 mL) and cyclohexanone (0.40 mL) were added to the reaction mixture at -78°C, and the mixture was stirred at -78°C for 3 hr. Saturated aqueous ammonium chloride and water was added to the reaction mixture, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 85:15) to give the title compound (0.36 g, 35%) as a colorless powder.
1H NMR (300 MHz, CDCl₃) δ ppm 0.96-1.21 (m, 1 H), 1.23-1.42 (m, 2 H), 1.42-1.88 (m, 8 H), 1.92-2.10 (m, 1 H), 2.59 (t, J=9.8 Hz, 1 H), 2.97-3.19 (m, 2 H), 4.41-4.51 (m, 1 H), 4.66 (d, J=14.0 Hz, 1 H), 4.94 (d, J=14.0 Hz, 1 H), 7.14-7.40 (m, 3 H).

### Example 119A 1-cyclohexyl-3-(2-naphthylmethyl)pyrrolidin-2-one

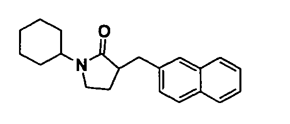

In the same manner as in Example 1A, the title compound was obtained from 1-cyclohexylpyrrolidin-2-one and 2-chloromethylnaphthalene.
1H NMR (300 MHz, CDCl₃) δ ppm 0.93-1.49 (m, 5 H), 1.56-1.88 (m, 8 H), 1.92-2.13 (m, 1 H), 2.73-2.91 (m, 2 H), 3.30-3.47 (m, 1 H), 3.87-4.07 (m, 1 H), 7.32-7.53 (m, 3 H), 7.64 (brs, 1 H), 7.71-7.88 (m, 3 H).

### Example 120A 1-cyclohexyl-3-(1-naphthylmethyl)pyrrolidin-2-one

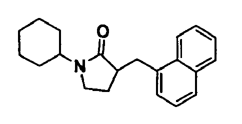

In the same manner as in Example 1A, the title compound was obtained from 1-cyclohexylpyrrolidin-2-one and 1-chloromethylnaphthalene.
1H NMR (300 MHz, CDCl₃) δ ppm 1.19-1.52 (m, 5 H), 1.62-2.02 (m, 7 H), 2.76-2.97 (m, 2 H), 3.04-3.29 (m, 2 H), 3.84-4.06 (m, 2 H), 7.30-7.43 (m, 2 H), 7.43-7.59 (m, 2 H), 7.74 (d, J=7.9 Hz, 1 H), 7.81-7.90 (m, 1 H), 8.15 (d, J=8.3 Hz, 1 H).

### Example 121A 3-(2-chlorobenzyl)-1-cyclohexylpyrrolidin-2-one

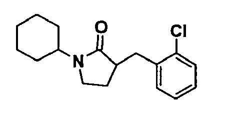

In the same manner as in Example 1A, the title compound was obtained from 1-cyclohexylpyrrolidin-2-one and 2-chlorobenzyl bromide.
1H NMR (300 MHz, CDCl₃) δ ppm 0.97-1.50 (m, 5 H), 1.54-1.86 (m, 6 H), 1.88-2.09 (m, 1 H), 2.68-2.92 (m, 2 H), 3.07-3.27 (m, 2 H), 3.29-3.49 (m, 1 H), 3.84-4.04 (m, 1 H), 7.07-7.43 (m, 4 H).

Example 122A 3-(3-chlorobenzyl)-1-cyclohexylpyrrolidin-2-one

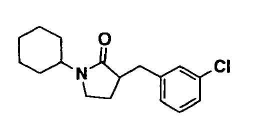

In the same manner as in Example 1A, the title compound was obtained from 1-cyclohexylpyrrolidin-2-one and 3-chlorobenzyl bromide.
1H NMR (300 MHz, CDCl₃) δ ppm 1.20-1.49 (m, 4 H), 1.53-1.86 (m, 6 H), 1.95-2.11 (m, 1 H), 2.57-2.79 (m, 2 H), 3.02-3.24 (m, 3 H), 3.86-4.03 (m, 1 H), 7.05-7.13 (m, 1 H), 7.16-7.24 (m, 3 H), 7.26 (s, 1 H).

### Example 123A 3-(4-chlorobenzyl)-1-cyclohexylpyrrolidin-2-one

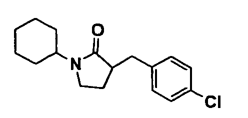

In the same manner as in Example 1A, the title compound was obtained from 1-cyclohexylpyrrolidin-2-one and 4-chlorobenzyl bromide.
1H NMR (300 MHz, CDCl₃) δ ppm 0.83-1.18 (m, 1 H), 1.19-1.49 (m, 5 H), 1.53-1.86 (m, 5 H), 1.90-2.11 (m, 1 H), 2.59-2.75 (m, 2 H), 3.01-3.27 (m, 3 H), 3.81-4.03 (m, 1 H), 7.09-7.18 (m, 2 H), 7.20-7.28 (m, 2 H).

### Example 124A 1-cyclohexyl-3-(3-methylbenzyl)pyrrolidin-2-one

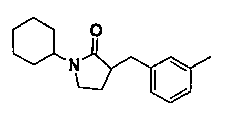

In the same manner as in Example 1A, the title compound was obtained from 1-cyclohexylpyrrolidin-2-one and 3-methylbenzyl bromide.
1H NMR (300 MHz, CDCl₃) δ ppm 1.21-1.50 (m, 5 H), 1.52-1.85 (m, 6 H), 1.91-2.11 (m, 1 H), 2.32 (s, 3 H), 2.54-2.79 (m, 2 H), 2.99-3.24 (m, 3 H), 3.85-4.03 (m, 1 H), 6.92-7.07 (m, 3 H), 7.09-7.22 (m, 1 H).

### Example 125A 1-cyclohexyl-3-(4-methylbenzyl)pyrrolidin-2-one

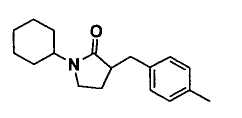

In the same manner as in Example 1A, the title compound was obtained from 1-cyclohexylpyrrolidin-2-one and 4-methylbenzyl bromide.
1H NMR (300 MHz, CDCl₃) δ ppm 1.15-1.50 (m, 4 H), 1.53-1.85 (m, 7 H), 1.88-2.11 (m, 1 H), 2.31 (s, 3 H), 2.43-2.82 (m, 2 H), 2.98-3.28 (m, 3 H), 3.82-4.03 (m, 1 H), 7.01-7.16 (m, 4 H).

### Example 126A 1-cyclohexyl-3-[4-(methylthio)benzyl]pyrrolidin-2-one

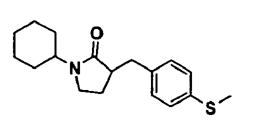

In the same manner as in Example 1A, the title compound was obtained from 1-cyclohexylpyrrolidin-2-one and 4-(methylthio)benzyl chloride.
1H NMR (300 MHz, CDCl₃) δ ppm 1.23-1.46 (m, 6 H), 1.56-1.83 (m, 6 H), 1.92-2.10 (m, 1 H), 2.46 (s, 3 H), 2.57-2.75 (m, 2 H), 3.03-3.23 (m, 3 H), 7.08-7.23 (m, 4 H).

### Example 127A 1-cyclohexyl-3-[4-(methylsulfinyl)benzyl]pyrrolidin-2-one

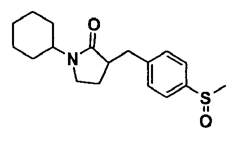

To a mixture of 1-cyclohexyl-3-[4-(methylthio)benzyl]pyrrolidin-2-one obtained in Example 126A (0.31 g) and tetrahydrofuran (5 mL) was added a mixture of Oxone (trade name)-persulfate compound (0.34 g) and water (5 mL) under ice-cooling, and the mixture was stirred for 30 min under ice-cooling, and then at room temperature for 1 hr. Aqueous sodium hydrogen sulfite and water were added to the reaction mixture, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (ethyl acetate-ethyl acetate-methanol 85:15) to give the title compound (0.22 g, 68%) as a colorless solid.
1H NMR (300 MHz, CDCl₃) δ ppm 0.98-1.48 (m, 5 H), 1.54-1.86 (m, 6 H), 1.95-2.13 (m, 1 H), 2.72 (s, 3 H), 2.74-2.86 (m, 2 H), 3.02-3.33 (m, 3 H), 3.78-4.04 (m, 1 H), 7.39 (d, J=8.2 Hz, 2 H), 7.58 (d, J=8.2 Hz, 2 H).

Example 128A 1-cyclohexyl-3-[4-(methylsulfonyl)benzyl]pyrrolidin-2-one

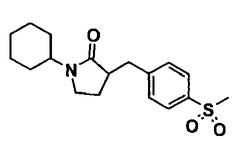

In the same manner as in Example 127A, the title compound was obtained from 1-cyclohexyl-3-[4-(methylsulfinyl)benzyl]pyrrolidin-2-one obtained in Example 127A.
1H NMR (300 MHz, CDCl₃) δ ppm 1.01-1.49 (m, 5 H), 1.54-1.84 (m, 6 H), 1.92-2.14 (m, 1 H), 2.64-2.89 (m, 2 H), 3.05 (s, 3 H), 3.10-3.37 (m, 3 H), 3.81-4.02 (m, 1 H), 7.42 (d, J=8.3 Hz, 2 H), 7.80-7.92 (m, 2 H).

### Example 129A 4-[(1-cyclohexyl-2-oxopyrrolidin-3-yl)methyl]benzoic acid

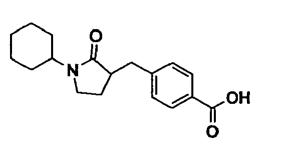

A mixture of methyl 4-[(1-cyclohexyl-2-oxopyrrolidin-3-yl)methyl]benzoate obtained in Example 115A (1.30 g), ethanol (30 mL) and 1N aqueous sodium hydroxide (6.2 mL) was heated under reflux for 2 hr. The reaction mixture was concentrated, and the residue was partitioned between ethyl acetate-water. The aqueous layer was washed with ethyl acetate, 1N hydrochloric acid (6.5 mL) was added, and the mixture was extracted with ethyl acetate. The ethyl acetate layer was washed with saturated brine, and dried over anhydrous magnesium sulfate. The solvent was evaporated under reduced pressure to give the title compound (1.30 g, 100%) as a colorless powder.
1H NMR (300 MHz, CDCl₃) δ ppm 0.95-1.51 (m, 5 H), 1.50-1.89 (m, 7 H), 1.91-2.15 (m, 1 H), 2.65-2.92 (m, 2 H), 3.02-3.41 (m, 3 H), 3.82-4.10 (m, 1 H), 7.32 (d, J=8.2 Hz, 2 H), 8.02 (d, J=8.2 Hz, 2 H).

### Example 130A 1-(2,6-dichlorobenzyl)-3-(4-methoxybenzyl)pyrrolidin-2-one

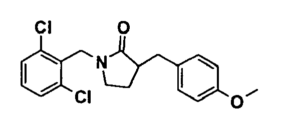

In the same manner as in Example 1A, the title compound was obtained from 1-(2,6-dichlorobenzyl)pyrrolidin-2-one obtained in Reference Example 2A and 4-methoxybenzyl chloride. 1H NMR (300 MHz, CDCl₃) δ ppm 1.50-1.76 (m, 1 H), 1.85-2.03 (m, 1 H), 2.52-2.80 (m, 2 H), 2.84-3.09 (m, 2 H), 3.10-3.30 (m, 1 H), 3.78 (s, 3 H), 4.69-4.94 (m, 2 H), 6.72-6.93 (m, 2 H), 7.02-7.44 (m, 5 H).

### Example 131A 1-(2,6-dichlorobenzyl)-3,3-bis(4-methoxybenzyl)pyrrolidin-2-one

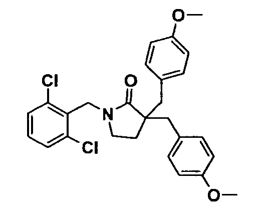

In the same manner as in Example 1A, the title compound was obtained from 1-(2,6-dichlorobenzyl)pyrrolidin-2-one obtained in Reference Example 2A and 4-methoxybenzyl chloride.
1H NMR (300 MHz, CDCl₃) δ ppm 1.76 (t, J=7.1 Hz, 2 H), 2.12 (t, J=7.1 Hz, 2 H), 2.57 (d, J=13.5 Hz, 2 H), 3.14 (d, J=13.5 Hz, 2 H), 3.65-3.88 (m, 6 H), 4.55 (s, 2 H), 6.71-6.87 (m, 4 H), 6.99-7.25 (m, 7 H).

### Example 132A 3-benzyl-1-cyclohexylpyrrolidin-2-one

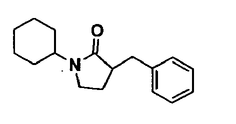

In the same manner as in Example 1A, the title compound was obtained from 1-cyclohexylpyrrolidin-2-one and benzyl bromide.
1H NMR (300 MHz, CDCl₃) δ ppm 1.00-1.50 (m, 5 H), 1.54-1.84 (m, 6 H), 1.89-2.10 (m, 1 H), 2.51-2.81 (m, 2 H), 2.94-3.39 (m, 3 H), 3.80-4.11 (m, 1 H), 6.95-7.57 (m, 5 H).

### Example 133A 4-[(1-cyclohexyl-2-oxopyrrolidin-3-yl)methyl]-N-methylbenzamide

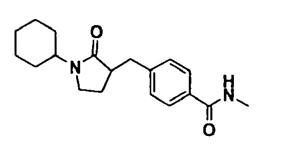

In the same manner as in Example 70A, the title compound was obtained from 4-[(1-cyclohexyl-2-oxopyrrolidin-3-yl)methyl]benzoic acid obtained in Example 129A and methylamine (2.0 M tetrahydrofuran solution).
1H NMR (300 MHz, CDCl₃) δ ppm 0.95-1.48 (m, 5 H), 1.53-1.84 (m, 6 H), 1.93-2.07 (m, 1 H), 2.64-2.84 (m, 2 H), 3.01 (d, J=4.9 Hz, 3 H), 3.05-3.31 (m, 3 H), 3.81-4.06 (m, 1 H), 6.18 (s, 1 H), 7.10-7.34 (d, J=8.2 Hz, 2 H), 7.68 (d, J=8.2 Hz, 2 H).

### Example 134A 4-[(1-cyclohexyl-2-oxopyrrolidin-3-yl)methyl]-N,N-dimethylbenzamide

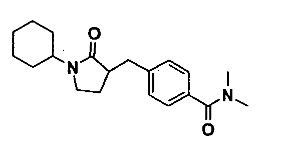

In the same manner as in Example 70A, the title compound was obtained from 4-[(1-cyclohexyl-2-oxopyrrolidin-3-yl)methyl]benzoic acid obtained in Example 129A and dimethylamine (2.0 M tetrahydrofuran solution).
1H NMR (300 MHz, CDCl₃) δ ppm 0.99-1.48 (m, 5 H), 1.54-1.84 (m, 6 H), 1.93-2.08 (m, 1 H), 2.64-2.80 (m, 2 H), 2.93-3.27 (m, 9 H), 3.84-4.01 (m, 1 H), 7.17-7.28 (m, 2 H), 7.28-7.41 (m, 2 H).

Example 135A 4-[(1-cyclohexyl-2-oxopyrrolidin-3-yl)methyl]-N-phenylbenzamide

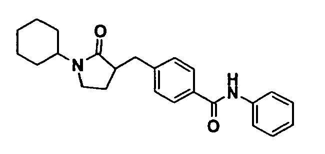

In the same manner as in Example 70A, the title compound was obtained from 4-[(1-cyclohexyl-2-oxopyrrolidin-3-yl)methyl]benzoic acid obtained in Example 129A and aniline.
1H NMR (300 MHz, CDCl₃) δ ppm 1.03-1.42 (m, 5 H), 1.60-1.85 (m, 6 H), 1.95-2.10 (m, 1 H), 2.67-2.84 (m, 2 H), 3.06-3.30 (m, 3 H), 3.84-4.01 (m, 1 H), 7.11-7.19 (m, 1 H), 7.30-7.43 (m, 4 H), 7.61-7.68 (m, 2 H), 7.80 (d, J=8.3 Hz, 2 H), 7.85 (s, 1 H). Example 136A N-benzyl-4-[(1-cyclohexyl-2-oxopyrrolidin-3-yl)methyl]benzamide

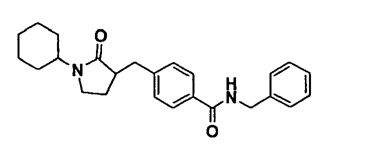

In the same manner as in Example 70A, the title compound was obtained from 4-[(1-cyclohexyl-2-oxopyrrolidin-3-yl)methyl]benzoic acid obtained in Example 129A and benzylamine.
1H NMR (300 MHz, CDCl₃) δ ppm 0.98-1.47 (m, 5 H), 1.48-1.84 (m, 6 H), 1.90-2.10 (m, 1 H), 2.58-2.82 (m, 2 H), 2.96-3.34 (m, 3 H), 3.81-4.05 (m, 1 H), 4.65 (d, J=5.7 Hz, 2 H), 6.30-6.47 (m, 1 H), 7.14-7.44 (m, 7 H), 7.71 (d, J=8.3 Hz, 2 H).

### Example 137A 3-(2,6-dichlorobenzyl)-1-(1-phenylcyclopropyl)pyrrolidin-2-one

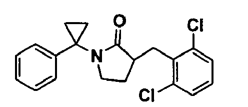

In the same manner as in Example 1A, the title compound was obtained from 1-(1-phenylcyclopropyl)pyrrolidin-2-one obtained in Reference Example 4A and α,2,6-trichlorotoluene.
1H NMR (300 MHz, CDCl₃) δ ppm 1.10-1.54 (m, 4 H), 1.78-1.99 (m, 2 H), 2.81-3.15 (m, 2 H), 3.18-3.60 (m, 3 H), 7.02-7.15 (m, 1 H), 7.16-7.40 (m, 7 H).

### Example 138A 3-(2-methoxybenzyl)-1-(1-phenylcyclopropyl)pyrrolidin-2-one

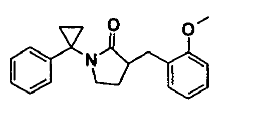

In the same manner as in Example 1A, the title compound was obtained from 1-(1-phenylcyclopropyl)pyrrolidin-2-one obtained in Reference Example 4A and 2-methoxybenzyl chloride.
1H NMR (300 MHz, CDCl₃) δ ppm 1.15-1.49 (m, 4 H), 1.62-1.83 (m, 1 H), 1.84-2.02 (m, 1 H), 2.51-2.72 (m, 1 H), 2.71-2.93 (m, 1 H), 3.12-3.44 (m, 3 H), 3.81 (s, 3 H), 6.78-6.94 (m, 2 H), 7.09-7.45 (m, 7 H).

### Example 139A 3-(2-methoxybenzyl)-1-(1-methyl-1-phenylethyl)pyrrolidin-2-one

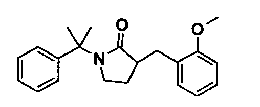

In the same manner as in Example 1A, the title compound was obtained from 1-(1-methyl-1-phenylethyl)pyrrolidin-2-one obtained in Reference Example 3A and 2-methoxybenzyl chloride.
1H NMR (300 MHz, CDCl₃) δ ppm 1.59-1.70 (m, 1 H), 1.74 (s, 3 H), 1.75 (s, 3 H), 1.78-2.02 (m, 1 H), 2.49-2.64 (m, 1 H), 2.67-2.89 (m, 1 H), 3.14-3. 38 (m, 3 H), 3. 80 (s, 3 H), 6.77-6.97 (m, 2 H), 7.09-7.27 (m, 3 H), 7.26-7.38 (m, 4 H).

### Example 140A 1-cyclohexyl-3-(2-hydroxybenzyl)pyrrolidin-2-one

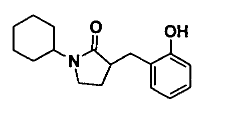

A mixture of 1-cyclohexyl-3-(2-methoxybenzyl)pyrrolidin-2-one obtained in Example 111A (40 mg) and dichloromethane (5 mL) was cooled to -78°C, boron tribromide (1.0 M dichloromethane solution, 0.42 mL) was added, and the mixture was stirred at -78°C for 15 min, and then at 0°C for 1 hr. Saturated aqueous ammonium chloride was added to the reaction mixture, and the mixture was adjusted with 0.1N aqueous sodium hydroxide solution to pH 6, and extracted with dichloromethane. The dichloromethane layer was washed with saturated brine, and dried over anhydrous magnesium sulfate. The solvent was evaporated under reduced pressure, and the residue was subjected to silica gel column chromatography (hexane-ethyl acetate 7:3) to give the title compound (0.04 g, 100%) as a colorless powder.
1H NMR (300 MHz, CDCl₃) δ ppm 0.97-1.44 (m, 6 H), 1.49-1.96 (m, 5 H), 2.14-2.36 (m, 1 H), 2.72-2.92 (m, 2 H), 2.96-3.11 (m, 1 H), 3.15-3.35 (m, 2 H), 3.78-4.00 (m, 1 H), 6.73-6.85 (m, 1 H), 6.89-7.06 (m, 2 H), 7.06-7.20 (m, 1 H), 9.21 (s, 1 H).

### Example 141A 1-cyclohexyl-3-[2-(trifluoromethoxy)benzyl]pyrrolidin-2-one

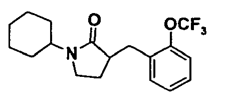

In the same manner as in Example 1A, the title compound was obtained from 1-cyclohexylpyrrolidin-2-one and 2-trifluoromethoxybenzyl bromide.
1H NMR (300 MHz, CDCl₃) δ ppm 0.98-1.50 (m, 5 H), 1.54-1.73 (m, 4 H), 1.73-1.85 (m, 2 H), 1.92-2.09 (m, 1H), 2.64-2.82 (m, 2 H), 3.09-3.21 (m, 2 H), 3.21-3.35 (m, 1 H), 3.87-4.03 (m, 1 H), 7.17-7.25 (m, 3 H), 7.29-7.40 (m, 1 H).

### Example 142A 1-cyclohexyl-3-(2-ethoxybenzyl)pyrrolidin-2-one

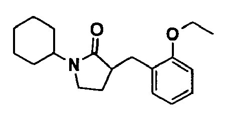

To a mixture of 1-cyclohexyl-3-(2-hydroxybenzyl)pyrrolidin-2-one obtained in Example 140A (0.25 g) and N,N-dimethylformamide (5 mL) was added 60% sodium hydride (27 mg), and the mixture was stirred at room temperature for 5 min. Ethyl iodide (69 µL) was added to the reaction solution, and the mixture was stirred at room temperature for 2 days. Water was added to the reaction solution, and the mixture was extracted with ethyl acetate. The ethyl acetate layer was washed with saturated brine, and dried over anhydrous magnesium sulfate, and the solvent was evaporated under reduced pressure. The residue was subjected to silica gel column chromatography (hexane-ethyl acetate 3:1) to give the title compound (0.11 g, 39%) as a colorless solid.
1H NMR (300 MHz, CDCl₃) δ ppm 1.01-1.21 (m, 1 H), 1.20-1.50 (m, 7 H), 1.54-1.84 (m, 6 H), 1.84-2.05 (m, 1 H), 2.52-2.69 (m, 1 H), 2.68-2.89 (m, 1 H), 3.07-3.21 (m, 2 H),3.21-3.38 (m, 1 H), 3.79-4.15 (m, 3 H), 6.69-6.95 (m, 2 H), 7.08-7.22 (m, 2 H). Example 143A 1-cyclohexyl-3-[2-(cyclopropylmethoxy)benzyl]pyrrolidin-2-one

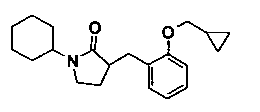

In the same manner as in Example 142A, the title compound was obtained from 1-cyclohexyl-3-(2-hydroxybenzyl)pyrrolidin-2-one obtained in Example 140A and cyclopropylmethyl bromide.
1H NMR (300 MHz, CDCl₃) δ ppm 0.18-0.42 (m, 2 H), 0.53-0.73 (m, 2 H), 0.85-1.19 (m, 1 H), 1.19-1.49 (m, 5 H), 1.55-1.84 (m, 6 H), 1.87-2.06 (m, 1 H), 2.52-2.93 (m, 2 H), 2.97-3.40 (m, 3 H), 3.81 (d, J=6.8 Hz, 2 H), 3.88-4.13 (m, 1H), 6.70-7.00 (m, 2 H), 7.00-7.24 (m, 2 H).

### Example 144A 3-(2-bromobenzyl)-1-cyclohexylpyrrolidin-2-one

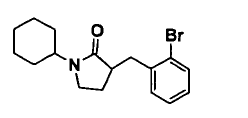

In the same manner as in Example 1A, the title compound was obtained from 1-cyclohexylpyrrolidin-2-one and 2-bromobenzyl bromide.
1H NMR (300 MHz, CDCl₃)δ ppm 1.02-1.18 (m, 1 H), 1.20-1.54 (m, 4 H), 1.61-1.88 (m, 6 H), 1.90-2.09 (m, 1 H), 2.70-2.97 (m, 2 H), 3.05-3.30 (m, 2 H), 3.33-3.51 (m, 1 H), 3.86-4.09 (m, 1 H), 6.97-7.15 (m, 1 H), 7.19-7.36 (m, 2 H), 7.45-7.66 (m, 1 H). Example 145A N-benzyl-4-[(1-cyclohexyl-2-oxopyrrolidin-3-yl)methyl]-N-methylbenzamide

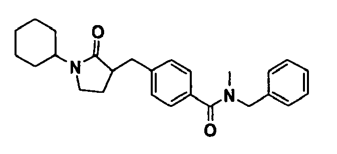

In the same manner as in Example 70A, the title compound was obtained from 4-[(1-cyclohexyl-2-oxopyrrolidin-3-yl)methyl]benzoic acid obtained in Example 129A and N-methylbenzylamine.
1H NMR (300 MHz, CDCl₃) δ ppm 1.15-1.47 (m, 5 H), 1.63 (s, 6 H), 1.89-2.17 (m, 1 H), 2.62-3.36 (m, 8 H), 3.78-4.06 (m, 1 H), 4.41-4.85 (m, 2 H), 7.13-7.41 (m, 9 H).

### Example 146A 4-[(1-cyclohexyl-2-oxopyrrolidin-3-yl)methyl]-N-(2-furylmethyl)benzamide

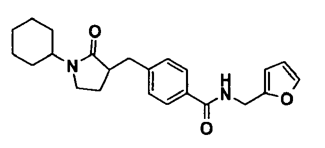

In the same manner as in Example 70A, the title compound was obtained from 4-[(1-cyclohexyl-2-oxopyrrolidin-3-yl)methyl]benzoic acid obtained in Example 129A and furfurylamine.
1H NMR (300 MHz, CDCl₃) δ ppm 1.29-1.47 (m, 5 H), 1.51-1.83 (m, 7 H), 1.90-2.03 (m, 1 H), 2.66-2.81 (m, 2 H), 3.01-3.28 (m, 3 H), 3.82-4.04 (m, 1 H), 4.64 (d, J=5.5 Hz, 2 H), 6.26-6.43 (m, 3 H), 7.29 (s, 1 H), 7.27-7.31 (m, 1 H), 7.34-7.42 (m, 1 H), 7.70 (d, J=8.3 Hz, 1 H).

### Example 147A 1-cyclohexyl-3-(2,6-dimethoxybenzoyl)pyrrolidin-2-one

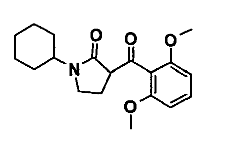

In the same manner as in Example 1A, the title compound was obtained from 1-cyclohexylpyrrolidin-2-one and methyl 2,6-dimethoxybenzoate.
1H NMR (300 MHz, CDCl₃) δ ppm 1.21-1.45 (m, 4 H), 1.58-1.83 (m, 6 H), 2.06-2.20 (m, 1 H), 2.52-2.66 (m, 1 H), 3.22-3.50 (m, 2 H), 3.73-3.94 (m, 7 H), 4.05 (dd, J=9.3, 4.6 Hz, 1 H), 6.51-6.61 (m, 2 H), 7.24-7.32 (m, 1 H).

### Example 148A 1-cyclohexyl-3-(2,6-dimethoxybenzyl)pyrrolidin-2-one

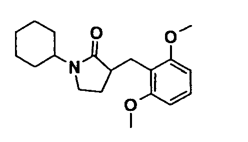

To a mixture of 1-cyclohexyl-3-(2, 6-dimethoxybenzoyl)pyrrolidin-2-one obtained in Example 147A (76 mg) and dichloromethane (5 mL) was added dropwise trifluoromethanesulfonic acid (81 µL) under ice-cooling, and then triethylsilane (0.10 mL) was added dropwise. The reaction mixture was added at room temperature for 2 hr, saturated sodium hydrogencarbonate was added, and the mixture was extracted with dichloromethane. The dichloromethane layer was washed with saturated brine, and dried over anhydrous magnesium sulfate, and the solvent was evaporated under reduced pressure. The residue was subjected to silica gel column chromatography (hexane-ethyl acetate 9:1-3:2) to give the title compound (0.04 g, 61%) as an oil.
1H NMR (300 MHz, CDCl₃) δ ppm 0.96-1.52 (m, 5 H), 1.59-1.92 (m, 7 H), 2.71-2.86 (m, 2 H), 3.05-3.20 (m, 2 H), 3.21-3.40 (m, 1 H), 3.75-3.84 (m, 6 H), 3.87-4.08 (m, 1 H), 6.53 (d, J=8.5 Hz, 2 H), 7.14 (t, J=8.3 Hz, 1 H).

Example 149A 3-[2-(benzyloxy)benzyl]-1-cyclohexylpyrrolidin-2-one

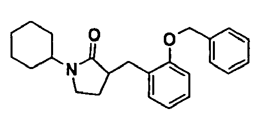

In the same manner as in Example 142A, the title compound was obtained from 1-cyclohexyl-3-(2-hydroxybenzyl)pyrrolidin-2-one obtained in Example 140A and benzyl bromide.
1H NMR (300 MHz, CDCl₃) δ ppm 1.00-1.15 (m, 1 H), 1.18-1.48 (m, 4 H), 1.55-1.83 (m, 6 H), 1.83-2.00 (m, 1 H), 2.60-2.90 (m, 2 H), 3.02-3.22 (m, 2 H), 3.24-3.43 (m, 1 H), 3.81-4.10 (m, 1 H), 5.02-5.15 (m, 2 H), 6.81-6.96 (m, 2 H), 7.09-7.24 (m, 2 H), 7.27-7.50 (m, 5 H).

### Example 150A methyl 2-[(1-cyclohexyl-2-oxopyrrolidin-3-yl)methyl]benzoate

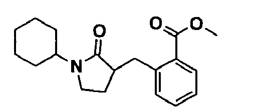

In the same manner as in Example 115A, the title compound was obtained from 3-(2-bromobenzyl)-1-cyclohexylpyrrolidin-2-one obtained in Example 144A.
1H NMR (300 MHz, CDCl₃) δ ppm 0.98-1.49 (m, 5 H), 1.62-1.84 (m, 6 H), 1.91-2.03 (m, 1 H), 2.66-2.85 (m, 1 H), 3.01 (m, 1 H), 3.08-3.25 (m, 2 H), 3. 52-3. 63 (m, 1 H), 3. 88-4. 04 (m, 1H), 3. 90 (s, 3H), 7.21-7.37 (m, 2 H), 7.38-7.46 (m, 1 H), 7.87 (m, 1 H).

### Example 151A 3-(2-chloro-4-fluorobenzyl)-1-cyclohexylpyrrolidin-2-one

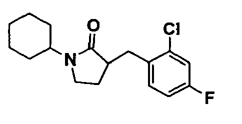

In the same manner as in Example 1A, the title compound was obtained from 1-cyclohexylpyrrolidin-2-one and 2-chloro-4-fluorobenzyl bromide.
1H NMR (300 MHz, CDCl₃) δ ppm 0.97-1.51 (m, 5 H), 1.55-1.88 (m, 6 H), 1.90-2.11 (m, 1 H), 2.65-2.88 (m, 2 H), 3.09-3.24 (m, 2 H), 3.24-3.41 (m, 1 H), 3.83-4.05 (m, 1 H), 6.83-6.99 (m, 1 H), 7.05-7.14 (m, 1 H), 7.20-7.32 (m, 1 H).

### Example 152A 1-cyclohexyl-3-(4-fluoro-2-methoxybenzyl) pyrrolidin-2-one

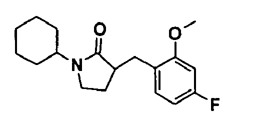

In the same manner as in Example 1A, the title compound was obtained from 1-cyclohexylpyrrolidin-2-one and 4-fluoro-2-methoxybenzyl chloride.
1H NMR (300 MHz, CDCl₃) δ ppm 1.00-1.46 (m, 5 H), 1.57-1.73 (m, 4 H), 1.72-1.85 (m, 2 H), 1.85-2.05 (m, 1 H), 2.49-2.62 (m, 1 H), 2.69-2.85 (m, 1 H), 3.11-3.19 (m, 2 H), 3.19-3.28 (m, 1 H), 3.79 (s, 3 H), 3.86-4.07 (m, 1 H), 6.47-6.64 (m, 2 H), 7.01-7.15 (m, 1 H).

Example 153A 1-cyclohexyl-3-(pyridin-2-ylmethyl)pyrrolidin-2-one

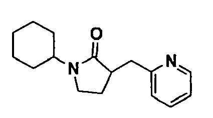

In the same manner as in Example 1A, the title compound was obtained from 1-cyclohexylpyrrolidin-2-one and 2-(bromomethyl)pyridine.
1H NMR (300 MHz, CDCl₃) δ ppm 0.97-1.47 (m, 6 H), 1.53-1.90 (m, 6 H), 1.97-2.22 (m, 1 H), 2.71-3.06 (m, 2 H), 3.07-3.26 (m, 2 H), 3.28-3.42 (m, 1 H), 3.80-4.04 (m, 1 H), 7.05-7.26 (m, 2 H), 7.50-7.69 (m, 1 H).

### Example 154A methyl 1-cyclohexyl-3-(2,6-dichlorobenzyl)-2-oxopyrrolidine-3-carboxylate

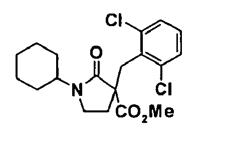

To a solution of methyl 1-cyclohexyl-2-oxopyrrolidine-3-carboxylate obtained in Reference Example 13A (3.0 g) in tetrahydrofuran (40 mL) was added sodium hydride (60% oil; 0.56 g) by small portions under ice-cooling. This mixture was stirred at 0°C for 1 hr, α,2,6-trichlorotoluene (3.12 g) was added, and the reaction mixture was further stirred at room temperature for 4 hr. Saturated aqueous ammonium chloride was added to the reaction mixture under ice-cooling, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 4:1-7:3) to give the title compound (2.83 g, 55%) as a white solid. Recrystallization from ethyl acetate-ether gave colorless crystals (1.36 g).
1H NMR (300 MHz, CDCl₃) δ ppm 1.00-1.12 (m, 1 H), 1.20-1.45 (m, 4 H), 1.64-1.87 (m, 6 H), 2.36-2.44 (m, 1 H), 2.90-2.97 (m, 1 H), 3.20-3.28 (m, 1 H), 3.72-3.99 (m, 3 H), 3.77 (s, 3 H), 7.12 (t, J=8.1 Hz, 1 H), 7.29 (d, J=7.8 Hz, 2 H).

### Example 155A 1-cyclohexyl-3-(2,6-dichlorobenzyl)-2-oxopyrrolidine-3-carboxylic acid

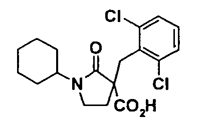

To a solution of methyl 1-cyclohexyl-3-(2,6-dichlorobenzyl)-2-oxopyrrolidine-3-carboxylate obtained in Example 154A (1.35 g) in methanol (20 mL) was added 2N sodium hydroxide (9 mL), and the mixture was stirred at room temperature for 4 hr. 6N Hydrochloric acid (3.5 mL) was added to the reaction mixture under ice-cooling, and the mixture was extracted with ethyl acetate. The organic layer was washed with saturated brine, and dried over anhydrous magnesium sulfate.

The solvent was evaporated under reduced pressure, and the residue was recrystallized from ethyl acetate to give the title compound (0.84 g, 64%) as white crystals.
1H NMR (300 MHz, CDCl₃)δ ppm 1.05-1.14 (m, 1 H), 1.24-1.45 (m, 4 H), 1.67-1.84 (m, 5 H), 2.36-2.41 (m, 2 H), 3.10-3.19 (m, 1 H), 3.26-3.33 (m, 1 H), 3.54, 3.59 (ABq, J=14.1 Hz, 2 H), 3.87-3.96 (m, 1 H), 7.16 (t, J=7.4 Hz, 1 H), 7.32 (d, J=7.8 Hz, 2 H).

### Example 156A 1-cyclohexyl-3-(2,6-dichlorobenzyl)-N-methyl-2-oxopyrrolidine-3-carboxamide

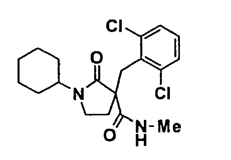

To a solution of 1-cyclohexyl-3-(2,6-dichlorobenzyl)-2-oxopyrrolidine-3-carboxylic acid obtained in Example 155A (0.20 g) in tetrahydrofuran (5 mL) were successively added oxalyl chloride (50 µL) and N,N-dimethylformamide (1 drop). The reaction mixture was stirred at room temperature for 2 hr, and the solvent was evaporated under reduced pressure. Toluene was added to the residue and the solvent was evaporated under reduced pressure, which operation was repeated twice. To a solution (5 mL) of the obtained residue in tetrahydrofuran was added 2 M methylamine-tetrahydrofuran solution (3 mL) under ice-cooling. The mixture was stirred at room temperature for 4 hr, water was added to the reaction mixture, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 3:2-1:1) to give the title compound (85 mg, 41%) as a colorless oil.
1H NMR (300 MHz, CDCl₃) δ ppm 1.05-1.15 (m, 1 H), 1.24-1.44 (m, 4 H), 1.65-1.83 (m, 5 H), 2.17-2.25 (m, 1 H), 2.46-2.56 (m, 1 H), 2.76 (d, J=5.1 Hz, 3 H), 3.12-3.27 (m, 2 H), 3.49 (s, 2 H), 3.84-3.93 (m, 1 H), 7.11 (t, J=7.4 Hz, 1 H), 7.28 (d, J=7.8Hz, 2 H), 7.81 (d, J=5.1 Hz, 1 H).

### Example 157A 1-cyclohexyl-3-(2,6-dichlorobenzyl)-N-benzyl-2-oxopyrrolidine-3-carboxamide

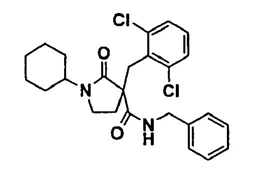

In the same manner as in Example 156A, the title compound was obtained from 1-cyclohexyl-3-(2,6-dichlorobenzyl)-2-oxopyrrolidine-3-carboxylic acid obtained in Example 155A and benzylamine.
1H NMR (300 MHz, CDCl₃) δ ppm 1.02-1.14 (m, 1 H), 1.24-1.43 (m, 4 H), 1.64-1.81 (m, 5 H), 2.16-2.24 (m, 1 H), 2.50-2.60 (m, 1 H), 3.09-3.27 (m, 2 H), 3.51, 3.56 (ABq, J=14.9 Hz, 2 H), 3.83-3.92 (m, 1 H), 4.37, 4.46 (dABq, J=15.0, 5.4 Hz, 2 H), 7.09 (t, J=7.9 Hz, 1 H), 7.20-7.31 (m, 7 H), 8.22 (t, J=5.1 Hz, 1 H).

### Example 158A 1-cyclohexyl-3-(2,6-dichlorobenzyl)-N-allyloxy-2-oxopyrrolidine-3-carboxamide

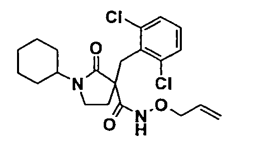

In the same manner as in Example 156A, the title compound was obtained from 1-cyclohexyl-3-(2,6-dichlorobenzyl)-2-oxopyrrolidine-3-carboxylic acid obtained in Example 155A and O-allylhydroxylamine hydrochloride.
1H NMR (300 MHz, CDCl₃) δ ppm 1.05-1.14 (m, 1 H), 1.20-1.43 (m, 4 H), 1.61-1.79 (m, 5 H), 2.16-2.24 (m, 1 H), 2.45-2.56 (m, 1 H), 3.02-3.10 (m, 1 H), 3.19-3.26 (m, 1 H), 3.51, 3.57 (ABq, J=14.1 Hz, 2 H), 3.82-3.91 (m, 1 H), 4.33-4.45 (m, 2 H), 5.26-5.32 (m, 2 H), 5.89-6.03 (m, 1 H), 7.13 (t, J=7.2 Hz, 1 H), 7.29 (d, J=7.5 Hz, 2 H).

Example 159A 1-[(1R,2R,4S)-bicyclo[2.2.1]hept-2-yl]-3-(2,6-dichlorobenzyl)pyrrolidin-2-one

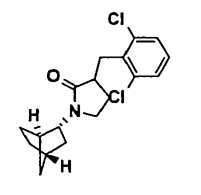

In the same manner as in Example 1A, the title compound was obtained from 1-[(1R,2R,4S)-bicyclo[2.2.1]hept-2-yl]pyrrolidin-2-one obtained in Reference Example 33A and α, 2, 6-trichlorotoluene.
1H NMR (300 MHz, CDCl₃) δ ppm 1.12-1.55 (m, 7 H), 1.64-1.98 (m, 3 H), 2.12-2.38 (m, 2 H), 2.81-3.11 (m, 2 H), 3.12-3.30 (m, 1 H), 3.31-3.56 (m, 2 H), 4.01-4.16 (m, 1 H), 7.03-7.14 (m, 1 H), 7.29 (d, J=7.9 Hz, 2 H).

### Example 160A 3-(2-acetylbenzyl)-1-cyclohexylpyrrolidin-2-one

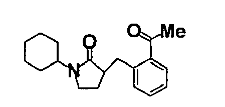

A mixture of methyl 2-[(1-cyclohexyl-2-oxopyrrolidin-3-yl)methyl]benzoate obtained in Example 150A (0.22 g) and tetrahydrofuran (10 mL) was cooled to 0°C, methyllithium (1.2 M diethyl ether solution, 1.8 mL) was added, and the mixture was stirred at this temperature for 1 hr. Saturated aqueous ammonium chloride was added to the reaction mixture, and the mixture was extracted with ethyl acetate. The ethyl acetate layer was washed with saturated brine, dried over anhydrous magnesium sulfate, and concentrated under reduced pressure. The obtained residue was subjected to silica gel column chromatography with ethyl acetate-hexane to give the title compound (0.021 g, 10%) as an oil.
1H NMR (300 MHz, CDCl₃) δ ppm 0.97-1.19 (m, 1 H), 1.21-1.49 (m, 4 H), 1.58-1.84 (m, 6 H), 1.90-2.08 (m, 1 H), 2.59 (s, 3 H), 2.68-2.84 (m, 1 H), 2.99 (dd, J=13.4, 8.5 Hz, 1 H), 3.08-3.27 (m, 2 H), 3.37 (dd, J=13.4, 5.5 Hz, 1 H), 3.82-4.03 (m, 1 H), 7.22-7.33 (m, 1 H), 7.34-7.45 (m, 2 H), 7.60-7.69 (m, 1 H).

### Example 161A 2-[(1-cyclohexyl-2-oxopyrrolidin-3-yl)methyl]benzoic acid

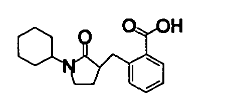

In the same manner as in Example 129A, the title compound was obtained from methyl 2-[(1-cyclohexyl-2-oxopyrrolidin-3-yl)methyl]benzoate obtained in Example 150A.
1H NMR (300 MHz, DMSO-d₆) δ ppm 0.95-1.15 (m, 1 H), 1.18-1.45 (m, 4 H), 1.46-1.65 (m, 4 H), 1.66-1.87 (m, 3 H), 2.58-2.72 (m, 1 H), 2.71-2.85 (m, 1 H), 3.02-3.23 (m, 2 H), 3.44-3.56 (m, 1 H), 3.63-3.79 (m, 1 H), 7.22-7.37 (m, 2 H), 7.38-7.53 (m, 1 H), 7.50-8.04 (m, 1 H), 12.91 (s, 1H).

### Example 162A 2-[(1-cyclohexyl-2-oxopyrrolidin-3-yl)methyl]-N-methylbenzamide

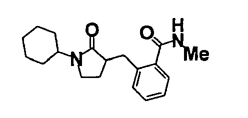

In the same manner as in Example 156A, the title compound was obtained from 2-[(1-cyclohexyl-2-oxopyrrolidin-3-yl)methyl]benzoic acid obtained in Example 161A and methylamine.
1H NMR (300 MHz, CDCl₃) δ ppm 1.00-1.15 (m, 1 H), 1.24-1.44 (m, 4 H), 1.56-1.70 (m, 3 H), 1.73-1.88 (m, 3 H), 2.05-2.16 (m, 1 H), 2.78-2.90 (m, 2 H), 2.97 (d, J=4.9 Hz, 3 H), 3.13-3.29 (m, 3 H), 3.83-3.97 (m, 1 H), 6.61 (brs, 1 H), 7.18-7.40 (m, 4 H). Example 163A methyl 3-benzyl-l-cyclohexyl-2-oxopyrrolidine-3-carboxylate

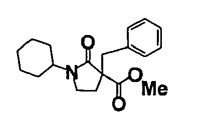

To a solution of methyl 1-cyclohexyl-2-oxopyrrolidine-3-carboxylate obtained in Reference Example 13A (0.20 g) in tetrahydrofuran (10 mL) was added sodium methoxide (50 mg), and the mixture was stirred at room temperature for 5 min. Benzyl bromide (0.16 mL) was added, and the mixture was stirred overnight at room temperature. The reaction mixture was concentrated under reduced pressure, ethyl acetate and water were added, and the mixture was extracted with ethyl acetate. The ethyl acetate layer was washed with saturated brine, dried over anhydrous magnesium sulfate, and concentrated. The obtained residue was silica gel column chromatography with ethyl acetate-hexane (1:3-1:2) to give the title compound (0.32 g, quantitative) as an orange oil.
1H NMR (300 MHz, CDCl₃) δ ppm 0.93-1.86 (m, 8 H), 2.35-2.49 (m, 1 H), 2.90-2.99 (m, 1 H), 3.12-3.42 (m, 2 H), 3.51-3.59 (m, 1 H), 3.70-3.79 (m, 2 H), 3.75-3.76 (m, 3 H), 3.84-4.01 (m, 1 H), 7.17-7.54 (m, 5 H), 7.83-7.88 (m, 1 H).

### Example 164A 2-[(1-cyclohexyl-2-oxopyrrolidin-3-yl)methyl]benzonitrile

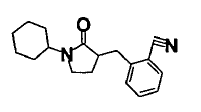

In the same manner as in Example 1A, the title compound was obtained from 1-cyclohexylpyrrolidin-2-one and 2-cyanobenzyl bromide.
1H NMR (300 MHz, CDCl₃) δ ppm 0.94-1.19 (m, 1 H), 1.21-1.50 (m, 4 H), 1.58-1.89 (m, 6 H), 1.99-2.14 (m, 1 H), 2.68-2.88 (m, 1 H), 2.93-3.08 (dd, J=14.0, 8.7 Hz, 1 H), 3.12-3.25 (m, 2 H), 3.37 (dd, J=14.0, 5.0 Hz, 1 H), 3.84-4.02 (m, 1 H), 7.28-7.37 (m, 1 H), 7.41-7.47 (m, 1 H), 7.49-7.58 (m, 1 H), 7.57-7.68 (m, 1 H).

### Example 165A 3-[2-(aminomethyl)benzyl]-1-cyclohexylpyrrolidin-2-one

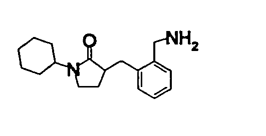

To a solution of 2-[(1-cyclohexyl-2-oxopyrrolidin-3-yl)methyl]benzonitrile obtained in Example 164A (1.02 g) and anhydrous cobalt (II) chloride (0.94 g) in methanol (25 mL) was added sodium borohydride (1.52 g), and the mixture was stirred at room temperature 8 hr. Water was added to the reaction mixture, the mixture was filtrated through celite, and the filtrate was extracted with ethyl acetate. The ethyl acetate layer was washed with saturated brine, dried over anhydrous magnesium sulfate, and concentrated under reduced pressure. The obtained residue was silica gel column chromatography with methanol-ethyl acetate (0:100-5:95) to give the title compound (0.32 g, 31%) as a yellow oil.
1H NMR (300 MHz, CDCl₃) δ ppm 1.04-1.17 (m, 1 H), 1.29-1.47 (m, 4 H), 1.61-1.85 (m, 6 H), 1.99-2.10 (m, 1 H), 2.54-2.65 (m, 1 H), 2.67-2.79 (m, 1 H), 3.13-3.29 (m, 2 H), 3.38 (dd, J=13.9, 3.6 Hz, 1 H), 3.89-4.02 (m, 3 H), 7.17-7.25 (m, 3 H), 7.33-7.39 (m, 1 H).

### Example 166A methyl 1-cyclohexyl-3-(2-nitrobenzyl)-2-oxopyrrolidine-3-carboxylate

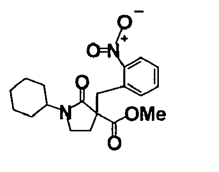

In the same manner as in Example 163A, the title compound was obtained from methyl 1-cyclohexyl-2-oxopyrrolidine-3-carboxylate obtained in Reference Example 13A and 2-nitrobenzyl bromide.
1H NMR (300 MHz, CDCl₃) δ ppm 0.98-1.85 (m, 13 H), 3.52-3.59 (m, 1 H), 3.71-3.78 (m, 1 H), 3.75 (s, 3 H), 3.85-3.97 (m, 1 H), 7.34-7.41 (m, 1 H), 7.44-7.54 (m, 2 H), 7.78-7.93 (m, 1 H).

### Example 167A 1-cyclohexyl-3-(2-nitrobenzyl)-2-oxopyrrolidine-3-carboxylic acid

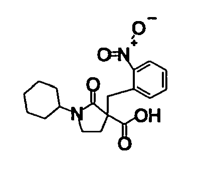

In the same manner as in Example 129A, the title compound was obtained from methyl 1-cyclohexyl-3-(2-nitrobenzyl)-2-oxopyrrolidine-3-carboxylate obtained in Example 166A.
1H NMR (300 MHz, CDCl₃) δ ppm 1.01-1.18 (m, 1 H), 1.24-1.47 (m, 4 H), 1.63-1.75 (m, 2 H), 1.76-1.88 (m, 3 H), 2.11-2.21 (m, 1 H), 2.35-2.47 (m, 1 H), 3.22-3.39 (m, 3 H), 3.78-3.92 (m, 2 H), 7.36-7.41 (m, 1 H), 7.42-7.49 (m, 1 H), 7.52-7.63 (m, 1 H), 7.94-8.03 (m, 1 H), 11.04 (brs, 1 H).

### Example 168A 1-cyclohexyl-3-(2-nitrobenzyl)pyrrolidin-2-one

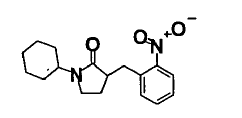

A solution of 1-cyclohexyl-3-(2-nitrobenzyl)-2-oxopyrrolidine-3-carboxylic acid obtained in Example 167A (0.26 g) in dioxane (10 mL) was heated under reflux overnight. After cooling, the reaction mixture was concentrated under reduced pressure, diisopropyl ether was added, and the insoluble substance was filtered off. The filtrate was concentrated, and the residue was recrystallized from hexane-diisopropyl ether to give the title compound (92 mg, 41%) as a yellow solid.
1H NMR (300 MHz, CDCl₃) δ ppm 0.98-1.18 (m, 1 H), 1.23-1.49 (m, 4 H), 1.57-1.87 (m, 6 H), 2.02-2.16 (m, 1 H), 2.69-2.89 (m, 1 H), 3.04 (dd, J=13.8, 7.8 Hz, 1 H), 3.12-3.31 (m, 2 H), 3.43 (dd, J=13.8, 5.7 Hz, 1H), 3.81-4.02 (m, 1H), 7.31-7.41 (m, 1H), 7.44-7.58 (m, 2 H), 7.88 (d, J=7.9 Hz, 1 H).

### Example 169A methyl {2-[(1-cyclohexyl-2-oxopyrrolidin-3-yl)methyl]phenoxy}acetate

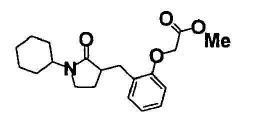

In the same manner as in Example 142A, the title compound was obtained from 1-cyclohexyl-3-(2-hydroxybenzyl)pyrrolidin-2-one obtained in Example 140A and methyl bromoacetate.
1H NMR (300 MHz, CDCl₃) δ ppm 1.01-1.14 (m, 1 H), 1.25-1.46 (m, 5 H), 1.63-1.80 (m, 5 H), 1.91-2.02 (m, 1 H), 2.64-2.72 (m, 1 H), 2.79-2.89 (m, 1 H), 3.10-3.23 (m, 2 H), 3.30-3.36 (m, 1 H), 3.79 (s, 3 H), 3.90-4.00 (m, 1 H), 4.65-4.67 (m, 2 H), 6.70 (d, J=8.1 Hz, 1 H), 6.89-6.95 (m, 1 H), 7.11-7.24 (m, 2 H).

### Example 170A 1-cyclohexyl-3-[2-(2-hydroxyethoxy)benzyl]pyrrolidin-2-one

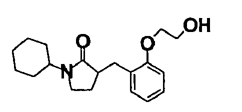

Methyl {2-[(1-cyclohexyl-2-oxopyrrolidin-3-yl)methyl]phenoxy}acetate obtained in Example 169A (0.25 g) was dissolved in a mixed solvent of tetrahydrofuran (8 mL) and ethanol (2 mL), lithium borohydride (19 mg) was added, and the mixture was stirred overnight at room temperature. Water and ethyl acetate were added successively to the reaction mixture acetone, and the mixture was extracted with ethyl acetate. The ethyl acetate layer was washed with saturated brine, dried over anhydrous magnesium sulfate, and concentrated under reduced pressure. The obtained residue was silica gel column chromatography with ethyl acetate-hexane (30:70-100:0) to give the title compound (0.11 g, 42%) as a white solid.
1H NMR (300 MHz, CDCl₃) δ ppm 1.00-1.47 (m, 5 H), 1.51-1.83 (m, 6 H), 1.99-2.13 (m, 1 H), 2.66-2.86 (m, 2 H), 2.91-3.01 (m, 1 H), 3.08-3.19 (m, 2 H), 3.86-4.08 (m, 5 H), 4.34-4.42 (m, 1 H), 6.79-6.91 (m, 2 H), 7.12-7.24 (m, 2 H).

### Example 171A 12-[(1-cyclohexyl-2-oxopyrrolidin-3-yl)methyl]phenoxy}acetic acid

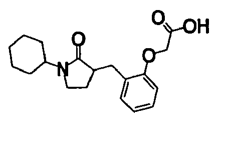

In the same manner as in Example 129A, the title compound was obtained from methyl {2-[(1-cyclohexyl-2-oxopyrrolidin-3-yl)methyl]phenoxy}acetate obtained in Example 169A.
1H NMR (300 MHz, DMSO-d₆) δ ppm 0.99-1.45 (m, 5 H), 1.48-1.66 (m, 4 H), 1.69-1.92 (m, 3 H), 2.37-2.48 (m, 1 H), 2.63-2.76 (m, 1 H), 3.06-3.24 (m, 3 H), 3.67-3.79 (m, 1 H), 4.69 (s, 2 H), 6.81-6.90 (m, 2 H), 7.08-7.21 (m, 2 H), 12.94 (brs, 1 H).

### Example 172A 2-{2-[(1-cyclohexyl-2-oxopyrrolidin-3-yl)methyl]phenoxy}-N-methylacetamide

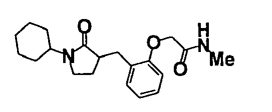

In the same manner as in Example 70A, the title compound was obtained from {2-[(1-cyclohexyl-2-oxopyrrolidin-3-yl)methyl]phenoxy}acetic acid obtained in Example 171A and methylamine.
1H NMR (300 MHz, CDCl₃) δ ppm 1.00-1.16 (m, 1 H), 1.24-1.44 (m, 4 H), 1.60-1.85 (m, 6 H), 2.04-2.19 (m, 1 H), 2.52-2.61 (m, 1 H), 2.63-2.72 (m, 1 H), 2.96 (d, J=4.9 Hz, 3 H), 3.12-3.26 (m, 2 H), 3.36 (m, 1 H), 3.83-3.96 (m, 1 H), 4.42-4.54 (m, 2 H), 6.77-6.82 (m, 1 H), 6.90-6.97 (m, 1 H), 7.14-7.26 (m, 2 H), 7.94 (brs, 1 H).

### Example 173A 2-{2-[(1-cyclohexyl-2-oxopyrrolidin-3-yl)methyl]phenoxy}-N,N-dimethylacetamide

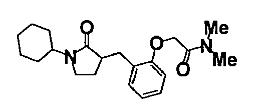

In the same manner as in Example 70A, the title compound was obtained from 12-[(1-cyclohexyl-2-oxopyrrolidin-3-yl)methyl]phenoxy}acetic acid obtained in Example 171A and dimethylamine.
1H NMR (300 MHz, CDCl₃) δ ppm 1.02-1.17 (m, 1 H), 1.28-1.47 (m, 4 H), 1.60-1.84 (m, 6 H), 1.88-2.03 (m, 1 H), 2.54-2.64 (m, 1 H), 2.74-2.89 (m, 1 H), 2.99 (s, 3 H), 3.12 (s, 3 H), 3.13-3.24 (m, 2 H), 3.32-3.39 (m, 1H), 3.87-4.01 (m, 1H), 4.71 (s, 2 H), 6.81-6.85 (m, 1 H), 6.88-6.94 (m, 1 H), 7.13-7.21 (m, 2 H). Example 174A {2-[(1-cyclohexyl-2-oxopyrrolidin-3-yl)methyl]phenoxy}acetonitrile

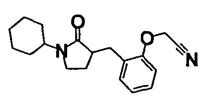

In the same manner as in Example 142A, the title compound was obtained from 1-cyclohexyl-3-(2-hydroxybenzyl)pyrrolidin-2-one obtained in Example 140A and bromoacetonitrile.
1H NMR (300 MHz, CDCl₃) δ ppm 1.03-1.15 (m, 1 H), 1.23-1.43 (m, 5 H), 1.58-1.83 (m, 6 H), 1.92-2.03 (m, 1 H), 2.57-2.65 (m, 1 H), 2.69-2.80 (m, 1 H), 3.14-3.20 (m, 1 H), 3.27-3.35 (m, 1 H), 3.89-4.00 (m, 1 H), 4.75-4.87 (m, 2 H), 6.94 (d, J=8.1 Hz, 1 H), 6.99-7.04 (m, 1 H), 7.22-7.28 (m, 2 H).

### Example 175A 3-(2-chloro-6-methoxybenzyl)-1-cyclohexylpyrrolidin-2-one

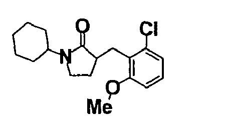

In the same manner as in Example 1A, the title compound was obtained from 1-cyclohexylpyrrolidin-2-one and 2-chloro-6-methoxybenzyl bromide.
1H NMR (300 MHz, CDCl₃) δ ppm 1.01-1.18 (m, 1 H), 1.22-1.49 (m, 5 H), 1.66-1.91 (m, 6 H), 2.78-2.94 (m, 2 H), 3.08-3.21 (m, 1 H), 3.23-3.38 (m, 2 H), 3.82 (s, 3 H), 3.90-4.03 (m, 1 H), 6.76 (m, 1 H), 6.97 (m, 1 H), 7.11 (t, J=8.1 Hz, 1 H).

### Example 176A 3-(2-chloro-4-methoxybenzyl)-1-cyclohexylpyrrolidin-2-one

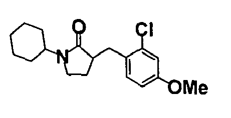

In the same manner as in Example 1A, the title compound was obtained from 1-cyclohexylpyrrolidin-2-one and 2-chloro-4-methoxybenzyl bromide.
1H NMR (300 MHz, CDCl₃) δ ppm 1.01-1.17 (m, 1 H), 1.24-1.48 (m, 4 H), 1.62-1.85 (m, 6 H), 1.91-2.06 (m, 1 H), 2.67-2.84 (m, 2 H), 3.13-3.22 (m, 2 H), 3.23-3.36 (m, 1 H), 3.78 (s, 3 H), 3.83-4.05 (m, 1 H), 6.74 (dd, J=8.6, 2.6 Hz, 1 H), 6.90 (d, J=2. 6 Hz, 1 H), 7.18 (d, J=8.6 Hz, 1 H).

### Example 177A 3-(2-chloro-6-hydroxybenzyl)-1-cyclohexylpyrrolidin-2-one

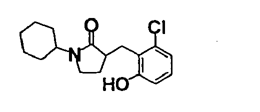

In the same manner as in Example 140A, the title compound was obtained from 3-(2-chloro-6-methoxybenzyl)-1-cyclohexylpyrrolidin-2-one obtained in Example 175A.
1H NMR (300 MHz, CDCl₃) δ ppm 0.97-1.18 (m, 1 H), 1.21-1.48 (m, 4 H), 1.62-1.72 (m, 3 H), 1.72-1.91 (m, 3 H), 2.33-2.46 (m, 1 H), 2.66-2.81 (m, 1 H), 2.97-3.16 (m, 2 H), 3.18-3.39 (m, 2 H), 3.84-4.00 (m, 1 H), 6.80-6.95 (m, 2 H), 7.04 (t, J=8.0 Hz, 1 H), 9.92 (s, 1 H).

### Example 178A 3-(2-chloro-6-ethoxybenzyl)-1-cyclohexylpyrrolidin-2-one

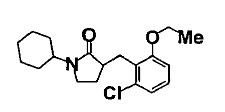

In the same manner as in Example 142A, the title compound was obtained from 3-(2-chloro-6-hydroxybenzyl)-1-cyclohexylpyrrolidin-2-one obtained in Example 177A and ethyl iodide.
1H NMR (300 MHz, CDCl₃) δ ppm 0.81-0.93 (m, 1 H), 1.01-1.48 (m, 6 H), 1.42 (t, J=7.0 Hz, 3 H), 1.65-1.94 (m, 5 H), 2.78-2.94 (m, 2 H), 3.10-3.19 (m, 1 H), 3.26-3.36 (m, 2 H), 3.91-4.01 (m, 1 H), 4.03 (q, J=7.0 Hz, 2 H), 6.74 (d, J=8.0 Hz, 1 H), 6.90-7.00 (d, J=8.0 Hz, 1 H), 7.08 (t, J=8.0 Hz, 1 H).

### Example 179A {3-chloro-2-[(1-cyclohexyl-2-oxopyrrolidin-3-yl)methyl]phenoxy}acetonitrile

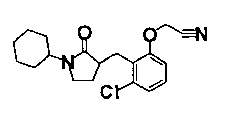

In the same manner as in Example 142A, the title compound was obtained from 3-(2-chloro-6-hydroxybenzyl)-1-cyclohexylpyrrolidin-2-one obtained in Example 177A and bromoacetonitrile.
1H NMR (300 MHz, CDCl₃) δ ppm 1.02-1.18 (m, 1 H), 1.26-1.49 (m, 4 H), 1.64-1.84 (m, 6 H), 1.87-1.99 (m, 1 H), 2.73-2.92 (m, 2 H), 3.12-3.22 (m, 1 H), 3.27-3.39 (m, 2 H), 3.86-4.01 (m, 1 H), 4.81-4.85 (m, 2 H), 6.84-6.90 (m, 1 H), 7.10-7.23 (m, 2 H).

### Example 180A 3-[2-chloro-6-(2-methoxyethoxy)benzyl]-1-cyclohexylpyrrolidin-2-one

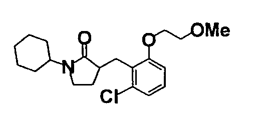

In the same manner as in Example 142A, the title compound was obtained from 3-(2-chloro-6-hydroxybenzyl)-1-cyclohexylpyrrolidin-2-one obtained in Example 177A and bromoethyl methyl ether.
1H NMR (300 MHz, CDCl₃) δ ppm 1.00-1.17 (m, 1 H), 1.29-1.48 (m, 4 H), 1.62-1.91 (m, 7 H), 2.82-2.97 (m, 2 H), 3.10-3.20 (m, 1 H), 3.27-3.36 (m, 2 H), 3.44 (s, 3 H), 3.73-3.79 (m, 2 H), 3.91-4.02 (m, 1 H), 4.08-4.14 (m, 2 H), 6.72-6.79 (m, 1 H), 6.96-7.02 (m, 1 H), 7.05-7.13 (m, 1 H).

### Example 181A 3-[2-chloro-6-(2-oxopropoxy)benzyl]-1-cyclohexylpyrrolidin-2-one

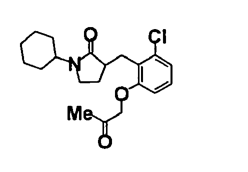

In the same manner as in Example 142A, the title compound was obtained from 3-(2-chloro-6-hydroxybenzyl)-1-cyclohexylpyrrolidin-2-one obtained in Example 177A and bromoacetone.
1H NMR (300 MHz, CDCl₃) δ ppm 1.04-1.17 (m, 1 H), 1.29-1.47 (m, 4 H), 1.62-1.99 (m, 7 H), 2.35 (s, 3 H), 2.79-2.99 (m, 2 H), 3.11-3.23 (m, 1 H), 3.28-3.38 (m, 1 H), 3.39-3.48 (m, 1 H), 3.88-4.03 (m, 1 H), 4.54 (s, 2 H), 6.54-6.64 (m, 1 H), 7.01-7.15 (m, 2 H).

### Example 182A 3-[2-chloro-4-(methoxymethoxy)benzyl]-1-cyclohexylpyrrolidin-2-one

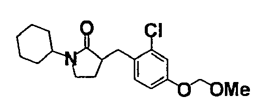

In the same manner as in Example 1A, the title compound was obtained from 1-cyclohexylpyrrolidin-2-one and 2-chloro-4-(methoxymethoxy)benzyl bromide obtained in Reference Example 24A.
1H NMR (300 MHz, CDCl₃) δ ppm 1.02-1.16 (m, 1 H), 1.28-1.44 (m, 4 H), 1.60-1.84 (m, 6 H), 1.93-2.03 (m, 1 H), 2.68-2.82 (m, 2 H), 3.14-3.24 (m, 2 H), 3.27-3.34 (m, 1 H), 3.47 (s, 3 H), 3.87-4.01 (m, 1 H), 5.13 (s, 2 H), 6.87 (dd, J=8. 5, 2.6 Hz, 1 H), 7.07 (d, J=2.6 Hz, 1 H), 7.18 (d, J=8.5 Hz, 1 H).

### Example 183A 3-(2-chloro-4-hydroxybenzyl)-1-cyclohexylpyrrolidin-2-one

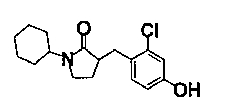

To a solution of 3-[2-chloro-4-(methoxymethoxy)benzyl]-1-cyclohexylpyrrolidin-2-one obtained in Example 182A (0.30 g) in tetrahydrofuran (5 mL) was added concentrated hydrochloric acid (1 mL), and the mixture was stirred at room temperature for 2 hr. Ethyl acetate and water were added to the reaction mixture, and the mixture was extracted with ethyl acetate. The ethyl acetate layer was washed with saturated brine, dried over anhydrous magnesium sulfate, and concentrated under reduced pressure. The obtained residue was recrystallized from hexane-ethyl acetate to give the title compound (0.17 g, 65%) as a white solid.
1H NMR (300 MHz, CDCl₃) δ ppm 0.99-1.17 (m, 1 H), 1.23-1.46 (m, 4 H), 1.62-1.83 (m, 6 H), 1.96-2.09 (m, 1 H), 2.69-2.86 (m, 2 H), 3.16-3.28 (m, 3 H), 3.87-4.01 (m, 1 H), 6.67 (dd, J=8.3, 2.5 Hz, 1 H), 6.75 (s, 1 H), 6.88 (d, J=2.5 Hz, 1 H), 7.08 (d, J=8.3 Hz, 1 H).

### Example 184A {3-chloro-4-[(1-cyclohexyl-2-oxopyrrolidin-3-yl)methyl]phenoxy}acetonitrile

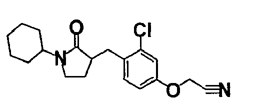

In the same manner as in Example 142A, the title compound was obtained from 3-(2-chloro-4-hydroxybenzyl)-1-cyclohexylpyrrolidin-2-one obtained in Example 183A and bromoacetonitrile.
1H NMR (300 MHz, CDCl₃) δ ppm 0.99-1.19 (m, 1 H), 1.24-1.49 (m, 4 H), 1.60-1.85 (m, 6 H), 1.93-2.09 (m, 1 H), 2.70-2.83 (m, 2 H), 3.13-3.23 (m, 2 H), 3.25-3.37 (m, 1 H), 3.89-4.01 (m, 1 H), 4.75 (s, 2 H), 6.81-6.86 (m, 1 H), 6.99-7.03 (m, 1 H), 7.24-7.27 (m, 1 H).

### Example 185A 3-[2 -chloro-4-(cyclopropylmethoxy)benzyl]-1-cyclohexylpyrrolidin-2-one

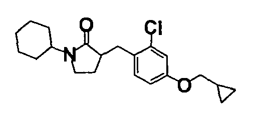

In the same manner as in Example 142A, the title compound was obtained from 3-(2-chloro-4-hydroxybenzyl)-1-cyclohexylpyrrolidin-2-one obtained in Example 183A and cyclopropylmethyl bromide.
1H NMR (300 MHz, CDCl₃)δ ppm 0.29-0.38 (m, 2 H), 0.59-0.69 (m, 2 H), 0.98-1.17 (m, 1 H), 1.18-1.49 (m, 5 H), 1.60-1.84 (m, 6 H), 1.90-2.07 (m, 1 H), 2.68-2.82 (m, 2 H), 3.11-3.22 (m, 2 H), 3.23-3.35 (m, 1 H), 3.76 (d, J=7.0 Hz, 2 H), 3.88-4.04 (m, 1 H), 6.74 (dd, J=8.6, 2.5 Hz, 1 H), 6.90 (d, J=2.5 Hz, 1 H), 7.16 (d, J=8.6 Hz, 1 H).

### Example 186A 3-[4-(benzyloxy)-2-chlorobenzyl]-1-cyclohexylpyrrolidin-2-one

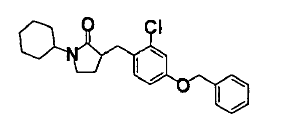

In the same manner as in Example 142A, the title compound was obtained from 3-(2-chloro-4-hydroxybenzyl)-1-cyclohexylpyrrolidin-2-one obtained in Example 183A and benzyl bromide.
1H NMR (300 MHz, CDCl₃)δ ppm 1.00-1.16 (m, 1 H), 1.24-1.48 (m, 4 H), 1.61-1.84 (m, 6 H), 1.93-2.06 (m, 1 H), 2.68-2.82 (m, 2 H), 3.12-3.23 (m, 2 H), 3.23-3.35 (m, 1 H), 3.87-4.02 (m, 1 H), 5.02 (s, 2 H), 6.81 (dd, J=8.5, 2.6 Hz, 1 H), 6.99 (d, J=2.6 Hz, 1 H), 7.18 (d, J=8.5 Hz, 1 H), 7.30-7.44 (m, 5 H).

### Example 187A 3-[2-chloro-4-(prop-2-yn-1-yloxy)benzyl]-1-cyclohexylpyrrolidin-2-one

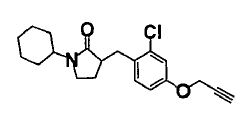

In the same manner as in Example 142A, the title compound was obtained from 3-(2-chloro-4-hydroxybenzyl)-1-cyclohexylpyrrolidin-2-one obtained in Example 183A and propargyl bromide.
1H NMR (300 MHz, CDCl₃)δ ppm 1.00-1.16 (m, 1 H), 1.24-1.47 (m, 4 H), 1.62-1.84 (m, 6 H), 1.93-2.06 (m, 1 H), 2.52-2.55 (t, J=2.4 Hz, 1 H), 2.69-2.83 (m, 2 H), 3.12-3.23 (m, 2 H), 3.24-3.36 (m, 1 H), 3.88-4.01 (m, 1H), 4.66 (d, J=2.4 Hz, 2 H), 6.82 (dd, J=8.5, 2.6 Hz, 1 H), 6.99 (d, J=2.6 Hz, 1 H), 7.20 (d, J=8.5 Hz, 1 H) .

### Example 188A 1-[(1R,2R,4S)-bicyclo[2.2.1]hept-2-yl]-3-(2,4-dichlorobenzyl)pyrrolidin-2-one

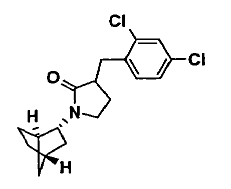

In the same manner as in Example 1A, the title compound was obtained from 1-[(1R,2R,4S)-bicyclo[2.2.1] hept-2-yl]pyrrolidin-2-one obtained in Reference Example 33A and 2,4-dichlorobenzyl chloride.
1H NMR (300 MHz, CDCl₃) δ ppm 1.08-1.27 (m, 2 H), 1.28-1.55 (m, 5 H), 1.61-1.81 (m, 2 H), 1.88-2.09 (m, 1 H), 2.16 (s, 1 H), 2.31 (s, 1 H), 2.65-2.85 (m, 2 H), 3.12-3.40 (m, 3 H), 3.96-4.12 (m, 1 H), 7.10-7.26 (m, 2 H), 7.37 (d, J=1.7 Hz, 1 H).

### Example 189A tert-butyl 4-[(1-cyclohexyl-2-oxopyrrolidin-3-yl)methyl]-3-methoxybenzoate

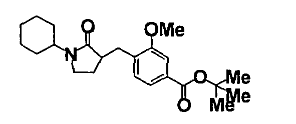

In the same manner as in Example 1A, the title compound was obtained from 1-cyclohexylpyrrolidin-2-one and 4-tert-butoxycarbonyl-2-methoxybenzyl bromide.
1H NMR (300 MHz, CDCl₃) δ ppm 1.20-1.49 (m, 5 H), 1.52-1.81 (m, 15 H), 1.84-1.98 (m, 1 H), 2.57-2.68 (m, 1 H), 2.72-2.85 (m, 1 H), 3.06-3.24 (m, 2 H), 3.28-3.37 (m, 1 H), 3.86 (s, 3 H), 3.88-4.01 (m, 1 H), 7.19 (d, J=7.7 Hz, 1 H), 7.47 (d, J=1.6 Hz, 1 H), 7.48-7.54 (dd, J=7.7, 1.6 Hz, 1 H).

### Example 190A 1-cyclohexyl-3-(pyridin-3-ylmethyl)pyrrolidin-2-one

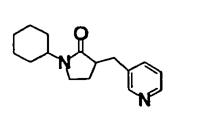

In the same manner as in Example 1A, the title compound was obtained from 1-cyclohexylpyrrolidin-2-one and 3-(chloromethyl)pyridine hydrochloride.
1H NMR (300 MHz, CDCl₃) δ ppm 1.02-1.49 (m, 6 H), 1.60-1.86 (m, 6 H), 1.96-2.14 (m, 1 H), 2.65-2.83 (m, 2 H), 3.01-3.27 (m, 3 H), 3.86-4.03 (m, 1 H), 7.17-7.24 (m, 1 H), 7.48-7.65 (m, 1 H), 8.46 (s, 1 H).

### Example 191A 1-cyclohexyl-3-(pyridin-4-ylmethyl)pyrrolidin-2-one

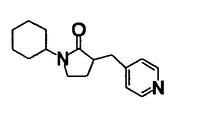

In the same manner as in Example 1A, the title compound was obtained from 1-cyclohexylpyrrolidin-2-one and 4-(chloromethyl)pyridine hydrochloride.
1H NMR (300 MHz, CDCl₃) δ ppm 0.95-1.55 (m, 5 H), 1.55-1.88 (m, 6 H), 1.96-2.17 (m, 1 H), 2.59-2.87 (m, 2 H), 3.00-3.32 (m, 3 H), 3.75-4.10 (m, 1 H), 7.07-7.22 (m, 2 H), 8.43-8.58 (m, 2 H).

### Example 192A 1-cyclohexyl-3-(2-fluorobenzyl)pyrrolidin-2-one

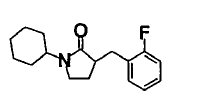

In the same manner as in Example 1A, the title compound was obtained from 1-cyclohexylpyrrolidin-2-one and 2-fluorobenzyl chloride.
1H NMR (300 MHz, CDCl₃) δ ppm 1.01-1.16 (m, 1 H), 1.24-1.47 (m, 4 H), 1.58-1.73 (m, 4 H), 1.73-1.84 (m, 2 H), 1.95-2.08 (m, 1 H), 2.66-2.80 (m, 2 H), 3.13-3.19 (m, 2 H), 3.20-3.32 (m, 1 H), 3.87-4.03 (m, 1 H), 6.96-7.10 (m, 2 H), 7.13-7.29 (m, 2 H).

### Example 193A 3-(4-bromo-2-chlorobenzyl)-1-cyclohexylpyrrolidin-2-one

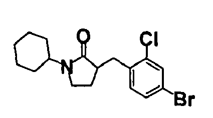

In the same manner as in Example 1A, the title compound was obtained from 1-cyclohexylpyrrolidin-2-one and 4-bromo-2-chlorobenzyl bromide.
1H NMR (300 MHz, CDCl₃) δ ppm 1.03-1.16 (m, 1 H), 1.23-1.48 (m, 4 H), 1.59-1.73 (m, 4 H), 1.74-1.84 (m, 2 H), 1.94-2.08 (m, 1 H), 2.70-2.84 (m, 2 H), 3.12-3.25 (m, 2 H), 3.25-3.37 (m, 1 H), 3.87-4.00 (m, 1 H), 7.17 (d, J=8.3 Hz, 1 H), 7.31 (dd, J=8.3, 2.1 Hz, 1 H), 7.52 (d, J=2.1 Hz, 1 H).

### Example 194A 1-cyclohexyl-3-(2-phenylethyl)pyrrolidin-2-one

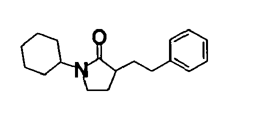

In the same manner as in Example 1A, the title compound was obtained from 1-cyclohexylpyrrolidin-2-one and 2-phenylethyl bromide.
1H NMR (300 MHz, CDCl₃) δ ppm 0.99-1.18 (m, 1 H), 1.25-1.48 (m, 4 H), 1.56-1.83 (m, 7 H), 2.10-2.29 (m, 2 H), 2.31-2.46 (m, 1 H), 2.61-2.81 (m, 2 H), 3.15-3.35 (m, 2 H), 3.86-4.02 (m, 1 H), 7.13-7.32 (m, 5 H).

### Example 195A 3-[2-(2-chlorophenyl)ethyl]-1-cyclohexylpyrrolidin-2-one

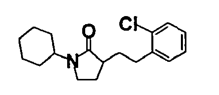

In the same manner as in Example 1A, the title compound was obtained from 1-cyclohexylpyrrolidin-2-one and 2-(2-chlorophenyl)ethyl bromide.
1H NMR (300 MHz, CDCl₃) δ ppm 1.00-1.18 (m, 1 H), 1.26-1.47 (m, 4 H), 1.59-1.84 (m, 7 H), 2.10-2.28 (m, 2 H), 2.38-2.50 (m, 1 H), 2.74-2.91 (m, 2 H), 3.19-3.36 (m, 2 H), 3.88-3.99 (m, 1 H), 7.09-7.21 (m, 2 H), 7.25-7.35 (m, 2 H).

### Example 196A tert-butyl {3-chloro-4-[(1-cyclohexyl-2-oxopyrrolidin-3-yl)methyl]phenyl}(methyl)carbamate

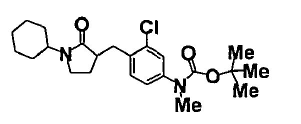

In the same manner as in Example 1A, the title compound was obtained from 1-cyclohexylpyrrolidin-2-one and tert-butyl [3-chloro-4-(chloromethyl)phenyl](methyl)carbamate obtained in Reference Example 29A.
1H NMR (300 MHz, CDCl₃) δ ppm 1.01-1.16 (m, 1 H), 1.24-1.43 (m, 4 H), 1.46 (s, 9 H), 1.62-1.84 (m, 6 H), 1.93-2.07 (m, 1 H), 2.70-2.85 (m, 2 H), 3.12-3.27 (m, 2 H), 3.23 (s, 3 H), 3.28-3.40 (m, 1 H), 3.87-4.02 (m, 1 H), 7.08 (dd, J=8.3, 2.1 Hz, 1 H), 7.20-7.29 (m, 2 H) .

### Example 197A 1-cyclohexyl-3-[2-(methylthio)benzyl]pyrrolidin-2-one

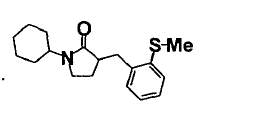

In the same manner as in Example 1A, the title compound was obtained from 1-cyclohexylpyrrolidin-2-one and 2-methylthiobenzyl chloride.
1H NMR (300 MHz, CDCl₃) δ ppm 1.00-1.17 (m, 1 H), 1.23-1.47 (m, 4 H), 1.63-1.83 (m, 6 H), 1.91-2.03 (m, 1 H), 2.45 (s, 3 H), 2.69-2.78 (m, 1 H), 2.78-2.90 (m, 1 H), 3.11-3.26 (m, 2 H), 3.34-3.41 (m, 1 H), 3.90-4.02 (m, 1 H), 7.05-7.14 (m, 1 H), 7.17-7.22 (m, 3 H).

### Example 198A 1-cyclohexyl-3-[2-(methylsulfinyl)benzyl]pyrrolidin-2-one (less polar product)

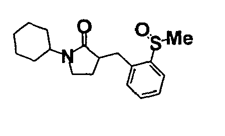

In the same manner as in Example 127A, the title compound was obtained from 1-cyclohexyl-3-[2-(methylthio)benzyl]pyrrolidin-2-one obtained in Example 197A.
1H NMR (300 MHz, CDCl₃) δ ppm 1.03-1.18 (m, 1 H), 1.23-1.49 (m, 4 H), 1.63-1.85 (m, 6 H), 1.98-2.14 (m, 1 H), 2.62-2.83 (m, 2 H), 2.70 (s, 3H), 3.18-3.37 (m, 3 H), 3.89-4.01 (m, 1 H), 7.29-7.35 (m, 1 H), 7.42-7.52 (m, 2 H), 7.94-8.05 (m, 1 H).

### Example 199A 1-cyclohexyl-3-[2-(methylsulfinyl)benzyl]pyrrolidin-2-one (more polar product)

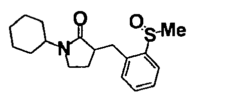

In the same manner as in Example 127A, the title compound was obtained from 1-cyclohexyl-3-[2-(methylthio)benzyl]pyrrolidin-2-one obtained in Example 197A.
1H NMR (300 MHz, CDCl₃) δ ppm 1.03-1.15 (m, 1 H), 1.23-1.46 (m, 4 H), 1.57-1.83 (m, 6 H), 1.96-2.07 (m, 1 H), 2.74 (s, 3 H), 2.76-2.96 (m, 2 H), 3.10-3.19 (m, 2 H), 3.24-3.31 (m, 1 H), 3.89-3.99 (m, 1 H), 7.23-7.27 (m, 1 H), 7.37-7.44 (m, 1 H), 7.46-7.52 (m, 1 H), 8.00-8.04 (m, 1 H).

### Example 200A 1-cyclohexyl-3-[2-(methylsulfonyl)benzyl]pyrrolidin-2-one

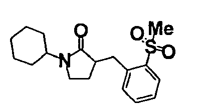

In the same manner as in Example 127A, the title compound was obtained from 1-cyclohexyl-3-[2-(methylthio)benzyl]pyrrolidin-2-one obtained in Example 197A.
1H NMR (300 MHz, CDCl₃) δ ppm 1.00-1.17 (m, 1 H), 1.29-1.47 (m, 4 H), 1.63-1.84 (m, 6 H), 1.96-2.08 (m, 2 H), 2.85-2.97 (m, 1 H), 3.10 (s, 3 H), 3.13-3.31 (m, 3 H), 3.53 (dd, J=13.9, 5.3 Hz, 1 H), 3.88-4.00 (m, 1 H), 7.37-7.44 (m, 1 H), 7.49-7.60 (m, 1 H), 8.07 (m, 1 H).

### Example 201A 3-[2-chloro-4-(trifluoromethyl)benzyl]-1-cyclohexylpyrrolidin-2-one

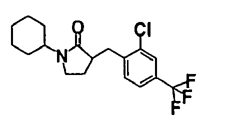

In the same manner as in Example 1A, the title compound was obtained from 1-cyclohexylpyrrolidin-2-one and 2-chloro-4-trifluoromethylbenzyl chloride.
1H NMR (300 MHz, CDCl₃) δ ppm 1.03-1.17 (m, 1 H), 1.26-1.47 (m, 4 H), 1.61-1.74 (m, 4 H), 1.74-1.85 (m, 2 H), 1.97-2.11 (m, 1 H), 2.75-2.91 (m, 2 H), 3.14-3.29 (m, 2 H), 3.34-3.46 (m, 1 H), 3.89-4.03 (m, 1 H), 7.40-7.47 (m, 2 H), 7.62 (brs, 1 H).

### Example 202A 1-cyclohexyl-3-(2,4-dichlorobenzyl)pyrrolidin-2-one

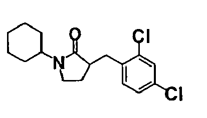

In the same manner as in Example 1A, the title compound was obtained from 1-cyclohexylpyrrolidin-2-one and 2,4-dichlorobenzyl chloride.
1H NMR (300 MHz, CDCl₃) δ ppm 0.98-1.18 (m, 1 H), 1.22-1.48 (m, 4 H), 1.57-1.85 (m, 6 H), 1.93-2.09 (m, 1 H), 2.69-2.85 (m, 2 H), 3.13-3.26 (m, 2 H), 3.27-3.38 (m, 1 H), 3.88-4.00 (m, 1 H), 7.14-7.19 (dd, J=8.3, 2.1 Hz, 1 H), 7.20-7.25 (d, J=8.3 Hz, 1 H), 7.37 (d, J=2.1 Hz, 1 H).

### Example 203A 3-(4-bromo-2-chlorobenzyl)-1-(1,4-dioxaspiro[4.5]dec-8-yl)pyrrolidin-2-one

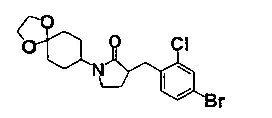

In the same manner as in Example 1A, the title compound was obtained from 1-(1,4-dioxaspiro[4.5]dec-8-yl)pyrrolidin-2-one and 4-bromo-2-chlorobenzyl bromide.
1H NMR (300 MHz, CDCl₃) δ ppm 1.59-1.84 (m, 9 H), 1.96-2.09 (m, 1 H), 2.71-2.84 (m, 2 H), 3.14-3.36 (m, 3 H), 3.90-3.96 (m, 4 H), 3.99-4.11 (m, 1 H), 7.16 (d, J=8.3 Hz, 1 H), 7.31 (dd, J=8.3, 2.0 Hz, 1 H), 7.51 (d, J=2.0 Hz, 1 H).

### Example 204A 3-(2-aminobenzyl)-1-cyclohexylpyrrolidin-2-one

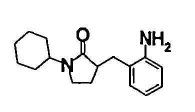

A mixture of 1-cyclohexyl-3-(2-nitrobenzyl)pyrrolidin-2-one obtained in Example 168A (0.17 g), 10% palladium-carbon (0.02 g), water (0.02 g) and ethanol (5 mL) was stirred at room temperature for 5 hr under hydrogen atmosphere. After the reaction system was substituted with nitrogen, the reaction mixture was filtrated through celite. The filtrate was concentrated under reduced pressure, ethyl acetate and water were added to the residue, and the mixture was extracted with ethyl acetate. The ethyl acetate layer was washed with saturated brine, dried over anhydrous magnesium sulfate, and concentrated under reduced pressure. The obtained residue was recrystallized from hexane-diisopropyl ether to give the title compound (90 mg, 60%) as a white solid.
1H NMR (300 MHz, CDCl₃) δ ppm 1.00-1.16 (m, 1 H), 1.24-1.47 (m, 4 H), 1.57-1.85 (m, 7 H), 2.03-2.18 (m, 1 H), 2.62-2.72 (m, 1 H), 2.72-2.84 (m, 1 H), 3.05 (dd, J=14.1, 3.8 Hz, 1 H), 3.13-3.23 (m, 2 H), 3.84-3.97 (m, 1 H), 4.06 (s, 2 H), 6.63-6.71 (m, 2 H), 6.96-7.08 (m, 2 H).

### Example 205A N-{2-[(1-cyclohexyl-2-oxopyrrolidin-3-yl)methyl]phenyl}acetamide

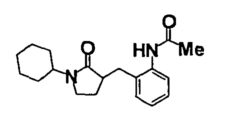

In the same manner as in Example 22A, the title compound was obtained from 3-(2-aminobenzyl)-1-cyclohexylpyrrolidin-2-one obtained in Example 204A and acetyl chloride.
1H NMR (300 MHz, CDCl₃) δ ppm 0.96-1.13 (m, 1 H), 1.19-1.54 (m, 5 H), 1.66 (s, 1 H), 1.71-1.92 (m, 4 H), 2.15-2.24 (m, 1 H), 2.26 (s, 3 H), 2.71-2.91 (m, 2 H), 3.03-3.26 (m, 3 H), 3.79-3.93 (m, 1 H), 6.99-7.13 (m, 2 H), 7.18-7.26 (m, 1 H), 7.89 (d, J=7.9 Hz, 1 H), 9.72 (s, 1 H).

### Example 206A N-{2-[(1-cyclohexyl-2-oxopyrrolidin-3-yl)methyl]phenyl}benzamide

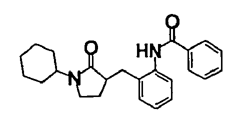

In the same manner as in Example 22A, the title compound was obtained from 3-(2-aminobenzyl)-1-cyclohexylpyrrolidin-2-one obtained in Example 204A and benzoyl chloride.
1H NMR (300 MHz, CDCl₃) δ ppm 0.93-1.45 (m, 6 H), 1.62-1.83 (m, 3 H), 1.86-2.04 (m, 1 H), 2.14-2.30 (m, 1 H), 2.75-2.96 (m, 2 H), 3.06-3.28 (m, 3 H), 3.78-3.94 (m, 1 H), 7.06-7.21 (m, 2 H), 7.24-7.33 (m, 2 H), 7.46-7.57 (m, 3 H), 7.77-7.86 (m, 1 H), 8.17-8.26 (m, 2 H), 10.31 (s, 1 H).

### Example 207A N-{2-[(1-cyclohexyl-2-oxopyrrolidin-3-yl)methyl]phenyl}-2-phenylacetamide

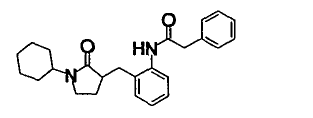

In the same manner as in Example 22A, the title compound was obtained from 3-(2-aminobenzyl)-1-cyclohexylpyrrolidin-2-one obtained in Example 204A and phenylacetyl chloride.
1H NMR (300 MHz, CDCl₃) δ ppm 0.96-1.16 (m, 1 H), 1.17-1.55 (m, 5 H), 1.60-1.84 (m, 5 H), 2.08-2.22 (m, 1 H), 2.64-2.84 (m, 2 H), 2.90-3.01 (m, 1 H), 3.06-3.25 (m, 2 H), 3.79 (s, 2 H), 3.82-3.97 (m, 1 H), 6.96-7.13 (m, 2 H), 7.15-7.28 (m, 2 H), 7.29-7.40 (m, 2 H), 7.49 (d, J=7.2 Hz, 2 H), 7.89 (d, J=8.1 Hz, 1 H), 9.62 (s, 1 H).

### Example 208A N-{2-[(1-cyclohexyl-2-oxopyrrolidin-3-yl)methyl]phenyl}methanesulfonamide

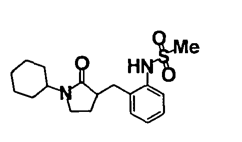

In the same manner as in Example 22A, the title compound was obtained from 3-(2-aminobenzyl)-1-cyclohexylpyrrolidin-2-one obtained in Example 204A and methanesulfonyl chloride.
1H NMR (300 MHz, CDCl₃) δ ppm 0.98-1.14 (m, 1 H), 1.19-1.42 (m, 4 H), 1.44-1.90 (m, 6 H), 2.17-2.30 (m, 1 H), 2.71-2.82 (m, 1 H), 2.89-2.97 (m, 1 H), 3.02 (s, 3 H), 3.03-3.11 (m, 1 H), 3.16-3.24 (m, 2 H), 3.84-3.99 (m, 1 H), 7.07-7.17 (m, 2 H), 7.20-7.29 (m, 1 H), 7.50-7.55 (m, 1 H), 9.74 (s, 1 H).

### Example 209A 1-cyclohexyl-3-[2-(hydroxymethyl)benzyl]pyrrolidin-2-one

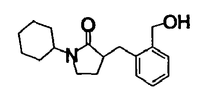

A solution of methyl 2-[(1-cyclohexyl-2-oxopyrrolidin-3-yl)methyl]benzoate obtained in Example 150A (0.11 g) in dichloromethane (5 mL) was cooled to -20°C, and diisobutylaluminum hydride (1.0 M hexane solution, 1.23 mL) was gradually added dropwise. The reaction mixture was stirred at room temperature for 4 hr, saturated aqueous sodium potassium (+)-tartrate was added, and the mixture was further stirred at room temperature overnight. The reaction mixture was extracted with dichloromethane, the dichloromethane layer was washed with saturated brine, dried over anhydrous magnesium sulfate, and concentrated under reduced pressure. The obtained residue was silica gel column chromatography with methanol-ethyl acetate (3:97) to give the title compound (31mg, 31%) as an oil.
1H NMR (300 MHz, CDCl₃) δ ppm 0.98-1.15 (m, 1 H), 1.23-1.46 (m, 4 H), 1.60-1.71 (m, 2 H), 1.72-1.84 (m, 3 H), 1.90 (brs, 1 H), 2.09-2.22 (m, 1 H), 2.65-2.84 (m, 2 H), 3.14-3.38 (m, 4 H), 3.85-3.97 (m, 1 H), 4.63-4.81 (m, 2 H), 7.17-7.26 (m, 3 H), 7.33-7.43 (m, 1 H).

### Example 210A N-{2-[(1-cyclohexyl-2-oxopyrrolidin-3-yl)methyl]phenyl}-N'-ethylurea

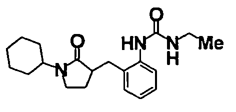

To a solution of 3-(2-aminobenzyl)-1-cyclohexylpyrrolidin-2-one obtained in Example 204A (0.19 g) in toluene (5 mL) was added ethyl isocyanate (61 µL) under nitrogen atmosphere, and the mixture was stirred at room temperature for 3 hr, then at 50°C for 3 hr, and then at 100°C for 3 hr. After cooling, ethyl acetate and water were added to the reaction mixture, and the mixture was extracted with ethyl acetate. The ethyl acetate layer was washed with saturated brine, dried over anhydrous magnesium sulfate, and concentrated under reduced pressure. The obtained residue was NH-silica gel column chromatography with ethyl acetate-hexane to give the title compound (0.16 g, 65%) as an amorphous form.
1H NMR (300 MHz, CDCl₃) δ ppm 1.01-1.14 (m, 1 H), 1.18 (t, J=7.2 Hz, 3 H), 1.24-1.45 (m, 4 H), 1.53-1.86 (m, 6 H), 2.13-2.25 (m, 1 H), 2.65-2.76 (m, 1 H), 2.86-3.03 (m, 2 H), 3.16-3.24 (m, 2 H), 3.26-3.37 (m, 2 H), 3.82-3.95 (m, 1 H), 5.11-5.18 (m, 1 H), 6.87-6.94 (m, 1 H), 7.01-7.06 (m, 1 H), 7.16-7.24 (m, 1 H), 8.05 (d, J=8.1 Hz, 1H), 8.26 (s, 1H) .

### Example 211A N-{-2-[(1-cyclohexyl-2-oxopyrrolidin-3-yl)methyl]phenyl}-N-methylacetamide

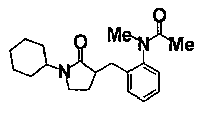

To a solution of N-{2-[(1-cyclohexyl-2-oxopyrrolidin-3-yl)methyl]phenyl}acetamide obtained in Example 205A (0.26 g) in N,N-dimethylformaldehyde (5 mL) were added successively 60% sodium hydride (40 mg) and methyl iodide (0.15 mL), and the mixture was stirred at room temperature for 10 hr. The reaction mixture was concentrated under reduced pressure, ethyl acetate and water were added to the residue, and the mixture was extracted with ethyl acetate. The ethyl acetate layer was washed with saturated brine, dried over anhydrous magnesium sulfate, and concentrated under reduced pressure. The obtained residue was NH-silica gel column chromatography with ethyl acetate-hexane (25:75-75:25) to give the title compound (0.20 g, 75%) as an oil.
1H NMR (300 MHz, CDCl₃) δ ppm 1.03-1.18 (m, 1 H), 1.21-1.46 (m, 4 H), 1.60-1.87 (m, 9 H), 1.98-2.18 (m, 1 H), 2.46-2.68 (m, 1 H), 2.72-2.89 (m, 1 H), 3.13-3.35 (m, 6 H), 3.90-4.02 (m, 1 H), 7.08-7.15 (m, 1 H), 7.22-7.44 (m, 3 H).

### Example 212A N-{2-[(1-cyclohexyl-2-oxopyrrolidin-3-yl)methyl]benzyl}acetamide

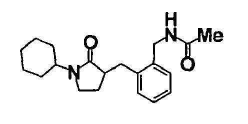

In the same manner as in Example 22A, the title compound was obtained from 3-[2-(aminomethyl)benzyl]-1-cyclohexylpyrrolidin-2-one obtained in Example 165A and acetyl chloride.
1H NMR (300 MHz, CDCl₃) δ ppm 1.03-1.15 (m, 1 H), 1.28-1.48 (m, 4 H), 1.60-1.85 (m, 6 H), 1.97 (s, 3 H), 2.14-2.27 (m, 1 H), 2.65-2.79 (m, 2 H), 3.14-3.33 (m, 3 H), 3.84-3.97 (m, 1 H), 4.36-4.53 (m, 2 H), 6.85 (brs, 1 H), 7.14-7.24 (m, 3 H), 7.29-7.38 (m, 1 H).

### Example 213A 3-[2-chloro-4-(methylamino)benzyl]-1-cyclohexylpyrrolidin-2-one

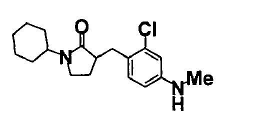

In the same manner as in Example 20A, the title compound was obtained from tert-butyl 13-chloro-4-[(1-cyclohexyl-2-oxopyrrolidin-3-yl)methyl]phenyl}(methyl)carbamate obtained in Example 196A.
1H NMR (300 MHz, CDCl₃)δ ppm 1.04-1.14 (m, 1 H), 1.26-1.44 (m, 4 H), 1.64-1.80 (m, 6 H), 1.92-2.05 (m, 1 H), 2.63-2.78 (m, 1 H), 2.80 (s, 3 H), 3.06-3.27 (m, 4 H), 3.71 (brs, 1 H), 3.91-3.98 (m, 1 H), 6.51 (dd, J=8.1, 2.4 Hz, 1 H), 6.59 (d, J=2.4 Hz, 1 H), 7.05 (d, J=8.1 Hz, 1 H).

### Example 214A N-{3-chloro-4-[(1-cyclohexyl-2-oxopyrrolidin-3-yl)methyl]phenyl}-N-methylacetamide

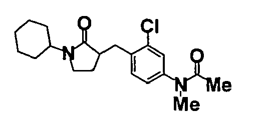

In the same manner as in Example 22A, the title compound was obtained from 3-[2-chloro-4-(methylamino)benzyl]-1-cyclohexylpyrrolidin-2-one obtained in Example 213A and acetyl chloride.
1H NMR (300 MHz, CDCl₃) δ ppm 1.01-1.18 (m, 1 H), 1.25-1.49 (m, 4 H), 1.63-1.83 (m, 6 H); 1.89 (s, 3 H), 1.98-2.16 (m, 1 H), 2.73-2.90 (m, 2 H), 3.12-3.28 (m, 5 H), 3.30-3.44 (m, 1 H), 3.87-4.03 (m, 1 H), 7.03 (dd, J=8.0, 2.0 Hz, 1 H), 7.22 (d, J=1.9 Hz, 1 H), 7.35 (d, J=8.1 Hz, 1 H).

### Example 215A 3-(4-amino-2-chlorobenzyl)-1-cyclohexylpyrrolidin-2-one

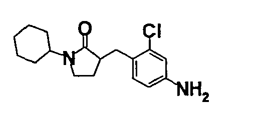

To a solution of 3-(4-bromo-2-chlorobenzyl)-1-cyclohexylpyrrolidin-2-one obtained in Example 193A (0.26 g), tris(dibenzylideneacetone)dipalladium (37 mg), 2-(dicyclohexylphosphino)-2',4',6'-triisopropyl-1,1'-biphenyl (38 mg) and potassium tert-butoxide (0.10 g) in tert-butyl alcohol (5 mL) was added benzophenonimine (0.15 mL) under argon atmosphere, and the mixture was heated under reflux overnight. After cooling, saturated aqueous ammonium chloride was added to the reaction mixture, and the mixture was extracted with ethyl acetate. The ethyl acetate layer was washed with saturated brine, dried over anhydrous magnesium sulfate, and concentrated under reduced pressure. The obtained residue was subjected to silica gel column chromatography with ethyl acetate-hexane (10:90-40:60) to give 3-{2-chloro-4-[(diphenylmethylene)amino]benzyl}-1-cyclohexylpyrrolidin-2-one (0.27 g, 81%) as an oil. The obtained compound (0.25 g) was dissolved in methanol (5 mL), sodium acetate (0.10 g) and hydroxylamine hydrochloride (65 mg) were added, and the mixture was stirred at room temperature for 1 hr. 1N Sodium hydroxide, ethyl acetate and water were added to the reaction mixture, and the mixture was extracted with ethyl acetate. The ethyl acetate layer was washed with saturated brine, dried over anhydrous magnesium sulfate, and concentrated under reduced pressure. The obtained residue was subjected to silica gel column chromatography with ethyl acetate-hexane (30:70-80:20.), and the obtained crystals were recrystallized from hexane-ethyl acetate to give the title compound (64 mg, 40%) as a white solid.
1H NMR (300 MHz, CDCl₃) δ ppm 0.99-1.15 (m, 1 H), 1.23-1.46 (m, 4 H), 1.60-1.85 (m, 6 H), 1.90-2.04 (m, 1 H), 2.62-2.81 (m, 2 H), 3.10-3.29 (m, 3 H), 3.63 (brs, 2 H), 3.87-4.01 (m, 1 H), 6.51 (dd, J=8.2, 2.4 Hz, 1 H), 6.69 (d, J=2.4 Hz, 1 H), 7.04 (d,

J=8.2 Hz, 1 H).

### Example 216A 3-[2-chloro-6-(2-hydroxy-2-methylpropoxy)benzyl]-1-cyclohexylpyrrolidin-2-one

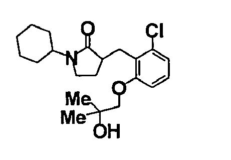

A solution of 3-[2-chloro-6-(2-oxopropoxy)benzyl]-1-cyclohexylpyrrolidin-2-one obtained in Example 181A (0.10 g) in tetrahydrofuran (5 mL) was cooled to 0°C, methylmagnesium bromide (3.0 M ether solution, 0.27 mL) was added, and the mixture was stirred at 0°C for 1 hr, and then at room temperature for 4.5 hr. Ethyl acetate and saturated aqueous ammonium chloride were added to the reaction mixture, and the mixture was extracted with ethyl acetate. The ethyl acetate layer was washed with saturated brine, dried over anhydrous magnesium sulfate, and concentrated under reduced pressure. The obtained residue was subjected to silica gel column chromatography with 2-butanone-hexane (1:3), and purified by normal phase high performance liquid chromatography to give the title compound (25 mg, 24%) as an oil.
1H NMR (300 MHz, CDCl₃) δ ppm 0.97-1.17 (m, 1 H), 1.23-1.39 (m, 5 H), 1.33 (s, 3 H), 1.43 (s, 3 H), 1.60-1.82 (m, 5 H), 1.85-2.00 (m, 1 H), 2.05-2.22 (m, 1 H), 2.56-2.71 (m, 1 H), 2.91 (m, 1 H), 3.12-3.31 (m, 2 H), 3.47 (m, 1 H), 3.69-3.84 (m, 2 H), 3.91-4.04 (m, 1 H), 6.68-6.75 (m, 1 H), 6.94-7.00 (m, 1 H), 7.10 (t, J=8.2 Hz, 1 H).

### Example 217A 3-[2-chloro-4-(dimethylamino)benzyl]-1-cyclohexylpyrrolidin-2-one

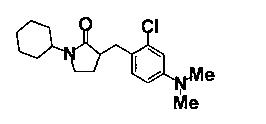

A mixture of 3-[2-chloro-4-(methylamino)benzyl]-1-cyclohexylpyrrolidin-2-one obtained in Example 213A (0.13 g), concentrated sulfuric acid (28µL), 37% aqueous formic acid (92 mL) and water (1.5 mL) was cooled to 0°C, a solution of sodium borohydride in tetrahydrofuran (5 mL) was added, and the mixture was stirred at room temperature overnight. 1N Aqueous sodium hydroxide solution was added to the reaction mixture, ethyl acetate and water were added, and the mixture was extracted with ethyl acetate. The ethyl acetate layer was washed with saturated brine, dried over anhydrous magnesium sulfate, and concentrated under reduced pressure. The residue was recrystallized from ethyl acetate-hexane to give the title compound (77 mg, 56%) as a pale-brown solid.
1H NMR (300 MHz, CDCl₃) δ ppm 1.01-1.15 (m, 1 H), 1.25-1.47 (m, 4 H), 1.61-1.84 (m, 6 H), 1.91-2.04 (m, 1 H), 2.64-2.81 (m, 2 H), 2.91 (s, 6 H), 3.11-3.21 (m, 2 H), 3.22-3.29 (m, 1 H), 3.89-4.01 (m, 1 H), 6.56 (dd, J=8.6, 2.6 Hz, 1 H), 6.69 (d, J=2.6 Hz, 1 H), 7.10 (d, J=8.6 Hz, 1 H).

### Example 218A 3-[(3-chlorobiphenyl-4-yl)methyl]-1-cyclohexylpyrrolidin-2-one

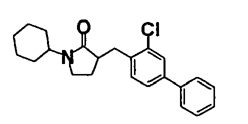

A mixture of 3-(4-bromo-2-chlorobenzyl)-1-cyclohexylpyrrolidin-2-one obtained in Example 193A (0.30 g), phenylboronic acid (0.15 g), 2 M aqueous sodium hydrogencarbonate (0.81 mL), tetrakis(triphenylphosphine)palladium (19 mg) and 1,2-dimethoxyethane (5 mL) was stirred overnight at 90°C under nitrogen atmosphere. Ethyl acetate and water were added to the reaction mixture, and the mixture was extracted with ethyl acetate. The ethyl acetate layer was washed with saturated brine, dried over anhydrous magnesium sulfate, and concentrated under reduced pressure. The residue was subjected to silica gel column chromatography with ethyl acetate-hexane (10:90-40:60), and the obtained solid was recrystallized from ethyl acetate-hexane to give the title compound (0.14g, 48%) as a white solid.
1H NMR (300 MHz, CDCl₃) δ ppm 1.00-1.18 (m, 1 H), 1.25-1.49 (m, 4 H), 1.62-1.86 (m, 6 H), 1.97-2.12 (m, 1 H), 2.78-2.91 (m, 2 H), 3.14-3.30 (m, 2 H), 3.35-3.47 (m, 1 H), 3.91-4.03 (m, 1 H), 7.32-7.47 (m, 5 H), 7.53-7.60 (m, 3 H).

### Example 219A 3-(2-chloro-4-pyridin-4-ylbenzyl)-1-cyclohexylpyrrolidin-2-one

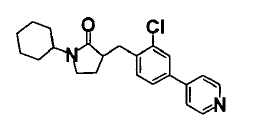

In the same manner as in Example 218A, the title compound was obtained from 3-(4-bromo-2-chlorobenzyl)-1-cyclohexylpyrrolidin-2-one obtained in Example 193A and 4-pyridylboronic acid.
1H NMR (300 MHz, CDCl₃) δ ppm 0.99-1.19 (m, 1 H), 1.24-1.49 (m, 4 H), 1.63-1.87 (m, 6 H), 1.99-2.13 (m, 1 H), 2.78-2.92 (m, 2 H), 3.15-3.30 (m, 2 H), 3.35-3.47 (m, 1 H), 3.90-4.03 (m, 1 H), 7.39-7.50 (m, 4 H), 7.62-7.66 (m, 1 H), 8.65-8.69 (m, 2 H).

### Example 220A 3-{[3-chloro-4'-(methylsulfonyl)biphenyl-4-yl]methyl}-1-cyclohexylpyrrolidin-2-one

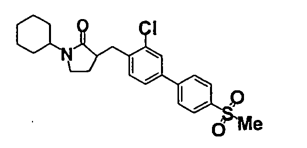

In the same manner as in Example 218A, the title compound was obtained from 3-(4-bromo-2-chlorobenzyl)-1-cyclohexylpyrrolidin-2-one obtained in Example 193A and 4-methylsulfonylphenylboronic acid.
1H NMR (300 MHz, CDCl₃) δ ppm 0.99-1.18 (m, 1 H), 1.25-1.51 (m, 4 H), 1.63-1.87 (m, 6 H), 2.00-2.16 (m, 1 H), 2.79-2.93 (m, 2 H), 3.10 (s, 3 H), 3.17-3.33 (m, 2 H), 3.34-3.48 (m, 1 H), 3.88-4.05 (m, 1 H), 7.38-7.47 (m, 2 H), 7.58-7.64 (m, 1 H), 7.70-7.78 (m, 2 H), 7.97-8.05 (m, 2 H).

### Example 221A 3-{4-[benzyl(methyl)amino]-2-chlorobenzyl}-1-cyclohexylpyrrolidin-2-one

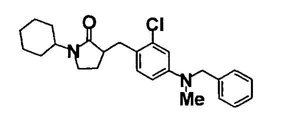

To a solution of 3-(4-bromo-2-chlorobenzyl)-1-cyclohexylpyrrolidin-2-one obtained in Example 193A (0.25 g), tris(dibenzylideneacetone)dipalladium (30 mg), 2-(dicyclohexylphosphino)-2',4',6'-triisopropyl-1,1'-biphenyl (32 mg) and potassium tert-butoxide (98 mg) in tert-butyl alcohol (5 mL) was added N-methylbenzylamine (0.11 mL) under argon atmosphere, and the mixture was heated under reflux overnight. After cooling, saturated aqueous ammonium chloride was added to the reaction mixture, and the mixture was extracted with ethyl acetate. The ethyl acetate layer was washed with saturated brine, dried over anhydrous magnesium sulfate, and concentrated under reduced pressure. The residue was subjected to silica gel column chromatography with ethyl acetate-hexane (10:90-40:60) to give the title compound (0.20 g, 73%) as a brown oil.
1H NMR (300 MHz, CDCl₃) δ ppm 1.03-1.16 (m, 1 H), 1.28-1.43 (m, 4 H), 1.62-1.84 (m, 6 H), 1.91-2.04 (m, 1 H), 2.62-2.81 (m, 2 H), 2.96-3.00 (m, 3 H), 3.11-3.31 (m, 3 H), 3.88-4.01 (m, 1 H), 4.49 (s, 2 H), 6.56 (dd, J=8.6, 2.7 Hz, 1 H), 6.72 (d, J=2.7 Hz, 1 H), 7.07 (d, J=8.6 Hz, 1 H), 7.17-7.37 (m, 5 H).

### Example 222A 3-13-chloro-4-[(1-cyclohexyl-2-oxopyrrolidin-3-yl)methyl]phenyl}-1,3-oxazolidin-2-one

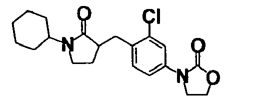

To a solution of 3-(4-bromo-2-chlorobenzyl)-1-cyclohexylpyrrolidin-2-one obtained in Example 193A (0.25 g), tris(dibenzylideneacetone)dipalladium (25 mg), 2-(dicyclohexylphosphino)-2',4',6'-triisopropyl-1,1'-biphenyl (26 mg) and cesium carbonate (0.31 g) in toluene (5 mL) was added 2-oxazolidone (58 mg), and the mixture was heated under reflux overnight. After cooling, saturated aqueous ammonium chloride was added to the reaction mixture, and the mixture was extracted with ethyl acetate. The ethyl acetate layer was washed with saturated brine, dried over anhydrous magnesium sulfate, and concentrated under reduced pressure. The obtained residue was filtrated through silica gel, and recrystallized from hexane-ethyl acetate to give the title compound (0.11 g, 45%) as a pale-yellow solid.
1H NMR (300 MHz, CDCl₃) δ ppm 1.02-1.17 (m, 1 H), 1.23-1.44 (m, 4 H), 1.61-1.84 (m, 6 H), 1.94-2.05 (m, 1 H), 2.72-2.85 (m, 2 H), 3.13-3.27 (m, 2 H), 3.28-3.39 (m, 1 H), 3.88-3.99 (m, 1 H), 3.99-4.07 (m, 2 H), 4.45-4.53 (m, 2 H), 7.27-7.31 (d, J=8.6 Hz, 1 H), 7.35-7.41 (dd, J=8.6, 2.4 Hz, 1 H), 7.59 (d, J=2.4 Hz, 1 H).

### Example 223A 3-(4-anilino-2-chlorobenzyl)-1-cyclohexylpyrrolidin-2-one

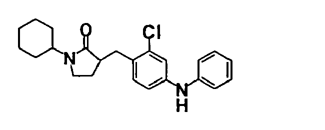

In the same manner as in Example 221A, the title compound was obtained from 3-(4-bromo-2-chlorobenzyl)-1-cyclohexylpyrrolidin-2-one obtained in Example 193A and aniline.
1H NMR (300 MHz, CDCl₃) δ ppm 1.02-1.16 (m, 1 H), 1.24-1.46 (m, 5 H), 1.61-1.83 (m, 6 H), 1.90-2.03 (m, 1 H), 2.61-2.80 (m, 2 H), 3.12-3.29 (m, 3 H), 4.22-4.33 (m, 2 H), 6.64 (m, 1 H), 6.98-7.07 (m, 1 H), 7.27-7.37 (m, 5 H).

### Example 224A 3-[4-(benzylamino)-2-chlorobenzyl]-1-cyclohexylpyrrolidin-2-one

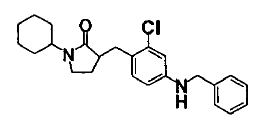

In the same manner as in Example 221A, the title compound was obtained from 3-(4-bromo-2-chlorobenzyl)-1-cyclohexylpyrrolidin-2-one obtained in Example 193A and benzylamine.
1H NMR (300 MHz, CDCl₃) δ ppm 1.02-1.16 (m, 1 H), 1.24-1.46 (m, 5 H), 1.61-1.83 (m, 6 H), 1.90-2.03 (m, 1 H), 2.61-2.80 (m, 2 H), 3.12-3.29 (m, 3 H), 3.87-4.07 (m, 2 H), 4.22-4.33 (m, 2 H), 6.64 (m, 1 H), 6.98-7.07 (m, 1 H), 7.27-7.37 (m, 5 H).

### Example 225A 3-{2-chloro-4-[(2-hydroxyethyl)(methyl)amino]benzyl}-1-cyclohexylpyrrolidin-2-one

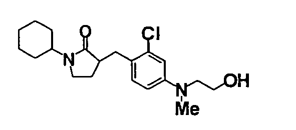

In the same manner as in Example 221A, the title compound was obtained from 3-(4-bromo-2-chlorobenzyl)-1-cyclohexylpyrrolidin-2-one obtained in Example 193A and 2-(N-methylamino)ethanol.
1H NMR (300 MHz, CDCl₃) δ ppm 1.01-1.18 (m, 1 H), 1.23-1.49 (m, 4 H), 1.58-1.85 (m, 7 H), 1.89-2.07 (m, 1 H), 2.62-2.82 (m, 2 H), 2.94 (s, 3 H), 3.14-3.30 (m, 3 H), 3.44 (t, J=5.7 Hz, 2 H), 3.81 (t, J=5.7 Hz, 2 H), 3.88-4.02 (m, 1 H), 6.62 (dd, J=8.6, 2.7 Hz, 1 H), 6.75 (d, J=2.7 Hz, 1 H), 7.10 (d, J=8.6 Hz, 1 H).

### Example 226A 3-[4-(4-acetylpiperazin-1-yl)-2-chlorobenzyl]-1-cyclohexylpyrrolidin-2-one

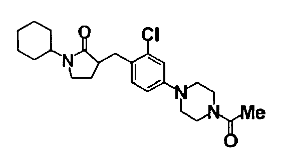

In the same manner as in Example 221A, the title compound was obtained from 3-(4-bromo-2-chlorobenzyl)-1-cyclohexylpyrrolidin-2-one obtained in Example 193A and N-acetylpiperazine.
1H NMR (300 MHz, CDCl₃) δ ppm 1.03-1.16 (m, 1 H), 1.24-1.46 (m, 4 H), 1.61-1.84 (m, 6 H), 1.92-2.04 (m, 1 H), 2.14 (s, 3 H), 2.66-2.80 (m, 2 H), 3.08-3.33 (m, 7 H), 3.56-3.64 (m, 2 H), 3.71-3.80 (m, 2 H), 3.87-4.03 (m, 1 H), 6.75 (dd, J=8.5, 2.5 Hz, 1 H), 6.89 (d, J=2.5 Hz, 1 H), 7.17 (d, J=8.5 Hz, 1 H).

### Example 227A 3-{2-chloro-4-[4-(2-hydroxyethyl)piperazin-1-yl]benzyl}-1-cyclohexylpyrrolidin-2-one

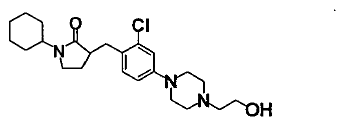

In the same manner as in Example 221A, the title compound was obtained from 3-(4-bromo-2-chlorobenzyl)-1-cyclohexylpyrrolidin-2-one obtained in Example 193A and N-(2-hydroxyethyl)piperazine.
1H NMR (300 MHz, CDCl₃) δ ppm 1.03-1.17 (m, 1 H), 1.22-1.47 (m, 5 H), 1.61-2.06 (m, 7 H), 2.57-2.80 (m, 8 H), 3.13-3.22 (m, 6 H), 3.23-3.31 (m, 1 H), 3.62-3.71 (m, 2 H), 3.87-4.01 (m, 1 H), 6.75 (dd, J=8.7, 2.6 Hz, 1 H), 6.88 (d, J=2.6 Hz, 1 H), 7.14 (d, J=8.7 Hz, 1 H).

### Example 228A 1-cyclohexyl-3-[(2,4-dimethoxyphenyl)(hydroxy)methyl]pyrrolidin-2-one

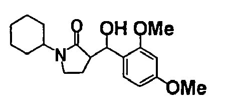

In the same manner as in Example 118A, the title compound was obtained from 1-cyclohexylpyrrolidin-2-one and 2,4-dimethoxybenzaldehyde.
1H NMR (300 MHz, CDCl₃) δ ppm 0.97-1.16 (m, 1 H), 1.19-1.46 (m, 4 H), 1.63-1.83 (m, 4 H), 1.84-1.99 (m, 1 H), 2.99-3.08 (m, 1 H), 3.15 (m, 2 H), 3.27-3.32 (m, 1 H), 3.79 (s, 3 H), 3.80 (s, 3 H), 3.86-4.05 (m, 1 H), 5.50-5.54 (m, 1 H), 6.43 (d, J=2.4 Hz, 1 H), 6.48 (dd, J=8.5, 2.4 Hz, 1 H), 7.34 (d, J=8.5 Hz, 1 H).

### Example 229A N-{3-chloro-4-[(1-cyclohexyl-2-oxopyrrolidin-3-yl)methyl]phenyl}-N'-ethylurea

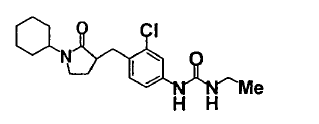

In the same manner as in Example 210A, the title compound was obtained from 3-(4-amino-2-chlorobenzyl)-1-cyclohexylpyrrolidin-2-one obtained in Example 215A and ethyl isocyanate.
1H NMR (300 MHz, CDCl₃) δ ppm 1.02-1.19 (m, 1 H), 1.15 (t, J=7.3 Hz, 3 H), 1.23-1.46 (m, 4 H), 1.60-1.87 (m, 6 H), 1.93-2.09 (m, 1 H), 2.67-2.86 (m, 2 H), 3.14-3.34 (m, 5 H), 3.82-4.01 (m, 1 H), 5.22-5.25 (m, 1 H), 7.01-7.16 (m, 3 H), 7.43 (d, J=1.9 Hz, 1 H).

### Example 230A N-{3-chloro-4-[(1-cyclohexyl-2-oxopyrrolidin-3-yl)methyl]phenyl}methanesulfonamide

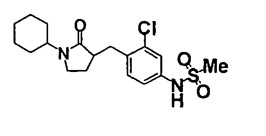

In the same manner as in Example 22A, the title compound was obtained from 3-(4-amino-2-chlorobenzyl)-1-cyclohexylpyrrolidin-2-one obtained in Example 215A and methanesulfonyl chloride.
1H NMR (300 MHz, CDCl₃) δ ppm 1.00-1.19 (m, 1 H), 1.23-1.47 (m, 4 H), 1.56-1.87 (m, 6 H), 1.96-2.12 (m, 1 H), 2.71-2.86 (m, 2 H), 3.02 (s, 3 H), 3.14-3.36 (m, 3 H), 3.86-4.02 (m, 1 H), 6.86 (s, 1 H), 7.01-7.08 (m, 1 H), 7.23-7.31 (m, 3 H).

### Example 231A N-{3-chloro-4-[(1-cyclohexyl-2-oxopyrrolidin-3-yl)methyl]phenyl}-N-(methylsulfonyl)methanesulfonamide

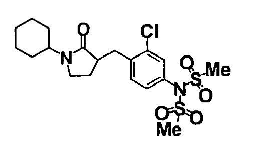

In the same manner as in Example 22A, the title compound was obtained from 3-(4-amino-2-chlorobenzyl)-1-cyclohexylpyrrolidin-2-one obtained in Example 215A and methanesulfonyl chloride.
1H NMR (300 MHz, CDCl₃) δ ppm 1.02-1.17 (m, 1 H), 1.28-1.47 (m, 4 H), 1.61-1.87 (m, 6 H), 2.01-2.13 (m, 1 H), 2.73-2.87 (m, 2 H), 3.14-3.27 (m, 2 H), 3.34-3.47 (m, 1 H), 3.40-3.41 (m, 6 H), 3.85-4.01 (m, 1 H), 7.20 (dd, J=8.3, 2.3 Hz, 1 H), 7.37-7.43 (m, 2 H).

### Example 232A N-{3-chloro-4-[(1-cyclohexyl-2-oxopyrrolidin-3-yl)methyl]phenyl}benzenesulfonamide

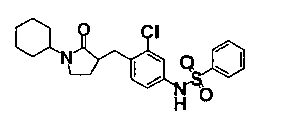

In the same manner as in Example 22A, the title compound was obtained from 3-(4-amino-2-chlorobenzyl)-1-cyclohexylpyrrolidin-2-one obtained in Example 215A and benzenesulfonyl chloride.
1H NMR (300 MHz, CDCl₃) δ ppm 1.00-1.17 (m, 1 H), 1.23-1.46 (m, 4 H), 1.56-1.84 (m, 6 H), 1.90-2.04 (m, 1 H), 2.66-2.85 (m, 2 H), 3.14-3.29 (m, 3 H), 3.86-4.00 (m, 1 H), 6.88 (dd, J=8.3, 2.3 Hz, 1 H), 7.09-7.16 (m, 3 H), 7.40-7.49 (m, 2 H), 7.51-7.60 (m, 1 H), 7.74-7.84 (m, 2 H).

### Example 233A N-{3-chloro-4-[(1-cyclohexyl-2-oxopyrrolidin-3-yl)methyl]phenyl}-N-(phenylsulfonyl)benzenesulfonamide

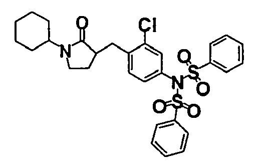

In the same manner as in Example 22A, the title compound was obtained from 3-(4-amino-2-chlorobenzyl)-1-cyclohexylpyrrolidin-2-one obtained in Example 215A and benzenesulfonyl chloride.
1H NMR (300 MHz, CDCl₃) δ ppm 1.02-1.17 (m, 1 H), 1.29-1.47 (m, 4 H), 1.61-1.87 (m, 6 H), 1.99-2.14 (m, 1 H), 2.73-2.89 (m, 2 H), 3.15-3.27 (m, 2 H), 3.34-3.47 (m, 1 H), 3.88-4.03 (m, 1 H), 6.85 (dd, J=8.2, 2.2 Hz, 1 H), 7.03 (d, J=2.3 Hz, 1 H), 7.26-7.31 (m, 1 H), 7.52-7.62 (m, 4 H), 7.65-7.75 (m, 2 H), 7.89-7.99 (m, 4 H) .

### Example 234A N-{3-chloro-4-[(1-cyclohexyl-2-oxopyrrolidin-3-yl)methyl]phenyl}benzamide

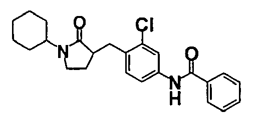

In the same manner as in Example 22A, the title compound was obtained from 3-(4-amino-2-chlorobenzyl)-1-cyclohexylpyrrolidin-2-one obtained in Example 215A and benzoyl chloride.
1H NMR (300 MHz, CDCl₃) δ ppm 1.00-1.17 (m, 1 H), 1.24-1.45 (m, 4 H), 1.61-1.85 (m, 6 H), 1.93-2.07 (m, 1 H), 2.71-2.86 (m, 2 H), 3.12-3.26 (m, 2 H), 3.27-3.40 (m, 1 H), 3.87-4.01 (m, 1 H), 7.24 (d, J=8.3 Hz, 1 H), 7.39-7.60 (m, 4 H), 7.81 (d, J=2.3 Hz, 1 H), 7.85-7.90 (m, 2 H), 8.05 (s, 1 H).

### Example 235A N-{3-chloro-4-[(1-cyclohexyl-2-oxopyrrolidin-3-yl)methyl]phenyl}-2-(1,3-dioxo-1,3-dihydro-2H-isoindol-2-yl)ethanesulfonamide

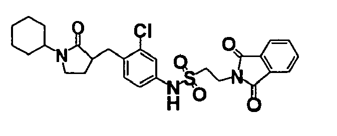

In the same manner as in Example 22A, the title compound was obtained from 3-(4-amino-2-chlorobenzyl)-1-cyclohexylpyrrolidin-2-one obtained in Example 215A and 2-(N-phthaloylamino)ethylsulfonyl chloride.
1H NMR (300 MHz, CDCl₃) δ ppm 1.03-1.16 (m, 1 H), 1.28-1.47 (m, 4 H), 1.60-1.84 (m, 6 H), 1.95-2.08 (m, 1 H), 2.67-2.83 (m, 2 H), 3.14-3.32 (m, 3 H), 3.44-3.51 (m, 2 H), 3.89-4.01 (m, 1 H), 4.07-4.14 (m, 2 H), 7.12-7.17 (dd, J=8.3, 2.2 Hz, 1 H), 7.21-7.25 (d, J=8.3, 1 H), 7.32 (d, J=2.2 Hz, 1 H), 7.50 (s, 1 H), 7.70-7.79 (m, 2 H), 7.82-7.88 (m, 2 H).

### Example 236A 2-({3-chloro-4-[(1-cyclohexyl-2-oxopyrrolidin-3-yl)methyl]phenyl}amino)-2-oxoethyl acetate

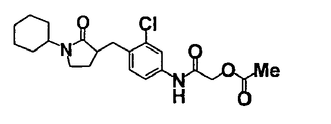

In the same manner as in Example 22A, the title compound was obtained from 3-(4-amino-2-chlorobenzyl)-1-cyclohexylpyrrolidin-2-one obtained in Example 215A and 2-acetoxyacetyl chloride.
1H NMR (300 MHz, CDCl₃) δ ppm 1.03-1.17 (m, 1 H), 1.26-1.45 (m, 4 H), 1.61-1.86 (m, 6 H), 1.94-2.07 (m, 1 H), 2.24 (s, 3 H), 2.72-2.85 (m, 2 H), 3.13-3.26 (m, 2 H), 3.26-3.40 (m, 1 H), 3.85-4.02 (m, 1 H), 4.68 (s, 2 H), 7.21-7.26 (d, J=8.3 Hz, 1 H), 7.30-7.37 (dd, J=8.3, 2.2 Hz, 1 H), 7.67 (d, J=2.2 Hz, 1 H), 7.94 (brs, 1 H).

### Example 237A N-{3-chloro-4-[(1-cyclohexyl-2-oxopyrrolidin-3-yl)methyl]phenyl}-2-hydroxyacetamide

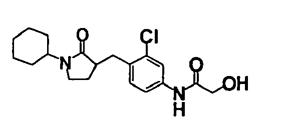

To a solution of 2-({3-chloro-4-[(1-cyclohexyl-2-oxopyrrolidin-3-yl)methyl]phenyl}amino)-2-oxoethyl acetate obtained in Example 236A (0.20 g) in tetrahydrofuran (5 mL) was added 2N aqueous potassium hydroxide solution (0.98 mL), and the mixture was stirred overnight at room temperature. 1N Hydrochloric acid was added to the reaction mixture, and the mixture was extracted with ethyl acetate. The ethyl acetate layer was washed with saturated brine, dried over anhydrous magnesium sulfate, and concentrated under reduced pressure to give the title compound (0.17 g, 95%) as a white solid.
1H NMR (300 MHz, CDCl₃) δ ppm 1.02-1.18 (m, 1 H), 1.29-1.46 (m, 4 H), 1.60-1.85 (m, 6 H), 1.95-2.09 (m, 1 H), 2.66-2.85 (m, 2 H), 3.15-3.32 (m, 3 H), 3.85-3.98 (m, 1 H), 3.98-4.05 (m, 1 H), 4.16-4.25 (m, 2 H), 7.15 (d, J=8.4 Hz, 1 H), 7.27-7.31 (dd, J=8.4, 2.2 Hz, 1 H), 7.68 (d, J=2.2 Hz, 1 H), 8.53 (s, 1 H).

### Example 238A ethyl N-[((3-chloro-4-[(1-cyclohexyl-2-oxopyrrolidin-3-yl)methyl]phenyl}amino)carbonyl]glycinate

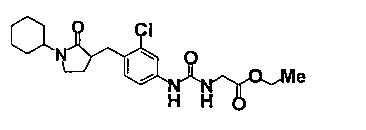

To a solution of 3-(4-amino-2-chlorobenzyl)-1-cyclohexylpyrrolidin-2-one obtained in Example 215A (0.60 g) in pyridine (3 mL) was added ethyl isocyanatoacetate (0.33 mL), and the mixture was stirred at 50°C for 3 hr. After cooling, the reaction mixture was concentrated, 1N hydrochloric acid and ethyl acetate were added, and the mixture was extracted with ethyl acetate. The ethyl acetate layer was washed with saturated brine, dried over anhydrous magnesium sulfate, and concentrated under reduced pressure. The obtained residue was recrystallized from hexane-tetrahydrofuran to give the title compound (0.76 g, 89%) as a white solid.
1H NMR (300 MHz, DMSO-d₆)δ ppm 0.99-1.15 (m, 1 H), 1.20 (t, J=7.2 Hz, 3 H), 1.18-1.46 (m, 5 H), 1.24-1.46 (m, 4 H), 1.68-1.79 (m, 2 H), 1.83-1.96 (m, 1 H), 2.56-2.70 (m, 1 H), 3.08-3.26 (m, 3 H), 3.66-3.79 (m, 1 H), 3.85 (d, J=5.8 Hz, 2 H), 4.11 (q, J=7.2 Hz, 2 H), 6.52 (t, J=5.8 Hz, 1 H), 7.09-7.24 (m, 2 H), 7.65 (d, J=2.1 Hz, 1 H), 8.96 (s, 1 H).

### Example 239A N-[((3-chloro-4-[(1-cyclohexyl-2-oxopyrrolidin-3-yl)methyl]phenyl}amino)carbonyl]glycine

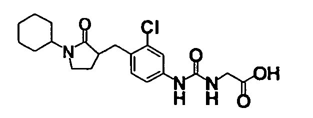

In the same manner as in Example 129A, the title compound was obtained from ethyl N-[({3-chloro-4-[(1-cyclohexyl-2-oxopyrrolidin-3-yl)methyl]phenyl}amino)carbonyl]glycinate obtained in Example 238A.
1H NMR (300 MHz, DMSO-d₆) δ ppm 0.97-1.17 (m, 1 H), 1.19-1.46 (m, 4 H), 1.48-1.65 (m, 4 H), 1.68-1.80 (m, 2 H), 1.81-1.96 (m, 1 H), 2.55-2.70 (m, 1 H), 3.07-3.27 (m, 4 H), 3.34 (brs, 1 H), 3.66-3.79 (m, 3 H), 6.48 (t, J=5.4 Hz, 1H), 7.07-7.25 (m, 2 H), 7.66 (s, 1 H), 9.00 (s, 1 H).

### Example 240A 2-[({3-chloro-4-[(1-cyclohexyl-2-oxopyrrolidin-3-yl)methyl]phenyl}amino)carbonylamino]-N-methylacetamide

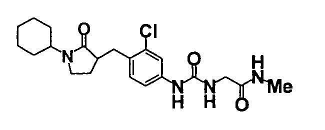

In the same manner as in Example 70A, the title compound was obtained from N-[({3-chloro-4-[(1-cyclohexyl-2-oxopyrrolidin-3-yl)methyl]phenyl}amino)carbonyl]glycine obtained in Example 239A and methylamine.
1H NMR (300 MHz, DMSO-d₆) δ ppm 1.00-1.16 (m, 1 H), 1.21-1.43 (m, 4 H), 1.46-1.64 (m, 4 H), 1.68-1.79 (m, 2 H), 1.81-1.95 (m, 1 H), 2.46-2.55 (m, 1 H), 2.56-2.69 (m, 1 H), 2.60 (d, J=4.7 Hz, 3 H), 3.07-3.25 (m, 3 H), 3.68 (d, J=5.4 Hz, 2 H), 3.70-3.78 (m, 1 H), 6.38 (t, J=5.4 Hz, 1 H), 7.11 (dd, J=8.4, 2.1 Hz, 1 H), 7.19 (d, J=8.4 Hz, 1 H), 7.65 (d, J=2.1 Hz, 1 H), 7.81-7.91 (m, 1 H), 8.90 (s, 1 H).

### Example 241A N-{3-chloro-4-[(1-cyclohexyl-2-oxopyrrolidin-3-yl)methyl]phenyl}-N'-methoxyurea

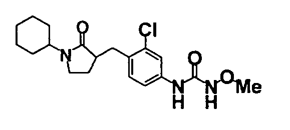

To a solution of 3-(4-amino-2-chlorobenzyl)-1-cyclohexylpyrrolidin-2-one obtained in Example 215A (0.20 g) in N,N-dimethylformamide (3 mL) was added N,N'-carbonyldiimidazole (0.21 g), and the mixture was stirred overnight at room temperature. Then, O-methylhydroxyammonium chloride (0.27 g) and diisopropylethylamine (0.57 mL) were added, and the mixture was stirred at room temperature for 2 days. The reaction mixture was concentrated under reduced pressure, ethyl acetate and water were added, and the mixture was extracted with ethyl acetate. The ethyl acetate layer was washed with saturated brine, dried over anhydrous magnesium sulfate, and concentrated under reduced pressure. The obtained residue was subjected to silica gel column chromatography with ethyl acetate-hexane (50:50-100:0), and the obtained crystals were recrystallized from hexane-ethyl acetate to give the title compound (0.12 g, 49%) as a white solid.
1H NMR (300 MHz, CDCl₃) δ ppm 1.00-1.19 (m, 1 H), 1.24-1.48 (m, 4 H), 1.60-1.85 (m, 6 H), 1.92-2.06 (m, 1 H), 2.71-2.84 (m, 2 H), 3. 12-3.25 (m, 2 H), 3.26-3. 39 (m, 1 H), 3. 79 (s, 3 H), 3. 88-4. 01 (m, 1 H), 7.19-7.30 (m, 2 H), 7.35 (brs, 1 H), 7.54 (brs, 1 H), 7.60 (d, J=2.1 Hz, 1 H).

### Example 242A N-{3-chloro-4-[(1-cyclohexyl-2-oxopyrrolidin-3-yl)methyl]phenyl}pyrrolidine-1-carboxamide

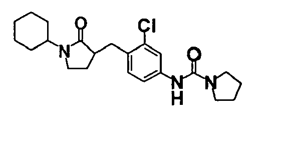

In the same manner as in Example 241A, the title compound was obtained from 3-(4-amino-2-chlorobenzyl)-1-cyclohexylpyrrolidin-2-one obtained in Example 215A and pyrrolidine.
1H NMR (300 MHz, CDCl₃) δ ppm 1.02-1.15 (m, 1 H), 1.23-1.45 (m, 4 H), 1.60-1.83 (m, 6 H), 1.91-2.03 (m, 5 H), 2.68-2.82 (m, 2 H), 3.11-3.23 (m, 2 H), 3.25-3.36 (m, 1 H), 3.41-3.49 (m, 4 H), 3.88-4.00 (m, 1 H), 6.21 (brs, 1 H), 7.12-7.22 (m, 2 H), 7.55 (d, J=1.9 Hz, 1 H).

### Example 243A N-{3-chloro-4-[(1-cyclohexyl-2-oxopyrrolidin-3-yl)methyl]phenyl}-N'-(2-hydroxyethyl)urea

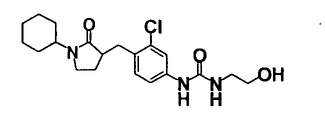

In the same manner as in Example 241A, the title compound was obtained from 3-(4-amino-2-chlorobenzyl)-1-cyclohexylpyrrolidin-2-one obtained in Example 215A and 2-aminoethanol.
1H NMR (300 MHz, CDCl₃) δ ppm 1.00-1.16 (m, 1 H), 1.25-1.44 (m, 5 H), 1.59-1.84 (m, 6 H), 1.93-2.04 (m, 1 H), 2.62-2.83 (m, 2 H), 3.11-3.29 (m, 3 H), 3.29-3.39 (m, 2 H), 3.63-3.71 (m, 2 H), 3.80-3.95 (m, 1 H), 6.14 (t, J=5.4 Hz, 1 H), 6.98-7.12 (m, 2 H), 7.35 (d, J=1.9 Hz, 1H), 8.05 (s, 1H).

### Example 244A N-{3-chloro-4-[(1-cyclohexyl-2-oxopyrrolidin-3-yl)methyl]phenyl}-N'-benzyloxyurea

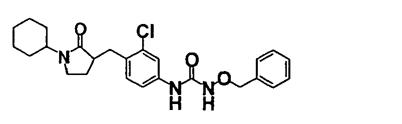

In the same manner as in Example 241A, the title compound was obtained from 3-(4-amino-2-chlorobenzyl)-1-cyclohexylpyrrolidin-2-one obtained in Example 215A and O-benzylhydroxylammonium chloride.
1H NMR (300 MHz, CDCl₃) δ ppm 0.99-1.16 (m, 1 H), 1.23-1.48 (m, 4 H), 1.60-1.85 (m, 6 H), 1.90-2.04 (m, 1 H), 2.68-2.82 (m, 2 H), 3.12-3.24 (m, 2 H), 3.25-3.36 (m, 1 H), 3.88-4.00 (m, 1 H), 4.88 (s, 2 H), 7.06-7.12 (m, 1 H), 7.15-7.20 (m, 1 H), 7.22 (s, 1 H), 7.32-7.37 (m, 1 H), 7.39-7.47 (m, 6 H).

### Example 245A 3-[2-chloro-4-(pyridin-2-yl)benzyl]-1-cyclohexylpyrrolidin-2-one

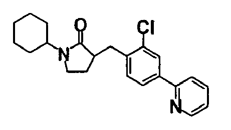

A solution of 3-(4-bromo-2-chlorobenzyl)-1-cyclohexylpyrrolidin-2-one obtained in Example 193A (0.20 g), 2-(tributylstannyl)pyridine (0.24g) and tetrakis(triphenylphosphine)palladium (31 mg) in toluene (5 mL) was heated under reflux overnight under nitrogen atmosphere. After cooling, water and ethyl acetate were added to the reaction mixture, and the mixture was extracted with ethyl acetate. The ethyl acetate layer was washed with saturated brine, dried over anhydrous magnesium sulfate, and concentrated under reduced pressure. The obtained residue was silica gel column chromatography with ethyl acetate-hexane (25:75-75:25) to give the title compound (0.11 g, 77%) as an oil.
1H NMR (300 MHz, CDCl₃) δ ppm 0.96-1.17 (m, 1 H), 1.20-1.49 (m, 4 H), 1.57-1.85 (m, 6 H), 1.87-2.11 (m, 1 H), 2.78-2.91 (m, 2 H), 3.13-3.28 (m, 2 H), 3.36-3.50 (m, 1 H), 3.88-4.03 (m, 1 H), 7.19-7.30 (m, 1 H), 7.38 (d, J=7.9 Hz, 1 H), 7.66-7.84 (m, 3 H), 8.03 (d, J=1.9 Hz, 1 H), 8.63-8.71 (m, 1 H).

### Example 246A 3-[2-chloro-4-(pyridin-3-yl)benzyl]-1-cyclohexylpyrrolidin-2-one

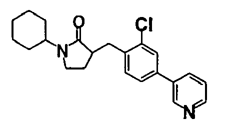

In the same manner as in Example 245A, the title compound was obtained from 3-(4-bromo-2-chlorobenzyl)-1-cyclohexylpyrrolidin-2-one obtained in Example 193A and 3-(tributylstannyl)pyridine.
1H NMR (300 MHz, CDCl₃) δ ppm 1.01-1.17 (m, 1 H), 1.25-1.48 (m, 4 H), 1.61-1.86 (m, 6 H), 1.99-2.12 (m, 1 H), 2.79-2.91 (m, 2 H), 3.16-3.30 (m, 2 H), 3.36-3.47 (m, 1 H), 3.90-4.03 (m, 1 H), 7.34-7.42 (m, 3 H), 7.58 (brs, 1 H), 7.81-7.88 (m, 1 H), 8.57-8.64 (m, 1 H), 8.79-8.84 (m, 1 H).

### Example 247A 3-[2-chloro-4-(2-furyl)benzyl]-1-cyclohexylpyrrolidin-2-one

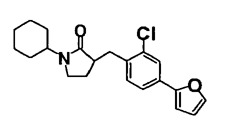

In the same manner as in Example 245A, the title compound was obtained from 3-(4-bromo-2-chlorobenzyl)-1-cyclohexylpyrrolidin-2-one obtained in Example 193A and 2-(tributylstannyl)furan.
1H NMR (300 MHz, CDCl₃) δ ppm 1.00-1.16 (m, 1 H), 1.25-1.48 (m, 4 H), 1.61-1.84 (m, 6 H), 1.93-2.08 (m, 1 H), 2.75-2.88 (m, 2 H), 3.12-3.26 (m, 2 H), 3.32-3.43 (m, 1 H), 3.85-4.02 (m, 1 H), 6.47 (dd, J=3. 4, 1.9 Hz, 1 H), 6.62-6.65 (m, 1 H), 7.28 (d, J=7.9 Hz, 1 H), 7.45-7.47 (m, 1 H), 7.45-7.50 (m, 1 H), 7.66 (d, J=1.7 Hz, 1 H).

### Example 248A 3-[2-chloro-4-(pyrimidin-2-yl)benzyl]-1-cyclohexylpyrrolidin-2-one

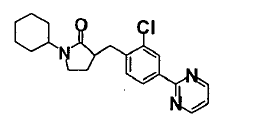

In the same manner as in Example 245A, the title compound was obtained from 3-(4-bromo-2-chlorobenzyl)-1-cyclohexylpyrrolidin-2-one obtained in Example 193A and 2-(tributylstannyl)pyrimidine.
1H NMR (300 MHz, CDCl₃) δ ppm 0.99-1.19 (m, 1 H), 1.22-1.51 (m, 4 H), 1.59-1.86 (m, 6 H), 1.93-2.09 (m, 1 H), 2.80-2.93 (m, 2 H), 3.12-3.30 (m, 2 H), 3.40-3.52 (m, 1 H), 3.88-4.05 (m, 1 H), 7.21 (t, J=4.9 Hz, 1 H), 7.40 (d, J=8.1 Hz, 1 H), 8.25 (dd, J=8.1, 1.8 Hz, 1H), 8.46 (d, J=1.8 Hz, 1H), 8.80 (d, J=4.9 Hz, 2 H).

### Example 249A 3-chloro-4-[(1-cyclohexyl-2-oxopyrrolidin-3-yl)methyl]benzonitrile

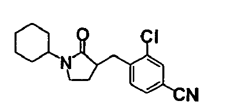

3-(4-Bromo-2-chlorobenzyl)-1-cyclohexylpyrrolidin-2-one obtained in Example 193A (0.30 g), copper (II) cyanide (52 mg) and tetrakis(triphenylphosphine)palladium (0.99 g) were dissolved in N,N-dimethylformamide (5 mL), and the mixture was heated under reflux overnight under nitrogen atmosphere. After cooling, the reaction mixture was filtrated, ethyl acetate and water were added to the filtrate, and the mixture was extracted with ethyl acetate. The ethyl acetate layer was washed with saturated brine, dried over anhydrous magnesium sulfate, and concentrated under reduced pressure. The obtained residue was silica gel column chromatography with ethyl acetate-hexane (50:50-100:0) to give the title compound (80 mg, 31%) as a white solid.
1H NMR (300 MHz, CDCl₃) δ ppm 0.98-1.18 (m, 1 H), 1.24-1.48 (m, 4 H), 1.57-1.94 (m, 6 H), 2.00-2.13 (m, 1 H), 2.74-2.93 (m, 2 H), 3.15-3.30 (m, 2 H), 3.32-3.45 (m, 1 H), 3.86-4.00 (m, 1 H), 7.44 (d, J=8.0 Hz, 1 H), 7.49 (dd, J=8.0, 1.5 Hz, 1 H), 7.65 (d, J=1.5 Hz, 1 H).

### Example 250A 3-[2-chloro-4-(2,2,2-trifluoroethoxy)benzyl]-1-cyclohexylpyrrolidin-2-one

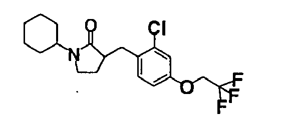

To a solution of 3-(2-chloro-4-hydroxybenzyl)-1-cyclohexylpyrrolidin-2-one obtained in Example 183A (0.20 g) in N,N-dimethylformamide (5 mL) were added cesium carbonate (0.42 g) and 2-bromo-1,1,1-trifluoroethane (0.12 mL), and the mixture was stirred at room temperature for 2 hr. Then, 2-bromo-1,1,1-trifluoroethane (0.60 mL) was further added, and the mixture was stirred overnight at 60°C. After cooling, the reaction mixture was concentrated under reduced pressure, ethyl acetate and water were added, and the mixture was extracted with ethyl acetate. The ethyl acetate layer was washed with saturated brine, dried over anhydrous magnesium sulfate, and concentrated under reduced pressure. The obtained residue was silica gel column chromatography with ethyl acetate-hexane (10:90-40:60) to give the title compound (64 mg, 25%) as a white solid.
1H NMR (300 MHz, CDCl₃) δ ppm 1.03-1.16 (m, 1 H), 1.23-1.47 (m, 4 H), 1.62-1.74 (m, 4 H), 1.74-1.85 (m, 2 H), 1.93-2.07 (m, 1 H), 2.70-2.83 (m, 2 H), 3.13-3.24 (m, 2 H), 3.24-3.36 (m, 1 H), 3.88-4.01 (m, 1 H), 4.32 (q, J=8.1 Hz, 2 H), 6.79 (dd, J=8.7, 2.7 Hz, 1H), 6.97 (d, J=2. 6 Hz, 1H), 7.23 (d, J=8.7 Hz, 1H).

### Example 251A 3-(1-hydroxy-4-oxocyclohexyl)-1-(4-methoxybenzyl)pyrrolidin-2-one

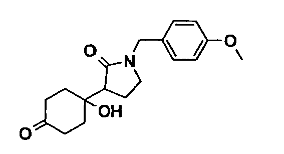

The compound which as obtained in the same manner as in Example 118A from 1-(4-methoxybenzyl)pyrrolidin-2-one and cyclohexane-1,4-dione monoethyleneketal, was subjected to hydrolysis in the same manner as in Example 28A to give the title compound.
1H NMR (300 MHz, CDCl₃) δ ppm 1.58-1.98 (m, 4 H), 1.95-2.16 (m, 2 H), 2.16-2.34 (m, 2 H), 2.67-3.05 (m, 3 H), 3.09-3.29 (m, 2 H), 3.80 (s, 3 H), 4.26-4.56 (m, 2 H), 5.17 (d, J=2.1 Hz, 1 H), 6.79-6.94 (m, 2 H), 7.07-7.22 (m, 2 H).

### Example 252A 3-[2-chloro-4-(3-hydroxyprop-1-yn-1-yl)benzyl]-1-cyclohexylpyrrolidin-2-one

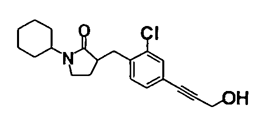

A solution of 3-(4-bromo-2-chlorobenzyl)-1-cyclohexylpyrrolidin-2-one obtained in Example 193A (0.20 g), tetrakis(triphenylphosphine)palladium (58 mg) and propargyl alcohol (63 µL) in piperidine (5 mL) was stirred at 80°C for 2 hr under argon atmosphere. After cooling, saturated aqueous ammonium chloride, water and ethyl acetate were added to the reaction mixture, and the mixture was extracted with ethyl acetate. The ethyl acetate layer was washed with saturated brine, dried over anhydrous magnesium sulfate, and concentrated under reduced pressure. The obtained residue was silica gel column chromatography with ethyl acetate-hexane (25:75-75:25) to give the title compound (0.14 g, 77%) as a brown oil.
1H NMR (300 MHz, CDCl₃) δ ppm 1.00-1.15 (m, 1 H), 1.31-1.47 (m, 4 H), 1.58-1.73 (m, 4 H), 1.74-1.89 (m, 3 H), 1.93-2.04 (m, 1 H), 2.73-2.86 (m, 2 H), 3.15-3.24 (m, 2 H), 3.31-3.42 (m, 1 H), 3.85-4.02 (m, 1 H), 4.49 (d, J=6.0 Hz, 2 H), 7.23 (brs, 2 H), 7.43 (brs, 1H).

### Example 253A ethyl (2E)-3-{3-chloro-4-[(1-cyclohexyl-2-oxopyrrolidin-3-yl)methyl]phenyl}acrylate

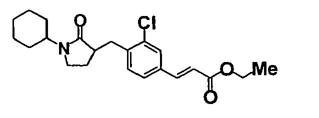

A solution of 3-(4-bromo-2-chlorobenzyl)-1-cyclohexylpyrrolidin-2-one obtained in Example 193A (0.20 g), ethyl acrylate (0.071 mL), palladium acetate (6.1mg), tri(o-tolyl)phosphine (16 mg) and N,N-diisopropylethylamine (0.70 mL) in N,N-dimethylformamide (3 mL) was stirred overnight at 115°C under argon atmosphere. After cooling, 1N hydrochloric acid and ethyl acetate were added, and the mixture was extracted with ethyl acetate. The ethyl acetate layer was washed with saturated brine, dried over anhydrous magnesium sulfate, and concentrated under reduced pressure. The obtained residue was silica gel column chromatography with ethyl acetate-hexane (20:80-70:30) to give the title compound (0.15 g, 71%) as yellow crystals.
1H NMR (300 MHz, CDCl₃) δ ppm 1.02-1.15 (m, 1 H), 1.24-1.43 (m, 4 H), 1.34 (t, J=7.2 Hz, 3 H), 1.62-1.84 (m, 6 H), 1.96-2.09 (m, 1 H), 2.74-2.87 (m, 2 H), 3.13-3.27 (m, 2 H), 3.31-3.43 (m, 1 H), 3.87-4.03 (m, 1 H), 4.26 (q, J=7.2 Hz, 2 H), 6.40 (d, J=16.0 Hz, 1 H), 7.28-7.38 (m, 2 H), 7.51 (s, 1 H), 7.58 (d, J=16.0 Hz, 1 H).

### Example 254A 3-(4-bromo-2-chlorobenzyl)-1-(4-oxocyclohexyl)pyrrolidin-2-one

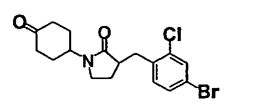

In the same manner as in Example 28A, the title compound was obtained from 3-(4-bromo-2-chlorobenzyl)-1-(1,4-dioxaspiro[4.5]dec-8-yl)pyrrolidin-2-one obtained in Example 203A.
1H NMR (300 MHz, CDCl₃) δ ppm 1.65-1.78 (m, 1 H), 1.79-1.94 (m, 2 H), 1.95-2.13 (m, 3 H), 2.38-2.62 (m, 4 H), 2.73-2.88 (m, 2 H), 3.14-3.26 (m, 2 H), 3.27-3.39 (m, 1 H), 4.41-4.55 (m, 1 H), 7.16 (d, J=8.3 Hz, 1 H), 7.33 (dd, J=8.3, 2.1 Hz, 1 H), 7.53 (d, J=2.1 Hz, 1 H).

### Example 255A (3S,4S)-1-(1-adamantyl)-3-(2,6-dichlorobenzyl)-4-hydroxypyrrolidin-2-one

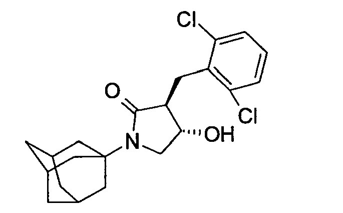

To a mixture of (4S)-1-(1-adamantyl)-4-hydroxypyrrolidin-2-one obtained in Reference Example 16A (0.235 g) and tetrahydrofuran (5 mL) was added lithium diisopropylamide (1.8 M tetrahydrofuran solution, 1.2 mL) at -78°C under nitrogen atmosphere, and the mixture was stirred for 1 hr.

N,N,N',N',N",N"-Hexamethylphosphoric triamide (1 mL) was added, and the mixture was stirred for 30 min. A solution of α,2,6-trichlorotoluene (0.46 g) in tetrahydrofuran (2 mL) was added to the obtained solution, and the mixture was further stirred at -78°C for 1 hr, and then at -40°C for 16 hr. Water was added to the reaction mixture, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 9:1-1:1) to give the title compound (0.133 g, 34%) as a colorless solid.
1H NMR (300 MHz, CDCl₃) δ ppm 1.53-1.69 (m, 12 H), 2.12-2.17 (m, 3 H), 2.36-2.44 (dd, J=16.8, 6.0 Hz, 1 H), 2.59-2.67 (dd, J=16.8, 7.8 Hz, 1 H), 2.97-3.03 (dd, J=10.2, 5.4 Hz, 1 H), 3.32-3.29 (m, 1 H), 4.41 (quint, J=6.6 Hz, 1 H), 4.73-4.89 (q, J=13.8 Hz, 2 H), 7.18-7.23 (m, 1 H), 7.28-7.35 (m, 2 H).

### Example 256A (3S,4S)-1-(1-adamantyl)-3-(2-chloro-4-fluorobenzyl)-4-hydroxypyrrolidin-2-one

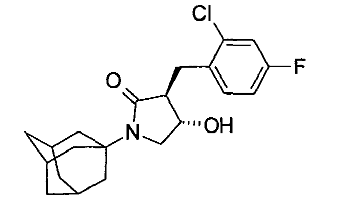

In the same manner as in Example 255A, the title compound was obtained from (4S)-1-(1-adamantyl)-4-hydroxypyrrolidin-2-one obtained in Reference Example 16A and 2-chloro-1-(chloromethyl)-4-fluorobenzene.
1H NMR (300 MHz, CDCl₃) δ ppm 1.53-1.61 (m, 6 H), 1.69-1.70 (m, 6 H), 2.14 (m, 3 H), 2.36-2.48 (m, 1 H), 2.61-2.73 (m, 1 H), 3.14-3.21 (dd, J=10.2, 4.4 Hz, 1 H), 3.44-3.52 (dd, J=10.0, 6.6 Hz, 1 H), 4.43-4.49 (m, 1 H), 4.50-4.65 (q, J=15.4 Hz, 2 H), 6.92-7.01 (m, 1 H), 7.09-7.14 (dd, J=8.4, 2.6 Hz, 1 H), 7.29-7.35 (dd, J=8.4, 5.8 Hz, 1 H).

### Example 257A (3S,4S)-1-(1-adamantyl)-3-(2-chloro-4-methoxybenzyl)-4-hydroxypyrrolidin-2-one

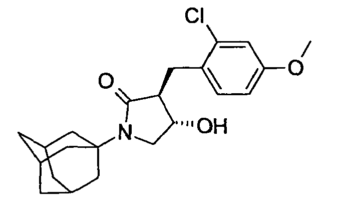

In the same manner as in Example 255A, the title compound was obtained from (4S)-1-(1-adamantyl)-4-hydroxypyrrolidin-2-one obtained in Reference Example 16A and 1-(bromomethyl)-2-chloro-4-methoxybenzene.
1H NMR (300 MHz, CDCl₃) δ ppm 1.55-1.62 (m, 6 H), 1.68-1.69 (m, 6 H), 2.13 (m, 3 H), 2.37-2.45 (dd, J=16.8, 5.1 Hz, 1 H), 2.60-2.69 (dd, J=16.8, 7.8 Hz, 1 H), 3.12-3.17 (dd, J=10.2, 4.8 Hz, 1 H), 3.41-3.47 (m, 1 H), 3.79 (s, 3 H), 4.42-4.46 (m, 1 H), 4.53 (s, 2 H), 6. 76-6. 80 (dd, J=8.4, 2.4 Hz, 1 H), 6.91 (d, J=2.4 Hz, 1 H), 7.22-7.25 (d, J=8.4 Hz, 1 H).

### Example 258A 1-cyclohexyl-3-(2,6-dichlorobenzyl)-4-(hydroxymethyl)pyrrolidin-2-one

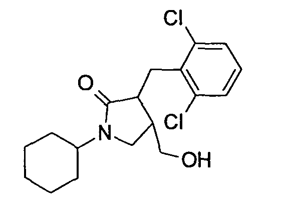

In the same manner as in Example 255A, the title compound was obtained from 1-cyclohexyl-4-(hydroxymethyl)pyrrolidin-2-one obtained in Reference Example 17A and α,2,6-trichlorotoluene.
1H NMR (300 MHz, CDCl₃) δ ppm 1.11 (m, 1 H), 1.26-1.44 (m, 5 H), 1.66-1.78 (m, 5 H), 2.35-2.41 (m, 1 H), 2.75-2.83 (m, 1 H), 3.10-3.20 (m, 2 H), 3.22-3.26 (m, 1 H), 3.29-3.36 (m, 1 H), 3.46-3.52 (m, 2 H), 3.96 (m, 1 H), 7.08-7.14 (m, 1 H), 7.26-7.31 (m, 2 H).

### Example 259A methyl 1-cyclohexyl-4-(2,6-dichlorobenzyl)-5-oxopyrrolidine-3-carboxylate

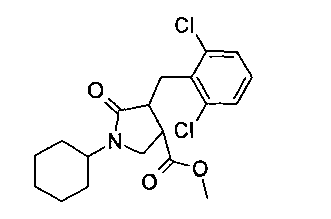

To a mixture of 1-cyclohexyl-3-(2,6-dichlorobenzyl)-4-(hydroxymethyl)pyrrolidin-2-one obtained in Example 258A (1.07 g), triethylamine (1.25 mL), dimethyl sulfoxide (0.70 mL) and dichloromethane (15 mL) was added sulfur trioxide pyridine complex (1.43 g) at 0°C, and the mixture was stirred at room temperature for 4 hr. Water was added to the reaction mixture, and the mixture was extracted with ethyl acetate. The ethyl acetate layer was washed with saturated brine, and dried over anhydrous magnesium sulfate. The solvent was evaporated under reduced pressure. N-Iodosuccinimide (1.69 g), potassium carbonate (1.04 g) and methanol (30 mL) were added to the obtained residue, and the mixture was stirred at room temperature for 16 hr under shading. Water was added to the reaction mixture, and the mixture was extracted with ethyl acetate. The ethyl acetate layer was washed with saturated brine, and dried over anhydrous magnesium sulfate. The solvent was evaporated under reduced pressure. The obtained residue was subjected to silica gel column chromatography (hexane-ethyl acetate 19:1-4:1) to give the title compound (0.22 g, 19%) as a pale-yellow solid.
1H NMR (300 MHz, CDCl₃) δ ppm 1.11 (m, 1 H), 1.31-1.49 (m, 4 H), 1.66-1.78 (m, 5 H), 3.04-3.22 (m, 2 H), 3.41 (s, 3 H), 3.43-3.65 (m, 4 H), 3.97 (m, 1 H), 7.06-7.31 (m, 3 H).

### Example 260A 1-cyclohexyl-4-(2,6-dichlorobenzyl)-5-oxopyrrolidine-3-carboxylic acid

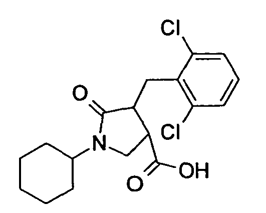

In the same manner as in Example 155A, the title compound was obtained from methyl 1-cyclohexyl-4-(2,6-dichlorobenzyl)-5-oxopyrrolidine-3-carboxylate obtained in Example 259A.
1H NMR (300 MHz, CDCl₃) δ ppm 1.10 (m, 1 H), 1.26-1.38 (m, 4 H), 1.65-1.80 (m, 5 H), 3.02-3.15 (m, 2 H), 3.33-3.41 (m, 2 H), 3.43-3.61 (m, 2 H), 3.95 (m, 1 H), 7.04-7.09 (t, J=8.4 Hz, 1H), 7.24-7.27 (m, 2 H).

### Example 261A [1-cyclohexyl-4-(2,6-dichlorobenzyl)-5-oxopyrrolidin-3-yl]methyl ethylcarbamate

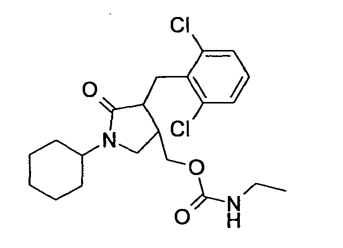

A mixture of 1-cyclohexyl-3-(2,6-dichlorobenzyl)-4-(hydroxymethyl)pyrrolidin-2-one obtained in Example 258A (0.200 g), ethyl isocyanate (0.071 g) and pyridine (3 mL) was stirred at 60°C for 16 hr. The reaction mixture was concentrated under reduced pressure, and the obtained residue was subjected to silica gel column chromatography (hexane-ethyl acetate 19:1-4:1) to give the title compound (0.180 g, 75%) as a colorless liquid.
1H NMR (300 MHz, CDCl₃) δ ppm 1.04-1.15 (m, 1 H), 1.09 (t, J=7.2 Hz, 3 H), 1.28-1.47 (m, 4 H), 1.66-1.78 (m, 5 H), 2.46-2.50 (m, 1 H), 2.91-3.00 (m, 1 H), 3.06-3.18 (m, 4 H), 3.42-3.56 (m, 2 H), 3.67-3.80 (m, 2 H), 3.95 (m, 1 H), 4.68 (m, 1H), 7.09-7.18 (m, 1 H), 7.22-7.31 (m, 2 H).

### Example 262A tert-butyl [1-cyclohexyl-4-(2,6-dichlorobenzyl)-5-oxopyrrolidin-3-yl]carbamate

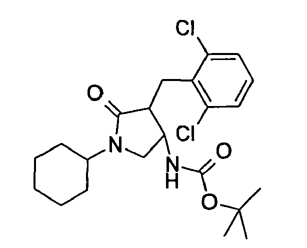

A mixture of 1-cyclohexyl-4-(2,6-dichlorobenzyl)-5-oxopyrrolidine-3-carboxylic acid obtained in Example 260A (0.290 g), diphenylphosphoryl azide (0.220 g), triethylamine (0.112 mL) and tert-butanol (20 mL) was stirred at 90°C for 16 hr. The solvent was evaporated under reduced pressure. The obtained residue was subjected to silica gel column chromatography (hexane-ethyl acetate 19:1-3:1) to give the title compound (0.330 g, 96%) as a colorless solid.
1H NMR (300 MHz, CDCl₃) δ ppm 1.09-1.13 (m, 1 H), 1.23-1.40 (m, 4 H), 1.35 (s, 9 H), 1.64-1.79 (m, 5 H), 2.81-2.89 (m, 1 H), 2.99-3.05 (m, 1 H), 3.11-3.19 (m, 1 H), 3.46-3.53 (dd, J=14.1, 5.4 Hz, 1 H), 3.66-3.72 (m, 1 H), 3.97-4.01 (m, 1 H), 4.06-4.11 (m, 1H), 4.27 (m, 1H), 7.08-7.13 (m, 1 H), 7.28-7.33 (m, 2 H).

### Example 263A 1-cyclohexyl-3-(2,6-dichlorobenzyl)-4-(1-hydroxy-1-methylethyl)pyrrolidin-2-one

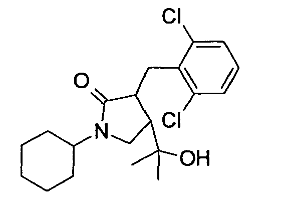

A solution of methyl 1-cyclohexyl-4-(2,6-dichlorobenzyl)-5-oxopyrrolidine-3-carboxylate obtained in Example 259A (0.24 g) in tetrahydrofuran (15 mL) was cooled to 10°C, and methyllithium (1.0 M ether solution, 1.9 mL) was added dropwise thereto. The reaction mixture was stirred at room temperature for 2 hr, water was added, and the mixture was extracted with ethyl acetate.

The organic layer was washed with saturated brine, and dried over magnesium sulfate, and the solvent was evaporated under reduced pressure. The obtained residue was subjected to silica gel column chromatography (hexane-ethyl acetate 9:1-1:1) to give the title compound (0.216 g, 90%) as a colorless solid.
1H NMR (300 MHz, CDCl₃) δ ppm 0.87 (s, 3 H), 0.98 (s, 3 H), 1.03-1.16 (m, 1 H), 1.35-1.51 (m, 4 H), 1.65-1.86 (m, 6 H), 1.92-2.02 (m, 1 H), 2.76-2.82 (m, 2 H), 3.02-3.11 (m, 1 H), 3.35-3.53 (m, 3 H), 3.90-3.97 (m, 1 H), 7.08-7.13 (m, 1 H), 7.28-7.30 (m, 2 H) .

### Example 264A 4-amino-1-cyclohexyl-3-(2,6-dichlorobenzyl)pyrrolidin-2-one hydrochloride

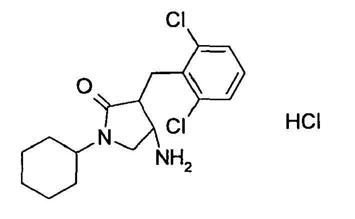

In the same manner as in Example 20A, the title compound was obtained from tert-butyl [1-cyclohexyl-4-(2,6-dichlorobenzyl)-5-oxopyrrolidin-3-yl]carbamate obtained in Example 262A.
1H NMR (300 MHz, DMSO-d₆) δ ppm 1.03-1.15 (m, 1 H), 1.22-1.41 (m, 4 H), 1.58-1.78 (m, 5 H), 2.81-2.86 (m, 1 H), 3.01-3.18 (m, 2 H), 3.43-3.47 (m, 2 H), 3.62-3.81 (m, 3 H), 7.27-7.33 (t, J=8.4 Hz, 1 H), 7.44-7.47 (m, 2 H), 8.55 (br, 3 H).

### Example 265A 3-(2-chloro-6-methoxybenzyl)-1-cyclohexyl-4-(hydroxymethyl)pyrrolidin-2-one

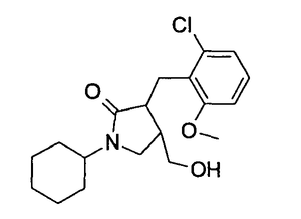

In the same manner as in Example 255A, the title compound was obtained from 1-cyclohexyl-4-(hydroxymethyl)pyrrolidin-2-one obtained in Reference Example 17A and 2-chloro-6-methoxybenzyl bromide.
1H NMR (300 MHz, CDCl₃) δ ppm 1.07-1.11 (m, 1 H), 1.34-1.44 (m, 5 H), 1.66-1.76 (m, 5 H), 2.26-2.32 (m, 1 H), 2.64-2.71 (m, 1 H), 2.91-2.99 (m, 1 H), 3.07-3.12 (m, 1 H), 3.17-3.24 (m, 2 H), 3.30-3.36 (dd, J=13.5, 4.8 Hz, 1 H), 3.42-3.48 (m, 1 H), 3.82 (s, 3H), 3.96 (m,1H), 6.75-6.78 (d, J=8.1 Hz, 1H), 6.96-6.99 (d, J=8.1 Hz, 1 H), 7.09-7.15 (t, J=8.1 Hz, 1 H).

### Example 266A 3-(2-chloro-6-hydroxybenzyl)-1-cyclohexyl-4-(hydroxymethyl)pyrrolidin-2-one

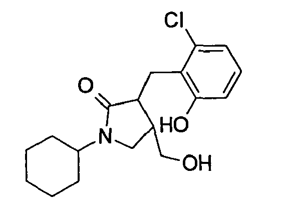

In the same manner as in Example 140A, the title compound was obtained from 3-(2-chloro-6-methoxybenzyl)-1-cyclohexyl-4-(hydroxymethyl)pyrrolidin-2-one obtained in Example 265A.
LC/MS (ESI+); m/z 338 (M+H)⁺

### Example 267A 1-cyclohexyl-3-(2,6-dichlorobenzyl)-4-(phenoxymethyl)pyrrolidin-2-one

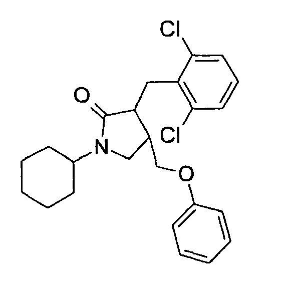

To a mixture of 1-cyclohexyl-3-(2,6-dichlorobenzyl)-4-(hydroxymethyl)pyrrolidin-2-one obtained in Example 258A (0.748 g), phenol (0.237 g), triphenylphosphine (0.661 g) and tetrahydrofuran (5 mL) was added diethyl azodicarboxylate (40% toluene solution, 1.08 g), and the mixture was stirred at room temperature for 16 hr. The solvent was evaporated under reduced pressure, and the residue was subjected to silica gel column chromatography (hexane-ethyl acetate 9:1-1:1) to give the title compound (0.617 g, 68%) as a colorless liquid.
1H NMR (300 MHz, CDCl₃) δ ppm 1.07-1.11 (m, 1 H), 1.33-1.44 (m, 4 H), 1.65-1.83 (m, 5 H), 2.60-2.66 (m, 1 H), 3.06-3.30 (m, 3 H), 3.46-3.65 (m, 4 H), 3.99 (m, 1 H), 6.65-6.68 (m, 2H), 7.04-7.10 (m, 1 H), 7.17-7.27 (m, 4 H).

### Example 268A 3-(4-bromo-2-fluorobenzyl)-1-cyclohexylpyrrolidin-2-one

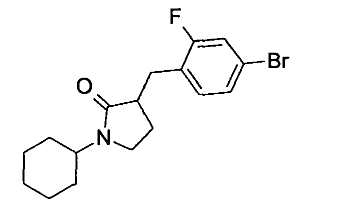

In the same manner as in Example 1A, the title compound was obtained from 1-cyclohexylpyrrolidin-2-one and 4-bromo-2-fluorobenzyl bromide.
1H NMR (300 MHz, CDCl₃) δ ppm 0.97-1.18 (m, 1 H), 1.21-1.49 (m, 4 H), 1.53-1.92 (m, 6 H), 1.93-2.12 (m, 1 H), 2.61-2.78 (m, 2 H), 3.10-3.26 (m, 3 H), 3.82-4.02 (m, 1 H), 7.08-7.25 (m, 3 H).

### Example 269A 1-cyclohexyl-3-[(3-fluorobiphenyl-4-yl)methyl]pyrrolidin-2-one

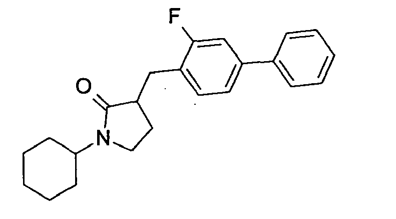

In the same manner as in Example 218A, the title compound was obtained from 3-(4-bromo-2-fluorobenzyl)-1-cyclohexylpyrrolidin-2-one obtained in Example 268A and phenylboronic acid.
1H NMR (300 MHz, CDCl₃) δ ppm 1.05-1.09 (m, 1 H), 1.23-1.41 (m; 4 H), 1.64-1.76 (m, 6 H), 2.02-2.09 (m, 1H), 2.71-2.80 (m, 2H), 3.16-3.22 (m, 2H), 3.26-3.29 (m, 1 H), 3.91-3.95 (m, 1 H), 7.23-7.40 (m, 4 H), 7.43-7.45 (m, 2 H), 7.54-7.56 (m, 2 H).

### Example 270A 3-(2,6-dichlorobenzyl)-1-[3-(hydroxymethyl)-1-adamantyl]pyrrolidin-2-one

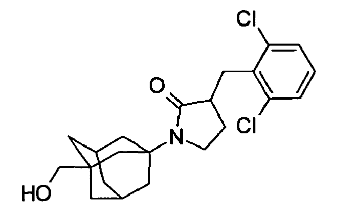

In the same manner as in Example 255A, the title compound was obtained from 1-[3-(hydroxymethyl)-1-adamantyl]pyrrolidin-2-one obtained in Reference Example 19A and α,2,6-trichlorotoluene.
1H NMR (300 MHz, CDCl₃) δ ppm 1.43-2.21 (m, 17 H), 2.81-2.88 (m, 1 H), 2.96-3.04 (m, 1 H), 3.23-3.31 (m, 3 H), 3.42-3.50 (m, 2 H), 7.05-7.11 (t, J=7.8 Hz, 1 H), 7.27-7.30 (d, J=8.1 Hz, 2 H).

### Example 271A methyl 3-[3-(2, 6-dichlorobenzyl)-2-oxopyrrolidin-1-yl]adamantane-1-carboxylate

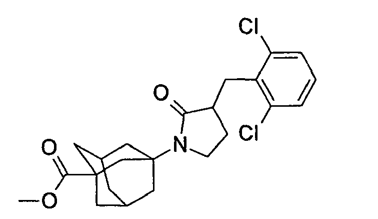

In the same manner as in Example 259A, the title compound was obtained from 3-(2,6-dichlorobenzyl)-1-[3-(hydroxymethyl)-1-adamantyl]pyrrolidin-2-one obtained in Example 270A.
1H NMR (300 MHz, CDCl₃) δ ppm 1.53-1.73 (m, 4 H), 1.77-1.91 (m, 3 H), 1.99 (m, 1 H), 2.05-2.23 (m, 6 H), 2.81-2.88 (m, 1 H), 2.96-3.05 (m, 1 H), 3.23-3.29 (m, 2 H), 3.32 (s, 3H), 3.66 (s, 1H), 3.70 (s, 1 H), 7.06-7.11 (m, 1 H), 7.27-7.30 (m, 2 H).

### Example 272A 3-[3-(2,6-dichlorobenzyl)-2-oxopyrrolidin-1-yl]adamantane-1-carboxylic acid

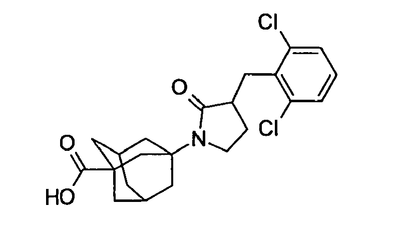

In the same manner as in Example 155A, the title compound was obtained from methyl 3-[3-(2, 6-dichlorobenzyl)-2-oxopyrrolidin-1-yl]adamantane-1-carboxylate obtained in Example 271A.
1H NMR (300 MHz, CDCl₃) δ ppm 1.62-1.79 (m, 6 H), 1.80-1.97 (m, 2 H), 2.11-2.23 (m, 6 H), 2.23-2.31 (m, 2 H), 2.87-2.99 (m, 1 H), 3.02 (m, 1 H), 3.29-3.35 (m, 1H), 3.37-3.45 (m, 1H), 3.48-3.55 (m, 1H), 7.07-7.12 (m, 1 H), 7.27-7.30 (m, 2 H), 9.36 (s, 1 H).

### Example 273A 3-(2,6-dichlorobenzyl)-1-[3-(1-hydroxy-1-methylethyl)-1-adamantyl]pyrrolidin-2-one

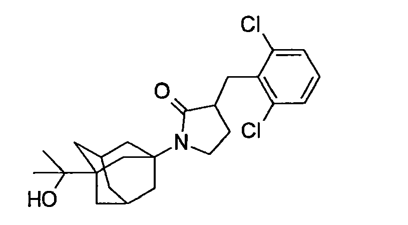

In the same manner as in Example 263A, the title compound was obtained from methyl 3-[3-(2,6-dichlorobenzyl)-2-oxopyrrolidin-1-yl]adamantane-1-carboxylate obtained in Example 271A.
1H NMR (300 MHz, CDCl₃) δ ppm 1.18 (s, 6 H), 1.43-1.74 (m, 6 H), 1.77-1.92 (m, 2 H), 2.00-2.31 (m, 8 H), 2.90-3.07 (m, 2 H), 3.29-3.55 (m, 3 H), 4.56 (br, 1 H), 7.07-7.12 (m, 1 H), 7.27-7.30 (m, 2 H).

### Example 274A 3-(2,6-dichlorobenzyl)-1-(piperidin-1-yl)pyrrolidin-2-one

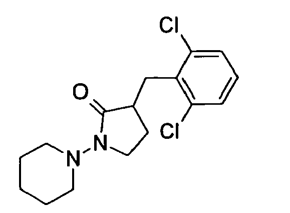

In the same manner as in Example 1A, the title compound was obtained from 1-(piperidin-1-yl)pyrrolidin-2-one obtained in Reference Example 22A and α,2,6-trichlorotoluene.
1H NMR (300 MHz, CDCl₃) δ ppm 1.39-1.45 (m, 2 H), 1.66-1.74 (m, 4 H), 1.81-1.94 (m, 2 H), 2.82-2.88 (m, 1 H), 2.91-3.04 (m, 4 H), 3.27-3.35 (m, 1 H), 3.41-3.49 (m, 2 H), 7.07-7.12 (m, 1 H), 7.27-7.30 (m, 2 H).

### Example 275A 3-(2-chloro-4-methoxybenzyl)-1-(piperidin-1-yl)pyrrolidin-2-one

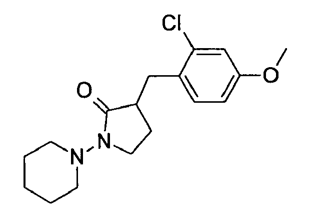

In the same manner as in Example 1A, the title compound was obtained from 1-(piperidin-1-yl)pyrrolidin-2-one obtained in Reference Example 22A and 2-chloro-4-methoxybenzyl bromide.
1H NMR (300 MHz, CDCl₃) δ ppm 1.40-1.43 (m, 2 H), 1.65-1.75 (m, 5 H), 1.94-2.01 (m, 1 H), 2.66-2.79 (m, 2 H), 2.85-2.97 (m, 4 H), 3.23-3.32 (m, 3 H), 3.77 (s, 3 H), 6.72-6.76 (dd, J=8.7, 2.4 Hz, 1 H), 6.89 (d, J=2.4 Hz, 1 H), 7.17-7.20 (d, J=8.7 Hz, 1 H). Example 276A 3-[2-chloro-4-(trifluoromethyl)benzyl]-1-(piperidin-1-yl)pyrrolidin-2-one

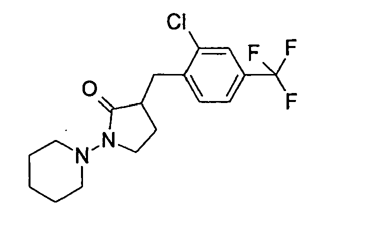

In the same manner as in Example 1A, the title compound was obtained from 1-(piperidin-1-yl)pyrrolidin-2-one obtained in Reference Example 22A and 2-chloro-4-(trifluoromethyl)benzyl chloride.
1H NMR (300 MHz, CDCl₃) δ ppm 1.35-1.46 (m, 2 H), 1.58-1.77 (m, 5 H), 1.96-2.12 (m, 1 H), 2.67-2.84 (m, 2 H), 2.89-2.94 (m, 4 H), 3.30-3.42 (m, 3 H), 7.44 (m, 2 H), 7.62 (s, 1 H).

### Example 277A 3-(2,6-dichlorobenzyl)-1-(morpholin-4-yl)pyrrolidin-2-one

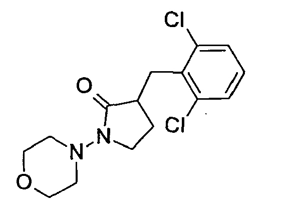

In the same manner as in Example 1A, the title compound was obtained from 1-(morpholin-4-yl)pyrrolidin-2-one obtained in Reference Example 23A and α,2,6-trichlorotoluene.
1H NMR (300 MHz, CDCl₃) δ ppm 1.82-1.97 (m, 3 H), 2.85-2.94 (m, 1 H), 3.01-3.09 (m, 4 H), 3.29-3.37 (m, 1 H), 3.43-3.50 (m, 2 H), 3.80-3.83 (m, 4 H), 7.08-7.13 (m, 1 H), 7.28-7.31 (m, 2 H).

### Example 278A 3-(2,4-dichlorobenzyl)-1-(piperidin-1-yl)pyrrolidin-2-one

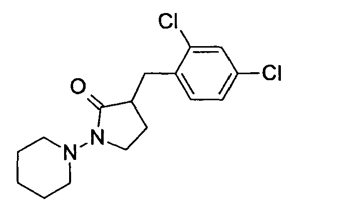

In the same manner as in Example 1A, the title compound was obtained from 1-(piperidin-1-yl)pyrrolidin-2-one obtained in Reference Example 22A and α,2,4-trichlorotoluene.
1H NMR (300 MHz, CDCl₃) δ ppm 1.36-1.44 (m, 2 H), 1.60-1.73 (m, 5 H), 1.96-2.06 (m, 1 H), 2.65-2.76 (m, 2 H), 2.79-2.96 (m, 4 H), 3.25-3.34 (m, 3 H), 7.14-7.18 (m, 4 H), 7.23-7.26 (m, 1 H), 7.35 (m, 1 H).

### Example 279A 3-(2,6-dichlorobenzyl)-1-(2,3-dihydro-1H-indol-1-yl)pyrrolidin-2-one

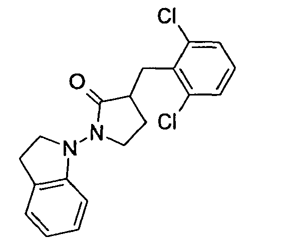

In the same manner as in Example 1A, the title compound was obtained from 1-(2,3-dihydro-1H-indol-1-yl)pyrrolidin-2-one obtained in Reference Example 20A and α,2,6-trichlorotoluene. 1H NMR (300 MHz, CDCl₃) δ ppm 1.97-2.06 (m, 2 H), 3.04-3.21 (m, 4 H), 3.30-3.46 (m, 2 H), 3.52-3.63 (m, 3 H), 6.52-6.55 (m, 1 H), 6.81-6.86 (m, 1 H), 7.09-7.14 (m, 3 H), 7.26-7.32 (m, 2 H).

### Example 280A 3-(2,4-dichlorobenzyl)-1-(2,3-dihydro-1H-indol-1-yl)pyrrolidin-2-one (less polar product)

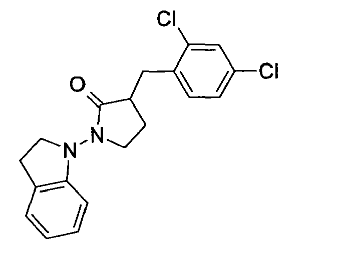

In the same manner as in Example 1A, the title compound was obtained from 1-(2,3-dihydro-1H-indol-1-yl)pyrrolidin-2-one obtained in Reference Example 20A and α,2,4-trichlorotoluene.
1H NMR (300 MHz, CDCl₃) δ ppm 2.18-2.28 (m, 1 H), 2.56-2.62 (dd, J=13.5, 5.7 Hz, 1 H), 3.00-3.07 (m, 2 H), 3.15-3.29 (m, 2 H), 3.50-3.54 (d, J=12.3 Hz, 2 H), 3.45-3.66 (m, 2 H), 5.92 (br, 1H), 6.02 (br, 1H), 6.58-6.32 (t, J=7.2 Hz, 1 H), 6.85-6.91 (m, 2 H), 6.98-7.01 (dd, J=8.4, 2.4 Hz, 1 H), 7.06-7.09 (dd, J=7.2, 0.9 Hz, 1 H), 7.34 (d, J=2.1 Hz, 1 H).

### Example 281A 3-(2,4-dichlorobenzyl)-1-(2,3-dihydro-1H-indol-1-yl)pyrrolidin-2-one (more polar product)

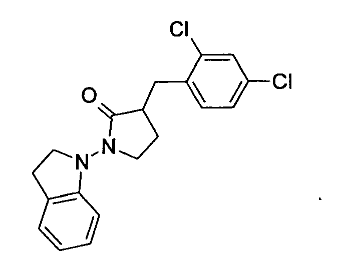

In the same manner as in Example 1A, the title compound was obtained from 1-(2,3-dihydro-1H-indol-1-yl)pyrrolidin-2-one obtained in Reference Example 20A and α,2,4-trichlorotoluene.
1H NMR (300 MHz, CDCl₃) δ ppm 1.74-1.87 (m, 1 H), 2.07-2.18 (m, 1 H), 2.81-2.94 (m, 2 H), 3.02-3.08 (t, J=7.8 Hz, 2 H), 3.25-3.43 (m, 3 H), 3.56 (br, 2 H), 6.38-6.41 (d, J=7.8 Hz, 1H), 6.81-6.86 (t, J=7.2 Hz, 1 H), 7.05-7.13 (m, 2 H), 7.17-7.20 (m, 1 H), 7.26-7.28 (m, 1H), 7.38 (d, J=2.1 Hz, 1 H).

### Example 282A 3-[2-chloro-4-(trifluoromethyl)benzyl]-1-(2,3-dihydro-1H-indol-1-yl)pyrrolidin-2-one

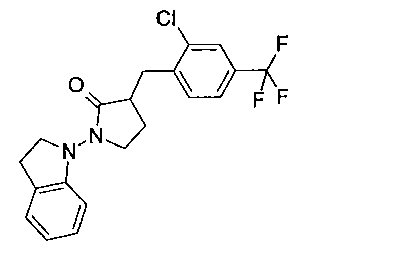

In the same manner as in Example 1A, the title compound was obtained from 1-(2,3-dihydro-1H-indol-1-yl)pyrrolidin-2-one obtained in Reference Example 20A and 2-chloro-4-(trifluoromethyl)benzyl chloride.
1H NMR (300 MHz, CDCl₃) δ ppm 1.79-1.90 (m, 1 H), 2.12-2.21 (m, 1 H), 2.88-3.09 (m, 4 H), 3.27-3.60 (m, 5 H), 6.38-6.41 (d, J=7.5 Hz, 1 H), 6.82-6.87 (m, 1H), 7.05-7.14 (m, 2 H), 7.48 (s, 2 H), 7.65 (s, 1 H).

### Example 283A 3-(2,4-dichlorobenzyl)-1-(6-methoxy-2,3-dihydro-1H-inden-1-yl)pyrrolidin-2-one

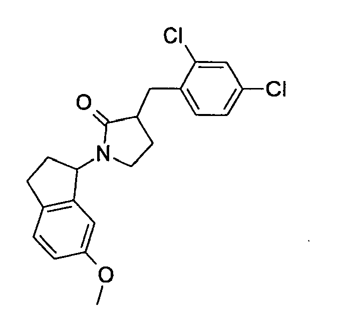

In the same manner as in Example 1A, the title compound was obtained from 1-(6-methoxy-2,3-dihydro-1H-inden-1-yl)pyrrolidin-2-one obtained in Reference Example 21A and α,2,4-trichlorotoluene.
1H NMR (300 MHz, CDCl₃) δ ppm 1.60-1.75 (m, 2 H), 1.87-2.01 (m, 1 H), 2.33-2.47 (m, 1 H), 2.75-2.90 (m, 5 H), 2.93-3.44 (m, 1 H), 3.76 (s, 3 H), 5.72-5.81 (m, 1 H), 6.53-6.64 (dd, J=20.0, 2.2 Hz, 1 H), 6.76-6.82 (dd, J=8.8, 2.6 Hz, 1 H), 7.11-7.32 (m, 3 H), 7. 38 (d, J=1.2 Hz, 1 H).

### Example 284A 3-(2,4-dichlorobenzyl)-1-(6-hydroxy-2,3-dihydro-1H-inden-1-yl)pyrrolidin-2-one

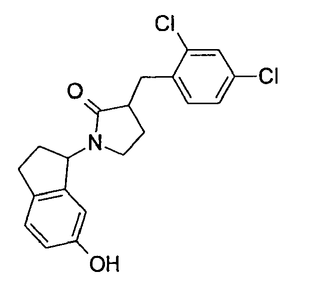

In the same manner as in Example 140A, the title compound was obtained from 3-(2,4-dichlorobenzyl)-1-(6-methoxy-2,3-dihydro-1H-inden-1-yl)pyrrolidin-2-one obtained in Example 283A.
1H NMR (300 MHz, CDCl₃) δ ppm 1.60-1.76 (m, 1 H), 1.83-2.00 (m, 2 H), 2.33-2.42 (m, 1 H), 2.65 -2.90 (m, 5 H), 2.94-3.18 (m, 1 H), 3.35-3.45 (m, 1 H), 5.69-5.81 (m, 1 H), 6.66 (m, 1 H), 6.78-6.92 (m, 2 H), 7.04-7.20 (m, 3 H), 7.32 (m, 1 H).

### Example 285A 3-(2,6-dichlorobenzyl)-1-(1-ethynylcyclohexyl)pyrrolidin-2-one

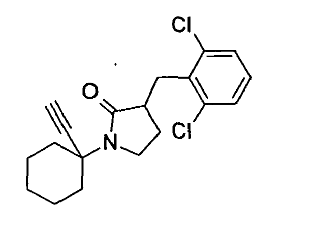

To a mixture of 1-ethynylcyclohexanamine (5.0 g), triethylamine (5.85 mL) and tetrahydrofuran (200 mL) was added a solution of 4-chlorobutyryl chloride (5.78 g) in tetrahydrofuran (50 mL) at 0°C, and the mixture was stirred at room temperature for 3 hr. The precipitate was removed by filtration, and the filtrate was concentrated under reduced pressure. The obtained residue was partitioned between ethyl acetate and water. The ethyl acetate layer was washed successively with 10% aqueous sodium bicarbonate and saturated brine, and dried over anhydrous magnesium sulfate, and the solvent was evaporated under reduced pressure to give a colorless solid (8.6 g). To a mixture of this solid (6.0 g) and N,N-dimethylformamide (50 mL) was added sodium hydride (60% oil, 1.2 g), and the mixture was stirred at room temperature for 1 hr. Water was added to the reaction mixture, and the mixture was extracted with ethyl acetate. The extract was washed successively with water and saturated brine, and dried over anhydrous magnesium sulfate. The solvent was evaporated under reduced pressure to give a colorless oil (4.0 g). To a mixture of this oil (0.5 g) and tetrahydrofuran (10 mL) was added lithium diisopropylamide (1.8 M tetrahydrofuran solution, 1.45 mL) at -78°C under nitrogen atmosphere, and the mixture was stirred for 10 min. A solution of α,2,6-trichlorotoluene (0.508 g) in tetrahydrofuran (5 mL) was added to the obtained solution, and the mixture was further stirred at -78°C for 10 min, and allowed to warm to room temperature. 10% Aqueous ammonium chloride was added to the reaction solution, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 9:1-4:1) to give the title compound (0.078 g, 7%) as a colorless solid.
1H NMR (300 MHz, CDCl₃) δ ppm 1.24-1.28 (m, 1 H), 1.59-1.68 (m, 5 H), 1.82-1.95 (m, 2 H), 2.01 -2.09 (m, 2 H), 2.22-2.38 (m, 2 H), 2.48 (s, 1H), 2.84-2.95 (m, 1 H), 3.03-3.09 (m, 1 H), 3.36-3.49 (m, 2 H), 3.55-3.67 (m, 1 H), 7.06-7.13 (m, 1 H), 7.27-7.35 (m, 2 H).

### Example 286A 1-((1S,2R)-2-{[tert-butyl(dimethyl)silyl]oxy}-2,3-dihydro-1H-inden-1-yl)-3-(2,6-dichlorobenzyl)pyrrolidin-2-one

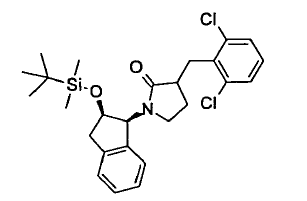

In the same manner as in Example 1A, the title compound was obtained from 1-((1S,2R)-2-{[tert-butyl(dimethyl)silyl]oxy}-2,3-dihydro-1H-inden-1-yl)pyrrolidin-2-one obtained in Reference Example 28A and 2,6-dichlorobenzyl chloride.
1H NMR (300 MHz, CDCl₃) δ ppm 0.09 (s, 3 H), 0.13 (s, 3 H), 0.88 (s, 9 H), 1.66-1.95 (m, 2 H), 2.79-3.32 (m, 6 H), 3.59 (dd, J=13.3, 3.9 Hz, 1 H), 4.64-4.76 (m, 1H), 5.60 (d, J=7.2 Hz, 1 H), 7.03-7.14 (m, 1 H), 7.17-7.34 (m, 6 H).

### Example 287A 3-(2,6-dichlorobenzyl)-1-[(1S)-2,3-dihydro-1H-inden-1-yl]pyrrolidin-2-one (less polar product)

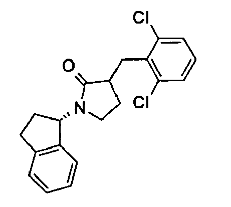

In the same manner as in Example 1A, the title compound was obtained from 1-[(1S)-2,3-dihydro-1H-inden-1-yl]pyrrolidin-2-one obtained in Reference Example 25A and 2,6-dichlorobenzyl chloride.
1H NMR (300 MHz, CDCl₃) δ ppm 1.81- 2.09 (m, 3 H), 2.37-2.53 (m, 1 H), 2.85-3.24 (m, 6 H), 3.45-3.60 (m, 1 H), 5.84 (t, J=7.6 Hz, 1 H), 7.04-7.35 (m, 7 H).

### Example 288A 3-(2,6-dichlorobenzyl)-1-[(1S)-2,3-dihydro-1H-inden-1-yl]pyrrolidin-2-one (more polar product)

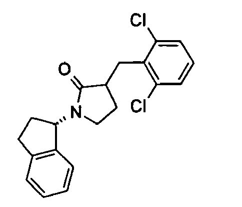

In the same manner as in Example 1A, the title compound was obtained from 1-[(1S)-2,3-dihydro-1H-inden-1-yl]pyrrolidin-2-one obtained in Reference Example 25A and 2,6-dichlorobenzyl chloride.
1H NMR (300 MHz, CDCl₃) δ ppm 1.76- 2.03 (m, 3 H), 2.32-2.48 (m, 1 H), 2.82-3.20 (m, 6 H), 3.54-3.66 (m, 1 H), 5.83 (t, J=7.8 Hz, 1 H), 7.01-7.40 (m, 7 H).

### Example 289A 3-(2,6-dichlorobenzoyl)-1-(2,3-dihydro-1H-inden-1-yl)pyrrolidin-2-one

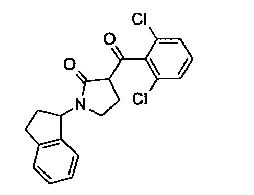

In the same manner as in Example 1A, the title compound was obtained from 1-(2,3-dihydro-1H-inden-1-yl)pyrrolidin-2-one obtained in Reference Example 5A and methyl 2,6-dichlorobenzoate.
LC-MS (ESI⁺) ; m/z 374 (M)⁺

### Example 290A 3-(2,6-dichlorobenzyl)-1-[(1R)-2,3-dihydro-1H-inden-1-yl]pyrrolidin-2-one (less polar product)

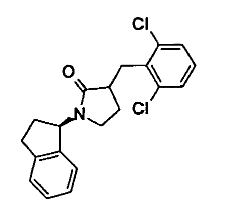

In the same manner as in Example 1A, the title compound was obtained from 1-[(1R)-2,3-dihydro-1H-inden-1-yl]pyrrolidin-2-one obtained in Reference Example 26A and 2,6-dichlorobenzyl chloride.
1H NMR (300 MHz, CDCl₃) δ ppm 1.79-2.08 (m, 3 H), 2.37-2.54 (m, 1 H), 2.83-3.26 (m, 6 H), 3.45-3.62 (m, 1 H), 5.84 (t, J=7.6 Hz, 1 H), 7.04-7.35 (m, 7 H).

### Example 291A 3-(2,6-dichlorobenzyl)-1-[(1R)-2,3-dihydro-1H-inden-1-yl]pyrrolidin-2-one (more polar product)

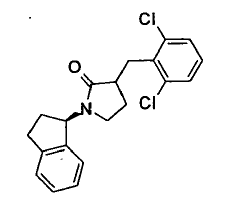

In the same manner as in Example 1A, the title compound was obtained from 1-[(1R)-2,3-dihydro-1H-inden-1-yl]pyrrolidin-2-one obtained in Reference Example 26A and 2,6-dichlorobenzyl chloride.
1H NMR (300 MHz, CDCl₃) δ ppm 1.75-2.04 (m, 3 H), 2.32-2.48 (m, 1 H), 2.83-3.20 (m, 6 H), 3.60 (dd, J=13.2, 4.1 Hz, 1 H), 5.83 (t, J=7.7 Hz, 1 H), 7.06-7.35 (m, 7 H).

### Example 292A 1-(2,3-dihydro-1H-inden-1-yl)-3-(2,6-dimethylbenzyl)pyrrolidin-2-one (less polar product)

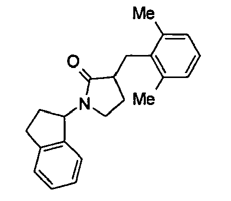

In the same manner as in Example 1A, the title compound was obtained from 1-(2,3-dihydro-1H-inden-1-yl)pyrrolidin-2-one obtained in Reference Example 5A and 2,6-dimethylbenzyl chloride.
1H NMR (300 MHz, CDCl₃) δ ppm 1.63-1.80 (m, 1 H), 1.89-2.07 (m, 2 H), 2.38 (s, 6 H), 2.39-2.54 (m, 1 H), 2.67-2.83 (m, 2 H), 2.84-3.20 (m, 4 H), 3.28-3.44 (m, 1 H), 5.85 (t, J=7.6 Hz, 1 H), 6.99-7.05 (m, 3 H), 7.08-7.28 (m, 4 H).

### Example 293A 1-(2,3-dihydro-1H-inden-1-yl)-3-(2,6-dimethylbenzyl)pyrrolidin-2-one (more polar product)

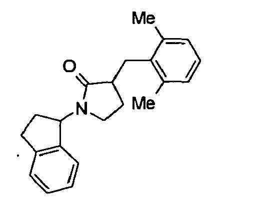

In the same manner as in Example 1A, the title compound was obtained from 1-(2,3-dihydro-1H-inden-1-yl)pyrrolidin-2-one obtained in Reference Example 5A and 2,6-dimethylbenzyl chloride.
1H NMR (300 MHz, CDCl₃) δ ppm 1.62-1.76 (m, 1 H), 1.87-2.07 (m, 2 H), 2.38 (s, 6 H), 2.31-2.48 (m, 1 H), 2.62-2.83 (m, 2 H), 2.85-3.13 (m, 4 H), 3.35-3.48 (m, 1 H), 5.83 (t, J=7.7 Hz, 1 H), 7.00-7.05 (m, 3 H), 7.13-7.29 (m, 4 H).

### Example 294A 3-(4-chloro-2-isopropoxybenzyl)-1-(2,3-dihydro-1H-inden-1-yl)pyrrolidin-2-one (less polar product)

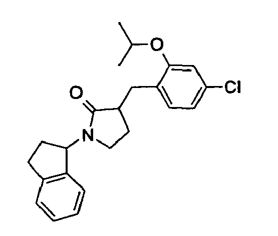

In the same manner as in Example 1A, the title compound was obtained from 1-(2,3-dihydro-1H-inden-1-yl)pyrrolidin-2-one obtained in Reference Example 5A and 4-chloro-2-isopropoxybenzyl chloride.
1H NMR (300 MHz, CDCl₃) δ ppm 1.35 (d, J=5.8 Hz, 6 H), 1.61-1.76 (m, 1 H), 1.81-1.98 (m, 2 H), 2.31-2.47 (m, 1 H), 2.64 (dd, J=13.4, 9.4 Hz, 1 H), 2.78-3.06 (m, 5 H), 3.25 (dd, J=13.4, 4.3 Hz, 1 H), 4.45-4.60 (m, 1 H), 5.81 (t, J=7.6 Hz, 1 H), 6.79-6.87 (m, 2 H), 7.07-7.28 (m, 5 H).

### Example 295A 3-(4-chloro-2-isopropoxybenzyl)-1-(2,3-dihydro-1H-inden-1-yl)pyrrolidin-2-one (more polar product)

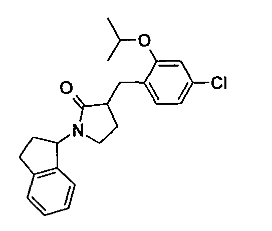

In the same manner as in Example 1A, the title compound was obtained from 1-(2,3-dihydro-1H-inden-1-yl)pyrrolidin-2-one obtained in Reference Example 5A and 4-chloro-2-isopropoxybenzyl chloride.
1H NMR (300 MHz, CDCl₃) δ ppm 1.33 (d, J=5.8 Hz, 6 H), 1.58-1.70 (m, 1 H), 1.85-2.01 (m, 2 H), 2.31-2.46 (m, 1 H), 2.59-2.71 (m, 1 H), 2.76-3.12 (m, 5 H), 3.30 (dd, J=13.5, 4.2 Hz, 1 H), 4.45-4.59 (m, 1 H), 5.80 (t, J=7.8 Hz, 1 H), 6.80-6.89 (m, 2 H), 6.96-7.04 (m, 1 H), 7.10-7.29 (m, 4 H).

### Example 296A 3-(4-chloro-2-methylbenzyl)-1-(2,3-dihydro-1H-inden-1-yl)pyrrolidin-2-one (less polar product)

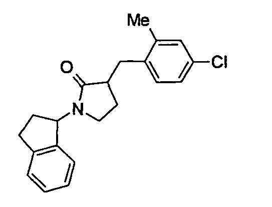

In the same manner as in Example 1A, the title compound was obtained from 1-(2,3-dihydro-1H-inden-1-yl)pyrrolidin-2-one obtained in Reference Example 5A and 4-chloro-2-methylbenzyl chloride.
1H NMR (300 MHz, CDCl₃) δ ppm 1.60-1.72 (m, 1 H), 1.85-2.07 (m, 2 H), 2.34 (s, 3 H), 2.35-2.50 (m, 1 H), 2.53-2.66 (m, 1 H), 2.70-3.11 (m, 5 H), 3.32 (dd, J=14.2, 4.1 Hz, 1 H), 5.83 (t, J=7.5 Hz, 1 H), 7.06-7.29 (m, 7 H).

### Example 297A 3-(4-chloro-2-methylbenzyl)-1-(2,3-dihydro-1H-inden-1-yl)pyrrolidin-2-one (more polar product)

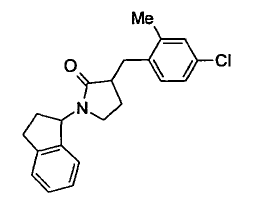

In the same manner as in Example 1A, the title compound was obtained from 1-(2,3-dihydro-1H-inden-1-yl)pyrrolidin-2-one obtained in Reference Example 5A and 4-chloro-2-methylbenzyl chloride.
1H NMR (300 MHz, CDCl₃) δ ppm 1.51-1.69 (m, 1 H), 1.85-2.11 (m, 2 H), 2.33 (s, 3 H), 2.35-2.46 (m, 1 H), 2.55-2.81 (m, 2 H), 2.83-3.17 (m, 4 H), 3.37 (dd, J=14.0, 3.7 Hz, 1 H), 5.81 (t, J=7.7 Hz, 1 H), 7.01-7.30 (m, 7 H).

### Example 298A 3-(2-chloro-4-methoxybenzyl)-1-(2,3-dihydro-1H-inden-1-yl)pyrrolidin-2-one (less polar product)

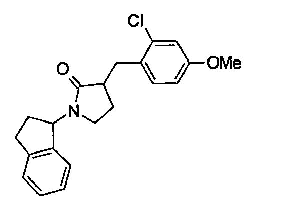

In the same manner as in Example 1A, the title compound was obtained from 1-(2,3-dihydro-1H-inden-1-yl)pyrrolidin-2-one obtained in Reference Example 5A and 2-chloro-4-methoxybenzyl chloride.
1H NMR (300 MHz, CDCl₃) δ ppm 1.64-1.81 (m, 1 H), 1.82-2.04 (m, 2 H), 2.31-2.49 (m, 1 H), 2.71-3.10 (m, 6 H), 3.35 (dd, J=13.2, 4.0 Hz, 1 H), 3.78 (s, 3H), 5.82 (t, J=7.6 Hz, 1 H), 6.76 (dd, J=8.5, 2.6 Hz, 1 H), 6.87-6.94 (m, 1 H), 7.07-7.29 (m, 5 H).

### Example 299A 3-(2-chloro-4-methoxybenzyl)-1-(2,3-dihydro-1H-inden-1-yl)pyrrolidin-2-one (more polar product)

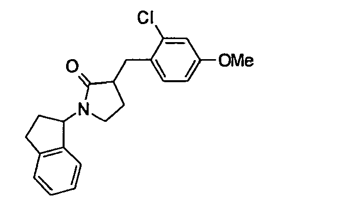

In the same manner as in Example 1A, the title compound was obtained from 1-(2,3-dihydro-1H-inden-1-yl)pyrrolidin-2-one obtained in Reference Example 5A and 2-chloro-4-methoxybenzyl chloride.
1H NMR (300 MHz, CDCl₃) δ ppm 1.61-1.75 (m, 1 H), 1.86-2.06 (m, 2 H), 2.30-2.47 (m, 1 H), 2.74-3.15 (m, 6 H), 3.32-3.48 (m, 1 H), 3.79 (s, 3H), 5.80 (t, J=7.7 Hz, 1 H), 6.77 (dd, J=8.5, 2.6 Hz, 1 H), 6.92 (d, J=2.6 Hz, 1 H), 7.00-7.08 (m, 1 H), 7.14-7.28 (m, 4 H).

### Example 300A 3-[2-chloro-4-(methoxymethoxy)benzyl]-1-(2,3-dihydro-1H-inden-1-yl)pyrrolidin-2-one (less polar product)

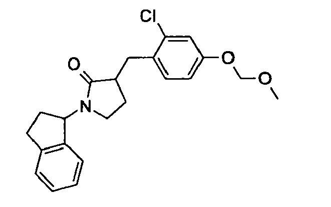

In the same manner as in Example 1A, the title compound was obtained from 1-(2,3-dihydro-1H-inden-1-yl)pyrrolidin-2-one obtained in Reference Example 5A and 2-chloro-4-(methoxymethoxy)benzyl bromide obtained in Reference Example 24A.
1H NMR (300 MHz, CDCl₃) δ ppm 1.65-1.80 (m, 1 H), 1.84-2.04 (m, 2 H), 2.33-2.48 (m, 1 H), 2.73-3.09 (m, 6 H), 3.36 (dd, J=13.4, 4.0 Hz, 1 H), 3.47 (s, 3 H), 5.14 (s, 2 H), 5.82 (t, J=7.6 Hz, 1 H), 6.89 (dd, J=8.5, 2.6 Hz, 1 H), 7.05-7.29 (m, 6 H).

### Example 301A 3-[2-chloro-4-(methoxymethoxy)benzyl]-1-(2,3-dihydro-1H-inden-1-yl)pyrrolidin-2-one (more polar product)

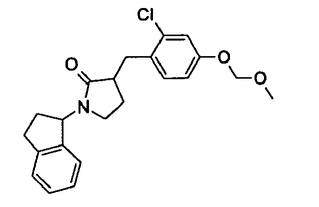

In the same manner as in Example 1A, the title compound was obtained from 1-(2,3-dihydro-1H-inden-1-yl)pyrrolidin-2-one obtained in Reference Example 5A and 2-chloro-4-(methoxymethoxy)benzyl bromide obtained in Reference Example 24A.
1H NMR (300 MHz, CDCl₃) δ ppm 1.60-1.74 (m, 1 H), 1.86-2.07 (m, 2 H), 2.31-2.46 (m, 1 H), 2.74-3.14 (m, 6 H), 3.35-3.45 (m, 1 H), 3.48 (s, 3H), 5.15 (s, 2 H), 5.81 (t, J=7.8 Hz, 1 H), 6.90 (dd, J=8.4, 2.6 Hz, 1 H), 7.02-7.10 (m, 2 H), 7.14-7.28 (m, 4 H).

### Example 302A 3-[2-chloro-4-(trifluoromethyl)benzyl]-1-(2,3-dihydro-1H-inden-1-yl)pyrrolidin-2-one (less polar product)

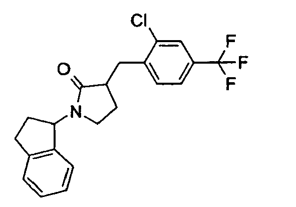

In the same manner as in Example 1A, the title compound was obtained from 1-(2,3-dihydro-1H-inden-1-yl)pyrrolidin-2-one obtained in Reference Example 5A and 2-chloro-4-(trifluoromethyl)benzyl chloride.
1H NMR (300 MHz, CDCl₃) δ ppm 1.62-1.80 (m, 1 H), 1.84-2.10 (m, 2 H), 2.33-2.51 (m, 1 H), 2.82-3.13 (m, 6 H), 3.38-3.55 (m, 1 H), 5.82 (t, J=7.5 Hz, 1 H), 7.07-7.29 (m, 4 H), 7.43-7.48 (m, 2 H), 7.64 (brs, 1 H).

### Example 303A 3-(2,4-dichlorobenzyl)-1-(2,3-dihydro-1H-inden-1-yl)pyrrolidin-2-one (less polar product)

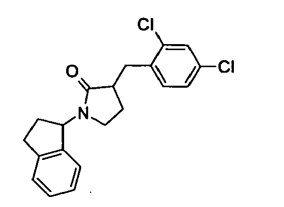

In the same manner as in Example 1A, the title compound was obtained from 1-(2,3-dihydro-1H-inden-1-yl)pyrrolidin-2-one obtained in Reference Example 5A and 2,4-dichlorobenzyl chloride.
1H NMR (300 MHz, CDCl₃) δ ppm 1.61-1.77 (m, 1 H), 1.82-2.08 (m, 2 H), 2.32-2.50 (m, 1 H), 2.76-3.11 (m, 6 H), 3.29-3.47 (m, 1 H), 5.82 (t, J=7.5 Hz, 1 H), 7.05-7.32 (m, 6 H), 7.38 (d, J=2.1 Hz, 1 H).

### Example 304A 3-(2-chloro-6-methoxybenzyl)-1-(2,3-dihydro-1H-inden-1-yl)pyrrolidin-2-one (less polar product)

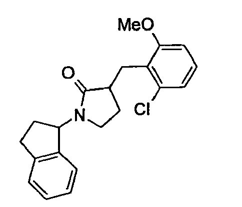

In the same manner as in Example 1A, the title compound was obtained from 1-(2,3-dihydro-1H-inden-1-yl)pyrrolidin-2-one obtained in Reference Example 5A and 2-chloro-6-methoxybenzyl chloride.
1H NMR (300 MHz, CDCl₃) δ ppm 1.73-2.08 (m, 3 H), 2.37-2.52 (m, 1 H), 2.83-3.21 (m, 6 H), 3.31-3.45 (m, 1 H), 3.84 (s, 3H), 5.84 (t, J=7.7 Hz, 1H), 6.72-6.81 (m, 1H), 6.98 (dd, J=8.1, 1.1 Hz, 1 H), 7.06-7.30 (m, 5 H).

### Example 305A 3-(2-chloro-6-methoxybenzyl)-1-(2,3-dihydro-1H-inden-1-yl)pyrrolidin-2-one (more polar product)

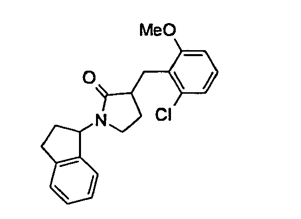

In the same manner as in Example 1A, the title compound was obtained from 1-(2,3-dihydro-1H-inden-1-yl)pyrrolidin-2-one obtained in Reference Example 5A and 2-chloro-6-methoxybenzyl chloride.
1H NMR (300 MHz, CDCl₃) δ ppm 1.65-2.12 (m, 3 H), 2.29-2.51 (m, 1 H), 2.81-3.14 (m, 6 H), 3.40-3.49 (m, 1 H), 3.82 (s, 3H), 5.83 (t, J=7.8 Hz, 1 H), 6. 77 (d, J=8.1 Hz, 1 H) , 6.94-7.02 (m, 1H), 7.06-7.28 (m, 5 H).

### Example 306A 3-(4-bromo-2-chlorobenzyl)-1-(2,3-dihydro-1H-inden-1-yl)pyrrolidin-2-one (less polar product)

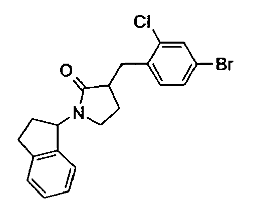

In the same manner as in Example 1A, the title compound was obtained from 1-(2,3-dihydro-1H-inden-1-yl)pyrrolidin-2-one obtained in Reference Example 5A and 4-bromo-2-chlorobenzyl bromide.
1H NMR (300 MHz, CDCl₃) δ ppm 1.63-1.78 (m, 1 H), 1.82-2.08 (m, 2 H), 2.31-2.50 (m, 1 H), 2.74-3.11 (m, 6 H), 3.28-3.46 (m, 1 H), 5.82 (t, J=7.6 Hz, 1 H), 7.04-7.38 (m, 6 H), 7.53 (d, J=1.9 Hz, 1 H).

### Example 307A 3-(4-bromo-2-chlorobenzyl)-1-(2,3-dihydro-1H-inden-1-yl)pyrrolidin-2-one (more polar product)

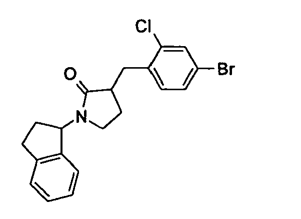

In the same manner as in Example 1A, the title compound was obtained from 1-(2,3-dihydro-1H-inden-1-yl)pyrrolidin-2-one obtained in Reference Example 5A and 4-bromo-2-chlorobenzyl bromide.
1H NMR (300 MHz, CDCl₃) δ ppm 1.58-1.70 (m, 1 H), 1.82-2.11 (m, 2 H), 2.29-2.48 (m, 1 H), 2.74-3.17 (m, 6 H), 3.32-3.50 (m, 1 H), 5.79 (t, J=7.7 Hz, 1 H), 6.98-7.06 (m, 1 H), 7.14-7.29 (m, 4 H), 7.31-7.39 (m, 1 H), 7.53 (d, J=2.1 Hz, 1 H).

### Example 308A 1-[(1S)-2,3-dihydro-1H-inden-1-yl]-3-(2,6-dimethylbenzyl)pyrrolidin-2-one (less polar product)

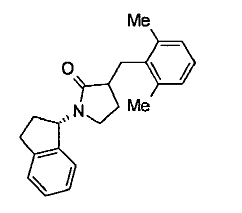

In the same manner as in Example 1A, the title compound was obtained from 1-[(1S)-2,3-dihydro-1H-inden-1-yl]pyrrolidin-2-one obtained in Reference Example 25A and 2,6-dimethylbenzyl chloride.
1H NMR (300 MHz, CDCl₃) δ ppm 1.63- 1.79 (m, 1 H), 1.89-2.06 (m, 2 H), 2.38 (s, 6 H), 2.40-2.53 (m, 1 H), 2.68-2.82 (m, 2 H), 2.84-3.18 (m, 4 H), 3.29-3.42 (m, 1 H), 5.85 (t, J=7.6 Hz, 1 H), 6.98-7.06 (m, 3 H), 7.08-7.29 (m, 4 H).

### Example 309A 1-[(1S)-2,3-dihydro-1H-inden-1-yl]-3-(2,6-dimethylbenzyl)pyrrolidin-2-one (more polar product)

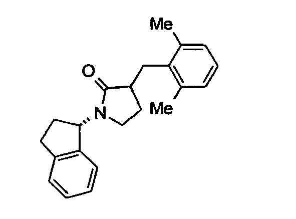

In the same manner as in Example 1A, the title compound was obtained from 1-[(1S)-2,3-dihydro-1H-inden-1-yl]pyrrolidin-2-one obtained in Reference Example 25A and 2,6-dimethylbenzyl chloride.
1H NMR (300 MHz, CDCl₃) δ ppm 1.60- 1.76 (m, 1 H), 1.85-2.07 (m, 2 H), 2.30-2.47 (m, 7 H), 2.63-3.13 (m, 6 H), 3.36-3.48 (m, 1 H), 5.83 (t, J=7.7 Hz, 1 H), 6.98-7.07 (m, 3 H), 7.13-7.28 (m, 4 H) .

### Example 310A 3-(2-chloro-4-methoxybenzyl)-1-[(1S)-2,3-dihydro-1H-inden-1-yl]pyrrolidin-2-one (less polar product)

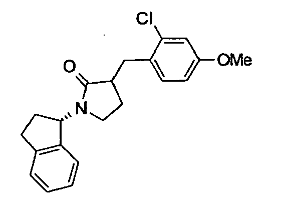

In the same manner as in Example 1A, the title compound was obtained from 1-[(1S)-2,3-dihydro-1H-inden-1-yl]pyrrolidin-2-one obtained in Reference Example 25A and 2-chloro-4-methoxybenzyl chloride.
1H NMR (300 MHz, CDCl₃) δ ppm 1.64-2.05 (m, 3 H), 2.32-2.49 (m, 1 H), 2.72-3.08 (m, 6 H), 3.35 (dd, J=13.3, 4.1 Hz, 1 H), 3.78 (s, 3H), 5.82 (t, J=7.6 Hz, 1 H), 6.76 (dd, J=8.6, 2.6 Hz, 1 H), 6.92 (d, J=2.6 Hz, 1 H), 7.06-7.29 (m, 5 H).

### Example 311A 3-(2-chloro-4-methoxybenzyl)-1-[(1S)-2,3-dihydro-1H-inden-1-yl]pyrrolidin-2-one (more polar product)

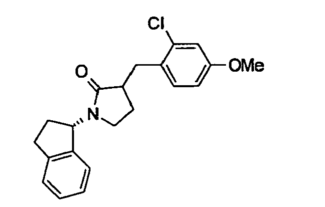

In the same manner as in Example 1A, the title compound was obtained from 1-[(1S)-2,3-dihydro-1H-inden-1-yl]pyrrolidin-2-one obtained in Reference Example 25A and 2-chloro-4-methoxybenzyl chloride.
1H NMR (300 MHz, CDCl₃) δ ppm 1.56-1.75 (m, 1 H), 1.85-2.07 (m, 2 H), 2.30-2.46 (m, 1 H), 2.74-3.15 (m, 6 H), 3.33-3.47 (m, 1 H), 3.79 (s, 3H), 5.80 (t, J=7.7 Hz, 1 H), 6.77 (dd, J=8.5, 2.6 Hz, 1 H), 6.92 (d, J=2.6 Hz, 1 H), 7.00-7.08 (m, 1 H), 7.14-7.29 (m, 4 H).

### Example 312A 3-(2-chloro-6-methoxybenzyl)-1-[(1S)-2,3-dihydro-1H-inden-1-yl]pyrrolidin-2-one (less polar product)

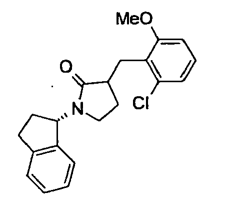

In the same manner as in Example 1A, the title compound was obtained from 1-[(1S)-2,3-dihydro-1H-inden-1-yl]pyrrolidin-2-one obtained in Reference Example 25A and 2-chloro-6-methoxybenzyl chloride.
1H NMR (300 MHz, CDCl₃) δ ppm 1.72-2.08 (m, 3 H), 2.36-2.52 (m, 1 H), 2.81-3.21 (m, 6 H), 3.30-3.45 (m, 1 H), 3.84 (s, 3H), 5.84 (t, J=7. 7 Hz, 1 H), 6.77 (d, J=8. 3 Hz, 1 H), 6.94-7.03 (m, 1 H), 7.06-7.29 (m, 5 H).

### Example 313A 3-(2-chloro-6-methoxybenzyl)-1-[(1S)-2,3-dihydro-1H-inden-1-yl]pyrrolidin-2-one (more polar product)

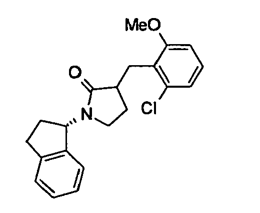

In the same manner as in Example 1A, the title compound was obtained from 1-[(1S)-2,3-dihydro-1H-inden-1-yl]pyrrolidin-2-one obtained in Reference Example 25A and 2-chloro-6-methoxybenzyl chloride.
1H NMR (300 MHz, CDCl₃) δ ppm 1.67-2.03 (m, 3 H), 2.30-2.47 (m, 1 H), 2.81-3.12 (m, 6 H), 3.35-3.53 (m, 1 H), 3.83 (s, 3H), 5.83 (t, J=7.9 Hz, 1 H), 6.77 (d, J=8.1 Hz, 1 H), 6.93-7.03 (m, 1 H), 7.06-7.31 (m, 5 H).

### Example 314A 3-(2-chloro-6-hydroxybenzyl)-1-[(1S)-2,3-dihydro-1H-inden-1-yl]pyrrolidin-2-one (less polar product)

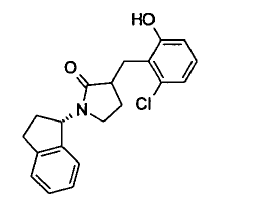

In the same manner as in Example 140A, the title compound was obtained from 3-(2-chloro-6-methoxybenzyl)-1-[(1S)-2,3-dihydro-1H-inden-1-yl]pyrrolidin-2-one obtained in Example 312A.
1H NMR (300 MHz, CDCl₃) δ ppm 1.76-2.05 (m, 3 H), 2.29-2.53 (m, 2 H), 2.75-3.24 (m, 7 H), 5.68-5.85 (m, 1 H), 6.80-7.36 (m, 7 H), 9.83 (s, 1 H).

### Example 315A 3-(2-chloro-6-hydroxybenzyl)-1-[(1S)-2,3-dihydro-1H-inden-1-yl]pyrrolidin-2-one (more polar product)

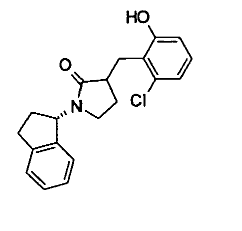

In the same manner as in Example 140A, the title compound was obtained from 3-(2-chloro-6-methoxybenzyl)-1-[(1S)-2,3-dihydro-1H-inden-1-yl]pyrrolidin-2-one obtained in Example 313A.
1H NMR (300 MHz, CDCl₃) δ ppm 1.70-2.01 (m, 3 H), 2.21-2.52 (m, 2 H), 2.70-3.34 (m, 7 H), 5.76 (t, J=7.7 Hz, 1 H), 6.75-7.36 (m, 7 H), 9.82 (brs, 1 H).

### Example 316A 3-(2-chloro-4-hydroxybenzyl)-1-[(1S)-2,3-dihydro-1H-inden-1-yl]pyrrolidin-2-one (less polar product)

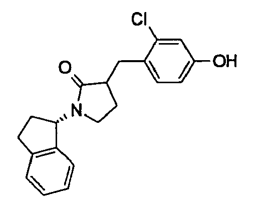

In the same manner as in Example 140A, the title compound was obtained from 3-(2-chloro-4-methoxybenzyl)-1-[(1S)-2,3-dihydro-1H-inden-1-yl]pyrrolidin-2-one obtained in Example 310A.
1H NMR (300 MHz, CDCl₃) δ ppm 1.66-2.09 (m, 3 H), 2.30-2.50 (m, 1 H), 2.66-2.50 (m, 1 H), 2.66-3.14 (m, 6 H), 3.29 (dd, J=13.8, 4.9 Hz, 1 H), 5.80 (t, J=7.5 Hz, 1 H), 6.72 (dd, J=8.3, 2.5 Hz, 1 H), 6.92 (d, J=2.5 Hz, 1 H), 7.03-7.30 (m, 5 H).

### Example 317A 3-(2-chloro-4-hydroxybenzyl)-1-[(1S)-2,3-dihydro-1H-inden-1-yl]pyrrolidin-2-one (more polar product)

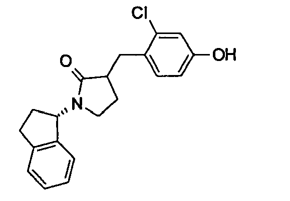

In the same manner as in Example 140A, the title compound was obtained from 3-(2-chloro-4-methoxybenzyl)-1-[(1S)-2,3-dihydro-1H-inden-1-yl]pyrrolidin-2-one obtained in Example 311A.
1H NMR (300 MHz, CDCl₃) δ ppm 1.58-1.80 (m, 1 H), 1.85-2.11 (m, 2 H), 2.30-2.49 (m, 1 H), 2.70-3.19 (m, 6 H), 3.33 (dd, J=13.6, 4.5 Hz, 1 H), 5.79 (t, J=7.6 Hz, 1 H), 6.73 (dd, J=8.4, 2.5 Hz, 1 H), 6.93 (d, J=2.5 Hz, 1 H), 6.99-7.30 (m, 5 H).

### Example 318A 3-[2-chloro-6-(prop-2-yn-1-yloxy)benzyl]-1-[(1S)-2,3-dihydro-1H-inden-1-yl]pyrrolidin-2-one

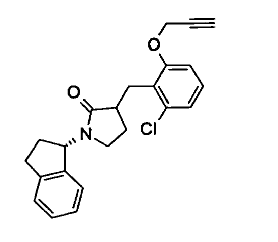

To a solution of 3-(2-chloro-6-hydroxybenzyl)-1-[(1S)-2,3-dihydro-1H-inden-1-yl]pyrrolidin-2-one obtained in Example 314A (342 mg) in N,N-dimethylformamide (7 mL) were added propargyl bromide (227 µL) and potassium carbonate (207 mg), and the mixture was stirred at 50°C for 2 hr. Water was added to the reaction mixture, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 95:5-85:15) to give the title compound (297 mg, 78%) as a colorless oil.
1H NMR (300 MHz, CDCl₃) δ ppm 1.71-2.10 (m, 3 H), 2.36-2.54 (m, 2 H), 2.80-3.24 (m, 6 H), 3.30-3.47 (m, 1 H), 4.75 (d, J=2.3 Hz, 2 H), 5.84 (t, J=7.7 Hz, 1 H), 6.91 (d, J=8.1 Hz, 1 H), 7.00-7.07 (m, 1 H), 7.08-7.30 (m, 5 H).

### Example 319A 3-[2-chloro-6-(cyclopropylmethoxy)benzyl]-1-[(1S)-2,3-dihydro-1H-inden-1-yl]pyrrolidin-2-one

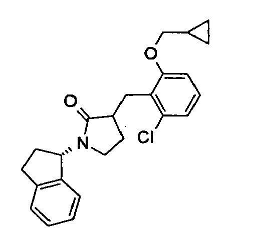

In the same manner as in Example 318A, the title compound was obtained from 3-(2-chloro-6-hydroxybenzyl)-1-[(1S)-2,3-dihydro-1H-inden-1-yl]pyrrolidin-2-one obtained in Example 314A and (bromomethyl)cyclopropane.
1H NMR (300 MHz, CDCl₃) δ ppm 0.24-0.44 (m, 2 H), 0.52-0.70 (m, 2 H), 1.16-1.39 (m, 1 H), 1.72-2.11 (m, 3 H), 2.35-2.55 (m, 1 H), 2.78-3.48 (m, 7 H), 3.72-3.90 (m, 2 H), 5.85 (t, J=7.6 Hz, 1 H), 6.72 (d, J=8.1 Hz, 1H), 6.92-7.31 (m, 6 H).

### Example 320A 2-[3-chloro-2-({1-[(1S)-2,3-dihydro-1H-inden-1-yl]-2-oxopyrrolidin-3-yl}methyl)phenoxy]-N,N-diethylacetamide

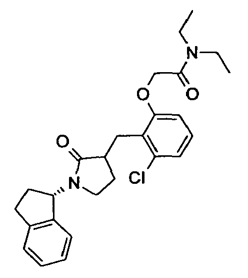

In the same manner as in Example 318A, the title compound was obtained from 3-(2-chloro-6-hydroxybenzyl)-1-[(1S)-2, 3-dihydro-1H-inden-1-yl]pyrrolidin-2-one obtained in Example 314A and 2-chloro-N, N-diethylacetamide.
1H NMR (300 MHz, CDCl₃) δ ppm 1.15 (t, J=7.1 Hz, 3 H), 1.23 (t, J=7.1 Hz, 3 H), 1.75-2.08 (m, 3 H), 2.35-2.51 (m, 1 H), 2.81-3.09 (m, 5 H), 3.10-3.21 (m, 1 H), 3.33-3.49 (m, 5 H), 4.73 (s, 2 H), 5.83 (t, J=7.7 Hz, 1 H), 6.70-6.78 (m, 1 H), 6.97-7.29 (m, 6 H).

### Example 321A 2-[3-chloro-4-({1-[(1S)-2,3-dihydro-1H-inden-1-yl]-2-oxopyrrolidin-3-yl}methyl)phenoxy]-N,N-diethylacetamide

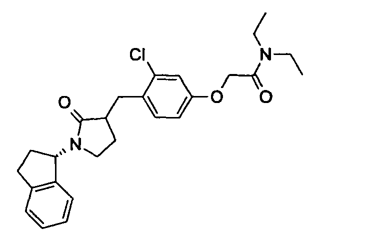

In the same manner as in Example 318A, the title compound was obtained from 3-(2-chloro-4-hydroxybenzyl)-1-[(1S)-2,3-dihydro-1H-inden-1-yl]pyrrolidin-2-one obtained in Example 316A and 2-chloro-N,N-diethylacetamide.
1H NMR (300 MHz, CDCl₃) δ ppm 1.15 (t, J=7.1 Hz, 3 H), 1.23 (t, J=7.1 Hz, 3 H), 1.64-1.79 (m, 1 H), 1.83-2.04 (m, 2 H), 2.31-2.48 (m, 1 H), 2.71-3.08 (m, 6 H), 3.29-3.47 (m, 5 H), 4.64 (s, 2 H), 5.82 (t, J=7.6 Hz, 1 H), 6.82 (dd, J=8.5, 2.6 Hz, 1 H), 6.96 (d, J=2.6 Hz, 1 H), 7.06-7.29 (m, 5 H).

### Example 322A 3-(2,6-dichlorobenzyl)-1-[(1S,2R)-2-hydroxy-2,3-dihydro-1H-inden-1-yl]pyrrolidin-2-one

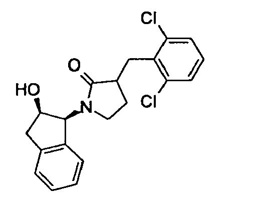

To a solution of 1-((1S,2R)-2-{[tert-butyl(dimethyl)silyl]oxy}-2,3-dihydro-1H-inden-1-yl)-3-(2,6-dichlorobenzyl)pyrrolidin-2-one obtained in Example 286A (240 mg) in tetrahydrofuran (10 mL) was added tetra-n-butylammonium fluoride (1.0 M tetrahydrofuran solution, 1.0 mL), and the mixture was stirred at room temperature for 2 hr. The solvent was evaporated under reduced pressure, and the residue was subjected to silica gel column chromatography (hexane-ethyl acetate 9:1-1:1) to give the title compound (164 mg, 89%) as a pale-yellow oil.
1H NMR (300 MHz, CDCl₃) δ ppm 1.67-2.01 (m, 2 H), 2.76-3.34 (m, 7 H), 3.46-3.61 (m, 1 H), 4.74-4.88 (m, 1 H), 5.51 (d, J=6.6 Hz, 1 H), 7.04-7.14 (m, 1 H), 7.22-7.35 (m, 6 H).

### Example 323A 1-(2,3-dihydro-1H-inden-1-yl)-3-[(3-methoxy-1-methyl-1H-pyrazol-5-yl)carbonyl]pyrrolidin-2-one

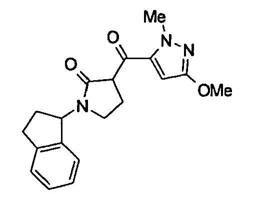

In the same manner as in Example 1A, the title compound was obtained from 1-(2,3-dihydro-1H-inden-1-yl)pyrrolidin-2-one obtained in Reference Example 5A and methyl 3-methoxy-1-methyl-1H-pyrazole-5-carboxylate.
LC-MS (ESI⁺); m/z 340 (M+H)⁺

### Example 324A 3-{[3-(benzyloxy)-1-methyl-1H-pyrazol-5-yl]carbonyl}-1-(2,3-dihydro-1H-inden-1-yl)pyrrolidin-2-one

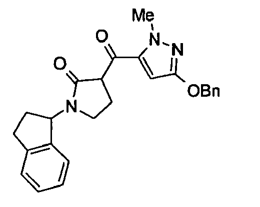

In the same manner as in Example 1A, the title compound was obtained from 1-(2,3-dihydro-1H-inden-1-yl)pyrrolidin-2-one obtained in Reference Example 5A and methyl 3-benzyloxy-1-methyl-1H-pyrazole-5-carboxylate.
LC-MS (ESI⁺); m/z 416 (M+H)⁺

### Example 325A 1-(2,3-dihydro-1H-inden-1-yl)-3-[hydroxy(3-methoxy-1-methyl-1H-pyrazol-5-yl)methyl]pyrrolidin-2-one

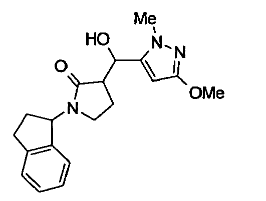

To a solution of 1-(2,3-dihydro-1H-inden-1-yl)-3-[(3-methoxy-1-methyl-1H-pyrazol-5-yl)carbonyl]pyrrolidin-2-one obtained in Example 323A (479 mg) in methanol (10 mL) was added sodium borohydride (53 mg), and the mixture was stirred at room temperature for 15 min. The solvent was evaporated under reduced pressure, and the residue was extracted with ethyl acetate. The organic layer was washed with saturated brine, and dried over anhydrous magnesium sulfate. The solvent was evaporated under reduced pressure, and the residue was recrystallized from hexane-ethyl acetate to give the title compound (162 mg, 34%) as a colorless prism.
LC-MS (ESI⁺); m/z 342 (M+H)⁺

### Example 326A 2-{3-chloro-2-[(1-cyclohexyl-2-oxopyrrolidin-3-yl)methyl]phenoxy}-N,N-diethylacetamide

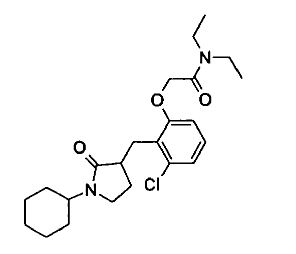

In the same manner as in Example 318A, the title compound was obtained from 3-(2-chloro-6-hydroxybenzyl)-1-cyclohexylpyrrolidin-2-one obtained in Example 177A and 2-chloro-N,N-diethylacetamide.
1H NMR (300 MHz, CDCl₃) δ ppm 1.13 (t, J=7.1 Hz, 3 H), 1.21 (t, J=7.1 Hz, 3 H), 1.27-1.48 (m, 4 H), 1.58-1.98 (m, 8 H), 2.82-2.99 (m, 2 H), 3.08-3.22 (m, 1 H), 3.24-3.48 (m, 6 H), 3.87-4.02 (m, 1 H), 4.71 (s, 2 H), 6.70-6.78 (m, 1 H), 6.96-7.14 (m, 6 H).

### Example 327A 1-cyclohexyl-3-[(2,4-dichloro-6-methoxyphenyl)(hydroxy)methyl]pyrrolidin-2-one (less polar product)

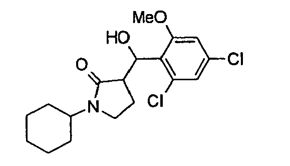

In the same manner as in Example 1A, the title compound was obtained from 1-cyclohexylpyrrolidin-2-one and 2,4-dichloro-6-methoxybenzaldehyde.
1H NMR (300 MHz, CDCl₃) δ ppm 1.00-1.20 (m, 1 H), 1.29-1.57 (m, 5 H), 1.62-1.90 (m, 6 H), 3.16-3.39 (m, 2 H), 3.42-3.56 (m, 1 H), 3.86 (s, 3 H), 3.89-4.04 (m, 1 H), 5.17 (d, J=2.1 Hz, 1 H), 5.40 (dd, J=10.0, 2.1 Hz, 1 H), 6.81 (d, J=1.9 Hz, 1 H), 7.02 (d, J=1.9 Hz, 1 H).

### Example 328A 1-cyclohexyl-3-[(2,4-dichloro-6-methoxyphenyl)(hydroxy)methyl]pyrrolidin-2-one (more polar product)

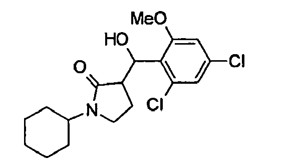

In the same manner as in Example 1A, the title compound was obtained from 1-cyclohexylpyrrolidin-2-one and 2,4-dichloro-6-methoxybenzaldehyde.
1H NMR (300 MHz, CDCl₃) δ ppm 0.97-1.17 (m, 1 H), 1.22-1.46 (m, 4 H), 1.60-1.85 (m, 5 H), 2.03-2.28 (m, 2 H), 2.92-3.04 (m, 1 H), 3.18-3.42 (m, 2 H), 3.80-3.94 (m, 2 H), 3.89 (s, 3 H), 5.34 (dd, J=11.1, 7.9 Hz, 1 H), 6.82 (d, J=1.9 Hz, 1 H), 7.03 (d, J=1.9

Hz, 1 H).

### Example 329A 1-cyclohexyl-3-(2,4-dichloro-6-methoxybenzyl)pyrrolidin-2-one

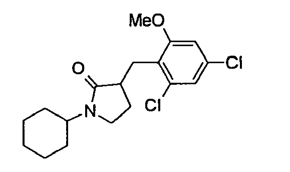

To a solution of 1-cyclohexyl-3-[(2, 4-dichloro-6-methoxyphenyl)(hydroxy)methyl]pyrrolidin-2-one obtained in Example 327A (168 mg) in trifluoroacetic acid (2 mL) was added triethylsilane (216 µL) at room temperature, and the mixture was stirred for 24 hr. The solvent was evaporated under reduced pressure, and the residue was extracted with ethyl acetate. The organic layer was washed with saturated brine, and dried over anhydrous magnesium sulfate. The solvent was evaporated under reduced pressure, and the residue was recrystallized from hexane-ethyl acetate to give the title compound (113 mg, 70%) as a colorless prism.
1H NMR (300 MHz, CDCl₃) δ ppm 0.99-1.18 (m, 1 H), 1.23-1.50 (m, 5 H), 1.60-1.94 (m, 6 H), 2.71-2.89 (m, 2 H), 3.09-3.36 (m, 3 H), 3.82 (s, 3 H), 3.88-4.03 (m, 1 H), 6.75 (d, J=1.9 Hz, 1 H), 7.00 (d, J=1.9 Hz, 1 H).

### Example 330A 3-{[2-chloro-4-methoxy-3-(methoxymethoxy)phenyl](hydroxy)methyl}-1-cyclohexylpyrrolidin-2-one (less polar product)

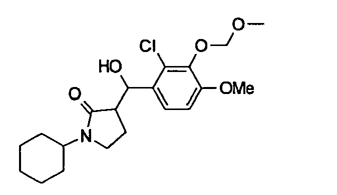

In the same manner as in Example 1A, the title compound was obtained from 1-cyclohexylpyrrolidin-2-one and 2-chloro-4-methoxy-3-(methoxymethoxy)benzaldehyde obtained in Reference Example 35A.
1H NMR (300 MHz, CDCl₃) δ ppm 0.99-1.21 (m, 1 H), 1.26-1.51 (m, 4 H), 1.62-1.89 (m, 7 H), 2.62-2.78 (m, 1 H), 3.11-3.25 (m, 1 H), 3.26-3.37 (m, 1 H), 3.64 (s, 3 H), 3.86 (s, 3 H), 3.89-4.03 (m, 1 H), 5.10-5.19 (m, 2 H), 5.25 (d, J=9.8 Hz, 1 H), 5.70 (s, 1 H), 6.91 (d, J=8.9 Hz, 1 H), 7.34 (d, J=8.9 Hz, 1 H).

### Example 331A 3-{[2-chloro-4-methoxy-3-(methoxymethoxy)phenyl](hydroxy)methyl}-1-cyclohexylpyrrolidin-2-one (more polar product)

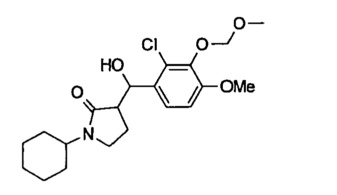

In the same manner as in Example 1A, the title compound was obtained from 1-cyclohexylpyrrolidin-2-one and 2-chloro-4-methoxy-3-(methoxymethoxy)benzaldehyde obtained in Reference Example 35A.
1H NMR (300 MHz, CDCl₃) δ ppm 0.97-1.19 (m, 1 H), 1.22-1.48 (m, 4 H), 1.63-1.97 (m, 7 H), 3.03-3.25 (m, 3 H), 3.58 (d, J=5.5 Hz, 1 H), 3.64 (s, 3 H), 3.85 (s, 3 H), 3.89-4.02 (m, 1 H), 5.16 (s, 2 H), 5.62 (dd, J=5.5, 3.8 Hz, 1 H), 6.86 (d, J=8.7 Hz, 1 H), 7.31 (d, J=8.7 Hz, 1 H).

### Example 332A 3-(2-chloro-3-hydroxy-4-methoxybenzyl)-1-cyclohexylpyrrolidin-2-one

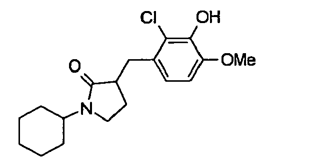

In the same manner as in Example 329A, the title compound was obtained from 3-{[2-chloro-4-methoxy-3-(methoxymethoxy)phenyl](hydroxy)methyl}-1-cyclohexylpyrrolidin-2-one obtained in Example 331A.
1H NMR (300 MHz, CDCl₃) δ ppm 0.98-1.18 (m, 1 H), 1.22-1.49 (m, 4 H), 1.58-1.87 (m, 6 H), 1.90-2.04 (m, 1 H), 2.65-2.85 (m, 2 H), 3.10-3.26 (m, 2 H), 3.37-3.38 (m, 1 H), 3.89 (s, 3 H), 3.90-4.02 (m, 1 H), 5.92 (s, 1 H), 6.69-6.75 (m, 1 H), 6.76-6.82 (m, 1 H).

### Example 333A 1-cyclohexyl-3-[(2,6-dichloro-4-methoxyphenyl)(hydroxy)methyl]pyrrolidin-2-one

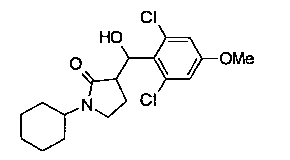

In the same manner as in Example 1A, the title compound was obtained from 1-cyclohexylpyrrolidin-2-one and 2,6-dichloro-4-methoxybenzaldehyde.
1H NMR (300 MHz, CDCl₃) δ ppm 0.99-1.21 (m, 1 H), 1.26-1.50 (m, 4 H), 1.51-1.90 (m, 7 H), 3.15-3.39 (m, 2 H), 3.53-3.69 (m, 1 H), 3.79 (s, 3 H), 3. 88-4. 05 (m, 1 H), 5. 38 (s, 1 H), 5.52 (d, J=10.4 Hz, 1 H), 6.87 (s, 2 H).

### Example 334A 1-cyclohexyl-3-{[2,6-dichloro-4-methoxy-3-(methoxymethoxy)phenyl](hydroxy)methyl}pyrrolidin-2-one (less polar product)

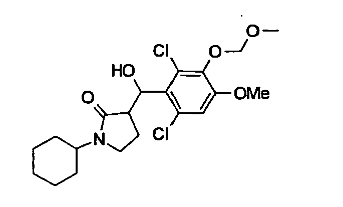

In the same manner as in Example 1A, the title compound was obtained from 1-cyclohexylpyrrolidin-2-one and 2,6-dichloro-4-methoxy-3-(methoxymethoxy)benzaldehyde obtained in Reference Example 36A.
1H NMR (300 MHz, CDCl₃) δ ppm 1.00-1.21 (m, 1 H), 1.23-1.51 (m, 4 H), 1.54-1.90 (m, 7 H), 3.16-3.38 (m, 2 H), 3.54-3.73 (m, 4 H), 3.65 (s, 3 H), 3.90-4.04 (m, 1 H), 5.13 (s, 2 H), 5.34-5.44 (m, 1H), 5.54 (d, J=10.4 Hz, 1H), 6.87 (s, 1H).

### Example 335A 1-cyclohexyl-3-{[2,6-dichloro-4-methoxy-3-(methoxymethoxy)phenyl](hydroxy)methyl}pyrrolidin-2-one (more polar product)

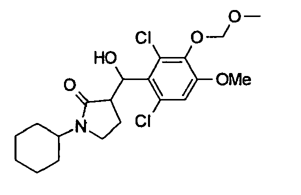

In the same manner as in Example 1A, the title compound was obtained from 1-cyclohexylpyrrolidin-2-one and 2,6-dichloro-4-methoxy-3-(methoxymethoxy)benzaldehyde obtained in Reference Example 36A.
1H NMR (300 MHz, CDCl₃) δ ppm 1.01-1.21 (m, 1 H), 1.27-1.49 (m, 4 H), 1.59-1.89 (m, 7 H), 3.16-3.40 (m, 2 H), 3.55-3.71 (m, 4 H), 3.85 (s, 3 H), 3.89-4.04 (m, 1 H), 5.13 (s, 2 H), 5.38 (brs, 1 H), 5.54 (d, J=10.7 Hz, 1 H), 6.87 (s, 1 H).

### Example 336A 1-cyclohexyl-3-(2,6-dichloro-3-hydroxy-4-methoxybenzyl) pyrrolidin-2-one

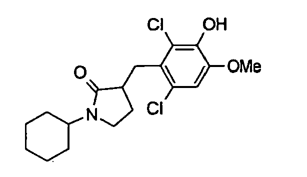

In the same manner as in Example 329A, the title compound was obtained from 1-cyclohexyl-3-{[2,6-dichloro-4-methoxy-3-(methoxymethoxy)phenyl](hydroxy)methyl}pyrrolidin-2-one obtained in Example 335A.
1H NMR (300 MHz, CDCl₃) δ ppm 0.98-1.18 (m, 1 H), 1.23-1.50 (m, 4 H), 1.62-1.99 (m, 7 H), 2.79-3.02 (m, 2 H), 3.10-3.24 (m, 1 H), 3.27-3.47 (m, 2 H), 3.84-4.03 (m, 4 H), 5.84 (s, 1 H), 6.84 (s, 1 H).

### Example 337A 2-{3-chloro-4-[(1-cyclohexyl-2-oxopyrrolidin-3-yl)methyl]phenoxy}-N,N-diethylacetamide

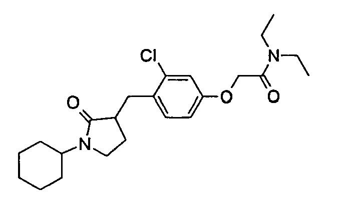

In the same manner as in Example 318A, the title compound was obtained from 3-(2-chloro-4-hydroxybenzyl)-1-cyclohexylpyrrolidin-2-one obtained in Example 183A and 2-chloro-N,N-diethylacetamide.
1H NMR (300 MHz, CDCl₃) δ ppm 0.98-1.48 (m, 11 H), 1.58-1.85 (m, 6 H), 1.92-2.06 (m, 1 H), 2.66-2.84 (m, 2 H), 3.11-3.47 (m, 7 H), 3.86-4.02 (m, 1 H), 4.63 (s, 2 H), 6.80 (dd, J=8.5, 2.6 Hz, 1H), 6.94 (d, J=2.6 Hz, 1H), 7.18 (d, J=8.5 Hz, 1H).

### Example 338A 3-[2-chloro-4-(2-methoxyethoxy)benzyl]-1-cyclohexylpyrrolidin-2-one

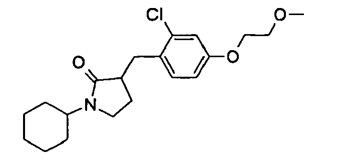

To a solution of 3-(2-chloro-4-hydroxybenzyl)-1-cyclohexylpyrrolidin-2-one obtained in Example 183A (308 mg), 2-methoxyethanol (118 µL) and n-tributylphosphine (500 µL) in tetrahydrofuran (5 mL) was added 1,1'-azodicarbonyldipiperidine (505 mg) at 50°C, and the mixture was stirred for 1.5 hr. The solvent was evaporated under reduced pressure, the residue was washed with diisopropyl ether, and filtrated, and the filtrate was concentrated. The residue was subjected to silica gel column chromatography (hexane-ethyl acetate 95:5-75:25) to give the title compound (261 mg, 71%) as a colorless oil.
1H NMR (300 MHz, CDCl₃) δ ppm 0.98-1.17 (m, 1 H), 1.22-1.49 (m, 4 H), 1.54-1.86 (m, 6 H), 1.90-2.07 (m, 1 H), 2.66-2.84 (m, 2 H), 3.12-3.36 (m, 3 H), 3.45 (s, 3 H), 3.69-3.77 (m, 2 H), 3.87-4.01 (m, 1 H), 4.03-4.13 (m, 2 H), 6.77 (dd, J=8.5, 2.6 Hz, 1 H), 6.94 (d, J=2.6 Hz, 1 H), 7.17 (d, J=8.5 Hz, 1 H).

### Example 339A 3-{4-[2-(benzyloxy)ethoxy]-2-chlorobenzyl}-1-cyclohexylpyrrolidin-2-one

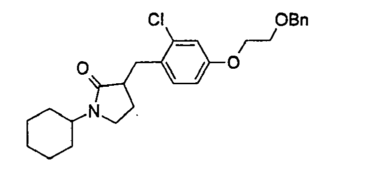

In the same manner as in Example 338A, the title compound was obtained from 3-(2-chloro-4-hydroxybenzyl)-1-cyclohexylpyrrolidin-2-one obtained in Example 183A and 2-benzyloxyethanol.
1H NMR (300 MHz, CDCl₃) δ ppm 0.96-1.18 (m, 1 H), 1.22-1.49 (m, 4 H), 1.49-1.86 (m, 6 H), 1.89-2.05 (m, 1 H), 2.66-2.84 (m, 2 H), 3.10-3.38 (m, 3 H), 3.75-3.85 (m, 2 H), 3.87-4.01 (m, 1 H), 4.06-4.16 (m, 2 H), 4. 63 (s, 2 H), 6.77 (dd, J=8.5, 2.6 Hz, 1 H), 6.93 (d, J=2.6 Hz, 1 H), 7.16 (d, J=8.5 Hz, 1 H), 7.24-7.39 (m, 5 H).

### Example 340A methyl 4-({3-chloro-4-[(1-cyclohexyl-2-oxopyrrolidin-3-yl)methyl]phenoxy}methyl)benzoate

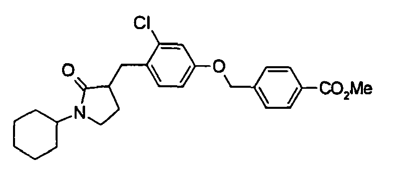

In the same manner as in Example 318A, the title compound was obtained from 3-(2-chloro-4-hydroxybenzyl)-1-cyclohexylpyrrolidin-2-one obtained in Example 183A and methyl 4-(bromomethyl)benzoate.
1H NMR (300 MHz, CDCl₃) δ ppm 0.96-1.16 (m, 1 H), 1.22-1.49 (m, 4 H), 1.56-1.85 (m, 6 H), 1.92-2.07 (m, 1 H), 2.67-2.83 (m, 2 H), 3.31-3.36 (m, 3 H), 3.87-4.03 (m, 4 H), 5.09 (s, 2 H), 6.80 (dd, J=8.5, 2.6 Hz, 1 H), 6.98 (d, J=2.6 Hz, 1 H), 7.19 (d, J=8.5 Hz, 1 H), 7.48 (d, J=8.2 Hz, 2 H), 8.06 (d, J=8.2 Hz, 2 H).

### Example 341A methyl {3-chloro-4-[(1-cyclohexyl-2-oxopyrrolidin-3-yl)methyl]phenoxy}acetate

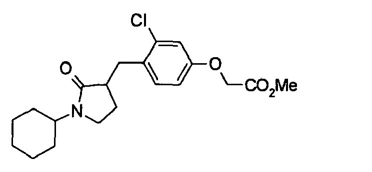

In the same manner as in Example 318A, the title compound was obtained from 3-(2-chloro-4-hydroxybenzyl)-1-cyclohexylpyrrolidin-2-one obtained in Example 183A and methyl bromoacetate.
1H NMR (300 MHz, CDCl₃) δ ppm 0.99-1.19 (m, 1 H), 1.21-1.49 (m, 4 H), 1.56-1.87 (m, 6 H), 1.92-2.08 (m, 1 H), 2.67-2.83 (m, 2 H), 3.11-3.36 (m, 3 H), 3.81 (s, 3 H), 3.87-4.03 (m, 1 H), 4.60 (s, 2 H), 6.75 (dd, J=8.5, 2.7 Hz, 1 H), 6.92 (d, J=2.7 Hz, 1 H), 7.20 (d, J=8.5 Hz, 1 H).

### Example 342A 4-({3-chloro-4-[(1-cyclohexyl-2-oxopyrrolidin-3-yl)methyl]phenoxy}methyl)benzoic acid

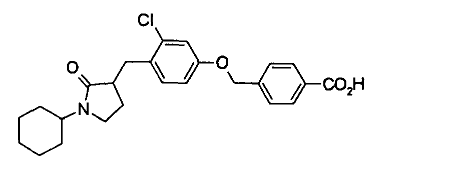

In the same manner as in Example 129A, the title compound was obtained from methyl 4-({3-chloro-4-[(1-cyclohexyl-2-oxopyrrolidin-3-yl)methyl]phenoxy}methyl)benzoate obtained in Example 340A.
1H NMR (300 MHz, CDCl₃) δ ppm 0.96-1.96 (m, 12 H), 2.54-2.70 (m, 1 H), 3.03-3.80 (m, 5 H), 5.20 (s, 2 H), 6.94 (dd, J=8.5, 2.5 Hz, 1 H), 7.11 (d, J=2.5 Hz, 1 H), 7.27 (d, J=8.5 Hz, 1 H), 7.54 (d, J=8.3 Hz, 2 H), 7.96 (d, J=8.3 Hz, 2 H).

### Example 343A {3-chloro-4-[(1-cyclohexyl-2-oxopyrrolidin-3-yl)methyl]phenoxy}acetic acid

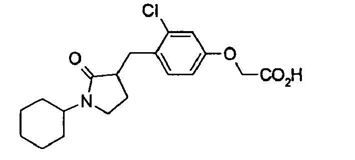

In the same manner as in Example 129A, the title compound was obtained from methyl {3-chloro-4-[(1-cyclohexyl-2-oxopyrrolidin-3-yl)methyl]phenoxy}acetate obtained in Example 341A.
1H NMR (300 MHz, CDCl₃) δ ppm 0.99-1.17 (m, 1 H), 1.18-1.66 (m, 8 H), 1.67-1.80 (m, 2 H), 1.81-1.97 (m, 1 H), 2.53-2.71 (m, 1 H), 3.04-3.27 (m, 3 H), 3.54-3.82 (m, 2 H), 4.69 (s, 2 H), 6.85 (dd, J=8.5, 2.7 Hz, 1 H), 6.99 (d, J=2.7 Hz, 1 H), 7.26 (d, J=8.5 Hz, 1 H), 13.07 (brs, 1 H).

### Example 344A 3-[2-chloro-4-(2-hydroxyethoxy)benzyl]-1-cyclohexylpyrrolidin-2-one

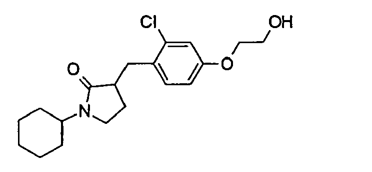

In the same manner as in Example 140A, the title compound was obtained from 3-{4-[2-(benzyloxy)ethoxy]-2-chlorobenzyl)-1-cyclohexylpyrrolidin-2-one obtained in Example 339A.
1H NMR (300 MHz, CDCl₃) δ ppm 0.96-1.19 (m, 1 H), 1.22-1.49 (m, 4 H), 1.55-1.87 (m, 5 H), 1.89-2.13 (m, 2 H), 2.67-2.83 (m, 2 H), 3.11-3.36 (m, 3 H), 3.86-4.10 (m, 5 H), 6.76 (dd, J=8.5, 2.5 Hz, 1 H), 6.93 (d, J=2.5 Hz, 1 H), 7.18 (d, J=8.5 Hz, 1 H).

### Example 345A 3-(2-chloro-4-{[3-chloro-5-(trifluoromethyl)pyridin-2-yl]oxy}benzyl)-1-cyclohexylpyrrolidin-2-one

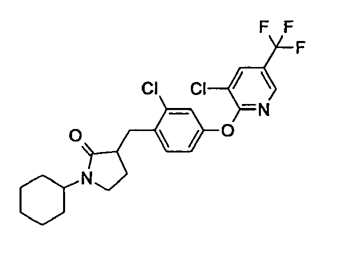

In the same manner as in Example 142A, the title compound was obtained from 3-(2-chloro-4-hydroxybenzyl)-1-cyclohexylpyrrolidin-2-one obtained in Example 183A and 2,3-dichloro-5-(trifluoromethyl)pyridine.
1H NMR (300 MHz, CDCl₃) δ ppm 0.96-1.18 (m, 1 H), 1.23-1.50 (m, 4 H), 1.57-1.88 (m, 6 H), 1.98-2.14 (m, 1 H), 2.74-2.90 (m, 2 H), 3.13-3.29 (m, 2 H), 3.32-3.46 (m, 1 H), 3.88-4.04 (m, 1 H), 7.03 (dd, J=8.5, 2.5 Hz, 1 H), 7.22 (d, J=2.5 Hz, 1 H), 7.36 (d, J=8.5 Hz, 1 H), 7.95-8.01 (m, 1 H), 8.25-8.31 (m, 1 H).

### Example 346A methyl 3-({3-chloro-4-[(1-cyclohexyl-2-oxopyrrolidin-3-yl)methyl]phenoxy}methyl)benzoate

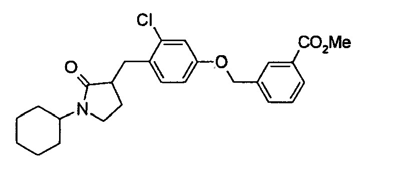

In the same manner as in Example 318A, the title compound was obtained from 3-(2-chloro-4-hydroxybenzyl)-1-cyclohexylpyrrolidin-2-one obtained in Example 183A and methyl 3-(bromomethyl)benzoate.
1H NMR (300 MHz, CDCl₃) δ ppm 0.98-1.17 (m, 1 H), 1.22-1.48 (m, 4 H), 1.57-1.85 (m, 6 H), 1.91-2.07 (m, 1 H), 2.67-2.84 (m, 2 H), 3.12-3.37 (m, 3 H), 3.86-4.03 (m, 4 H), 5.06 (s, 2 H), 6.81 (dd, J=8.5, 2.6 Hz, 1 H), 7.00 (d, J=2.6 Hz, 1 H), 7.19 (d, J=8.5 Hz, 1 H), 7.42-7.53 (m, 1 H), 7.57-7.67 (m, 1H), 7.97-8.14 (m, 2 H). Example 347A 3-(2-chloro-5-methoxybenzyl)-1-cyclohexylpyrrolidin-2-one

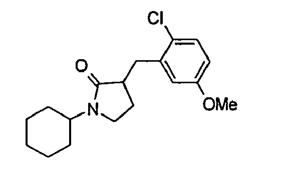

In the same manner as in Example 1A, the title compound was obtained from 1-cyclohexylpyrrolidin-2-one and 2-chloro-5-methoxybenzyl bromide.
1H NMR (300 MHz, CDCl₃) δ ppm 1.00-1.17 (m, 1 H), 1.24-1.49 (m, 4 H), 1.57-1.85 (m, 6 H), 1.93-2.07 (m, 1 H), 2.68-2.86 (m, 2 H), 3.12-3.39 (m, 3 H), 3.77 (s, 3 H), 3.88-4.02 (m, 1 H), 6.70 (dd, J=8.8, 3.1 Hz, 1 H), 6.83 (d, J=3.1 Hz, 1 H), 7.24 (d, J=8.8 Hz, 1 H).

### Example 348A 3-({3-chloro-4-[(1-cyclohexyl-2-oxopyrrolidin-3-yl)methyl]phenoxy}methyl)benzoic acid

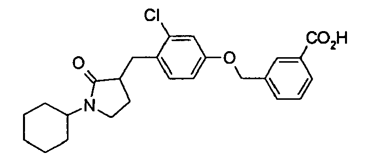

In the same manner as in Example 129A, the title compound was obtained from methyl 3-({3-chloro-4-[(1-cyclohexyl-2-oxopyrrolidin-3-yl)methyl]phenoxy}methyl)benzoate obtained in Example 346A.
1H NMR (300 MHz, CDCl₃) δ ppm 0.97-1.18 (m, 1 H), 1.22-1.50 (m, 4 H), 1.57-1.86 (m, 6 H), 1.91-2.09 (m, 1 H), 2.67-2.88 (m, 2 H), 3.11-3.37 (m, 3 H), 3.88-4.04 (m, 1 H), 5.08 (s, 2 H), 6.82 (dd, J=8.5, 2.6 Hz, 1 H), 7.00 (d, J=2.6 Hz, 1 H), 7.20 (d, J=8.5 Hz, 1 H), 7.44-7.56 (m, 1 H), 7.61-7.70 (m, 1H), 8.02-8.20 (m, 2 H).

### Example 349A 3-(2-chloro-5-hydroxybenzyl)-1-cyclohexylpyrrolidin-2-one

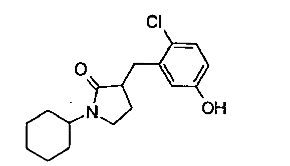

In the same manner as in Example 140A, the title compound was obtained from 3-(2-chloro-5-methoxybenzyl)-1-cyclohexylpyrrolidin-2-one obtained in Example 347A.
1H NMR (300 MHz, CDCl₃) δ ppm 0.96-1.17 (m, 1 H), 1.20-1.44 (m, 4 H), 1.58-1.90 (m, 6 H), 1.97-2.14 (m, 1 H), 2.79-2.95 (m, 2 H), 3.06-3.28 (m, 3 H), 3.87-4.02 (m, 1 H), 6.69 (dd, J=8.6, 3.0 Hz, 1 H), 6.96 (d, J=3.0 Hz, 1 H), 7.16 (d, J=8.6 Hz, 1 H).

### Example 350A 3-[2-chloro-5-(2-methoxyethoxy)benzyl]-1-cyclohexylpyrrolidin-2-one

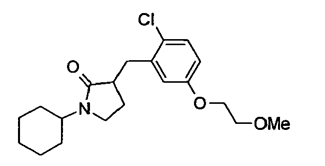

In the same manner as in Example 338A, the title compound was obtained from 3-(2-chloro-5-hydroxybenzyl)-1-cyclohexylpyrrolidin-2-one obtained in Example 349A and 2-methoxyethanol.
1H NMR (300 MHz, CDCl₃) δ ppm 0.97-1.18 (m, 1 H), 1.22-1.50 (m, 4 H), 1.52-1.86 (m, 6 H), 1.91-2.07 (m, 1 H), 2.65-2.86 (m, 2 H), 3.10-3.27 (m, 2 H), 3.28-3.39 (m, 1 H), 3.45 (s, 3 H), 3.68-3.77 (m, 2 H), 3.88-4.01 (m, 1 H), 4.02-4.10 (m, 2 H), 6.73 (dd, J=8.9, 3.0 Hz, 1 H), 6.86 (d, J=3.0 Hz, 1 H), 7.23 (d, J=8.9 Hz, 1 H).

### Example 351A methyl {4-chloro-3-[(1-cyclohexyl-2-oxopyrrolidin-3-yl)methyl]phenoxy}acetate

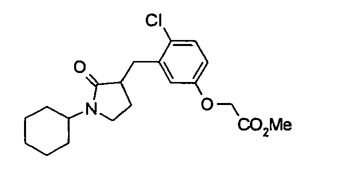

In the same manner as in Example 318A, the title compound was obtained from 3-(2-chloro-5-hydroxybenzyl)-1-cyclohexylpyrrolidin-2-one obtained in Example 349A and methyl 2-bromoacetate.
1H NMR (300 MHz, CDCl₃) δ ppm 0.97-1.19 (m, 1 H), 1.22-1.50 (m, 4 H), 1.55-1.87 (m, 6 H), 1.92-2.08 (m, 1 H), 2.65-2.86 (m, 2 H), 3.11-3.41 (m, 3 H), 3.81 (s, 3 H), 3.88-4.02 (m, 1 H), 4.60 (s, 2 H), 6.71 (dd, J=8.7, 3.0 Hz, 1 H), 6.85 (d, J=3.0 Hz, 1 H), 7.20-7.29 (m, 1 H).

### Example 352A {4-chloro-3-[(1-cyclohexyl-2-oxopyrrolidin-3-yl)methyl]phenoxy}acetic acid

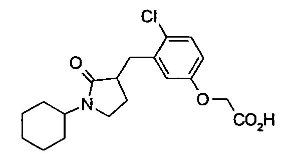

In the same manner as in Example 129A, the title compound was obtained from methyl {4-chloro-3-[(1-cyclohexyl-2-oxopyrrolidin-3-yl)methyl]phenoxy}acetate obtained in Example 351A.
1H NMR (300 MHz, CDCl₃) δ ppm 0.96-1.19 (m, 1 H), 1.20-1.50 (m, 4 H), 1.54-1.93 (m, 6 H), 1.95-2.14 (m, 1 H), 2.77-2.96 (m, 2 H), 3.11-3.33 (m, 3 H), 3.85-4.03 (m, 1 H), 4.62 (s, 2 H), 6.77 (dd, J=8.7, 3.1 Hz, 1 H), 6.96 (d, J=3.1 Hz, 1 H), 7.23 (d, J=8.7 Hz, 1 H).

### Example 353A 3-{4-[3-(benzyloxy)propoxy]-2-chlorobenzyl}-1-cyclohexylpyrrolidin-2-one

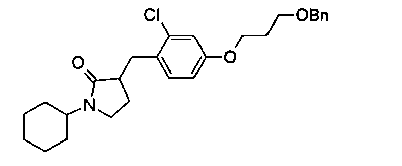

In the same manner as in Example 338A, the title compound was obtained from 3-(2-chloro-4-hydroxybenzyl)-1-cyclohexylpyrrolidin-2-one obtained in Example 183A and 3-(benzyloxy)propanol.
1H NMR (300 MHz, CDCl₃) δ ppm 0.95-1.20 (m, 1 H), 1.22-1.50 (m, 4 H), 1.53-1.87 (m, 6 H), 1.91-2.15 (m, 3 H), 2.66-2.84 (m, 2 H), 3.10-3.36 (m, 3 H), 3.64 (t, J=6.1 Hz, 2 H), 3.87-4.01 (m, 1 H), 4.04 (t, J=6.2 Hz, 2 H), 4.52 (s, 2 H), 6.73 (dd, J=8.5, 2.6 Hz, 1 H), 6.90 (d, J=2.6 Hz, 1 H), 7.16 (d, J=8.5 Hz, 1 H), 7.24-7.37 (m, 5 H).

### Example 354A 3-(2-chloro-4-{[4-(hydroxymethyl)benzyl]oxy}benzyl)-1-cyclohexylpyrrolidin-2-one

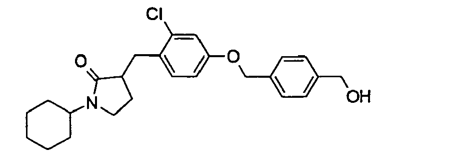

In the same manner as in Reference Example 19A, the title compound was obtained from methyl 4-({3-chloro-4-[(1-cyclohexyl-2-oxopyrrolidin-3-yl)methyl]phenoxy}methyl)benzoate obtained in Example 340A.
1H NMR (300 MHz, CDCl₃) δ ppm 0.97-1.17 (m, 1 H), 1.22-1.51 (m, 4 H), 1.54-1.87 (m, 7 H), 1.91-2.06 (m, 1 H), 2.65-2.83 (m, 2 H), 3.09-3.36 (m, 3 H), 3.86-4.01 (m, 1 H), 4.72 (d, J=5.3 Hz, 2 H), 5.02 (s, 2 H), 6.80 (dd, J=8.5, 2.6 Hz, 1 H), 6.98 (d, J=2.6 Hz, 1 H), 7.17 (d, J=8.5 Hz, 1 H), 7.36-7.45 (m, 4 H).

### Example 355A 3-[2-chloro-5-(2-hydroxyethoxy)benzyl]-1-cyclohexylpyrrolidin-2-one

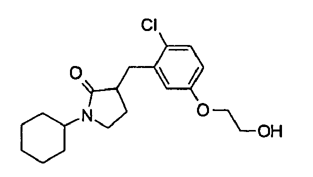

In the same manner as in Example 354A, the title compound was obtained from methyl {4-chloro-3-[(1-cyclohexyl-2-oxopyrrolidin-3-yl)methyl]phenoxy}acetate obtained in Example 351A.
1H NMR (300 MHz, CDCl₃) δ ppm 0.98-1.19 (m, 1 H), 1.21-1.52 (m, 4 H), 1.54-1.86 (m, 6 H), 1.94-2.18 (m, 2 H), 2.68-2.87 (m, 2 H), 3.10-3.41 (m, 3 H), 3.84-4.10 (m, 5 H), 6.72 (dd, J=8.7, 3.0 Hz, 1H), 6.87 (d, J=3.0 Hz, 1H), 7.24 (d, J=8.7 Hz, 1H).

### Example 356A 3-[2-chloro-4-(2-isopropoxyethoxy)benzyl]-1-cyclohexylpyrrolidin-2-one

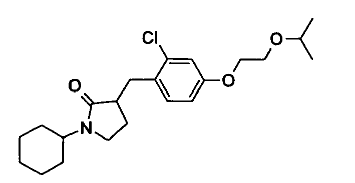

In the same manner as in Example 338A, the title compound was obtained from 3-(2-chloro-4-hydroxybenzyl)-1-cyclohexylpyrrolidin-2-one obtained in Example 183A and 2-isopropoxyethanol.
1H NMR (300 MHz, CDCl₃) δ ppm 0.94-1.18 (m, 1 H), 1.20 (d, J=6.2 Hz, 6 H), 1.24-1.49 (m, 4 H), 1.58-1.86 (m, 6 H), 1.90-2.05 (m, 1 H), 2.66-2.83 (m, 2 H), 3.12-3.35 (m, 3 H), 3.60-3.79 (m, 3 H), 3.87-4.01 (m, 1 H), 4.02-4.11 (m, 2 H), 6.76 (dd, J=8.5, 2.6 Hz, 1 H), 6.93 (d, J=2.6 Hz, 1 H), 7.16 (d, J=8.5 Hz, 1 H).

### Example 357A 3-[2-chloro-4-(3-hydroxypropoxy)benzyl]-1-cyclohexylpyrrolidin-2-one

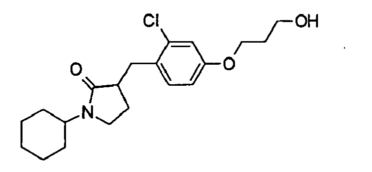

In the same manner as in Example 140A, the title compound was obtained from 3-{4-[3-(benzyloxy)propoxy]-2-chlorobenzyl}-1-cyclohexylpyrrolidin-2-one obtained in Example 353A.
1H NMR (300 MHz, CDCl₃) δ ppm 0.98-1.18 (m, 1 H), 1.22-1.49 (m, 4 H), 1.54-1.88 (m, 6 H), 1.91-2.13 (m, 3 H), 2.66-2.84 (m, 2 H), 3.11-3.36 (m, 3 H), 3.80-4.03 (m, 3 H), 4.09 (t, J=5.9 Hz, 2 H), 6. 75 (dd, J=8. 5, 2.6 Hz, 1 H), 6.92 (d, J=2.6 Hz, 1 H), 7.17 (d, J=8.5 Hz, 1 H).

### Example 358A 3-{2-chloro-4-[2-(pyrrolidin-1-yl)ethoxy]benzyl}-1-cyclohexylpyrrolidin-2-one

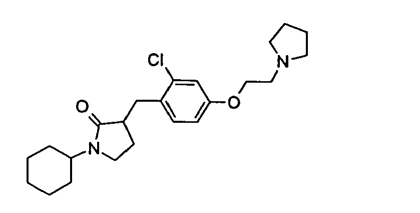

In the same manner as in Example 338A, the title compound was obtained from 3-(2-chloro-4-hydroxybenzyl)-1-cyclohexylpyrrolidin-2-one obtained in Example 183A and 2-(pyrrolidin-1-yl)ethanol.
1H NMR (300 MHz, CDCl₃) δ ppm 1.02-1.17 (m, 1 H), 1.22-1.89 (m, 14 H), 1. 90-2. 05 (m, 1 H), 2. 55-2. 82 (m, 6 H), 2.88 (t, J=5. 9 Hz, 2 H), 3.12-3.35 (m, 3 H), 3.86-4.01 (m, 1 H), 4.06 (t, J=5.9 Hz, 2 H), 6.76 (dd, J=8 . 5, 2.6 Hz, 1 H), 6.92 (d, J=2.6 Hz, 1 H), 7.16 (d, J=8.5 Hz, 1 H).

### Example 359A 3-[2-chloro-4-(methoxymethoxy)benzyl]-1-(1, 4-dioxaspiro[4.5]dec-8-yl)pyrrolidin-2-one

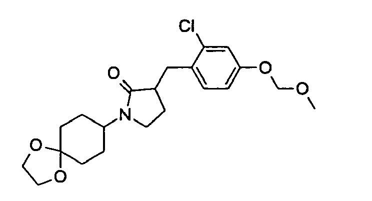

In the same manner as in Example 1A, the title compound was obtained from 1-(1,4-dioxaspiro[4.5]dec-8-yl)pyrrolidin-2-one and 2-chloro-4-(methoxymethoxy)benzyl bromide obtained in Reference Example 24A.
1H NMR (300 MHz, CDCl₃) δ ppm 1.57-1.88 (m, 9 H), 1.93-2.09 (m, 1 H), 2.66-2.84 (m, 2 H), 3.12-3.36 (m, 3 H), 3.47 (s, 3 H), 3.94 (s, 4 H), 3.99-4.12 (m, 1 H), 5.13 (s, 2 H), 6.87 (dd, J=8.5, 2.5 Hz, 1 H), 7.07 (d, J=2.5 Hz, 1 H), 7.17 (d, J=8.5 Hz, 1 H).

### Example 360A 3-(2-chloro-4-hydroxybenzyl)-1-(4-oxocyclohexyl)pyrrolidin-2-one

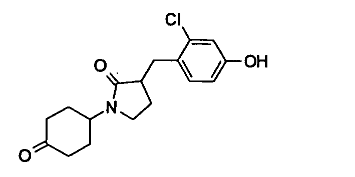

In the same manner as in Example 28A, the title compound was obtained from 3-[2-chloro-4-(methoxymethoxy)benzyl]-1-(1,4-dioxaspiro[4.5]dec-8-yl)pyrrolidin-2-one obtained in Example 359A.
1H NMR (300 MHz, CDCl₃) δ ppm 1.72 -2.15 (m, 6 H), 2.36-2.60 (m, 4 H), 2.68-2.90 (m, 2 H), 3.14-3.31 (m, 3 H), 4.47 (tt, J=12.0, 3.8 Hz, 1 H), 6.68 (dd, J=8.3, 2.5 Hz, 1 H), 6.81 (brs, 1 H), 6.89 (d, J=2.5 Hz, 1 H), 7.09 (d, J=8.3 Hz, 1 H).

### Example 361A 1-(1-adamantyl)-4-(2, 6-dichlorobenzyl)-5-oxoproline ethyl ester

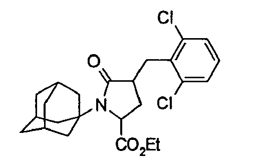

To a solution of 1-(1-adamantyl)-5-oxoproline ethyl ester obtained in Reference Example 15A (291 mg) in tetrahydrofuran (10 mL) was added lithium bis(trimethylsilyl)amide (1.1 M tetrahydrofuran solution, 2.0 mL) at -78°C under argon stream, and the mixture was stirred for 20 min. A solution of 2,6-dichlorobenzyl chloride (215 mg) in tetrahydrofuran (5 mL) was added to the reaction mixture, and the mixture was further stirred for 1 hr. Saturated aqueous ammonium chloride was added to the reaction mixture, and the mixture was extracted with ethyl acetate. The organic layer was washed with saturated brine, and dried over anhydrous magnesium sulfate. The solvent was evaporated under reduced pressure, the residue was subjected to silica gel column chromatography (hexane-ethyl acetate 95:5-75:25), and the obtained oil was recrystallized from hexane-ethyl acetate to give the title compound (124 mg, 28%) as a colorless prism.
1H NMR (300 MHz, CDCl₃) δ ppm 1.26 (t, J=7.2 Hz, 3 H), 1.60 -1.76 (m, 6 H), 1.84-1.95 (m, 1 H), 2.03-2.28 (m, 10 H), 2.90-3.10 (m, 2 H), 3.49-3.60 (m, 1 H), 4.10-4.24 (m, 2 H), 4.30 (d, J=9.2 Hz, 1 H), 7.03-7.14 (m, 1 H), 7.23-7.32 (m, 2 H).

### Example 362A 1-(1-adamantyl)-4-(2-chloro-4-fluorobenzyl)-5-oxoproline ethyl ester

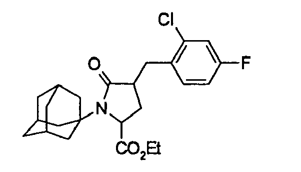

In the same manner as in Example 361A, the title compound was obtained from 1-(1-adamantyl)-5-oxoproline ethyl ester obtained in Reference Example 15A and 2-chloro-4-fluorobenzyl bromide.
1H NMR (300 MHz, CDCl₃) δ ppm 1.28 (t, J=7.2 Hz, 3 H), 1.59 -1.75 (m, 6 H), 1.80-2.25 (m, 11 H), 2.68-2.81 (m, 1 H), 2.83-2.99 (m, 1 H), 3.30 (dd, J=13.9, 4.5 Hz, 1 H), 4.12-4.29 (m, 3 H), 6.91 (td, J=8.3, 2.6 Hz, 1 H), 7.09 (dd, J=8.5, 2.6 Hz, 1 H), 7.20-7.31 (m, 1 H).

### Example 363A 1-(1-adamantyl)-4-(2-chloro-6-methoxybenzyl)-5-oxoproline ethyl ester

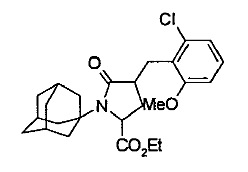

In the same manner as in Example 361A, the title compound was obtained from 1-(1-adamantyl)-5-oxoproline ethyl ester obtained in Reference Example 15A and 2-chloro-6-methoxybenzyl bromide.
1H NMR (300 MHz, CDCl₃) δ ppm 1.25 (t, J=7.2 Hz, 3 H), 1.59 -1.89 (m, 7 H), 2.01-2.29 (m, 10 H), 2.72-2.85 (m, 1 H), 2.87-3.02 (m, 1 H), 3.39 (dd, J=13.0, 3.6 Hz, 1 H), 3.81 (s, 3 H), 4.08-4.22 (m, 2 H), 4.28 (d, J=9.0 Hz, 1 H), 6.70-6.77 (m, 1 H), 6.91-7.00 (m, 1 H), 7.05-7.14 (m, 1 H).

### Example 364A tert-butyl (3-chloro-4-{[1-(2,3-dihydro-1H-inden-1-yl)-2-oxopyrrolidin-3-yl]methyl}phenyl)(methyl)carbamate

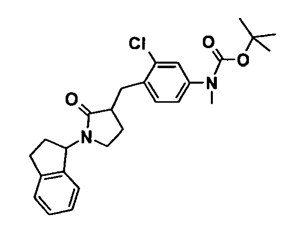

In the same manner as in Example 1A, the title compound was obtained from 1-(2,3-dihydro-1H-inden-1-yl)pyrrolidin-2-one obtained in Reference Example 5A and tert-butyl [3-chloro-4-(chloromethyl)phenyl](methyl)carbamate obtained in Reference Example 29A.
1H NMR (300 MHz, CDCl₃) δ ppm 1.46 (s, 9 H), 1.59-2.03 (m, 3 H), 2.30-2.52 (m, 1 H), 2.72-3.17 (m, 6 H), 3.20-3.29 (m, 3 H), 3.33-3.53 (m, 1 H), 5.73-5.90 (m, 1 H), 7.01-7.15 (m, 2 H), 7.14-7.33 (m, 5 H).

### Example 365A 3-[2-chloro-4-(methylamino)benzyl]-1-(2,3-dihydro-1H-inden-1-yl)pyrrolidin-2-one

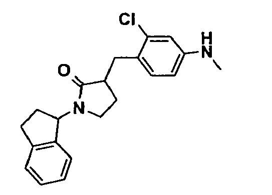

In the same manner as in Example 20A, the title compound was obtained from tert-butyl (3-chloro-4-{[1-(2,3-dihydro-1H-inden-1-yl)-2-oxopyrrolidin-3-yl]methyl}phenyl)(methyl)carbamate obtained in Example 364A.
1H NMR (300 MHz, CDCl₃) δ ppm 1.60 (dd, J=12.7, 8.5 Hz, 1 H), 1.82-2.05 (m, 2 H), 2.25 (dd, J=8.5, 4.3 Hz, 1 H), 2.47-2.64 (overlapping, 3 H), 2.63-3.02 (m, 6 H), 3.02-3.24 (m, 2 H), 5.60 (t, J=7.6 Hz, 1 H), 6.68 (d, J=7.7 Hz, 1 H), 6.79 (s, 1 H), 7.08 (d, J=7.0 Hz, 1 H), 7.12-7.33 (m, 4 H).

### Example 366A 1-(1-adamantyl)-3-(4-fluoro-2-methoxybenzyl)pyrrolidin-2-one

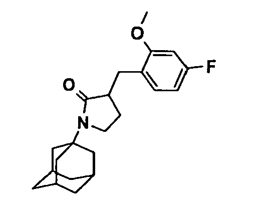

In the same manner as in Example 1A, the title compound was obtained from 1-(1-adamantyl)pyrrolidin-2-one and 4-fluoro-2-methoxybenzyl chloride.
1H NMR (300 MHz, CDCl₃) δ ppm 1.46-1.77 (m, 6 H), 1.77-1.95 (m, 1 H), 2.02-2.21 (m, 10 H), 2.43-2.57 (m, 1 H), 2.56-2.74 (m, 1 H), 3.14-3.41 (m, 3 H), 3.79 (s, 3 H), 6.52-6.61 (m, 2 H), 7.08 (dd, J=8. 9, 6.9 Hz, 1 H) .

### Example 367A tert-butyl (4-{[1-(1-adamantyl)-2-oxopyrrolidin-3-yl]methyl}-3-chlorophenyl)(methyl)carbamate

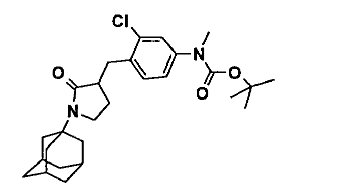

In the same manner as in Example 1A, the title compound was obtained from 1-(1-adamantyl)pyrrolidin-2-one and tert-butyl [3-chloro-4-(chloromethyl)phenyl](methyl)carbamate obtained in Reference Example 29A.
1H NMR (300 MHz, CDCl₃) δ ppm 1.46 (s, 9 H), 1.58-1.76 (m, 8 H), 1.83-2.00 (m, 1 H), 2.04-2.23 (m, 8 H), 2.66-2.78 (m, 2 H), 3.20-3.44 (m, 3 H), 3.23 (s, 3 H), 7.08 (dd, J=8.3, 2.3 Hz, 1 H), 7.21 (d, J=8.3 Hz, 1 H), 7.26 (m, 1 H).

### Example 368A 1-(1-adamantyl)-3-[2-chloro-4-(methylamino)benzyl]pyrrolidin-2-one

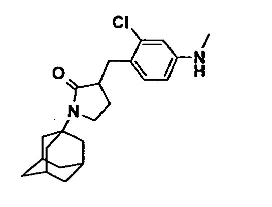

In the same manner as in Example 20A, the title compound was obtained from tert-butyl (4-{[1-(1-adamantyl)-2-oxopyrrolidin-3-yl]methyl}-3-chlorophenyl)(methyl)carbamate obtained in Example 367A.
1H NMR (300 MHz, CDCl₃) δ ppm 1.39-1.71 (m, 8 H), 1.72-1.89 (m, 1 H), 1.95-2.19 (m, 8 H), 2.39-2.63 (m, 2 H), 2.74 (s, 3 H), 2.97-3.15 (m, 1 H), 3.15-3.47 (m, 2 H), 6.82 (d like, 1 H), 6.94 (s, 1 H), 7.21 (d like, 1 H).

### Example 369A 1-(1-adamantyl)-3-[2-chloro-4-(dimethylamino)benzyl]pyrrolidin-2-one

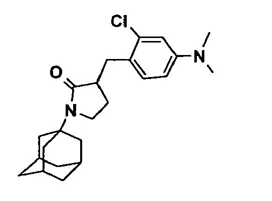

In the same manner as in Reference Example 1A, the title compound was obtained from 1-(1-adamantyl)-3-[2-chloro-4-(methylamino)benzyl]pyrrolidin-2-one obtained in Example 368A and methyl iodide.
1H NMR (300 MHz, CDCl₃) δ ppm 1.51-1.82 (m, 8 H), 1.82-1.99 (m, 1 H), 2.02-2.21 (m, 8 H), 2.54-2.71 (m, 2 H), 2.91 (s, 6 H), 3.17-3.44 (m, 3 H), 6.56 (dd, J=8.5, 2.6 Hz, 1 H), 6.69 (d, J=2.6 Hz, 1 H), 7.10 (d, J=8.5 Hz, 1 H).

### Example 370A 3-(2,6-dichlorobenzyl)-1-(trans-4-hydroxycyclohexyl)pyrrolidin-2-one

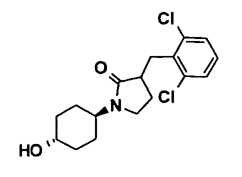

In the same manner as in Example 255A, the title compound was obtained from 1-(trans-4-hydroxycyclohexyl)pyrrolidin-2-one obtained in Reference Example 30A and α,2,6-trichlorotoluene.
1H NMR (300 MHz, CDCl₃) δ ppm 1.34-1.63 (m, 5 H), 1.67-2.18 (m, 6 H), 2.84-3.10 (m, 2 H), 3.10-3.24 (m, 1 H), 3.25-3.39 (m, 1 H), 3.48 (dd, J=13.2, 4.3 Hz, 1 H), 3.53-3.68 (m, 1 H), 3.89-4.06 (m, 1 H), 7.01-7.17 (m, 1 H), 7.29 (d, J=7.9 Hz, 2 H).

### Example 371A 3-(2,6-dichlorobenzyl)-1-[4-(methylamino)cyclohexyl]pyrrolidin-2-one

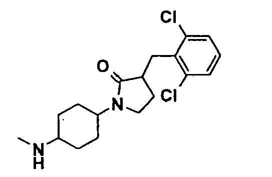

A mixture of 3-(2,6-dichlorobenzyl)-1-(4-oxocyclohexyl)pyrrolidin-2-one obtained in Example 28A, methylamine (40% methanol solution, 0.16 g), sodium triacetoxyborohydride (0.75 g), acetic acid (0.13 g) and dichloromethane (20 mL) was stirred at room temperature for 16 hr. The reaction solution was concentrated, and the residue was partitioned between ethyl acetate and 10% aqueous sodium bicarbonate. The ethyl acetate layer was dried over anhydrous magnesium sulfate. The solvent was evaporated under reduced pressure, and the residue was recrystallized from ethyl acetate to give the title compound (0.04 g, 6%).
LC-MS (ESI+); m/z 355 (M)⁺

### Example 372A 3-(2,6-dichlorobenzyl)-1-[4-(methylamino)cyclohexyl]pyrrolidin-2-one hydrochloride

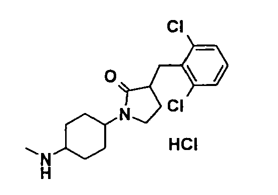

3-(2,6-Dichlorobenzyl)-1-[4-(methylamino)cyclohexyl]pyrrolidin-2-one obtained in Example 371A was converted to hydrochloride with 4N hydrogen chloride-ethyl acetate to give the title compound.
LC-MS (ESI+); m/z 355 (M)⁺

### Example 373A 3-(2-chlorobenzyl)-1-(1,4-dioxaspiro[4.5]dec-8-yl)pyrrolidin-2-one

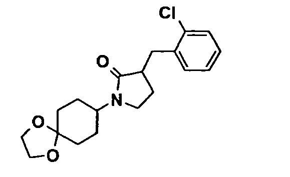

In the same manner as in Example 1A, the title compound was obtained from 1-(1,4-dioxaspiro[4.5]dec-8-yl)pyrrolidin-2-one and 2-chlorobenzyl bromide.
1H NMR (300 MHz, CDCl₃) δ ppm 1.61-1.87 (m, 9 H), 1.91-2.10 (m, 1 H), 2.75-2.89 (m, 2 H), 3.08-3.30 (m, 2 H), 3.39 (d like, 1 H), 3.87-3.98 (m, 4 H), 3.99-4.18 (m, 1 H), 7.08-7.23 (m, 2 H), 7.23-7.31 (m, 1 H), 7.31-7.39 (m, 1 H).

### Example 374A 3-(2-chlorobenzyl)-1-(4-oxocyclohexyl)pyrrolidin-2-one

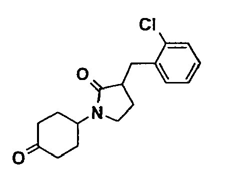

In the same manner as in Example 28A, the title compound was obtained from 3-(2-chlorobenzyl)-1-(1,4-dioxaspiro[4.5]dec-8-yl)pyrrolidin-2-one obtained in Example 373A.
1H NMR (300 MHz, CDCl₃) δ ppm 1.66-2.15 (m, 6 H), 2.37-2.61 (m, 4 H), 2.73-2.96 (m, 2 H), 3.13-3.27 (m, 2 H), 3.34-3.51 (m, 1 H), 4.38-4.59 (m, 1 H), 7.10-7.24 (m, 2 H), 7.26-7.31 (m, 1 H), 7.33-7.41 (m, 1 H).

### Example 375A 3-(2-chlorobenzyl)-1-(4-hydroxy-4-methylcyclohexyl)pyrrolidin-2-one (less polar product) and Example 376A 3-(2-chlorobenzyl)-1-(4-hydroxy-4-methylcyclohexyl)pyrrolidin-2-one (more polar product)

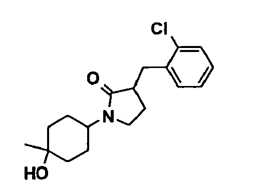

The reaction product (a mixture of four steric isomers) which was obtained in the same manner as in Example 30A from 3-(2-chlorobenzyl)-1-(4-oxocyclohexyl)pyrrolidin-2-one obtained in Example 374A was subjecting to silica gel column chromatography (hexane-ethyl acetate 2:3-ethyl acetate 100%) to give the title compound (Example 375A: less polar product) as a mixture of two steric isomers eluted earlier, and then to give the title compound (Example 376A: more polar product) as a mixture of two steric isomers eluted later.

### Example 375A (less polar product)

1H NMR (300 MHz, CDCl₃) δ ppm 1.03 (d, J=4.5 Hz, 1 H), 1.25 (s, 3 H), 1.41-1.91 (m, 9 H), 1.91-2.10 (m, 1 H), 2.69-2.92 (m, 2 H), 3.11-3.31 (m, 2 H), 3.32-3.50 (m, 1 H), 3.87-4.05 (m, 1 H), 7.09-7.23 (m, 2 H), 7.22-7.31 (m, 1 H), 7.31-7.41 (m, 1 H).

### Example 376A (more polar product)

1H NMR (300 MHz, CDCl₃)δ ppm 1.27 (s, 3 H), 1.40 (s, 1 H), 1.42-1.86 (m, 9 H), 1.90-2.12 (m, 1 H), 2.70-2.93 (m, 2 H), 3.10-3.29 (m, 2 H), 3.28-3.51 (m, 1 H), 3.83-4.13 (m, 1 H), 7.08-7.23 (m, 2 H), 7.23-7.31 (m, 1 H), 7.31-7.42 (m, 1 H).

### Example 377A 3-(2-chloro-4-methoxybenzyl)-1-(1,4-dioxaspiro[4.5]dec-8-yl)pyrrolidin-2-one

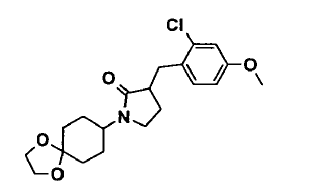

In the same manner as in Example 1A, the title compound was obtained from 1-(1,4-dioxaspiro[4.5]dec-8-yl)pyrrolidin-2-one and 2-chloro-4-methoxybenzyl bromide.
1H NMR (300 MHz, CDCl₃) δ ppm 1.59-1.87 (m, 9 H), 1.92-2.09 (m, 1 H), 2.63-2.88 (m, 2 H), 3.13-3.39 (m, 3 H), 3.78 (s, 3 H), 3.94 (s, 4 H), 4.05 (s, 1 H), 6.74 (dd, J=8.6, 2.5 Hz, 1 H), 6.90 (d, J=2.5 Hz, 1 H), 7.17 (d, J=8.6 Hz, 1 H).

### Example 378A 3-(2-chloro-4-methoxybenzyl)-1-(4-oxocyclohexyl)pyrrolidin-2-one

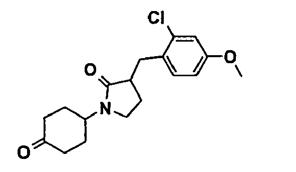

In the same manner as in Example 28A, the title compound was obtained from 3-(2-chloro-4-methoxybenzyl)-1-(1,4-dioxaspiro[4.5]dec-8-yl)pyrrolidin-2-oneobtained in Example 377A.
1H NMR (300 MHz, CDCl₃) δ ppm 1.65-2.15 (m, 6 H), 2.36-2.64 (m, 4 H), 2.67-2.91 (m, 2 H), 3.12-3.23 (m, 2 H), 3.26-3.38 (m, 1 H), 3.78 (s, 3 H), 4.38-4.60 (m, 1 H), 6.75 (dd, J=8.7, 2.6 Hz, 1 H), 6. 91 (d, J=2.6 Hz, 1 H), 7.17 (d, J=8. 7 Hz, 1 H).

### Example 379A 3-(2-chloro-4-methoxybenzyl)-1-(4-hydroxy-4-methylcyclohexyl)pyrrolidin-2-one

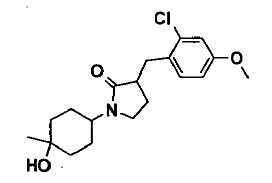

In the same manner as in Example 30A, the title compound was obtained from 3-(2-chloro-4-methoxybenzyl)-1-(4-oxocyclohexyl)pyrrolidin-2-one obtained in Example 378A.
LC-MS (ESI+); m/z 352 (M+H)⁺

### Example 380A 1-benzyl-3-(1-hydroxycyclohexyl)pyrrolidin-2-one

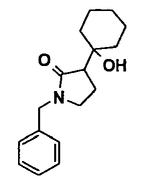

In the same manner as in Example 118A, the title compound was obtained from 1-benzylpyrrolidinone and cyclohexanone.
1H NMR (300 MHz, CDCl₃) δ ppm 0.97-1.22 (m, 1 H), 1.21-1.45 (m, 2 H), 1.43-1.93 (m, 8 H), 1.96-2.23 (m, 1 H), 2.62 (m, 1 H), 3.18 (m, 2 H), 4.26-4.66 (m, 3 H), 7.12-7.43 (m, 5 H).

### Example 381A 1-benzyl-3-(cyclohex-1-en-1-yl)pyrrolidin-2-one

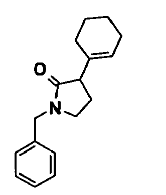

A mixture of 1-benzyl-3-(1-hydroxycyclohexyl)pyrrolidin-2-one obtained in Example 380A (5.0 g), p-toluenesulfonic acid monohydrate (0.70 g) and toluene (100 mL) was heated under reflux while dehydrating using Dean-Stark. The reaction mixture was concentrated under reduced pressure, and the residue was partitioned between ethyl acetate (50 mL) and 10% aqueous sodium bicarbonate (50 mL). The ethyl acetate layer was washed with saturated brine, and dried over anhydrous magnesium sulfate. The solvent was evaporated under reduced pressure, and the residue was subjected to silica gel column chromatography (hexane-ethyl acetate 7:3) to give the title compound (3.34 g, 71%).
1H NMR (300 MHz, CDCl₃) δ ppm 1.42-1.79 (m, 4H), 1.80-2.30 (m, 6 H), 3.09 (t, J=8.6 Hz, 1 H), 3.14-3.31 (m, 2 H), 4.36-4.45 (m, 1 H), 4.50-4.60 (m, 1 H), 5.61 (s, 1 H), 7.15-7.43 (m, 5 H).

### Example 382A 1-benzyl-3-cyclohexylpyrrolidin-2-one

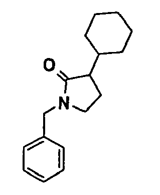

A mixture of 1-benzyl-3-(cyclohex-1-en-1-yl)pyrrolidin-2-one obtained in Example 381A (0.5 g), 10% palladium-carbon (0.1 g) and methanol (15 mL) was stirred at room temperature for 16 hr under hydrogen atmosphere. The reaction solution was filtrated through celite, and the filtrate was concentrated under reduced pressure. The residue was subjected to silica gel column chromatography (hexane-ethyl acetate 1:1) to give the title compound (0.44 g, 87%).
1H NMR (300 MHz, CDCl₃) δ ppm 0.96-1.43 (m, 5 H), 1.56 (s, 1 H), 1.62-2.19 (m, 7 H), 2.34-2.53 (m, 1 H), 3.14 (dd, J=7.8, 6.3 Hz, 2 H), 4.32-4.43 (m, 1 H), 4.47-4.56 (m, 1 H), 7.14-7.39 (m, 5 H).

### Example 383A 3-(1-hydroxycyclohexyl)-1-(4-methoxybenzyl)pyrrolidin-2-one

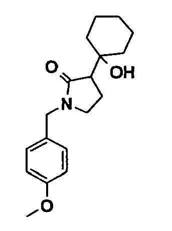

In the same manner as in Example 118A, the title compound was obtained from 1-(4-methoxybenzyl)pyrrolidin-2-one and cyclohexanone.
1H NMR (300 MHz, CDCl₃) δ ppm 1.02-1.19 (m, 1 H), 1.26-1.43 (m, 2 H), 1.51 (m, 3 H), 1.59-1.87 (m, 5 H), 1.97-2.13 (m, 1 H), 2.60 (m, 1 H), 3.16 (m, 2 H), 3.80 (s, 3 H), 4.26-4.36 (m, 1 H), 4.40-4.50 (m, 1 H), 4.52 (d, J=1.9 Hz, 1 H), 6.81-6.91 (m, 2 H), 7.10-7.19 (m, 2 H).

### Example 384A 3-(cyclohex-1-en-1-yl)-1-(4-methoxybenzyl)pyrrolidin-2-one

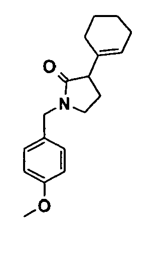

In the same manner as in Example 381A, the title compound was obtained from 3-(1-hydroxycyclohexyl)-1-(4-methoxybenzyl)pyrrolidin-2-one obtained in Example 383A.
1H NMR (300 MHz, CDCl₃) δ ppm 1.48-1.75 (m, 4 H), 1.79-2.25 (m, 6 H), 3.00-3.11 (m, 1 H), 3.10-3.28 (m, 2 H), 3.80 (s, 3 H), 4.29-4.37 (m, 1 H), 4.41-4.52 (m, 1 H), 5.60 (s, 1 H), 6.80-6.90 (m, 2 H), 7.12-7.21 (m, 2 H).

### Example 385A 3-cyclohexyl-1-(4-methoxybenzyl)pyrrolidin-2-one

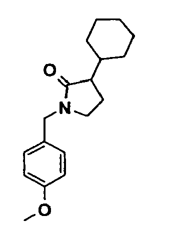

In the same manner as in Example 382A, the title compound was obtained from 3-(cyclohex-1-en-1-yl)-1-(4-methoxybenzyl)pyrrolidin-2-one obtained in Example 384A.
1H NMR (300 MHz, CDCl₃) δ ppm 0.97-1.41 (m, 5 H), 1.46-1.59 (m, 1 H), 1.62-2.05 (m, 7 H), 2.34-2.49 (m, 1 H), 3.12 (m, 2 H), 3.79 (s, 3 H), 4.25-4.36 (m, 1 H), 4.39-4.53 (m, 1 H), 6.80-6.89 (m, 2 H), 7.08-7.20 (m, 2 H).

### Example 386A 3-(2,6-dichlorobenzyl)-1-(4-methoxybenzyl)pyrrolidin-2-one

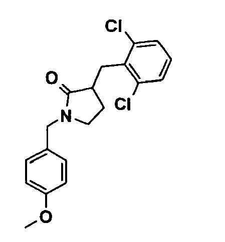

In the same manner as in Example 1A, the title compound was obtained from 1-(4-methoxybenzyl)pyrrolidin-2-one and α, 2, 6-trichlorotoluene.
1H NMR (300 MHz, CDCl₃) δ ppm 1.80-1.99 (m, 2 H), 2.86-3.33 (m, 4 H), 3.53 (dd, J=13.1, 4.2 Hz, 1 H), 3.80 (s, 3 H), 4.31-4.54 (m, 2 H), 6.79-6.94 (m, 2 H), 7.04-7.16 (m, 1 H), 7.15-7.23 (m, 2 H), 7.26-7.33 (m, 2 H).

### Example 387A 3-cyclohexyl-1-(2,6-dichlorobenzyl)pyrrolidin-2-one

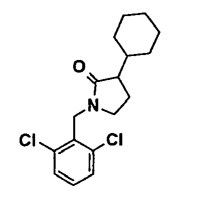

A mixture of 3-cyclohexyl-1-(4-methoxybenzyl)pyrrolidin-2-one obtained in Example 385A (3.80 g), diammonium cerium (IV) nitrate (21.7 g), acetonitrile (80 mL) and water (20 mL) was stirred for 2 hr under ice-cooling. The reaction mixture was partitioned between ethyl acetate (300 mL) and saturated brine (80 mL), and the ethyl acetate layer was dried over anhydrous magnesium sulfate. The solvent was evaporated under reduced pressure, and the residue was subjected to silica gel column chromatography (hexane-ethyl acetate 9:1-3:2) to give 3-cyclohexylpyrrolidin-2-one (1.28 g, 58%) as a colorless solid. In the same manner as in Example 56A, the title compound was obtained from this compound and α,2,6-trichlorotoluene.
1H NMR (300 MHz, CDCl₃) δ ppm 0.95-1.20 (m, 3 H), 1.19-1.42 (m, 2 H), 1.47-1.82 (m, 6 H), 1.81-2.00 (m, 2 H), 2.31-2.47 (m, 1 H), 2.95-3.13 (m, 2 H), 4.68 (d, J=13. 9 Hz, 1 H), 4.92 (d, J=13. 9 Hz, 1 H), 7.13-7.24 (m, 1 H), 7.29-7.38 (m, 2 H).

### Example 388A 3-(2,6-dichlorobenzyl)-1-(4-methoxycyclohexyl)pyrrolidin-2-one (less polar product) and Example 389A 3-(2,6-dichlorobenzyl)-1-(4-methoxycyclohexyl)pyrrolidin-2-one (more polar product)

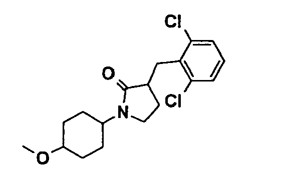

The reaction product (a mixture of four steric isomers) which was obtained in the same manner as in Example 1A from 1-(4-methoxycyclohexyl)pyrrolidin-2-one (a mixture of cis and trans) obtained in Reference Example 31A and α,2,6-trichlorotoluene was subjected to silica gel column chromatography (hexane-ethyl acetate 4:1-3:7) to give the title compound (Example 388A: less polar product) as a mixture of two steric isomers eluted earlier, and then to give the title compound (Example 389A: more polar product) as a mixture of two steric isomers eluted later.

### Example 388A (less polar product)

1H NMR (300 MHz, CDCl₃) δ ppm 1.38-1.61 (m, 4 H), 1.63-2.12 (m, 6 H), 2.83-3.10 (m, 2 H), 3.14-3.26 (m, 1 H), 3.31 (s, 3 H), 3.32-3.41 (m, 1 H), 3.41-3.54 (m, 2 H), 3.94-4.10 (m, 1 H), 7.03-7.14 (m, 1 H), 7.23-7.32 (m, 2 H).

### Example 389A (more polar product)

1H NMR (300 MHz, CDCl₃) δ ppm 1.26-1.59 (m, 4 H), 1.71-2.03 (m, 4 H), 2.04-2.21 (m, 2 H), 2.82-3.24 (m, 4 H), 3.30 (m, 1 H), 3.35 (s, 3 H), 3.48 (m, 1 H), 3.90-4.05 (m, 1 H), 7.05-7.14 (m, 1 H), 7.29 (m, 2 H).

### Example 390A benzyl 3-[3-(2,6-dichlorobenzyl)-2-oxopyrrolidin-1-yl]piperidine-l-carboxylate (less polar product) and Example 391A benzyl 3-[3-(2,6-dichlorobenzyl)-2-oxopyrrolidin-1-yl]piperidine-1-carboxylate (more polar product)

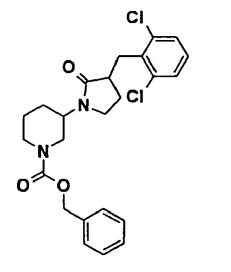

The reaction product (a mixture of four diastereomers) which was obtained in the same manner as in Example 1A from benzyl 3-(2-oxopyrrolidin-1-yl)piperidine-1-carboxylate obtained in Reference Example 32A and α,2,6-trichlorotoluene was subjected to silica gel column chromatography (hexane-ethyl acetate 9:1-1:1) to give the title compound (Example 390A: less polar product) as a mixture of two diastereomers, and then to give the title compound (Example 391A: more polar product) as a mixture of two diastereomers eluted later.
Example 390A (less polar product)
LC-MS (ESI+); m/z 461 (M)⁺

### Example 391A (more polar product)

LC-MS (ESI+); m/z 461 (M)⁺

### Example 392A 3-(2,6-dichlorobenzyl)-1-(piperidin-3-yl)pyrrolidin-2-one

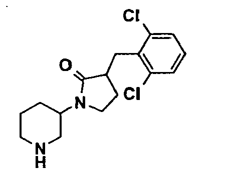

Benzyl 3-[3-(2,6-dichlorobenzyl)-2-oxopyrrolidin-1-yl]piperidine-1-carboxylate obtained in Examples 390A and 391A (a mixture of four diastereomers, 2.86 g) was dissolved in acetic acid (5 mL) and hydrobromic acid (48%, 20 mL), and the mixture was stirred at room temperature for 24 hr. The reaction mixture was poured into ice water (50 mL), and the organic layer was washed with diethyl ether (100 mL). The aqueous layer was adjusted with 8N sodium hydroxide to pH 10, and the mixture was extracted with ethyl acetate (100 mL). The ethyl acetate layer was washed with 10% aqueous sodium bicarbonate and saturated brine, and dried over anhydrous magnesium sulfate. The solvent was evaporated under reduced pressure, and the obtained colorless solid was recrystallized from hexane-ethyl acetate to give the title compound (1.00 g, 49%).
LC-MS (ESI+); m/z 327 (M)⁺

### Example 393A 3-(2, 6-dichlorobenzyl)-1-(1-methylpiperidin-3-yl)pyrrolidin-2-one hydrochloride

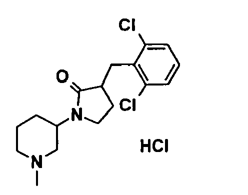

To a mixture of 3-(2,6-dichlorobenzyl)-1-(piperidin-3-yl)pyrrolidin-2-one obtained in Example 392A (0.15 g), formaldehyde (36%, 57 mg), acetic acid (55 mg) and dichloromethane (5 mL) was added sodium triacetoxyborohydride (0.19 g), and the mixture was stirred at room temperature for 3 hr. The reaction mixture was partitioned between ethyl acetate and 10% aqueous sodium bicarbonate, and the ethyl acetate layer was washed with saturated brine, and dried over anhydrous magnesium sulfate. The solvent was evaporated under reduced pressure, and the residue was purified by NH-silica gel column chromatography (ethyl acetate) to give a colorless amorphous form. This was treated with hydrogen chloride-ethyl acetate, and the obtained colorless solid was triturated with diethyl ether to give the title compound (0.13 g, 75%) as a colorless powder.
LC-MS (ESI+); m/z 341 (M)⁺

### Example 394A 1-(1-benzylpiperidin-3-yl)-3-(2,6-dichlorobenzyl)pyrrolidin-2-one hydrochloride

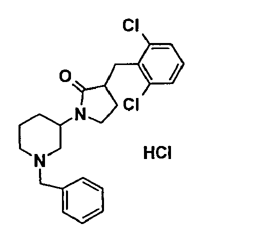

In the same manner as in Example 94A, the title compound was obtained from 3-(2,6-dichlorobenzyl)-1-(piperidin-3-yl)pyrrolidin-2-one obtained in Example 392A and benzyl bromide. LC-MS (ESI+); m/z 417 (M)⁺

### Example 395A 1-(1-acetylpiperidin-3-yl)-3-(2,6-dichlorobenzyl)pyrrolidin-2-one

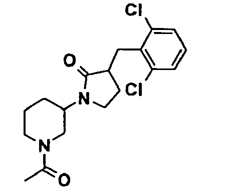

In the same manner as in Example 22A, the title compound was obtained from 3-(2,6-dichlorobenzyl)-1-(piperidin-3-yl)pyrrolidin-2-one obtained in Example 392A and acetyl chloride.
LC-MS (ESI+); m/z 369 (M)⁺

### Example 396A 3-(2,6-dichlorobenzyl)-1-[1-(methylsulfonyl)piperidin-3-yl]pyrrolidin-2-one

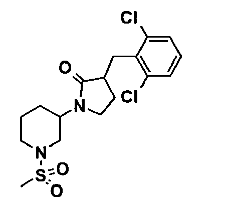

In the same manner as in Example 22A, the title compound was obtained from 3-(2,6-dichlorobenzyl)-1-(piperidin-3-yl)pyrrolidin-2-one obtained in Example 392A and methanesulfonyl chloride.
LC-MS (ESI+); m/z 405 (M)⁺

### Example 397A 3-[2-chloro-4-(trifluoromethyl)benzyl]-1-(1,4-dioxaspiro[4.5]dec-8-yl)pyrrolidin-2-one

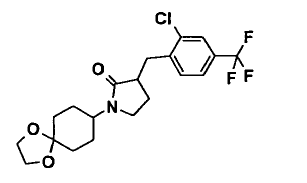

In the same manner as in Example 1A, the title compound was obtained from 1-(1,4-dioxaspiro[4.5]dec-8-yl)pyrrolidin-2-one and 2-chloro-4-(trifluoromethyl)benzyl chloride.
1H NMR (300 MHz, CDCl₃) δ ppm 1.60-1.87 (m, 9 H), 1.94-2.19 (m, 1 H), 2.77-2.94 (m, 2 H), 3.13-3.31 (m, 2 H), 3.41 (d, J=9.0 Hz, 1 H), 3.92-3.98 (m, 4 H), 4.05 (s, 1 H), 7.44 (d, J=1.7 Hz, 2 H), 7.62 (s, 1 H).

### Example 398A 3-(2,4-dichlorobenzyl)-1-(1,4-dioxaspiro[4.5]dec-8-yl)pyrrolidin-2-one

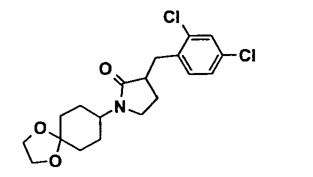

In the same manner as in Example 1A, the title compound was obtained from 1-(1,4-dioxaspiro[4.5]dec-8-yl)pyrrolidin-2-one and 2,4-dichlorobenzyl chloride.
1H NMR (300 MHz, CDCl₃) δ ppm 1.59-1.88 (m, 9 H), 1.94-2.13 (m, 1 H), 2.66-2.91 (m, 2 H), 3.10-3.44 (m, 3 H), 3.94 (s, 4 H), 4.04 (s, 1 H), 7.07-7.25 (m, 2 H), 7.37 (d, J=2.1 Hz, 1 H).

### Example 399A 3-[2-chloro-4-(trifluoromethyl)benzyl]-1-(4-oxocyclohexyl)pyrrolidin-2-one

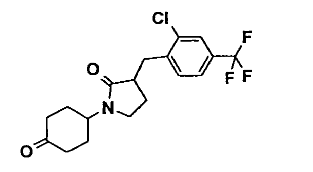

In the same manner as in Example 28A, the title compound was obtained from 3-[2-chloro-4-(trifluoromethyl)benzyl]-1-(1,4-dioxaspiro[4.5]dec-8-yl)pyrrolidin-2-one obtained in Example 397A.
1H NMR (300 MHz, CDCl₃) δ ppm 1.63-2.20 (m, 6 H), 2.34-2.64 (m, 4 H), 2.77-2.97 (m, 2 H), 3.15-3.33 (m, 2 H), 3.35-3.53 (m, 1 H), 4.38-4.59 (m, 1 H), 7.37-7.52 (m, 2 H), 7.64 (s, 1 H).

### Example 400A 3-(2,4-dichlorobenzyl)-1-(4-oxocyclohexyl)pyrrolidin-2-one

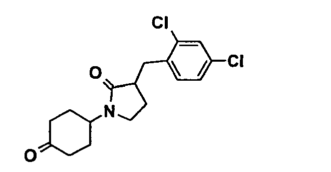

In the same manner as in Example 28A, the title compound was obtained from 3-(2,4-dichlorobenzyl)-1-(1,4-dioxaspiro[4.5]dec-8-yl)pyrrolidin-2-one obtained in Example 398A.
1H NMR (300 MHz, CDCl₃) δ ppm 1.63-2.18 (m, 6 H), 2.33-2.64 (m, 4 H), 2.71-2.92 (m, 2 H), 3.12-3.28 (m, 2 H), 3.27-3.42 (m, 1 H), 4.37-4.58 (m, 1 H), 7.12-7.25 (m, 2 H), 7.38 (d, J=2.1 Hz, 1 H).

### Example 401A 3-[2-chloro-4-(trifluoromethyl)benzyl]-1-(4-hydroxy-4-methylcyclohexyl)pyrrolidin-2-one

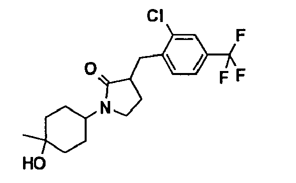

In the same manner as in Example 30A, the title compound was obtained from 3-[2-chloro-4-(trifluoromethyl)benzyl]-1-(4-oxocyclohexyl)pyrrolidin-2-one obtained in Example 399A.
LC-MS (ESI+); m/z 390 (M)⁺

### Example 402A 3-(2,4-dichlorobenzyl)-1-(4-hydroxy-4-methylcyclohexyl)pyrrolidin-2-one

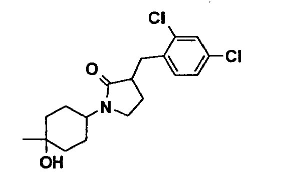

In the same manner as in Example 263A, the title compound was obtained from 3-(2,4-dichlorobenzyl)-1-(4-oxocyclohexyl)pyrrolidin-2-one obtained in Example 400A.
LC-MS (ESI+); m/z 356 (M)⁺

### Example 403A tert-butyl (3-chloro-4-{[1-(1,4-dioxaspiro[4.5]dec-8-yl)-2-oxopyrrolidin-3-yl]methyl}phenyl)(methyl)carbamate

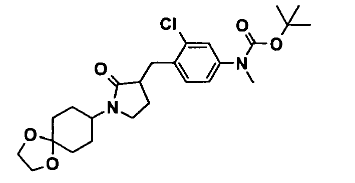

In the same manner as in Example 1A, the title compound was obtained from 1-(1,4-dioxaspiro[4.5]dec-8-yl)pyrrolidin-2-one and tert-butyl [3-chloro-4-(chloromethyl)phenyl](methyl)carbamate obtained in Reference Example 29A.
1H NMR (300 MHz, CDCl₃) δ ppm 1.46 (s, 9 H), 1.61-1.87 (m, 9 H), 1.93-2.10 (m, 1 H), 2.68-2.86 (m, 2 H), 3.10-3.30 (m, 2 H), 3.23 (s, 3H), 3.34 (d, J=9.6 Hz, 1 H), 3.94 (s, 4 H), 4.06 (s, 1 H), 7.02-7.12 (m, 1 H), 7.22 (d, J=8.3 Hz, 1 H), 7.24-7.30 (m, 1 H). Example 404A methyl N-(3-chloro-4-{[2-oxo-1-(4-oxocyclohexyl)pyrrolidin-3-yl]methyl}phenyl)-N-methyl glycinate

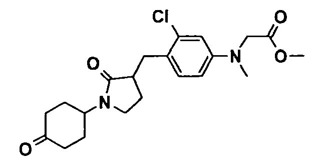

In the same manner as in Example 28A, a colorless amorphous form was obtained from tert-butyl (3-chloro-4-{[1-(1,4-dioxaspiro[4.5]dec-8-yl)-2-oxopyrrolidin-3-yl]methyl}phenyl)(methyl)carbamate obtained in Example 403A (1.29 g). In the same manner as in Example 20A, a colorless solid was obtained from this compound. This was triturated with diethyl ether to give a colorless powder (0.90 g). In the same manner as in Example 94A, the title compound was obtained from the obtained product and methyl bromoacetate.
1H NMR (300 MHz, CDCl₃) δ ppm 1.59-2.14 (m, 6 H), 2.33-2.61 (m, 4 H), 2.62-2.88 (m, 2 H), 3.03 (s, 3 H), 3.10-3.39 (m, 3 H), 3.73 (s, 3 H), 4.04 (s, 2 H), 4.36-4.60 (m, 1 H), 6.50 (dd, J=8.7, 2.6 Hz, 1 H), 6.67 (d, J=2.6 Hz, 1 H), 7.09 (d, J=8.7 Hz, 1 H).

### Example 405A 3-{2-chloro-4-[(2-hydroxy-2-methylpropyl)(methyl)amino]benzyl}-1-(4-hydroxy-4-methylcyclohexyl)pyrrolidin-2-one

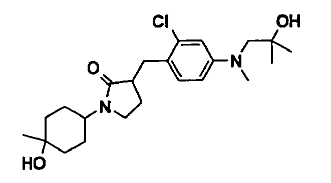

In the same manner as in Example 263A, the title compound was obtained from methyl N-(3-chloro-4-{[2-oxo-1-(4-oxocyclohexyl)pyrrolidin-3-yl]methyl}phenyl)-N-methyl glycinate obtained in Example 404A.
LC-MS (ESI+); m/z 423 (M)⁺

### Example 406A 3-cyclohexyl-1-(2,4-dichlorobenzyl)pyrrolidin-2-one

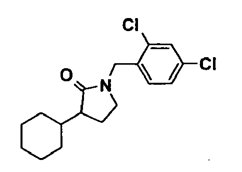

In the same manner as in Example 387A, the title compound was obtained from 3-cyclohexyl-1-(4-methoxybenzyl)pyrrolidin-2-one obtained in Example 385A and 2,4-dichlorobenzyl chloride.
1H NMR (300 MHz, CDCl₃) δ ppm 0.95-1.22 (m, 3 H), 1.20-1.43 (m, 2 H), 1.42-1.59 (m, 1 H), 1.60-2.13 (m, 6 H), 2.34-2.53 (m, 1 H), 3.11-3.26 (m, 2 H), 4.41-4.54 (m, 1 H), 4.55-4.69 (m, 2 H), 7.16-7.25 (m, 2 H), 7.38 (s, 1 H).

### Example 407A 3-(2,6-dichlorobenzyl)-1-[4-(hydroxymethyl)cyclohexyl]pyrrolidin-2-one

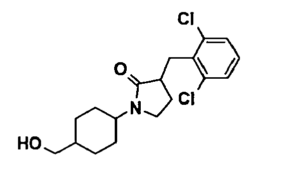

In the same manner as in Example 255A, the title compound was obtained from 1-[4-(hydroxymethyl)cyclohexyl]pyrrolidin-2-one obtained in Reference Example 34A and α,2,6-trichlorotoluene.
LC-MS (ESI+); m/z 356 (M)⁺

### Example 408A 1-cyclohexyl-3-(2-methoxybenzyl)pyrrolidin-2-one (an optically active form, retention time 9 min) and Example 409A 1-cyclohexyl-3-(2-methoxybenzyl)pyrrolidin-2-one (an optically active form, retention time 12 min)

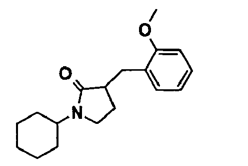

1-Cyclohexyl-3-(2-methoxybenzyl)pyrrolidin-2-one (racemate) obtained in Example 111A was subjected to optical resolution with normal phase chiral high performance liquid chromatography (manufactured by Daicel Chemical Industries, Ltd., chiralpak AD (trade name), 50 mmID X 500 mmL, hexane-ethanol 90:10) to give the title compound (Example 408A) from a fraction with a retention time of 9 min and the title compound (Example 409A) from a fraction with a retention time of 12 min. Example 408A (retention time 9 min)
1H NMR (300 MHz, CDCl₃) δ ppm 0.97-1.17 (m, 1 H), 1.27-1.49 (m, 4 H), 1.58-1.84 (m, 6 H), 1.86-2.00 (m, 1 H), 2.57 (dd, J=13.6, 10.1 Hz, 1 H), 2.70-2.86 (m, 1 H), 3.11-3.21 (m, 2 H), 3.31 (dd, J=13.6, 4.1 Hz, 1 H), 3.81 (s, 3 H), 3.87-4.04 (m, 1 H), 6.77-6.93 (m, 2 H), 7.11-7.23 (m, 2 H). Example 409A (retention time 12 min)
1H NMR (300 MHz, CDCl₃) δ ppm 0.97-1.17 (m, 1 H), 1.27-1.49 (m, 4 H), 1.58-1.84 (m, 6 H), 1.86-2.00 (m, 1 H), 2.57 (dd, J=13.6, 10.1 Hz, 1 H), 2.70-2.86 (m, 1 H), 3.11-3.21 (m, 2 H), 3.31 (dd, J=13.6, 4.1 Hz, 1 H), 3.81 (s, 3 H), 3.87-4.04 (m, 1 H), 6.77-6.93 (m, 2 H), 7.11-7.23 (m, 2 H).

### Example 410A 3-(2-chloro-4-methoxybenzyl)-1-(4-hydroxy-4-methylcyclohexyl)pyrrolidin-2-one (an optically active form, retention time 22 min)

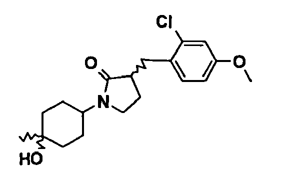

3-(2-Chloro-4-methoxybenzyl)-1-(4-hydroxy-4-methylcyclohexyl)pyrrolidin-2-one (a mixture of four steric isomers) obtained in Example 379A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15, retention time 22 min), and purified (manufactured by Daicel Chemical Industries, Ltd., chiralpak AD (trade name), 50 mmID X 500 mmL, hexane-ethanol 85:15, retention time 150 min) to give the title compound.
1H NMR (300 MHz, CDCl₃) δ ppm 1.27 (s, 3 H), 1.39-1.84 (m, 10 H), 1.90-2.11 (m, 1 H), 2.64-2.85 (m, 2 H), 3.13-3.24 (m, 2 H), 3.24-3.35 (m, 1 H), 3.78 (s, 3 H), 3.89-4.06 (m, 1 H), 6.75 (dd, J=8.5, 2.6 Hz, 1 H), 6.91 (d, J=2.6 Hz, 1 H), 7.17 (d, J=8.5 Hz, 1 H) .

### Example 411A 3-(2-chloro-4-methoxybenzyl)-1-(4-hydroxy-4-methylcyclohexyl)pyrrolidin-2-one (an optically active form, retention time 35 min)

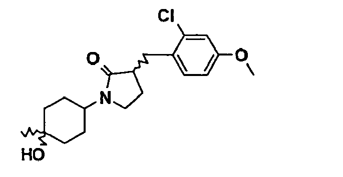

3-(2-Chloro-4-methoxybenzyl)-1-(4-hydroxy-4-methylcyclohexyl)pyrrolidin-2-one (a mixture of four steric isomers) obtained in Example 379A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15, retention time 35 min) to give the title compound.
1H NMR (300 MHz, CDCl₃)δ ppm 1.27 (s, 3 H), 1.39-1.86 (m, 10 H), 1.90-2.12 (m, 1 H), 2.62-2.86 (m, 2 H), 3.13-3.24 (m, 2 H), 3.24-3.36 (m, 1 H), 3.78 (s, 3 H), 3.91-4.07 (m, 1 H), 6.75 (dd, J=8.5, 2.6 Hz, 1 H), 6.91 (d, J=2.6 Hz, 1 H), 7.17 (d, J=8.5 Hz, 1 H).

### Example 412A 3-(2,4-dichlorobenzyl)-1-(4-hydroxy-4-methylcyclohexyl)pyrrolidin-2-one (an optically active form, retention time 18 min, main product)

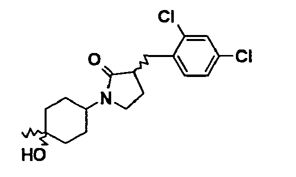

3-(2,4-Dichlorobenzyl)-1-(4-hydroxy-4-methylcyclohexyl)pyrrolidin-2-one (a mixture of four steric isomers) obtained in Example 402A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15, retention time 18 min), and purified by normal phase chiral high performance liquid chromatography (SUMICHIRAL OA (trade name) manufactured by Sumika Chemical Analysis Service, Ltd., 20 mmID X 250 mmL, hexane-ethanol 85:15, retention time 4.16 min) to give the title compound.
1H NMR (300 MHz, CDCl₃) δ ppm 1.27 (s, 3 H), 1.38-1.86 (m, 10 H), 1.94-2.12 (m, 1 H), 2.69-2.89 (m, 2 H), 3.11-3.41 (m, 3 H), 3.88-4.06 (m, 1 H), 7.11-7.25 (m, 2 H), 7.37 (d, J=2.1 Hz, 1 H).

### Example 413A 3-(2,4-dichlorobenzyl)-1-(4-hydroxy-4-methylcyclohexyl)pyrrolidin-2-one (an optically active form, retention time 27 min, main product)

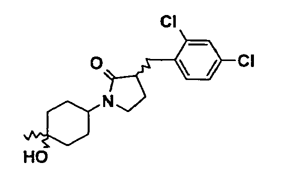

3-(2,4-Dichlorobenzyl)-1-(4-hydroxy-4-methylcyclohexyl)pyrrolidin-2-one (a mixture of four steric isomers) obtained in Example 402A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15, retention time 27 min) to give the title compound.
1H NMR (300 MHz, CDCl₃) δ ppm 1.27 (s, 3 H), 1.41-1.85 (m, 10 H), 1.95-2.10 (m, 1 H), 2.71-2.87 (m, 2 H), 3.13-3.26 (m, 2 H), 3.26-3.41 (m, 1 H), 3.87-4.06 (m, 1 H), 7.12-7.25 (m, 2 H), 7.37 (d, J=1.9 Hz, 1 H).

### Example 414A 3-(2,4-dichlorobenzyl)-1-(4-hydroxy-4-methylcyclohexyl)pyrrolidin-2-one (an optically active form, retention time 18 min, by-product)

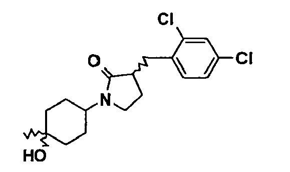

3-(2,4-Dichlorobenzyl)-1-(4-hydroxy-4-methylcyclohexyl)pyrrolidin-2-one (a mixture of four steric isomers) obtained in Example 402A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15, retention time 18 min), and purified by normal phase chiral high performance liquid chromatography (SUMICHIRAL OA (trade name) manufactured by Sumika Chemical Analysis Service, Ltd., 20 mmID X 250 mmL, hexane-ethanol 85:15, retention time 4.09 min) to give the title compound.
1H NMR (300 MHz, CDCl₃) δ ppm 1.01 (s, 1 H), 1.25 (s, 3 H), 1.41-1.90 (m, 9 H), 1.92-2.10 (m, 1 H), 2.68-2.86 (m, 2 H), 3.14-3.40 (m, 3 H), 3.82-4.04 (m, 1 H), 7.12-7.25 (m, 2 H), 7.37 (d, J=1.9 Hz, 1 H).

### Example 415A 3-(2,4-dichlorobenzyl)-1-(4-hydroxy-4-methylcyclohexyl)pyrrolidin-2-one (an optically active form, retention time 23 min, by-product)

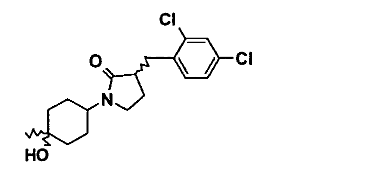

3-(2,4-Dichlorobenzyl)-1-(4-hydroxy-4-methylcyclohexyl)pyrrolidin-2-one (a mixture of four steric isomers) obtained in Example 402A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15, retention time 23 min) to give the title compound.
1H NMR (300 MHz, CDCl₃) δ ppm 1.01 (s, 1 H), 1.25 (s, 3 H), 1.37-1.92 (m, 9 H), 1.93-2.15 (m, 1 H), 2.68-2.88 (m, 2 H), 3.14-3.41 (m, 3 H), 3.83-4.07 (m, 1 H), 7.12-7.25 (m, 2 H), 7.37 (d, J=1.9 Hz, 1 H).

### Example 416A 3-(2-chlorobenzyl)-1-(4-hydroxy-4-methylcyclohexyl)pyrrolidin-2-one (an optically active form, retention time 24 min) and Example 417A 3-(2-chlorobenzyl)-1-(4-hydroxy-4-methylcyclohexyl)pyrrolidin-2-one (an optically active form, retention time 66 min)

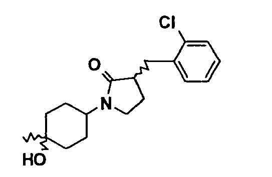

3-(2-Chlorobenzyl)-1-(4-hydroxy-4-methylcyclohexyl)pyrrolidin-2-one (less polar product) obtained in Example 375A was subjected to optical resolution with normal phase chiral high performance liquid chromatography (manufactured by Daicel Chemical Industries, Ltd., chiralpak AD (trade name), 50 mmID X 500 mmL, hexane-ethanol 1:1) to give the title compound (Example 416A) from a fraction with a retention time of 24 min and the title compound (Example 417A) from a fraction with a retention time of 66 min.

### Example 416A (retention time 24 min)

1H NMR (300 MHz, CDCl₃) δ ppm 1.05 (s, 1 H), 1.25 (s, 3 H), 1.41-1.91 (m, 9 H), 1.92-2.13 (m, 1 H), 2.68-2.96 (m, 2 H), 3.12-3.32 (m, 2 H), 3.34-3.47 (m, 1 H), 3.86-4.07 (m, 1 H), 7.06-7.23 (m, 2 H), 7.23-7.30 (m, 1 H), 7.35 (dd, J=7.2, 1.9 Hz, 1 H). Example 417A (retention time 66 min)
1H NMR (300 MHz, CDCl₃)δ ppm 1.04 (s, 1 H), 1.25 (s, 3 H), 1.39-1.90 (m, 9 H), 1.91-2.10 (m, 1 H), 2.69-2.95 (m, 2 H), 3.14-3.32 (m, 2 H), 3.35-3.46 (m, 1 H), 3.82-4.09 (m, 1 H), 7.10-7.24 (m, 2 H), 7.23-7.31 (m, 1 H), 7.35 (dd, J=7.3, 2.0 Hz, 1 H).

### Example 418A 3-(2-chlorobenzyl)-1-(4-hydroxy-4-methylcyclohexyl)pyrrolidin-2-one (an optically active form, retention time 90 min) and Example 419A 3-(2-chlorobenzyl)-1-(4-hydroxy-4-methylcyclohexyl)pyrrolidin-2-one (an optically active form, retention time 114 min)

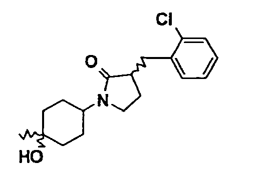

3-(2-Chlorobenzyl)-1-(4-hydroxy-4-methylcyclohexyl)pyrrolidin-2-one (more polar product) obtained in Example 376A was subjected to optical resolution with normal phase chiral high performance liquid chromatography (manufactured by Daicel Chemical Industries, Ltd., chiralpak AD (trade name), 50 mmID X 500 mmL, hexane-isopropanol 9:1) to give the title compound (Example 418A) from a fraction with a retention time of 90 min and the title compound (Example 419A) from a fraction with a retention time of 114 min.

### Example 418A (retention time 90 min)

1H NMR (300 MHz, CDCl₃)δ ppm 1.26-1.30 (m, 3 H), 1.41-1.86 (m, 10 H), 1.92-2.12 (m, 1 H), 2.70-2.91 (m, 2 H), 3.09-3.27 (m, 2 H), 3.31-3.49 (m, 1 H), 3.90-4.09 (m, 1 H), 7.08-7.23 (m, 2 H), 7.23-7.31 (m, 1 H), 7.30-7.40 (m, 1 H).

### Example 419A (retention time 114 min)

1H NMR (300 MHz, CDCl₃)δppm 1.27 (s, 3 H), 1.42-1.85 (m, 10 H), 1.91-2.10 (m, 1 H), 2.67-2.93 (m, 2 H), 3.14-3.27 (m, 2 H), 3.32-3.49 (m, 1 H), 3.90-4.09 (m, 1 H), 7.09-7.23 (m, 2 H), 7.23-7.31 (m, 1 H), 7.35 (dd, J=7.0, 2.1 Hz, 1 H).

### Example 420A 3-(2-chloro-4-fluorobenzyl)-1-(4-hydroxy-4-methylcyclohexyl)pyrrolidin-2-one (an optically active form, retention time 14 min) and Example 421A 3-(2-chloro-4-fluorobenzyl)-1-(4-hydroxy-4-methylcyclohexyl)pyrrolidin-2-one (an optically active form, retention time 24 min)

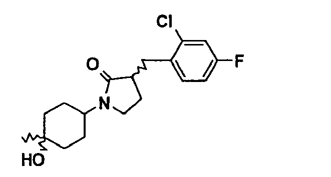

3-(2-Chloro-4-fluorobenzyl)-1-(4-hydroxy-4-methylcyclohexyl)pyrrolidin-2-one (less polar product) obtained in Example 86A was subjected to optical resolution with normal phase chiral high performance liquid chromatography (manufactured by Daicel Chemical Industries, Ltd., chiralpak AD (trade name), 50 mmID X 500 mmL, hexane-2-propanol 4:1) to give the title compound (Example 420A) from a fraction with a retention time of 14 min and the title compound (Example 421A) from a fraction with a retention time of 24 min.

### Example 420A (retention time 14 min)

1H NMR (300 MHz, CDCl₃) δ ppm 1.25 (s, 3 H), 1.40-1.89 (m, 9 H), 1.94-2.11 (m, 1 H), 2.68-2.87 (m, 2 H), 3.13-3.43 (m, 3 H), 3.86-4.03 (m, 1 H), 6.83-6.99 (m, 1 H), 7.10 (dd, J=8.5, 2.6 Hz, 1 H), 7.18-7.32 (m, 1 H).

### Example 421A (retention time 24 min)

1H NMR (300 MHz, CDCl₃) δ ppm 1.25 (s, 3 H), 1.40-1.91 (m, 9 H), 1.93-2.17 (m, 1 H), 2.67-2.88 (m, 2 H), 3.11-3.44 (m, 3 H), 3.83-4.03 (m, 1 H), 6.83-6.98 (m, 1 H), 7.10 (dd, J=8.7, 2.6 Hz, 1H), 7.21-7.29 (m, 1H).

Example 422A 3-(2-chloro-4-fluorobenzyl)-1-(4-hydroxy-4-methylcyclohexyl)pyrrolidin-2-one (an optically active form, retention time 18 min) Example 423A 3-(2-chloro-4-fluorobenzyl)-1-(4-hydroxy-4-methylcyclohexyl)pyrrolidin-2-one (an optically active form, retention time 26 min)

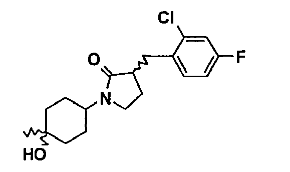

3-(2-Chloro-4-fluorobenzyl)-1-(4-hydroxy-4-methylcyclohexyl)pyrrolidin-2-one (more polar product) obtained in Example 87A was subjected to optical resolution with normal phase chiral high performance liquid chromatography (manufactured by Daicel Chemical Industries, Ltd., chiralpak AD (trade name), 50 mmID X 500 mmL, hexane-2-propanol 4:1) to give the title compound (Example 422A) from a fraction with a retention time of 18 min and the title compound (Example 423A) from a fraction with a retention time of 26 min.

### Example 422A (retention time 18 min)

1H NMR (300 MHz, CDCl₃) δ ppm 1.25 (s, 3 H), 1.36-1.91 (m, 9 H), 1.93-2.11 (m, 1 H), 2.68-2.89 (m, 2 H), 3.10-3.42 (m, 3 H), 3.82-4.08 (m, 1H), 6.84-6.99 (m, 1 H), 7.10 (dd, J=8.5, 2.6 Hz, 1 H), 7.19-7.35 (m, 1 H).

### Example 423A (retention time 26 min)

1H NMR (300 MHz, CDCl₃) δ ppm 1.27 (s, 3 H), 1.41-1.87 (m, 9 H), 1.92-2.16 (m, 1 H), 2.65-2.91 (m, 2 H), 3.14-3.25 (m, 2 H), 3.25-3.43 (m, 1 H), 3.82-4.08 (m, 1 H), 6.81-7.01 (m, 1H), 7.11 (dd, J=8.5, 2.6 Hz, 1 H), 7.17-7.34 (m, 1 H).

### Example 424A 3-(2,6-dichlorobenzyl)-1-(5-methoxy-1,2,3,4-tetrahydronaphthalen-1-yl)pyrrolidin-2-one

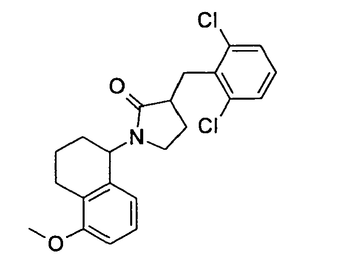

In the same manner as in Example 1A, the title compound was obtained from 1-(5-methoxy-1,2,3,4-tetrahydronaphthalen-1-yl)pyrrolidin-2-one obtained in Reference Example 37A and α,2,6-trichlorotoluene.
1H NMR (300 MHz, CDCl₃) δ ppm 1.66-2.09 (m, 6 H), 2.45-2.62 (m, 1 H), 2.72-2.86 (m, 1 H), 2.88-3.14 (m, 3 H), 3.14-3.28 (m, 1 H), 3.46-3.69 (m, 1 H), 3.77-3.87 (s, 3 H), 5.33-5.51 (m, 1 H), 6.57-6.77 (m, 2 H), 7.05-7.20 (m, 2 H), 7.25-7.33 (m, 2 H).

### Example 425A 3-(2,6-dichlorobenzyl)-1-(5-hydroxy-1,2,3,4-tetrahydronaphthalen-1-yl)pyrrolidin-2-one

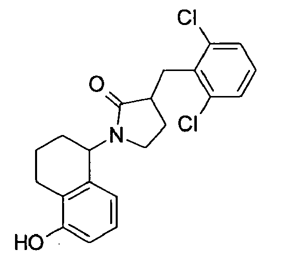

In the same manner as in Example 140A, the title compound was obtained from 3-(2,6-dichlorobenzyl)-1-(5-methoxy-1,2,3,4-tetrahydronaphthalen-1-yl)pyrrolidin-2-one obtained in Example 424A.
1H NMR (300 MHz, CDCl₃) δ ppm 1.67-2.12 (m, 6 H), 2.33-2.85 (m, 2 H), 2.86-3.41 (m, 4 H), 3.42-3.68 (m, 1 H), 5.33-5.56 (m, 1 H), 5.92-5.56 (m, 1 H), 5.92-6.23 (m, 1 H), 6.44-7.17 (m, 4 H), 7.21-7.38 (m, 2 H).

### Example 426A 3-(2,4-dichlorobenzyl)-1-(5-methoxy-1,2,3,4-tetrahydronaphthalen-1-yl)pyrrolidin-2-one

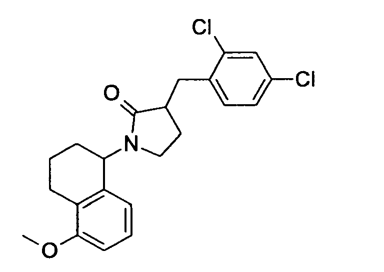

In the same manner as in Example 1A, the title compound was obtained from 1-(5-methoxy-1,2,3,4-tetrahydronaphthalen-1-yl)pyrrolidin-2-one obtained in Reference Example 37A and α,2,4-trichlorotoluene.
1H NMR (300 MHz, CDCl₃) δ ppm 1.67-1.82 (m, 2 H), 1.91-2.04 (m, 2 H), 2.52-2.55 (m, 1 H), 2.75-2.98 (m, 4 H), 3.03-3.11 (m, 1 H), 3.35-3.41 (dd, J=12.9, 3.9 Hz, 1 H), 3.81 (s, 3 H), 5.39-5.44 (m, 1 H), 6.58-6.60 (d, J=7.5 Hz, 1 H), 6.69-6.71 (d, J=8.4 Hz, 1 H), 7.08-7.13 (m, 1 H), 7.17-7.25 (m, 1 H), 7.26-7.31 (m, 1 H), 7.37-7.39 (m, 1 H).

### Example 427A 3-(2,4-dichlorobenzyl)-1-(5-hydroxy-1,2,3,4-tetrahydronaphthalen-1-yl)pyrrolidin-2-one

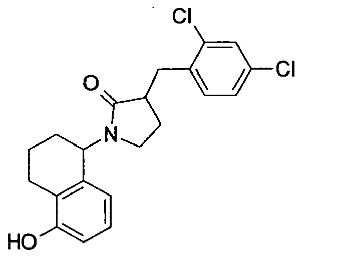

In the same manner as in Example 140A, the title compound was obtained from 3-(2,4-dichlorobenzyl)-1-(5-methoxy-1,2,3,4-tetrahydronaphthalen-1-yl)pyrrolidin-2-one obtained in Example 426A.
LC/MS (ESI+); m/z 390 (M+H)⁺

### Example 428A 3-(2,6-dichlorobenzyl)-1-(5-hydroxy-2-adamantyl)pyrrolidin-2-one (more polar product)

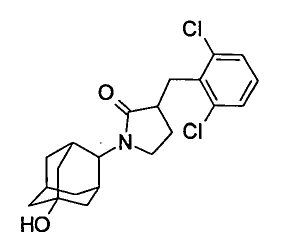

In the same manner as in Example 255A, the title compound was obtained from 1-(5-hydroxy-2-adamantyl)pyrrolidin-2-one obtained in Reference Example 38A and α,2,6-trichlorotoluene.
1H NMR (300 MHz, CD₃OD) δ ppm 1.61-2.03 (m, 12 H), 2.11 (m, 1 H), 2.45-2.52 (m, 2 H), 2.91-3.09 (m, 2 H), 3.37-3.43 (m, 1 H), 3.54-3.62 (m, 1 H), 3.72-3.81 (m, 1 H), 4.87 (m, 1 H), 7.17-7.23 (m, 1 H), 7.33-7.38 (m, 2 H).

### Example 429A 3-(2,6-dichlorobenzyl)-1-(5-hydroxy-2-adamantyl)pyrrolidin-2-one (less polar product)

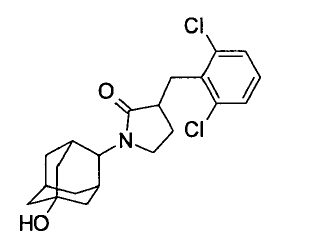

In the same manner as in Example 255A, the title compound was obtained from 1-(5-hydroxy-2-adamantyl)pyrrolidin-2-one obtained in Reference Example 38A and α,2,6-trichlorotoluene.
1H NMR (300 MHz, CDCl₃) δ ppm 1.57 (m, 2H), 1.78-2.04 (m, 10 H), 2.19-2.30 (m, 2 H), 2.46-2.50 (m, 2 H), 2.89-2.95 (m, 1 H), 2.99-3.07 (t, J=11.1 Hz, 1 H), 3.44-3.50 (m, 2 H), 3.62-3.69 (m, 1 H), 3.95 (m, 1 H), 7.07-7.12 (m, 1 H), 7.23-7.31 (m, 2 H).

### Example 430A 3-[2-chloro-4-(methoxymethoxy)benzyl]-1-(5-hydroxy-2-adamantyl)pyrrolidin-2-one

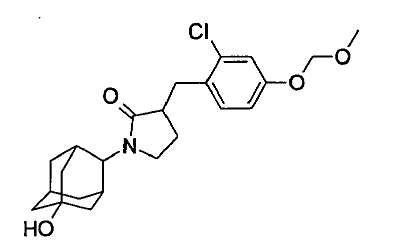

In the same manner as in Example 255A, the title compound was obtained from 1-(5-hydroxy-2-adamantyl)pyrrolidin-2-one obtained in Reference Example 38A and 2-chloro-4-(methoxymethoxy)benzyl bromide obtained in Reference Example 24A.
1H NMR (300 MHz, CDCl₃) δ ppm 1.50-1.54 (m, 2 H), 1.70-1.79 (m, 9 H), 1.90-1.94 (m, 2 H), 2.18 (m, 1H), 2.43-2.47 (m, 2 H), 2.72-2.78 (m, 2 H), 3.28-3.31 (m, 1 H), 3.42-3.53 (m, 2 H), 3.47 (s, 3 H), 3.92 (s, 1 H), 5.14 (s, 2 H), 6.86-6.89 (dd, J=8.4, 2.4 Hz, 1 H), 7.07-7.08 (d, J=2.4 Hz, 1 H), 7.16-7.19 (d, J=8.4 Hz, 1 H).

### Example 431A 3-(2-chloro-4-hydroxybenzyl)-1-(5-hydroxy-2-adamantyl)pyrrolidin-2-one

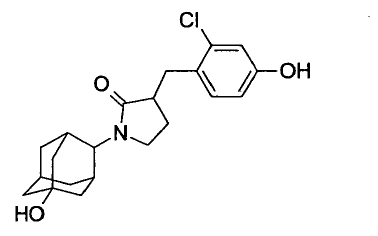

In the same manner as in Example 183A, the title compound was obtained from 3-[2-chloro-4-(methoxymethoxy)benzyl]-1-(5-hydroxy-2-adamantyl)pyrrolidin-2-oneobtained in Example 430A.
1H NMR (300 MHz, DMSO-d₆) δ ppm 1.37-1.41 (m, 2 H), 1.52-1.76 (m, 9 H), 1.84-1.94 (m, 2 H), 2.03 (br, 1H), 2.27-2.34 (m, 2 H), 2.43-2.64 (m, 2 H), 3.05-3.11 (dd, J=13.2, 3.9 Hz, 1 H), 3.35-3.51 (m, 2 H), 3.69 (s, 1 H), 4.10 (br, 1 H), 6.64-6.67 (dd, J=8.4, 2.7 Hz, 1 H), 6.78 (d, J=2.7 Hz, 1 H), 7.11-7.14 (d, J=8.4 Hz, 1 H), 9.67 (br, 1 H).

### Example 432A 3-[2-chloro-4-(methoxymethoxy)benzyl]-1-(5-methoxy-1,2,3,4-tetrahydronaphthalen-1-yl)pyrrolidin-2-one

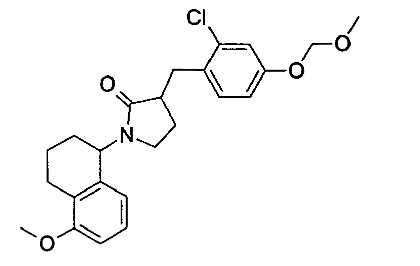

In the same manner as in Example 1A, the title compound was obtained from 1-(5-methoxy-1,2,3,4-tetrahydronaphthalen-1-yl)pyrrolidin-2-one obtained in Reference Example 37A and 2-chloro-4-(methoxymethoxy)benzyl bromide obtained in Reference Example 24A.
1H NMR (300 MHz, CDCl₃) δ ppm 1.65-1.81 (m, 3 H), 1.89-2.02 (m, 3 H), 2.48-2.54 (m, 1 H), 2.79-2.96 (m, 4 H), 3.02-3.10 (m, 1 H), 3.32-3.38 (dd, J=13.5, 4.2 Hz, 1 H), 3.47 (d, J=3.0 Hz, 3 H), 3.81 (s, 3 H), 5.14 (d, J=3.0 Hz, 2 H), 5.40-5.43 (m, 1 H), 6.54-6.61 (m, 1 H), 6.68-6.70 (d, J=8.1 Hz, 1 H), 6.86-6.92 (m, 1 H), 7.05-7.12 (m, 2 H), 7.19-7.25 (m, 1 H).

### Example 433A 3-(2-chloro-4-hydroxybenzyl)-1-(5-methoxy-1,2,3,4-tetrahydronaphthalen-1-yl)pyrrolidin-2-one

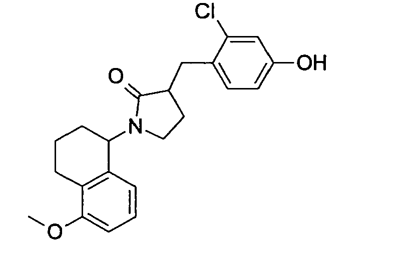

In the same manner as in Example 183A, the title compound was obtained from 3-[2-chloro-4-(methoxymethoxy)benzyl]-1-(5-methoxy-1,2,3,4-tetrahydronaphthalen-1-yl)pyrrolidin-2-oneobtained in Example 432A.
1H NMR (300 MHz, CDCl₃) δ ppm 1.73-1.86 (m, 3 H), 1.92-1.95 (m, 3 H), 2.47-2.52 (m, 1 H), 2.73-2.84 (m, 2 H), 2.90-2.97 (m, 2 H), 3.03-3.11 (m, 1 H), 3.27-3.32 (dd, J=13.8, 4.5 Hz, 1 H), 3.79 (s, 3 H), 5.39 (m, 1 H), 6.50-6.59 (m, 1 H), 6.65-6.77 (m, 2 H), 6.92 (m, 1 H), 7.04-7.12 (m, 2 H), 7.81 (m, 1 H).

### Example 434A 3-[2-chloro-4-(methoxymethoxy)benzyl]-1-(piperidin-1-yl)pyrrolidin-2-one

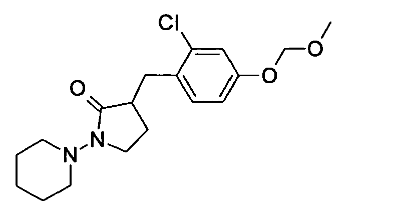

In the same manner as in Example 1A, the title compound was obtained from 1-(piperidin-1-yl)pyrtolidin-2-one obtained in Reference Example 22A and 2-chloro-4-(methoxymethoxy)benzyl bromide obtained in Reference Example 24A.
1H NMR (300 MHz, CDCl₃) δ ppm 1.36-1.44 (m, 2 H), 1.63-1.75 (m, 5 H), 1.93-2.04 (m, 1 H), 2.64-2.79 (m, 2 H), 2.86-2.98 (m, 4 H), 3.22-3.33 (m, 3 H), 3.46 (m, 3 H), 5.31 (m, 2 H), 6.48-6.88 (dd, J=8.7, 2.4 Hz, 1 H), 7.05-7.06 (d, J=2.4 Hz, 1 H), 7.17-7.20 (d, J=8.7 Hz, 1 H).

### Example 435A 3-[2-chloro-4-(methoxymethoxy)benzyl]-1-(2,3-dihydro-1H-indol-1-yl)pyrrolidin-2-one

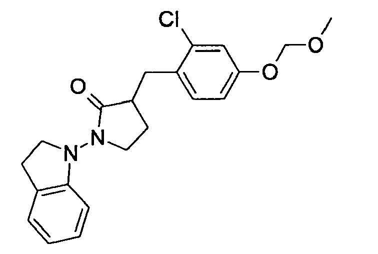

In the same manner as in Example 1A, the title compound was obtained from 1-(2,3-dihydro-1H-indol-1-yl)pyrrolidin-2-one obtained in Reference Example 20A and 2-chloro-4-(methoxymethoxy)benzyl bromide obtained in Reference Example 24A.
1H NMR (300 MHz, CDCl₃) δ ppm 1.81-1.88 (m, 1 H), 2.04-2.14 (m, 1 H), 2.82-2.91 (m, 2 H), 3.03-3.08 (t, J=7.8 Hz, 1 H), 3.23-3.42 (m, 4 H), 3. 50 (s, 3 H), 3.52-3. 54 (m, 1 H), 5. 13 (d, J=3. 3 Hz, 2 H), 6.40-6.43 (d, J=7.5 Hz, 1 H), 6.88-6.91 (dd, J=8.4, 2.7 Hz, 1 H), 7.05-7.09 (m, 3 H), 7.21-7.24 (d, J=8.4 Hz, 1 H).

### Example 436A 3-(2-chloro-4-hydroxybenzyl)-1-(piperidin-1-yl)pyrrolidin-2-one

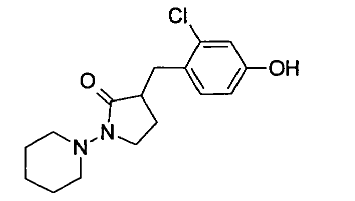

In the same manner as in Example 183A, the title compound was obtained from 3-[2-chloro-4-(methoxymethoxy)benzyl]-1-(piperidin-1-yl)pyrrolidin-2-one obtained in Example 434A.
1H NMR (300 MHz, CDCl₃) δ ppm 1.36 (m, 2 H), 1.66-1.73 (m, 3 H), 1.74-1.92 (m, 2 H), 1.96-2.05 (m, 1 H), 2.68-2.77 (m, 1 H), 2.82 (m, 2 H), 3.21-3.27 (q, J=9.3 Hz, 1 H), 3.30-3.35 (m, 1 H), 3.54-3.59 (t, J=6.6 Hz, 2 H), 3.67-3.71 (t, J=6.3 Hz, 2 H), 5.80 (br, 1 H), 6.69-6.73 (dd, J=8.4, 2.7 Hz, 1 H), 6.92 (d, J=2.7 Hz, 1 H), 7.02-7.05 (d, J=8.4 Hz, 1 H).

### Example 437A 3-(2-chloro-4-hydroxybenzyl)-1-(5-hydroxy-1,2,3,4-tetrahydronaphthalen-1-yl)pyrrolidin-2-one

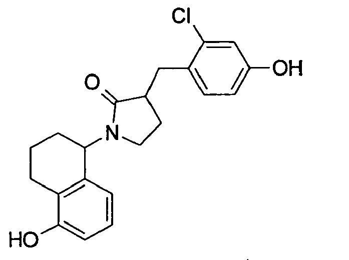

In the same manner as in Example 140A, the title compound was obtained from 3-(2-chloro-4-hydroxybenzyl)-1-(5-methoxy-1,2,3,4-tetrahydronaphthalen-1-yl)pyrrolidin-2-one obtained in Example 433A.
1H NMR (300 MHz, DMSO-d₆)δ ppm 1.49-1.78 (m, 4 H), 1.84-1.99 (m, 2 H), 2.38-2.85 (m, 5 H), 3.03-3.23 (m, 2 H), 5.12-5.14 (m, 1 H), 6.31-6.37 (m, 1 H), 6.64-6.71 (m, 2 H), 6.81-6.82 (m, 1 H), 6.89-6.96 (m, 1 H), 7.11-7.28 (m, 1 H), 9.31 (br, 1 H), 9.71 (br, 1 H).

### Example 438A 3-(2,4-dichlorobenzyl)-1-(5-hydroxy-2-adamantyl)pyrrolidin-2-one

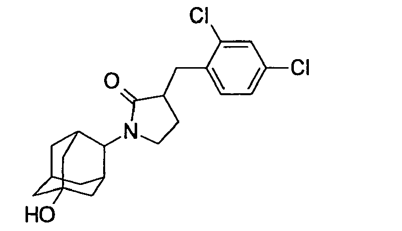

In the same manner as in Example 255A, the title compound was obtained from 1-(5-hydroxy-2-adamantyl)pyrrolidin-2-one obtained in Reference Example 38A and α,2,4-trichlorotoluene. 1H NMR (300 MHz, CDCl₃) δ ppm 1.50-1.54 (m, 2 H), 1.66-1.78 (m, 7 H), 1.91 -1.95 (m, 2 H), 2.00-2.10 (m, 1 H), 2.17 (m, 2 H), 2.42-2.44 (m, 2 H), 2.72-2.81 (m, 2 H), 3.27-3.35 (m, 1 H), 3.41-3.58 (m, 2 H), 3.92 (s, 1 H), 7.14-7.23 (m, 2 H), 7.35 (d, J=2.1 Hz, 1 H).

### Example 439A 1-(5-hydroxy-2-adamantyl)-3-(1-phenylethyl)pyrrolidin-2-one

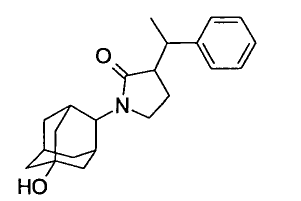

In the same manner as in Example 255A, the title compound was obtained from 1-(5-hydroxy-2-adamantyl)pyrrolidin-2-one obtained in Reference Example 38A and (1-bromoethyl)benzene. LC/MS (ESI+); m/z 340 (M+H)⁺

### Example 440A 3-(2-chloro-4-fluorobenzyl)-1-(5-hydroxy-2-adamantyl)pyrrolidin-2-one

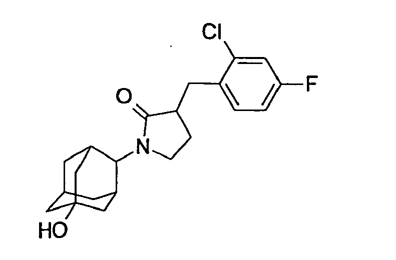

In the same manner as in Example 255A, the title compound was obtained from 1-(5-hydroxy-2-adamantyl)pyrrolidin-2-one obtained in Reference Example 38A and 1-(bromomethyl)-2-chloro-4-fluorobenzene.
1H NMR (300 MHz, CDCl₃) δ ppm 1.47-1.54 (m, 2 H), 1.60-1.76 (m, 7 H), 1.82 -1.99 (m, 3 H), 2.04-2.10 (m, 2 H), 2.42-2.46 (m, 2 H), 2.74-2.81 (m, 2 H), 3.26-3.35 (q, J=9.0 Hz, 1 H), 3.40-3.57 (m, 2 H), 3.91 (s, 1 H), 6.88-6.94 (m, 1 H), 7.08-7.12 (m, 1 H), 7.25-7.27 (m, 1 H).

### Example 441A 3-(4-bromo-2-chlorobenzyl)-1-(5-hydroxy-2-adamantyl)pyrrolidin-2-one

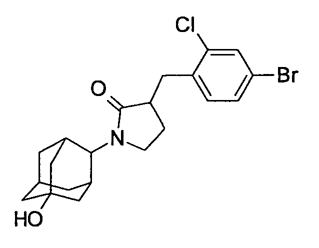

In the same manner as in Example 255A, the title compound was obtained from 1-(5-hydroxy-2-adamantyl)pyrrolidin-2-one obtained in Reference Example 38A and 4-bromo-1-(bromomethyl)-2-chlorobenzene.
LC/MS (ESI+); m/z 438, 440 (M+H)⁺

### Example 442A 3-(4-amino-2-chlorobenzyl)-1-(5-hydroxy-2-adamantyl)pyrrolidin-2-one

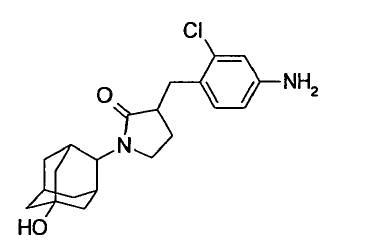

In the same manner as in Example 215A, the title compound was obtained from 3-(4-bromo-2-chlorobenzyl)-1-(5-hydroxy-2-adamantyl)pyrrolidin-2-one obtained in Example 441A.
LC/MS (ESI+); m/z 375, 377 (M+H)⁺

### Example 443A N-(3-chloro-4-{[1-(5-hydroxy-2-adamantyl)-2-oxopyrrolidin-3-yl]methyl}phenyl)methanesulfonamide

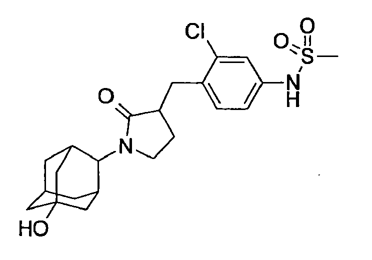

In the same manner as in Example 22A, the title compound was obtained from 3-(4-amino-2-chlorobenzyl)-1-(5-hydroxy-2-adamantyl)pyrrolidin-2-one obtained in Example 442A and methanesulfonyl chloride.
1H NMR (300 MHz, DMSO-d₆) δ ppm 1.38-1.42 (m, 2 H), 1.61-1.72 (m, 9 H), 1.90 (m, 1 H), 2.04 (m, 1 H), 2.28-2.34 (m, 2 H), 2.54-2.62 (m, 2 H), 3.01 (s, 3 H), 3.12-3.15 (m, 1 H), 3.34-3.42 (m, 1 H), 3.48-3.51 (m, 1 H), 3.70 (s, 1 H), 4.45 (s, 1 H), 7.08-7.11 (d, J=7.5 Hz, 1 H), 7.23 (s, 1 H), 7.31-7.33 (d, J=8.1 Hz, 1H), 9.89 (s, 1H).

### Example 444A 1-(3-chloro-4-{[1-(5-hydroxy-2-adamantyl)-2-oxopyrrolidin-3-yl]methyl}phenyl)imidazolidin-2-one

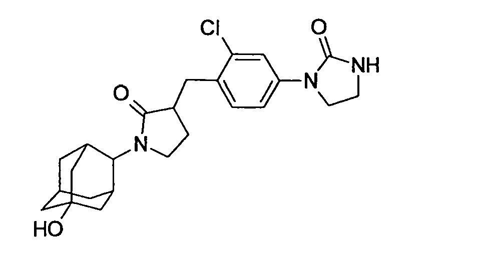

To a solution of 3-(4-amino-2-chlorobenzyl)-1-(5-hydroxy-2-adamantyl)pyrrolidin-2-one obtained in Example 442A (0.13 g) in tetrahydrofuran (3 mL) was added 2-chloroethyl isocyanate (42.2 mg), and the mixture was stirred at room temperature for 3 hr. After completion of the reaction, ethyl acetate and water were added to the reaction mixture, and the mixture was extracted with ethyl acetate. The ethyl acetate layer was washed with saturated brine, dried over anhydrous magnesium sulfate, and concentrated under reduced pressure. The obtained residue was dissolved in N,N-dimethylformamide (3 mL), sodium hydride was added to the reaction mixture at 0°C, and the mixture was stirred at room temperature for 3 hr. After completion of the reaction, ethyl acetate and water were added to the reaction mixture, and the mixture was extracted with ethyl acetate. The ethyl acetate layer was washed with saturated brine, dried over anhydrous magnesium sulfate, and concentrated under reduced pressure. The obtained residue was dissolved in ethyl acetate, and recrystallized to give the title compound (0.083 g, 54%) as colorless crystals.
1H NMR (300 MHz, DMSO-d₆) δ ppm 1.38-1.42 (m, 2 H), 1.53-1.72 (m, 9 H), 1.85-1.92 (m, 1 H), 2.04 (m, 1 H), 2.28-2.34 (m, 2 H), 2.54-2.65 (m, 2 H), 3.11-3.16 (dd, J=12.9, 3.6 Hz, 1 H), 3.37-3.44 (m, 3 H), 3.46-3.54 (m, 1 H), 3.70 (s, 1 H), 3.79-3.85 (m, 2 H), 4.45 (s, 1 H), 7.08 (s, 1 H), 7.31-7.34 (m, 2 H), 7.75 (d, J=2.4 Hz, 1 H).

### Example 445A N-{[(3-chloro-4-{[1-(5-hydroxy-2-adamantyl)-2-oxopyrrolidin-3-yl]methyl}phenyl)amino]carbonyl}glycine

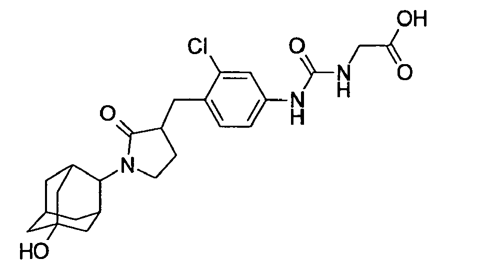

To a solution of 3-(4-amino-2-chlorobenzyl)-1-(5-hydroxy-2-adamantyl)pyrrolidin-2-one obtained in Example 442A (0.13 g) in tetrahydrofuran (3 mL) was added ethyl isocyanatoacetate (51.6 mg), and the mixture was stirred at room temperature for 3 hr. After completion of the reaction, ethyl acetate and water were added to the reaction mixture, and the mixture was extracted with ethyl acetate. The ethyl acetate layer was washed with saturated brine, dried over anhydrous magnesium sulfate, and concentrated under reduced pressure. A mixture of the obtained residue, methanol (3 mL), tetrahydrofuran (3 mL) and 4N aqueous sodium hydroxide solution (1 mL) was stirred at room temperature for 1 hr. The reaction mixture was concentrated, and the residue was partitioned between ethyl acetate-water. The aqueous layer was washed with ethyl acetate, 6N hydrochloric acid (1.5 mL) was added, and the mixture was extracted with ethyl acetate. The ethyl acetate layer was washed with saturated brine, and dried over anhydrous magnesium sulfate. The solvent was evaporated under reduced pressure to give the title compound (92 mg, 55%) as colorless crystals.
1H NMR (300 MHz, CDCl₃) δ ppm 1.38-1.41 (m, 2 H), 1.60-1.76 (m, 9 H), 1.89 (m, 1 H), 2.04 (m, 1 H), 2.28-2.34 (m, 2 H), 2.50-2.55 (m, 1 H), 2.60-2.61 (m, 1 H), 3.08-3.14 (dd, J=13.5, 3.6 Hz, 1 H), 3.39-3.54 (m, 2 H), 3.70 (s, 1 H), 3.77 (s, 2 H), 6.42 (m, 2 H), 7.11-7.20 (m, 3 H), 7.64 (d, J=1.8 Hz, 1 H), 8.92 (s, 1 H).

### Example 446A 3'-chloro-4'-{[1-(5-hydroxy-2-adamantyl)-2-oxopyrrolidin-3-yl]methyl}biphenyl-4-carboxylic acid

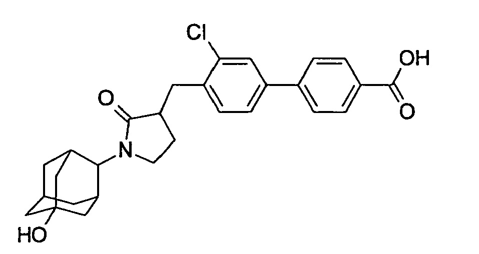

In the same manner as in Example 218A, the title compound was obtained from 3-(4-bromo-2-chlorobenzyl)-1-(5-hydroxy-2-adamantyl)pyrrolidin-2-one obtained in Example 441A and 4-carboxyphenylboronic acid.
1H NMR (300 MHz, DMSO-d₆)δ ppm 1.39-1.43 (m, 2 H), 1.61-1.76 (m, 9 H), 1.94 -1.99 (m, 1 H), 2.04 (m, 1 H), 2.30-2.35 (m, 2 H), 2.62-2.69 (m, 2 H), 3.23-3.26 (m, 1 H), 3.38-3.44 (m, 1 H), 3.46-3.57 (m, 1 H), 3.72 (s, 1 H), 4.45 (s, 1 H), 7.47-7.50 (d, J=8.1 Hz, 1 H), 7.65-7.67 (d, J=7.8 Hz, 1 H), 7.81-7.84 (d, J=9.0 Hz, 3 H), 7.99-8.02 (d, J=8.4 Hz, 3 H), 13.03 (br, 1 H).

### Example 447A 3-{2-chloro-4-[5-(hydroxymethyl)-2-furyl]benzyl}-1-(5-hydroxy-2-adamantyl)pyrrolidin-2-one

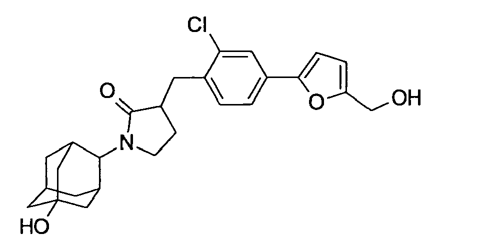

A mixture of 3-(4-bromo-2-chlorobenzyl)-1-(5-hydroxy-2-adamantyl)pyrrolidin-2-one obtained in Example 441A (0.38 g), (5-formyl-2-furyl)boronic acid (0.175 g), 2 M aqueous sodium hydrogencarbonate solution (0.81mL), tetrakis(triphenylphosphine)palladium (19 mg) and 1,2-dimethoxyethane (5 mL) was stirred overnight at 90°C under nitrogen atmosphere. Ethyl acetate and water were added to the reaction mixture, and the mixture was extracted with ethyl acetate. The ethyl acetate layer was washed with saturated brine, dried over anhydrous magnesium sulfate, and concentrated under reduced pressure. The residue was subjected to silica gel column chromatography with ethyl acetate-hexane (10:90-100:0), and the obtained solid was recrystallized from ethyl acetate-hexane to give a colorless solid (0.18 g). A mixture of the obtained colorless solid (0.18 g), sodium borohydride (0.019 g), tetrahydrofuran (2 mL) and methanol (2 mL) was stirred at room temperature for 1 hr. The reaction solution was concentrated under reduced pressure, and the residue was partitioned between water-ethyl acetate. The ethyl acetate layer was washed with saturated brine, and dried over anhydrous magnesium sulfate.

The solvent was evaporated under reduced pressure, and the residue was subjected to silica gel column chromatography (ethyl acetate) to give the title compound (0.071 g, 37%) as a colorless powder.
1H NMR (300 MHz, CDCl₃) δ ppm 1.48-1.53 (m, 2 H), 1.69-1.76 (m, 8 H), 1.90 -1.94 (m, 2 H), 1.98-2.02 (m, 1 H), 2.16 (m, 1 H), 2.41-2.44 (m, 3 H), 2.72-2.82 (m, 2 H), 3.31-3.34 (m, 1 H), 3.39-3.53 (m, 2 H), 3.92 (s, 1 H), 4.66 (s, 2 H), 6.36-6.37 (d, J=3.3 Hz, 1 H), 6. 55-6. 58 (m, 1 H), 7 . 22-7 . 25 (d, J=8 .1 Hz, 1 H), 7.42-7.45 (dd, J=7.8, 1.5 Hz, 1 H), 7.64 (d, J=1.8 Hz, 1 H).

### Example 448A 5-(3-chloro-4-{[1-(5-hydroxy-2-adamantyl)-2-oxopyrrolidin-3-yl]methyl}phenyl)-2-furoic acid

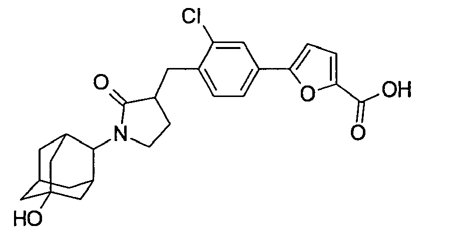

A mixture of 3-(4-bromo-2-chlorobenzyl)-1-(5-hydroxy-2-adamantyl)pyrrolidin-2-one obtained in Example 441A (0.38 g), (5-formyl-2-furyl)boronic acid (0.175 g), 2 M aqueous sodium hydrogencarbonate (0.81 mL), tetrakis(triphenylphosphine)palladium (19 mg) and 1,2-dimethoxyethane (5 mL) was stirred overnight at 90°C under nitrogen atmosphere. Ethyl acetate and water were added to the reaction mixture, and the mixture was extracted with ethyl acetate. The ethyl acetate layer was washed with saturated brine, dried over anhydrous magnesium sulfate, and concentrated under reduced pressure. The residue was subjected to silica gel column chromatography with ethyl acetate-hexane (10:90-100:0), and the obtained solid was recrystallized from ethyl acetate-hexane to give a colorless solid (0.18 g). A mixture of the obtained colorless solid (0.18 g), N-iodosuccinimide (0.304 g), potassium carbonate (0.187 g) and methanol (10 mL) was stirred at room temperature for 16 hr under shading. Water was added to the reaction solution, and the mixture was extracted with ethyl acetate. The ethyl acetate layer was washed with saturated brine, and dried over anhydrous magnesium sulfate. The solvent was evaporated under reduced pressure. The residue was subjected to silica gel column chromatography with ethyl acetate-hexane (10:90-100:0) to give a colorless solid (0.13 g). A mixture of the obtained colorless solid (0.13 g), methanol (3 mL), tetrahydrofuran (3 mL) and 8N aqueous sodium hydroxide solution (1 mL) was stirred at room temperature for 1 hr. The reaction mixture was concentrated, and the residue was partitioned between ethyl acetate-water. The aqueous layer was washed with ethyl acetate, 6N hydrochloric acid (2 mL) was added, and the mixture was extracted with ethyl acetate. The ethyl acetate layer was washed with saturated brine, and dried over anhydrous magnesium sulfate. The solvent was evaporated under reduced pressure to give the title compound (109 mg, 44%) as colorless crystals.
1H NMR (300 MHz, DMSO-d₆) δ ppm 1.39-1.43 (m, 2 H), 1.62-1.74 (m, 9 H), 1.94 -2.05 (m, 2 H), 2.30-2.36 (m, 2 H), 2.51 (m, 1 H), 2.65-2.69 (m, 2 H), 3.21-3.25 (d, J=11.4 Hz, 1 H), 3.41-3.44 (m, 1H), 3.51-3.54 (m, 1H), 3.72 (s, 1H), 7.24 (d, J=3.0 Hz, 1 H), 7.33 (d, J=3.0 Hz, 1 H), 7.48-7.50 (d, J=8.1 Hz, 1 H), 7.70-7.72 (d, J=8.1 Hz, 1 H), 7.87 (s, 1 H).

### Example 449A 3-[2-chloro-4-(5-cyclopropyl-1,2,4-oxadiazol-3-yl)benzyl]-1-(5-hydroxy-2-adamantyl)pyrrolidin-2-one

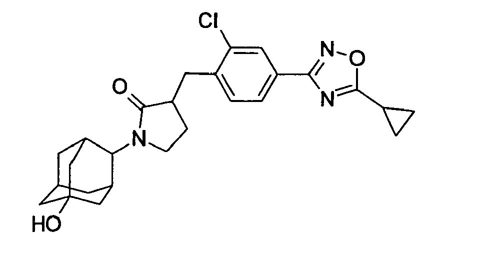

In the same manner as in Example 255A, the title compound was obtained from 1-(5-hydroxy-2-adamantyl)pyrrolidin-2-one obtained in Reference Example 38A and 3-(4-bromomethyl-3-chlorophenyl)-5-cyclopropyl-1,2,4-oxadiazol obtained in Reference Example 48A.
LC/MS (ESI+); m/z 468, 470 (M+H)⁺

### Example 450A N-(3'-chloro-4'-{[1-(5-hydroxy-2-adamantyl)-2-oxopyrrolidin-3-yl]methyl}biphenyl-4-yl)methanesulfonamide

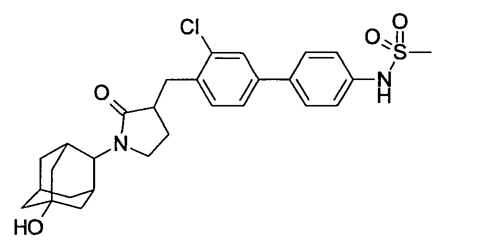

In the same manner as in Example 218A, the title compound was obtained from 3-(4-bromo-2-chlorobenzyl)-1-(5-hydroxy-2-adamantyl)pyrrolidin-2-one obtained in Example 441A and {4-[(methylsulfonyl)amino]phenyl}boronic acid.
LC/MS (ESI+); m/z 529, 531 (M+H)⁺

### Example 451A 3-[(3-chloro-4'-fluorobiphenyl-4-yl)methyl]-1-(5-hydroxy-2-adamantyl)pyrrolidin-2-one

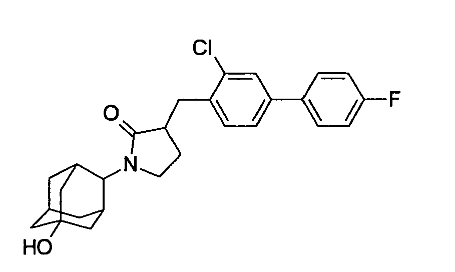

In the same manner as in Example 218A, the title compound was obtained from 3-(4-bromo-2-chlorobenzyl)-1-(5-hydroxy-2-adamantyl)pyrrolidin-2-one obtained in Example 441A and (4-fluorophenyl)boronic acid.
1H NMR (300 MHz, CDCl₃) δ ppm 1.50-1.55 (m, 2 H), 1.73-1.83 (m, 8 H), 1.92 -1.96 (m, 2 H), 2.12-2.17 (m, 2 H), 2.43-2.47 (m, 2 H), 2.77-2.86 (m, 2 H), 3.34-3.59 (m, 3 H), 3.95 (s, 1 H), 7.08-7.15 (m, 2 H), 7.31-7.37 (m, 2 H), 7.47-7.53 (m, 3 H).

### Example 452A 3-[2-chloro-4-(5-chloro-2-thienyl)benzyl]-1-(5-hydroxy-2-adamantyl)pyrrolidin-2-one

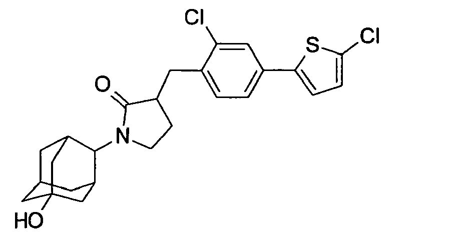

In the same manner as in Example 218A, the title compound was obtained from 3-(4-bromo-2-chlorobenzyl)-1-(5-hydroxy-2-adamantyl)pyrrolidin-2-one obtained in Example 441A and (5-chloro-2-thienyl)boronic acid.
1H NMR (300 MHz, CDCl₃) δ ppm 1.50-1.54 (m, 2 H), 1.67-1.79 (m, 8 H), 1.91 -2.12(m, 3 H), 2.17 (m, 1 H), 2.42-2.46 (m, 2 H), 2.73-2.84 (m, 2 H), 3.30-3.58 (m, 3 H), 3.92 (s, 1 H), -6.87-6.88 (d, J=3.9 Hz, 1 H), 7.03-7.04 (d, J=3.9 Hz, 1 H), 7.25-7.32 (m, 2 H), 7.48 (d, J=1.2 Hz, 1H).

### Example 453A N-(3-chloro-4-{[1-(5-hydroxy-2-adamantyl)-2-oxopyrrolidin-3-yl]methyl}phenyl)cyclopropanesulfonamide

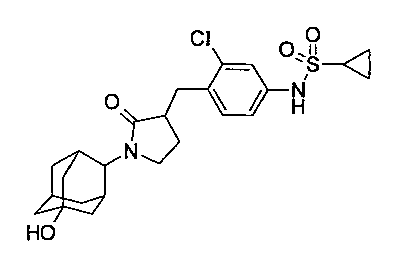

In the same manner as in Example 22A, the title compound was obtained from 3-(4-amino-2-chlorobenzyl)-1-(5-hydroxy-2-adamantyl)pyrrolidin-2-one obtained in Example 442A and cyclopropanesulfonyl chloride.
1H NMR (300 MHz, CDCl₃) δ ppm 0.92-0.99 (m, 2 H), 1.14-1.19 (m, 2 H), 1.50-1.54 (m, 2 H), 1.67-1.76 (m, 8 H), 2.17 (m, 2 H), 2.40-2.54 (m, 3 H), 2.71-2.82 (m, 2 H), 3.28-3.31 (m, 1 H), 3.43-3.60 (m, 2 H), 3.93 (s, 1 H), 7.11-7.15 (dd, J=8.1, 2.1 Hz, 1 H), 7.20-7.23 (m, 1 H), 7.34 (d, J=1.8 Hz, 1 H), 7.86 (br, 1 H). Example 454A N-(3-chloro-4-{[1-(5-hydroxy-2-adamantyl)-2-oxopyrrolidin-3-yl]methyl}phenyl)-3,5-dimethylisoxazole-4-sulfonamide

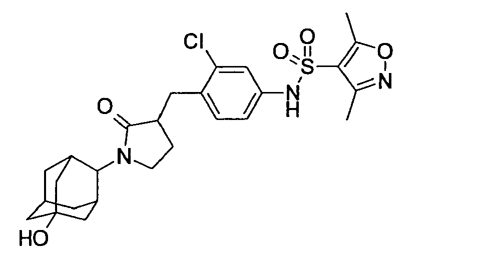

In the same manner as in Example 22A, the title compound was obtained from 3-(4-amino-2-chlorobenzyl)-1-(5-hydroxy-2-adamantyl)pyrrolidin-2-one obtained in Example 442A and 3,5-dimethylisoxazole-4-sulfonyl chloride.
1H NMR (300 MHz, CDCl₃) δ ppm 1.51-1.55 (m, 2 H), 1.76 (m, 8 H), 1.87 -1.91 (m, 2 H), 2.18 (m, 1 H), 2.31 (s, 3 H), 2.47 (m, 2 H), 2.52 (s, 3 H), 2.76-2.87 (m, 2 H), 3.21-3.26 (m, 2 H), 3.50-3.59 (m, 2 H), 3.92 (s, 1 H), 6.90-6.93 (dd, J=8.4, 2.7 Hz, 1 H), 7.16-7.20 (m, 2 H), 8.16 (s, 1 H).

### Example 455A N-(3-chloro-4-{[1-(5-hydroxy-2-adamantyl)-2-oxopyrrolidin-3-yl]methyl}phenyl)-N'-(cyclopropylmethyl)thiourea

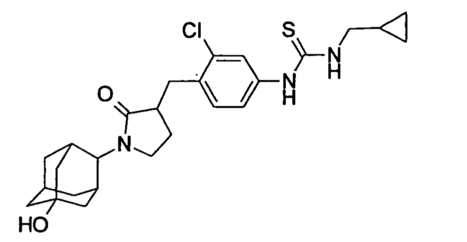

In the same manner as in Example 210A, the title compound was obtained from 3-(4-amino-2-chlorobenzyl)-1-(5-hydroxy-2-adamantyl)pyrrolidin-2-one obtained in Example 442A and (isothiocyanatomethyl)cyclopropane.
LC/MS (ESI+); m/z 488, 490 (M+H)⁺

### Example 456A N-(3-chloro-4-{[1-(5-hydroxy-2-adamantyl)-2-oxopyrrolidin-3-yl]methyl}phenyl)benzamide

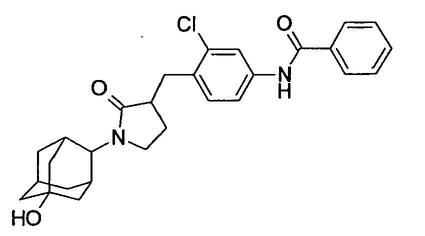

In the same manner as in Example 22A, the title compound was obtained from 3-(4-amino-2-chlorobenzyl)-1-(5-hydroxy-2-adamantyl)pyrrolidin-2-one obtained in Example 442A and benzoyl chloride.
1H NMR (300 MHz, CDCl₃) δ ppm 1.42-1.51 (m, 2 H), 1.65-1.88 (m, 9 H), 1.96 -2.00 (m, 1 H), 2.15 (m, 1 H), 2.23 (m, 1 H), 2.37-2.41 (m, 2 H), 2.66-2.75 (m, 2 H), 3.22-3.31 (q, J=9.0 Hz, 1 H), 3.39-3.56 (m, 2 H), 3.88 (s, 1 H), 7.13-7.16 (d, J=8.4 Hz, 1 H), 7.38-7.53 (m, 4 H), 7.81 (d, J=2.1 Hz, 1 H), 7.86-7.88 (m, 2 H), 8.79 (s, 1 H).

### Example 457A ethyl 1-{[(3-chloro-4-{[1-(5-hydroxy-2-adamantyl)-2-oxopyrrolidin-3-yl]methyl}phenyl)amino]carbonyl}cyclopropanecarboxylate

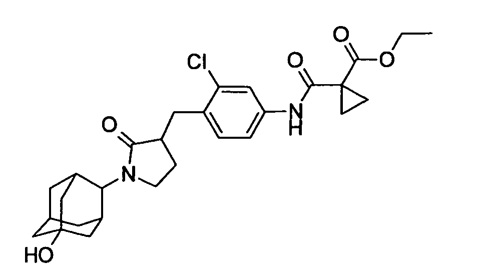

In the same manner as in Example 68A, the title compound was obtained from 3-(4-amino-2-chlorobenzyl)-1-(5-hydroxy-2-adamantyl)pyrrolidin-2-one obtained in Example 442A and 1-(ethoxycarbonyl)cyclopropanecarboxylic acid.
1H NMR (300 MHz, CDCl₃) δ ppm 1.26-1.30 (t, J=7.2 Hz, 3 H), 1.45-1. 53 (m, 3 H), 1.63-1.81 (m, 11 H), 1.93 -1.96 (m, 2 H), 1. 98 - 2.05 (m, 1 H), 2.17 (br, 1 H), 2.43-2.47 (m, 2 H), 2.71-2.88 (m, 2 H), 3.28-3.55 (m, 3 H), 3.91 (s, 1 H), 4.16-4.23 (q, J=7.2 Hz, 2 H), 7.18-7.21 (d, J=8.1 Hz, 1 H), 7.30-7.33 (dd, J=8.1, 2.1 Hz, 1 H), 7. 74 (d, J=2. 1 Hz, 1 H), 10.92 (s, 1 H).

### Example 458A N-(3-chloro-4-{[1-(5-hydroxy-2-adamantyl)-2-oxopyrrolidin-3-yl]methyl}phenyl)-1-(hydroxymethyl)cyclopropanecarboxamide

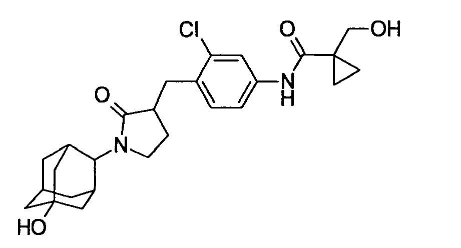

In the same manner as in Reference Example 19A, the title compound was obtained from Ethyl 1-{[(3-chloro-4-{[1-(5-hydroxy-2-adamantyl)-2-oxopyrrolidin-3-yl]methyl}phenyl)amino]carbonyl}cyclopropanecarboxylate obtained in Example 457A.
1H NMR (300 MHz, CDCl₃) δ ppm 0.72-0.76 (m, 2 H), 1.28-1.31 (m, 2 H), 1.48-1.53 (m, 2 H), 1.66-1.75 (m, 8 H), 1.88 -1.92 (m, 2 H), 1.96-1.99 (m, 1 H), 2.16 (br, 1H), 2.40 (m, 2 H), 2.69-2.75 (m, 2 H), 3.24-3.29 (m, 1 H), 3.43-3.53 (m, 2 H), 3.74 (s, 2 H), 3.90 (s, 1 H), 4.21 (br, 1 H), 7.09-7.12 (d, J=8.1 Hz, 1 H), 7.20-7.24 (dd, J=8.1, 2.1 Hz, 1 H), 7.67 (d, J=2.1 Hz, 1 H), 9.44 (s, 1 H).

### Example 459A N-(3-chloro-4-{[1-(5-hydroxy-2-adamantyl)-2-oxopyrrolidin-3-yl]methyl}phenyl)-4-fluorobenzenesulfonamide

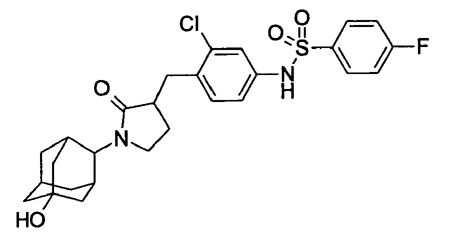

In the same manner as in Example 22A, the title compound was obtained from 3-(4-amino-2-chlorobenzyl)-1-(5-hydroxy-2-adamantyl)pyrrolidin-2-one obtained in Example 442A and 4-fluorobenzenesulfonyl chloride.
1H NMR (300 MHz, CDCl₃) δ ppm 1.49-1.53 (m, 2 H), 1.72-1.80 (m, 8 H), 1.88-1.92 (m, 2 H), 1.98-2.09 (m, 1 H), 2.17 (br, 1 H), 2.41 (m, 2 H), 2.67-2.82 (m, 2 H), 3.20-3.26 (dd, J=12.6, 3.9 Hz, 1 H), 3.39-3.57 (m, 2 H), 3.92 (s, 1 H), 6.90-6.97 (dd, J=8.1, 1.8 Hz, 1 H), 7.04-7.22 (m, 4 H), 7.74-7.83 (m, 2 H), 7.93-7.97 (m, 1 H).

### Example 460A 3-[2-chloro-4-(2-oxopyrrolidin-1-yl)benzyl]-1-(5-hydroxy-2-adamantyl)pyrrolidin-2-one

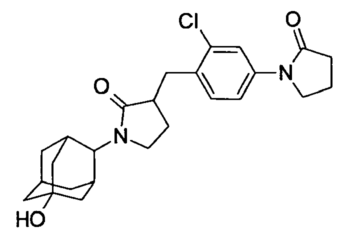

In the same manner as in Reference Example 3A, the title compound was obtained from 3-(4-amino-2-chlorobenzyl)-1-(5-hydroxy-2-adamantyl)pyrrolidin-2-one obtained in Example 442A.
1H NMR (300 MHz, CDCl₃) δ ppm 1.50-1.54 (m, 2 H), 1.68-1.84 (m, 8 H), 1.90-1.94 (m, 2 H), 1.98-2.08 (m, 1 H), 2.12-2.22 (m, 3 H), 2.43-2.47 (m, 2 H), 2.58-2.64 (t, J=7.8 Hz, 2 H), 2.75-2.80 (m, 2 H), 3.29-3.40 (q, J=9.0 Hz, 1 H), 3.43-3.56 (m, 2 H), 3.81-3.85 (t, J=6.9 Hz, 2 H), 3.92 (s, 1 H), 7.24-7.28 (d, J=8.1 Hz, 1 H), 7.44-7.48 (dd, J=8.1, 2.1 Hz, 1 H), 7.68 (d, J=2.1 Hz, 1 H).

### Example 461A tert-butyl (1-{[(3-chloro-4-{[1-(5-hydroxy-2-adamantyl)-2-oxopyrrolidin-3-yl]methyl}phenyl)amino]carbonyl}cyclopropyl)carbamate

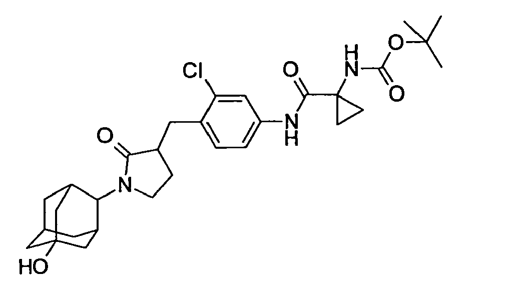

In the same manner as in Example 68A, the title compound was obtained from 3-(4-amino-2-chlorobenzyl)-1-(5-hydroxy-2-adamantyl)pyrrolidin-2-one obtained in Example 442A and 1-[(tert-butoxycarbonyl)amino]cyclopropanecarboxylic acid.
1H NMR (300 MHz, CDCl₃) δ ppm 1.08-1.12 (m, 2 H), 1.49 (s, 9 H), 1.53 (m, 2 H), 1.63-1.67 (m, 2 H), 1.70-1.78 (m, 8 H), 1.89-1.93 (m, 2 H), 1.96-2.02 (m, 1 H), 2.17 (br, 1 H), 2.43-2.47 (m, 2 H), 2.71-2.80 (m, 2 H), 3.27-3.35 (m, 1 H), 3.39-3.55 (m, 2 H), 3.91 (s, 1 H), 5.23 (br, 1 H), 7.16-7.24 (m, 2 H), 7.66 (s, 1 H), 8.47 (br, 1 H).

### Example 462A 1-amino-N-(3-chloro-4-{[1-(5-hydroxy-2-adamantyl)-2-oxopyrrolidin-3-yl]methyl}phenyl)cyclopropanecarboxamide hydrochloride

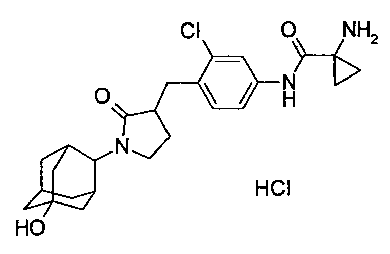

In the same manner as in Example 20A, the title compound was obtained from tert-butyl (1-{[(3-chloro-4-{[1-(5-hydroxy-2-adamantyl)-2-oxopyrrolidin-3-yl]methyl}phenyl)amino]carbonyl}cyclopropyl)carbamate obtained in Example 461A.
1H NMR (300 MHz, DMSO-d₆) δ ppm 1.38-1.40 (m, 5 H), 1.59-1.72 (m, 10 H), 1.89 (m, 1 H), 2.04 (br, 1 H), 2.28-2.34 (m, 2 H), 2.50-2.64 (m, 2 H), 3.12-3.17 (dd, J=13.2, 3.6 Hz, 1 H), 3.39-3.44 (m, 1 H), 3.49-3.54 (m, 1 H), 3.70 (s, 1 H), 3.76 (br, 1 H), 7.30-7.33 (d, J=8.4 Hz, 1 H), 7.51-7.54 (dd, J=8.4, 2.1 Hz, 1 H), 7. 77 (d, J=2.4 Hz, 1 H), 8.80 (br, 3 H), 9.69 (s, 1 H).

### Example 463A N-(3-chloro-4-{[1-(5-hydroxy-2-adamantyl)-2-oxopyrrolidin-3-yl]methyl}phenyl)-4-oxocyclohexanecarboxamide

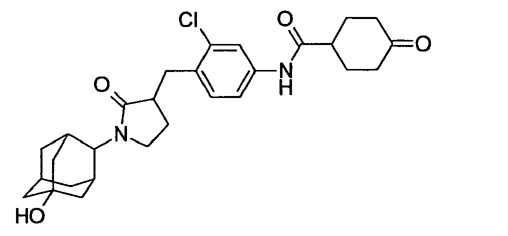

In the same manner as in Example 68A, the title compound was obtained from 3-(4-amino-2-chlorobenzyl)-1-(5-hydroxy-2-adamantyl)pyrrolidin-2-one obtained in Example 442A and 4-oxocyclohexanecarboxylic acid.
1H NMR (300 MHz, CDCl₃) δ ppm 1.50-1.55 (m, 2 H), 1.77-1.92 (m, 10 H), 2.05-2.22 (m, 6 H), 2.31-2.42 (m, 4 H), 2.55-2.60 (m, 2 H), 2.69-2.80 (m, 3 H), 3.26-3.29 (q, J=8.4 Hz, 1 H), 3.44-3.58 (m, 2 H), 3.92 (s, 1 H), 7.17-7.20 (d, J=8.1 Hz, 1 H), 7.27-7.31 (m, 1 H), 7. 70 (d, J=1.8 Hz, 1 H), 8.06 (s, 1 H).

### Example 464A N-(3-chloro-4-{[1-(5-hydroxy-2-adamantyl)-2-oxopyrrolidin-3-yl]methyl}phenyl)-2-(phenylsulfonyl)acetamide

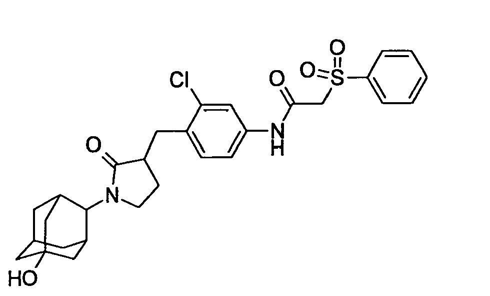

In the same manner as in Example 68A, the title compound was obtained from 3-(4-amino-2-chlorobenzyl)-1-(5-hydroxy-2-adamantyl)pyrrolidin-2-one obtained in Example 442A and (phenylsulfonyl)acetic acid.
1H NMR (300 MHz, CDCl₃) δ ppm 1.49-1.54 (m, 2 H), 1.64-1.93 (m, 10 H), 2.01 -2.08 (m, 1 H), 2.18 (br, 1 H), 2.42-2.45 (m, 2 H), 2.72-2.81 (q, J=9.3 Hz, 2 H), 3.28-3.31 (q, J=9.0 Hz, 1 H), 3.42-3.56 (m, 2 H), 3.93 (s, 1 H), 4.20 (s, 2 H), 6.98 (s, 1 H), 7.18-7.20 (d, J=6.9 Hz, 1 H), 7.27-7.30 (m, 1 H), 7.58-7.68 (m, 2 H), 7.70-7.73 (m, 1 H), 7.91-7.94 (m, 2 H), 8.94 (s, 1 H).

### Example 465A ethyl 4-({[(3-chloro-4-{[1-(5-hydroxy-2-adamantyl)-2-oxopyrrolidin-3-yl]methyl}phenyl)amino]carbonyl}amino)benzoate

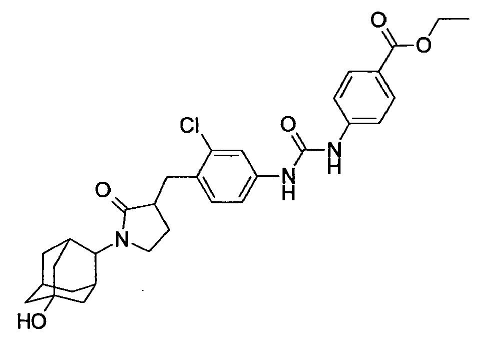

In the same manner as in Example 210A, the title compound was obtained from 3-(4-amino-2-chlorobenzyl)-1-(5-hydroxy-2-adamantyl)pyrrolidin-2-one obtained in Example 442A and ethyl 4-isocyanatobenzoate.
1H NMR (300 MHz, CDCl₃) δ ppm 1.36-1.39 (t, J=6.9 Hz, 3 H), 1.49-1.53 (m, 2 H), 1.75-1.86 (m, 10 H), 2.08-2.12 (m, 1 H), 2.17 (br, 1 H), 2.36-2.40 (m, 2 H), 2.70-2.82 (m, 2 H), 3.15-3.17 (m, 2 H), 3.49-3.60 (m, 2 H), 3.92 (s, 1 H), 4.28-4.36 (q, J=6.9 Hz, 2 H), 7.03 (s, 1 H), 7.40-7.44 (m, 3 H), 7.90-7.93 (d, J=8.7 Hz, 2 H), 8.08 (s, 1 H), 8.29 (s, 1 H).

### Example 466A N-(3-chloro-4-{[1-(5-hydroxy-2-adamantyl)-2-oxopyrrolidin-3-yl]methyl}phenyl)-5-(hydroxymethyl)-2-furamide

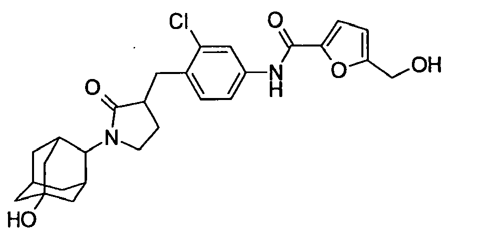

A mixture of 3-(4-amino-2-chlorobenzyl)-1-(5-hydroxy-2-adamantyl)pyrrolidin-2-one obtained in Example 442A (0.133 g), 5-formyl-2-furancarboxylic acid (0.076 g), bis(2-oxo-3-oxazolidinyl)phosphinic chloride (0.183 g), triethylamine (0.139 mL) and dichloromethane (2 mL) was stirred at room temperature for 16 hr. The reaction solution was concentrated, and the residue was partitioned between ethyl acetate-water. The ethyl acetate layer was washed successively with 2N hydrochloric acid and saturated brine, and dried over anhydrous magnesium sulfate, and the solvent was evaporated under reduced pressure to give a colorless solid. A mixture of the obtained colorless solid, sodium borohydride (0.038 g), tetrahydrofuran (3 mL) and methanol (1 mL) was stirred at room temperature for 1 hr. The reaction solution was concentrated under reduced pressure, and the residue was partitioned between water-ethyl acetate. The ethyl acetate layer was washed with saturated brine, and dried over anhydrous magnesium sulfate. The solvent was evaporated under reduced pressure, and the residue was subjected to silica gel column chromatography (ethyl acetate) to give the title compound (0.048 g, 26%) as a colorless powder.
1H NMR (300 MHz, CD₃OD) δ ppm 1.14-1.20 (m, 2 H), 1.51 -1.55 (m, 2 H), 1.74 (m, 5 H), 1.85 -1.89 (m, 4 H), 2.01-2.06 (m, 1 H), 2.13 (br, 1 H), 2.37-2.42 (m, 2 H), 2.68-2.82 (m, 2 H), 3.30 (m, 1 H), 3.45-3.62 (m, 3 H), 3.89 (s, 1 H), 4.62 (s, 2 H), 6.50 (s, 1 H), 7.20 (m, 1 H), 7.27-7.30 (d, J=8.7 Hz, 1 H), 7.51-7.55 (dd, J=8 . 4, 1.2 Hz, 1 H), 7.67 (d, J=1.2 Hz, 1 H) .

### Example 467A N-(3-chloro-4-{[1-(5-hydroxy-2-adamantyl)-2-oxopyrrolidin-3-yl]methyl}phenyl)-4-hydroxycyclohexanecarboxamide

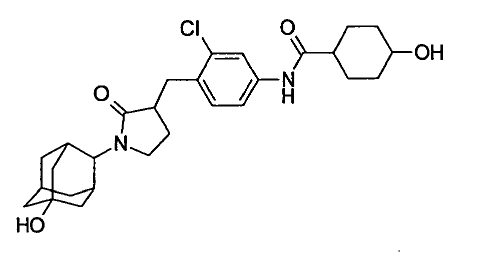

In the same manner as in Example 29A, the title compound was obtained from N-(3-chloro-4-{[1-(5-hydroxy-2-adamantyl)-2-oxopyrrolidin-3-yl]methyl}phenyl)-4-oxocyclohexanecarboxamide obtained in Example 463A.
1H NMR (300 MHz, CD₃OD) δ ppm 1.26-1.36 (m, 2 H), 1.50-1.65 (m, 7 H), 1.74-1.79 (m, 6 H), 1.85-1.93 (m, 7 H), 1.96-2.03 (m, 1 H), 2.13 (br, 1 H), 2.29-2.40 (m, 3 H), 2.65-2.73 (m, 1 H), 2.79-2.83 (m, 1 H), 3.25-3.30 (m, 1 H), 3.47-3.61 (m, 3 H), 3.88 (s, 1 H), 7.21-7.24 (d, J=8.1 Hz, 1 H), 7.32-7.36 (dd, J=8.4, 2.1 Hz, 1 H), 7.74 (d, J=2.1 Hz, 1 H).

### Example 468A N-(3-chloro-4-{[1-(5-hydroxy-2-adamantyl)-2-oxopyrrolidin-3-yl]methyl}phenyl)-N'-[4-(hydroxymethyl)phenyl]urea

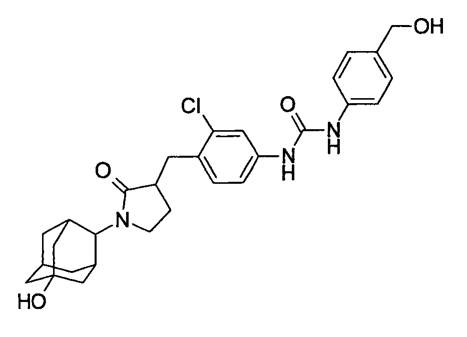

In the same manner as in Reference Example 19A, the title compound was obtained from ethyl 4-({[(3-chloro-4-{[1-(5-hydroxy-2-adamantyl)-2-oxopyrrolidin-3-yl]methyl}phenyl)amino]carbonyl}amino)benzoate obtained in Example 465A.
1H NMR (300 MHz, DMSO-d₆) δ ppm 1.38-1.42 (m, 2 H), 1.61-1.72 (m, 10 H), 1.90-1.93 (m, 1 H), 2.04 (m, 1 H), 2.28-2.35 (m, 2 H), 2.49 (m, 1 H), 2.61-2.63 (m, 1 H), 3.10-3.17 (dd, J=13.5, 3.6 Hz, 1 H), 3.39-3.42 (q, J=8.4 Hz, 1 H), 3.48-3.51 (m, 1 H), 3.70 (s, 1 H), 4.40-4.42 (d, J=5.7 Hz, 2 H), 5.04-5.08 (t, J=5.7 Hz, 1 H), 7.17-7.25 (m, 4 H), 7.37-7.40 (d, J=8.7 Hz, 2 H), 7.69 (d, J=1.8 Hz, 1 H), 8.76 (s, 1 H), 8.86 (s, 1 H).

### Example 469A 4-({[(3-chloro-4-{[1-(5-hydroxy-2-adamantyl)-2-oxopyrrolidin-3-yl]methyl}phenyl)amino]carbonyl}amino)benzoic acid

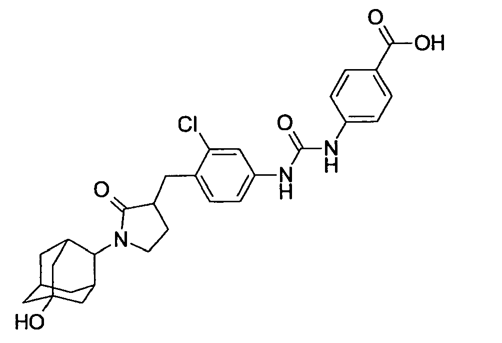

In the same manner as in Example 155A, the title compound was obtained from ethyl 4-({[(3-chloro-4-([1-(5-hydroxy-2-adamantyl)-2-oxopyrrolidin-3-yl]methyl}phenyl)amino]carbonyl}amino)benzoate obtained in Example 465A.
1H NMR (300 MHz, DMSO-d₆) δ ppm 1.38-1.42 (m, 2 H), 1.60-1.72 (m, 10 H), 2.04 (m, 1 H), 2.28 -2.34 (m, 2 H), 2.51 (m, 1 H), 2.54-2.66 (m, 1 H), 3.11-3.17 (dd, J=13.2, 3.9 Hz, 1 H), 3.34-3.56 (m, 2 H), 3.71 (s, 1 H), 6.05 (br, 1 H), 7.20-7.28 (m, 2 H), 7.54-7.57 (d, J=8.7 Hz, 2 H), 7.70 (d, J=1.5 Hz, 1 H), 7.84-7.87 (d, J=9.0 Hz, 2 H), 8.95 (s, 1H), 9.14 (s, 1H).

### Example 470A N-(3'-chloro-4'-{[1-(5-hydroxy-2-adamantyl)-2-oxopyrrolidin-3-yl]methyl}biphenyl-3-yl)methanesulfonamide

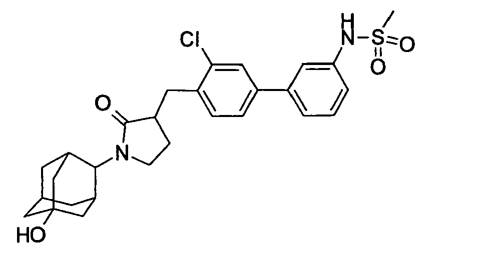

In the same manner as in Example 218A, the title compound was obtained from 3-(4-bromo-2-chlorobenzyl)-1-(5-hydroxy-2-adamantyl)pyrrolidin-2-one obtained in Example 441A and N-[3-(4, 4, 5, 5-tetramethyl-1, 3, 2-dioxaboloran-2-yl)phenyl]methanesulfonamide.
1H NMR (300 MHz, DMSO-d₆) δ ppm 1.38-1.43 (m, 2 H), 1.61-1.73 (m, 10 H), 1.94-1.99 (m, 1 H), 2.04 (br, 1 H), 2.29 -2.36 (m, 2 H), 2.61-2.73 (m, 2 H), 3.04 (s, 3 H), 3.21-3.26 (dd, J=12.3, 3.0 Hz, 1 H), 3.37-3.46 (m, 1 H), 3.50-3.54 (m, 1 H), 4.45 (s, 1 H), 7.20-7.24 (m, 1 H), 7.41-7.53 (m, 5 H), 7.64 (d, J=0.9 Hz, 1 H), 9.84 (s, 1 H).

### Example 471A 3'-chloro-4'-{[1-(5-hydroxy-2-adamantyl)-2-oxopyrrolidin-3-yl]methyl}biphenyl-4-carboxamide

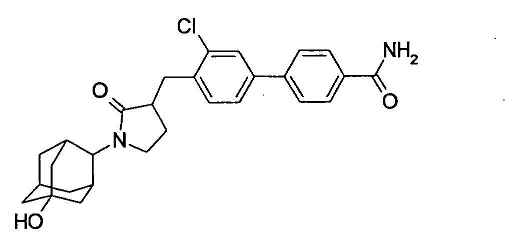

In the same manner as in Example 218A, the title compound was obtained from 3-(4-bromo-2-chlorobenzyl)-1-(5-hydroxy-2-adamantyl)pyrrolidin-2-one obtained in Example 441A and [4-(aminocarbonyl)phenyl]boronic acid.
1H NMR (300 MHz, DMSO-d₆) δ ppm 1.39-1.43 (m, 2 H), 1.62-1.73 (m, 10 H), 1.94-1.99 (m, 1 H), 2.04 (br, 1 H), 2.30 -2.36 (m, 2 H), 2.61-2.74 (m, 2 H), 3.04 (s, 3 H), 3.23-3.26 (m, 1 H), 3.41-3.52 (m, 1 H), 3.54-3.56 (m, 1 H), 3.72 (s, 1 H), 4.45 (s, 1H), 7.42 (s, 1 H), 7.46-7.49 (d, J=8.1 Hz, 1 H), 7.64-7.67 (d, J=7.8 Hz, 1 H), 7.77-7.80 (m, 2 H), 7.94-7.97 (d, J=8.1 Hz, 2 H), 8.05 (s, 1 H).

### Example 472A 3-(4-amino-2-chlorobenzyl)-1-(1,4-dioxaspiro[4.5]dec-8-yl)pyrrolidin-2-one

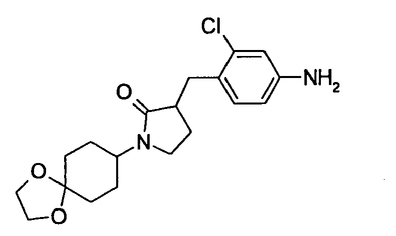

In the same manner as in Example 215A, the title compound was obtained from 3-(4-bromo-2-chlorobenzyl)-1-(1,4-dioxaspiro[4.5]dec-8-yl)pyrrolidin-2-one obtained in Example 203A.
1H NMR (300 MHz, CDCl₃) δ ppm 1.64-1.78 (m, 9 H), 1.95-2.04 (m, 1 H), 2.63-2.76 (m, 2 H), 3.15-3.27 (m, 3 H), 3.66 (br, 2 H), 3.94 (s, 4 H), 4.04 (m, 1 H), 6.48-6.52 (dd, J=8.4, 2.4 Hz, 1 H), 6.68-6.69 (d, J=2.4 Hz, 1 H), 7.00-7.03 (d, J=8.4 Hz, 1 H).

### Example 473A 3-(4-amino-2-chlorobenzyl)-1-(4-oxocyclohexyl)pyrrolidin-2-one

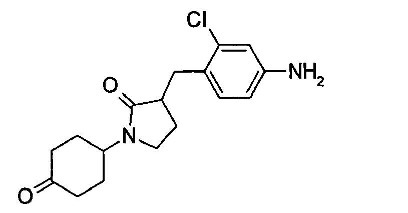

In the same manner as in Example 28A, the title compound was obtained from 3-(4-amino-2-chlorobenzyl)-1-(1,4-dioxaspiro[4.5]dec-8-yl)pyrrolidin-2-one obtained in Example 472A.
1H NMR (300 MHz, CDCl₃) δ ppm 1.67-1.91 (m, 4 H), 1.97-2.01 (m, 2 H), 2.41-2.58 (m, 4 H), 2.65-2.80 (m, 2 H), 3.15-3.20 (m, 2 H), 3.23-3.29 (dd, J=13.5, 3.9 Hz, 1 H), 3.66 (br, 1 H), 4.43-4.51 (m, 1 H), 6.49-6.52 (dd, J=8.1, 2.4 Hz, 1 H), 6.69-6.70 (d, J=2.4 Hz, 1 H), 7.01-7.03 (d, J=8.1 Hz, 1H).

### Example 474A N-(3-chloro-4-([2-oxo-1-(4-oxocyclohexyl)pyrrolidin-3-yl]methyl}phenyl)methanesulfonamide

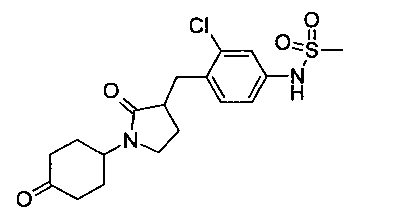

In the same manner as in Example 22A, the title compound was obtained from 3-(4-amino-2-chlorobenzyl)-1-(4-oxocyclohexyl)pyrrolidin-2-one obtained in Example 473A and methanesulfonyl chloride.
LC/MS (ESI+); m/z 399, 401 (M+H)⁺

### Example 475A N-(3-chloro-4-{[1-(4-hydroxy-4-methylcyclohexyl)-2-oxopyrrolidin-3-yl]methyl}phenyl)methanesulfonamide

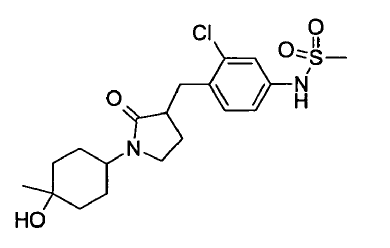

In the same manner as in Example 263A, the title compound was obtained from N-(3-chloro-4-{[2-oxo-1-(4-oxocyclohexyl)pyrrolidin-3-yl]methyl}phenyl)methanesulfonamide obtained in Example 474A and methyl lithium.
1H NMR (300 MHz, CDCl₃) δ ppm 1.24, 1.27 (each s, 3 H), 1.50-1.74 (m, 9 H), 2.05-2.09 (m, 2 H), 2.69-2.85 (m, 2 H), 3.00 (s, 3 H), 3.22-3.31 (m, 3 H), 3.93-3.98 (m, 1 H), 7.10-7.13 (dd, J=8.1, 2.1 Hz, 1 H), 7.21-7.23 (d, J=8.1 Hz, 1 H), 7.33 (d, J=2.1 Hz, 1 H), 8.37 (br, 1 H).

### Example 476A N-(3-chloro-4-{[2-oxo-1-(4-oxocyclohexyl)pyrrolidin-3-yl]methyl}phenyl)-2,2,2-trifluoroacetamide

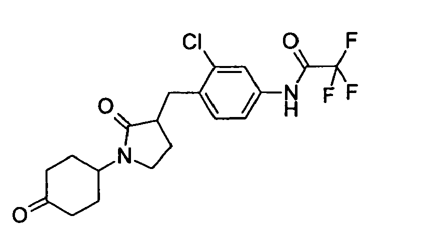

A mixture of 3-(4-amino-2-chlorobenzyl)-1-(4-oxocyclohexyl)pyrrolidin-2-one obtained in Example 473A (0.50 g), trifluoroacetic anhydride (0.357 g) and dichloromethane (20 mL) was stirred at room temperature for 16 hr. Water was added to the reaction solution, and the mixture was extracted with ethyl acetate. The ethyl acetate layer was washed with saturated brine, and dried over anhydrous magnesium sulfate, and the solvent was evaporated under reduced pressure to give the title compound (0.58 g, 89%) as a colorless solid.
LC/MS (ESI+); m/z 417, 419 (M+H)⁺

### Example 477A 3-(2,6-dichlorobenzyl)-1-(5-hydroxy-2-adamantyl)pyrrolidin-2-one (an optically active form, retention time 29 min)

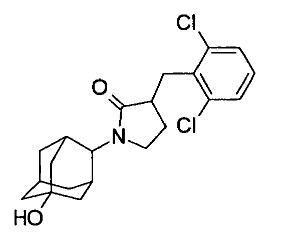

3-(2,6-Dichlorobenzyl)-1-(5-hydroxy-2-adamantyl)pyrrolidin-2-one (a mixture of two steric isomers) obtained in Example 429A was subjected to optical resolution with normal phase chiral high performance liquid chromatography (manufactured by Daicel Chemical Industries, Ltd., Chiralpak AS (trade name), 50 mmID X 500 mmL, hexane-2-propanol 85:15, retention time 29 min) to give the title compound.
LC/MS (ESI+); m/z 394, 396 (M+H)⁺

### Example 478A 3-(2,6-dichlorobenzyl)-1-(5-hydroxy-2-adamantyl)pyrrolidin-2-one (an optically active form, retention time 40 min)

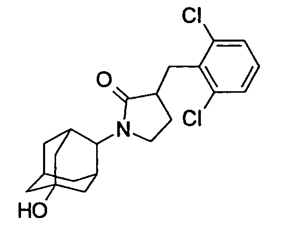

3-(2,6-Dichlorobenzyl)-1-(5-hydroxy-2-adamantyl)pyrrolidin-2-one (a mixture of two steric isomers) obtained in Example 429A was subjected to optical resolution with normal phase chiral high performance liquid chromatography (manufactured by Daicel Chemical Industries, Ltd., Chiralpak AS (trade name), 50 mmID X 500 mmL, hexane-2-propanol 85:15, retention time 40 min) to give the title compound.
LC/MS (ESI+); m/z 394, 396 (M+H)⁺

### Example 479A 3-(2-chloro-4-hydroxybenzyl)-1-(5-hydroxy-2-adamantyl)pyrrolidin-2-one (an optically active form, retention time for 20 min)

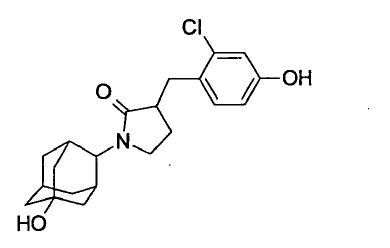

3-(2-Chloro-4-hydroxybenzyl)-1-(5-hydroxy-2-adamantyl)pyrrolidin-2-one (a mixture of two steric isomers) obtained in Example 431A was subjected to optical resolution with normal phase chiral high performance liquid chromatography (manufactured by Daicel Chemical Industries, Ltd., Chiralpak AS (trade name), 50 mmID X 500 mmL, hexane-2-propanol 75:25, retention time for 20 min) to give the title compound.
LC/MS (ESI+); m/z 376, 378 (M+H)⁺

### Example 480A 3-(2-chloro-4-hydroxybenzyl)-1-(5-hydroxy-2-adamantyl)pyrrolidin-2-one (an optically active form, retention time 45 min)

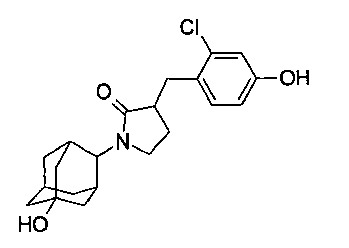

3-(2-Chloro-4-hydroxybenzyl)-1-(5-hydroxy-2-adamantyl)pyrrolidin-2-one (a mixture of two steric isomers) obtained in Example 431A was subjected to optical resolution with normal phase chiral high performance liquid chromatography (manufactured by Daicel Chemical Industries, Ltd., Chiralpak AS (trade name), 50 mmID X 500 mmL, hexane-2-propanol 75:25, retention time 45 min) to give the title compound.
LC/MS (ESI+); m/z 376, 378 (M+H)⁺

### Example 481A N-(3-chloro-4-{[1-(5-hydroxy-2-adamantyl)-2-oxopyrrolidin-3-yl]methyl}phenyl)methanesulfonamide (an optically active form, retention time 32 min)

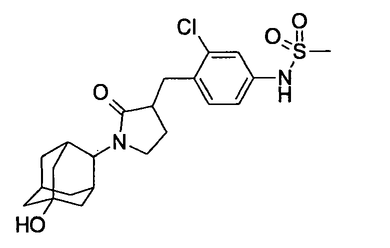

N-(3-Chloro-4-{[1-(5-hydroxy-2-adamantyl)-2-oxopyrrolidin-3-yl]methyl}phenyl)methanesulfonamide (a mixture of two steric isomers) obtained in Example 443A was subjected to optical resolution normal phase chiral high performance liquid chromatography (manufactured by Daicel Chemical Industries, Ltd., chiralpak AD (trade name), 50 mmID X 500 mmL, hexane-ethanol 50:50, retention time 32 min) to give the title compound.
LC/MS (ESI+); m/z 453, 455 (M+H)⁺

### Example 482A N-(3-chloro-4-{[1-(5-hydroxy-2-adamantyl)-2-oxopyrrolidin-3-yl]methyl}phenyl)methanesulfonamide (an optically active form, retention time 39 min)

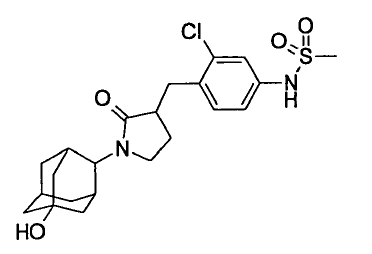

N-(3-Chloro-4-{[1-(5-hydroxy-2-adamantyl)-2-oxopyrrolidin-3-yl]methyl}phenyl)methanesulfonamide (a mixture of two steric isomers) obtained in Example 443A was subjected to optical resolution normal phase chiral high performance liquid chromatography (manufactured by Daicel Chemical Industries, Ltd., chiralpak AD (trade name), 50 mmID X 500 mmL, hexane-ethanol 50:50, retention time 39 min) to give the title compound.
LC/MS (ESI+); m/z 453, 455 (M+H)⁺

### Example 483A 4-({[(3-chloro-4-{[1-(5-hydroxy-2-adamantyl)-2-oxopyrrolidin-3-yl]methyl}phenyl)amino]carbonyl}amino)benzamide

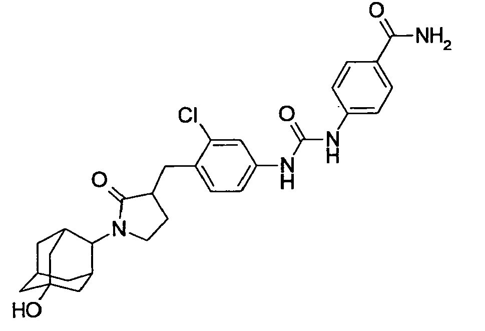

In the same manner as in Example 70A, the title compound was obtained from 4-({[(3-chloro-4-{[1-(5-hydroxy-2-adamantyl)-2-oxopyrrolidin-3-yl]methyl}phenyl)amino]carbonyl}amino)benzoic acid obtained in Example 469A and ammonium 1H-1,2,3-benzotriazol-1-ol.
1H NMR (300 MHz, CD₃OD)δ ppm 1.51-1.55 (m, 2 H), 1.75-1.86 (m, 10 H), 1.96-2.04 (m, 1 H), 2.13 (br, 1 H), 2.36-2.41 (m, 2 H), 2.64-2.69 (dd, J=9.6, 3.9 Hz, 1 H), 2.79-2.83 (m, 1 H), 3.25-3.31 (m, 1 H), 3.52-3.61 (m, 2 H), 3.88 (s, 1 H), 4.89 (m, 4 H), 7.22 (m, 2 H), 7.52-7.55 (d, J=8.7 Hz, 2 H), 7.64 (s, 1 H), 7.81-7.83 (d, J=9.0 Hz, 2 H).

### Example 484A N-(1-{[(3-chloro-4-{[1-(5-hydroxy-2-adamantyl)-2-oxopyrrolidin-3-yl]methyl}phenyl)amino]carbonyl}cyclopropyl)benzamide

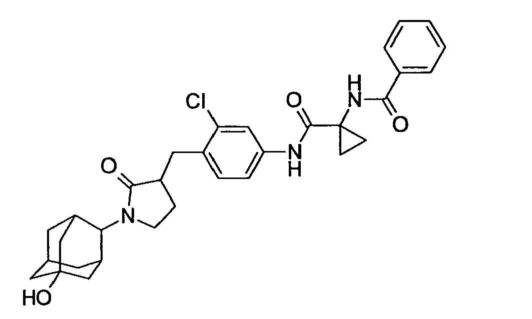

In the same manner as in Example 22A, the title compound was obtained from 1-amino-N-(3-chloro-4-1[1-(5-hydroxy-2-adamantyl)-2-oxopyrrolidin-3-yl]methyl}phenyl)cyclopropanecarboxamide hydrochloride obtained in Example 462A and benzoyl chloride.
1H NMR (300 MHz, CDCl₃) δ ppm 1.16-1.19 (m, 2 H), 1.47-1.51 (m, 4 H), 1.59-1.89 (m, 10 H), 1.93-2.01 (m, 1 H), 2.15 (m, 1 H), 2.38-2.41 (m, 2 H), 2.59-2.72 (m, 2 H), 3.21-3.26 (dd, J=12.9, 3.3 Hz, 1 H), 3.38-3.46 (q, J=8.7 Hz, 1 H), 3.47-3.54 (m, 1 H), 3.87 (s, 1 H), 7.03-7.06 (d, J=8.4 Hz, 1 H), 7.18-7.21 (m, 1 H), 7.39-7.44 (m, 1 H), 7.50-7.54 (m, 2 H), 7.86-7.92 (m, 3 H), 8.98 (s, 1 H).

### Example 485A N-(3-chloro-4-{[1-(5-hydroxy-2-adamantyl)-2-oxopyrrolidin-3-yl]methyl}phenyl)-1-[(phenylsulfonyl)amino]cyclopropanecarboxamide

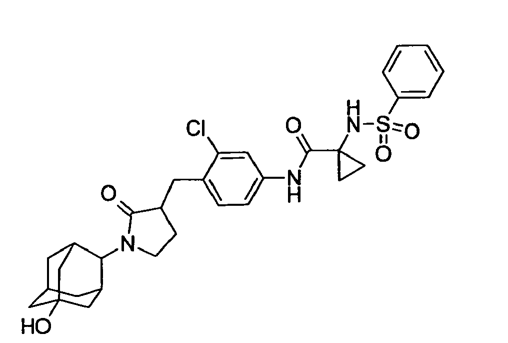

In the same manner as in Example 22A, the title compound was obtained from 1-amino-N-(3-chloro-4-{[1-(5-hydroxy-2-adamantyl)-2-oxopyrrolidin-3-yl]methyl}phenyl)cyclopropanecarboxamide hydrochloride obtained in Example 462A and phenylsulfonyl chloride.
1H NMR (300 MHz, CDCl₃) δ ppm 0.94-0.98 (m, 2 H), 1.44-1.54 (m, 4 H), 1.62-1.76 (m, 8 H), 1.89-1.92 (m, 2 H), 1.98-2.01 (m, 1 H), 2.17 (br, 1 H), 2.43 (m, 2 H), 2.67-2.82 (m, 2 H), 3.27-3.32 (dd, J=12.9, 3.6 Hz, 1 H), 3.44-3.56 (m, 2 H), 3.93 (s, 1 H), 7.09-7.21 (m, 3 H), 7.46-7.55 (m, 4 H), 7.86-7.89 (d, J=7.2 Hz, 2 H), 8.83 (s, 1 H).

### Example 486A 3-(2-chloro-4-hydroxybenzyl)-1-[3-(hydroxymethyl)-1-adamantyl]pyrrolidin-2-one

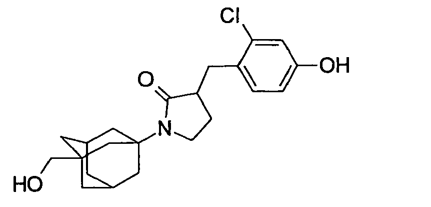

To a mixture of 1-[3-(hydroxymethyl)-1-adamantyl]pyrrolidin-2-one obtained in Reference Example 19A (4.0 g) and tetrahydrofuran (40 mL) was added lithium diisopropylamide (1.8 M tetrahydrofuran solution, 19.6 mL) at -78°C under nitrogen atmosphere, and the mixture was stirred for 1 hr. N,N,N',N',N",N"-Hexamethylphosphoric triamide (15 mL) was added, and the mixture was stirred for 30 min. A solution of 2-chloro-4-(methoxymethoxy)benzyl bromide obtained in Reference Example 24A (4.25 g) in tetrahydrofuran (3 mL) was added to the obtained solution, and the mixture was further stirred at -78°C for 1 hr, and then at -40°C for 16 hr. Water was added to the reaction mixture, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 9:1-1:1) to give a colorless oil (1.9 g). Then, a mixture of the colorless oil (1.9 g), 6N hydrochloric acid (10 mL), tetrahydrofuran (20 mL) and methanol (20 mL) was stirred at 60°C for 3 hr. The reaction solution was extracted with ethyl acetate. The ethyl acetate layer was washed with saturated brine, and dried over anhydrous magnesium sulfate. The solvent was evaporated under reduced pressure. The residue was triturated with hexane-ethyl acetate to give the title compound (1.1 g, 18%) as a colorless powder.
1H NMR NMR (300 MHz, CDCl₃) δ ppm 1.42-1.71 (m, 10 H), 1.88-2.05 (m, 7 H), 2.19 (m, 2 H), 2.66-2.76 (m, 2 H), 3.18-3.21 (m, 1 H), 3.27 (s, 2 H), 3.29-3.35 (m, 1 H), 6.67-6.70 (dd, J=8.7, 2.4 Hz, 1 H), 6.88 (d, J=2.4 Hz, 1 H), 7.04-7.07 (d, J=8.7 Hz, 1 H).

### Example 487A N-(3-chloro-4-{[1-(5-hydroxy-2-adamantyl)-2-oxopyrrolidin-3-yl]methyl}phenyl)-1,1,1-trifluoromethanesulfonamide

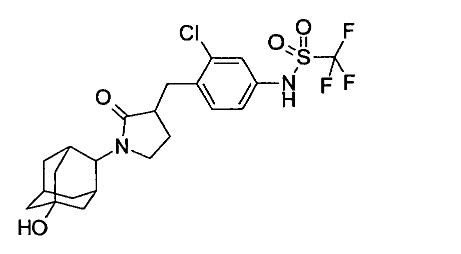

In the same manner as in Example 22A, the title compound was obtained from 3-(4-amino-2-chlorobenzyl)-1-(5-hydroxy-2-adamantyl)pyrrolidin-2-one obtained in Example 442A and trifluoromethanesulfonyl chloride.
1H NMR (300 MHz, CDCl₃)δ ppm 1.51-1.55 (m, 2 H), 1.76-1.86 (m, 10 H), 2.02-2.07 (m, 1 H), 2.13 (m, 1 H), 2.37-2.41 (m, 2 H), 2.73-2. 84 (m, 2 H), 3.30-3.32 (m, 1 H), 3.57-3.60 (m, 2 H), 3.89 (s, 1 H), 7.14-7.21 (m, 2 H), 7.32-7.36 (m, 2 H).

### Example 488A N-(3-chloro-4-{[1-(5-hydroxy-2-adamantyl)-2-oxopyrrolidin-3-yl]methyl}phenyl)-4-(trifluoromethyl)benzamide

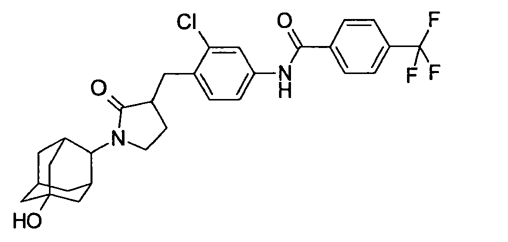

In the same manner as in Example 22A, the title compound was obtained from 3-(4-amino-2-chlorobenzyl)-1-(5-hydroxy-2-adamantyl)pyrrolidin-2-one obtained in Example 442A and 4-(trifluoromethyl)benzoyl chloride.
1H NMR (300 MHz, CDCl₃) δ ppm 1.47-1.51 (m, 2 H), 1.69-1.86 (m, 9 H), 1.97-2.09 (m, 1 H), 2.15 (br, 1 H), 2.35-2.40 (m, 2 H), 2.48 (m, 1 H), 2.65-2.74 (m, 2 H), 3.18-3.27 (q, J=9.6 Hz, 1 H), 3.42-3.50 (m, 1 H), 3.53-3.60 (m, 1 H), 3.87 (s, 1 H), 7.12-7.15 (d, J=9.0 Hz, 1 H), 7.50-7.53 (dd, J=8.4; 2.1 Hz, 1 H), 7.63-7.66 (d, J=8.4 Hz, 2 H), 7.81 (d, J=2.1 Hz, 1 H), 8.00-8.02 (d, J=8.4 Hz, 2 H), 9.43 (s, 1 H).

### Example 489A N-(3-chloro-4-{[1-(5-hydroxy-2-adamantyl)-2-oxopyrrolidin-3-yl]methyl}phenyl)-4-(trifluoromethyl)benzenesulfonamide

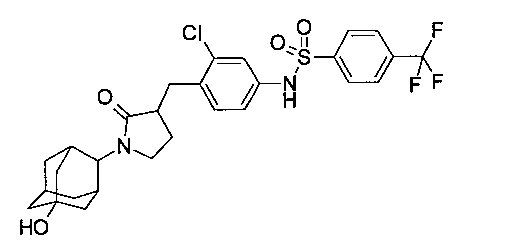

In the same manner as in Example 22A, the title compound was obtained from 3-(4-amino-2-chlorobenzyl)-1-(5-hydroxy-2-adamantyl)pyrrolidin-2-one obtained in Example 442A and 4-(trifluoromethyl)benzenesulfonyl chloride.
1H NMR (300 MHz, CDCl₃) δ ppm 1.49-1.53 (m, 2 H), 1.66-1.76 (m, 8 H), 1.88-1.96 (m, 3 H), 2.17 (br, 1 H), 2.39-2.42 (m, 2 H), 2.66-2.81 (m, 2 H), 3.19-3.25 (dd, J=12.9, 4.5 Hz, 1 H), 3.44-3.59 (m, 2 H), 3.93 (s, 1 H), 6.93-6.96 (dd, J=8.1, 2.1 Hz, 1 H), 7.10-7.13 (d, J=8.1 Hz, 1 H), 7.17-7.18 (d, J=2.1 Hz, 1 H), 7.66-7.00 (m, 2 H), 7.90-7.93 (d, J=8.1 Hz, 2 H), 8.53 (br, 1 H).

### Example 490A 4-(aminosulfonyl)-N-(3-chloro-4-{[1-(5-hydroxy-2-adamantyl)-2-oxopyrrolidin-3-yl]methyl}phenyl)benzamide

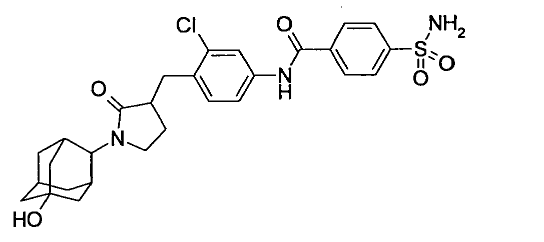

In the same manner as in Example 68A, the title compound was obtained from 3-(4-amino-2-chlorobenzyl)-1-(5-hydroxy-2-adamantyl)pyrrolidin-2-one obtained in Example 442A and 4-(aminosulfonyl)benzoic acid.
LC/MS (ESI+); m/z 558, 560 (M+H)⁺

### Example 491A tert-butyl 4-{[(3-chloro-4-{[1-(5-hydroxy-2-adamantyl)-2-oxopyrrolidin-3-yl]methyl}phenyl)amino]carbonyl}piperidine-1-carboxylate

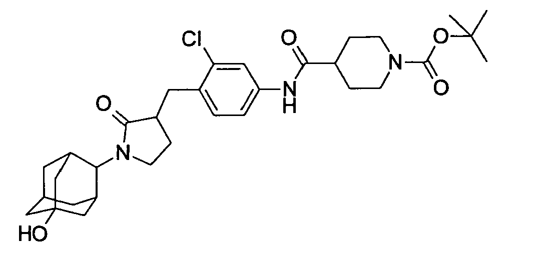

In the same manner as in Example 68A, the title compound was obtained from 3-(4-amino-2-chlorobenzyl)-1-(5-hydroxy-2-adamantyl)pyrrolidin-2-one obtained in Example 442A and 1-(tert-butoxycarbonyl)piperidine-4-carboxylic acid.
LC/MS (ESI+); m/z 530, 532 (M-55)⁺

### Example 492A N-(3-chloro-4-{[1-(5-hydroxy-2-adamantyl)-2-oxopyrrolidin-3-yl]methyl}phenyl)piperidine-4-carboxamide hydrochloride

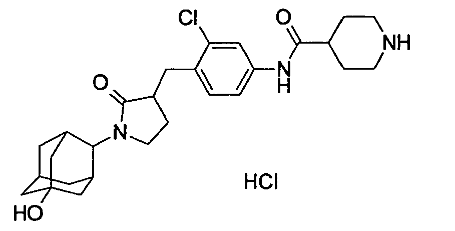

In the same manner as in Example 20A, the title compound was obtained from tert-butyl 4-{[(3-chloro-4-{[1-(5-hydroxy-2-adamantyl)-2-oxopyrrolidin-3-yl]methyl}phenyl)amino]carbonyl}piperidine-1-carboxylate obtained in Example 491A.
LC/MS (ESI+); m/z 486, 488 (M+H)⁺

### Example 493A N-(3-chloro-4-{[1-(5-hydroxy-2-adamantyl)-2-oxopyrrolidin-3-yl]methyl}phenyl)-1-[4-(trifluoromethyl)benzyl]piperidine-4-carboxamide

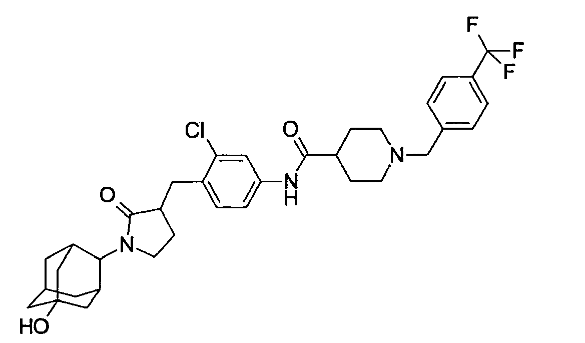

In the same manner as in Example 64A, the title compound was obtained from N-(3-chloro-4-{[1-(5-hydroxy-2-adamantyl)-2-oxopyrrolidin-3-yl]methyl}phenyl)piperidine-4-carboxamide hydrochloride obtained in Example 492A and 1-(bromomethyl)-4-(trifluoromethyl)benzene.
1H NMR (300 MHz, CDCl₃) δ ppm 1.49-1.53 (m, 2 H), 1.64 (m, 8 H), 1.75 (m, 4 H), 1.89-1.92 (m, 5 H), 2.05 (m, 1 H), 2.17 (br, 1 H), 2.45 (m, 2 H), 2.73-2.77 (m, 2 H), 2.88-2.96 (m, 2 H), 3.29 (m, 1 H), 3.41-3.54 (m, 2 H), 3.56 (s, 2 H), 3.90 (s, 1 H), 7.14 (m, 1 H), 7.19-7.25 (m, 2 H), 7.43-7.46 (d, J=8.1 Hz, 2 H), 7.55-7.58 (d, J=8.4 Hz, 2 H), 7.68 (d, J=2.1 Hz, 1 H).

### Example 494A N-(3-chloro-4-{[1-(5-hydroxy-2-adamantyl)-2-oxopyrrolidin-3-yl]methyl}phenyl)-N-methyl-4-(trifluoromethyl)benzamide

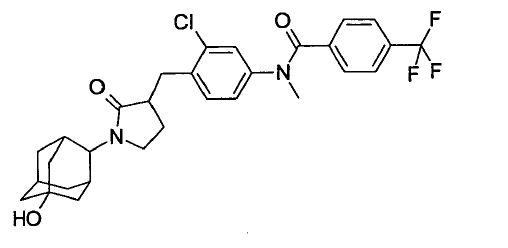

In the same manner as in Reference Example 1A, the title compound was obtained from N-(3-chloro-4-{[1-(5-hydroxy-2-adamantyl)-2-oxopyrrolidin-3-yl]methyl}phenyl)-4-(trifluoromethyl)benzamide obtained in Example 488A and methyl iodide.
1H NMR (300 MHz, CDCl₃) δ ppm 1.50-1.57 (m, 2 H), 1.76-1.82 (m, 8 H), 1.89-1.93 (m, 3 H), 2.18 (br, 1 H), 2.43 (m, 2 H), 2.70-2.75 (m, 2 H), 3.27-3.42 (q, J=8.7 Hz, 1 H), 3.42-3.53 (m, 2 H), 3.47 (s, 3 H), 3.90 (s, 1 H), 6.81-6.83 (d, J=8.1 Hz, 1 H), 7.11-7.15 (m, 2 H), 7.39-7.48 (m, 4 H).

### Example 495A N-(3-chloro-4-{[1-(5-hydroxy-2-adamantyl)-2-oxopyrrolidin-3-yl]methyl}phenyl)-N'-[4-(trifluoromethyl)phenyl]urea

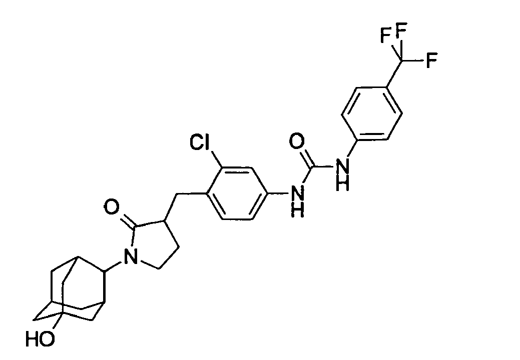

In the same manner as in Example 210A, the title compound was obtained from 3-(4-amino-2-chlorobenzyl)-1-(5-hydroxy-2-adamantyl)pyrrolidin-2-one obtained in Example 442A and ethyl 1-isocyanato-4-(trifluoromethyl)benzene.
1H NMR (300 MHz, CDCl₃) δ ppm 1.47-1.51 (m, 2 H), 1.68-1.76 (m, 8 H), 1.80 (m, 1 H), 1.85-1.87 (m, 2 H), 2.15 (br, 1 H), 2.38 (m, 2 H), 2.71-2.81 (m, 2 H), 3.19-3.21 (m, 1 H), 3.46-3.56 (m, 2 H), 3.91 (s, 1 H), 7.05-7.16 (m, 3 H), 7.45-7.55 (m, 4 H), 7.97-8.00 (d, J=8.4 Hz, 1 H), 8.57 (s, 1 H).

### Example 496A N-(3-chloro-4-{[1-(5-hydroxy-2-adamantyl)-2-oxopyrrolidin-3-yl]methyl}phenyl)-N'-[3-(trifluoromethyl)phenyl]urea

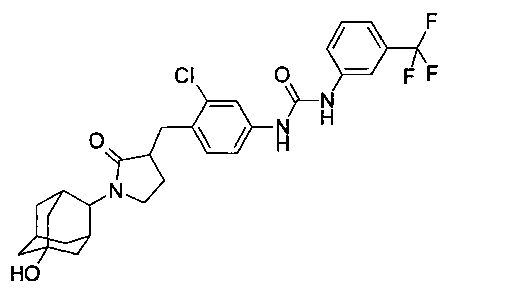

In the same manner as in Example 210A, the title compound was obtained from 3-(4-amino-2-chlorobenzyl)-1-(5-hydroxy-2-adamantyl)pyrrolidin-2-one obtained in Example 442A and ethyl 1-isocyanato-3-(trifluoromethyl)benzene.
1H NMR (300 MHz, CD₃OD) δ ppm 1.51-1.55 (m, 2 H), 1.74-1.86 (m, 10 H), 1.99-2.06 (m, 1 H), 2.13 (br, 1 H), 2.36-2.41 (m, 2 H), 2.64-2.72 (m, 1 H), 2.79-2.83 (m, 1 H), 3.25-3.32 (m, 1 H), 3.51-3.61 (m, 2 H), 3.89 (s, 1 H), 7.15-7.33 (m, 5 H), 7.42-7.47 (t, J=7.5 Hz, 1 H), 7.57-7.60 (d, J=7.5 Hz, 1 H), 7.63 (s, 1 H), 7.86 (s, 1 H).

### Example 497A N-(3-chloro-4-{[1-(5-hydroxy-2-adamantyl)-2-oxopyrrolidin-3-yl]methyl}phenyl)-2-(pyridin-3-yl)acetamide

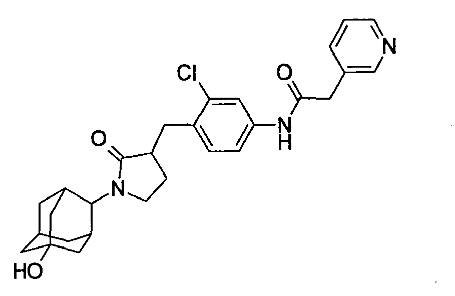

In the same manner as in Example 68A, the title compound was obtained from 3-(4-amino-2-chlorobenzyl)-1-(5-hydroxy-2-adamantyl)pyrrolidin-2-one obtained in Example 442A and (pyridin-3-yl)acetic acid.
1H NMR (300 MHz, CD₃OD) δ ppm 1.49-1.54 (m, 2 H), 1.69-1.88 (m, 10 H), 1.97-2.07 (m, 1 H), 2.13 (m, 1 H), 2.35-2.39 (m, 2 H), 2.65-2.83 (m, 2 H), 3.25-3.34 (m, 1 H), 3.48-3.63 (m, 2 H), 3.74 (s, 2 H), 3.87 (s, 1 H), 7.23-7.25 (d, J=8.1 Hz, 1 H), 7.34-7.45 (m, 2 H), 7.74 (d, J=2.1 Hz, 1 H), 7.82-7.84 (d, J=7.8 Hz, 1 H), 8.43 (m, 1 H), 8.50 (s, 1 H).

### Example 498A 3-{[3-chloro-3'-(trifluoromethyl)biphenyl-4-yl]methyl}-1-(5-hydroxy-2-adamantyl)pyrrolidin-2-one

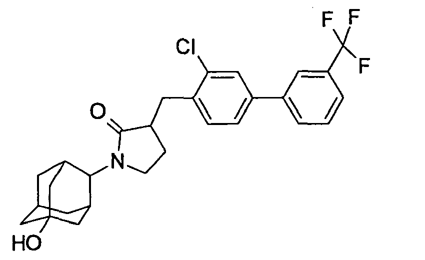

In the same manner as in Example 218A, the title compound was obtained from 3-(4-bromo-2-chlorobenzyl)-1-(5-hydroxy-2-adamantyl)pyrrolidin-2-one obtained in Example 441A and [3-(trifluoromethyl)phenyl]boronic acid.
1H NMR (300 MHz, CDCl₃) δ ppm 1.51-1.55 (m, 2 H), 1.71-1.80 (m, 8 H), 1.92-1.96 (m, 2 H), 2.07-2.15 (m, 1 H), 2.18 (br, 2 H), 2.44-2.48 (m, 2 H), 2.82-2.89 (m, 2 H), 3.36-3.51 (m, 2 H), 3.54-3.60 (m, 1 H), 3.95 (s, 1 H), 7.36-7.43 (m, 2 H), 7.49-7.63 (m, 3 H), 7.71-7.73 (d, J=7.5 Hz, 1 H), 7.78 (s, 1 H).

### Example 499A N-(3-chloro-4-{[1-(5-hydroxy-2-adamantyl)-2-oxopyrrolidin-3-yl]methyl}phenyl)-N'-[4-(trifluoromethoxy)phenyl]urea

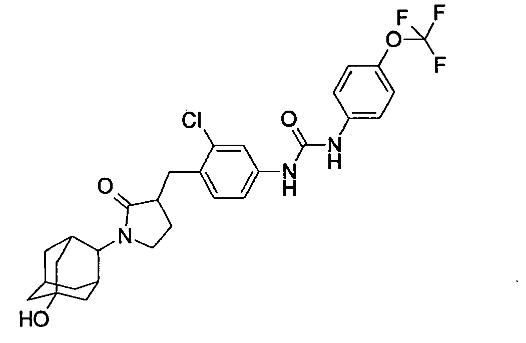

In the same manner as in Example 210A, the title compound was obtained from 3-(4-amino-2-chlorobenzyl)-1-(5-hydroxy-2-adamantyl)pyrrolidin-2-one obtained in Example 442A and 1-isocyanato-4-(trifluoromethoxy)benzene.
1H NMR (300 MHz, DMSO-d₆) δ ppm 1.38-1.42 (m, 2 H), 1.60-1.73 (m, 9 H), 1.90-1.93 (m, 1 H), 2.04 (br, 1 H), 2.28-2.34 (m, 2 H), 2.49-2.53 (m, 1 H), 2.58-2.66 (m, 1 H), 3.11-3.16 (dd, J=13.5, 3.6 Hz, 1 H), 3.37-3.45 (q, J=7.2 Hz, 1 H), 3.48-3.50 (m, 2 H), 3.71 (s, 1H), 4.44 (d, J=1.5 Hz, 1H), 7.19-7.33 (m, 4 H), 7.53-7.56 (m, 2 H), 7.69 (d, J=1.8 Hz, 1 H), 8.87 (s, 1 H), 8.95 (s, 1H).

### Example 500A tert-butyl {2-[(3-chloro-4-{[1-(5-hydroxy-2-adamantyl)-2-oxopyrrolidin-3-yl]methyl}phenyl)amino]-2-oxoethyl}carbamate

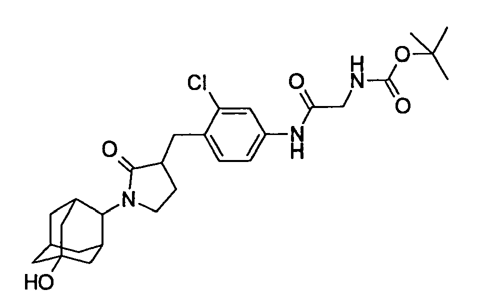

In the same manner as in Example 68A, the title compound was obtained from 3-(4-amino-2-chlorobenzyl)-1-(5-hydroxy-2-adamantyl)pyrrolidin-2-one obtained in Example 442A and N-(tert-butoxycarbonyl)glycine.
1H NMR (300 MHz, CDCl₃) δ ppm 1.42-1.53 (m, 2 H), 1.47 (s, 9 H), 1.68-1.93 (m, 10 H), 1.99 -2.05 (m, 1 H), 2.17 (br, 1 H), 2.41-2.45 (m, 2 H), 2.71-2.79 (m, 2 H), 3.28-3.31 (q, J=9.0 Hz, 1 H), 3.41-3.56 (m, 2 H), 3.91-3.93 (m, 3 H), 5.41 (m, 1 H), 7.17-7.20 (m, 1 H), 7.26-7.30 (m, 1 H), 7.64-7.65 (d, J=2.1 Hz, 1 H), 8.56 (br, 1 H).

### Example 501A N-(3-chloro-4-{[1-(5-hydroxy-2-adamantyl)-2-oxopyrrolidin-3-yl]methyl}phenyl)glycinamide hydrochloride

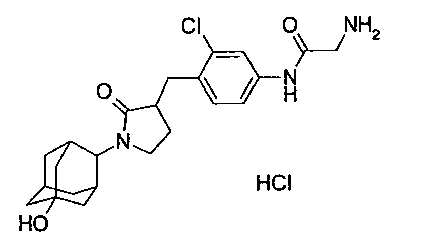

In the same manner as in Example 20A, the title compound was obtained from tert-butyl {2-[(3-chloro-4-{[1-(5-hydroxy-2-adamantyl)-2-oxopyrrolidin-3-yl]methyl}phenyl)amino]-2-oxoethyl}carbamate obtained in Example 500A.
LC/MS (ESI+); m/z 432, 434 (M+H)⁺

### Example 502A 1-(3-chloro-4-{[1-(5-hydroxy-2-adamantyl)-2-oxopyrrolidin-3-yl]methyl}phenyl)-3-[(pyridin-2-yl)methyl]imidazolidin-2-one

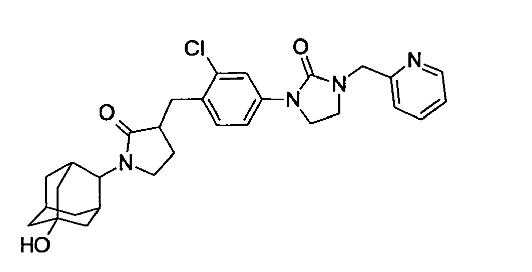

In the same manner as in Reference Example 1A, the title compound was obtained from 1-(3-chloro-4-{[1-(5-hydroxy-2-adamantyl)-2-oxopyrrolidin-3-yl]methyl}phenyl)imidazolidin-2-one obtained in Example 444A and 2-(bromomethyl)pyridine hydrobromide.
1H NMR (300 MHz, CDCl₃) δ ppm 1.45-1.53 (m, 2 H), 1.68-1.82 (m, 8 H), 1.90-1.93 (m, 2 H), 1.96-2.06 (m, 1 H), 2.17 (br, 1 H), 2.42-2.47 (m, 2 H), 2.70-2.79 (m, 2 H), 3.30-3.33 (q, J=9.3 Hz, 1 H), 3.42-3.45 (m, 1 H), 3.48-3.56 (m, 3 H), 3.78-3.83 (m, 2 H), 3.91 (s, 1 H), 4.60 (s, 2 H), 7.15-7.22 (m, 2 H), 7.34-7.47 (m, 2 H), 7.64-7.71 (m, 2 H), 8.54-8.56 (m, 1 H).

### Example 503A N-{2-[(3-chloro-4-{[1-(5-hydroxy-2-adamantyl)-2-oxopyrrolidin-3-yl]methyl}phenyl)amino]-2-oxoethyl}benzamide

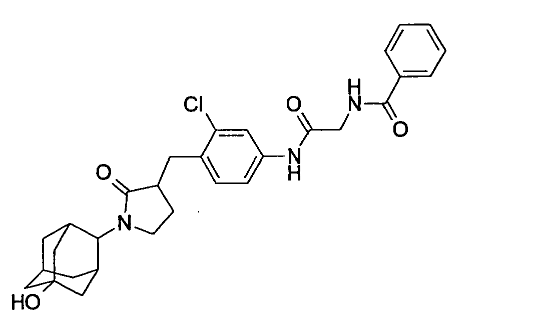

In the same manner as in Example 22A, the title compound was obtained from N-(3-chloro-4-{[1-(5-hydroxy-2-adamantyl)-2-oxopyrrolidin-3-yl]methyl}phenyl)glycinamide hydrochloride obtained in Example 501A and benzoyl chloride.
1H NMR (300 MHz, CD₃OD) δ ppm 1.51-1.55 (m, 2 H), 1.74-1.89 (m, 10 H), 2.01-2.08 (m, 1 H), 2.13 (br, 1 H), 2.36-2.41 (m, 2 H), 2.66-2.74 (m, 1 H), 2.80-2.84 (m, 1 H), 3.19-3.30 (m, 1 H), 3.47-3.61 (m, 2 H), 3.88 (s, 1 H), 4.18 (s, 1 H), 7.18-7.26 (m, 1 H), 7.36-7.40 (dd, J =8.1, 2.1 Hz, 1 H), 7.44-7.58 (m, 4 H), 7.76 (d, J=2.1 Hz, 1 H), 7.87-7.90 (m, 2 H).

### Example 504A N-(3-chloro-4-{[1-(5-hydroxy-2-adamantyl)-2-oxopyrrolidin-3-yl]methyl}phenyl)-2-phenylacetamide

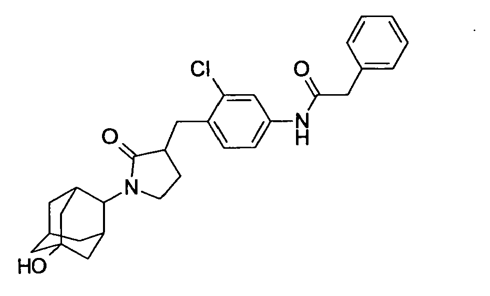

In the same manner as in Example 22A, the title compound was obtained from 3-(4-amino-2-chlorobenzyl)-1-(5-hydroxy-2-adamantyl)pyrrolidin-2-one obtained in Example 442A and phenylacetyl chloride.
1H NMR (300 MHz, CD₃OD) δ ppm 1.49-1.54 (m, 2 H), 1.72-1.89 (m, 10 H), 1.94-2.05 (m, 1 H), 2.14 (m, 1 H), 2.35-2.39 (m, 2 H), 2.65-2.82 (m, 2 H), 3.25-3.32 (m, 1 H), 3.50-3.60 (m, 2 H), 3.64 (s, 2 H), 3.87 (s, 1 H), 4.85 (m, 1 H), 7.20-7.37 (m, 7 H), 7.74 (d, J=2.1 Hz, 1 H).

### Example 505A N-(3-chloro-4-{[1-(5-hydroxy-2-adamantyl)-2-oxopyrrolidin-3-yl] methyl}phenyl)acetamide

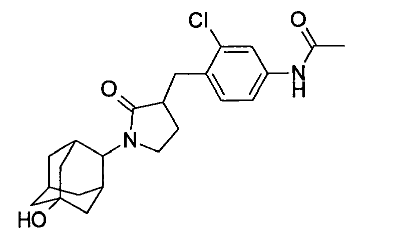

In the same manner as in Example 22A, the title compound was obtained from 3-(4-amino-2-chlorobenzyl)-1-(5-hydroxy-2-adamantyl)pyrrolidin-2-one obtained in Example 442A and acetyl chloride.
1H NMR (300 MHz, CD₃OD) δ ppm 1.51-1.55 (m, 2 H), 1.72-1.79 (m, 6 H), 1.86-1.89 (m, 4 H), 1.98-2.05 (m, 1 H), 2.16 (s, 3 H), 2.36 (br, 1 H), 2.36-2.40 (m, 2 H), 2.65-2.72 (m, 1 H), 2.79-2.83 (m, 1H), 3.25-3.30 (m, 1H), 3.51-3.61 (m, 2 H), 3.88 (s, 1H), 4.87 (s, 1 H), 7.21-7.24 (d, J=8.4 Hz, 1 H), 7.31-7.35 (dd, J=8.4, 2.1 Hz, 1 H), 7.73 (d, J=2.1 Hz, 1 H).

### Example 506A 3-(4-bromo-2-chlorobenzyl)-1-[3-(hydroxymethyl)-1-adamantyl]pyrrolidin-2-one

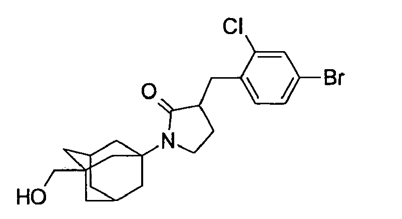

In the same manner as in Example 255A, the title compound was obtained from 1-[3-(hydroxymethyl)-1-adamantyl]pyrrolidin-2-one obtained in Reference Example 19A and 4-bromo-1-(bromomethyl)-2-chlorobenzene.
LC/MS (ESI+); m/z 454, 456 (M+H)⁺

### Example 507A N-[3'-chloro-4'-((1-[3-(hydroxymethyl)-1-adamantyl]-2-oxopyrrolidin-3-yl}methyl)biphenyl-3-yl]methanesulfonamide

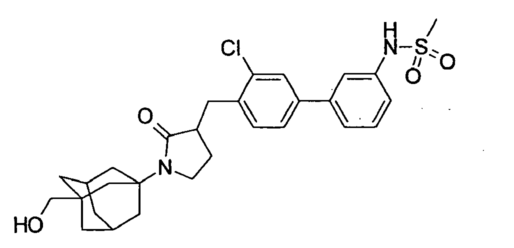

In the same manner as in Example 218A, the title compound was obtained from 3-(4-bromo-2-chlorobenzyl)-1-[3-(hydroxymethyl)-1-adamantyl]pyrrolidin-2-one obtained in Example 506A and N-[3-(4,4,5,5-tetramethyl-1,3,2-dioxaboloran-2-yl)phenyl]methanesulfonamide.
1H NMR NMR (300 MHz, CDCl₃) δ ppm 1.41-1.69 (m, 8 H), 1.89-2.09 (m, 8 H), 2.18 (m, 2 H), 2.73-2.79 (m, 2 H), 3.02 (s, 3 H), 3.26 (s, 2 H), 3.29-3.39 (m, 2 H), 7.25-7.38 (m, 4 H), 7.41-7.48 (m, 1 H), 7.51-7.54 (m, 1 H), 7.62-7.69 (m, 1 H), 7.98 (br, 1 H). Example 508A N-(3-chloro-4-{[1-(5-hydroxy-2-adamantyl)-2-oxopyrrolidin-3-yl]methyl}phenyl)-1-phenylmethanesulfonamide

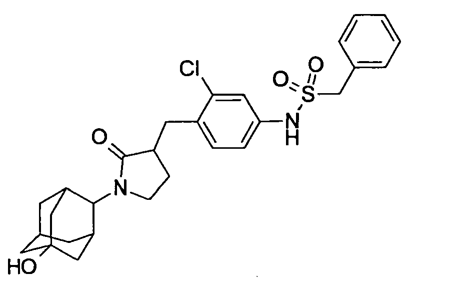

In the same manner as in Example 22A, the title compound was obtained from 3-(4-amino-2-chlorobenzyl)-1-(5-hydroxy-2-adamantyl)pyrrolidin-2-one obtained in Example 442A and phenylmethanesulfonyl chloride.
1H NMR (300 MHz, CDCl₃) δ ppm 1.49-1.53 (m, 2 H), 1.69-1.78 (m, 8 H), 1.88-1.91 (m, 2 H), 2.02-2.08 (m, 1 H), 2.17 (br, 1 H), 2.40-2.45 (m, 2 H), 2.70-2.78 (m, 2 H), 3.23-3.29 (q, J=8.7 Hz, 1 H), 3.41-3.57 (m, 2 H), 3.90 (s, 1 H), 4.32 (s, 2 H), 6.95-6.99 (dd, J=8.4, 2.1 Hz, 1 H), 7.15-7.48 (m, 8 H).

### Example 509A 1-benzyl-3-(3-chloro-4-{[1-(5-hydroxy-2-adamantyl)-2-oxopyrrolidin-3-yl]methyl}phenyl)imidazolidin-2-one

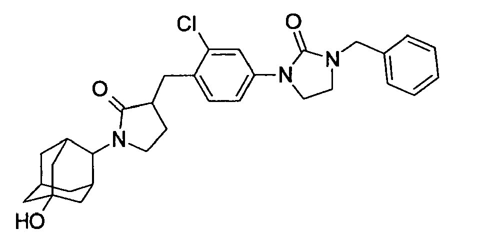

In the same manner as in Reference Example 1A, the title compound was obtained from 1-(3-chloro-4-{[1-(5-hydroxy-2-adamantyl)-2-oxopyrrolidin-3-yl]methyl}phenyl)imidazolidin-2-one obtained in Example 444A and benzyl bromide.
1H NMR (300 MHz, CDCl₃) δ ppm 1.45-1.53 (m, 2 H), 1.68-1.94 (m, 10 H), 1.97-2.04 (m, 1 H), 2.17 (br, 1 H), 2.42-2.46 (m, 2 H), 2.70-2.79 (m, 2 H), 3.30-3.55 (m, 5 H), 3.72-3.77 (t, J=7.2 Hz, 2 H), 3.92 (s, 1 H), 4.66 (s, 2 H), 7.19-7.41 (m, 7 H), 7.64 (d, J=2.1 Hz, 1 H).

### Example 510A 1-(3-chloro-4-{[1-(5-hydroxy-2-adamantyl)-2-oxopyrrolidin-3-yl]methyl}phenyl)-3-[4-(trifluoromethyl)benzyl]imidazolidin-2-one

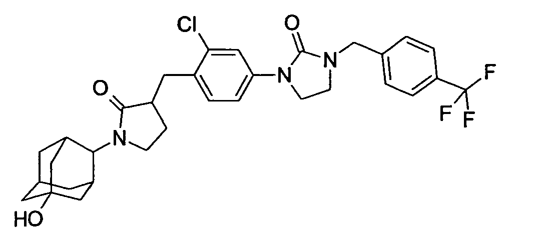

In the same manner as in Reference Example 1A, the title compound was obtained from 1-(3-chloro-4-{[1-(5-hydroxy-2-adamantyl)-2-oxopyrrolidin-3-yl]methyl}phenyl)imidazolidin-2-one obtained in Example 444A and 1-(bromomethyl)-4-(trifluoromethyl)benzene.
1H NMR (300 MHz, CDCl₃) δ ppm 1.50-1.54 (m, 2 H), 1.76-2.04 (m, 10 H), 2.17 (br, 1 H), 2.43-2.46 (m, 2 H), 2.74-2.79 (m, 2 H), 3.30-3.54 (m, 6 H), 3.76-3.82 (t, J=8.7 Hz, 2 H), 3.92 (s, 1 H), 4.52 (s, 2 H), 7.21-7.24 (d, J=8.7 Hz, 1 H), 7.37-7.44 (m, 3 H), 7.59-7.65 (m, 3 H).

### Example 511A 1-(3-chloro-4-{[1-(5-hydroxy-2-adamantyl)-2-oxopyrrolidin-3-yl]methyl}phenyl)-3-[(pyridin-3-yl)methyl]imidazolidin-2-one

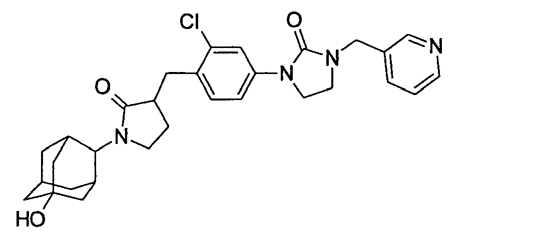

In the same manner as in Reference Example 1A, the title compound was obtained from 1-(3-chloro-4-{[1-(5-hydroxy-2-adamantyl)-2-oxopyrrolidin-3-yl]methyl}phenyl)imidazolidin-2-one obtained in Example 444A and 3-(chloromethyl)pyridine hydrochloride.
1H NMR (300 MHz, CDCl₃) δ ppm 1.49-1.53 (m, 2 H), 1.68-1.76 (m, 8 H), 1.90-1.94 (m, 2 H), 1.98-2.07 (m, 1 H), 2.16 (br, 1 H), 2.42-2.45 (m, 2 H), 2.70-2.79 (m, 2 H), 3.30-3.53 (m, 6 H), 3.77-3.81 (m, 2 H), 3.91 (s, 1 H), 4.48 (s, 2 H), 7.19-7.23 (m, 1 H), 7.28-7.30 (m, 1 H), 7.32-7.45 (m, 1 H), 7.63 (d, J=2.1 Hz, 1 H), 7.67-7.70 (m, 1 H), 8.55 (s, 2 H).

### Example 512A 3-(2,6-dichlorobenzyl)-1-[4-(pyrrolidin-1-yl)cyclohexyl]pyrrolidin-2-one

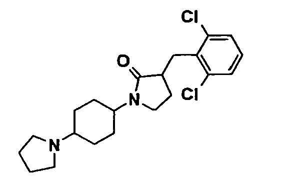

In the same manner as in Example 371A, the title compound was obtained from 3-(2,6-dichlorobenzyl)-1-(4-oxocyclohexyl)pyrrolidin-2-one obtained in Example 28A and pyrrolidine.
1H NMR (300 MHz, CDCl₃) δ ppm 1.34-1.62 (m, 4 H), 1.68-2.18 (m, 11 H), 2.33-2.65 (m, 4 H), 2.82-3.10 (m, 2 H), 3.11-3.57 (m, 3 H), 3.90-4.08 (m, 1 H), 7.09 (t, J=8.1 Hz, 1 H), 7.24-7.33 (m, 2 H).

### Example 513A 3-(2,6-dichlorobenzyl)-1-(4-ethyl-4-hydroxycyclohexyl)pyrrolidin-2-one (less polar product) and Example 514A 3-(2,6-dichlorobenzyl)-1-(4-ethyl-4-hydroxycyclohexyl)pyrrolidin-2-one (more polar product)

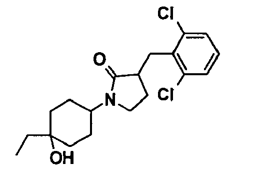

The reaction product (a mixture of four steric isomers) which was obtained in the same manner as in Example 30A from 3-(2,6-dichlorobenzyl)-1-(4-oxocyclohexyl)pyrrolidin-2-one obtained in Example 28A and ethylmagnesium bromide, was subjected to silica gel column chromatography (hexane-ethyl acetate 4:1, ethyl acetate) to give the title compound (Example 513A: less polar product) as a mixture of two steric isomers eluted earlier, and then to give the title compound (Example 514A: more polar product) as a mixture of two steric isomers eluted later.

### Example 513A (less polar product)1H NMR (300 MHz, CDCl₃) δ ppm 0.92 (t, J=7.5 Hz, 3 H), 1.37-1.63 (m, 6 H), 1.62-2.02 (m, 6 H), 2.83-3.14 (m, 2 H), 3.16-3.29 (m, 1 H), 3.32-3.44 (m, 1 H), 3.49 (dd, J=13.2, 4.3 Hz, 1 H), 3.89-4.05 (m, 1 H), 7.04-7.13 (m, 1 H), 7.27-7.33 (m, 2 H).

### Example 514A (more polar product)1H NMR (300 MHz, CDCl₃) δ ppm 0.92 (t, J=7.4 Hz, 3 H), 1.38-1.75 (m, 7 H), 1.76-2.03 (m, 5 H), 2.84-3.11 (m, 2 H), 3.12-3.26 (m, 1 H), 3.28-3.40 (m, 1 H), 3.48 (dd, J=13.1, 4.2 Hz, 1 H), 3.91-4.08 (m, 1 H), 6.99-7.18 (m, 1 H), 7.25-7.32 (m, 2 H).

### Example 515A 3-(2, 6-dichlorobenzyl)-1-(4-hydroxy-4-phenylcyclohexyl)pyrrolidin-2-one

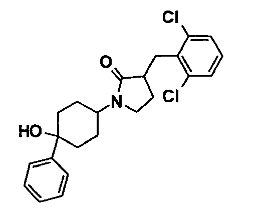

In the same manner as in Example 30A, the title compound was obtained from 3-(2,6-dichlorobenzyl)-1-(4-oxocyclohexyl)pyrrolidin-2-one obtained in Example 28A and phenylmagnesium bromide.
1H NMR (300 MHz, CDCl₃) δ ppm 1.33-2.13 (m, 8 H), 2.44-2.66 (m, 2 H), 2.80-2.95 (m, 1 H), 2.94-3.07 (m, 2 H), 3.08-3.19 (m, 1 H), 3.39-3.53 (m, 1 H), 4.08-4.25 (m, 1 H), 7.00-7.13 (m, 1 H), 7.26 (s, 1 H), 7.28-7.35 (m, 2 H), 7.36-7.45 (m, 2 H), 7.52-7.61 (m, 2 H).

### Example 516A ethyl {4-[3-(2,6-dichlorobenzyl)-2-oxopyrrolidin-1-yl]cyclohexylidene}acetate

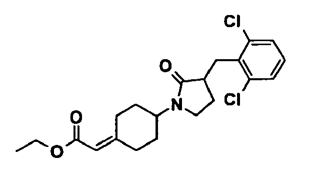

A mixture of 3-(2,6-dichlorobenzyl)-1-(4-oxocyclohexyl)pyrrolidin-2-one obtained in Example 28A (1.50 g), (carboethoxymethyl)triphenylphosphine bromide (5.68 g), sodium ethoxide (4.94 g) and ethanol (40 mL) was stirred at 90°C for 16 hr. The reaction mixture was concentrated under reduced pressure, and the residue was subjected to silica gel column chromatography (hexane-ethyl acetate 95:5-1:1) to give the title compound (1.60 g, 88%) as a yellow oil.
1H NMR (300 MHz, CDCl₃) δ ppm 1.23-1.30 (m, 3 H), 1.51-2.09 (m, 5 H), 2.09-2.42 (m, 3 H), 2.86-3.55 (m, 7 H), 4.08-4.19 (m, 2 H), 4.20-4.34 (m, 1 H), 5.45-5.69 (m, 1 H), 7.03-7.15 (m, 1 H), 7.29 (d, J=7.9 Hz, 2 H).

### Example 517A {4-[3-(2,6-dichlorobenzyl)-2-oxopyrrolidin-1-yl]cyclohexylidene}acetic acid

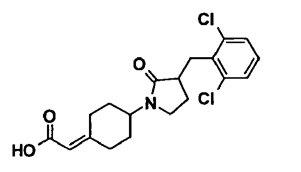

In the same manner as in Example 155A, the title compound was obtained from ethyl {4-[3-(2,6-dichlorobenzyl)-2-oxopyrrolidin-1-yl]cyclohexylidene}acetate obtained in Example 516A.
1H NMR (300 MHz, CDCl₃) δ ppm 1.67-2.08 (m, 5 H), 2.09-2.45 (m, 3 H), 2.83-3.13 (m, 3 H), 3.13-3.25 (m, 1 H), 3.25-3.43 (m, 1 H), 3.49 (dd, J=13.0, 4.0 Hz, 1 H), 3.68-3.82 (m, 1 H), 4.18-4.35 (m, 1 H), 5.58 (d, J=2.8 Hz, 1 H), 7.01-7.16 (m, 1 H), 7.28-7.31 (m, 2 H).

### Example 518A ethyl {4-[3-(2,6-dichlorobenzyl)-2-oxopyrrolidin-1-yl]cyclohexyl}acetate

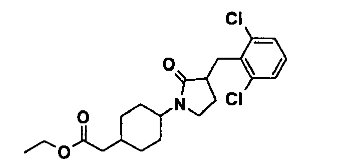

In the same manner as in Example 527A, the title compound was obtained from ethyl {4-[3-(2,6-dichlorobenzyl)-2-oxopyrrolidin-1-yl]cyclohexylidene}acetate obtained in Example 516A.
LC-MS (ESI+); m/z 412 (M+H)⁺

### Example 519A {4-[3-(2,6-dichlorobenzyl)-2-oxopyrrolidin-1-yl]cyclohexyl}acetic acid

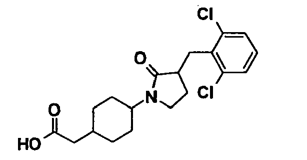

In the same manner as in Example 155A, the title compound was obtained from ethyl {4-[3-(2,6-dichlorobenzyl)-2-oxopyrrolidin-1-yl]cyclohexyl}acetate obtained in Example 518A.
1H NMR (300 MHz, CDCl₃) δ ppm 1.07-1.33 (m, 2 H), 1.39-2.05 (m, 8 H), 2.27 (d, J=7.0 Hz, 1 H), 2.40-2.48 (m, 1 H), 2.85-3.11 (m, 2 H), 3.12-3.53 (m, 4 H), 3.87-4.05 (m, 1 H), 7.00-7.17 (m, 1 H), 7.29 (d, J=7.9 Hz, 2 H).

### Example 520A 3-(2,6-dichlorobenzyl)-1-[4-(2-hydroxy-2-methylpropyl)cyclohexyl]pyrrolidin-2-one

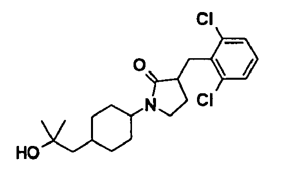

In the same manner as in Example 263A, the title compound was obtained from ethyl {4-[3-(2,6-dichlorobenzyl)-2-oxopyrrolidin-1-yl]cyclohexyl}acetate obtained in Example 518A and methyl lithium.
1H NMR (300 MHz, CDCl₃) δ ppm 1.08-1.34 (m, 8 H), 1.36-2.01 (m, 12 H), 2.84-3.10 (m, 2 H), 3.12-3.42 (m, 2 H), 3.49 (dd, J=13.1, 4.2 Hz, 1 H), 3.86-4.04 (m, 1 H), 7.03-7.16 (m, 1 H), 7.29 (d, J=7.9 Hz, 2 H).

### Example 521A 3-(2,6-dichlorobenzyl)-1-[4-(2-oxo-2-(pyrrolidin-1-yl)ethyl)cyclohexyl]pyrrolidin-2-one

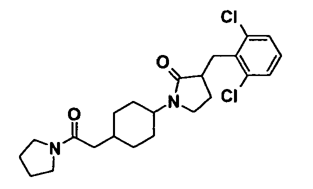

In the same manner as in Example 70A, the title compound was obtained from {4-[3-(2,6-dichlorobenzyl)-2-oxopyrrolidin-1-yl]cyclohexyl}acetic acid obtained in Example 519A and pyrrolidine.
1H NMR (300 MHz, CDCl₃) δ ppm 0.98-1.32 (m, 1 H), 1.39-2.02 (m, 13 H), 2.16 (d, J=6.4 Hz, 1 H), 2.34 (s, 2 H), 2.80-3.10 (m, 2 H), 3.11-3.57 (m, 7 H), 3.81-4.05 (m, 1 H), 7.02-7.16 (m, 1 H), 7.29 (d, J=8.1 Hz, 2 H).

### Example 522A 2-{4-[3-(2,6-dichlorobenzyl)-2-oxopyrrolidin-1-yl]cyclohexyl}-N-phenylacetamide

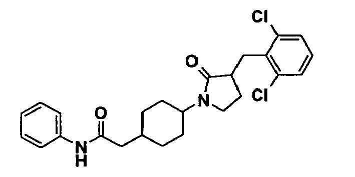

In the same manner as in Example 70A, the title compound was obtained from {4-[3-(2,6-dichlorobenzyl)-2-oxopyrrolidin-1-yl]cyclohexyl}acetic acid obtained in Example 519A and aniline.
1H NMR (300 MHz, CDCl₃) δ ppm 1.07-1.28 (m, 1 H), 1.41-2.03 (m, 10 H), 2.26 (d, J=6.6 Hz, 1 H), 2.31-2.52 (m, 2 H), 2.80-3.13 (m, 2 H), 3.10-3.56 (m, 3 H), 3.72-4.08 (m, 1 H), 7.00-7.16 (m, 2 H), 7.27-7.43 (m, 4 H), 7.54 (t, J=8.1 Hz, 2 H).

### Example 523A N-benzyl-2-{4-[3-(2,6-dichlorobenzyl)-2-oxopyrrolidin-1-yl]cyclohexyl}acetamide

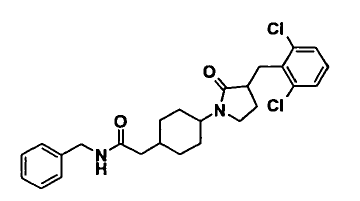

In the same manner as in Example 70A, the title compound was obtained from {4-[3-(2,6-dichlorobenzyl)-2-oxopyrrolidin-1-yl]cyclohexyl}acetic acid obtained in Example 519A and benzylamine.
1H NMR (300 MHz, CDCl₃) δ ppm 0.99-1.25 (m, 1 H), 1.36-2.01 (m, 10 H), 2.11 (d, J=6.6 Hz, 1 H), 2.30 (s, 2 H), 2.80-3.09 (m, 2 H), 3.10-3.27 (m, 1 H), 3.27-3.55 (m, 2 H), 3.80-4.04 (m, 1 H), 4.44 (d, J=5.7 Hz, 1 H), 5.76-6.18 (m, 1 H), 7.03-7.14 (m, 1 H), 7.19-7.38 (m, 7 H).

### Example 524A 2-{4-[3-(2,6-dichlorobenzyl)-2-oxopyrrolidin-1-yl]cyclohexyl}acetamide

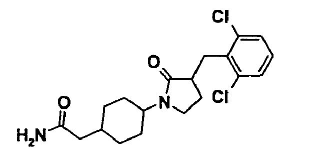

In the same manner as in Example 70A, the title compound was obtained from {4-[3-(2,6-dichlorobenzyl)-2-oxopyrrolidin-1-yl]cyclohexyl}acetic acid obtained in Example 519A and ammonia (25% solution).
1H NMR (300 MHz, CDCl₃) δ ppm 1.03-1.32 (m, 1 H), 1.37-1.79 (m, 8 H), 1.79-2.03 (m, 3 H), 2.16-2.38 (m, 2 H), 2.82-3.10 (m, 2 H), 3.13-3.43 (m, 2 H), 3.48 (dd, J=13.1, 4.4 Hz, 1 H), 3.81-4.04 (m, 1 H), 5.38 (br d like, 1 H), 7.04-7.15 (m, 1 H), 7.29 (d, J=8.0 Hz, 2 H).

### Example 525A 3-(2,6-dichlorobenzyl)-1-(4-{[3-(4-fluorophenyl)-1,2,4-oxadiazol-5-yl]methyl}cyclohexyl)pyrrolidin-2-one

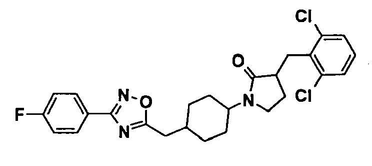

A mixture of {4-[3-(2,6-dichlorobenzyl)-2-oxopyrrolidin-1-yl]cyclohexyl}acetic acid obtained in Example 519A (0.5 g), 1,1'-carbonylbis-1H-imidazole (0.32 g) and dichloromethane (10 mL) was stirred at room temperature for 1 hr. 4-Fluorobenzamidoxime (0.60 g) was added to the reaction solution, the mixture was further stirred at room temperature for 16 hr, and the reaction mixture was concentrated under reduced pressure. The residue was heated under reflux in toluene (15 mL) for 16 hr, the reaction mixture was concentrated under reduced pressure, and the residue was subjected to silica gel column chromatography (hexane-ethyl acetate 95:5-1:1) to give the title compound (0.45 g, 69%) as a colorless solid.
1H NMR (300 MHz, CDCl₃) δ ppm 1.21-2.08 (m, 10 H), 2.37-2.54 (m, 1 H), 2.82-3.11 (m, 4 H), 3.12-3.57 (m, 3 H), 3.88-4.11 (m, 1 H), 7.02-7.23 (m, 3 H), 7.27-7.34 (m, 2 H), 8.07 (dd, J=8.3, 5.3 Hz, 2 H).

### Example 526A 3-(2,6-dichlorobenzyl)-1-{3-[3-(4-fluorophenyl)-1,2,4-oxadiazol-5-yl]-1,1-dimethylpropyl}pyrrolidin-2-one

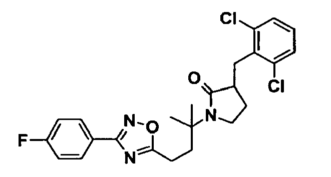

In the same manner as in Example 525A, the title compound was obtained from 4-[3-(2,6-dichlorobenzyl)-2-oxopyrrolidin-1-yl]-4-methylpentanoic acid obtained in Reference Example 47A and 4-fluorobenzamidoxime.
1H NMR (300 MHz, CDCl₃) δ ppm 1.44 (s, 6 H), 1.71-1.97 (m, 2 H), 2.33-2.69 (m, 2 H), 2.75-3.10 (m, 4 H), 3.19-3.37 (m, 1 H), 3.37-3.52 (m, 2 H), 7.00-7.21 (m, 3 H), 7.22-7.33 (m, 2 H), 7.98-8.13 (m, 2 H).

### Example 527A ethyl 3-{3-chloro-4-[(1-cyclohexyl-2-oxopyrrolidin-3-yl)methyl]phenyl}propanoate

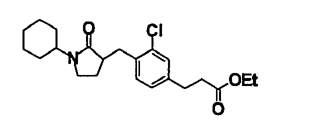

To a solution of ethyl (2E)-3-{3-chloro-4-[(1-cyclohexyl-2-oxopyrrolidin-3-yl)methyl]phenyl}acrylate obtained in Example 253A (1.92 g) in ethyl acetate (80 mL) was added platinum oxide (0.057 g), and the mixture was stirred at room temperature for 12 hr under hydrogen atmosphere. The reaction mixture was filtrated, the solvent was evaporated under reduced pressure, and the residue was subjected to silica gel column chromatography (hexane-ethyl acetate 9:1-3:2) to give the title compound (1.67 g, 87%) as a colorless oil.
1H NMR (300 MHz, CDCl₃) δ ppm 0.97-1.16 (m, 1 H), 1.24 (t, J=7.0 Hz, 3 H), 1.28-1.50 (m, 4 H), 1.51-1.85 (m, 6 H), 1.90-2.06 (m, 1 H), 2.52-2.65 (m, 2 H), 2.67-2.82 (m, 2 H), 2.83-2.95 (m, 2 H), 3.08-3.26 (m, 2 H), 3.26-3.39 (m, 1 H), 3.85-4.02 (m, 1 H), 4.13 (q, J=7.2 Hz, 2 H), 7.02 (dd, J=8.0, 1.5 Hz, 1 H), 7.16-7.22 (m, 2 H).

### Example 528A 3-{3-chloro-4-[(1-cyclohexyl-2-oxopyrrolidin-3-yl)methyl]phenyl}propanoic acid

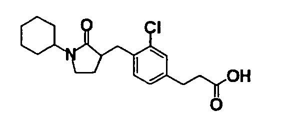

In the same manner as in Example 155A, the title compound was obtained from ethyl 3-{3-chloro-4-[(1-cyclohexyl-2-oxopyrrolidin-3-yl)methyl]phenyl}propanoate obtained in Example 527A.
1H NMR (300 MHz, CDCl₃) δ ppm 0.96-1.19 (m, 1 H), 1.19-1.49 (m, 5 H), 1.51-1.88 (m, 6 H), 1.88-2.09 (m, 1 H), 2.57-2.86 (m, 4 H), 2.91 (t, J=7.5 Hz, 2 H), 3.07-3.37 (m, 3 H), 3.82-4.07 (m, J=4.0 Hz, 1 H), 7.03 (dd, J=7.8, 1.8 Hz, 1 H), 7.16 (d, J=7.9 Hz, 1 H), 7.21 (d, J=1.7 Hz, 1 H).

### Example 529A 3-[2-chloro-4-(3-hydroxypropyl)benzyl]-1-cyclohexylpyrrolidin-2-one

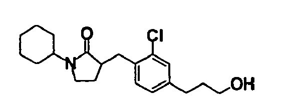

To a solution of 3-{3-chloro-4-[(1-cyclohexyl-2-oxopyrrolidin-3-yl)methyl]phenyl}propanoic acid obtained in Example 528A (0.21 g) in tetrahydrofuran (5 mL) was added thionyl chloride (0.21 g), and the mixture was heated under reflux for 3 hr. The reaction mixture was concentrated under reduced pressure, and tetrahydrofuran (5 mL) was added to the obtained residue An aqueous solution (5 mL) of lithium borohydride (0.11 g) was added dropwise at 0°C, and the mixture was stirred for 3 hr while allowing to warm to room temperature. Water was added to the reaction mixture, and the mixture was extracted with ethyl acetate. The extract was dried over anhydrous magnesium sulfate, and the solvent was evaporated under reduced pressure. The residue was subjected to silica gel column chromatography (hexane-ethyl acetate 1:1, ethyl acetate), and the obtained solid was recrystallized from ethyl acetate to give the title compound (0.062 g, 31%) as a white powder.
1H NMR (300 MHz, CDCl₃)δ ppm 1.13 (d, J=6.0 Hz, 1 H), 1.22-2.10 (m, 14 H), 2.58-2.98 (m, 5 H), 3.08-3.40 (m, 3 H), 3.67 (t, J=6.4 Hz, 1 H), 3.95 (s, 1 H), 6.98-7.07 (m, 1 H), 7.14-7.23 (m, 2 H).

### Example 530A 3-{3-chloro-4-[(1-cyclohexyl-2-oxopyrrolidin-3-yl)methyl]phenyl}propanamide

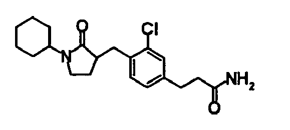

In the same manner as in Example 70A, the title compound was obtained from 3-{3-chloro-4-[(1-cyclohexyl-2-oxopyrrolidin-3-yl)methyl]phenyl}propanoic acid obtained in Example 528A and saturated aqueous ammonia.
1H NMR (300 MHz, DMSO-d₆) δ ppm 1.09 (s, 1 H), 1.18-1.47 (m, 4 H), 1.47-1.65 (m, 4 H), 1.74 (d, J=11.7 Hz, 2 H), 1.81-1.96 (m, 1 H), 2.34 (t, J=7.6 Hz, 2 H), 2.55-2.70 (m, 2 H), 2.77 (t, J=7.5 Hz, 2 H), 3.06-3.28 (m, 3 H), 3.73 (s, 1 H), 6.77 (s, 1 H), 7.10 (dd, J=7.8, 1.6 Hz, 1H), 7.19-7.35 (m, 3 H).

### Example 531A 3-[2-chloro-4-(3-oxo-3-(pyrrolidin-1-yl)propyl)benzyl]-1-cyclohexylpyrrolidin-2-one

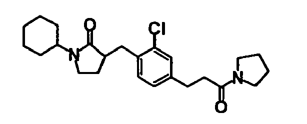

In the same manner as in Example 70A, the title compound was obtained from 3-{3-chloro-4-[(1-cyclohexyl-2-oxopyrrolidin-3-yl)methyl]phenyl}propanoic acid obtained in Example 528A and pyrrolidine.
1H NMR (300 MHz, CDCl₃) δ ppm 0.95-1.19 (m, J=3.4 Hz, 1 H), 1.20-1.49 (m, 4 H), 1.60-2.11 (m, 11 H), 2.53 (t, J=7.8 Hz, 2 H), 2.66-2.85 (m, 2 H), 2.86-3.01 (m, 2 H), 3.09-3.26 (m, 2 H), 3.26-3.39 (m, 3 H), 3.47 (t, J=6.6 Hz, 2 H), 3.85-4.04 (m, 1 H), 7.04 (d, J=8.0 Hz, 1 H), 7.14-7.23 (m, 2 H).

### Example 532A 3-(2,4-dichlorobenzyl)-1-[4-(hydroxymethyl)cyclohexyl]pyrrolidin-2-one

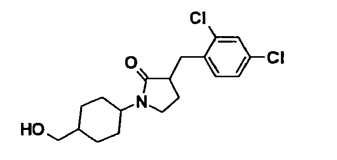

In the same manner as in Example 255A, the title compound was obtained from 1-[4-(hydroxymethyl)cyclohexyl]pyrrolidin-2-one obtained in Reference Example 34A and 2,4-dichlorobenzyl chloride.
LC-MS (ESI+); m/z 456 (M)⁺

### Example 533A 4-[3-(2,4-dichlorobenzyl)-2-oxopyrrolidin-1-yl]cyclohexanecarbaldehyde

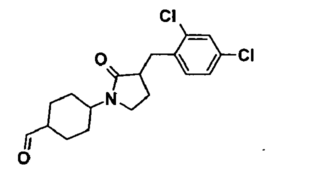

In the same manner as in Reference Example 44A, the title compound was obtained from 3-(2,4-dichlorobenzyl)-1-[4-(hydroxymethyl)cyclohexyl]pyrrolidin-2-one obtained in Example 532A.
1H NMR (300 MHz, CDCl₃) δ ppm 1.28-1.91 (m, 6 H), 1.92-2.21 (m, 2 H), 2.22-2.38 (m, 2 H), 2.48 (t, J=5.6 Hz, 1 H), 2.65-2.87 (m, 2 H), 3.04-3.42 (m, 3 H), 3.88-4.05 (m, 1 H), 7.10-7.25 (m, 2 H), 7. 37 (t, J=2.1 Hz, 1 H), 9. 62 and 9.70 (s, 1 H).

### Example 534A methyl 4-[3-(2,4-dichlorobenzyl)-2-oxopyrrolidin-1-yl]cyclohexanecarboxylate

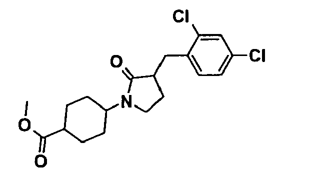

In the same manner as in the second step of Example 259A, the title compound was obtained from 4-[3-(2,4-dichlorobenzyl)-2-oxopyrrolidin-1-yl]cyclohexanecarbaldehyde obtained in Example 533A, N-iodosuccinimide, potassium carbonate and methanol.
1H NMR (300 MHz, CDCl₃) δ ppm 1.29-1.88 (m, 8 H), 1.95-2.15 (m, 2 H), 2.14-2.30 (m, 1 H), 2.66-2.85 (m, 2 H), 3.11-3.24 (m, 2 H), 3.25-3.38 (m, 1 H), 3.67 (s, 3 H), 3.87-4.06 (m, 1 H), 7.13-7.25 (m, 2 H), 7.37 (d, J=2.1 Hz, 1 H).

### Example 535A 4-[3-(2,4-dichlorobenzyl)-2-oxopyrrolidin-1-yl]cyclohexanecarboxylic acid

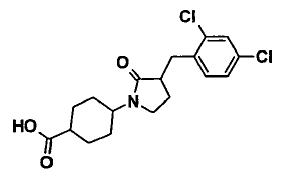

In the same manner as in Example 155A, the title compound was obtained from methyl 4-[3-(2,4-dichlorobenzyl)-2-oxopyrrolidin-1-yl]cyclohexanecarboxylate obtained in Example 534A.
1H NMR (300 MHz, DMSO-d₆) δ ppm 1.26-1.68 (m, 7 H), 1.83-2.02 (m, 3 H), 2.04-2.20 (m, 1 H), 2.55-2.79 (m, 2 H), 3.02-3.46 (m, 3 H), 3.60-3.79 (m, 1 H), 7.31-7.46 (m, 2 H), 7.58 (d, J=1.9 Hz, 1 H), 12.17 (s, 1 H).

### Example 536A 3-(2,4-dichlorobenzyl)-1-[4-(1-hydroxyethyl)cyclohexyl]pyrrolidin-2-one

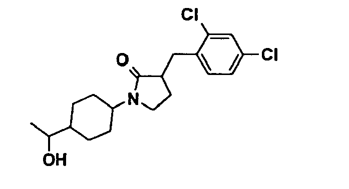

In the same manner as in Example 30A, the title compound was obtained from 4-[3-(2,4-dichlorobenzyl)-2-oxopyrrolidin-1-yl]cyclohexanecarbaldehyde obtained in Example 533A and methylmagnesium bromide.
LC-MS (ESI+); m/z 370 (M+H)⁺

### Example 537A 4-[3-(2,4-dichlorobenzyl)-2-oxopyrrolidin-1-yl]cyclohexanecarboxamide

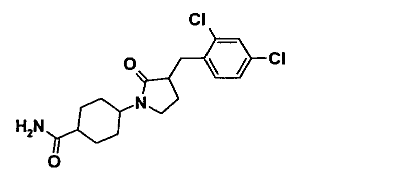

In the same manner as in Example 70A, the title compound was obtained from 4-[3-(2,4-dichlorobenzyl)-2-oxopyrrolidin-1-yl]cyclohexanecarboxylic acid obtained in Example 535A and ammonia (25% solution).
1H NMR (300 MHz, CDCl₃) δ ppm 1.36-1.91 (m, 8 H), 1.94-2.18 (m, 3 H), 2.70-2.85 (m, 2 H), 3.14-3.26 (m, 2 H), 3.26-3.40 (m, 1 H), 3.86-4.05 (m, 1 H), 5.30-5.42 (m, 2 H), 7.12-7.25 (m, 2 H), 7.37 (d, J=1. 9 Hz, 1 H).

### Example 538A 3-[2-chloro-4-(methoxymethoxy)benzyl]-1-(1,4-dioxaspiro[4.5]dec-8-yl)pyrrolidin-2-one

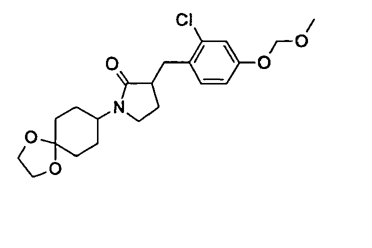

In the same manner as in Example 1A, the title compound was obtained from 1-(1,4-dioxaspiro[4.5]dec-8-yl)pyrrolidin-2-one and 2-chloro-4-(methoxymethoxy)benzyl bromide obtained in Reference Example 24A.
1H NMR (300 MHz, CDCl₃) δ ppm 1.57-1.88 (m, 9 H), 1.93-2.09 (m, 1 H), 2.66-2.84 (m, 2 H), 3.12-3.36 (m, 3 H), 3.47 (s, 3 H), 3.94 (s, 4 H), 3.99-4.12 (m, 1 H), 5.13 (s, 2 H), 6.87 (dd, J=8.5, 2.5 Hz, 1 H), 7.07 (d, J=2.5 Hz, 1 H), 7.17 (d, J=8.5Hz, 1 H).

### Example 539A 3-(2-chloro-4-hydroxybenzyl)-1-(4-oxocyclohexyl)pyrrolidin-2-one

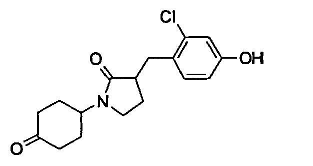

In the same manner as in Example 28A, the title compound was obtained from 3-[2-chloro-4-(methoxymethoxy)benzyl]-1-(1,4-dioxaspiro[4.5]dec-8-yl)pyrrolidin-2-one obtained in Example 538A.
1H NMR (300 MHz, CDCl₃) δ ppm 1.72-2.15 (m, 6 H), 2.36-2.60 (m, 4 H), 2.68-2.90 (m, 2 H), 3.14-3.31 (m, 3 H), 4.47 (tt, J=12.0, 3.8 Hz, 1 H), 6.68 (dd, J=8.3, 2.5 Hz, 1 H), 6.81 (brs, 1 H), 6.89 (d, J=2.5 Hz, 1 H), 7.09 (d, J=8.3 Hz, 1 H).

### Example 540A 3-(4-{[tert-butyl(diphenyl)silyl]oxy}-2-chlorobenzyl)-1-(4-oxocyclohexyl)pyrrolidin-2-one

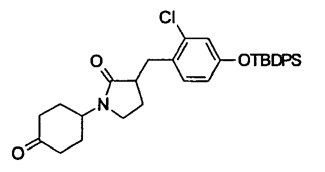

To a solution of 3-(2-chloro-4-hydroxybenzyl)-1-(4-oxocyclohexyl)pyrrolidin-2-one obtained in Example 539A (643 mg) in N,N-dimethylformamide (1 mL) were added tert-butyl(chloro)diphenylsilane (572 µL) and imidazole (150 mg), and the mixture was stirred at 60°C for 1 hr. Water was added to the reaction mixture, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 95:5-75:25) to give the title compound (673 mg, 60%) as a colorless oil.
1H NMR (300 MHz, CDCl₃) δ ppm 1.09 (s, 9 H), 1.62-2.06 (m, 6 H), 2.32-2.59 (m, 4 H), 2.59-2.81 (m, 2 H), 3.01-3.31 (m, 3 H), 4.43 (tt, J=12.0, 3.9 Hz, 1 H), 6.49 (dd, J=8.4, 2.5 Hz, 1 H), 6.86 (d, J=2.5 Hz, 1 H), 6.91 (d, J=8.4 Hz, 1 H), 7.32-7.48 (m, 6 H), 7.64-7.72 (m, 4 H).

### Example 541A 3-(4-{[tert-butyl(diphenyl)silyl]oxy}-2-chlorobenzyl)-1-(4-hydroxy-4-methylcyclohexyl)pyrrolidin-2-one

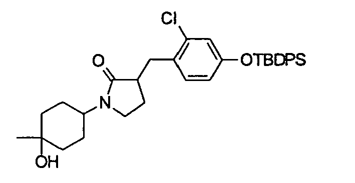

To a solution of 2,6-di-tert-butyl-4-methylphenol (608 mg) in toluene (5 mL) was added trimethylaluminum (2.0 M hexane solution, 0.7 mL) at room temperature, and the mixture was stirred for 1 hr. This reaction mixture was cooled to -78°C, and a solution of 3-(4-{[tert-butyl(diphenyl)silyl]oxy}-2-chlorobenzyl)-1-(4-oxocyclohexyl)pyrrolidin-2-one obtained in Example 540A (260 mg) in tetrahydrofuran (3 mL) was added. Methyllithium (1.5 M tetrahydrofuran solution, 0.7 mL) was added dropwise to the reaction mixture, and the mixture was stirred for 3 hr. 1N Hydrochloric acid was added to the reaction mixture, and the mixture was extracted with ethyl acetate. The organic layer was washed with saturated brine, and dried over anhydrous magnesium sulfate. The solvent was evaporated under reduced pressure, and the residue was subjected to silica gel column chromatography (hexane-ethyl acetate 95:5-1:4) to give the title compound (188 mg, 70%) as a colorless oil.

Recrystallization from hexane-diethyl ether gave colorless crystals.
1H NMR (300 MHz, CDCl₃)δ ppm 1.09 (s, 9 H), 1.26 (s, 3 H), 1.36 (s, 1 H), 1.42-1.79 (m, 9 H), 1.86-2.01 (m, 1 H), 2.57-2.79 (m, 2 H), 3.04-3.29 (m, 3 H), 3.86-4.01 (m, 1 H), 6.50 (dd, J=8.3, 2.5 Hz, 1 H), 6.84 (d, J=2.5 Hz, 1 H), 6.91 (d, J=8.3Hz, 1 H), 7.32-7.48 (m, 6 H), 7.63-7.74 (m, 4 H).

### Example 542A 3-(2-chloro-4-hydroxybenzyl)-1-(4-hydroxy-4-methylcyclohexyl)pyrrolidin-2-one

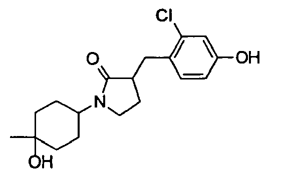

In the same manner as in Example 155A, the title compound was obtained from 3-(4-{[tert-butyl(diphenyl)silyl]oxy}-2-chlorobenzyl)-1-(4-hydroxy-4-methylcyclohexyl)pyrrolidin-2-one obtained in Example 541A.
1H NMR (300 MHz, DMSO-d₆) δ ppm 1.12 (s, 3 H), 1.36-1.63 (m, 9 H), 1.80-1.94 (m, 1 H), 2.40-2.67 (m, 2 H), 3.02-3.27 (m, 3 H), 3.63-3.78 (m, 1 H), 4.35 (s, 1 H), 6.66 (dd, J=8.5, 2.5 Hz, 1 H), 6.79 (d, J=2.5 Hz, 1 H), 7.13 (d, J=8.5 Hz, 1 H), 9.70 (brs, 1 H).

### Example 543A 3-(2-chloro-4-hydroxybenzyl)-1-(4-hydroxy-4-methylcyclohexyl)pyrrolidin-2-one (an optically active form, retention time 15 min) and Example 544A 3-(2-chloro-4-hydroxybenzyl)-1-(4-hydroxy-4-methylcyclohexyl)pyrrolidin-2-one (an optically active form, retention time 18 min)

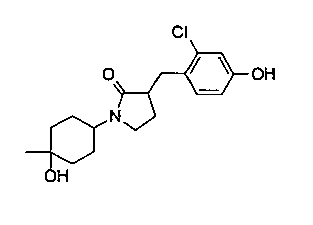

3-(2-Chloro-4-hydroxybenzyl)-1-(4-hydroxy-4-methylcyclohexyl)pyrrolidin-2-one (a mixture of two steric isomers) obtained in Example 542A was subjected to optical resolution with normal phase chiral high performance liquid chromatography (manufactured by Daicel Chemical Industries, Ltd., Chiralcel OJ (trade name), 50 mmID X 500 mmL, hexane-ethanol 85:15) to give the title compound (Example 543A) from a fraction with a retention time of 15 min and the title compound (Example 544A) from a fraction with a retention time of 18 min. Example 543A (retention time 15 min)
1H NMR (300 MHz, DMSO-d₆) δ ppm 1.12 (s, 3 H), 1.36-1.64 (m, 9 H), 1.79-1.94 (m, 1 H), 2.39-2.67 (m, 2 H), 3.02-3.28 (m, 3 H), 3.62-3.77 (m, 1 H), 4.36 (s, 1 H), 6.66 (dd, J=8.4, 2.5 Hz, 1 H), 6.79 (d, J=2.5 Hz, 1 H), 7.13 (d, J=8.4 Hz, 1 H), 9.70 (brs, 1 H).

### Example 544A (retention time 18 min)

1H NMR (300 MHz, DMSO-d₆) δ ppm 1.12 (s, 3 H), 1.37-1.64 (m, 9 H), 1.79-1.94 (m, 1 H), 2.40-2.67 (m, 2 H), 3.01-3.28 (m, 3 H), 3.62-3.77 (m, 1 H), 4.36 (s, 1 H), 6.66 (dd, J=8.3, 2.5 Hz, 1 H), 6.79 (d, J=2.5 Hz, 1 H), 7.13 (d, J=8.3 Hz, 1 H), 9.70 (brs, 1 H).

### Example 545A 3-[2-chloro-4-(2-(morpholin-4-yl)ethoxy)benzyl]-1-cyclohexylpyrrolidin-2-one

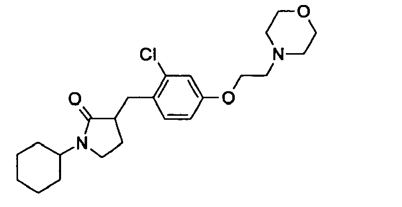

In the same manner as in Example 338A, the title compound was obtained from 3-(2-chloro-4-hydroxybenzyl)-1-cyclohexylpyrrolidin-2-one obtained in Example 183A and 2-morpholinoethanol.
1H NMR (300 MHz, CDCl₃) δ ppm 0.99-1.17 (m, 1 H), 1.26-1.48 (m, 4 H), 1.57-1.86 (m, 6 H), 1.93-2.07 (m, 1 H), 2.67-2.83 (m, 2 H), 3.11-3.35 (m, 5 H), 3.37-3.49 (m, 4 H), 3.86-4.13 (m, 5 H), 4.37-4.48 (m, 2 H), 6.73 (dd, J=8.5, 2.6 Hz, 1 H), 6.90 (d, J=2.6 Hz, 1 H), 7.21 (d, J=8.5 Hz, 1 H).

### Example 546A 3-{4-[2-(benzyloxy)ethoxy]-2-chlorobenzyl}-1-(4-hydroxy-4-methylcyclohexyl)pyrrolidin-2-one

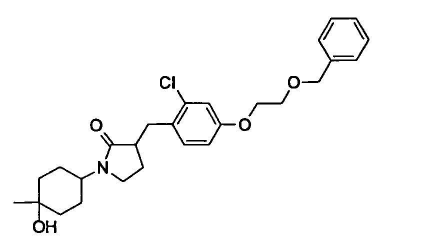

In the same manner as in Example 338A, the title compound was obtained from 3-(2-chloro-4-hydroxybenzyl)-1-(4-hydroxy-4-methylcyclohexyl)pyrrolidin-2-one obtained in Example 542A and 2-benzyloxyethanol.
1H NMR (300 MHz, CDCl₃) δ ppm 1.27 (s, 3 H), 1.30-1.84 (m, 10 H), 1.90-2.09 (m, 1 H), 2.66-2.84 (m, 2 H), 3.10-3.37 (m, 3 H), 3.76-3.85 (m, 2 H), 3.89-4.15 (m, 3 H), 4.62 (s, 2 H), 6.77 (dd, J=8.5, 2.5 Hz, 1 H), 6.94 (d, J=2.5 Hz, 1 H), 7.16 (d, J=8.5 Hz, 1 H), 7.22-7.41 (m, 5 H).

### Example 547A 3-[2-chloro-4-(2-phenoxyethoxy)benzyl]-1-cyclohexylpyrrolidin-2-one

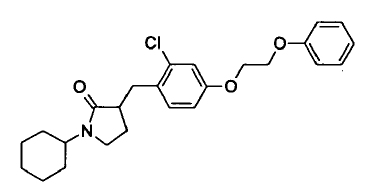

In the same manner as in Example 338A, the title compound was obtained from 3-[2-chloro-4-(2-hydroxyethoxy)benzyl]-1-cyclohexylpyrrolidin-2-one obtained in Example 344A and phenol.
1H NMR (300 MHz, CDCl₃) δ ppm 0.96-1.17 (m, 1 H), 1.20-1.50 (m, 4 H), 1.57-1.86 (m, 6 H), 1.90-2.08 (m, 1 H), 2.64-2.87 (m, 2 H), 3.09-3.39 (m, 3 H), 3.88-4.01 (m, 1 H), 4.25-4.34 (m, 4 H), 6.80 (dd, J=8.5, 2.5 Hz, 1 H), 6.90-7.02 (m, 4 H), 7.19 (d, J=8.5 Hz, 1 H), 7.24-7.35 (m, 2 H).

### Example 548A methyl 4-[(3-chloro-4-{[1-(4-hydroxy-4-methylcyclohexyl)-2-oxopyrrolidin-3-yl]methyl}phenoxy)methyl]benzoate

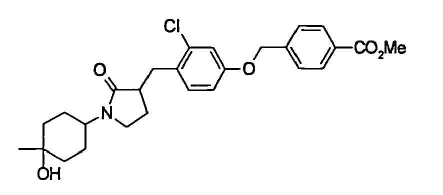

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and methyl 4-(bromomethyl)benzoate.
1H NMR (300 MHz, CDCl₃) δ ppm 1.27 (s, 3 H), 1.34-1.82 (m, 10 H), 1.93-2.09 (m, 1 H), 2.67-3.00 (m, 2 H), 3.12-3.36 (m, 3 H), 3.86-4.05 (m, 4 H), 5.09 (s, 2 H), 6.80 (dd, J=8.5, 2.6 Hz, 1 H), 6.98 (d, J=2.6 Hz, 1 H), 7.18 (d, J=8.5 Hz, 1 H), 7.48 (d, J=8.2 Hz, 2 H), 8.06 (d, J=8.2 Hz, 2 H).

### Example 549A 3-[2-chloro-4-(2-cyclopropylethoxy)benzyl]-1-cyclohexylpyrrolidin-2-one

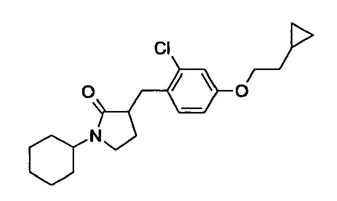

In the same manner as in Example 338A, the title compound was obtained from 3-(2-chloro-4-hydroxybenzyl)-1-cyclohexylpyrrolidin-2-one obtained in Example 183A and 2-cyclopropylethanol.
1H NMR (300 MHz, CDCl₃) δ ppm 0.06-0.17 (m, 2 H), 0.41-0.54 (m, 2 H), 0.76-0.91 (m, 1 H), 0.99-1.18 (m, 1 H), 1.22-1.50 (m, 4 H), 1.57-1.86 (m, 8 H), 1.90-2.07 (m, 1 H), 2.66-2.83 (m, 2 H), 3.10-3.36 (m, 3 H), 3.85-4.05 (m, 3 H), 6.74 (dd, J=8.5, 2.6 Hz, 1 H), 6.91 (d, J=2.6 Hz, 1 H), 7.16 (d, J=8.5 Hz, 1 H).

### Example 550A 3-(2-chloro-4-{[4-(hydroxymethyl)benzyl]oxy}benzyl)-1-(4-hydroxy-4-methylcyclohexyl)pyrrolidin-2-one

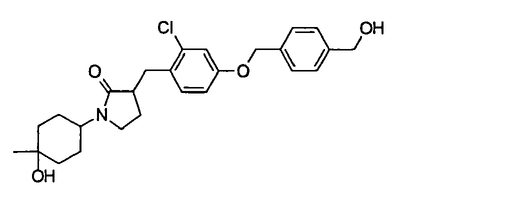

In the same manner as in Reference Example 19A, the title compound was obtained from methyl 4-[(3-chloro-4-{[1-(4-hydroxy-4-methylcyclohexyl)-2-oxopyrrolidin-3-yl]methyl}phenoxy)methyl]benzoate obtained in Example 548A.
1H NMR (300 MHz, CDCl₃) δ ppm 1.25 (s, 3 H), 1.51-1.81 (m, 9 H), 1.91-2.06 (m, 1 H), 2.58-2.71 (m, 2 H), 2.73-2.87 (m, 1 H), 3.19-3.36 (m, 3 H), 3.77-3.93 (m, 1 H), 4.60 (s, 2 H), 5.06 (s, 2 H), 6.88 (dd, J=8.5, 2.6 Hz, 1 H), 7.02 (d, J=2.6 Hz, 1 H), 7.20 (d, J=8.5 Hz, 1 H), 7.32-7.44 (m, 4 H).

### Example 551A methyl (3-chloro-4-{[1-(4-hydroxy-4-methylcyclohexyl)-2-oxopyrrolidin-3-yl]methyl}phenoxy)acetate

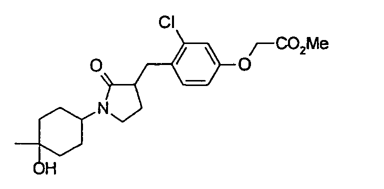

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and methyl bromoacetate.
1H NMR (300 MHz, CDCl₃) δ ppm 1.27 (s, 3 H), 1.41-1.81 (m, 10 H), 1.94-2.08 (m, 1 H), 2.67-2.82 (m, 2 H), 3.15-3.36 (m, 3 H), 3.81 (s, 3 H), 3.91-4.04 (m, 1 H), 4.60 (s, 2 H), 6.75 (dd, J=8.5, 2.6 Hz, 1 H), 6.92 (d, J=2.6 Hz, 1 H), 7.19 (d, J=8.5 Hz, 1 H).

### Example 552A 3-[2-chloro-4-(2-hydroxyethoxy)benzyl]-1-(4-hydroxy-4-methylcyclohexyl)pyrrolidin-2-one

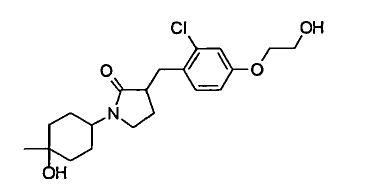

In the same manner as in Reference Example 19A, the title compound was obtained from methyl (3-chloro-4-{[1-(4-hydroxy-4-methylcyclohexyl)-2-oxopyrrolidin-3-yl]methyl}phenoxy)acetate obtained in Example 551A.
1H NMR (300 MHz, CDCl₃) δ ppm 1.27 (s, 3 H), 1.41-1.82 (m, 10 H), 1.93-2.12 (m, 2 H), 2.67-2.82 (m, 2 H), 3.13-3.36 (m, 3 H), 3.90-4.10 (m, 5 H), 6.77 (dd, J=8.5, 2.6 Hz, 1 H), 6.93 (d, J=2.6 Hz, 1 H), 7.18 (d, J=8.5 Hz, 1 H).

### Example 553A 3-[2-chloro-4-(2-{[5-(trifluoromethyl)pyridin-2-yl]oxy}ethoxy)benzyl]-1-cyclohexylpyrrolidin-2-one

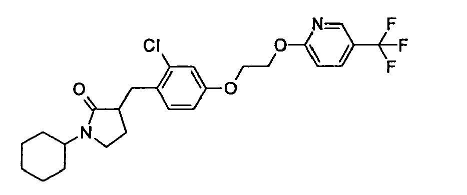

In the same manner as in Example 142A, the title compound was obtained from 3-[2-chloro-4-(2-hydroxyethoxy)benzyl]-1-cyclohexylpyrrolidin-2-one obtained in Example 344A and 2-chloro-5-(trifluoromethyl)pyridine.
1H NMR (300 MHz, CDCl₃) δ ppm 0.98-1.17 (m, 1 H), 1.22-1.49 (m, 4 H), 1.57-1.86 (m, 6 H), 1.91-2.06 (m, 1 H), 2.67-2.83 (m, 2 H), 3.10-3.36 (m, 3 H), 3.87-4.01 (m, 1 H), 4.24-4.35 (m, 2 H), 4.72-4.77 (m, 2 H), 6.79 (dd, J=8.5, 2.6 Hz, 1 H), 6.88 (d, J=8.8 Hz, 1 H), 6.97 (d, J=2.6 Hz, 1 H), 7.19 (d, J=8.5 Hz, 1 H), 7.79 (dd, J=8.8, 2.5 Hz, 1 H), 8.39-8.47 (m, 1 H).

### Example 554A 3-[2-chloro-4-(2-isopropoxyethoxy)benzyl]-1-(4-hydroxy-4-methylcyclohexyl)pyrrolidin-2-one

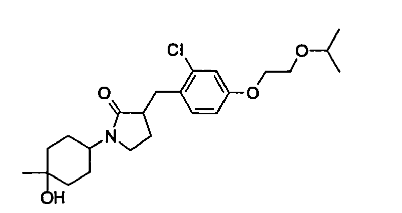

In the same manner as in Example 338A, the title compound was obtained from 3-(2-chloro-4-hydroxybenzyl)-1-(4-hydroxy-4-methylcyclohexyl)pyrrolidin-2-one obtained in Example 542A and 2-isopropoxyethanol.
1H NMR (300 MHz, CDCl₃) δ ppm 1.20 (d, J=6.2 Hz, 6 H), 1.27 (s, 3 H), 1.34-1.85 (m, 11 H), 1.91-2.08 (m, 1 H), 2.66-2.84 (m, 2 H), 3.11-3.37 (m, 2 H), 3.56-3.82 (m, 3 H), 3.89-4.16 (m, 3 H), 6.76 (dd, J=8.5, 2.6 Hz, 1 H), 6.94 (d, J=2.6 Hz, 1 H), 7.15 (d, J=8.5 Hz, 1 H).

### Example 555A 2-(2-{3-chloro-4-[(1-cyclohexyl-2-oxopyrrolidin-3-yl)methyl]phenoxy}ethyl)-1H-isoindole-1,3(2H)-dione

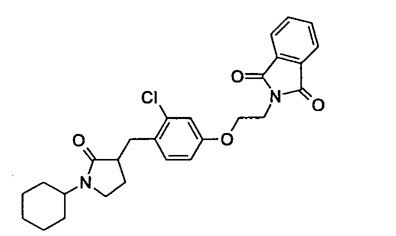

In the same manner as in Example 338A, the title compound was obtained from 3-(2-chloro-4-hydroxybenzyl)-1-cyclohexylpyrrolidin-2-one obtained in Example 183A and 2-(2-hydroxyethyl)-1H-isoindole-1,3(2H)-dione.
1H NMR (300 MHz, CDCl₃) δ ppm 0.97-1.16 (m, 1 H), 1.22-1.48 (m, 4 H), 1.53-1.65 (m, 6 H), 1.87-2.01 (m, 1 H), 2.62-2.80 (m, 2 H), 3.08-3.33 (m, 3 H), 3.86-4.01 (m, 1 H), 4.03-4.25 (m, 4 H), 6.71 (dd, J=8.5, 2.6 Hz, 1 H), 6.89 (d, J=2.6 Hz, 1 H), 7.13 (d, J=8.5 Hz, 1 H), 7.68-7.78 (m, 2 H), 7.82-7.91 (m, 2 H).

### Example 556A 3-{2-chloro-4-[2-(1H-imidazol-1-yl)ethoxy]benzyl}-1-cyclohexylpyrrolidin-2-one

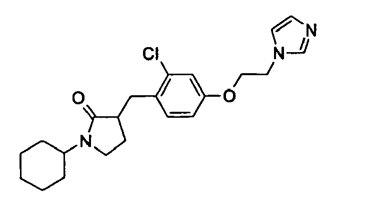

In the same manner as in Example 338A, the title compound was obtained from 3-(2-chloro-4-hydroxybenzyl)-1-cyclohexylpyrrolidin-2-one obtained in Example 183A and 2-(1H-imidazol-1-yl)ethanol.
1H NMR (300 MHz, CDCl₃) δ ppm 1.02-1.17 (m, 1 H), 1.28-1.49 (m, 4 H), 1.53-1.86 (m, 7 H), 1.91-2.04 (m, 1 H), 2.66-2.83 (m, 2 H), 3.10-3.35 (m, 3 H), 3.87-4.00 (m, 1 H), 4.14-4.22 (m, 2 H), 4.32 (t, J=5.0 Hz, 2 H), 6. 70 (dd, J=8. 5, 2.5 Hz, 1 H), 6.88 (d, J=2.5 Hz, 1 H), 7.05 (d, J=13.6 Hz, 2 H), 7.18 (d, J=8.5 Hz, 1 H), 7.58 (brs, 1 H).

### Example 557A 3-(2-chloro-4-isopropoxybenzyl)-1-(4-hydroxy-4-methylcyclohexyl)pyrrolidin-2-one

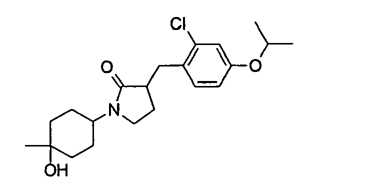

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and 2-iodopropane.
1H NMR (300 MHz, CDCl₃) δ ppm 1.27 (s, 3 H), 1.32 (d, J=6.0 Hz, 6 H), 1.42-1.82 (m, 10 H), 1.93-2.09 (m, 1 H), 2.64-2.85 (m, 2 H), 3.13-3.35 (m, 3 H), 3.91-4.05 (m, 1 H), 4.41-4.56 (m, 1 H), 6.72 (dd, J=8.5, 2.5 Hz, 1 H), 6.89 (d, J=2.5 Hz, 1 H), 7.14 (d, J=8.5 Hz, 1 H).

### Example 558A 3-[4-(2-aminoethoxy)-2-chlorobenzyl]-1-cyclohexylpyrrolidin-2-one hydrochloride

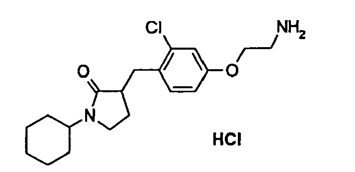

To a solution of 2-(2-{3-chloro-4-[(1-cyclohexyl-2-oxopyrrolidin-3-yl)methyl]phenoxy}ethyl)-1H-isoindole-1,3(2H)-dione obtained in Example 555A (240 mg) in ethanol (5 mL) was added hydrazine monohydrate (28 mg), and the mixture was heated under reflux for 1.5 hr. The reaction mixture was filtrated, and the filtrate was concentrated. To a solution of the residue in ethyl acetate (1 mL) was added 4N hydrogen chloride-ethyl acetate (125 µL), and the mixture was stirred. The precipitated crystals were collected by filtration to give the title compound (82 mg, 42%) as pale-yellow crystals.
1H NMR (300 MHz, CDCl₃) δ ppm 0.97-1.16 (m, 1 H), 1.17-1.80 (m, 10 H), 1.81-1.96 (m, 1 H), 2.52-2.72 (m, 2 H), 3.07-3.27 (m, 5 H), 3.64-3.80 (m, 1 H), 4.18 (t, J=5.1 Hz, 2 H), 6.92 (dd, J=8.5, 2.6 Hz, 1 H), 7.06 (d, J=2.6 Hz, 1 H), 7.30 (d, J=8.5 Hz, 1 H), 8.16 (brs, 1 H).

### Example 559A 3-(2-chloro-4-{2-[(2-chloro-4-fluorobenzyl)oxy]ethoxy}benzyl)-1-cyclohexylpyrrolidin-2-one

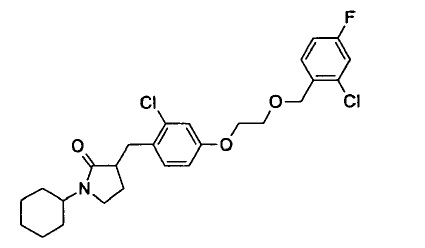

In the same manner as in Example 142A, the title compound was obtained from 3-[2-chloro-4-(2-hydroxyethoxy)benzyl]-1-cyclohexylpyrrolidin-2-one obtained in Example 344A and 1-(bromomethyl)-2-chloro-4-fluorobenzene.
1H NMR (300 MHz, CDCl₃) δ ppm 1.00-1.18 (m, 1 H), 1.22-1.49 (m, 4 H), 1.52-1.85 (m, 6 H), 1.90-2.06 (m, 1 H), 2.66-2.83 (m, 2 H), 3.10-3.36 (m, 3 H), 3.83-4.02 (m, 3 H), 4.10-4.18 (m, 2 H), 4.67 (s, 2 H), 6.77 (dd, J=8.6, 2.5 Hz, 1 H), 6.90-7.03 (m, 2 H), 7.11 (d, J=8.5, 2.6 Hz, 1 H), 7.17 (d, J=8.5 Hz, 1 H), 7.48 (dd, J=8.5, 6.2 Hz, 1 H).

### Example 560A 3-[2-chloro-4-(cyclopropylmethoxy)benzyl]-1-(4-hydroxy-4-methylcyclohexyl)pyrrolidin-2-one

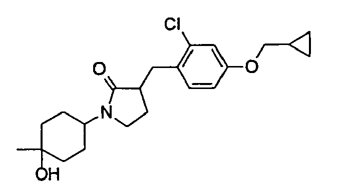

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and (bromomethyl)cyclopropane.
1H NMR (300 MHz, CDCl₃) δ ppm 0.28-0.38 (m, 2 H), 0.59-0.70 (m, 2 H), 1.17-1.33 (m, 4 H), 1.41-1.82 (m, 10 H), 1.93-2.07 (m, 1 H), 2.66-2.84 (m, 2 H), 3.12-3.35 (m, 3 H), 3.76 (d, J=7.0 Hz, 1 H), 3. 91-4 . 06 (m, 1 H), 6.74 (dd, J=8.5, 2.6 Hz, 1 H), 6.90 (d, J=2.6 Hz, 1 H), 7.15 (d, J=8.5 Hz, 1 H).

### Example 561A 3-(2-chloro-4-{[5-(trifluoromethyl)pyridin-2-yl]oxy}benzyl)-1-(4-hydroxy-4-methylcyclohexyl)pyrrolidin-2-one.

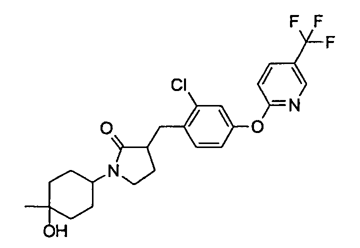

In the same manner as in Example 142A, the title compound was obtained from 3-(2-chloro-4-hydroxybenzyl)-1-(4-hydroxy-4-methylcyclohexyl)pyrrolidin-2-one obtained in Example 542A and 2-chloro-5-(trifluoromethyl)pyridine.
1H NMR (300 MHz, CDCl₃) δ ppm 1.28 (s, 3 H), 1.42-1.85 (m, 10 H), 2.01-2.16 (m, 1 H), 2.72-2.92 (m, 2 H), 3.14-3.48 (m, 3 H), 3.90-4.08 (m, 1 H), 6.97-7.08 (m, 2 H), 7.20 (d, J=2.3 Hz, 1 H), 7.34 (d, J=8.5 Hz, 1 H), 7.92 (dd, J=8.5, 2.5 Hz, 1 H), 8.39-8.48 (m, 1 H).

### Example 562A 4-[(3-chloro-4-{[1-(4-hydroxy-4-methylcyclohexyl)-2-oxopyrrolidin-3-yl]methyl}phenoxy)methyl]benzoic acid

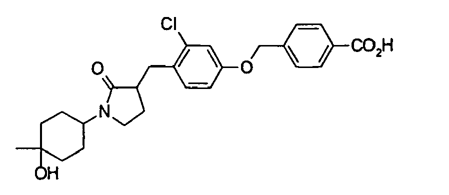

In the same manner as in Example 155A, the title compound was obtained from methyl 4-[(3-chloro-4-{[1-(4-hydroxy-4-methylcyclohexyl)-2-oxopyrrolidin-3-yl]methyl}phenoxy)methyl]benzoate obtained in Example 548A.
1H NMR (300 MHz, DMSO-d₆) δ ppm 1.13 (s, 3 H), 1.35-1.65 (m, 9 H), 1.81-1.95 (m, 1 H), 2.53-2.68 (m, 1 H), 3.04-3.28 (m, 4 H), 3.63-3.79 (m, 1 H), 4.35 (brs, 1 H), 5.20 (s, 2 H), 6.95 (dd, J=8.5, 2.6 Hz, 1 H), 7.11 (d, J=2.6 Hz, 1 H), 7.27 (d, J=8.5 Hz, 1 H), 7.55 (d, J=8.3 Hz, 2 H), 7.96 (d, J=8.3 Hz, 2 H).

### Example 563A 2-(3-chloro-4-{[1-(4-hydroxy-4-methylcyclohexyl)-2-oxopyrrolidin-3-yl] methyl} phenoxy)-N,N-diethylacetamide

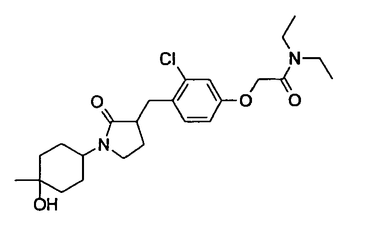

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and 2-chloro-N,N-diethylacetamide.
1H NMR (300 MHz, CDCl₃) δ ppm 1.15 (t, J=7.1 Hz, 3 H), 1.24 (t, J=7.2 Hz, 3 H), 1.27 (s, 3 H), 1.46-1.82 (m, 7 H), 1.94-2.09(m, 1 H), 2.66-3.01 (m, 5 H), 3.16-3.48 (m, 7 H), 3.90-4.05 (m, 1 H), 4.62 (s, 2 H), 6.79 (dd, J=8.5, 2.6 Hz, 1 H), 6.94 (d, J=2.6 Hz, 1 H), 7.17 (d, J=8.5 Hz, 1 H).

### Example 564A 3-[2-chloro-4-(cyclopropylmethoxy)benzyl]-1-(4-hydroxy-4-methylcyclohexyl)pyrrolidin-2-one (an optically active form 1)

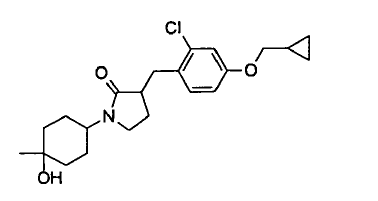

In the same manner as in Example 318A, the title compound was obtained from 3-(2-chloro-4-hydroxybenzyl)-1-(4-hydroxy-4-methylcyclohexyl)pyrrolidin-2-one obtained in Example 543A and (bromomethyl)cyclopropane.
1H NMR (300 MHz, CDCl₃) δ ppm 0.29-0.38 (m, 2 H), 0.60-0.69 (m, 2 H), 1.17-1.32 (m, 4 H), 1.42-1.82 (m, 10 H), 1.93-2.07 (m, 1 H), 2.66-2.84 (m, 2 H), 3.14-3.35 (m, 3 H), 3.76 (d, J=7.0 Hz, 1 H), 3.91-4.05 (m, 1 H), 6.74 (dd, J=8.5, 2.6 Hz, 1 H), 6.90 (d, J=2.6 Hz, 1 H), 7.15 (d, J=8.5 Hz, 1 H).

### Example 565A 3-[2-chloro-4-(cyclopropylmethoxy)benzyl]-1-(4-hydroxy-4-methylcyclohexyl)pyrrolidin-2-one (an optically active form 2)

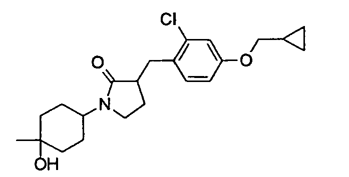

In the same manner as in Example 318A, the title compound was obtained from 3-(2-chloro-4-hydroxybenzyl)-1-(4-hydroxy-4-methylcyclohexyl)pyrrolidin-2-one obtained in Example 544A and (bromomethyl)cyclopropane.
1H NMR (300 MHz, CDCl₃) δ ppm 0.29-0.38 (m, 2 H), 0.58-0.69 (m, 2 H), 1.17-1.33 (m, 4 H), 1.43-1.82 (m, 10 H), 1.93-2.07 (m, 1 H), 2.65-2.83 (m, 2 H), 3.13-3.34 (m, 3 H), 3.76 (d, J=7.0 Hz, 1 H), 3.90-4.06 (m, 1 H), 6.74 (dd, J=8.5, 2.6 Hz, 1 H), 6.90 (d, J=2.6 Hz, 1 H), 7.15 (d, J=8.5 Hz, 1 H).

### Example 566A 3-12-chloro-4-[(1-cyclohexyl-5-oxopyrrolidin-3-yl)methoxy]benzyl}-1-(4-hydroxy-4-methylcyclohexyl)pyrrolidin-2-one

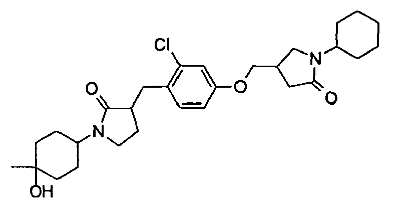

In the same manner as in Example 338A, the title compound was obtained from 3-(2-chloro-4-hydroxybenzyl)-1-(4-hydroxy-4-methylcyclohexyl)pyrrolidin-2-one obtained in Example 542A and 1-cyclohexyl-4-(hydroxymethyl)pyrrolidin-2-one obtained in Reference Example 17A.
1H NMR (300 MHz, CDCl₃) δ ppm 1.01-1.18 (m, 1 H), 1.20-1.87 (m, 22 H), 1.94-2.10 (m, 1 H), 2.29 (dd, J=16.8, 5.8 Hz, 1 H), 2.54-2.87 (m, 4 H), 3.12-3.34 (m, 4 H), 3.55 (dd, J=10.0, 7.9 Hz, 1 H), 3.78-4.05 (m, 4 H), 6.72 (dd, J=8.5, 2.5 Hz, 1 H), 6.89 (d, J=2.5 Hz, 1 H), 7.18 (d, J=8.5 Hz, 1 H).

### Example 567A methyl 4-[(3-chloro-4-{[1-(4-hydroxy-4-methylcyclohexyl)-2-oxopyrrolidin-3-yl]methyl}phenoxy)methyl]benzoate(an optically active form 1)

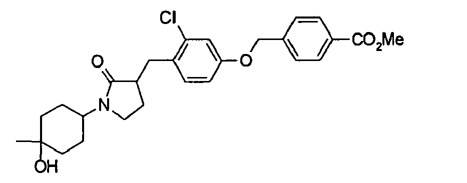

In the same manner as in Example 318A, the title compound was obtained from 3-(2-chloro-4-hydroxybenzyl)-1-(4-hydroxy-4-methylcyclohexyl)pyrrolidin-2-one obtained in Example 543A and methyl 4-(bromomethyl)benzoate.
1H NMR (300 MHz, CDCl₃) δ ppm 1.27 (s, 3 H), 1.39-1.82 (m, 10 H), 1.93-2.09 (m, 1 H), 2.68-3.83 (m, 2 H), 3.13-3.35 (m, 3 H), 3.88-4.05 (m, 4 H), 5.09 (s, 2 H), 6.80 (dd, J=8.5, 2.6 Hz, 1 H), 6.98 (d, J=2.6 Hz, 1 H), 7.18 (d, J=8.5 Hz, 1 H), 7.48 (d, J=8.1 Hz, 2 H), 7.99-8.10 (m, 2 H).

### Example 568A 3-(2-chloro-4-{[4-(hydroxymethyl)benzyl]oxy}benzyl)-1-(4-hydroxy-4-methylcyclohexyl)pyrrolidin-2-one (an optically active form 1)

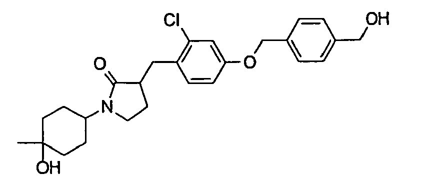

In the same manner as in Reference Example 19A, the title compound was obtained from methyl 4-[(3-chloro-4-{[1-(4-hydroxy-4-methylcyclohexyl)-2-oxopyrrolidin-3-yl]methyl}phenoxy)methyl]benzoate obtained in Example 567A.
1H NMR (300 MHz, CDCl₃) δ ppm 1.27 (s, 3 H), 1.37-1.85 (m, 11 H), 1.94-2.08 (m, 1 H), 2.65-2.84 (m, 2 H), 3.13-3.35 (m, 3 H), 3.93-4.04 (m, 1 H), 4.71 (d, J=5.7 Hz, 2 H), 5.02 (s, 2 H), 6.80 (dd, J=8.6, 2.5 Hz, 1 H), 6.99 (d, J=2.5 Hz, 1 H), 7.16 (d, J=8.6 Hz, 1 H), 7.35-7.44 (m, 4 H).

### Example 569A 3-[2-chloro-4-[(2-morpholin-4-yl)ethoxy)benzyl]-1-(4-hydroxy-4-methylcyclohexyl)pyrrolidin-2-one

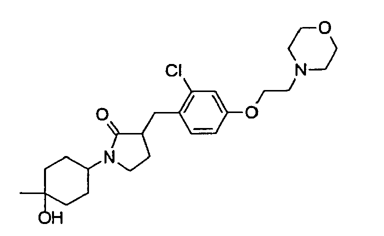

In the same manner as in Example 338A, the title compound was obtained from 3-(2-chloro-4-hydroxybenzyl)-1-(4-hydroxy-4-methylcyclohexyl)pyrrolidin-2-one obtained in Example 542A and 2-morpholinoethanol.
LC-MS (ESI+) ; m/z 451(M⁺)

### Example 570A 2-(3-chloro-4-{[1-(4-hydroxy-4-methylcyclohexyl)-2-oxopyrrolidin-3-yl] methyl}phenoxy)-N-[4-(trifluoromethyl)benzyl]acetamide

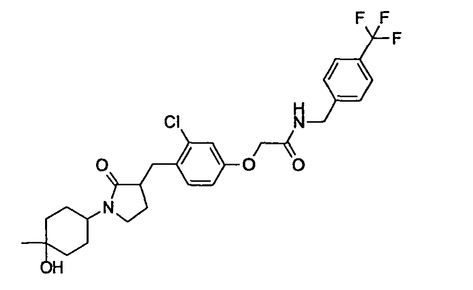

Methyl (3-chloro-4-{[1-(4-hydroxy-4-methylcyclohexyl)-2-oxopyrrolidin-3-yl]methyl}phenoxy)acetate obtained in Example 551A (112 mg) and 1-[4-(trifluoromethyl)phenyl]methanamine (58 µL) were stirred at 175°C for 13 hr. The reaction mixture was recrystallized from hexane-ethyl acetate to give the title compound (67 mg, 44%).
1H NMR (300 MHz, CDCl₃) δ ppm 1.27 (s, 3 H), 1.33-1.83 (m, 10 H), 1.94-2.08 (m, 1 H), 2.68-2.83 (m, 2 H), 3.12-3.36 (m, 3 H), 3.90-4.04 (m, 1 H), 4.53 (s, 2 H), 4.60 (d, J=6.2 Hz, 2 H), 6.76 (dd, J=8.5, 2.7 Hz, 1 H), 6.86-6.97 (m, 2 H), 7.22 (d, J=8.5 Hz, 1 H), 7.40 (d, J=8.0 Hz, 2 H), 7.60 (d, J=8.0 Hz, 2 H).

### Example 571A methyl 3-[(3-chloro-4-{[1-(4-hydroxy-4-methylcyclohexyl)-2-oxopyrrolidin-3-yl]methyl}phenoxy)methyl]benzoate

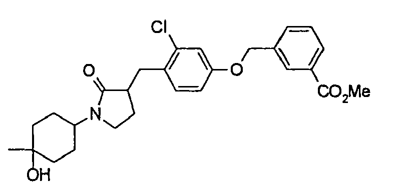

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and methyl 3-(bromomethyl)benzoate.
1H NMR (300 MHz, CDCl₃) δ ppm 1.27 (s, 3 H), 1.36-1.83 (m, 10 H), 1.93-2.09 (m, 1 H), 2.67-2.84 (m, 2 H), 3.14-3.37 (m, 3 H), 3.88-4.06 (m, 4 H), 5.06 (s, 2 H), 6.81 (dd, J=8.7, 2.6 Hz, 1 H), 7.00 (d, J=2.6 Hz, 1 H), 7.18 (d, J=8.7 Hz, 1 H), 7.42-7.52 (m, 1 H), 7.62 (d, J=8.1 Hz, 1 H), 7.96-8.05 (m, 1 H), 8.07-8.12 (m, 1 H).

### Example 572A 3-(2-chloro-4-isopropoxybenzyl)-1-(4-hydroxy-4-methylcyclohexyl)pyrrolidin-2-one (an optically active form 1)

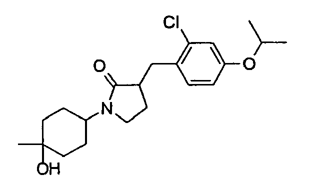

In the same manner as in Example 318A, the title compound was obtained from 3-(2-chloro-4-hydroxybenzyl)-1-(4-hydroxy-4-methylcyclohexyl)pyrrolidin-2-one obtained in Example 543A and 2-iodopropane.
1H NMR (300 MHz, CDCl₃) δ ppm 1.27 (s, 3 H), 1.32 (d, J=6.2 Hz, 6 H), 1.41-1.82 (m, 10 H), 1.93-2.08 (m, 1 H), 2.64-2.84 (m, 2 H), 3.13-3.36 (m, 3 H), 3.90-4.05 (m, 1 H), 4.41-4.56 (m, 1 H), 6.72 (dd, J=8.5, 2.6 Hz, 1 H), 6.89 (d, J=2.6 Hz, 1 H), 7.14 (d, J=8.5 Hz, 1 H).

### Example 573A 3-(2-chloro-4-{[3-(hydroxymethyl)benzyl]oxy}benzyl)-1-(4-hydroxy-4-methylcyclohexyl)pyrrolidin-2-one

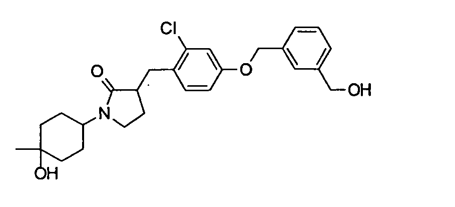

In the same manner as in Reference Example 19A, the title compound was obtained from methyl 3-[(3-chloro-4-{[1-(4-hydroxy-4-methylcyclohexyl)-2-oxopyrrolidin-3-yl]methyl}phenoxy)methyl]benzoate obtained in Example 571A.
1H NMR (300 MHz, CDCl₃) δ ppm 1.27 (s, 3 H), 1.40-1.83 (m, 12 H), 1.94-2.09 (m, 1 H), 2.67-2.83 (m, 2 H), 3.13-3.35 (m, 3 H), 3.91-4.05 (m, 1 H), 4.73 (s, 1 H), 5.02 (s, 2 H), 6.81 (dd, J=8.5, 2.5 Hz, 1 H), 6.99 (d, J=2.5 Hz, 1 H), 7.17 (d, J=8.5 Hz, 1 H), 7.31-7.46 (m, 4 H).

### Example 574A 3-(2-chloro-4-isopropoxybenzyl)-1-(4-hydroxy-4-methylcyclohexyl)pyrrolidin-2-one (an optically active form 2)

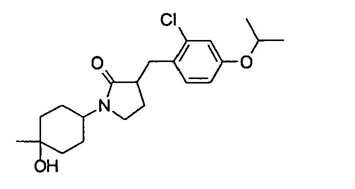

In the same manner as in Example 318A, the title compound was obtained from 3-(2-chloro-4-hydroxybenzyl)-1-(4-hydroxy-4-methylcyclohexyl)pyrrolidin-2-one obtained in Example 544A and 2-iodopropane.
1H NMR (300 MHz, CDCl₃) δ ppm 1.27 (s, 3 H), 1.32 (d, J=6.2 Hz, 6 H), 1.41-1.82 (m, 10 H), 1.93-2.08 (m, 1 H), 2.65-2.85 (m, 2 H), 3.11-3.35 (m, 3 H), 3.89-4.06 (m, 1 H), 4.40-4.58 (m, 1 H), 6.72 (dd, J=8.5, 2.5 Hz, 1 H), 6.89 (d, J=2.5 Hz, 1 H), 7.14 (d, J=8.5 Hz, 1 H).

### Example 575A methyl (3-chloro-4-{[1-(5-hydroxy-2-adamantyl)-2-oxopyrrolidin-3-yl]methyl}phenoxy)acetate

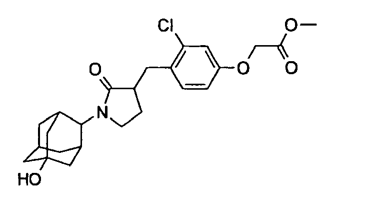

In the same manner as in Example 318A, the title compound was obtained from 3-(2-chloro-4-hydroxybenzyl)-1-[5-hydroxy-2-adamantyl]pyrrolidin-2-one obtained in Example 431A and methyl bromoacetate.
1H NMR (300 MHz, CDCl₃) δ ppm 1.41 (brs, 1 H), 1.46-1.57 (m, 2 H), 1.64-2.23 (m, 11 H), 2.38-2.52 (m, 2 H), 2.66-2.82 (m, 2 H), 3.19-3.59 (m, 3 H), 3.81 (s, 3 H), 3.87-3.94 (m, 1 H), 4.60 (s, 2 H), 6.75 (dd, J=8.5, 2.6 Hz, 1 H), 6.93 (d, J=2.6 Hz, 1 H), 7.20 (d, J=8.5 Hz, 1 H).

### Example 576A methyl 4-[(3-chloro-4-{[1-(5-hydroxy-2-adamantyl)-2-oxopyrrolidin-3-yl]methyl}phenoxy)methyl]benzoate

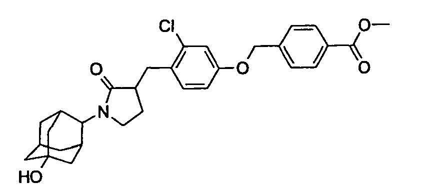

In the same manner as in Example 318A, the title compound was obtained from 3-(2-chloro-4-hydroxybenzyl)-1-[5-hydroxy-2-adamantyl]pyrrolidin-2-one obtained in Example 431A and methyl 4-(bromomethyl)benzoate.
1H NMR (300 MHz, CDCl₃) δ ppm 1.39 (brs, 1 H), 1.45-1.56 (m, 2 H), 1.63-2.21 (m, 11 H), 2.38-2.52 (m, 2 H), 2.68-2.81 (m, 2 H), 3.22-3.56 (m, 3 H), 3.86-3.97 (m, 4 H), 5.09 (s, 2 H), 6.80 (dd, J=8.5, 2.5 Hz, 1 H), 6.99 (d, J=2.5 Hz, 1 H), 7.19 (d, J=8.5 Hz, 1 H), 7.48 (d, J=8.5 Hz, 2 H), 8.01-8.11 (m, 2 H).

### Example 577A 3-[2-chloro-4-(cyclopropylmethoxy)benzyl]-1-(5-hydroxy-2-adamantyl)pyrrolidin-2-one

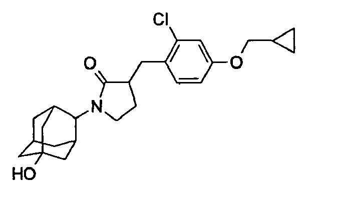

In the same manner as in Example 318A, the title compound was obtained from 3-(2-chloro-4-hydroxybenzyl)-1-[5-hydroxy-2-adamantyl]pyrrolidin-2-one obtained in Example 431A and (bromomethyl)cyclopropane.
1H NMR (300 MHz, CDCl₃) δ ppm 0.26-0.42 (m, 2 H), 0.56-0.72 (m, 2 H), 1.14-1.36 (m, 1 H), 1.42-2.11 (m, 13 H), 2.13-2.22 (m, 1 H), 2.38-2.51 (m, 2 H), 2.66-2.82 (m, 2 H), 3.21-3.57 (m, 3 H), 3.76 (d, J=7.0 Hz, 2 H), 3.87-3.96 (m, 1 H), 6.75 (dd, J=8.5, 2.6 Hz, 1 H), 6.90 (d, J=2.6 Hz, 1 H), 7.16 (d, J=8.5 Hz, 1 H).

### Example 578A 2-(3-chloro-4-{[1-(5-hydroxy-2-adamantyl)-2-oxopyrrolidin-3-yl]methyl}phenoxy)-N,N-diethylacetamide

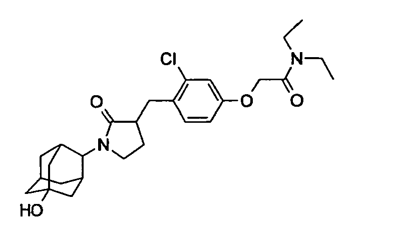

In the same manner as in Example 318A, the title compound was obtained from 3-(2-chloro-4-hydroxybenzyl)-1-[5-hydroxy-2-adamantyl]pyrrolidin-2-one obtained in Example 431A and 2-chloro-N,N-diethylacetamide.
1H NMR (300 MHz, CDCl₃) δ ppm 1.15 (t, J=7.1 Hz, 3 H), 1.23 (t, J=7.2 Hz, 3 H), 1.45-1.58 (m, 2 H), 1.62-2.11 (m, 11 H), 2.12-2.22 (m, 1 H), 2.39-2.52 (m, 2 H), 2.66-2.82 (m, 2 H), 3.21-3.58 (m, 7 H), 3.87-3.95 (m, 1 H), 4.64 (s, 2 H), 6.80 (dd, J=8.5, 2.6 Hz, 1 H), 6.95 (d, J=2.6 Hz, 1 H), 7.18 (d, J=8.5 Hz, 1 H).

### Example 579A 3-(2-chloro-4-isopropoxybenzyl)-1-(5-hydroxy-2-adamantyl)pyrrolidin-2-one

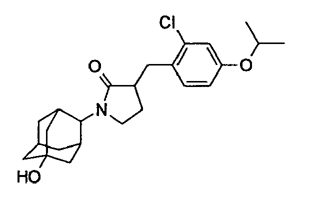

In the same manner as in Example 318A, the title compound was obtained from 3-(2-chloro-4-hydroxybenzyl)-1-[5-hydroxy-2-adamantyl]pyrrolidin-2-one obtained in Example 431A and 2-iodopropane.
1H NMR (300 MHz, CDCl₃) δ ppm 1.32 (d, J=6.0 Hz, 6 H), 1.35-1.42 (m, 1 H), 1.45-1.56 (m, 2 H), 1.65-2.22 (m, 11 H), 2.39-2.52 (m, 2 H), 2.66-2.79 (m, 2 H), 3.21-3.57 (m, 3 H), 3.87-3.94 (m, 1 H), 4.42-4.56 (m, 1 H), 6.72 (dd, J=8.5, 2.5 Hz, 1 H), 6.89 (d, J=2.5 Hz, 1 H), 7.15 (d, J=8.5 Hz, 1 H).

### Example 580A 3-(2-chloro-4-{[4-(hydroxymethyl)benzyl]oxy}benzyl)-1-(5-hydroxy-2-adamantyl)pyrrolidin-2-one

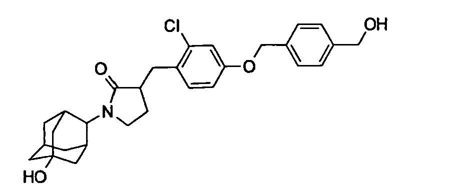

In the same manner as in Reference Example 19A, the title compound was obtained from methyl 4-[(3-chloro-4-{[1-(5-hydroxy-2-adamantyl)-2-oxopyrrolidin-3-yl]methyl}phenoxy)methyl]benzoate obtained in Example 576A.
1H NMR (300 MHz, CDCl₃) δ 1.14-1.58 (m, 2 H), 1.68-1.96 (m, 6 H), 1.97-2.23 (m, 2 H), 2.43 (brs, 1 H), 2.56-2.89 (m, 7 H), 3.27 (dd, J=13.2, 3.8 Hz, 1 H), 3.41-3.58 (m, 2H), 3.87-3.95 (m, 1 H), 4.72 (s, 2 H), 5.03 (s, 2 H), 6.80 (dd, J=8.5, 2.5 Hz, 1 H), 6.99 (d, J=2.5 Hz, 1 H), 7.16 (d, J=8.5 Hz, 1 H), 7.36-7.46 (m, 4 H).

### Example 581A 3-[2-chloro-4-(2-hydroxyethoxy)benzyl]-1-(5-hydroxy-2-adamantyl)pyrrolidin-2-one

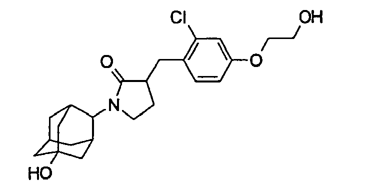

In the same manner as in Reference Example 19A, the title compound was obtained from methyl (3-chloro-4-{[1-(5-hydroxy-2-adamantyl)-2-oxopyrrolidin-3-yl]methyl}phenoxy)acetate obtained in Example 575A.
1H NMR (300 MHz, CDCl₃) δ ppm 1.35-2.24 (m, 15 H), 2.37-2.52 (m, 2 H), 2.68-2.83 (m, 2 H), 3.21-3.59 (m, 3 H), 3.86-4.11 (m, 5 H), 6.77 (dd, J=8.5, 2.5 Hz, 1H), 6.94 (d, J=2.5 Hz, 1 H), 7.19 (d, J=8.5 Hz, 1 H).

### Example 582A 3-[2-chloro-4-(2-hydroxyethoxy)benzyl]-1-(5-hydroxy-2-adamantyl)pyrrolidin-2-one (an optically active form, retention time 14 min) and Example 583A 3-[2-chloro-4-(2-hydroxyethoxy)benzyl]-1-(5-hydroxy-2-adamantyl)pyrrolidin-2-one (an optically active form, retention time for 20 min)

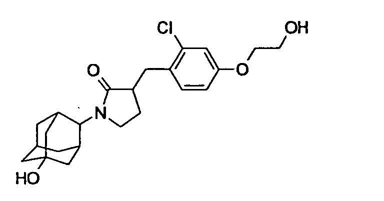

3-[2-Chloro-4-(2-hydroxyethoxy)benzyl]-1-(5-hydroxy-2-adamantyl)pyrrolidin-2-one (a mixture of two steric isomers) obtained in Example 581A was subjected to optical resolution with normal phase chiral high performance liquid chromatography (manufactured by Daicel Chemical Industries, Ltd., chiralpak AD (trade name), 50 mmID X 500 mmL, hexane-ethanol 1:1) to give the title compound (Example 582A) from a fraction with a retention time of 14 min and the title compound (Example 583A) from a fraction with a retention time of 20 min.

### Example 582A (retention time 14 min)

1H NMR (300 MHz, CDCl₃) δ ppm 1.42 (s, 1 H), 1.46-1.57 (m, 2 H), 1.58-1.86 (m, 7 H), 1.86-2.10 (m, 4 H), 2.13-2.22 (m, 1 H), 2.39-2.51 (m, 2 H), 2.68-2.81 (m, 2 H), 3.22-3.35 (m, 1 H), 3.38-3.57 (m, 2 H), 3.87-4.00 (m, 3 H), 4.01-4.09 (m, 2 H), 6.77 (dd, J=8.5, 2.6 Hz, 1 H), 6.94 (d, J=2.6 Hz, 1 H), 7.19 (d, J=8.5 Hz, 1 H).

### Example 583A (retention time for 20 min)

1H NMR (300 MHz, CDCl₃) δ ppm 1.45 (s, 1 H), 1.47-1.56 (m, 2 H), 1.61-1.85 (m, 7 H), 1.86-2.10 (m, 4 H), 2.13-2.21 (m, 1 H), 2.39-2.52 (m, 2 H), 2.67-2.81 (m, 2 H), 3.22-3.35 (m, 1 H), 3.38-3.57 (m, 2 H), 3.87-4.00 (m, 3 H), 4.01-4.09 (m, 2 H), 6.77 (dd, J=8.5, 2.6 Hz, 1 H), 6.94 (d, J=2.6 Hz, 1 H), 7.18 (d, J=8.5 Hz, 1 H).

### Example 584A 4-[(3-chloro-4-{[1-(5-hydroxy-2-adamantyl)-2-oxopyrrolidin-3-yl]methyl}phenoxy)methyl]benzoic acid

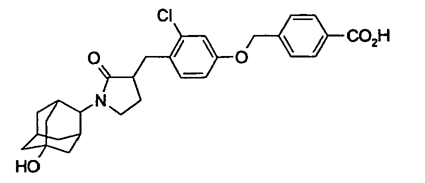

In the same manner as in Example 155A, the title compound was obtained from methyl 4-[(3-chloro-4-{[1-(5-hydroxy-2-adamantyl)-2-oxopyrrolidin-3-yl]methyl}phenoxy)methyl]benzoate obtained in Example 576A.
1H NMR (300 MHz, DMSO-d₆) δ 1.31-1.46 (m, 2 H), 1.50-1.81 (m, 9 H), 1.82-1.97 (m, 1 H), 2.03 (brs, 1 H), 2.20-2.39 (m, 2 H), 2.45-2.70 (m, 2 H), 3.12 (dd, J=12.9, 3.5 Hz, 1 H), 3.24-3.65 (m, 3H), 4.43 (brs, 1 H), 5.20 (s, 2 H), 6.94 (dd, J=8.5, 2.5 Hz, 1 H), 7.12 (d, J=2.6 Hz, 1 H), 7.27 (d, J=8.5 Hz, 1 H), 7.55 (d, J=8.3 Hz, 2 H), 7.96 (d, J=8.3 Hz, 2 H), 12.98 (brs, 1 H). Example 585A (3-chloro-4-{[1-(5-hydroxy-2-adamantyl)-2-oxopyrrolidin-3-yl]methyl}phenoxy)acetic acid

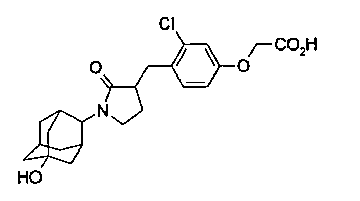

In the same manner as in Example 155A, the title compound was obtained from methyl (3-chloro-4-{[1-(5-hydroxy-2-adamantyl)-2-oxopyrrolidin-3-yl]methyl}phenoxy)acetate obtained in Example 575A.
1H NMR (300 MHz, DMSO-d₆) δ ppm 1.33-1.46 (m, 2 H), 1.53-1.82 (m, 9 H), 1.83-1.97 (m, 1 H), 2.04 (brs, 1 H), 2.23-2.38 (m, 2 H), 2.55-2.70 (m, 1 H), 3.12 (dd, J=13.1, 3.7 Hz, 1 H), 3.23-3.57 (m, 3 H), 3.70 (brs, 1 H), 4.43 (brs, 1 H), 4.69 (s, 2 H), 6.85 (dd, J=8.5, 2.6 Hz, 1 H), 6.99 (d, J=2.6 Hz, 1 H), 7.26 (d, J=8.5 Hz, 1 H), 13.05 (brs, 1 H).

### Example 586A 3-{2-chloro-4-[(5-methyl-2-phenyl-1,3-oxazol-4-yl)methoxy]benzyl}-1-(4-hydroxy-4-methylcyclohexyl)pyrrolidin-2-one

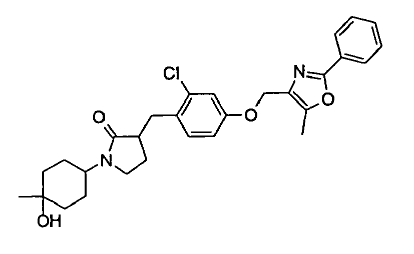

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and 4-(chloromethyl)-5-methyl-2-phenyl-1,3-oxazole.
1H NMR (300 MHz, CDCl₃) δ ppm 1.27 (s, 3 H), 1.38-1.83 (m, 10 H), 1.93-2.09 (m, 1 H), 2.44 (s, 3 H), 2.68-2.86 (m, 2 H), 3.12-3.38 (m, 3 H), 3.89-4.06 (m, 4 H), 4.95 (s, 2 H), 6.86 (dd, J=8.6, 2.5 Hz, 1 H), 7.05 (d, J=2.5 Hz, 1 H), 7.18 (d, J=8.6 Hz, 1 H), 7.39-7.49 (m, 3 H), 7.96-8.06 (m, 1 H).

### Example 587A 3-(2-chloro-4-{[5-(trifluoromethyl)-1,3-benzothiazol-2-yl]methoxy}benzyl)-1-(4-hydroxy-4-methylcyclohexyl)pyrrolidin-2-one

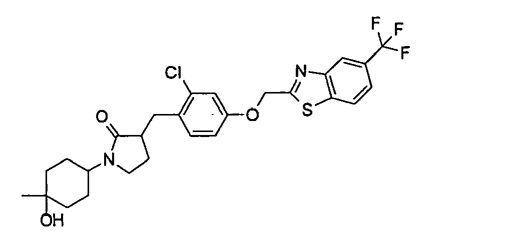

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and 2-(chloromethyl)-5-(tri.fluoromethyl)-1,3-benzothiazole.
1H NMR (300 MHz, CDCl₃) δ ppm 1.27 (s, 3 H), 1.36-1.81 (m, 10 H), 1.95-2.10 (m, 1 H), 2.68-2.85 (m, 2 H), 3.12-3.36 (m, 3 H), 3.90-4.05 (m, 1 H), 5.47 (s, 2 H), 6.88 (dd, J=8.5, 2.6 Hz, 1 H), 7.07 (d, J=2.6 Hz, 1 H), 7.22 (d, J=8.5 Hz, 1 H), 7.65 (dd, J=8.5, 1.3 Hz, 1 H), 8.02 (d, J=8.5 Hz, 1 H), 8.30 (brs, 1 H).

### Example 588A 3-{2-chloro-4-[(5-methyl-2-phenyl-1,3-oxazol-4-yl)methoxy]benzyl}-1-(5-hydroxy-2-adamantyl)pyrrolidin-2-one

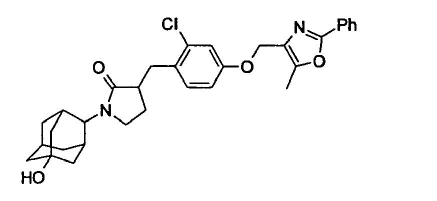

In the same manner as in Example 318A, the title compound was obtained from 3-(2-chloro-4-hydroxybenzyl)-1-[5-hydroxy-2-adamantyl]pyrrolidin-2-one obtained in Example 431A and 4-(chloromethyl)-5-methyl-2-phenyl-1,3-oxazole.
1H NMR (300 MHz, CDCl₃) δ ppm 1.38-1.57 (m, 3 H), 1.67-2.10 (m, 10 H), 2.12-2.22 (m, 1 H), 2.38-2.53 (m, 5 H), 2.67-2.83 (m, 2 H), 3.23-3.56 (m, 3 H), 3.88-3.94 (m, 1 H), 4.95 (s, 2 H), 6.86 (dd, J=8.5, 2.5 Hz, 1 H), 7.05 (d, J=2.5 Hz, 1 H), 7.19 (d, J=8.5 Hz, 1 H), 7.39-7.48 (m, 3 H), 7.96-8.06 (m, 1 H).

### Example 589A methyl 3-[(3-chloro-4-{[1-(5-hydroxy-2-adamantyl)-2-oxopyrrolidin-3-yl]methyl}phenoxy)methyl]benzoate

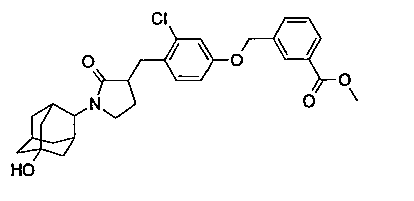

In the same manner as in Example 318A, the title compound was obtained from 3-(2-chloro-4-hydroxybenzyl)-1-[5-hydroxy-2-adamantyl]pyrrolidin-2-one obtained in Example 431A and methyl 3-(bromomethyl)benzoate.
1H NMR (300 MHz, CDCl₃) δ ppm 1.44-1.59 (m, 3 H), 1.62-1.85 (m, 7 H), 1.86-1.95 (m, 2 H), 1.98-2.10 (m, 1 H), 2.13-2.21 (m, 1 H), 2.38-2.51 (m, 2 H), 2.67-2.80 (m, 2 H), 3.21-3.59 (m, 3 H), 3.87-3.97 (m, 4 H), 5.06 (s, 2 H), 6.81 (dd, J=8.5, 2.6 Hz, 1 H), 7.00 (d, J=2.6 Hz, 1 H), 7.19 (d, J=8.5 Hz, 1 H), 7.41-7.52 (m, 1 H), 7.58-7.66 (m, 1 H), 7.97-8.13 (m, 2 H).

### Example 590A 2-[2-(3-chloro-4-{[1-(5-hydroxy-2-adamantyl)-2-oxopyrrolidin-3-yl]methyl}phenoxy)ethyl]-1H-isoindole-1,3(2H)-dione

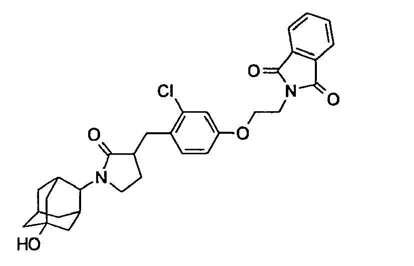

In the same manner as in Example 338A, the title compound was obtained from 3-(2-chloro-4-hydroxybenzyl)-1-[5-hydroxy-2-adamantyl]pyrrolidin-2-one obtained in Example 431A and 2-(2-hydroxyethyl)-1H-isoindole-1,3(2H)-dione.
1H NMR (300 MHz, CDCl₃) δ ppm 1.44-1.57 (m, 2 H), 1.65-1.83 (m, 5 H), 1.85-2.07 (m, 3 H), 2.11-2.20 (m, 1 H), 2.36-2.49 (m, 2 H), 2.65-2.85 (m, 2 H), 2.87-3.08 (m, 3 H), 3.17-3.31 (m, 1 H), 3.38-3.55 (m, 2 H), 3.87-3.94 (m, 1 H), 4.05-4.13 (m, 2 H), 4.15-4.24 (m, 2 H), 6.72 (dd, J=8.6, 2.5 Hz, 1 H), 6.89 (d, J=2.5 Hz, 1 H), 7.12 (d, J=8.6 Hz, 1 H), 7.69-7.78 (m, 2 H), 7.82-7.91 (m, 2 H).

### Example 591A 3-[(3-chloro-4-{[1-(5-hydroxy-2-adamantyl)-2-oxopyrrolidin-3-yl]methyl}phenoxy)methyl]benzoic acid

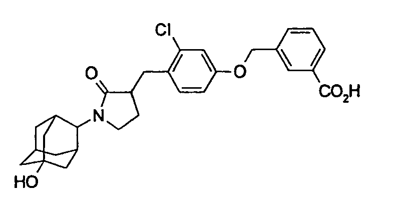

In the same manner as in Example 155A, the title compound was obtained from methyl 3-[(3-chloro-4-{[1-(5-hydroxy-2-adamantyl)-2-oxopyrrolidin-3-yl]methyl}phenoxy)methyl]benzoate obtained in Example 589A.
1H NMR (300 MHz, DMSO-d₆) δ ppm 1.32-1.45 (m, 2 H), 1.52-1.80 (m, 9 H), 1.83-1.97 (m, 1 H), 2.00-2.07 (m, 1 H), 2.23-2.38 (m, 2 H), 2.52-2.68 (m, 2 H), 3.12 (dd, J=13.1, 3.5 Hz, 1 H), 3.66-3.74 (m, 3 H), 3.70 (brs, 1 H), 4.43 (brs; 1 H), 5.19 (s, 2 H), 6.95 (dd, J=8.5, 2.5 Hz, 1 H), 7.12 (d, J=2.5 Hz, 1 H), 7.27 (d, J=8.5 Hz, 1 H), 7.66-7.72 (m, 1-H), 7.88-7.93 (m, 1 H), 7.98-8.05 (m, 1 H), 13.06 (brs, 1 H).

### Example 592A 3-(2-chloro-4-{[3-(hydroxymethyl)benzyl]oxy}benzyl)-1-(5-hydroxy-2-adamantyl)pyrrolidin-2-one

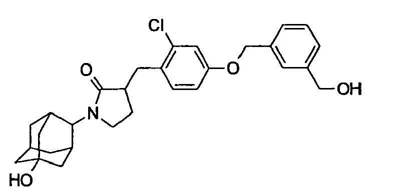

In the same manner as in Reference Example 19A, the title compound was obtained from methyl 3-[(3-chloro-4-{[1-(5-hydroxy-2-adamantyl)-2-oxopyrrolidin-3-yl]methyl}phenoxy)methyl]benzoate obtained in Example 589A.
1H NMR (300 MHz, CDCl₃) δ ppm 1.35-1.43 (m, 1 H), 1.46-1.56 (m, 2 H), 1.66-2.10 (m, 11 H), 2.13-2.21 (m, 1 H), 2.38-2.50 (m, 2 H), 2.67-2.81 (m, 2 H), 3.22-3.34 (m, 1 H), 3.38-3.57 (m, 2 H), 3.88-3.93 (m, 1 H), 4.69-4.77 (m, 2 H), 5.03 (s, 2 H), 6.81 (dd, J=8.5, 2.5 Hz, 1 H), 7.00 (d, J=2.5 Hz, 1 H), 7.18 (d, J=8.5 Hz, 1 H), 7.31-7.46 (m, 4 H).

### Example 593A 2-(3-chloro-4-{[1-(5-hydroxy-2-adamantyl)-2-oxopyrrolidin-3-yl]methyl}phenoxy)-N-[4-(trifluoromethyl)benzyl]acetamide

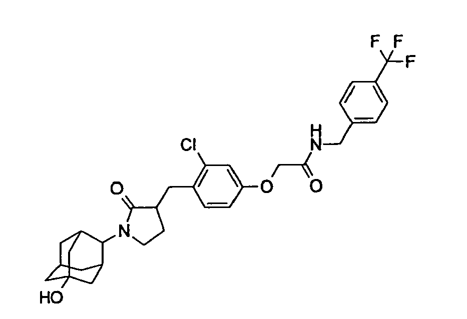

In the same manner as in Example 570A, the title compound was obtained from methyl (3-chloro-4-{[1-(5-hydroxy-2-adamantyl)-2-oxopyrrolidin-3-yl]methyl}phenoxy)acetate obtained in Example 575A and 1-[4-(trifluoromethyl)phenyl]methanamine.
1H NMR (300 MHz, CDCl₃) δ ppm 1.46-1.85 (m, 11 H), 1.86-2.11 (m, 2 H), 2.13-2.22 (m, 1 H), 2.38-2.51 (m, 2 H), 2.67-2.82 (m, 2 H), 3.21-3.59 (m, 3 H), 3.85-3.94 (m, 1 H), 4.54 (s, 2 H), 4.60 (d, J=6.2 Hz, 2 H), 6.76 (dd, J=8.5, 2.7 Hz, 1 H), 6.87-6.99 (m, 2 H), 7.23 (d, J=8.5 Hz, 1 H), 7.40 (d, J=8.1 Hz, 1 H), 7.60 (d, J=8.1 Hz, 2 H).

### Example 594A 2-(3-chloro-4-{[1-(5-hydroxy-2-adamantyl)-2-oxopyrrolidin-3-yl]methyl}phenoxy)-N-(cyclopropylmethyl)acetamide

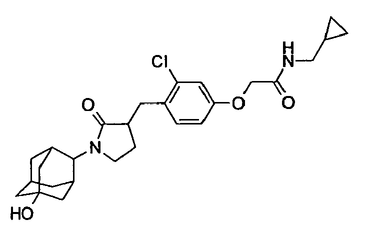

In the same manner as in Example 570A, the title compound was obtained from methyl (3-chloro-4-{[1-(5-hydroxy-2-adamantyl)-2-oxopyrrolidin-3-yl]methyl}phenoxy)acetate obtained in Example 575A and 1-cyclopropylmethanamine.
1H NMR (300 MHz, CDCl₃) δ ppm 0.18-0.28 (m, 2 H), 0.48-0.59 (m, 2 H), 0.90-1.07 (m, 1 H), 1.40-1.97 (m, 12 H), 1.98-2.11 (m, 1 H), 2.13-2.22 (m, 1 H), 2.39-2.53 (m, 2 H), 2.68-2.83 (m, 2 H), 3.16-3.37 (m, 3 H), 3.38-3.60 (m, 2 H), 3.87-3.96 (m, 1 H), 4.46 (s, 2 H), 6.56-6.67 (m, 1 H), 6.79 (dd, J=8.5, 2.6 Hz, 1 H), 6.98 (d, J=2.6 Hz, 1 H), 7.23 (d, J=8.5 Hz, 1 H).

### Example 595A 2-(3-chloro-4-{[1-(4-hydroxy-4-methylcyclohexyl)-2-oxopyrrolidin-3-yl]methyl}phenoxy)-N-(4-methoxybenzyl)acetamide

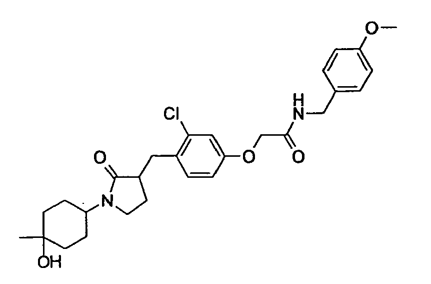

In the same manner as in Example 570A, the title compound was obtained from methyl (3-chloro-4-{[1-(4-hydroxy-4-methylcyclohexyl)-2-oxopyrrolidin-3-yl]methyl}phenoxy)acetate obtained in Example 551A and 4-methoxybenzylamine.
1H NMR (300 MHz, CDCl₃) δ ppm 1.27 (s, 3 H), 1.34-1.82 (m, 11 H), 1.94-2.08 (m, 1 H), 2.68-2.81 (m, 2 H), 3.14-3.35 (m, 3 H), 3.80 (s, 3 H), 3.91-4.04 (m, 1 H), 4.42-4.54 (m, 3 H), 6.71-6.81 (m, 2 H), 6.83-6.96 (m, 3 H), 7.16-7.25 (m, 3 H).

### Example 596A 2-(3-chloro-4-{[1-(4-hydroxy-4-methylcyclohexyl)-2-oxopyrrolidin-3-yl]methyl}phenoxy)-N-(cyclopropylmethyl)acetamide

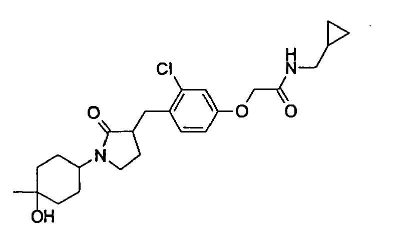

In the same manner as in Example 570A, the title compound was obtained from methyl (3-chloro-4-{[1-(4-hydroxy-4-methylcyclohexyl)-2-oxopyrrolidin-3-yl]methyl}phenoxy)acetate obtained in Example 551A and 1-cyclopropylmethanamine.
1H NMR (300 MHz, CDCl₃) δ ppm 0.18-0.28 (m, 2 H), 0.48-0.59 (m, 2 H), 0.91-1.07 (m, 1 H), 1.27 (s, 3 H), 1.42-1.82 (m, 10 H), 1.95-2.10 (m, 1 H), 2.70-2.84 (m, 2 H), 3.14-3.36 (m, 5 H), 3.91-4.05 (m, 1 H), 4.46 (s, 2 H), 6.65-6.66 (m, 1 H), 6.79 (dd, J=8.5, 2.6 Hz, 1 H), 6.97 (d, J=2.6 Hz, 1 H), 7.23 (d, J=8.5 Hz, 1 H).

### Example 597A methyl (3-chloro-4-{[1-(4-hydroxy-4-methylcyclohexyl)-2-oxopyrrolidin-3-yl]methyl}phenoxy)acetate (an optically active form 1)

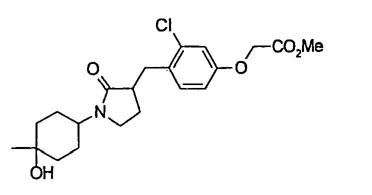

In the same manner as in Example 318A, the title compound was obtained from 3-(2-chloro-4-hydroxybenzyl)-1-(4-hydroxy-4-methylcyclohexyl)pyrrolidin-2-one obtained in Example 543A and methyl bromoacetate.
1H NMR (300 MHz, CDCl₃) δ ppm 1.27 (s, 3 H), 1.41-1.81 (m, 10 H), 1.94-2.08 (m, 1 H), 2.67-2.83 (m, 2 H), 3.14-3.36 (m, 3 H), 3.81 (s, 3 H), 3.91-4.05 (m, 1 H), 4.61 (s, 2 H), 6.75 (dd, J=8.5, 2.6 Hz, 1 H), 6.92 (d, J=2.6 Hz, 1 H), 7.19 (d, J=8.5 Hz, 1 H). Example 598A methyl (3-chloro-4-{[1-(4-hydroxy-4-methylcyclohexyl)-2-oxopyrrolidin-3-yl]methyl}phenoxy)acetate (an optically active form 2)

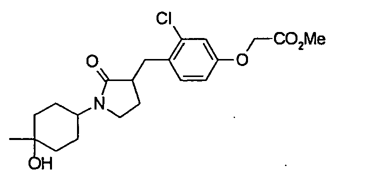

In the same manner as in Example 318A, the title compound was obtained from 3-(2-chloro-4-hydroxybenzyl)-1-(4-hydroxy-4-methylcyclohexyl)pyrrolidin-2-one obtained in Example 544A and methyl bromoacetate.
1H NMR (300 MHz, CDCl₃) δ ppm 1.27 (s, 3 H), 1.41-1.82 (m, 10 H), 1.94-2.09 (m, 1 H), 2.67-2.81 (m, 2 H), 3.14-3.36 (m, 3 H), 3.81 (s, 3 H), 3.90-4.05 (m, 1 H), 4.60 (s, 2 H), 6.75 (dd, J=8.5, 2.6 Hz, 1 H), 6.92 (d, J=2.6 Hz, 1 H), 7.19 (d, J=8.5 Hz, 1 H).

### Example 599A 3-(2-chloro-4-{[5-(trifluoromethyl)-1,3-benzothiazol-2-yl]methoxy}benzyl)-1-(5-hydroxy-2-adamantyl)pyrrolidin-2-one

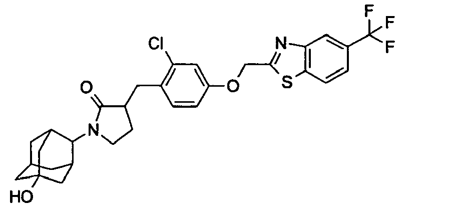

In the same manner as in Example 318A, the title compound was obtained from 3-(2-chloro-4-hydroxybenzyl)-1-(5-hydroxy-2-adamantyl)pyrrolidin-2-one obtained in Example 431A and 2-(chloromethyl)-5-(trifluoromethyl)-1,3-benzothiazole.
1H NMR (300 MHz, CDCl₃) δ ppm 1.37-1.84 (m, 10 H), 1.85-2.11 (m, 4 H), 2.37-2.51 (m, 2 H), 2.68-2.82 (m, 2 H), 3.22-3.36 (m, 1 H), 3.37-3.58 (m, 2 H), 3.87-3.94 (m, 1 H), 5.47 (s, 2 H), 6.88 (dd, J=8.5, 2.6 Hz, 1 H), 7.07 (d, J=2.6 Hz, 1 H), 7.23 (d, J=8.5 Hz, 1 H), 7.62-7.69 (m, 1 H), 8.02 (d, J=8.5 Hz, 1 H), 8.30 (s, 1 H).

### Example 600A 2-(3-chloro-4-{[1-(5-hydroxy-2-adamantyl)-2-oxopyrrolidin-3-yl]methyl}phenoxy)-N-(4-methoxybenzyl)acetamide

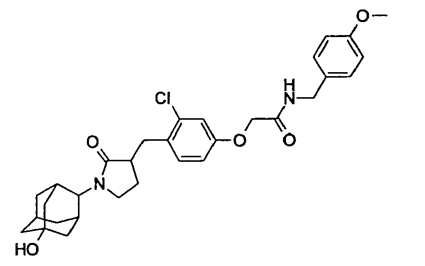

In the same manner as in Example 570A, the title compound was obtained from methyl (3-chloro-4-1[1-(5-hydroxy-2-adamantyl)-2-oxopyrrolidin-3-yl]methyl}phenoxy)acetate obtained in Example 575A and 4-methoxybenzylamine.
1H NMR (300 MHz, CDCl₃) δ ppm 1.43-2.09 (m, 13 H), 2.13-2.22 (m, 1 H), 2.38-2.51 (m, 2 H), 2.67-2.81 (m, 2 H), 3.20-3.35 (m, 1 H), 3.37-3.59 (m, 2 H), 3.80 (s, 3 H), 3.87-3.94 (m, 1 H), 4.42-4.54 (m, 4 H), 6.71-6.81 (m, 2 H), 6.84-6.95 (m, 3 H), 7.16-7.26 (m, 3 H).

### Example 601A 2-(3-chloro-4-{[1-(5-hydroxy-2-adamantyl)-2-oxopyrrolidin-3-yl]methyl}phenoxy)-N-(2,4-difluorobenzyl)acetamide

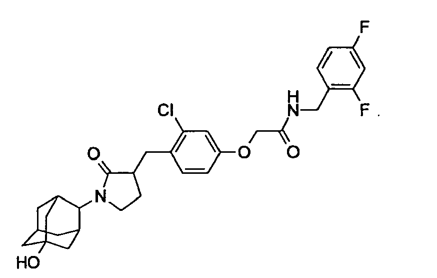

In the same manner as in Example 570A, the title compound was obtained from methyl (3-chloro-4-{[1-(5-hydroxy-2-adamantyl)-2-oxopyrrolidin-3-yl]methyl}phenoxy)acetate obtained in Example 575A and 2,4-difluorobenzylamine.
1H NMR (300 MHz, CDCl₃) δ ppm 1.34-1.41 (m, 1 H), 1.45-2.10 (m, 12 H), 2.13-2.22 (m, 1 H), 2.40-2.51 (m, 2 H), 2.68-2.81 (m, 2 H), 3.22-3.33 (m, 1 H), 3.39-3.58 (m, 2 H), 3.88-3.94 (m, 1 H), 4.48 (s, 2 H), 4.54 (d, J=6.2 Hz, 2 H), 6.71-6.97 (m, 5 H), 7.17-7.40 (m, 2 H).

### Example 602A 2-(3-chloro-4-{[1-(4-hydroxy-4-methylcyclohexyl)-2-oxopyrrolidin-3-yl]methyl}phenoxy)-N-[4-(trifluoromethyl)benzyl]acetamide (an optically active form 1)

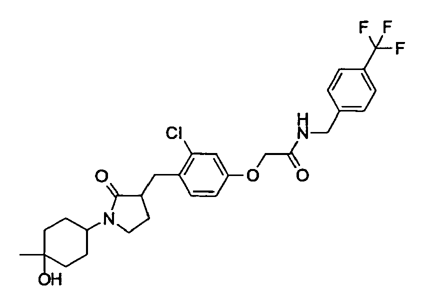

In the same manner as in Example 570A, the title compound was obtained from methyl (3-chloro-4-{[1-(4-hydroxy-4-methylcyclohexyl)-2-oxopyrrolidin-3-yl]methyl}phenoxy)acetate obtained in Example 597A and 1-[4-(trifluoromethyl)phenyl]methanamine.
1H NMR (300 MHz, CDCl₃) δ ppm 1.27 (s, 3 H), 1.41-1.83 (m, 10 H), 1.94-2.09 (m, 1 H), 2.67-2.85 (m, 2 H), 3.13-3.35 (m, 3 H), 3.91-4.04 (m, 1 H), 4.54 (s, 2 H), 4.60 (d, J=6.2 Hz, 2 H), 6.76 (dd, J=8.5, 2.7 Hz, 1 H), 6.86-6.98 (m, 2 H), 7.22 (d, J=8.5 Hz, 1 H), 7.40 (d, J=8.1 Hz, 2 H), 7.60 (d, J=8.1 Hz, 2 H).

### Example 603A 2-(3-chloro-4-{[1-(4-hydroxy-4-methylcyclohexyl)-2-oxopyrrolidin-3-yl]methyl}phenoxy)-N-[4-(trifluoromethyl)benzyl]acetamide (an optically active form 2)

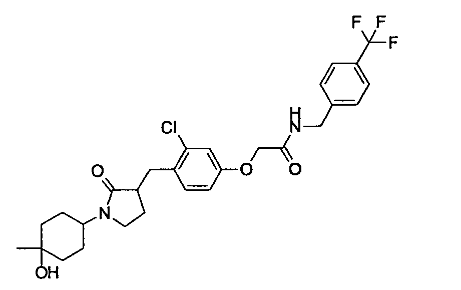

In the same manner as in Example 570A, the title compound was obtained from methyl (3-chloro-4-{[1-(4-hydroxy-4-methylcyclohexyl)-2-oxopyrrolidin-3-yl]methyl}phenoxy)acetate obtained in Example 598A and 1-[4-(trifluoromethyl)phenyl]methanamine.
1H NMR (300 MHz, CDCl₃) δ ppm 1.27 (s, 3 H), 1.36-1.82 (m, 10 H), 1.94-2.09 (m, 1 H), 2.68-2.82 (m, 2 H), 3.13-3.35 (m, 3 H), 3.91-4.04 (m, 1H), 4.54 (s, 2 H), 4.60 (d, J=6.0 Hz, 2 H), 6.76 (dd, J=8.5, 2.7 Hz, 1 H), 6.87-6.98 (m, 2 H), 7.22 (d, J=8.5 Hz, 1 H), 7.40 (d, J=8.1 Hz, 2 H), 7.60 (d, J=8.1 Hz, 2 H).

### Example 604A 3-{2-chloro-4-[(1,3-thiazol-4-yl)methoxy]benzyl}-1-(4-hydroxy-4-methylcyclohexyl)pyrrolidin-2-one

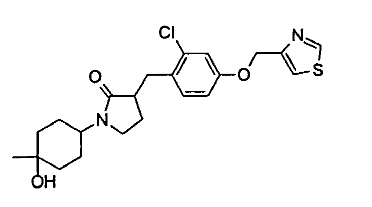

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and 4-(chloromethyl)-1,3-thiazole.
1H NMR (300 MHz, CDCl₃) δ ppm 1.27 (s, 3 H), 1.37-1.86 (m, 10 H), 1.92-2.11 (m, 1 H), 2.67-2.86 (m, 2 H), 3.11-3.39 (m, 3 H), 3.90-4.06 (m, 1 H), 5.23 (s, 2 H), 6.84 (dd, J=8.5, 2.5 Hz, 1 H), 7.03 (d, J=2.5 Hz, 1 H), 7.19 (d, J=8.5 Hz, 1 H), 7.34-7.46 (m, 1 H), 8.84 (d, J=1.9 Hz, 1 H).

### Example 605A 3-{2-chloro-4-[(1,3-thiazol-4-yl)methoxy]benzyl}-1-(5-hydroxy-2-adamantyl)pyrrolidin-2-one

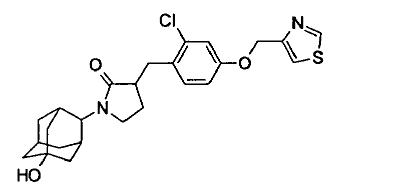

In the same manner as in Example 318A, the title compound was obtained from 3-(2-chloro-4-hydroxybenzyl)-1-[5-hydroxy-2-adamantyl]pyrrolidin-2-one obtained in Example 431A and 4-(chloromethyl)-1,3-thiazole.
1H NMR (300 MHz, CDCl₃) δ ppm 1.46-1.60 (m, 3 H), 1.62-2.26 (m, 11 H), 2.37-2.55 (m, 2 H), 2.65-2.85 (m, 2 H), 3.22-3.59 (m, 3 H), 3.86-3.99 (m, 1 H), 5.23 (s, 2 H), 6.84 (dd, J=8.5, 2.5 Hz, 1 H), 7.03 (d, J=2.5 Hz, 1 H), 7.19 (d, J=8.5 Hz, 1 H), 7.33-7.49 (m, 1 H), 8.84 (d, J=2.1 Hz, 1 H).

### Example 606A 3-{2-chloro-4-[(2-methyl-1,3-thiazol-4-yl)methoxy]benzyl}-1-(4-hydroxy-4-methylcyclohexyl)pyrrolidin-2-one

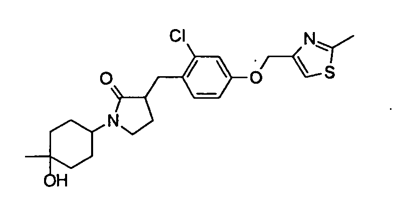

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and 4-(chloromethyl)-2-methyl-1,3-thiazole.
1H NMR (300 MHz, CDCl₃) δ ppm 1.27 (s, 3 H), 1.39-1.82 (m, 10 H), 1.94-2.09 (m, 1 H), 2.68-2.83 (m, 5 H), 3.13-3.37 (m, 3 H), 3.90-4.06 (m, 1 H), 5.10 (s, 2 H), 6.83 (dd, J=8.3, 2.7 Hz, 1 H), 7.01 (d, J=2.7 Hz, 1 H), 7.12-7.22 (m, 2 H).

### Example 607A 3-{2-chloro-4-[(2-methyl-1,3-thiazol-4-yl)methoxy]benzyl}-1-(5-hydroxy-2-adamantyl)pyrrolidin-2-one

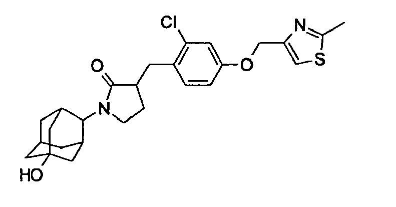

In the same manner as in Example 318A, the title compound was obtained from 3-(2-chloro-4-hydroxybenzyl)-1-[5-hydroxy-2-adamantyl]pyrrolidin-2-one obtained in Example 431A and 4-(chloromethyl)-2-methyl-1,3-thiazole.
1H NMR (300 MHz, CDCl₃) δ ppm 1.46-1.58 (m, 3 H), 1.65-1.86 (m, 7 H), 1.87-2.11 (m, 1 H), 2.12-2.22 (m, 1 H), 2.37-2.53 (m, 2 H), 2.67-2.83 (m, 5 H), 3.21-3.58 (m, 3 H), 3.87-3.96 (m, 1 H), 5.11 (s, 2 H), 6.83 (dd, J=8.7, 2.7 Hz, 1 H), 7.01 (d, J=2.7 Hz, 1 H), 7.12-7.24 (m, 2 H).

### Example 608A ethyl 4-[(3-chloro-4-{[1-(4-hydroxy-4-methylcyclohexyl)-2-oxopyrrolidin-3-yl]methyl}phenoxy)methyl]-1,3-thiazole-2-carboxylate

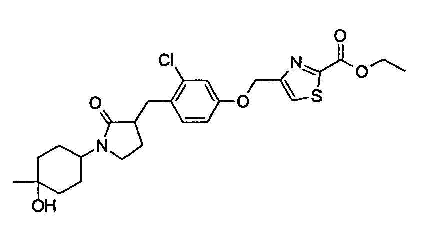

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and ethyl 4-(chloromethyl)-1,3-thiazole-2-carboxylate.
1H NMR (300 MHz, CDCl₃) δ ppm 1.27 (s, 3 H), 1.35-1.84 (m, 13 H), 1.95-2.10 (m, 1 H), 2.67-2.85 (m, 2 H), 3.13-3.37 (m, 3 H), 3.91-4.06 (m, 1 H), 4.51 (q, J=7.2 Hz, 2 H), 5.26 (s, 2 H), 6.81 (dd, J=8.3, 2.7 Hz, 1 H), 6.99 (d, J=2.7 Hz, 1 H), 7.19 (d, J=8.3 Hz, 1H), 7.62 (s, 1H).

### Example 609A ethyl 4-[(3-chloro-4-{[1-(5-hydroxy-2-adamantyl)-2-oxopyrrolidin-3-yl]methyl}phenoxy)methyl]-1,3-thiazole-2-carboxylate

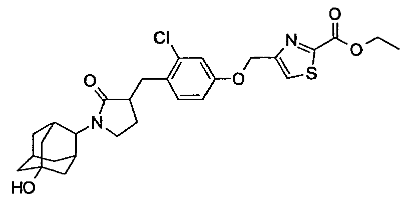

In the same manner as in Example 318A, the title compound was obtained from 3-(2-chloro-4-hydroxybenzyl)-1-[5-hydroxy-2-adamantyl]pyrrolidin-2-one obtained in Example 431A and ethyl 4-(chloromethyl)-1,3-thiazole-2-carboxylate.
1H NMR (300 MHz, CDCl₃) δ ppm 1.39-2.12 (m, 16 H), 2.12-2.24 (m, 1 H), 2.36-2.54 (m, 2 H), 2.67-2.84 (m, 2 H), 3.20-3.59 (m, 3 H), 3.86-3.96 (m, 1 H), 4.51 (q, J=7.2 Hz, 2 H), 5.26 (s, 2 H), 6.81 (dd, J=8.7, 2.7 Hz, 1 H), 7.00 (d, J=2.7 Hz, 1 H), 7.20 (d, J=8.7 Hz, 1 H), 7.63 (s, 1 H).

### Example 610A 2-(3-chloro-4-{[1-(4-hydroxy-4-methylcyclohexyl)-2-oxopyrrolidin-3-yl]methyl}phenoxy)-N-(4-fluorophenyl)acetamide

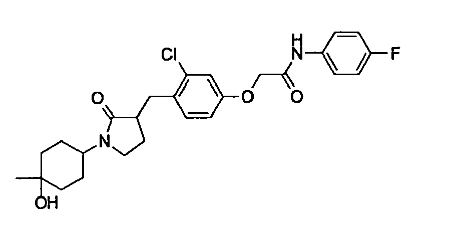

In the same manner as in Example 570A, the title compound was obtained from methyl (3-chloro-4-{[1-(4-hydroxy-4-methylcyclohexyl)-2-oxopyrrolidin-3-yl]methyl}phenoxy)acetate obtained in Example 551A and 4-fluoroaniline.
1H NMR (300 MHz, CDCl₃) δ ppm 1.27 (s, 3 H), 1.33-1.82 (m, 10 H), 1.97-2.11 (m, 1 H), 2.71-2.85 (m, 2 H), 3.14-3.38 (m, 3 H), 3.91-4.06 (m, 1 H), 4.58 (s, 2 H), 6.84 (dd, J=8.3, 2.7 Hz, 1 H), 6.99-7.12 (m, 3 H), 7,22-7.30 (m, 1 H), 7.50-7.62 (m, 2 H), 8.19 (brs, 1 H).

### Example 611A 3-(2-chloro-4-{[2-(hydroxymethyl)-1,3-thiazol-4-yl]methoxy}benzyl)-1-(4-hydroxy-4-methylcyclohexyl)pyrrolidin-2-one

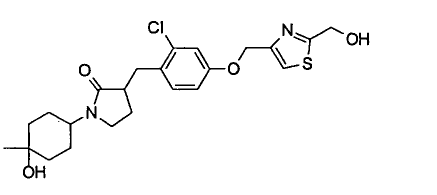

In the same manner as in Reference Example 19A, the title compound was obtained from ethyl 4-[(3-chloro-4-{[1-(4-hydroxy-4-methylcyclohexyl)-2-oxopyrrolidin-3-yl]methyl}phenoxy)methyl]-1,3-thiazole-2-carboxylate obtained in Example 608A.
1H NMR (300 MHz, CDCl₃) δ ppm 1.27 (s, 3 H), 1.41-1.62 (m, 10 H), 1.93-2.08 (m, 1 H), 2.67-2.84 (m, 2 H), 3.01 (t, J=6.1 Hz, 2 H), 3.11-3.34 (m, 3 H), 3.89-4.05 (m, 1 H), 4.97 (d, J=6.1 Hz, 2 H), 5.12 (s, 2 H), 6.82 (dd, J=8.7, 2.7 Hz, 1 H), 7.01 (d, J=2.7 Hz, 1H), 7.18 (d, J=8.7 Hz, 1H), 7.30 (s, 1H).

### Example 612A 3-(2-chloro-4-{[2-(hydroxymethyl)-1,3-thiazol-4-yl]methoxy}benzyl)-1-(5-hydroxy-2-adamantyl)pyrrolidin-2-one

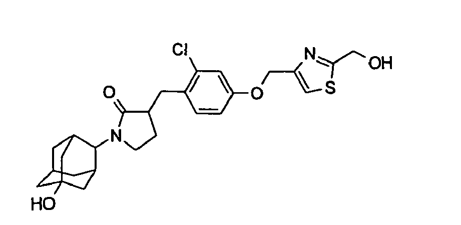

In the same manner as in Reference Example 19A, the title compound was obtained from ethyl 4-[(3-chloro-4-1[1-(5-hydroxy-2-adamantyl)-2-oxopyrrolidin-3-yl]methyl}phenoxy)methyl]-1,3-thiazole-2-carboxylate obtained in Example 609A.
1H NMR (300 MHz, CDCl₃) δ ppm 1.39-2.11 (m, 13 H), 2.13-2.22 (m, 1 H), 2.39-2.49 (m, 2 H), 2.68-2.95 (m, 3 H), 3.21-3.55 (m, 3 H), 3.86-3.94 (m, 1 H), 4.97 (s, 2 H), 5.13 (s, 2 H), 6.83 (dd, J=8.5, 2.7 Hz, 1 H), 7.01 (d, J=2.7 Hz, 1 H), 7.18 (d, J=8.5 Hz, 1 H), 7.30 (s, 1 H).

### Example 613A N-benzyl-2-(3-chloro-4-{[1-(4-hydroxy-4-methylcyclohexyl)-2-oxopyrrolidin-3-yl]methyl}phenoxy)acetamide

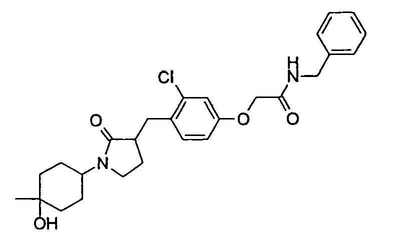

In the same manner as in Example 570A, the title compound was obtained from methyl (3-chloro-4-{[1-(4-hydroxy-4-methylcyclohexyl)-2-oxopyrrolidin-3-yl]methyl}phenoxy)acetate obtained in Example 551A and benzylamine.
1H NMR (300 MHz, CDCl₃) δ ppm 1.27 (s, 3 H), 1.38-1.82 (m, 10 H), 1.95-2.08 (m, 1 H), 2.69-2.83 (m, 2 H), 3.13-3.37 (m, 3 H), 3.90-4.05 (m, 1 H), 4.52 (s, 2 H), 4.55 (d, J=5.8 Hz, 2 H), 6.75 (dd, J=8.5, 2.6 Hz, 1 H), 6.79-6.88 (m, 1 H), 6.94 (d, J=2.6 Hz, 1H), 7.21 (d, J=8.5 Hz, 1H), 7.25-7.40 (m, 5 H).

### Example 614A N-benzyl-2-(3-chloro-4-{[1-(5-hydroxy-2-adamantyl)-2-oxopyrrolidin-3-yl]methyl}phenoxy)acetamide

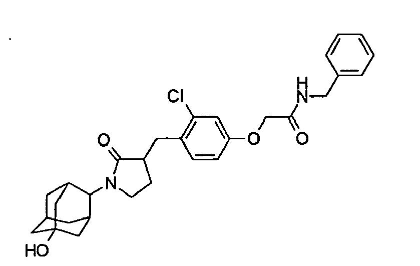

In the same manner as in Example 570A, the title compound was obtained from methyl (3-chloro-4-{[1-(5-hydroxy-2-adamantyl)-2-oxopyrrolidin-3-yl]methyl}phenoxy)acetate obtained in Example 575A and benzylamine.
1H NMR (300 MHz, CDCl₃) δ ppm 1.35-1.57 (m, 3 H), 1.62-2.10 (m, 10 H), 2.13-2.22 (m, 1 H), 2.38-2.51 (m, 2 H), 2.67-2.82 (m, 2 H), 3.21-3.57 (m, 3 H), 3.88-3.93 (m, 1 H), 4.52 (s, 2 H), 4.55 (d, J=6.0 Hz, 2 H), 6.75 (dd, J=8.5, 2.6 Hz, 1 H), 6.78-6.87 (m, 1 H), 6.94 (d, J=2.6 Hz, 1 H), 7.21 (d, J=8.5 Hz, 1 H), 7.24-7.39 (m, 5 H).

### Example 615A 3-(2-chloro-4-{[3-(4-fluorophenyl)-1,2,4-oxadiazol-5-yl]methoxy}benzyl)-1-(5-hydroxy-2-adamantyl)pyrrolidin-2-one

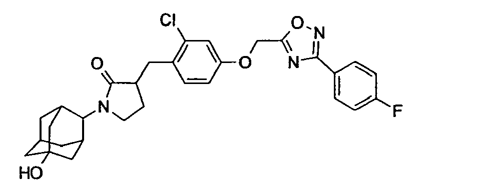

In the same manner as in Example 525A, the title compound was obtained from (3-chloro-4-{[1-(5-hydroxy-2-adamantyl)-2-oxopyrrolidin-3-yl]methyl}phenoxy)acetic acid obtained in Example 585A and 4-fluorobenzamidoxime.
1H NMR (300 MHz, CDCl₃) δ ppm 1.39 (s, 1 H), 1.45-1.96 (m, 11 H), 1.97-2.11 (m, 1 H), 2.13-2.21 (m, 1 H), 2.39-2.51 (m, 2 H), 2.69-2.83 (m, 2 H), 3.23-3.36 (m, 1 H), 3.38-3.57 (m, 2 H), 3.87-3.95 (m, 1 H), 5.31 (s, 2 H), 6.87 (dd, J=8.5, 2.7 Hz, 1 H), 7.07 (d, J=2.7 Hz, 1 H), 7.14 -7.28 (m, 3 H), 8.04-8.16 (m, 2 H).

### Example 616A 2-(3-chloro-4-{[1-(5-hydroxy-2-adamantyl)-2-oxopyrrolidin-3-yl]methyl}phenoxy)acetamide

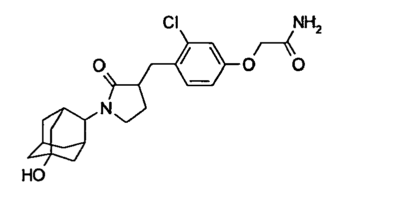

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and ammonium 1H-1,2,3-benzotriazol-1-ol.
1H NMR (300 MHz, CDCl₃) δ ppm 1.33-1.46 (m, 2 H), 1.51-1.96 (m, 10 H), 2.00-2.08 (m, 1 H), 2.23-2.38 (m, 1 H), 2.53-2.70 (m, 2 H), 3.12 (dd, J=13.2, 3.6 Hz, 1 H), 3.28-3.57 (m, 3 H), 3.66-3.74 (m, 1 H), 4.40-4.47 (m, 3 H), 6.88 (dd, J=8.5, 2.6 Hz, 1 H), 7.03 (d, J=2.6 Hz, 1 H), 7.28 (d, J=8.5 Hz, 1 H), 7.41 (brs, 1 H), 7.56 (brs, 1 H).

### Example 617A (3-chloro-4-{[1-(4-hydroxy-4-methylcyclohexyl)-2-oxopyrrolidin-3-yl]methyl}phenoxy)acetic acid

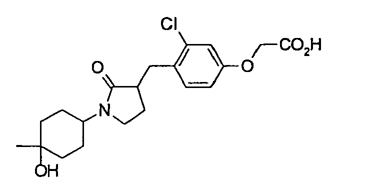

In the same manner as in Example 155A, the title compound was obtained from methyl (3-chloro-4-{[1-(4-hydroxy-4-methylcyclohexyl)-2-oxopyrrolidin-3-yl]methyl}phenoxy)acetate obtained in Example 551A.
1H NMR (300 MHz, DMSO-d₆) δ ppm 1.13 (s, 3 H), 1.37-1.65 (m, 9 H), 1.81-1.95 (m, 1 H), 2.44-2.72 (m, 2 H), 3.05-3.43 (m, 3 H), 3.64-3.78 (m, 1 H), 4.37 (brs, 1 H), 4.69 (s, 2 H), 6.85 (dd, J=8.5, 2.6 Hz, 1 H), 6.99 (d, J=2.6 Hz, 1 H), 7.26 (d, J=8.5 Hz, 1 H), 13.07 (brs, 1 H).

### Example 618A 2-(3-chloro-4-{[1-(4-hydroxy-4-methylcyclohexyl)-2-oxopyrrolidin-3-yl]methyl}phenoxy)acetamide

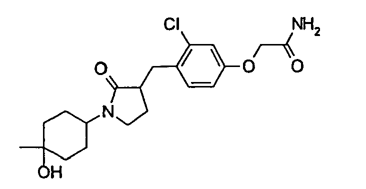

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and ammonium 1H-1,2,3-benzotriazol-1-ol.
1H NMR (300 MHz, CDCl₃) δ ppm 1.27 (s, 3 H), 1.41-1.82 (m, 10 H), 1.96-2.10 (m, 1 H), 2.69-2.84 (m, 2 H), 3.13-3.35 (m, 3 H), 3.90-4.05 (m, 1 H), 4.47 (s, 2 H), 5.71 (brs, 1 H), 6.49 (brs, 1 H), 6.78 (dd, J=8.5, 2.7 Hz, 1 H), 6.95 (d, J=2.7 Hz, 1 H), 7.23 (d, J=8.5 Hz, 1 H).

### Example 619A 3-(2-chloro-4-{[3-(4-fluorophenyl)-1,2,4-oxadiazol-5-yl]methoxy}benzyl)-1-(4-hydroxy-4-methylcyclohexyl)pyrrolidin-2-one

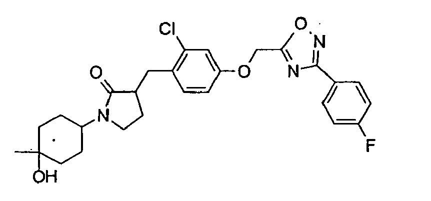

In the same manner as in Example 525A, the title compound was obtained from (3-chloro-4-{[1-(4-hydroxy-4-methylcyclohexyl)-2-oxopyrrolidin-3-yl]methyl}phenoxy)acetic acid obtained in Example 617A and 4-fluorobenzamidoxime.
1H NMR (300 MHz, CDCl₃) δ ppm 1.27 (s, 3 H), 1.41-1.82 (m, 10 H), 1.95-2.10 (m, 1 H), 2.69-2.85 (m, 2 H), 3.13-3.37 (m, 3 H), 3.91-4.04 (m, 1 H), 5.31 (s, 2 H), 6.87 (dd, J=8.5, 2.6 Hz, 1 H), 7.06 (d, J=2.6 Hz, 1 H), 7.14-7.25 (m, 3 H), 8.04-8.16 (m, 2 H).

### Example 620A 2-(3-chloro-4-{[1-(4-hydroxy-4-methylcyclohexyl)-2-oxopyrrolidin-3-yl]methyl}phenoxy)-N-cyclopropylacetamide

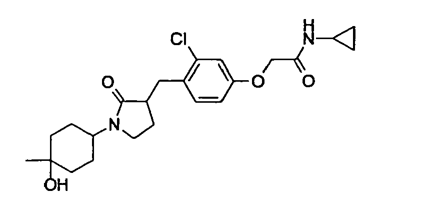

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and cyclopropylamine.
1H NMR (300 MHz, CDCl₃) δ ppm 0.53-0.63 (m, 2 H), 0.79-0.90 (m, 2 H), 1.27 (s, 3 H), 1.41 (s, 1 H), 1.45-1.83 (m, 9 H), 1.95-2.10 (m, 1 H), 2.68-2.84 (m, 3 H), 3.13-3.36 (m, 3 H), 3.90-4.05 (m, 1 H), 4.43 (s, 2 H), 6.57 (brs, 1 H), 6.74 (dd, J=8.5, 2.7 Hz, 1 H), 6.93 (d, J=2.7 Hz, 1 H), 7.22 (d, J=8.5 Hz, 1 H).

### Example 621A 2-(3-chloro-4-{[1-(5-hydroxy-2-adamantyl)-2-oxopyrrolidin-3-yl]methyl}phenoxy)-N-cyclopropylacetamide

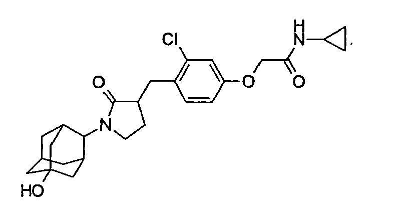

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and cyclopropylamine.
1H NMR (300 MHz, CDCl₃) δ ppm 0.53-0.62 (m, 2 H), 0.78-0.89 (m, 2 H), 1.45-1.58 (m, 2 H), 1.63-1.96 (m, 10 H), 1.98-2.12 (m, 1 H), 2.13-2.22 (m, 1 H), 2.38-2.51 (m, 2 H), 2.68-2.83 (m, 3 H), 3.21-3.36 (m, 1 H), 3.38-3.59 (m, 2 H), 3.88-3.94 (m, 1 H), 4.43 (s, 2 H), 6.58 (brs, 1 H), 6.74 (dd, J=8.5, 2.6 Hz, 1 H), 6.93 (d, J=2.6 Hz, 1H), 7.22 (d, J=8. 5 Hz, 1H).

### Example 622A 2-(3-chloro-4-{[1-(5-hydroxy-2-adamantyl)-2-oxopyrrolidin-3-yl]methyl}phenoxy)-N-[(pyridin-3-yl)methyl]acetamide

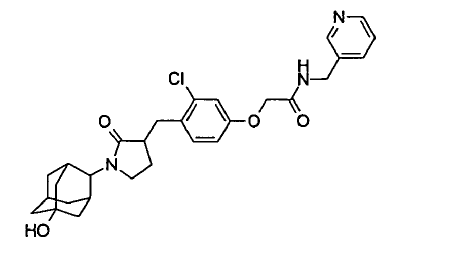

In the same manner as in Example 570A, the title compound was obtained from methyl (3-chloro-4-{[1-(5-hydroxy-2-adamantyl)-2-oxopyrrolidin-3-yl]methyl}phenoxy)acetate obtained in Example 575A and 1-(pyridin-3-yl)methanamine.
1H NMR (300 MHz, CDCl₃) δ ppm 1.35-2.22 (m, 14 H), 2.38-2.51 (m, 2 H), 2.67-2.82 (m, 2 H), 3.21-3.35 (m, 1 H), 3.38-3.59 (m, 2 H), 3.88-3.93 (m, 1 H), 4.52 (s, 2 H), 4.57 (d, J=6.2 Hz, 2 H), 6.75 (dd, J=8.6, 2.7 Hz, 1 H), 6.84-6.97 (m, 2 H), 7.18-7.33 (m, 3 H), 7.59-7.70 (m, 1 H), 8.49-8.62 (m, 2 H).

### Example 623A 2-(3-chloro-4-{[1-(4-hydroxy-4-methylcyclohexyl)-2-oxopyrrolidin-3-yl]methyl}phenoxy)-N-(2-furylmethyl)acetamide

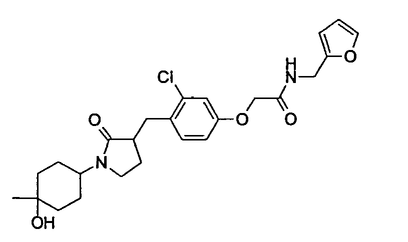

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and 1-(2-furyl)methanamine.
1H NMR (300 MHz, CDCl₃) δ ppm 1.27 (s, 3 H), 1.44-1.82 (m, 10 H), 1.95-2.09 (m, 1 H), 2.68-2.84 (m, 2 H), 3.12-3.35 (m, 3 H), 3.91-4.04 (m, 1 H), 4.48 (s, 2 H), 4.54 (d, J=5.7 Hz, 2 H), 6.23-6.29 (m, 1 H), 6.34 (dd, J=3.1, 1.8 Hz, 1 H), 6.76 (dd, J=8.5, 2.6 Hz, 1 H), 6.80-6.89 (m, 1 H), 6.94 (d, J=2.6 Hz, 1 H), 7.21 (d, J=8.5 Hz, 1 H), 7.37 (dd, J=1.8, 0.9 Hz, 1 H).

### Example 624A 2-(3-chloro-4-{[1-(5-hydroxy-2-adamantyl)-2-oxopyrrolidin-3-yl]methyl}phenoxy)-N-(2-furylmethyl)acetamide

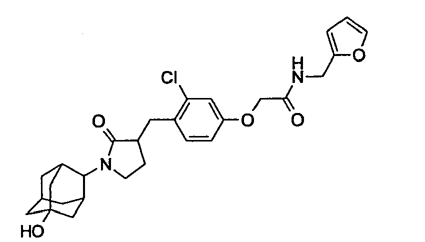

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and 1-(2-furyl)methanamine.
1H NMR (300 MHz, CDCl₃) δ ppm 1.45-1.57 (m, 2 H), 1. 62-1. 84 (m, 7 H), 1.85-2.11 (m, 4 H), 2.13-2.22 (m, 1 H), 2.37-2.51 (m, 2 H), 2.68-2.82 (m, 2 H), 3.21-3.35 (m, 1 H), 3.38-3.58 (m, 2 H), 3.88-3.93 (m, 1 H), 4.48 (s, 2 H), 4.54 (d, J=5.8 Hz, 2 H), 6.26 (dd, J=3.2, 0.8 Hz, 1H), 6.33 (dd, J=3.2, 1.9 Hz, 1H), 6.75 (dd, J=8.5, 2.7 Hz, 1H), 6.80-6.88 (m, 1H), 6.94 (d, J=2.7 Hz, 1 H), 7.21 (d, J=8.5 Hz, 1 H), 7.37 (dd, J=1.9, 0.8 Hz, 1 H).

### Example 625A 2-(3-chloro-4-{[1-(4-hydroxy-4-methylcyclohexyl)-2-oxopyrrolidin-3-yl]methyl}phenoxy)-N-[(pyridin-3-yl)methyl]acetamide

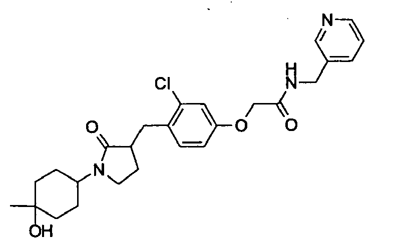

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and 1-(pyridin-3-yl)methanamine.
1H NMR (300 MHz, CDCl₃) δ ppm 1.27 (s, 3 H), 1.42-1.81 (m, 10 H), 1.95-2.10 (m, 1 H), 2.68-2.83 (m, 2 H), 3.13-3.35 (m, 3 H), 3.91-4.04 (m, 1 H), 4.52 (s, 2 H), 4.57 (d, J=6.0 Hz, 2 H), 6.75 (dd, J=8.5, 2.6 Hz, 1 H), 6.88-6.98 (m, 2 H), 7.21 (d, J=8.5 Hz, 1 H), 7.25-7.31 (m, 1 H), 7.60-7.68 (m, 1 H), 8.50-8.60 (m, 2 H).

### Example 626A 3-[2-chloro-4-(2-hydroxy-2-methylpropoxy)benzyl]-1-(5-hydroxy-2-adamantyl)pyrrolidin-2-one

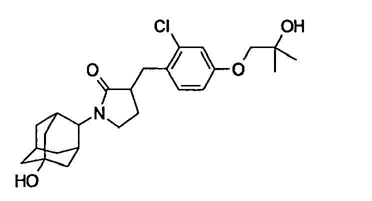

In the same manner as in Example 263A, the title compound was obtained from methyl (3-chloro-4-{[1-(5-hydroxy-2-adamantyl)-2-oxopyrrolidin-3-yl]methyl}phenoxy)acetate obtained in Example 575A.
1H NMR (300 MHz, CDCl₃) δ ppm 1.31-1.38 (m, 9 H), 1.39-2.22 (m, 12 H), 2.39-2.51 (m, 2 H), 2.67-2.82 (m, 2 H), 3.21-3.58 (m, 3 H), 3.76 (s, 2 H), 3.88-3.95 (m, 1 H), 6.77 (dd, J=8.5, 2.6 Hz, 1 H), 6.94 (d, J=2.6 Hz, 1 H), 7.18 (d, J=8.5 Hz, 1 H).

### Example 627A 2-(3-chloro-4-{[1-(4-hydroxy-4-methylcyclohexyl)-2-oxopyrrolidin-3-yl]methyl}phenoxy)-N-[(pyridin-2-yl)methyl]acetamide

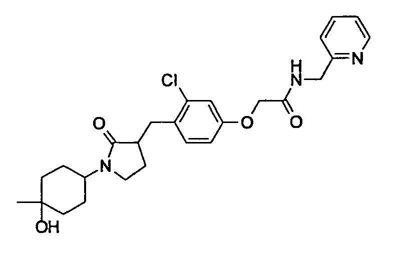

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and 1-(pyridin-2-yl)methanamine.
1H NMR (300 MHz, CDCl₃) δ ppm 1.27 (s, 3 H), 1.43-1.81 (m, 10 H), 1.94-2.10 (m, 1 H), 2.68-2.84 (m, 2 H), 3.12-3.36 (m, 3 H), 3.90-4.05 (m, 1 H), 4.53 (s, 2 H), 4.65 (d, J=5.3 Hz, 2 H), 6.81 (dd, J=8.6, 2.6 Hz, 1 H), 6.99 (d, J=2.6 Hz, 1 H), 7.17-7.32 (m, 3 H), 7.63-7.76 (m, 2 H), 8.53-8.60 (m, 1 H).

### Example 628A 2-(3-chloro-4-{[1-(5-hydroxy-2-adamantyl)-2-oxopyrrolidin-3-yl]methyl}phenoxy)-N-[(pyridin-2-yl)methyl]acetamide

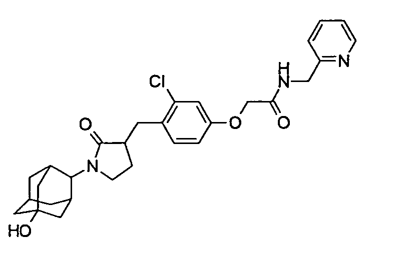

In the same manner as in Example 570A, the title compound was obtained from methyl (3-chloro-4-{[1-(5-hydroxy-2-adamantyl)-2-oxopyrrolidin-3-yl]methyl}phenoxy)acetate obtained in Example 575A and 1-(pyridin-2-yl)methanamine.
1H NMR (300 MHz, CDCl₃) δ ppm 1.45-1.57 (m, 3 H), 1.62-2.22 (m, 11 H), 2.39-2.50 (m, 2 H), 2.67-2.82 (m, 2 H), 3.22 -3.58 (m, 3 H), 3.88-3.94 (m, 1 H), 4.53 (s, 2 H), 4.65 (d, J=5.3 Hz, 2 H), 6.81 (dd, J=8.5, 2.6 Hz, 1 H), 7.00 (d, J=2.6 Hz, 1 H), 7.17-7.32 (m, 3 H), 7.62-7.75 (m, 2 H), 8.54-8.60 (m, 1 H).

### Example 629A 3-[2-chloro-4-(2-hydroxy-2-methylpropoxy)benzyl]-1-(4-hydroxy-4-methylcyclohexyl)pyrrolidin-2-one

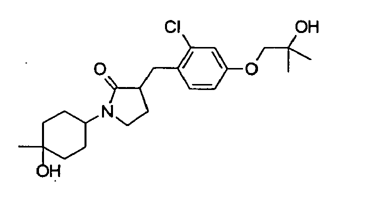

In the same manner as in Example 263A, the title compound was obtained from methyl (3-chloro-4-{[(1-(4-hydroxy-4-methylcyclohexyl)-2-oxopyrrolidin-3-yl]methyl}phenoxy)acetate obtained in Example 551A.
1H NMR (300 MHz, CDCl₃) δ ppm 1.27 (s, 3 H), 1.36 (s, 6 H), 1.41-1.81 (m, 10 H), 1.94-2.09 (m, 1 H), 2.20 (s, 1 H), 2.67-2.84 (m, 2 H), 3.13-3.36 (m, 3 H), 3.76 (s, 2 H), 3.92-4.05 (m, 1 H), 6.77 (dd, J=8.5, 2.6 Hz, 1 H), 6.94 (d, J=2.6 Hz, 1 H), 7.18 (d, J=8.5 Hz, 1 H).

### Example 630A 2-(3-chloro-4-{[1-(5-hydroxy-2-adamantyl)-2-oxopyrrolidin-3-yl]methyl}phenoxy)-N-methyl-N-[(pyridin-2-yl)methyl]acetamide

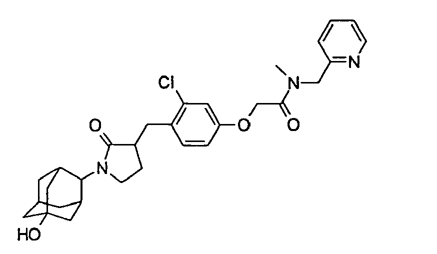

In the same manner as in Reference Example 1A, the title compound was obtained from 2-(3-chloro-4-{[1-(5-hydroxy-2-adamantyl)-2-oxopyrrolidin-3-yl]methyl}phenoxy)-N-[(pyridin-2-yl)methyl]acetamide obtained in Example 628A and methyl iodide.
1H NMR (300 MHz, CDCl₃) δ ppm 1.41 (s, 1 H), 1.45-2.10 (m, 13 H), 2.12-2.24 (m, 1 H), 2.37-2.53 (m, 2 H), 2.64-2.81 (m, 2 H), 3.00 (s, 3 H×0.5), 3.13 (s, 3 Hx0.5), 3.21-3.58 (m, 3 H), 3.85-3.96 (m, 1 H), 4.64 (s, 2 H×0.5), 4.74 (d, J=7.5 Hz, 2 H), 4.86 (s, 2 H×0.5), 6.72-6.88 (m, 1 H), 6.96 (dd, J=6.2, 2.6 Hz, 1 H), 7.09-7.31 (m, 3 H), 7.57-7.76 (m, 1 H), 8.48-8.70 (m, 1 H).

### Example 631A N-benzyl-2-(3-chloro-4-{[1-(5-hydroxy-2-adamantyl)-2-oxopyrrolidin-3-yl]methyl}phenoxy)-N-methylacetamide

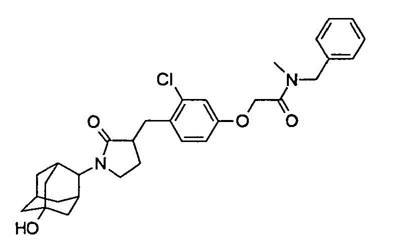

In the same manner as in Reference Example 1A, the title compound was obtained from N-benzyl-2-(3-chloro-4-{[1-(5-hydroxy-2-adamantyl)-2-oxopyrrolidin-3-yl]methyl}phenoxy)acetamide obtained in Example 614A and methyl iodide.
1H NMR (300 MHz, CDCl₃) δ ppm 1.34-2.22 (m, 14 H), 2.39-2.51 (m, 2 H), 2.65-2.80 (m, 2 H), 2.97 (s, 3 H), 3.21-3.57 (m, 3 H), 3.87-3.94 (m, 1 H), 4.61 (s, 2 H), 4.68-4.75 (m, 2 H), 6.67-7.01 (m, 2 H), 7.10-7.43 (m, 6 H).

### Example 632A N-(2-chlorobenzyl)-2-(3-chloro-4-{[1-(5-hydroxy-2-adamantyl)-2-oxopyrrolidin-3-yl]methyl}phenoxy)acetamide

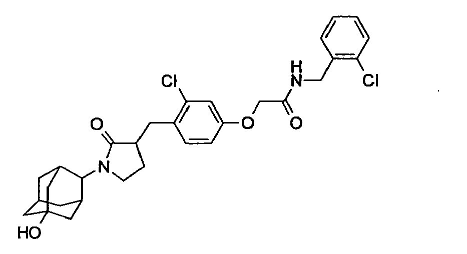

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and 1-(2-chlorophenyl)methanamine.
1H NMR (300 MHz, CDCl₃) δ ppm 1.44-1.58 (m, 3 H), 1.61-1.96 (m, 9 H), 1.97-2.10 (m, 1 H), 2.13-2.21 (m, 1 H), 2.38-2.51 (m, 2 H), 2.68-2.82 (m, 2 H), 3.22-3.34 (m, 1 H), 3.37-3.58 (m, 2 H), 3.87-3.94 (m, 1 H), 4.49 (s, 2 H), 4.63 (d, J=6.2 Hz, 2 H), 6.75 (dd, J=8 . 5, 2.6 Hz, 1 H), 6.94 (d, J=2.6 Hz, 1 H), 6.96-7.04 (m, 1 H), 7.17-7.29 (m, 3 H), 7.33-7.42 (m, 2 H).

### Example 633A N-(3-chlorobenzyl)-2-(3-chloro-4-{[1-(5-hydroxy-2-adamantyl)-2-oxopyrrolidin-3-yl]methyl}phenoxy)acetamide

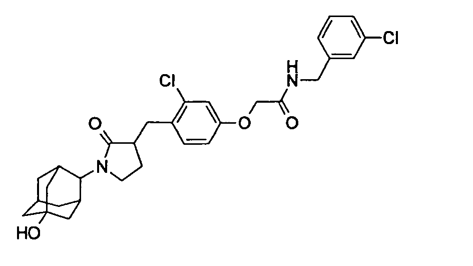

In the same manner as in Example 70A, the title compound was obtained from 3-chloro-4-{[1-(5-hydroxy-2-adamantyl)-2-oxopyrrolidin-3-yl]methyl}phenoxy)acetic acid obtained in Example 585A and 1-(3-chlorophenyl)methanamine.
1H NMR (300 MHz, CDCl₃) δ ppm 1.45-1.58 (m, 2 H), 1.62-1.85 (m, 8 H), 1.86-1.96 (m, 2 H), 1.97-2.10 (m, 1 H), 2.13-2.22 (m, 1 H), 2.38-2.51 (m, 2 H), 2.68-2.82 (m, 2 H), 3.21-3.35 (m, 1 H), 3.38-3.59 (m, 2 H), 3.87-3.95 (m, 1 H), 4.46-4.57 (m, 4 H), 6.76 (dd, J=8.5, 2.6 Hz, 1 H), 6.83-6.93 (m, 1 H), 6.95 (d, J=2.6 Hz, 1 H), 7.12-7.30 (m, 5 H).

### Example 634A N-(4-chlorobenzyl)-2-(3-chloro-4-{[1-(5-hydroxy-2-adamantyl)-2-oxopyrrolidin-3-yl]methyl}phenoxy)acetamide

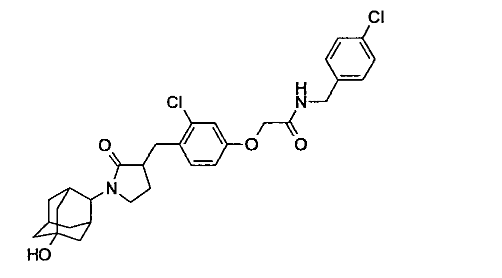

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and 1-(4-chlorophenyl)methanamine.
1H NMR (300 MHz, CDCl₃) δ ppm 1.42-1.58 (m, 3 H), 1.60-2.10 (m, 10 H), 2.13-2.21 (m, 1 H), 2.38-2.51 (m, 2 H), 2.67-2.81 (m, 2 H), 3.21-3.35 (m, 1 H), 3.37-3.58 (m, 2 H), 3.86-3.95 (m, 1 H), 4.46-4.55 (m, 4 H), 6.75 (dd, J=8.5, 2.6 Hz, 1 H), 6.80-6.88 (m, 1 H), 6. 94 (d, J=2.6 Hz, 1 H), 7.18-7.24 (m, 3 H), 7.28-7.33 (m, 2 H).

### Example 635A 2-(3-chloro-4-{[1-(5-hydroxy-2-adamantyl)-2-oxopyrrolidin-3-yl]methyl}phenoxy)-N-(2,4-dichlorobenzyl)acetamide

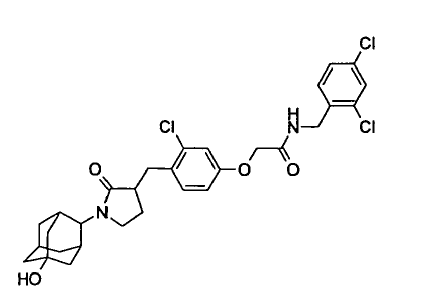

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and 1-(2,4-dichlorophenyl)methanamine.
1H NMR (300 MHz, CDCl₃) δ ppm 1.46-1.58 (m, 3 H), 1.62-2.11 (m, 10 H), 2.13-2.22 (m, 1 H), 2.39-2.51 (m, 2 H), 2.67-2.81 (m, 2 H), 3.20-3.35 (m, 1 H), 3.38-3.59 (m, 2 H), 3.87-3.94 (m, 1 H), 4.49 (s, 2 H), 4.58 (d, J=6.2 Hz, 2 H), 6.75 (dd, J=8.6, 2.6 Hz, 1 H), 6.93 (d, J=2.6 Hz, 1 H), 6.94-7.03 (m, 1 H), 7.17-7.35 (m, 3 H), 7.38 (d, J=2.1 Hz, 2 H).

### Example 636A 2-(3-chloro-4-{[1-(5-hydroxy-2-adamantyl)-2-oxopyrrolidin-3-yl]methyl}phenoxy)-N-[4-chloro-3-(trifluoromethyl)benzyl]acetamide

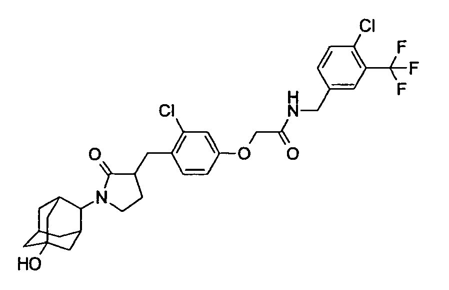

In the same manner as in Example 70A, the title compound was obtained from (3-chloro-4-{[1-(5-hydroxy-2-adamantyl)-2-oxopyrrolidin-3-yl]methyl}phenoxy)acetic acid obtained in Example 585A and 1-[4-chloro-3-(trifluoromethyl)phenyl]methanamine.
1H NMR (300 MHz, CDCl₃) δ ppm 1.44-1.58 (m, 2 H), 1.61-1.96 (m, 10 H), 1.97-2.11 (m, 1 H), 2.13-2.22 (m, 1 H), 2.38-2.51 (m, 2 H), 2.67-2.82 (m, 2 H), 3.21-3.33 (m, 1 H), 3.39-3.59 (m, 2 H), 3.87-3.93 (m, 1 H), 4.53 (s, 2 H), 4.56 (d, J=6.2 Hz, 2 H), 6.76 (dd, J=8.5, 2.6 Hz, 1 H), 6.90-7.00 (m, 2 H), 7.22 (d, J=8.5 Hz, 1 H), 7.37-7.51 (m, 2 H), 7.56-7.62 (m, 1 H).

### Example 637A methyl 2-[(3-chloro-4-{[1-(5-hydroxy-2-adamantyl)-2-oxopyrrolidin-3-yl]methyl}phenoxy)methyl]benzoate

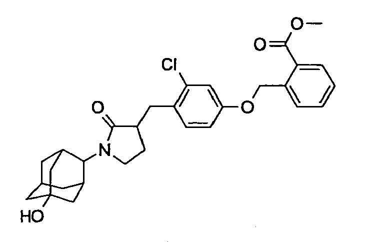

In the same manner as in Example 318A, the title compound was obtained from 3-(2-chloro-4-hydroxybenzyl)-1-(5-hydroxy-2-adamantyl)pyrrolidin-2-one obtained in Example 431A and methyl 2-(chloromethyl)benzoate.
1H NMR (300 MHz, CDCl₃) δ ppm 1.39 -2.10 (m, 13 H), 2.13-2.21 (m, 1 H), 2.39-2.51 (m, 1 H), 2.67-2.82 (m, 2 H), 3.24-3.56 (m, 3 H), 3.87-3.96 (m, 4 H), 5.47 (s, 2 H), 6.83 (dd, J=8.5, 2.6 Hz, 1 H), 7.02 (d, J=2.6 Hz, 1 H), 7.18 (d, J=8.5 Hz, 1 H), 7.35-7.43 (m, 1 H), 7.52-7.60 (m, 1 H), 7.68-7.75 (m, 1 H), 8.04 (dd, J=7.9, 1.2 Hz, 1 H).

### Example 638A methyl [3-chloro-4-({1-[3- (hydroxymethyl)-1-adamantyl]-2-oxopyrrolidin-3-yl}methyl)phenoxy]acetate

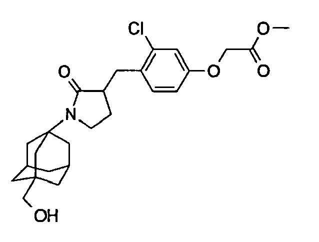

In the same manner as in Example 318A, the title compound was obtained from 3-(2-chloro-4-hydroxybenzyl)-1-[3-(hydroxymethyl)-1-adamantyl]pyrrolidin-2-one obtained in Example 486A and methyl bromoacetate.
1H NMR (300 MHz, CDCl₃) δ ppm 1.32 (t, J=6.1 Hz, 1 H), 1.40-1.75 (m, 6 H), 1.85-2.24 (m, 10 H), 2.59-2.75 (m, 2 H), 3.18-3.41 (m, 5 H), 3.81 (s, 3 H), 4.60 (s, 2 H), 6.75 (dd, J=8.6, 2.7 Hz, 1 H), 6.92 (d, J=2.7 Hz, 1 H), 7.18 (d, J=8.7 Hz, 1 H).

### Example 639A [3-chloro-4-({1-[3-(hydroxymethyl)-1-adamantyl]-2-oxopyrrolidin-3-yl}methyl)phenoxy]acetic acid

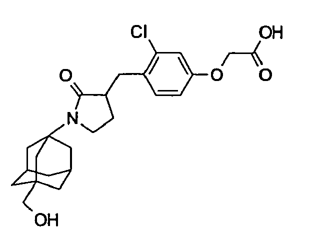

In the same manner as in Example 155A, the title compound was obtained from methyl [3-chloro-4-({1-[3-(hydroxymethyl)-1-adamantyl]-2-oxopyrrolidin-3-yl}methyl)phenoxy]acetate obtained in Example 638A.
1H NMR (300 MHz, DMSO-d₆) δ ppm 1.27-1.66 (m, 7 H), 1.70-1.88 (m, 3 H), 1.89-2.16 (m, 6 H), 2.41-2.61 (m, 1 H), 2.96-3.13 (m, 3 H), 3.17-3.43 (m, 3 H), 4.68 (s, 2 H), 6.84 (dd, J=8. 7, 2.6 Hz, 1 H), 6.98 (d, J=2.6 Hz, 1 H), 7.25 (d, J=8.7 Hz, 1 H).

### Example 640A 2-[3-chloro-4-({1-[3-(hydroxymethyl)-1-adamantyl]-2-oxopyrrolidin-3-yl}methyl)phenoxy]-N-[4-(trifluoromethyl)benzyl]acetamide

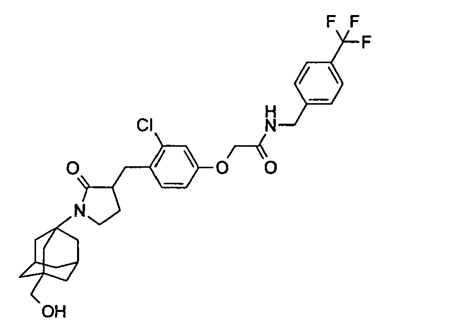

In the same manner as in Example 70A, the title compound was obtained from [3-chloro-4-({1-[3-(hydroxymethyl)-1-adamantyl]-2-oxopyrrolidin-3-yl}methyl)phenoxy]acetic acid obtained in Example 639A and 1-[4-(trifluoromethyl)phenyl]methanamine.
1H NMR (300 MHz, CDCl₃) δ ppm 1.40-1.74 (m, 8 H), 1.85-2.24 (m, 9 H), 2.61-2.75 (m, 2 H), 3.19-3.42 (m, 5 H), 4.53 (s, 2 H), 4.60 (d, J=6.2 Hz, 2 H), 6.76 (dd, J=8.6, 2.7 Hz, 1 H), 6.87-6.97 (m, 2 H), 7.21 (d, J=8.6 Hz, 1 H), 7.40 (d, J=8.1 Hz, 2 H), 7.60 (d, J=8.1 Hz, 2 H).

### Example 641A 3-[2-chloro-4-(2-hydroxyethoxy)benzyl]-1-[3-(hydroxymethyl)-1-adamantyl]pyrrolidin-2-one

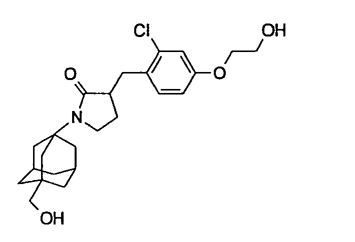

In the same manner as in Reference Example 19A, the title compound was obtained from methyl [3-chloro-4-({1-[3-(hydroxymethyl)-1-adamantyl]-2-oxopyrrolidin-3-yl}methyl)phenoxy]acetate obtained in Example 638A.
1H NMR (300 MHz, CDCl₃) δ ppm 1.31-1.75 (m, 8 H), 1.84-2.24 (m, 10 H), 2.61-2.75 (m, 2 H), 3.20-3.40 (m, 5 H), 3.91-3.99 (m, 2 H), 4.01-4.08 (m, 2 H), 6.77 (dd, J=8.5, 2.5 Hz, 1 H), 6.93 (d, J=2.5 Hz, 1 H), 7.17 (d, J=8.5 Hz, 1 H).

### Example 642A 3-(2,6-dichloro-4-methoxybenzyl)-1-(1,4-dioxaspiro[4.5]dec-8-yl)pyrrolidin-2-one and

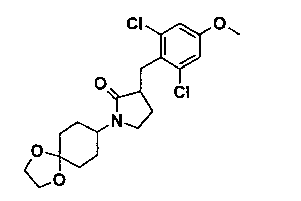

### Example 643A 3-(2,4-dichloro-6-methoxybenzyl)-1-(1,4-dioxaspiro[4.5]dec-8-yl)pyrrolidin-2-one

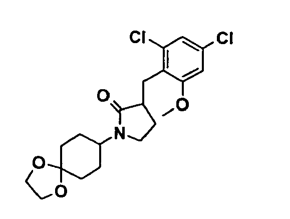

The reaction product which was obtained in the same manner as in Reference Example 1A from 1-(1,4-dioxaspiro[4.5]dec-8-yl)pyrrolidin-2-one and a mixture of 1,3-dichloro-2-(chloromethyl)-5-methoxybenzene obtained in Reference Example 41A and 1,5-dichloro-2-(chloromethyl)-3-methoxybenzene obtained in Reference Example 42A, was subjected to silica gel column chromatography (hexane-ethyl acetate 4:1-9:1) to give the title compound (Example 642A) eluted earlier, and then to give the title compound (Example 643A) eluted later.

### Example 642A

1H NMR (300 MHz, CDCl₃) δ ppm 1.64-2.04 (m, 10 H), 2.78-3.01 (m, 2 H), 3.10-3.25 (m, 1 H), 3.28-3.46 (m, 2 H), 3.77 (s, 3 H), 3.95 (s, 4 H), 4.06 (d, J=8.1 Hz, 1 H), 6.87 (s, 2 H).

### Example 643A

1H NMR (300 MHz, CDCl₃)δ ppm 1.65-1.98 (m, 10 H), 2.66-2.90 (m, 2 H), 3.05-3.45 (m, 3 H), 3.82 (s, 3 H), 3.95 (s, 4 H), 3.99-4.12 (m, 1H), 6.75 (d, J=2.0 Hz, 1H), 7.00 (d, J=2.0 Hz, 1H).

### Example 644A 3-(2,6-dichloro-4-methoxybenzyl)-1-(4-oxocyclohexyl)pyrrolidin-2-one

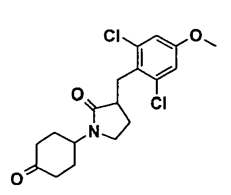

In the same manner as in Example 28A, the title compound was obtained from 3-(2,6-dichloro-4-methoxybenzyl)-1-(1,4-dioxaspiro[4.5]dec-8-yl)pyrrolidin-2-one obtained in Example 642A.
1H NMR (300 MHz, CDCl₃) δ ppm 1.74-2.19 (m, 6 H), 2.32-2.69 (m, 4 H), 2.82-3.05 (m, 2 H), 3.11-3.26 (m, 1 H), 3.28-3.47 (m, 2 H), 3.78 (s, 3 H), 4.37-4.58 (m, 1H), 6.88 (s, 2 H).

### Example 645A 3-(2,6-dichloro-4-methoxybenzyl)-1-(4-hydroxy-4-methylcyclohexyl)pyrrolidin-2-one

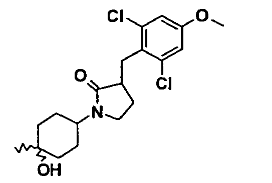

In the same manner as in Example 541A, the title compound was obtained from 3-(2,6-dichloro-4-methoxybenzyl)-1-(4-oxocyclohexyl)pyrrolidin-2-one obtained in Example 644A and methyllithium.
1H NMR (300 MHz, CDCl₃) δ ppm 1.28 (s, 3 H), 1.37 (s, 1 H), 1.46-2.05 (m, 10 H), 2.73-3.03 (m, 2 H), 3.10-3.26 (m, 1 H), 3.27-3.46 (m, 2 H), 3.78 (s, 3 H), 3.87-4.09 (m, 1 H), 6.87 (s, 2 H).

### Example 646A 3-chloro-4-{[1-(4-hydroxy-4-methylcyclohexyl)-2-oxopyrrolidin-3-yl]methyl}phenyl trifluoromethanesulfonate

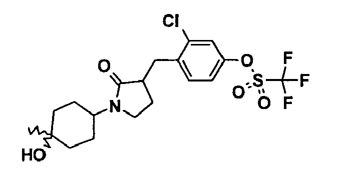

To a solution of 3-(2-chloro-4-hydroxybenzyl)-1-(4-hydroxy-4-methylcyclohexyl)pyrrolidin-2-one obtained in Example 542A (0.50 g) in pyridine (10 mL) was added anhydrous trifluoromethanesulfonic acid (0.63 g), and the mixture was stirred at 0°C for 30 min, and then at room temperature for 30 min. The reaction mixture was concentrated under reduced pressure, and the residue was partitioned between ethyl acetate and 2N hydrochloric acid. The ethyl acetate layer was washed successively with saturated sodium hydrogencarbonate solution and saturated brine, and dried over anhydrous magnesium sulfate. The solvent was evaporated under reduced pressure, and the residue was subjected to silica gel column chromatography (hexane-ethyl acetate 1:1-1:4, ethyl acetate-methanol 20:1) to give the title compound (0.60 g, 86%) as a pale-yellow solid.
1H NMR (300 MHz, CDCl₃) δ ppm 1.27 (s, 3 H), 1.40 (s, 1 H), 1.46-1.89 (m, 9 H), 1.95-2.16 (m, 1 H), 2.69-2.94 (m, 2 H), 3.14-3.28 (m, 2 H), 3.29-3.46 (m, 1 H), 3.84-4.07 (m, 1 H), 7.13 (dd, J=8.6, 2.6 Hz, 1 H), 7.32 (d, J=2.6 Hz, 1 H), 7.40 (d, J=8.6 Hz, 1 H).

### Example 647A 3-(4-bromo-2-chlorobenzyl)-1-(4-hydroxy-4-methylcyclohexyl)pyrrolidin-2-one (less polar product) and Example 648A 3-(4-bromo-2-chlorobenzyl)-1-(4-hydroxy-4-methylcyclohexyl)pyrrolidin-2-one (more polar product)

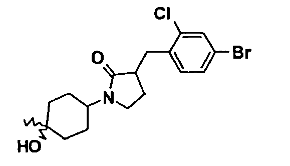

The reaction product (a mixture of four steric isomers) which was obtained in the same manner as in Example 30A from 3-(4-bromo-2-chlorobenzyl)-1-(4-oxocyclohexyl)pyrrolidin-2-one obtained in Example 254A and methylmagnesium bromide, was subjected to silica gel column chromatography (hexane-ethyl acetate 1:1, ethyl acetate, ethyl acetate-methanol 20:1) to give the title compound (Example 647A: less polar product) as a mixture of two steric isomers eluted earlier, and the to give the title compound (Example 648A: more polar product) as a mixture of two steric isomers eluted later.

### Example 647A (less polar product)

1H NMR (300 MHz, CDCl₃) δ ppm 1.00 (s, 1 H), 1.25 (s, 3 H), 1.37-1.92 (m, 10 H), 1.94-2.12 (m, 1 H), 2.65-2.86 (m, 2 H), 3.10-3.38 (m, 3 H), 3.81-4.02 (m, 1 H), 7.16 (d, J=8.3 Hz, 1 H), 7.31 (dd, J=8.3, 1.9 Hz, 1 H), 7.52 (d, J=1.9 Hz, 1 H).

### Example 648A (more polar product)

1H NMR (300 MHz, CDCl₃) δ ppm 1.27 (s, 3 H), 1.35 (s, 1 H), 1.43-1.86 (m, 9 H), 1.90-2.14 (m, 1 H), 2.65-2.86 (m, 2 H), 3.09-3.40 (m, 3 H), 3.83-4.06 (m, 1 H), 7.16 (d, J=8.3 Hz, 1 H), 7.32 (dd, J=8. 1, 2.1 Hz, 1 H), 7.52 (d, J=2.3 Hz, 1 H).

### Example 649A N-(3'-chloro-4'-{[1-(4-hydroxy-4-methylcyclohexyl)-2-oxopyrrolidin-3-yl]methyl}biphenyl-2-yl)acetamide

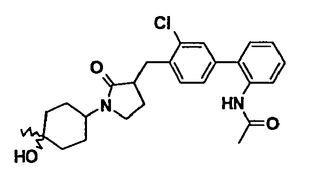

In the same manner as in Example 218A, the title compound was obtained from 3-(4-bromo-2-chlorobenzyl)-1-(4-hydroxy-4-methylcyclohexyl)pyrrolidin-2-one (more polar product) obtained in Example 648A and o-acetamidophenylboronic acid.
1H NMR (300 MHz, CDCl₃) δ ppm 1.28 (s, 3 H), 1.39 (s, 1 H), 1.52-1.87 (m, 9 H), 2.06 (s, 3 H), 2.05-2.20 (m, 1 H), 2.77-2.96 (m, 2 H), 3.16-3.34 (m, 2 H), 3.40 (t, J=8.9 Hz, 1 H), 3.91-4.11 (m, 1 H), 7.06 (s, 1 H), 7.13-7.25 (m, 3 H), 7.34-7.42 (m, 3 H), 8.20 (d, J=8.1 Hz, 1 H).

### Example 650A N-(3'-chloro-4'-{[1-(4-hydroxy-4-methylcyclohexyl)-2-oxopyrrolidin-3-yl]methyl}biphenyl-3-yl)acetamide

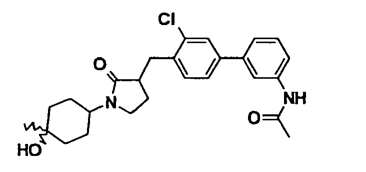

In the same manner as in Example 218A, the title compound was obtained from 3-(4-bromo-2-chlorobenzyl)-1-(4-hydroxy-4-methylcyclohexyl)pyrrolidin-2-one (more polar product) obtained in Example 648A and m-acetamidophenylboronic acid.
1H NMR (300 MHz, CDCl₃) δ ppm 1.28 (s, 3 H), 1.35 (s, 1 H), 1.57-1.85 (m, 9 H), 1.97-2.15 (m, 1 H), 2.21 (s, 3 H), 2.75-2.91 (m, 2 H), 3.15-3.28 (m, 2 H), 3.31-3.48 (m, 2 H), 3.99 (dd, J=10.9, 4.5 Hz, 1 H), 7.27-7.50 (m, 5 H), 7.58 (d, J=1.9 Hz, 1 H), 7.76 (s, 1H) .

### Example 651A N-(3'-chloro-4'-{[1-(4-hydroxy-4-methylcyclohexyl)-2-oxopyrrolidin-3-yl]methyl}biphenyl-4-yl)acetamide

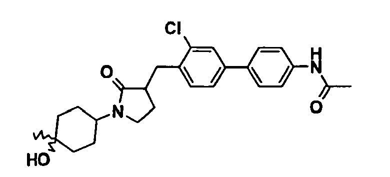

In the same manner as in Example 218A, the title compound was obtained from 3-(4-bromo-2-chlorobenzyl)-1-(4-hydroxy-4-methylcyclohexyl)pyrrolidin-2-one (more polar product) obtained in Example 648A and p-acetamidophenylboronic acid.
1H NMR (300 MHz, CDCl₃) δ ppm 1.28 (s, 3 H), 1.36 (s, 1 H), 1.47-1.85 (m, 9 H), 1.95-2.13 (m, 1 H), 2.21 (s, 3 H), 2.73-2.91 (m, 2 H), 3.14-3.30 (m, 2 H), 3.31-3.46 (m, 1 H), 3.89-4.07 (m, 1 H), 7.20-7.26 (m, 1 H), 7.28-7.43 (m, 2 H), 7.45-7.64 (m, 5 H).

### Example 652A 3'-chloro-4'-{[1-(4-hydroxy-4-methylcyclohexyl)-2-oxopyrrolidin-3-yl]methyl}biphenyl-3-carbaldehyde

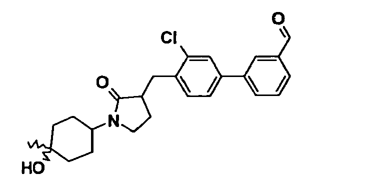

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 3-formylbenzeneboronic acid.
1H NMR (300 MHz, CDCl₃) δ ppm 1.28 (s, 3 H), 1.36 (s, 1 H), 1.43-1.87 (m, 9 H), 1.99-2.18 (m, 1 H), 2.77-2.94 (m, 2 H), 3.15-3.31 (m, 2 H), 3.34-3.51 (m, 1 H), 3.89-4.09 (m, 1 H), 7.34-7.42 (m, 1 H), 7.42-7.49 (m, 1 H), 7.57-7.68 (m, 2 H), 7.85 (dd, J=14.2, 7.8 Hz, 2 H), 8.07 (s, 1H), 10.09 (s, 1H).

### Example 653A 3'-chloro-4'-{[1-(4-hydroxy-4-methylcyclohexyl)-2-oxopyrrolidin-3-yl]methyl}biphenyl-4-carbaldehyde

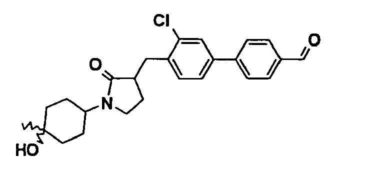

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 4-formylbenzeneboronic acid.
1H NMR (300 MHz, CDCl₃) δ ppm 1.28 (s, 3 H), 1.35 (s, 1 H), 1.43-1.84 (m, 9 H), 1.95-2.17 (m, 1 H), 2.72-2.96 (m, 2 H), 3.11-3.31 (m, 2 H), 3.34-3.48 (m, 1 H), 3.83-4.11 (m, 1 H), 7.32-7.43 (m, 1 H), 7.44-7.50 (m, 1 H), 7.64 (d, J=1.9 Hz, 1 H), 7.72 (d, J=8.2 Hz, 2 H), 7.96 (d, J=8.2 Hz, 2 H), 10.06 (s, 1 H).

### Example 654A 3-{[3-chloro-3'-(hydroxymethyl)biphenyl-4-yl]methyl}-1-(4-hydroxy-4-methylcyclohexyl)pyrrolidin-2-one

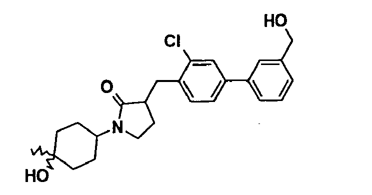

In the same manner as in Example 29A, the title compound was obtained from 3'-chloro-4'-{[1-(4-hydroxy-4-methylcyclohexyl)-2-oxopyrrolidin-3-yl]methyl}biphenyl-3-carbaldehyde obtained in Example 652A.
1H NMR (300 MHz, CDCl₃) δ ppm 1.28 (s, 3 H), 1.35 (s, 1 H), 1.50-1.86 (m, 9 H), 1.99-2.14 (m, 1 H), 2.75-2.92 (m, 2 H), 3.17-3.30 (m, 2 H), 3.39 (t, J=9.0 Hz, 1 H), 3.90-4.11 (m, 1 H), 4.77 (d, J=5.8 Hz, 2 H), 7.30-7.54 (m, 5 H), 7.54-7.63 (m, 2 H).

### Example 655A 3-{[3-chloro-4'-(hydroxymethyl)biphenyl-4-yl]methyl}-1-(4-hydroxy-4-methylcyclohexyl)pyrrolidin-2-one

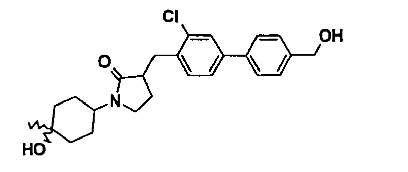

In the same manner as in Example 29A, the title compound was obtained from 3'-chloro-4'-{[1-(4-hydroxy-4-methylcyclohexyl)-2-oxopyrrolidin-3-yl]methyl}biphenyl-4-carbaldehyde obtained in Example 653A.
1H NMR (300 MHz, CDCl₃) δ ppm 1.28 (s, 3 H), 1.38 (s, 1 H), 1.46-1.86 (m, 9 H), 1.96-2.14 (m, 1 H), 2.75-2.92 (m, 2 H), 3.16-3.30 (m, 2 H), 3.34-3.47 (m, 1 H), 3.90-4.08 (m, 1 H), 4.75 (d, J=5.7 Hz, 2 H), 7.30-7.37 (m, 1 H), 7.37-7.47 (m, 3 H), 7.51-7.62 (m, 3 H).

### Example 656A 3-[(3'-acetyl-3-chlorobiphenyl-4-yl)methyl]-1-(4-hydroxy-4-methylcyclohexyl)pyrrolidin-2-one

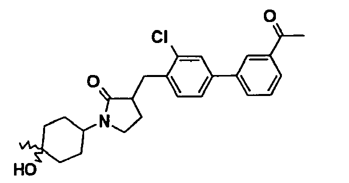

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 3-acetylphenylboronic acid.
1H NMR (300 MHz, CDCl₃) δ ppm 1.28 (s, 3 H), 1.37 (s, 1 H), 1.46-1.87 (m, 9 H), 1.95-2.18 (m, 1 H), 2.66 (s, 3 H), 2.78-2.95 (m, 2 H), 3.15-3.32 (m, 2 H), 3.32-3.53 (m, 1 H), 3.88-4.10 (m, 1 H), 7.33-7.40 (m, 1 H), 7.41-7.48 (m, 1 H), 7.54 (t, J=7.8 Hz, 1 H), 7.62 (d, J=1.9 Hz, 1 H), 7.75 (d, J=8.3 Hz, 1 H), 7.94 (d,

J=8.0 Hz, 1H), 8.14 (s, 1H).

### Example 657A 3-[(4'-acetyl-3-chlorobiphenyl-4-yl)methyl]-1-(4-hydroxy-4-methylcyclohexyl)pyrrolidin-2-one

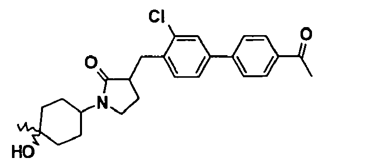

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 4-acetylphenylboronic acid.
1H NMR (300 MHz, CDCl₃) δ ppm 1.28 (s, 3 H), 1.37 (s, 1 H), 1.47-1.88 (m, 9 H), 1.98-2.16 (m, 1 H), 2.64 (s, 3 H), 2.76-2.94 (m, 2 H), 3.15-3.32 (m, 2 H), 3.33-3.49 (m, 1 H), 3.90-4.11 (m, 1 H), 7.34-7.41 (m, 1 H), 7.41-7.51 (m, 1 H), 7.63 (d, J=3.0 Hz, 2 H), 7.67 (s, 1 H), 8.03 (d, J=8.7 Hz, 2 H).

### Example 658A 3'-chloro-4'-{[1-(4-hydroxy-4-methylcyclohexyl)-2-oxopyrrolidin-3-yl]methyl}biphenyl-3-carboxamide

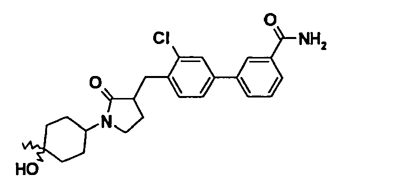

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and (3-aminocarbonylphenyl)boronic acid.
1H NMR (300 MHz, DMSO-d₆) δ ppm 1.13 (s, 3 H), 1.38-1.75 (m, 8 H), 1.85-2.02 (m, 1 H), 2.58-2.79 (m, 2 H), 3.12-3.35 (m, 4 H), 3.65-3.82 (m, 1 H), 4.36 (s, 1 H), 7.38-7.73 (m, 4 H), 7.80-7.91 (m, 3 H), 8.07-8.21 (m, 2 H).

### Example 659A 3'-chloro-4'-{[1-(4-hydroxy-4-methylcyclohexyl)-2-oxopyrrolidin-3-yl]methyl}biphenyl-4-carboxamide

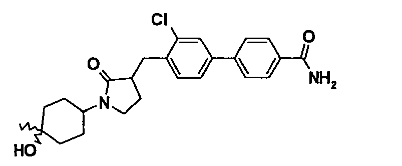

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and (4-aminocarbonylphenyl)boronic acid.
1H NMR (300 MHz, CDCl₃) δ ppm 1.28 (s, 3 H), 1.46-1.88 (m, 12 H), 2.05 (s, 1 H), 2.86 (d, J=3.0 Hz, 2 H), 3.24 (d, J=8.0 Hz, 2 H), 3.32-3.52 (m, 1 H), 3.84-4.19 (m, 1 H), 7.33-7.53 (m, 2 H), 7.55-7.74 (m, 3 H), 7. 89 (d, J=8. 3 Hz, 2 H).

### Example 660A 3-[(3,3'-dichlorobiphenyl-4-yl)methyl]-1-(4-hydroxy-4-methylcyclohexyl)pyrrolidin-2-one

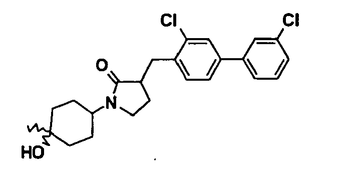

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 3-chlorophenylboronic acid.
1H NMR (300 MHz, CDCl₃) δ ppm 1.28 (s, 3 H), 1.36 (s, 1 H), 1.46-1.85 (m, 9 H), 1.95-2.17 (m, 1 H), 2.76-2.94 (m, 2 H), 3.14-3.29 (m, 2 H), 3.34-3.48 (m, 1 H), 3.89-4.08 (m, 1 H), 7.28-7.48 (m, 5 H), 7.50-7.60 (m, 2 H).

### Example 661A 3-[(3,4'-dichlorobiphenyl-4-yl)methyl]-1-(4-hydroxy-4-methylcyclohexyl)pyrrolidin-2-one

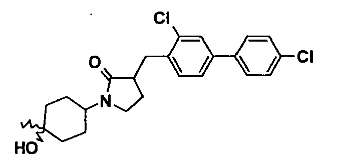

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 4-chlorophenylboronic acid.
1H NMR (300 MHz, CDCl₃) δ ppm 1.28 (s, 3 H), 1.36 (s, 1 H), 1.46-1.84 (m, 9 H), 1.96-2.16 (m, 1 H), 2.71-2.91 (m, 2 H), 3.11-3.30 (m, 2 H), 3.40 (q, J=9.2 Hz, 1 H), 3.88-4.11 (m, 1 H), 7.30-7.60 (m, 7 H).

### Example 662A 3'-chloro-4'-{[1-(4-hydroxy-4-methylcyclohexyl)-2-oxopyrrolidin-3-yl]methyl}biphenyl-3-carbonitrile

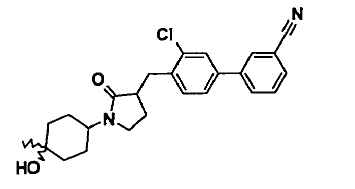

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 3-cyanophenylboronic acid.
1H NMR (300 MHz, CDCl₃) δ ppm 1.28 (s, 3 H), 1.36 (s, 1 H), 1.48-1.85 (m, 9 H), 2.00-2.16 (m, 1 H), 2.76-2.95 (m, 2 H), 3.14-3.33 (m, 2 H), 3.34-3.50 (m, 1 H), 3.88-4.08 (m, 1 H), 7.39 (s, 2 H), 7.49-7.59 (m, 2 H), 7.61-7.69 (m, 1 H), 7.78 (d, J=8.0 Hz, 1 H), 7.83 (s, 1 H).

### Example 663A 5-(3-chloro-4-{[1-(4-hydroxy-4-methylcyclohexyl)-2-oxopyrrolidin-3-yl]methyl}phenyl)-2-furaldehyde

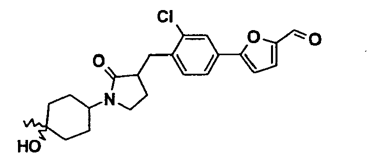

In the same manner as in Example 218A, the title compound was obtained from 3-chloro-4-{[1-(4-hydroxy-4-methylcyclohexyl)-2-oxopyrrolidin-3-yl]methyl}phenyl trifluoromethanesulfonate obtained in Example 646A and 5-formyl-2-furanboronic acid.
1H NMR (300 MHz, CDCl₃) δ ppm 1.28 (s, 3 H), 1.33 (s, 1H), 1.56-1.84 (m, 9 H), 2.00-2.13 (m, 1 H), 2.75-2.92 (m, 2 H), 3.22 (t, J=7.3 Hz, 2 H), 3.40 (d, J=8.9 Hz, 1 H), 3.86-4.11 (m, 1 H), 6.83 (d, J=3.8 Hz, 1 H), 7.32 (d, J=3.8 Hz, 1 H), 7.37 (d, J=8.1 Hz, 1 H), 7.63 (dd, J=8.0, 1.6 Hz, 1 H), 7.83 (d, J=1.9 Hz, 1 H), 9.66 (s, 1 H).

Example 664A 3-{2-chloro-4-[5-(hydroxymethyl)-2-furyl]benzyl}-1-(4-hydroxy-4-methylcyclohexyl)pyrrolidin-2-one

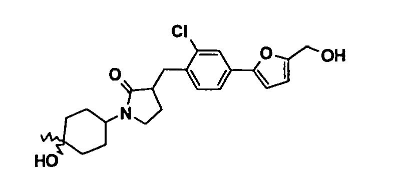

In the same manner as in Example 29A, the title compound was obtained from 5-(3-chloro-4-{[1-(4-hydroxy-4-methylcyclohexyl)-2-oxopyrrolidin-3-yl]methyl}phenyl)-2-furaldehyde obtained in Example 663A.
1H NMR (300 MHz, CDCl₃) δ ppm 1.27 (s, 3 H), 1.34 (s, 1 H), 1.47-1.83 (m, 12 H), 2.74-2.88 (m, 2 H), 3.12-3.28 (m, 2 H), 3.22-3. 30 (m, 1 H), 4.67 (d, J=6. 1 Hz, 2 H), 6.38 (d, J=3. 4 Hz, 1 H), 6.59 (d, J=3.4 Hz, 1 H), 7.29 (s, 1 H), 7.47 (dd, J=8.1, 1.8 Hz, 1 H), 7.67 (d, J=1.8 Hz, 1 H).

### Example 665A N-(3-chloro-4-{[1-(4-hydroxy-4-methylcyclohexyl)-2-oxopyrrolidin-3-yl]methyl}phenyl)-2,2,2-trifluoroacetamide

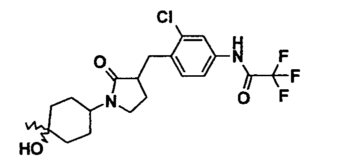

In the same manner as in Example 541A, the title compound was obtained from N-(3-chloro-4-{[2-oxo-1-(4-oxocyclohexyl)pyrrolidin-3-yl]methyl}phenyl)-2,2,2-trifluoroacetamide obtained in Example 476A and methyllithium. 1H NMR (300 MHz, CDCl₃) δ ppm 1.27 (s, 3 H), 1.40 (s, 1 H), 1.46-1.86 (m, 9 H), 1.96-2.17 (m, 1 H), 2.69-2.93 (m, 2 H), 3.13-3.41 (m, 3 H), 3.87-4.09 (m, 1 H), 7.27-7.33 (m, 1 H), 7.33-7.42 (m, 1H), 7.71 (d, J=2.1 Hz, 1H), 8.16 (s, 1H).

### Example 666A 3-(4-amino-2-chlorobenzyl)-1-(4-hydroxy-4-methylcyclohexyl)pyrrolidin-2-one

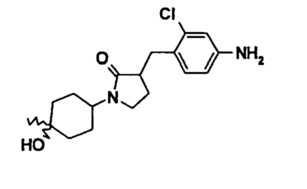

In the same manner as in Example 155A, the title compound was obtained from N-(3-chloro-4-{[1-(4-hydroxy-4-methylcyclohexyl)-2-oxopyrrolidin-3-yl]methyl}phenyl)-2,2,2-trifluoroacetamide obtained in Example 665A.
1H NMR (300 MHz, CDCl₃) δ ppm 1.27 (s, 3 H), 1.35 (s, 1 H), 1.44-1.84 (m, 9 H), 1.89-2.06 (m, 1 H), 2.61-2.82 (m, 2 H), 3.09-3.31 (m, 3 H), 3.64 (s, 2 H), 3.89-4.06 (m, 1 H), 6.51 (dd, J=8.1, 2.5 Hz, 1 H), 6.70 (d, J=2.3 Hz, 1 H), 7.03 (d, J=8.0 Hz, 1 H).

### Example 667A N-(3-chloro-4-{[1-(4-hydroxy-4-methylcyclohexyl)-2-oxopyrrolidin-3-yl]methyl}phenyl)acetamide

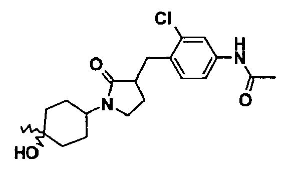

In the same manner as in Example 22A, the title compound was obtained from 3-(4-amino-2-chlorobenzyl)-1-(4-hydroxy-4-methylcyclohexyl)pyrrolidin-2-one obtained in Example 666A and acetyl chloride.
1H NMR (300 MHz, CDCl₃) δ ppm 1.27 (s, 3 H), 1.37 (s, 1 H), 1.45-1.85 (m, 9 H), 1.91-2.11 (m, 1 H), 2.17 (s, 3 H), 2.65-2.85 (m, 2 H), 3.12-3.26 (m, 2 H), 3.32 (d, J=9.1 Hz, 1 H), 3.86-4.04 (m, 1 H), 7.14-7.26 (m, 3 H), 7.65 (d, J=1.5 Hz, 1 H).

### Example 668A N-(3-chloro-4-{[1-(4-hydroxy-4-methylcyclohexyl)-2-oxopyrrolidin-3-yl]methyl}phenyl)cyclopropanecarboxamide

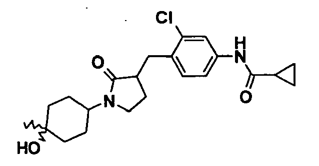

In the same manner as in Example 22A, the title compound was obtained from 3-(4-amino-2-chlorobenzyl)-1-(4-hydroxy-4-methylcyclohexyl)pyrrolidin-2-one obtained in Example 666A and cyclopropanecarbonyl chloride.
1H NMR (300 MHz, CDCl₃) δ ppm 0.81-0.92 (m, 2 H), 1.04-1.13 (m, 2 H), 1.27 (s, 3 H), 1.36 (s, 1 H), 1.42-1.83 (m, 10 H), 1.92-2.12 (m, 1 H), 2.70-2.85 (m, 2 H), 3.14-3.25 (m, 2 H), 3.30 (t, J=9.1 Hz, 1 H), 3.89-4.05 (m, 1 H), 7.15-7.26 (m, 2 H), 7.37 (s, 1 H), 7.68 (s, 1 H).

### Example 669A N-(3-chloro-4-{[1-(4-hydroxy-4-methylcyclohexyl)-2-oxopyrrolidin-3-yl]methyl}phenyl)benzamide

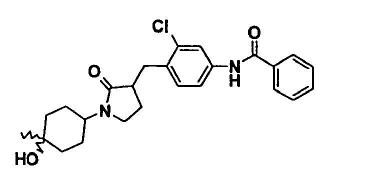

In the same manner as in Example 22A, the title compound was obtained from 3-(4-amino-2-chlorobenzyl)-1-(4-hydroxy-4-methylcyclohexyl)pyrrolidin-2-one obtained in Example 666A and benzoyl chloride.
1H NMR (300 MHz, CDCl₃) δ ppm 1.27 (s, 3 H), 1.39 (s, 1 H), 1.47-1.82 (m, 9 H), 1.95-2.13 (m, 1 H), 2.73-2.88 (m, 2 H), 3.15-3.25 (m, 2 H), 3.27-3.41 (m, 1 H), 3.85-4.06 (m, 1 H), 7.22-7.31 (m, 1 H), 7.41 (dd, J=8.3, 2.3 Hz, 1 H), 7.45-7.68 (m, 3 H), 7.82 (d, J=2.3 Hz, 1 H), 7.83-7.91 (m, 3 H).

### Example 670A N-(3-chloro-4-{[1-(4-hydroxy-4-methylcyclohexyl)-2-oxopyrrolidin-3-yl]methyl}phenyl)-2-phenylacetamide

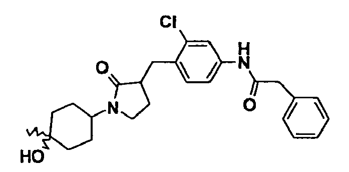

In the same manner as in Example 22A, the title compound was obtained from 3-(4-amino-2-chlorobenzyl)-1-(4-hydroxy-4-methylcyclohexyl)pyrrolidin-2-one obtained in Example 666A and phenylacetyl chloride.
1H NMR (300 MHz, CDCl₃) δ ppm 1.27 (s, 3 H), 1.34 (s, 1 H), 1.46-1.80 (m, 9 H), 1.89-2.06 (m, 1 H), 2.66-2.85 (m, 2 H), 3.11-3.25 (m, 2 H), 3.29 (d, J=9.1 Hz, 1 H), 3.73 (s, 2 H), 3.88-4.03 (m, 1 H), 7.02 (s, 1 H), 7.17 (s, 2 H), 7.29-7.47 (m, 5 H), 7.54 (s, 1 H).

### Example 671A N-(3-chloro-4-{[1-(4-hydroxy-4-methylcyclohexyl)-2-oxopyrrolidin-3-yl]methyl}phenyl)-3-phenylpropanamide

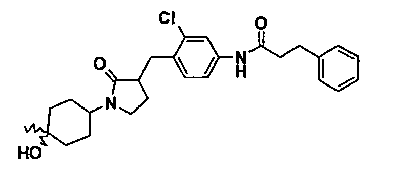

In the same manner as in Example 22A, the title compound was obtained from 3-(4-amino-2-chlorobenzyl)-1-(4-hydroxy-4-methylcyclohexyl)pyrrolidin-2-one obtained in Example 666A and 3-phenylpropionyl chloride.
1H NMR (300 MHz, CDCl₃) δ ppm 1.27 (s, 3 H), 1.39 (s, 1 H), 1.46-1.84 (m, 9 H), 1.90-2.09 (m, 1 H), 2.59-2.71 (m, 2 H), 2.71-2.83 (m, 2 H), 3.05 (t, J=7.6 Hz, 2 H), 3.13-3.24 (m, 2 H), 3.24-3.38 (m, 1H), 3.87-4.05 (m, 1H), 7.09 (s, 1H), 7.14-7.38 (m, 7 H), 7.60 (s, 1 H).

### Example 672A N-(3-chloro-4-{[1-(4-hydroxy-4-methylcyclohexyl)-2-oxopyrrolidin-3-yl]methyl}phenyl)-N-methylbenzamide

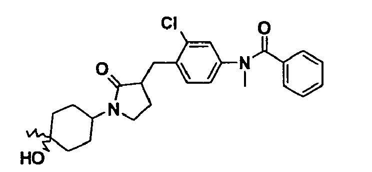

In the same manner as in Reference Example 1A, the title compound was obtained from N-(3-chloro-4-{[1-(4-hydroxy-4-methylcyclohexyl)-2-oxopyrrolidin-3-yl]methyl}phenyl)benzamide obtained in Example 669A and methyl iodide.
1H NMR (300 MHz, CDCl₃) δ ppm 1.27 (s, 3 H), 1.43-1.80 (m, 9 H), 1.88-2.02 (m, 1 H), 2.64-2.82 (m, 2 H), 3.11-3.22 (m, 2 H), 3.25-3.32 (m, 1 H), 3.46 (s, 3 H), 3.86-4.04 (m, 1 H), 6.82 (dd, J=8.3, 2.3 Hz, 1 H), 7.07-7.14 (m, 2 H), 7.15-7.24 (m, 2 H), 7.24-7.36 (m, 3 H).

### Example 673A N-(3-chloro-4-{[1-(4-hydroxy-4-methylcyclohexyl)-2-oxopyrrolidin-3-yl]methyl}phenyl)-N-methyl-3-phenylpropanamide

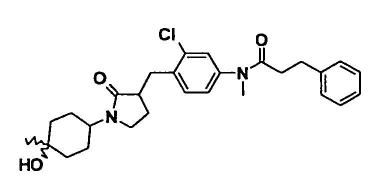

In the same manner as in Reference Example 1A, the title compound was obtained from N-(3-chloro-4-{[1-(4-hydroxy-4-methylcyclohexyl)-2-oxopyrrolidin-3-yl]methyl}phenyl)-3-phenylpropanamide obtained in Example 671A and methyl iodide.
1H NMR (300 MHz, CDCl₃) δ ppm 1.27 (s, 3 H), 1.42-1.85 (m, 9 H), 1.98-2.17 (m, 1 H), 2.38 (d, J=6.8 Hz, 2 H), 2.70-2.85 (m, 2 H), 2.87-3.00 (m, 2 H), 3.14-3.29 (m, 5 H), 3.35 (q, J=9.2 Hz, 1 H), 3.89-4.07 (m, 1 H), 6.84 (d, J=7.2 Hz, 1 H), 6.98 (s, 1 H), 7.08 (d, J=6.8 Hz, 2 H), 7.13-7.34 (m, 4 H).

### Example 674A 3-[2-chloro-4-(4-methylpiperazin-1-yl)benzyl]-1-(4-oxocyclohexyl)pyrrolidin-2-one

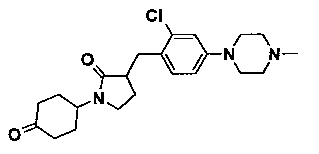

In the same manner as in Example 221A, 3-[2-chloro-4-(4-methylpiperazin-1-yl)benzyl]-1-(1,4-dioxaspiro[4.5]dec-8-yl)pyrrolidin-2-one was synthesized from 3-(4-bromo-2-chlorobenzyl)-1-(1,4-dioxaspiro[4.5]dec-8-yl)pyrrolidin-2-one obtained in Example 203A and N-methylpiperazine. Then, the compound was treated in the same manner as in Example 28A, the title compound was obtained.
1H NMR (300 MHz, CDCl₃) δ ppm 1.63-2.16 (m, 6 H), 2.35 (s, 3 H), 2.38-2.62 (m, 8 H), 2.64-2.89 (m, 2 H), 3.09-3.23 (m, 6 H), 3.29 (dd, J=13.3, 3.8 Hz, 1 H), 4.41-4.58 (m, 1 H), 6.75 (dd, J=8.5, 2.7 Hz, 1H), 6.89 (d, J=2.7 Hz, 1H), 7.13 (d, J=8.5 Hz, 1H).

### Example 675A 3-[2-chloro-4-(4-methylpiperazin-1-yl)benzyl]-1-(4-hydroxy-4-methylcyclohexyl)pyrrolidin-2-one

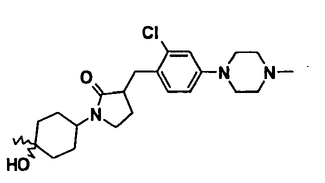

In the same manner as in Example 541A, the title compound was obtained from 3-[2-chloro-4-(4-methylpiperazin-1-yl)benzyl]-1-(4-oxocyclohexyl)pyrrolidin-2-one obtained in Example 674A and methyllithium.
1H NMR (300 MHz, CDCl₃) δ ppm 1.27 (s, 3 H), 1.36 (s, 1 H), 1.45-1.83 (m, 9 H), 1.90-2.10 (m, 1 H), 2.35 (s, 3 H), 2.51-2.60 (m, 4 H), 2.65-2.85 (m, 2 H), 3.10-3.23 (m, 6 H), 3.22-3.31 (m, 1 H), 3.84-4.08 (m, 1 H), 6.75 (dd, J=8.7, 2.7 Hz, 1 H), 6.89 (d, J=2.7 Hz, 1 H), 7.13 (d, J=8.7 Hz, 1 H).

### Example 676A N-benzyl-3-chloro-4-{[2-oxo-1-(4-oxocyclohexyl)pyrrolidin-3-yl]methyl}benzamide

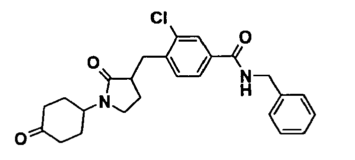

To a solution of 3-(4-bromo-2-chlorobenzyl)-1-(1,4-dioxaspiro[4.5]dec-8-yl)pyrrolidin-2-one obtained in Example 203A (3.10 g) in tetrahydrofuran (50 mL) was added dropwise n-butyllithium (1.6 M hexane solution) at -78°C. The solution was stirred at -78°C for 30 min, an excess amount of dry ice was added, and the mixture was stirred at -78°C for 1 hr. The reaction solution was allowed to warm to room temperature, 2N hydrochloric acid (5 mL) was added, and the mixture was partitioned between ethyl acetate and water. The ethyl acetate layer was washed with saturated brine, and dried over anhydrous magnesium sulfate. The solvent was evaporated under reduced pressure, and the obtained colorless amorphous was treated in the same manner as in Example 28A to give a pale-brown amorphous (1.41 g). In the same manner as in Example 70A, the title compound (0.13 g, 12%) was obtained from the obtained amorphous (0.92 g) and benzylamine.
1H NMR (300 MHz, CDCl₃) δ ppm 1.62-2.18 (m, 6 H), 2.33-2.62 (m, 4 H), 2.75-2.95 (m, 2 H), 3.13-3.28 (m, 2 H), 3.33-3.51 (m, 1 H), 4.34-4.54 (m, 1 H), 4.63 (d, J=5.5 Hz, 2 H), 6.47 (t, J=5.5 Hz, 1 H), 7.30-7.43 (m, 6 H), 7.60 (dd, J=8.0, 1.7 Hz, 1 H), 7.81 (d, J=1.7 Hz, 1 H).

### Example 677A 3-chloro-4-{[2-oxo-1-(4-oxocyclohexyl)pyrrolidin-3-yl]methyl}-N-(2-phenylethyl)benzamide

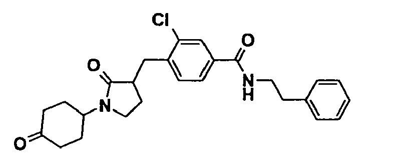

In the same manner as in Example 676A, the title compound was obtained from 3-(4-bromo-2-chlorobenzyl)-1-(1,4-dioxaspiro[4.5]dec-8-yl)pyrrolidin-2-one obtained in Example 203A and phenethylamine.
1H NMR (300 MHz, CDCl₃) δ ppm 1.65-2.14 (m, 6 H), 2.35-2.64 (m, 4 H), 2.75-2.88 (m, 2 H), 2.89-2.98 (m, 2 H), 3.15-3.28 (m, 2 H), 3.33-3.48 (m, 1 H), 3.71 (q like, 2 H), 4.37-4.57 (m, 1 H), 6.07 (t, J=5.7 Hz, 1 H), 7.16-7.30 (m, 3 H), 7.34 (t like, 3 H), 7.47 (dd, J=8.0, 1.7 Hz, 1 H), 7.71 (d, J=1.7 Hz, 1 H).

### Example 678A N-benzyl-3-chloro-4-{[1-(4-hydroxy-4-methylcyclohexyl)-2-oxopyrrolidin-3-yl]methyl}benzamide

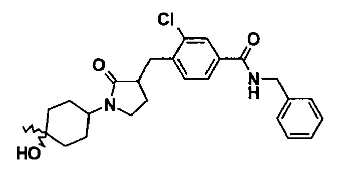

In the same manner as in Example 541A, the title compound was obtained from N-benzyl-3-chloro-4-{[2-oxo-1-(4-oxocyclohexyl)pyrrolidin-3-yl]methyl}benzamide obtained in Example 676A and methyllithium.
1H NMR (300 MHz, CDCl₃) δ ppm 1.27 (s, 3 H), 1.38-1.84 (m, 9 H), 1.90-2.12 (m, 2 H), 2.75-2.90 (m, 2 H), 3.16-3.26 (m, 2 H), 3.31-3.47 (m, 1 H), 3.86-4.05 (m, 1 H), 4.63 (d, J=5.7 Hz, 2 H), 6.44 (t, J=5.5 Hz, 1 H), 7.29-7.42 (m, 6 H), 7.59 (dd, J=8.0, 1.5 Hz, 1 H), 7.81 (d, J=1.5 Hz, 1 H).

### Example 679A 3-chloro-4-{[1-(4-hydroxy-4-methylcyclohexyl)-2-oxopyrrolidin-3-yl]methyl}-N-(2-phenylethyl)benzamide

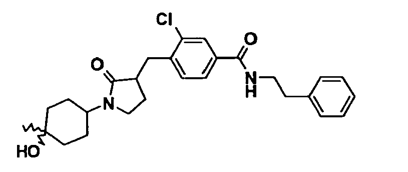

In the same manner as in Example 541A, the title compound was obtained from 3-chloro-4-{[2-oxo-1-(4-oxocyclohexyl)pyrrolidin-3-yl]methyl}-N-(2-phenylethyl)benzamide obtained in Example 677A and methyllithium.
1H NMR (300 MHz, CDCl₃) δ ppm 1.27 (s, 3 H), 1.39-1.84 (m, 9 H), 1.90-2.13 (m, 2 H), 2.72-2.86 (m, 2 H), 2.93 (t, J=7.0 Hz, 2 H), 3.14-3.26 (m, 2 H), 3.29-3.43 (m, 1 H), 3.71 (q like, 2 H), 3.86-4.04 (m, 1 H), 6.12 (t, J=5.7 Hz, 1 H), 7.17-7.29 (m, 3 H), 7.29-7.40 (m, 3 H), 7.46 (dd, J=8.0, 1.9 Hz, 1 H), 7.71 (d, J=1.9 Hz, 1 H).

### Example 680A tert-butyl [2-(3-chloro-4-{[1-(4-hydroxy-4-methylcyclohexyl)-2-oxopyrrolidin-3-yl]methyl}phenoxy)ethyl]carbamate

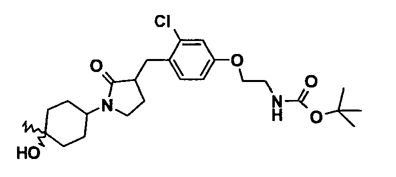

In the same manner as in Example 318A, the title compound was obtained from 3-(2-chloro-4-hydroxybenzyl)-1-(4-hydroxy-4-methylcyclohexyl)pyrrolidin-2-one obtained in Example 542A and N-Boc-2-bromoethylamine.
1H NMR (300 MHz, CDCl₃) δ ppm 1.27 (s, 3 H), 1.37 (s, 1 H), 1.45 (s, 9 H), 1.47-1.83 (m, 8 H), 1.89-2.09 (m, 2 H), 2.63-2.85 (m, 2 H), 3.14-3.24 (m, 2 H), 3.23-3.35 (m, 1 H), 3.51 (q, J=5.2 Hz, 2 H), 3.98 (t, J=5.2 Hz, 3 H), 4.86-5.03 (m, 1 H), 6.73 (dd, J=8.5, 2.7 Hz, 1 H), 6.90 (d, J=2.7 Hz, 1 H), 7.17 (d, J=8.5 Hz, 1 H).

### Example 681A 3-[4-(2-aminoethoxy)-2-chlorobenzyl]-1-(4-hydroxy-4-methylcyclohexyl)pyrrolidin-2-one hydrochloride

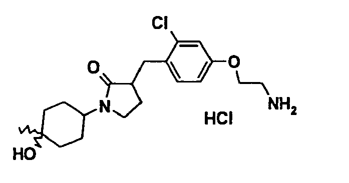

In the same manner as in Example 20A, the title compound was obtained from tert-butyl [2-(3-chloro-4-{[1-(4-hydroxy-4-methylcyclohexyl)-2-oxopyrrolidin-3-yl]methyl}phenoxy)ethyl]carbamate obtained in Example 680A.
1H NMR (300 MHz, DMSO-d₆) δ ppm 1.13 (s, 3 H), 1.38-1.68 (m, 9 H), 1.76-1.99 (m, 1 H), 2.54-2.69 (m, 2 H), 3.05-3.29 (m, 5 H), 3.62-3.79 (m, 1 H), 4.19 (t, J=5.1 Hz, 2 H), 6.92 (dd, J=8.5, 2.6 Hz, 1 H), 7.06 (d, J=2.6 Hz, 1 H), 7.30 (d, J=8.5 Hz, 1 H), 8.23 (s, 2 H).

### Example 682A N-[2-(3-chloro-4-{[1-(4-hydroxy-4-methylcyclohexyl)-2-oxopyrrolidin-3-yl]methyl}phenoxy)ethyl]benzamide

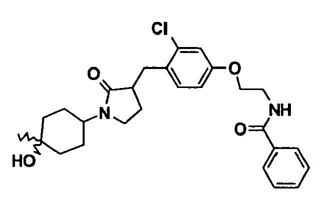

In the same manner as in Example 22A, the title compound was obtained from 3-[4-(2-aminoethoxy)-2-chlorobenzyl]-1-(4-hydroxy-4-methylcyclohexyl)pyrrolidin-2-one hydrochloride obtained in Example 681A and benzoyl chloride.
1H NMR (300 MHz, CDCl₃) δ ppm 1.27 (s, 3 H), 1.34 (s, 1 H), 1.48-1.84 (m, 9 H), 2.00 (m, 1 H), 2.67-2.83 (m, 2 H), 3.11-3.24 (m, 2 H), 3.30 (t like, 1 H), 3.87 (q, J=5.7 Hz, 2 H), 3.93-4.05 (m, 1 H), 4.08-4.18 (m, 2 H), 6.50-6.60 (m, 1 H), 6.76 (dd, J=8.5, 2.5 Hz, 1H), 6.94 (d, J=2.5 Hz, 1H), 7.18 (d, J=8.5 Hz, 1H), 7.39-7.54 (m, 3 H), 7.74-7.81 (m, 2 H).

### Example 683A N-[2-(3-chloro-4-{[1-(4-hydroxy-4-methylcyclohexyl)-2-oxopyrrolidin-3-yl]methyl}phenoxy)ethyl]-N-methylbenzamide

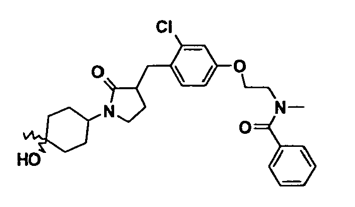

In the same manner as in Reference Example 1A, the title compound was obtained from N-[2-(3-chloro-4-{[1-(4-hydroxy-4-methylcyclohexyl)-2-oxopyrrolidin-3-yl]methyl}phenoxy)ethyl]benzamide obtained in Example 682A and methyl iodide.
1H NMR (300 MHz, CDCl₃) δ ppm 1.27 (s, 3 H), 1.41-1.90 (m, 9 H), 1.90-2.16 (m, 2 H), 2.60-2.89 (m, 2 H), 3.04-3.39 (m, 6 H), 3.68 (s, 1 H), 3.82-4.06 (m, 3 H), 4.26 (s, 1 H), 6.57-7.06 (m, 2 H), 7.18 (d, J=8.7 Hz, 1 H), 7.40 (s, 5 H).

### Example 684A N-[2-(3-chloro-4-{[1-(4-hydroxy-4-methylcyclohexyl)-2-oxopyrrolidin-3-yl]methyl}phenoxy)ethyl]-4-(trifluoromethyl)benzamide

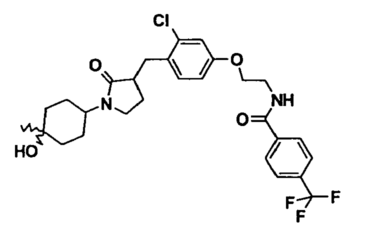

In the same manner as in Example 22A, the title compound was obtained from 3-[4-(2-aminoethoxy)-2-chlorobenzyl]-1-(4-hydroxy-4-methylcyclohexyl)pyrrolidin-2-one hydrochloride obtained in Example 681A and p-trifluoromethylbenzoyl chloride. 1H NMR (300 MHz, CDCl₃) δ ppm 1.27 (s, 3 H), 1.43 (s, 1 H), 1.45-1.81 (m, 9 H), 1.92-2.04 (m, 1 H), 2.66-2.82 (m, 2 H), 3.12-3.37 (m, 3 H), 3.80-4.03 (m, 3 H), 4.06-4.18 (m, 2 H), 6.66 (t, J=5.1 Hz, 1 H), 6.76 (dd, J=8.5, 2.5 Hz, 1 H), 6.93 (d, J=2.5 Hz, 1 H), 7.18 (d, J=8.5 Hz, 1 H), 7.71 (d, J=8.3 Hz, 2 H), 7.90 (d, J=8.3 Hz, 2 H).

### Example 685A N-[2-(3-chloro-4-{[1-(4-hydroxy-4-methylcyclohexyl)-2-oxopyrrolidin-3-yl]methyl}phenoxy)ethyl]benzenesulfonamide

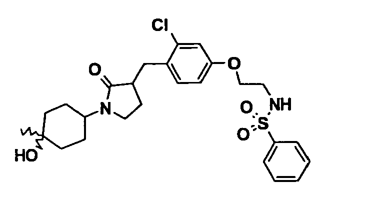

In the same manner as in Example 22A, the title compound was obtained from 3-[4-(2-aminoethoxy)-2-chlorobenzyl)-1-(4-hydroxy-4-methylcyclohexyl)pyrrolidin-2-one hydrochloride obtained in Example 681A and benzenesulfonyl chloride.
1H NMR (300 MHz, CDCl₃) δ ppm 1.27 (s, 3 H), 1.34 (s, 1 H), 1.43-1.85 (m, 9 H), 1.92-2.13 (m, 1 H), 2.68-2.84 (m, 2 H), 3.12-3.31 (m, 3 H), 3.33-3.43 (m, 2 H), 3.90-4.03 (m, 3 H), 4.80-4.98 (m, 1H), 6.65 (dd, J=8.5, 2.7 Hz, 1H), 6.80 (d, J=2.7 Hz, 1H), 7.16 (d, J=8.5 Hz, 1 H), 7.47-7.65 (m, 3 H), 7.89 (d, J=6.8 Hz, 2 H).

### Example 686A 3-(4-bromo-2-chlorobenzyl)-1-(4-hydroxy-4-methylcyclohexyl)pyrrolidin-2-one (an optically active form, retention time 9 min) and Example 687A 3-(4-bromo-2-chlorobenzyl)-1-(4-hydroxy-4-methylcyclohexyl)pyrrolidin-2-one (an optically active form, retention time 14 min)

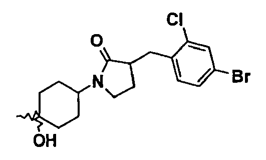

3-(4-Bromo-2-chlorobenzyl)-1-(4-hydroxy-4-methylcyclohexyl)pyrrolidin-2-one (a mixture of two steric isomers) obtained in Example 647A was subjected to optical resolution with normal phase chiral high performance liquid chromatography (manufactured by Daicel Chemical Industries, Ltd., Chiralcel OJ (trade name), 50 mmID X 500 mmL, hexane-ethanol 75:25) to give the title compound (Example 686A) from a fraction with a retention time of 9 min and the title compound (Example 687A) f from a fraction with a retention time of 14 min.

### Example 686A (retention time 9 min)

1H NMR (300 MHz, CDCl₃) δ ppm 1.00 (s, 1 H), 1.25 (s, 3 H), 1.37-1.92 (m, 10 H), 1.94-2.12 (m, 1 H), 2.65-2.86 (m, 2 H), 3.10-3.38 (m, 3 H), 3.81-4.02 (m, 1 H), 7.16 (d, J=8.3 Hz, 1 H), 7.31 (dd, J=8.3, 1.9 Hz, 1 H), 7.52 (d, J=1.9 Hz, 1 H).

### Example 687A (retention time 14 min)

1H NMR (300 MHz, CDCl₃) δ ppm 1.00 (s, 1 H), 1.25 (s, 3 H), 1.37-1.92 (m, 10 H), 1.94-2.12 (m, 1 H), 2.65-2.86 (m, 2 H), 3.10-3.38 (m, 3 H), 3.81-4.02 (m, 1 H), 7.16 (d, J=8.3 Hz, 1 H), 7.31 (dd, J=8.3, 1.9 Hz, 1 H), 7.52 (d, J=1.9 Hz, 1 H).

### Example 688A 3-(4-bromo-2-chlorobenzyl)-1-(4-hydroxy-4-methylcyclohexyl)pyrrolidin-2-one (an optically active form, retention time 10 min) and Example 689A 3-(4-bromo-2-chlorobenzyl)-1-(4-hydroxy-4-methylcyclohexyl)pyrrolidin-2-one (an optically active form, retention time 15 min)

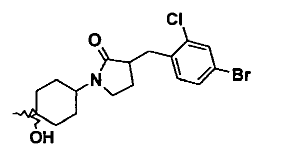

3-(4-Bromo-2-chlorobenzyl)-1-(4-hydroxy-4-methylcyclohexyl)pyrrolidin-2-one (a mixture of two steric isomers) obtained in Example 648A was subjected to optical resolution with normal phase chiral high performance liquid chromatography (manufactured by Daicel Chemical Industries, Ltd., Chiralcel OJ (trade name), 50 mmID X 500 mmL, hexane-ethanol 75:25) to give the title compound (Example 688A) from a fraction with a retention time of 10 min and the title compound (Example 689A) from a fraction with a retention time of 15 min. Example 688A (retention time 10 min)
1H NMR (300 MHz, CDCl₃)δ ppm 1.27 (s, 3 H), 1.35 (s, 1 H), 1.43-1.86 (m, 9 H), 1.90-2.14 (m, 1 H), 2.65-2.86 (m, 2 H), 3.09-3.40 (m, 3 H), 3.83-4.06 (m, 1 H), 7.16 (d, J=8.1 Hz, 1 H), 7.32 (dd, J=8.1, 2.1 Hz, 1 H), 7.52 (d, J=2.1 Hz, 1 H).

### Example 689A (retention time 15 min)

1H NMR (300 MHz, CDCl₃) δ ppm 1.27 (s, 3 H), 1.35 (s, 1 H), 1.43-1.86 (m, 9 H), 1.90-2.14 (m, 1 H), 2.65-2.86 (m, 2 H), 3.09-3.40 (m, 3 H), 3.83-4.06 (m, 1 H), 7.16 (d, J=8.1 Hz, 1 H), 7.32 (dd, J=8.1, 2.1 Hz, 1 H), 7.52 (d, J=2.1 Hz, 1 H).

### Example 690A 3-{3-chloro-4-[(1-cyclohexyl-2-oxopyrrolidin-3-yl)methyl]phenyl}-N-phenylpropanamide

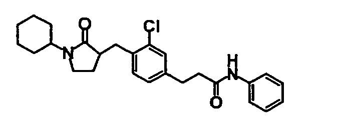

In the same manner as in Example 70A, the title compound was obtained from 3-{3-chloro-4-[(1-cyclohexyl-2-oxopyrrolidin-3-yl)methyl]phehyl}propanoic acid obtained in Example 528A, aniline and 4-(N, N-dimethylamino)pyridine.
1H NMR (300 MHz, DMSO-d₆) δ ppm 0.94-1.17 (m, 1 H) 1.18-1.46 (m, 4 H) 1.46-1.66 (m, 4 H) 1.66-1.79 (m, 2 H) 1.80-1.97 (m, 1 H) 2.55-2.74 (m, 4 H) 2.88 (t, J=7.5 Hz, 2 H) 3.04-3.25 (m, 3 H) 3.62-3.82 (m, 1 H) 7.02 (t, J=7.4 Hz, 1 H) 7.15 (dd, J=7.8, 1.6 Hz, 1 H) 7.19-7.39 (m, 4 H) 7.47-7.65 (m, 2 H) 9.89 (s, 1 H).

### Example 691A tert-butyl 4-(3-{3-chloro-4-[(1-cyclohexyl-2-oxopyrrolidin-3-yl)methyl]phenyl}propanoyl)piperazine-1-carboxylate

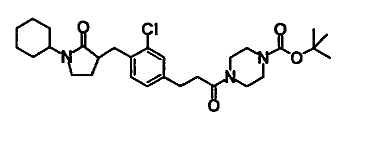

In the same manner as in Example 70A, the title compound was obtained from 3-{3-chloro-4-[(1-cyclohexyl-2-oxopyrrolidin-3-yl)methyl]phenyl}propanoic acid obtained in Example 528A and tert-butyl piperazine-1-carboxylate.
1H NMR (300 MHz, CDCl₃) δ ppm 0.93-1.18 (m, 1 H), 1.18-1.43 (m, 4 H), 1.46 (s, 9 H), 1.60-1.87 (m, 6 H), 1.89-2.09 (m, 1 H), 2.60 (t, J=7.8 Hz, 2 H), 2.67-2.85 (m, 2 H), 2.93 (t, J=7.8 Hz, 2 H), 3.07-3.46 (m, 9 H), 3.52-3.68 (m, 2 H), 3.84-4.07 (m, 1 H), 7.03 (dd, J=8.0, 1.7 Hz, 1 H), 7.14-7.23 (m, 2 H).

### Example 692A 3-[2-chloro-4-(3-oxo-3-(piperazin-1-yl)propyl)benzyl]-1-cyclohexylpyrrolidin-2-one hydrochloride

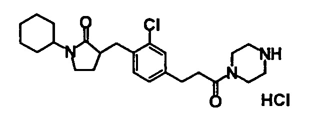

In the same manner as in Example 20A, the title compound was obtained from tert-butyl 4-(3-{3-chloro-4-[(1-cyclohexyl-2-oxopyrrolidin-3-yl)methyl]phenyl}propanoyl)piperazine-1-carboxylate obtained in Example 691A.
1H NMR (300 MHz, DMSO-d₆) δ ppm 0.96-1.17 (m, 1 H), 1.18-1.66 (m, 8 H), 1.74 (d, J=10.6 Hz, 2 H), 1.81-1.96 (m, 1 H), 2.55-2.86 (m, 5 H), 2.94-3.28 (m, 7 H), 3.56-3.82 (m, 5 H), 7.14 (d, J=8.0 Hz, 2 H), 7.23-7.29 (m, 1H), 7.32 (s, 1H), 9.02 (br s, 2 H). Example 693A ethyl4-({3-chloro-4-[(1-cyclohexyl-2-oxopyrrolidin-3-yl)methyl]phenyl}amino)-4-oxobutanoate

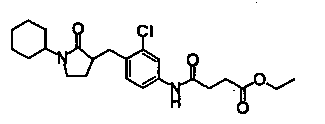

In the same manner as in Example 70A, the title compound was obtained from 3-(4-amino-2-chlorobenzyl)-1-cyclohexylpyrrolidin-2-one obtained in Example 215A and monoethyl succinate.
1H NMR (300 MHz, CDCl₃) δ ppm 0.94-1.18 (m, 1 H), 1.19-1.48 (m, 7 H), 1.59-1.87 (m, 6 H), 1.89-2.07 (m, 1 H), 2.58-2.69 (m, 2 H), 2.69-2.84 (m, 4 H), 3.09-3.40 (m, 3 H), 3.83-4.04 (m, 1 H), 4.17 (q, J=7.2 Hz, 2 H), 7.16-7.25 (m, 2 H), 7.55-7.73 (m, 2 H).

### Example 694A 4-(13-chloro-4-[(l-cyclohexyl-2-oxopyrrolidin-3-yl)methyl]phenyl}amino)-4-oxobutanoic acid

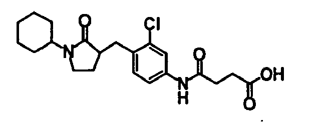

In the same manner as in Example 155A, the title compound was obtained from ethyl 4-({3-chloro-4-[(1-cyclohexyl-2-oxopyrrolidin-3-yl)methyl]phenyl}amino)-4-oxobutanoate obtained in Example 693A and aqueous lithium hydroxide solution.
1H NMR (300 MHz, DMSO-d₆) δ ppm 0.93-1.18 (m, 1 H), 1.18-1.66 (m, 8 H), 1.66-1.80 (m, 2 H), 1.81-1.97 (m, 1 H), 2.36-2.78 (m, 6 H), 3.06-3.26 (m, 3 H), 3.61-3.86 (m, 1 H), 7.26 (d, J=8.4 Hz, 1 H), 7.34 (m, 1 H), 7.80 (d, J=2.1 Hz, 1 H), 10.14-10.37 (m, 1 H), 12.16 (br s, 1 H).

### Example 695A tert-butyl [4-({3-chloro-4-[(1-cyclohexyl-2-oxopyrrolidin-3-yl)methyl]phenyl}amino)-4-oxobutyl]carbamate

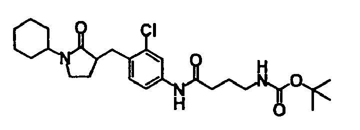

In the same manner as in Example 70A, the title compound was obtained from 3-(4-amino-2-chlorobenzyl)-1-cyclohexylpyrrolidin-2-one obtained in Example 215A and 4-(tert-butoxycarbonylamino)butanoic acid.
1H NMR (300 MHz, CDCl₃) δ ppm 0.91-1.19 (m, 1 H), 1.19-1.52 (m, 12 H), 1.59-2.10 (m, 9 H), 2.27-2.45 (m, 2 H), 2.64-2.88 (m, 2 H), 3.08-3.41 (m, 5 H), 3.81-4.07 (m, 1 H), 4.66-4.88 (m, 1 H), 7.14-7.32 (m, 2 H), 7.32-7.47 (m, 1 H), 7.78 (s, 1 H), 9.00 (s, 1 H).

### Example 696A 4-amino-N-{3-chloro-4-[(1-cyclohexyl-2-oxopyrrolidin-3-yl)methyl]phenyl}butanamide hydrochloride

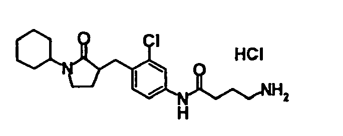

In the same manner as in Example 20A, the title compound was obtained from tert-butyl [4-({3-chloro-4-[(1-cyclohexyl-2-oxopyrrolidin-3-yl)methyl]phenyl}amino)-4-oxobutyl]carbamate obtained in Example 695A.
1H NMR (300 MHz, DMSO-d₆) δ ppm 0.92-1.67 (m, 9 H), 1.66-1.97 (m, 5 H), 2.23-2.70 (m, 3 H), 2.82 (s, 2 H), 3.04-3.28 (m, 3 H), 3.35-3.64 (m, 2 H), 3.62-3.82 (m, 1 H), 7.20-7.32 (m, 1 H), 7.32-7.43 (m, 1 H), 7.58-7.98 (m, 3 H), 10.05-10.25 (m, 1 H).

### Example 697A 3-{2-chloro-4-[(2-hydroxyethyl)amino]benzyl}-1-cyclohexylpyrrolidin-2-one hydrochloride

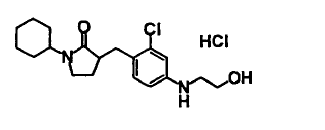

To 3-(4-amino-2-chlorobenzyl)-1-cyclohexylpyrrolidin-2-one obtained in Example 215A (0.50 g) was added 2-bromoethyl acetate (0.21 g), and the mixture was stirred at 120°C for 30 min. The reaction mixture was allowed to cool to room temperature, saturated aqueous sodium hydrogencarbonate was added, and the mixture was extracted with ethyl acetate. The extract was dried over anhydrous magnesium sulfate, and the solvent was evaporated under reduced pressure. The obtained residue was subjected to silica gel column chromatography (hexane-ethyl acetate 1:1-ethyl acetate) to give a mixture containing 2-({3-chloro-4-[(1-cyclohexyl-2-oxopyrrolidin-3-yl)methyl]phenyl}amino)ethyl acetate as a main component. To a solution of this mixture in tetrahydrofuran (3 mL) was added 2N aqueous sodium hydroxide solution (6 mL), and the mixture was stirred at room temperature for 4 hr. The reaction mixture was extracted with ethyl acetate, and the organic layer was dried over anhydrous magnesium sulfate, filtrated, and concentrated under reduced pressure. The residue was subjected to silica gel column chromatography with hexane-ethyl acetate (hexane-ethyl acetate 1:1-ethyl acetate) to give 3-(2-chloro-4-[(2-hydroxyethyl)amino]benzyl}-1-cyclohexylpyrrolidin-2-one (0.11 g, 25%) as a colorless oil. The obtained oil was dissolved in ethyl acetate (2 mL), 4N hydrogen chloride-ethyl acetate (0.5 mL) was added thereto, and the mixture was stirred. The precipitated crystals were collected by filtration to give the title compound (0.096 g, 15%) as a white powder.
1H NMR (300 MHz, DMSO-d₆) δ ppm 0.90-1.18 (m, 1 H), 1.47-1.67 (m, 8 H), 1.67-1.80 (m, 2 H), 1.81-1.97 (m, 1 H), 2.33-2.69 (m, 4 H), 2.98-3.30 (m, 5 H), 3.63-3.84 (m, 1 H), 5.88-7.54 (m, 6 H).

### Example 698A 3-(2-chloro-4-phenoxybenzyl)-1-(4-oxocyclohexyl) pyrrolidin-2-one

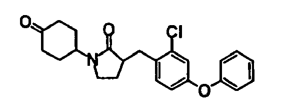

A mixture of 3-(4-bromo-2-chlorobenzyl)-1-(1,4-dioxaspiro[4.5]dec-8-yl)pyrrolidin-2-one obtained in Example 203A (0.80 g), phenol (0.19 g), copper iodide (I) (0.036 g), 8-quinolinol (0.028 g), potassium carbonate (0.13 g) and 1,3-dimethyl-2-imidazolidinone (5 mL) was stirred at 170°C for 20 hr, and the mixture was filtrated. Water was added, and the mixture was extracted with ethyl acetate. The organic layer was washed with saturated brine, dried over anhydrous magnesium sulfate, filtrated, and concentrated under reduced pressure. The obtained residue was subjected to silica gel column chromatography (hexane-ethyl acetate 1:1-ethyl acetate) to give a mixture containing 3-(2-chloro-4-phenoxybenzyl)-1-(1,4-dioxaspiro[4.5]dec-8-yl)pyrrolidin-2-one as a main component. In the same manner as in Example 28A, the title compound (0.30 g, 68%) was obtained as a white powder from this mixture.
1H NMR (300 MHz, CDCl₃) δ ppm 1.62-2.20 (m, 6 H), 2.37-2.62 (m, 4 H), 2.70-2.91 (m, 2 H), 3.13-3.27 (m, 2 H), 3.28-3.44 (m, 1 H), 4.33-4.61 (m, 1 H), 6.85 (dd, J=8.5, 2.4 Hz, 1 H), 6.96-7.04 (m, 3 H), 7.10-7.18 (m, 1 H), 7.22 (d, J=8.5 Hz, 1 H), 7.30-7.41 (m, 2 H).

### Example 699A 3-(2-chloro-4-phenoxybenzyl)-1-(4-hydroxy-4-methylcyclohexyl)pyrrolidin-2-one (less polar product) and Example 700A 3-(2-chloro-4-phenoxybenzyl)-1-(4-hydroxy-4-methylcyclohexyl)pyrrolidin-2-one (more polar product)

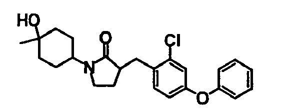

In the same manner as in Example 263A, the title compound was obtained from 3-(2-chloro-4-phenoxybenzyl)-1-(4-oxocyclohexyl)pyrrolidin-2-one obtained in Example 698A.

Example 699A (less polar product)
1H NMR (300 MHz, CDCl₃) δ ppm 0.99 (s, 1 H), 1.25 (s, 3 H), 1.38-1.63 (m, 4 H), 1.63-1.91 (m, 5 H), 1.92-2.14 (m, 1 H), 2.67-2.89 (m, 2 H), 3.13-3.44 (m, 3 H), 3.85-4.06 (m, 1 H), 6.84 (dd, J=8.4, 2.5 Hz, 1 H), 6.97-7.05 (m, 3 H), 7.14 (t, J=7.4 Hz, 1 H), 7.22 (d, J=8.5 Hz, 1 H), 7.31-7.40 (m, 2 H).

### Example 700A (more polar product)

1H NMR (300 MHz, CDCl₃) δ ppm 1.28 (s, 3 H), 1.33 (s, 1 H), 1.38-1.84 (m, 12 H), 1.95-2.13 (m, 1 H), 2.67-2.87 (m, 2 H), 3.13-3.26 (m, 2 H), 3.26-3.43 (m, 1 H), 3.90-4.07 (m, 1 H), 6.84 (dd, J=8.5, 2.5 Hz, 1 H), 6.97-7.04 (m, 3 H), 7.14 (t, J=7.4 Hz, 1 H), 7.22 (d, J=8.3 Hz, 1 H), 7.30-7.39 (m, 2 H).

### Example 701A N-(3-chloro-4-{[2-oxo-1-(4-oxocyclohexyl)pyrrolidin-3-yl]methyl}phenyl)-1-hydroxycyclopropanecarboxamide

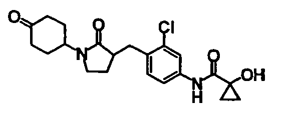

In the same manner as in Example 70A, the title compound was obtained from 3-(4-amino-2-chlorobenzyl)-1-(4-oxocyclohexyl)pyrrolidin-2-one obtained in Example 473A and 1-hydroxy-1-cyclopropanecarboxylic acid.
1H NMR (300 MHz, CDCl₃)δ ppm 1.04-1.22 (m, 2 H), 1.43-1.54 (m, 2 H), 1.59-2.15 (m, 6 H), 2.33-2.62 (m, 4 H), 2.66-2.92 (m, 3 H), 3.19 (dd, J=8.0, 6.1 Hz, 2 H), 3.27-3.44 (m, 1 H), 4.36-4.57 (m, 1 H), 7.18-7.24 (m, 1 H), 7.29-7.35 (m, 1 H), 7.72 (d, J=2.3 Hz, 1H), 8.68 (s, 1H).

### Example 702A N-(3-chloro-4-{[1-(4-hydroxy-4-methylcyclohexyl)-2-oxopyrrolidin-3-yl]methyl}phenyl)-1-hydroxy cyclopropanecarboxamide (less polar product) and Example 703A N-(3-chloro-4-{[1-(4-hydroxy-4-methylcyclohexyl)-2-oxopyrrolidin-3-yl]methyl}phenyl)-1-hydroxy cyclopropanecarboxamide (more polar product)

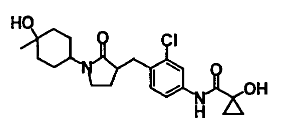

In the same manner as in Example 263A, the title compound was obtained from N-(3-chloro-4-{[2-oxo-1-(4-oxocyclohexyl)pyrrolidin-3-yl]methyl}phenyl)-1-hydroxy cyclopropanecarboxamide obtained in Example 701A.

### Example 702A (less polar product)

1H NMR (300 MHz, CDCl₃) δ ppm 1.11-1.20 (m, 2 H), 1.27 (s, 3 H), 1.33 (s, 1 H), 1.40-1.86 (m, 11 H), 1.89-2.15 (m, 1 H), 2.62-2.89 (m, 2 H), 2.95 (s, 1 H), 3.11-3.25 (m, 2 H), 3.26-3.41 (m, 1 H), 3.88-4.08 (m, 1 H), 7.12-7.40 (m, 2 H), 7.74 (d, J=2.1 Hz, 1 H), 8.66 (s, 1 H).

### Example 703A (more polar product)

1H NMR (300 MHz, CDCl₃) δ ppm 1.10-1.19 (m, 2 H), 1.27 (s, 3 H), 1.35 (s, 1 H), 1.39-1.86 (m, 11 H), 1.91-2.14 (m, 1 H), 2.62-2.90 (m, 2 H), 2.95 (s, 1 H), 3.03-3.25 (m, 2 H), 3.25-3.41 (m, 1 H), 3.86-4.06 (m, 1 H), 7.12-7.35 (m, 2 H), 7.73 (d, J=1.9 Hz, 1 H), 8.68 (s, 1 H).

### Example 704A ethyl 4-({[(3-chloro-4-{[1-(4-hydroxy-4-methylcyclohexyl)-2-oxopyrrolidin-3-yl]methyl}phenyl)amino]carbonyl}amino)benzoate

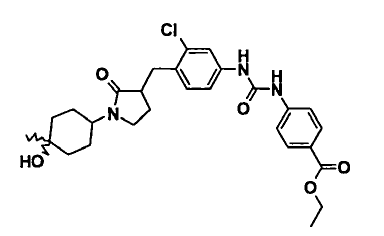

In the same manner as in Example 210A, the title compound was obtained from 3-(4-amino-2-chlorobenzyl)-1-(4-hydroxy-4-methylcyclohexyl)pyrrolidin-2-one obtained in Example 666A and ethyl 4-isocyanatobenzoate.
1H NMR (300 MHz, CDCl₃) δ ppm 1.13 (s, 3 H), 1.31 (t, J=7.2 Hz, 3 H), 1.38-1.71 (m, 9 H), 1.79-1.97 (m, 1 H), 2.55-2.73 (m, 2 H), 3.06-3.30 (m, 3 H), 3.61-3.80 (m, 1 H), 4.28 (q, J=7.2 Hz, 2 H), 4.36 (s, 1 H), 7.16-7.32 (m, 2 H), 7.52-7.63 (m, 2 H), 7.71 (d, J=1.9 Hz, 1 H), 7.80-7.94 (m, 2 H), 8.94 (s, 1 H), 9.17 (s, 1 H).

### Example 705A 4-({[(3-chloro-4-{[1-(4-hydroxy-4-methylcyclohexyl)-2-oxopyrrolidin-3-yl]methyl}phenyl)amino]carbonyl}amino)benzoic acid

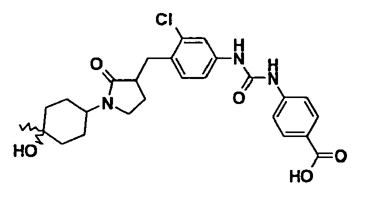

In the same manner as in Example 155A, the title compound was obtained from ethyl 4-(([(3-chloro-4-([1-(4-hydroxy-4-methylcyclohexyl)-2-oxopyrrolidin-3-yl]methyl}phenyl)amino]carbonyl}amino)benzoate obtained in Example 704A.
LC/MS(ESI+); m/z 500 (M⁺)

### Example 706A N-(3'-chloro-4'-{[1-(4-hydroxy-4-methylcyclohexyl)-2-oxopyrrolidin-3-yl]methyl}biphenyl-4-yl)methanesulfonamide

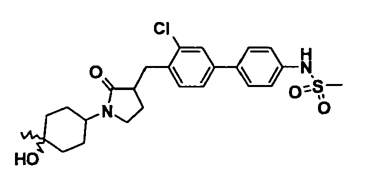

In the same manner as in Example 218A, the title compound was obtained from 3-chloro-4-{(1-(4-hydroxy-4-methylcyclohexyl)-2-oxopyrrolidin-3-yl]methyl}phenyl trifluoromethanesulfonic acid obtained in Example 646A and 4-(methanesulfonylamino)phenylboronic acid.
1H NMR (300 MHz, CDCl₃) δ ppm 1.28 (s, 3 H), 1.35 (s, 1 H), 1.47-1.88 (m, 9 H), 1.96-2.18 (m, 1 H), 2.73-2.92 (m, 2 H), 3.05 (s, 3 H), 3.16-3.30 (m, 2 H), 3.39 (d, J=8.9 Hz, 1 H), 3.87-4.12 (m, 1 H), 6.54 (s, 1 H), 7.27-7.40 (m, 4 H), 7.47-7.62 (m, 3 H). Example 707A methyl (2E)-3-(3-chloro-4-{[1-(4-hydroxy-4-methylcyclohexyl)-2-oxopyrrolidin-3-yl]methyl}phenyl)acrylate

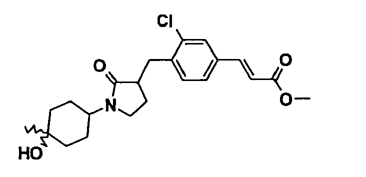

In the same manner as in Example 253A, the title compound was obtained from 3-chloro-4-{[1-(4-hydroxy-4-methylcyclohexyl)-2-oxopyrrolidin-3-yl]methyl}phenyl trifluoromethanesulfonic acid obtained in Example 646A and methyl acrylate.
1H NMR (300 MHz, CDCl₃) δ ppm 1.27 (s, 3 H), 1.35 (s, 1 H), 1.47-1.85 (m, 9 H), 1.98-2.13 (m, 1 H), 2.72-2.91 (m, 2 H), 3.13-3.28 (m, 2 H), 3.36 (t, J=9.7 Hz, 1 H), 3.81 (s, 3 H), 3.91-4.06 (m, 1H), 6.41 (d, J=16.0 Hz, 1H), 7.30-7.37 (m, 2 H), 7.51 (d, J=1.3 Hz, 1 H), 7.60 (d, J=16.0 Hz, 1 H).

### Example 708A methyl 3-(3-chloro-4-{[1-(4-hydroxy-4-methylcyclohexyl)-2-oxopyrrolidin-3-yl]methyl}phenyl)propanoate

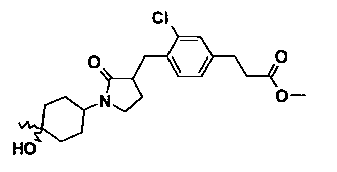

In the same manner as in Example 527A, the title compound was obtained from methyl (2E)-3-(3-chloro-4-{[1-(4-hydroxy-4-methylcyclohexyl)-2-oxopyrrolidin-3-yl]methyl}phenyl)acrylate obtained in Example 707A.
1H NMR (300 MHz, CDCl₃) δ ppm 1.27 (s, 3 H), 1.38-1.85 (m, 10 H), 1.93-2.18 (m, 1 H), 2.61 (t, J=7.7 Hz, 2 H), 2.67-2.84 (m, 2 H), 2.90 (t, J=7.7 Hz, 2 H), 3.11-3.27 (m, 2 H), 3.28-3.38 (m, 1 H), 3.68 (s, 3 H), 3.89-4.05 (m, 1 H), 7.02 (dd, J=7.7, 1.7 Hz, 1 H), 7.15-7.23 (m, 2 H).

### Example 709A 2-[(3-chloro-4-{[1-(5-hydroxy-2-adamantyl)-2-oxopyrrolidin-3-yl]methyl}phenoxy)methyl]benzoic acid

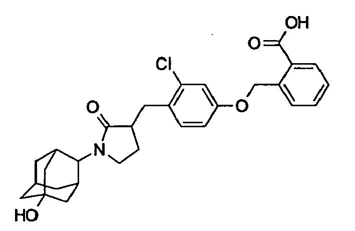

In the same manner as in Example 155A, the title compound was obtained from methyl 2-[(3-chloro-4-{[1-(5-hydroxy-2-adamantyl)-2-oxopyrrolidin-3-yl]methyl}phenoxy)methyl]benzoate obtained in Example 637A.
1H NMR (300 MHz, DMSO-d₆) δ ppm 1.34-1.45 (m, 2 H), 1.52-1.81 (m, 9 H), 1.84-2.07 (m, 2 H), 2.24-2.38 (m, 2 H), 2.46-2.70 (m, 2 H), 3.13 (dd, J=13.1, 3.5 Hz, 1 H), 3.22-3.56 (m, 3 H), 4.43 (brs, 1 H), 5.43 (s, 2 H), 6.90 (dd, J=8.5, 2.6 Hz, 1 H), 7.06 (d, J=2.6 Hz, 1 H), 7.27 (d, J=8.5 Hz, 1 H), 7.40-7.51 (m, 1 H), 7.54-7.66 (m, 2 H), 7.89-7.96 (m, 1 H), 13.09 (brs, 1 H).

### Example 710A 2-(3-chloro-4-{[1-(5-hydroxy-2-adamantyl)-2-oxopyrrolidin-3-yl]methyl}phenoxy)-N-{[4-(trifluoromethyl)phenyl]sulfonyl}acetamide

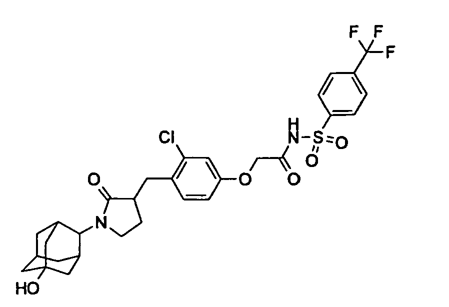

A solution of (3-chloro-4-{[1-(5-hydroxy-2-adamantyl)-2-oxopyrrolidin-3-yl]methyl}phenoxy)acetic acid obtained in Example 585A (217 mg), 4-(trifluoromethyl)benzenesulfonamide (118 mg), 4-dimethylaminopyridine (92 mg) and N-[3-(dimethylamino)propyl]-N'-ethylcarbodiimide hydrochloride (144 mg) in acetonitrile (3 mL) was stirred at room temperature for 15 hr. Water was added to the reaction mixture, and the mixture was extracted with ethyl acetate. The organic layer was washed with saturated brine, and dried over anhydrous magnesium sulfate. The solvent was evaporated under reduced pressure, and the residue was subjected to silica gel column chromatography (ethyl acetate-methanol 100:0-95:5) to give an oil. Recrystallization from ethyl acetate-diethyl ether gave the title compound (110 mg, 34%) as colorless crystals.
1H NMR (300 MHz, DMSO-d₆) δ ppm 1.34-1.46 (m, 2 H), 1.49-1.95 (m, 10 H), 2.00-2.07 (m, 1 H), 2.24-2.39 (m, 2 H), 2.41-2.65 (m, 2 H), 3.09 (dd, J=13.3, 3.7 Hz, 1 H), 3.24-3.56 (m, 2 H), 3.67-3.73 (m, 1 H), 4.28-4.48 (m, 3 H), 6.68 (dd, J=8.5, 2.6 Hz, 1 H), 6.76 (d, J=2.6 Hz, 1 H), 7.17 (d, J=8.5 Hz, 1 H), 7.80 (d, J=8.1 Hz, 2 H), 7.96 (d, J=8.1 Hz, 2 H).

### Example 711A 2-(3-chloro-4-{[1-(4-hydroxy-4-methylcyclohexyl)-2-oxopyrrolidin-3-yl]methyl}phenoxy)-N-(2,4-dichlorobenzyl)acetamide

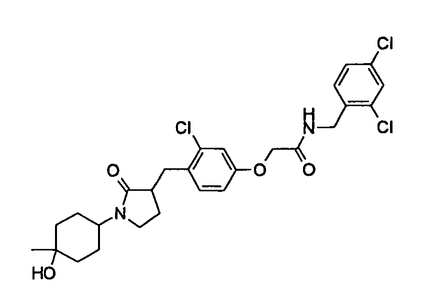

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and 1-(2,4-dichlorophenyl)methanamine.
1H NMR (300 MHz, CDCl₃) δ ppm 1.27 (s, 3 H), 1.42-1.82 (m, 10 H), 1.95-2.08 (m, 1 H), 2.68-2.82 (m, 2 H), 3.13-3.34 (m, 3 H), 3.90-4.04 (m, 1 H), 4.49 (s, 2 H), 4.58 (d, J=6.4 Hz, 2 H), 6.75 (dd, J=8.5, 2.6 Hz, 1 H), 6.92 (d, J=2.6 Hz, 1 H), 6.94-7.02 (m, 1 H), 7.18-7.25 (m, 2 H), 7.29-7.34 (m, 1 H), 7.38 (d, J=2.1 Hz, 1 H) .

### Example 712A 2-(3-chloro-4-{[1-(4-hydroxy-4-methylcyclohexyl)-2-oxopyrrolidin-3-yl]methyl}phenoxy)-N-[4-(trifluoromethyl)phenyl]acetamide

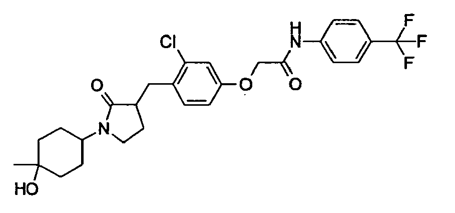

In the same manner as in Example 70A, the title compound was obtained from (3-chloro-4-{[1-(4-hydroxy-4-methylcyclohexyl)-2-oxopyrrolidin-3-yl]methyl}phenoxy)acetic acid obtained in Example 617A and 4-(trifluoromethyl)aniline.
1H NMR (300 MHz, CDCl₃) δ ppm 1.27 (s, 3 H), 1.41-2.14 (m, 11 H), 2.70-2.84 (m, 2 H), 3.15-3.36 (m, 3 H), 3.90-4.04 (m, 1 H), 4.60 (s, 2 H), 6.84 (dd, J=8.5, 2.6 Hz, 1 H), 7.03 (d, J=2.6 Hz, 1 H), 7.22-7.30 (m, 1 H), 7.57-7.66 (m, 2 H), 7.70-7.79 (m, 2 H), 8.37 (brs, 1 H).

### Example 713A 2-(3-chloro-4-{[1-(4-hydroxy-4-methylcyclohexyl)-2-oxopyrrolidin-3-yl]methyl}phenoxy)-N-{[4-(trifluoromethyl)phenyl]sulfonyl}acetamide

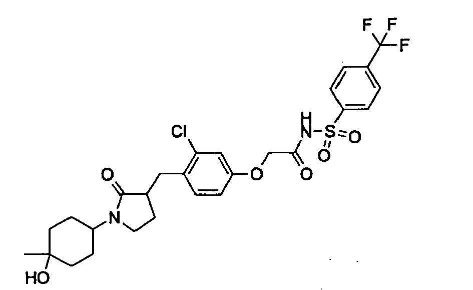

In the same manner as in Example 710A, the title compound was obtained from (3-chloro-4-{[1-(4-hydroxy-4-methylcyclohexyl)-2-oxopyrrolidin-3-yl]methyl}phenoxy)acetic acid obtained in Example 617A and 4-(trifluoromethyl)benzenesulfonamide.
1H NMR (300 MHz, DMSO-d₆) δ ppm 1.13 (s, 3 H), 1.37-1.64 (m, 10 H), 1.78-1.92 (m, 1 H), 2.38-2.66 (m, 2 H), 3.02-3.39 (m, 3 H), 3.64-3.77 (m, 1 H), 4.26 (s, 2 H), 4.35 (brs, 1 H), 6.67 (dd, J=8.5, 2.5 Hz, 1 H), 6.73 (d, J=2.5 Hz, 1 H), 7.16 (d, J=8.5 Hz, 1 H), 7.74 (d, J=8.1 Hz, 2 H), 7.91 (d, J=8.1 Hz, 2 H).

### Example 1B 1,3-bis(2,6-dichlorobenzyl)imidazolidin-2-one

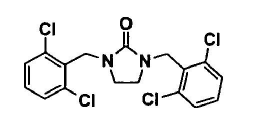

In the same manner as in Reference Example 1B, the title compound was obtained from ethyleneurea and α,2,6-trichlorotoluene.
1H NMR (300 MHz, CDCl₃) δ ppm 3.10 (s, 4 H), 4.76 (s, 4 H), 7.08-7.24 (m, 2 H), 7.28-7.45 (m, 4 H).

### Example 2B 1-benzyl-3-(2,6-dichlorobenzyl)imidazolidin-2-one

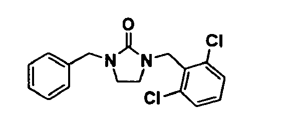

In the same manner as in Reference Example 1B, the title compound was obtained from 1-(2,6-dichlorobenzyl) imidazolidin-2-one and benzyl bromide.
1H NMR (300 MHz, CDCl₃) δ ppm 3.01-3.22 (m, 4 H), 4.41 (s, 2 H), 4.77 (s, 2 H), 7.12-7.42 (m, 8 H).

### Example 3B 1-(2,6-dichlorobenzyl)-3-(2-methylbenzyl)imidazolidin-2-one

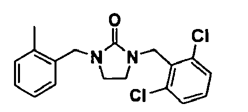

In the same manner as in Reference Example 1B, the title compound was obtained from 1-(2,6-dichlorobenzyl)imidazolidin-2-one and α-bromo-o-xylene.
1H NMR (300 MHz, CDCl₃) δ ppm 2.34 (s, 3H), 2.98-3.28 (m, 4 H), 4.42 (s, 2 H), 4.77 (s, 2 H), 7.07-7.24 (m, 5 H), 7.29-7.38 (m, 2 H).

### Example 4B 1-(2,6-dichlorobenzyl)-3-(2,6-dimethylbenzyl)imidazolidin-2-one

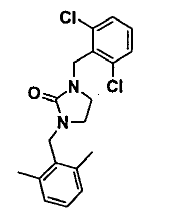

In the same manner as in Reference Example 1B, the title compound was obtained from 1-(2,6-dichlorobenzyl)imidazolidin-2-one and 2,6-dimethylbenzyl chloride.
1H NMR (300 MHz, CDCl₃) δ ppm 2.36 (s, 6 H), 2.85-3.18 (m, 4 H), 4.50 (s, 2 H), 4.75 (s, 2 H), 6.97-7.05 (m, 2 H), 7.05-7.13 (m, 1 H), 7.13-7.22 (m, 1 H), 7.28-7.39 (m, 2 H).

### Example 5B 1-(2,6-dichlorobenzyl)-3-(1-phenylethyl)imidazolidin-2-one

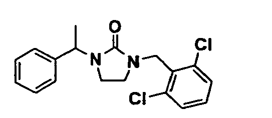

In the same manner as in Reference Example 1B, the title compound was obtained from 1-(2,6-dichlorobenzyl)imidazolidin-2-one and (1-bromoethyl)benzene.
1H NMR (300 MHz, CDCl₃) δ ppm 1.53 (d, J=7.2 Hz, 3 H), 2.79-2.92 (m, 1 H), 3.00-3.27 (m, 3 H), 4.75 (s, 2 H), 5.33 (q, J=7.2 Hz, 1 H), 7.05-7.46 (m, 8 H).

### Example 6B 1-(2-methylbenzyl)-3-phenylimidazolidin-2-one

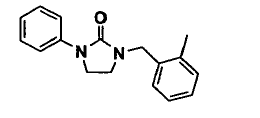

In the same manner as in Reference Example 1B, the title compound was obtained from 1-phenylimidazolidin-2-one and α-bromo-o-xylene.
1H NMR (300 MHz, CDCl₃) δ ppm 2.36 (s, 3 H), 3.24-3.38 (m, 2 H), 3.72-3.86 (m, 2 H), 4.50 (s, 2 H), 6.96-7.10 (m, 1 H), 7.12-7.28 (m, 4 H), 7.29-7.43 (m, 2 H), 7.52-7.65 (m, 2 H).

### Example 7B 1-phenyl-3-(1-phenylethyl)imidazolidin-2-one

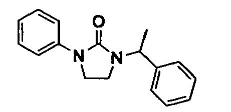

In the same manner as in Reference Example 1B, the title compound was obtained from 1-phenylimidazolidin-2-one and (1-bromoethyl)benzene.
1H NMR (300 MHz, CDCl₃) δ ppm 1.59 (d, J=7.2 Hz, 3 H), 3.02-3.17 (m, 1 H), 3.37-3.52 (m, 1 H), 3.64-3.86 (m, 2 H), 5.41 (q, J=7.2 Hz, 1 H), 6.96-7.10 (m, 1 H), 7.23-7.44 (m, 7 H), 7.50-7.64 (m, 2 H).

### Example 8B 1-(cyclopropylmethyl)-3-(2-methylbenzyl)imidazolidin-2-one

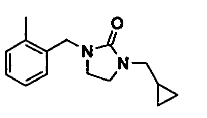

In the same manner as in Reference Example 1B, the title compound was obtained from 1-(2-methylbenzyl)imidazolidin-2-one obtained in Reference Example 1B and cyclopropylmethyl bromide.
1H NMR (300 MHz, CDCl₃) δ ppm 0.16-0.24 (m, 2 H), 0.46-0.57 (m, 2 H), 2.34 (s, 3 H), 3.10 (d, J=7.2 Hz, 2 H), 3.12-3.23 (m, 2 H), 3.34-3.45 (m, 2 H), 4.38 (s, 2 H), 7.11-7.24 (m, 4 H).

### Example 9B 1,3-bis(2-methylbenzyl) imidazolidin-2-one

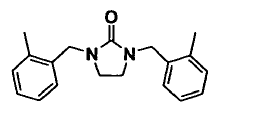

In the same manner as in Reference Example 1B, the title compound was obtained from ethyleneurea and α-bromo-o-xylene.
1H NMR (300 MHz, CDCl₃) δ ppm 2.35 (s, 6 H), 3.12 (s, 4 H), 4.43 (s, 4 H), 7.09-7.24 (m, 8 H).

### Example 10B 1-benzyl-3-(2-methylbenzyl)imidazolidin-2-one

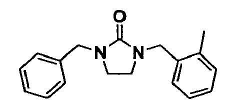

In the same manner as in Reference Example 1B, the title compound was obtained from 1-(2-methylbenzyl)imidazolidin-2-one obtained in Reference Example 1B and benzyl bromide.
1H NMR (300 MHz, CDCl₃) δ ppm 2.35 (s, 3 H), 3.06-3.25 (m, 4 H), 4.41 (s, 2 H), 4.43 (s, 2 H), 7.06-7.45 (m, 9 H).

### Example 11B 1-(2-chlorobenzyl)-3-(2-methylbenzyl)imidazolidin-2-one

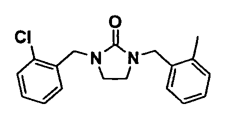

In the same manner as in Reference Example 1B, the title compound was obtained from 1-(2-methylbenzyl)imidazolidin-2-one obtained in Reference Example 1B and 2-chlorobenzyl bromide.
1H NMR (300 MHz, CDCl₃) δ ppm 2.35 (s, 3 H), 3.06-3.36 (m, 4 H), 4.43 (s, 2 H), 4.57 (s, 2 H), 7.06-7.31 (m, 6 H), 7.32-7.44 (m, 2 H).

### Example 12B 1-(2-methylbenzyl)-3-(4-nitrobenzyl)imidazolidin-2-one

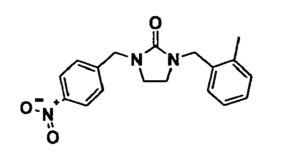

In the same manner as in Reference Example 1B, the title compound was obtained from 1-(2-methylbenzyl)imidazolidin-2-one obtained in Reference Example 1B and 4-nitrobenzyl bromide.
1H NMR (300 MHz, CDCl₃) δ ppm 2.36 (s, 3 H), 3.19 (s, 4 H), 4.45 (s, 2 H), 4.51 (s, 2 H), 7.19 (s, 4 H), 7.46 (d, J=8.9 Hz, 2 H), 8.16-8.23 (m, 2 H).

### Example 13B 1-(2,6-dichlorobenzyl)-3-(4-nitrobenzyl)imidazolidin-2-one

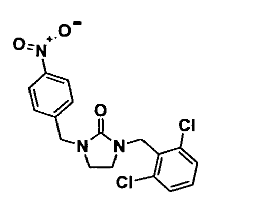

In the same manner as in Reference Example 1B, the title compound was obtained from 1-(2,6-dichlorobenzyl)imidazolidin-2-one and 4-nitrobenzyl bromide.
1H NMR (300 MHz, CDCl₃) δ ppm 3.13-3.23 (m, 4 H), 4.50 (s, 2 H), 4.78 (s, 2 H), 7.17-7.22 (m, 1 H), 7.33 (d, J=8.1 Hz, 2 H), 7.44 (d, J=8.1Hz, 2 H), 8.17-8.20 (m, 2 H).

### Example 14B 1-(4-aminobenzyl)-3-(2-methylbenzyl)imidazolidin-2-one

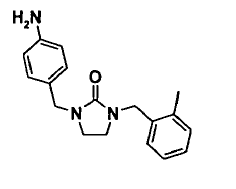

A mixture of 1-(2-methylbenzyl)-3-(4-nitrobenzyl)imidazolidin-2-one obtained in Example 12B (0.60 g), zinc powder (1.0 g) and acetic acid-tetrahydrofuran (1:1, 20 mL) was stirred at room temperature for 2 hr. The reaction solution was filtrated, and the filtrate was concentrated under reduced pressure. The residue was partitioned between ethyl acetate and 10% aqueous sodium bicarbonate, and the ethyl acetate layer was washed with saturated brine, and dried over anhydrous magnesium sulfate. The solvent was evaporated under reduced pressure, and the residue was subjected to silica gel column chromatography (hexane-ethyl acetate 4:1-3:7) to give the title compound (0.78 g, 69%) as a pale-yellow solid.
1H NMR (300 MHz, CDCl₃) δ ppm 2.34 (s, 3 H), 3.11 (s, 4 H), 3.64 (brs, 2H), 4.29 (s, 2 H), 4.41 (s, 2 H), 6.64 (d, J=12.6 Hz, 2 H), 7.33 (d, J=12.6 Hz, 2 H), 7.17 (s, 4 H).

### Example 15B 1-(4-aminobenzyl)-3-(2,6-dichlorobenzyl)imidazolidin-2-one

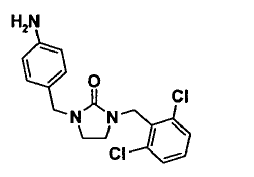

In the same manner as in Example 14B, the title compound was obtained from 1-(2,6-dichlorobenzyl)-3-(4-nitrobenzyl)imidazolidin-2-one obtained in Example 13B.
1H NMR (300 MHz, CDCl₃) δ ppm 3.07-3.12 (m, 4 H), 4.28 (s, 2 H), 4.74 (s, 2 H), 6.63 (d, J=8.7 Hz, 2 H), 7.04-7.07 (m, 2 H), 7.14-7.17 (m, 1 H), 7.31 (d, J=8.7 Hz, 2 H).

### Example 16B N-(4-{[3-(2-methylbenzyl)-2-oxoimidazolidin-1-yl]methyl}phenyl)acetamide

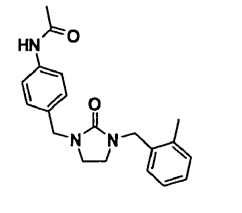

A mixture of 1-(4-aminobenzyl)-3-(2-methylbenzyl)imidazolidin-2-one obtained in Example 14B (0.15 g), acetyl chloride (44 mg), triethylamine (76 µL) and tetrahydrofuran (6 mL) was stirred at room temperature for 1 hr. 10% Aqueous sodium bicarbonate was added to the reaction solution, and the mixture was extracted with ethyl acetate. The ethyl acetate layer was washed with saturated brine, and dried over anhydrous magnesium sulfate. The solvent was evaporated under reduced pressure, and the residue was subjected to NH-silica gel column chromatography (hexane-ethyl acetate 1:1) to give the title compound (0.15 g, 82%) as a pale-yellow solid.
1H NMR (300 MHz, CDCl₃) δ ppm 2.17 (s, 3 H), 2.34 (s, 3 H), 3.03-3.22 (m, 4 H), 4.37 (s, 2 H), 4.42 (s, 2 H), 7.08-7.25 (m, 6 H), 7.37 (s, 1 H), 7.46 (d, J=8.3 Hz, 2 H).

### Example 17B N-(4-{[3-(2-methylbenzyl)-2-oxoimidazolidin-1-yl]methyl}phenyl)benzamide

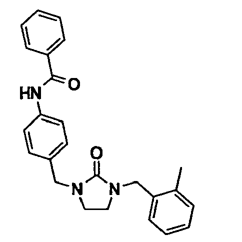

In the same manner as in Example 16B, the title compound was obtained from 1-(4-aminobenzyl)-3-(2-methylbenzyl)imidazolidin-2-one obtained in Example 14B and benzoyl chloride.
1H NMR (300 MHz, CDCl₃) δ ppm 2.34 (s, 3 H), 3.07-3.22 (m, 4 H), 4.40 (s, 2 H), 4.42 (s, 2 H), 7.12-7.23 (m, 4 H), 7.29 (d, J=8.5 Hz, 2 H), 7.43-7.57 (m, 3 H), 7.62 (d, J=8.5 Hz, 2 H), 7.82-7.96 (m, 3 H) .

### Example 18B N-(4-{[3-(2,6-dichlorobenzyl)-2-oxoimidazolidin-1-yl]methyl}phenyl)benzamide

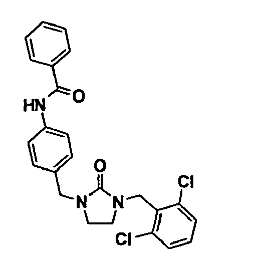

In the same manner as in Example 16B, the title compound was obtained from 1-(4-aminobenzyl)-3-(2,6-dichlorobenzyl)imidazolidin-2-one obtained in Example 15B and benzoyl chloride.
1H NMR (300 MHz, CDCl₃) δ ppm 2.93-3.31 (m, 4 H), 4.39 (s, 2 H), 4.76 (s, 2 H), 7.14-7.23 (m, 1 H), 7.28-7.38 (m, 3 H), 7.44-7.67 (m, 5 H), 7.84-7.91 (m, 2 H), 7.96 (s, 1 H).

### Example 19B N-(4-{[3-(2-methylbenzyl)-2-oxoimidazolidin-1-yl]methyl}phenyl)-2-phenylacetamide

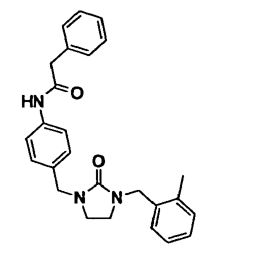

In the same manner as in Example 16B, the title compound was obtained from 1-(4-aminobenzyl)-3-(2-methylbenzyl)imidazolidin-2-one obtained in Example 14B and phenylacetyl chloride.
1H NMR (300 MHz, CDCl₃) δ ppm 2.33 (s, 3 H), 3.10 (s, 4 H), 3.74 (s, 2 H), 4.35 (s, 2 H), 4.40 (s, 2 H), 7.11 (s, 1 H), 7.13-7.24 (m, 6 H), 7.30-7.50 (m, 7 H).

### Example 20B N-(4-{[3-(2,6-dichlorobenzyl)-2-oxoimidazolidin-1-yl]methyl}phenyl)acetamide

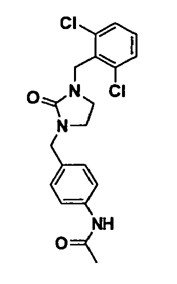

In the same manner as in Example 16B, the title compound was obtained from 1-(4-aminobenzyl)-3-(2,6-dichlorobenzyl)imidazolidin-2-one obtained in Example 15B and acetyl chloride.
1H NMR (300 MHz, CDCl₃) δ ppm 2.18 (s, 3 H), 3.01-3.25 (m, 4 H), 4.36 (s, 2 H), 4.76 (s, 2 H), 7.11-7.25 (m, 3 H), 7.29-7.37 (m, 2 H), 7.39-7.54 (m, 3 H).

### Example 21B 1-(3,3-dimethyl-2-oxobutyl)-3-(2-methylbenzyl)imidazolidin-2-one

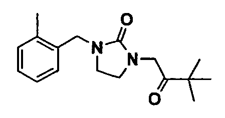

In the same manner as in Reference Example 1B, the title compound was obtained from 1-(2-methylbenzyl)imidazolidin-2-one obtained in Reference Example 1B and 1-chloropinacolone.
1H NMR (300 MHz, CDCl₃) δ ppm 1.20 (s, 9 H), 2.34 (s, 3 H), 3.17-3.25 (m, 2 H), 3.32-3.40 (m, 2 H), 4.20 (s, 2 H), 4.40 (s, 2 H), 7.14-7.24 (m, 4 H).

### Example 22B 2-{[3-(2-methylbenzyl)-2-oxoimidazolidin-1-yl]methyl}benzonitrile

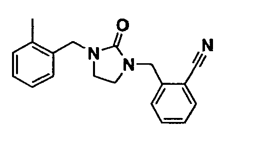

In the same manner as in Reference Example 1B, the title compound was obtained from 1-(2-methylbenzyl)imidazolidin-2-one obtained in Reference Example 1B and 2-(bromomethyl)benzonitrile.
1H NMR (300 MHz, CDCl₃) δ ppm 2.34 (s, 3 H), 3.11-3.23 (m, 2 H), 3.25-3.37 (m, 2 H), 4.43 (s, 2 H), 4.64 (s, 2 H), 7.09-7.24 (m, 4 H), 7.31-7.45 (m, 1 H), 7.51-7.73 (m, 3 H).

### Example 23B 3-{[3-(2-methylbenzyl)-2-oxoimidazolidin-1-yl]methyl}benzonitrile

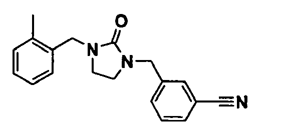

In the same manner as in Reference Example 1B, the title compound was obtained from 1-(2-methylbenzyl)imidazolidin-2-one obtained in Reference Example 1B and 3-(bromomethyl)benzonitrile.
1H NMR (300 MHz, CDCl₃) δ ppm 2.36 (s, 3 H), 3.18 (s, 4 H), 4.44 (s, 4 H), 7.11-7.25 (m, 4 H), 7.45 (t, J=7.9 Hz, 1 H), 7.51-7.66 (m, 3 H).

### Example 24B 4-{[3-(2-methylbenzyl)-2-oxoimidazolidin-1-yl]methyl}benzonitrile

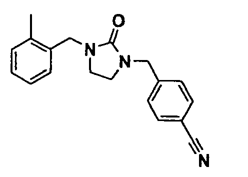

In the same manner as in Reference Example 1B, the title compound was obtained from 1-(2-methylbenzyl)imidazolidin-2-one obtained in Reference Example 1B and 4-(bromomethyl)benzonitrile.
1H NMR (300 MHz, CDCl₃) δ ppm 2.35 (s, 3 H), 3.18 (s, 4 H), 4.44 (s, 2 H), 4.46 (s, 2 H), 7.10-7.24 (m, 4 H), 7.40 (d, J=8.5 Hz, 2 H), 7.58-7.68 (m, 2 H).

### Example 25B 1-(2-methylbenzyl)-3-(pyridin-2-ylmethyl)imidazolidin-2-one

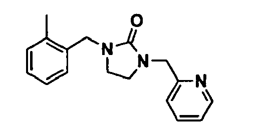

In the same manner as in Reference Example 1B, the title compound was obtained from 1-(2-methylbenzyl)imidazolidin-2-one obtained in Reference Example 1B and 2-(chloromethyl)pyridine.
1H NMR (300 MHz, CDCl₃) δ ppm 2.36 (s, 3 H), 3.11-3.23 (m, 2 H), 3.24-3.42 (m, 2 H), 4.43 (s, 2 H), 4.55 (s, 2 H), 7.10-7.25 (m, 5 H), 7.35 (d, J=7.9 Hz, 1 H), 7.60-7.76 (m, 1 H), 8.50-8.57 (m, 1 H).

### Example 26B 1-(2-methylbenzyl)-3-(pyridin-3-ylmethyl)imidazolidin-2-one

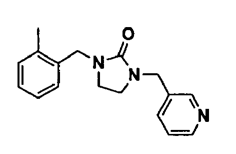

In the same manner as in Reference Example 1B, the title compound was obtained from 1-(2-methylbenzyl)imidazolidin-2-one obtained in Reference Example 1B and 3-(chloromethyl)pyridine.
1H NMR (300 MHz, CDCl₃) δ ppm 2.35 (s, 3 H), 3.05-3.22 (m, 4 H), 4.43 (s, 4 H), 7.12-7.24 (m, 4 H), 7.25-7.36 (m, 1 H), 7.56-7.79 (m, 1 H), 8.44-8.60 (m, 2 H).

### Example 27B 1-(2-methylbenzyl)-3-(pyridin-4-ylmethyl)imidazolidin-2-one

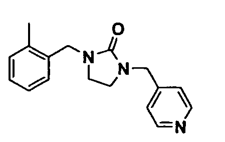

In the same manner as in Reference Example 1B, the title compound was obtained from 1-(2-methylbenzyl)imidazolidin-2-one obtained in Reference Example 1B and 4-(chloromethyl)pyridine.
1H NMR (300 MHz, CDCl₃) δ ppm 2.36 (s, 3 H), 3.19 (s, 4 H), 4.42 (s, 2 H), 4.45 (s, 2 H), 7.09-7.25 (m, 6 H), 8.47-8.63 (m, 2 H).

### Example 28B methyl 4-{[3-(2-methylbenzyl)-2-oxoimidazolidin-1-yl]methyl}benzoate

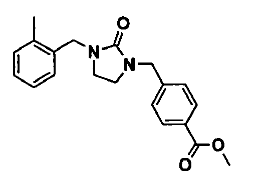

In the same manner as in Reference Example 1B, the title compound was obtained from 1-(2-methylbenzyl)imidazolidin-2-one obtained in Reference Example 1B and methyl 4-bromomethylbenzoate.
1H NMR (300 MHz, CDCl₃) δ ppm 2.35 (s, 3 H), 3.16 (s, 4 H), 3.91 (s, 3 H), 4.44 (s, 2 H), 4.47 (s, 2 H), 7.08-7.25 (m, 4 H), 7.36 (d, J=8.5 Hz, 2 H), 8.01 (d, J=8.5 Hz, 2 H).

### Example 29B 4-{[3-(2-methylbenzyl)-2-oxoimidazolidin-1-yl]methyl}benzoic acid

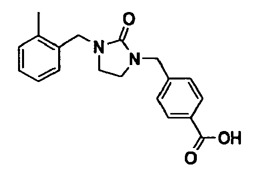

A mixture of methyl 4-{[3-(2-methylbenzyl)-2-oxoimidazolidin-1-yl]methyl}benzoate obtained in Example 28B (1.36 g), 2N sodium hydroxide (4.8 mL) and methanol (20 mL) was stirred at 45°C for 1 hr. The reaction solution was concentrated under reduced pressure, and the residue was diluted with water, and adjusted with 6N hydrochloric acid to pH 2. The solution was extracted with ethyl acetate, and the ethyl acetate layer was washed with saturated brine, and dried over anhydrous magnesium sulfate. The solvent was evaporated under reduced pressure to give the title compound (1.20 g, 92%) as a colorless powder.
1H NMR (300 MHz, CDCl₃) δ ppm 2.36 (s, 3 H), 3.18 (s, 4 H), 4.45 (s, 2 H), 4.49 (s, 2 H), 7.09-7.24 (m, 4 H), 7.40 (d, J=8.3 Hz, 2 H), 8.07 (d, J=8.3 Hz, 2 H).

### Example 30B 1-[4-(hydroxymethyl)benzyl]-3-(2-methylbenzyl)imidazolidin-2-one

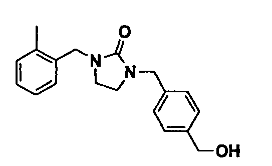

To a mixture of methyl 4-{[3-(2-methylbenzyl)-2-oxoimidazolidin-1-yl]methyl}benzoate methyl obtained in Example 28B (0.20 g) and tetrahydrofuran (15 mL) was added lithium aluminum hydride (0.03 g) at 0°C, and the mixture was stirred for 1 hr. Sodium sulfate decahydrate was added to the reaction mixture, and the mixture was filtrated through celite. The filtrate was concentrated, and the residue was subjected to silica gel column chromatography (ethyl acetate) to give the title compound (0.19 g, 100%) as a colorless oil.
1H NMR (300 MHz, CDCl₃) δ ppm 1.79 (t, J=5.8 Hz, 1 H), 2.35 (s, 3 H), 3.06-3.20 (m, 4 H), 4.40 (s, 2 H), 4.42 (s, 2 H), 4.69 (d, J=5.8 Hz, 2 H), 7.08-7.25 (m, 4 H), 7.27-7.45 (m, 4 H).

### Example 31B 1-(2,6-dichlorobenzyl)-3-(2-methylbenzyl)imidazolidine-2,4-dione

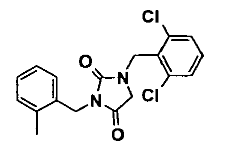

In the same manner as in Reference Example 1B, the title compound was obtained from 3-(2-methylbenzyl)imidazolidine-2,4-dione obtained in Reference Example 2B and α,2,6-trichlorotoluene.
1H NMR (300 MHz, CDCl₃) δ ppm 2.45 (s, 3 H), 3.70 (s, 2 H), 4.71 (s, 2 H), 4.95 (s, 2 H), 7.06-7.20 (m, 3 H), 7.22-7.32 (m, 2 H), 7.33-7.43 (m, 2H).

### Example 32B 1-(2,6-dichlorobenzyl)-4-hydroxy-4-methyl-3-(2-methylbenzyl)imidazolidin-2-one

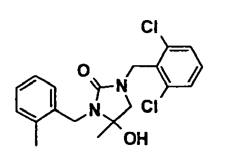

To a mixture of 1-(2,6-dichlorobenzyl)-3-(2-methylbenzyl)imidazolidine-2,4-dione obtained in Example 31B (0.20 g) and tetrahydrofuran (10 mL) was added methylmagnesium bromide (3 M tetrahydrofuran solution, 1.65 mL), and the mixture was stirred at room temperature for 1 hr. 10% Aqueous ammonium chloride was added to the reaction solution, and the mixture was extracted with ethyl acetate. The ethyl acetate layer was washed with saturated brine, and dried over anhydrous magnesium sulfate. The solvent was evaporated under reduced pressure to give the title compound (0.18 g, 86%) as a colorless solid.
1H NMR (300 MHz, CDCl₃) δ ppm 1.30 (s, 3 H), 2.37 (s, 3 H), 3.13 (d, J=9.9 Hz, 1 H), 3.21 (d, J=9.9 Hz, 1 H), 4.45 (d, J=16.2 Hz, 1 H), 4.57 (d, J=16.2 Hz, 1 H), 4.73 (d, J=14.1 Hz, 1 H), 4.98 (d, J=14.1 Hz, 1 H), 7.06-7.42 (m, 7 H).

### Example 33B tert-butyl (methyl)(2-{[3-(2-methylbenzyl)-2-oxoimidazolidin-1-yl]methyl}phenyl)carbamate

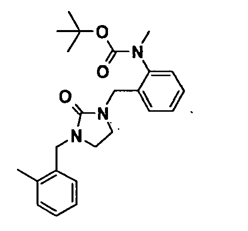

In the same manner as in Reference Example 1B, the title compound was obtained from 1-(2-methylbenzyl)imidazolidin-2-one obtained in Reference Example 1B and tert-butyl [2-(bromomethyl)phenyl](methyl)carbamate.
1H NMR (300 MHz, CDCl₃) δ ppm 1.34 and 1.51 (s, 9 H), 2.36 (s, 3 H), 3.16 (brs, 7 H), 4.26-4.51 (m, 4 H), 7.04-7.30 (m, 7 H), 7.31-7.41 (m, 1 H).

### Example 34B tert-butyl (methyl)(3-{[3-(2-methylbenzyl)-2-oxoimidazolidin-1-yl]methyl}phenyl)carbamate

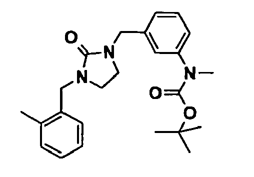

In the same manner as in Reference Example 1B, the title compound was obtained from 1-(2-methylbenzyl)imidazolidin-2-one obtained in Reference Example 1B and tert-butyl [3-(bromomethyl)phenyl](methyl)carbamate.
1H NMR (300 MHz, CDCl₃) δ ppm 1.44 (s, 9 H), 2.35 (s, 3 H), 3.10-3.22 (m, 4 H), 3.25 (s, 3 H), 4.38-4.46 (m, 4 H), 7.08 (d, J=7.5 Hz, 2 H), 7.12-7.24 (m, 5 H), 7.26-7.35 (m, 1 H).

### Example 35B tert-butyl (methyl)(4-{[3-(2-methylbenzyl)-2-oxoimidazolidin-1-yl]methyl}phenyl)carbamate

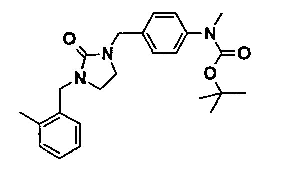

In the same manner as in Reference Example 1B, the title compound was obtained from 1-(2-methylbenzyl)imidazolidin-2-one obtained in Reference Example 1B and tert-butyl [4-(bromomethyl)phenyl](methyl)carbamate obtained in Reference Example 12A.
1H NMR (300 MHz, CDCl₃) δ ppm 1.45 (s, 9 H), 2.35 (s, 3 H), 3.05-3.19 (m, 4 H), 3.25 (s, 3 H), 4.39 (s, 2 H), 4.42 (s, 2 H), 7.04-7.34 (m, 8 H).

### Example 36B 1-(cyclopropylmethyl)-3-(2,6-dichlorobenzyl)imidazolidin-2-one

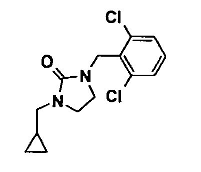

In the same manner as in Reference Example 1B, the title compound was obtained from 3-(2,6-dichlorobenzyl)imidazolidin-2-one and cyclopropylmethyl bromide.
1H NMR (300 MHz, CDCl₃) δ ppm 0.15-0.24 (m, 2 H), 0.45-0.55 (m, 2 H), 0.81-0.98 (m, 1 H), 3.09 (d, J=7.0 Hz, 2 H), 3.13-3.24 (m, 2 H), 3.30-3.42 (m, 2 H), 4.72 (s, 2 H), 7.12-7.23 (m, 1 H), 7.28-7.42 (m, 2 H).

### Example 37B methyl 2-{[3-(2,6-dichlorobenzyl)-2-oxoimidazolidin-1-yl]methyl}benzoate

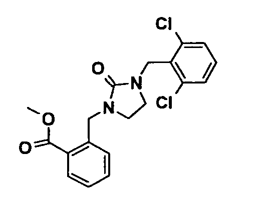

In the same manner as in Example 1B, the title compound was obtained from 1-(2,6-dichlorobenzyl)imidazolidin-2-one and methyl 2-bromomethylbenzoate.
1H NMR (300 MHz, CDCl₃) δ ppm 3.08-3.30 (m, 4 H), 3.90 (s, 3 H), 4.78 (s, 2 H), 4.80 (s, 2 H), 7.12-7.24 (m, 1 H), 7.28-7.38 (m, 3 H), 7.43-7.54 (m, 2 H), 7.82-8.00 (m, 1 H).

### Example 38B 1-(2,6-dichlorobenzyl)-3-[2-(hydroxymethyl)benzyl]imidazolidin-2-one

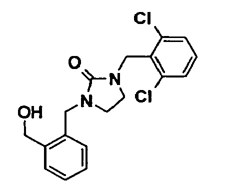

In the same manner as in Example 30B, the title compound was obtained from methyl 2-{[3-(2,6-dichlorobenzyl)-2-oxoimidazolidin-1-yl]methyl}benzoate obtained in Example 37B.
1H NMR (300 MHz, CDCl₃) δ ppm 3.11-3.27 (m, 4 H), 3.72 (t, J=6.6 Hz, 1 H), 4.54 (s, 2 H), 4.75 (t like, 4 H), 7.13-7.43 (m, 7 H). Example 39B methyl [3-(2,6-dichlorobenzyl)-2-oxoimidazolidin-1-yl]acetate

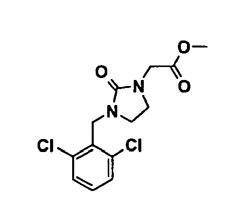

In the same manner as in Example 1B, the title compound was obtained from 1-(2,6-dichlorobenzyl)imidazolidin-2-one and methyl bromoacetate.
1H NMR (300 MHz, CDCl₃) δ ppm 3.17-3.28 (m, 2 H), 3.32-3.45 (m, 2 H), 3.73 (s, 3 H), 4.02 (s, 2 H), 4.75 (s, 2 H), 7.11-7.24 (m, 1 H), 7.30-7.41 (m, 2 H).

### Example 40B [3-(2,6-dichlorobenzyl)-2-oxoimidazolidin-1-yl]acetic acid

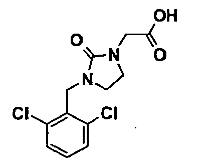

In the same manner as in Example 29B, the title compound was obtained from methyl [3-(2,6-dichlorobenzyl)-2-oxoimidazolidin-1-yl]acetate obtained in Example 39B.
1H NMR (300 MHz, CDCl₃) δ ppm 3.23-3.32 (m, 2 H), 3.38-3.50 (m, 2 H), 4.02 (s, 2 H), 4.76 (s, 2 H), 7.16-7.24 (m, 1 H), 7.31-7.38 (m, 2 H).

### Example 41B 2-[3-(2,6-dichlorobenzyl)-2-oxoimidazolidin-1-yl]-N-methyl-N-phenylacetamide

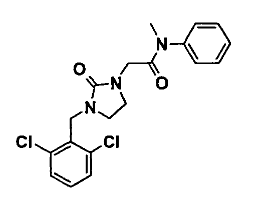

A mixture of [3-(2,6-dichlorobenzyl)-2-oxoimidazolidin-1-yl]acetic acid obtained in Example 40B (0.18 g), N-methylaniline (0.07 g), 1-ethyl-3-(3'-dimethylaminopropyl)carbodiimide (0.12 g), N-hydroxybenzotriazole (0.10 g) and acetonitrile (10 mL) was stirred at room temperature for 16 hr. Water was added to the reaction solution, and the mixture was extracted with ethyl acetate. The ethyl acetate layer was washed successively with 1N hydrochloric acid, 10% aqueous sodium bicarbonate and saturated brine, and dried over anhydrous magnesium sulfate, and the solvent was evaporated under reduced pressure. The residue was subjected to silica gel column chromatography (hexane-ethyl acetate 4:1-ethyl acetate) to give the title compound (0.12 g, 52%) as a colorless solid.
1H NMR (300 MHz, CDCl₃) δ ppm 3.14-3.25 (m, 2 H), 3.26 (s, 3 H), 3.40 (t, J=7.8 Hz, 2 H), 3.74 (s, 2 H), 4.70 (s, 2 H), 7.10-7.22 (m, 1 H), 7.21-7.52 (m, 7 H).

### Example 42B 1,3-bis(2-methylbenzyl)-1,3-dihydro-2H-benzoimidazol-2-one

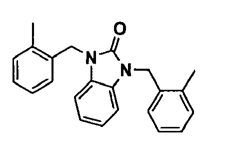

In the same manner as in Reference Example 1B, the title compound was obtained from 1,3-dihydro-2H-benzoimidazol-2-one and α-bromo-o-xylene.
1H NMR (300 MHz, CDCl₃) δ ppm 2.41 (s, 6 H), 5.14 (s, 4 H), 6.71-6.86 (m, 2 H), 6.90-7.01 (m, 2 H), 7.02-7.23 (m, 8 H).

### Example 43B 1-(2,6-dichlorobenzyl)-3-(2-methylbenzyl)-1,3-dihydro-2H-benzoimidazol-2-one

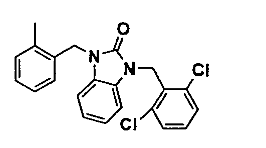

In the same manner as in Reference Example 1B, the title compound was obtained from 1-(2-methylbenzyl)-1,3-dihydro-2H-benzoimidazol-2-one and α,2,6-trichlorotoluene.
1H NMR (300 MHz, CDCl₃) δ ppm 2.39 (s, 3 H), 5.11 (s, 2 H), 5.45 (s, 2 H), 6.69-6.82 (m, 2 H), 6.85-6.95 (m, 2 H), 6.97-7.05 (m, 1 H), 7.05-7.15 (m, 1 H), 7.15-7.21 (m, 2 H), 7.21-7.28 (m, 1 H), 7.35 (s, 1 H), 7.38 (s, 1 H).

### Example 44B 1-cyclohexyl-3-(2,6-dichlorobenzyl)imidazolidin-2-one

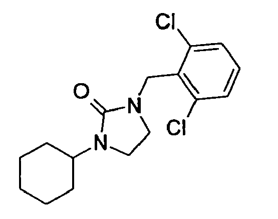

In the same manner as in Reference Example 1B, the title compound was obtained from 1-(2,6-dichlorobenzyl)imidazolidin-2-one and iodocyclohexane.
1H NMR (300 MHz, CDCl₃) δ ppm 1.04-1.08 (m, 1 H), 1.28-1.39 (m, 4 H), 1.63-1.98 (m, 5 H), 3.12-3.26 (m, 4 H), 3.73 (m, 1 H), 4.70 (s, 2 H), 7.15-7.20 (t, J=6.9 Hz, 1 H), 7.31-7.33 (m, 2 H). Example 45B 1-[2-chloro-4-(trifluoromethyl)benzyl]-3-cyclohexylimidazolidin-2-one

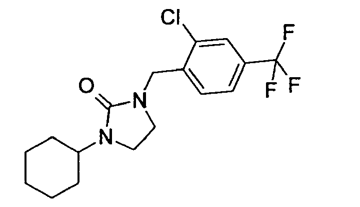

In the same manner as in Reference Example 1B, the title compound was obtained from 1-cyclohexylimidazolidin-2-one and 2-chloro-4-(trifluoromethyl)benzyl chloride.
1H NMR (300 MHz, CDCl₃) δ ppm 1.05-1.10 (m, 1 H), 1.32-1.47 (m, 4 H), 1.65-1.79 (m, 5 H), 3.23-3.36 (m, 4 H), 3.71-3.79 (m, 1 H), 4.53 (s, 2 H), 7.50 (m, 2 H), 7.63 (m, 1 H).

### Example 46B 1-(2-chloro-4-methoxybenzyl)-3-cyclohexylimidazolidin-2-one

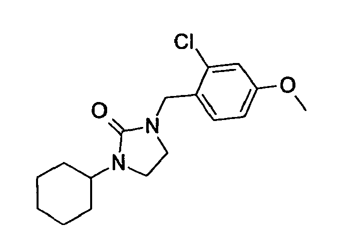

In the same manner as in Reference Example 1B, the title compound was obtained from 1-cyclohexylimidazolidin-2-one and 2-chloro-4-methoxybenzyl bromide.
1H NMR (300 MHz, CDCl₃) δ ppm 1.04-1.07 (m, 1 H), 1.25-1.40 (m, 4 H), 1.63-1.79 (m, 5 H), 3.16-3.28 (m, 3 H), 3.70-3.78 (m, 2 H), 3.78 (s, 3 H), 4.44 (s, 2 H), 6.76-6.82 (m, 1 H), 6.89-6.92 (m, 1 H), 7.27-7.30 (m, 1 H).

### Example 47B 1-(2,6-dichlorobenzyl)-3-(2,3-dihydro-1H-inden-1-yl)imidazolidin-2-one

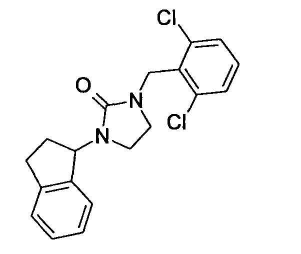

In the same manner as in Reference Example 1B, the title compound was obtained from 1-(2,3-dihydro-1H-inden-1-yl)imidazolidin-2-one obtained in Reference Example 4B and α,2,6-trichlorotoluene.
1H NMR (300 MHz, CDCl₃) δ ppm 1.89-2.01 (m, 1 H), 2.33-2.44 (m, 1 H), 2.82-3.00 (m, 3 H), 3.05-3.18 (m, 3 H), 4.78 (s, 2 H), 5.65 (t, J=7.8 Hz, 1 H), 7.15-7.26 (m, 5 H), 7.32-7.34 (m, 2 H). Example 48B 1-[2-chloro-4-(trifluoromethyl)benzyl]-3-(2,3-dihydro-1H-inden-1-yl)imidazolidin-2-one

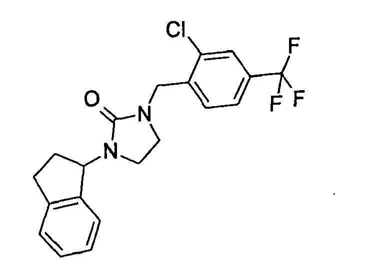

In the same manner as in Reference Example 1B, the title compound was obtained from 1-(2,3-dihydro-1H-inden-1-yl)imidazolidin-2-one obtained in Reference Example 4B and 2-chloro-4-(trifluoromethyl)benzyl chloride.
1H NMR (300 MHz, CDCl₃) δ ppm 1.93-2.01 (m, 1H), 2.31-2.50 (m, 1 H), 2.93-3.07 (m, 3 H), 3.15-3.33 (m, 3 H), 4.61 (s, 2 H), 5.61-5.69 (t, J=7.6 Hz, 1 H), 7.20-7.27 (m, 5 H), 7.53 (m, 1 H), 7.65 (m, 1 H).

### Example 49B 1-[(1R,2R,4R)-bicyclo[2.2.1]hept-2-yl]-3-(2,4-dichlorobenzyl)imidazolidin-2-one

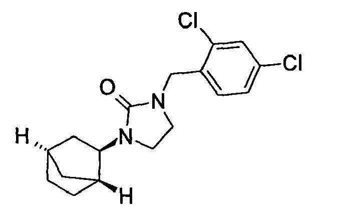

In the same manner as in Reference Example 1B, the title compound was obtained from 1-[(1R,2R,4R)-bicyclo[2.2.1]hept-2-yl]imidazolidin-2-one obtained in Reference Example 5B and α,2,4-trichlorotoluene.
1H NMR (300 MHz, CDCl₃) δ ppm 1.16-1.21 (m, 2 H), 1.29-1.53 (m, 4 H), 1.65-1.73 (m, 1 H), 2.23 (m, 1 H), 2.28-2.30 (m, 2 H), 3.17-3.38 (m, 4 H), 3.84-3.88 (m, 1 H), 4.38-4.51 (m, 2 H), 7.20-7.23 (m, 1 H), 7.29-7.37 (m, 2 H).

### Example 50B 1-(2,6-dichlorobenzyl)-3-(piperidin-1-yl)imidazolidin-2-one

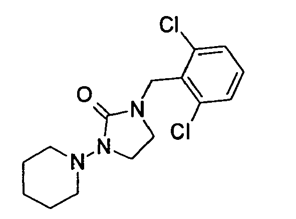

In the same manner as in Reference Example 1B, the title compound was obtained from 1-(piperidin-1-yl)imidazolidin-2-one and α,2,6-trichlorotoluene.
1H NMR (300 MHz, CDCl₃) δ ppm 1.37-1.43 (m, 2 H), 1.64-1.71 (m, 4 H), 2.93-2.97 (m, 4 H), 3.07-3.12 (m, 2 H), 3.29-3.34 (m, 2 H), 4.68 (s, 2 H), 7.17-7.22 (m, 1 H), 7.31-7.34 (m, 2 H).

### Example 51B 1-[2-chloro-4-(trifluoromethyl)benzyl]-3-(piperidin-1-yl)imidazolidin-2-one

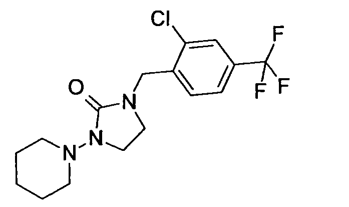

In the same manner as in Reference Example 1B, the title compound was obtained from 1-(piperidin-1-yl)imidazolidin-2-one and 2-chloro-4-(trifluoromethyl)benzyl chloride.
1H NMR (300 MHz, CDCl₃) δ ppm 1.39-1.44 (m, 2 H), 1.67-1.74 (m, 4 H), 2.91-2.96 (m, 4 H), 3.21-3.27 (m, 2 H), 3.40-3.47 (m, 2 H), 4.52 (d, J=4.5 Hz, 2 H), 7.46-7.54 (m, 1 H), 7.61-7.63 (m, 2 H). Example 52B 1-(2,6-dichlorobenzyl)-3-(morpholin-4-yl)imidazolidin-2-one

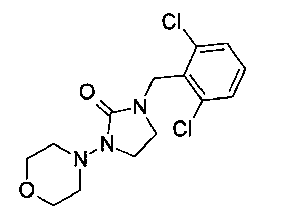

In the same manner as in Reference Example 1B, the title compound was obtained from 1-(morpholin-4-yl)imidazolidin-2-oneobtained in Reference Example 3B and α,2,6-trichlorotoluene.
1H NMR (300 MHz, CDCl₃)δ ppm 2.99-3.02 (t, J=4.8 Hz, 2 H), 3.12-3.14 (m, 2 H), 3.31-3.36 (m, 2 H), 3.77-3.81 (t, J=4.8 Hz, 2 H), 4.70 (s, 2 H), 7.18-7.23 (m, 1 H), 7.32-7.34 (m, 2 H).

### Example 53B 1-[2-chloro-4-(trifluoromethyl)benzyl]-3-(morpholin-4-yl)imidazolidin-2-one

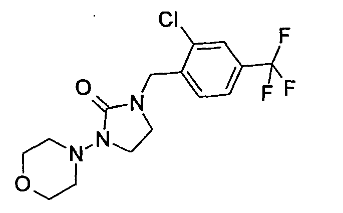

In the same manner as in Reference Example 1B, the title compound was obtained from 1-(morpholin-4-yl)imidazolidin-2-oneobtained in Reference Example 3B and 2-chloro-4-(trifluoromethyl)benzyl chloride.
1H NMR (300 MHz, CDCl₃) δ ppm 3.00-3.03 (m, 4 H), 3.24-3.29 (m, 2 H), 3.41-3.46 (m, 2 H), 3.80-3.83 (m, 4 H), 4.54 (m, 2 H), 7.50 (m, 2 H), 7.64 (s, 1 H).

### Example 54B 1-(2,6-dichlorobenzyl)-3-(2,3-dihydro-1H-indol-1-yl)imidazolidin-2-one

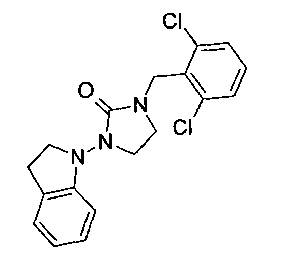

In the same manner as in Reference Example 1B, the title compound was obtained from 1-(2,3-dihydro-1H-indol-1-yl)imidazolidin-2-one obtained in Reference Example 6B and α,2,6-trichlorotoluene.
1H NMR (300 MHz, CDCl₃)δ ppm 2.97-3.02 (t, J=8 .1 Hz, 2 H), 3.15-3.21 (m, 2 H), 3.30-3.35 (m, 2 H), 3.55 (m, 2 H), 4.74 (s, 2 H), 6.58-6.61 (m, 1 H), 6.74-6.79 (m, 1 H), 7.03-7.07 (m, 2 H), 7.16-7.22 (m, 1 H), 7.31-7.34 (m, 2 H).

### Example 55B 1-[2-chloro-4-(trifluoromethyl)benzyl]-3-(2,3-dihydro-1H-indol-1-yl)imidazolidin-2-one

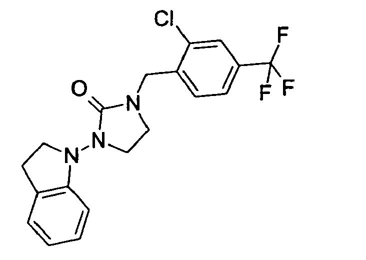

In the same manner as in Reference Example 1B, the title compound was obtained from 1-(2,3-dihydro-1H-indol-1-yl)imidazolidin-2-one obtained in Reference Example 6B and 2-chloro-4-(trifluoromethyl)benzyl chloride.
1H NMR (300 MHz, CDCl₃)δ ppm 1.79-1.90 (m, 1 H), 2.12-2.21 (m, 1 H), 2.88-3.09 (m, 4 H), 3.27-3.60 (m, 5 H), 6.38-6.41 (d, J=7.5 Hz, 1 H), 6.82-6.87 (m, 1 H), 7.05-7.14 (m, 2 H), 7.48 (s, 2 H), 7.65 (s, 1 H).

### Example 56B 1-(2,4-dichlorobenzyl)-3-(1,4-dioxaspiro[4.5]dec-8-yl)imidazolidin-2-one

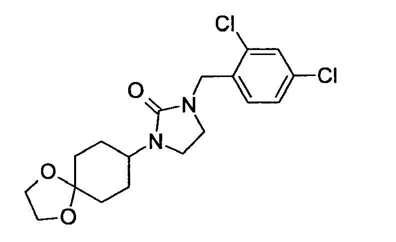

To a solution of 1,4-dioxaspiro[4.5]decan-8-amine (7.22 g) and triethylamine (5.16 mL) in tetrahydrofuran (150 mL) was added dropwise 1-chloro-2-isocyanatoethane (4.00 g) at room temperature, and the mixture was stirred at room temperature for 30 min. Then, potassium tert-butoxide (8.75 g) was added to the reaction mixture, and the mixture was stirred at room temperature for 30 min. Water was added to the reaction mixture, and the mixture was extracted with ethyl acetate. The ethyl acetate layer was washed successively with water and saturated brine, and dried over anhydrous magnesium sulfate, and the solvent was evaporated under reduced pressure. To a mixture of the residue, α,2,4-trichlorotoluene (2.93 g) and N,N-dimethylformamide (50 mL) was added 60% sodium hydride (0.72 g), and the mixture was stirred at room temperature for 1 hr. Water was added to the reaction solution, and the mixture was extracted with ethyl acetate. The ethyl acetate layer was washed successively with water and saturated brine, and dried over anhydrous magnesium sulfate, and the solvent was evaporated under reduced pressure. The residue was subjected to silica gel column chromatography to give the title compound (1.1 g, 20%) as a colorless solid from a fraction eluted with ethyl acetate. 1H NMR (300 MHz, CDCl₃) δ ppm 1.66-1.82 (m, 8 H), 3.20-3.35 (m, 4 H), 3. 84 (m, 1 H), 3. 94 (s, 2 H), 3. 94 (s, 2 H), 4. 46 (s, 2 H), 7.19-7.24 (dd, J=8.0, 1.8 Hz, 1 H), 7.29 (m, 1 H), 7.36 (m, 1 H).

### Example 57B 1-(2,4-dichlorobenzyl)-3-(4-oxocyclohexyl)imidazolidin-2-one

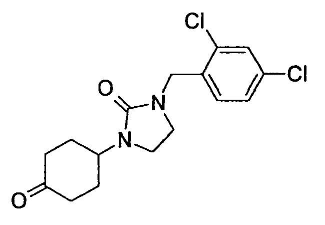

A mixture of 1-(2,4-dichlorobenzyl)-3-(1,4-dioxaspiro[4.5]dec-8-yl)imidazolidin-2-one (1.0 g), 2N hydrochloric acid (2.6 mL) and tetrahydrofuran (70 mL) was stirred at 60°C for 3 hr. Saturated aqueous sodium hydrogencarbonate was added to the reaction solution, and the mixture was extracted with ethyl acetate. The ethyl acetate layer was washed with saturated brine, and dried over anhydrous magnesium sulfate. The solvent was evaporated under reduced pressure, and the residue was triturated with hexane-ethyl acetate to give the title compound (0.46 g, 52%) as a colorless powder.
1H NMR (300 MHz, CDCl₃) δ ppm 1.81-1.94 (m, 2 H), 2.07-2.10 (m, 2 H), 2.42-2.59 (m, 4 H), 3.28-3.29 (m, 4 H), 4.24-4.32 (m, 1 H), 4.49 (s, 2 H), 7.21-7.25 (dd, J=8.4, 1.8 Hz, 1 H), 7.30-7.33 (d, J=8.4 Hz, 1 H), 7.38-7.39 (d, J=1.8 Hz, 1 H).

### Example 58B 1-(2,4-dichlorobenzyl)-3-(4-hydroxy-4-methylcyclohexyl)imidazolidin-2-one

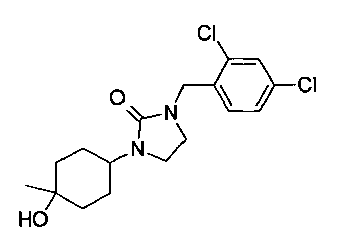

A solution of 1-(2,4-dichlorobenzyl)-3-(4-oxocyclohexyl)imidazolidin-2-one obtained in Example 57B (0.41 g) in tetrahydrofuran (10 mL) was cooled to 0°C, and methyllithium (1.0 M diethyl ether solution, 2.3 mL) was added dropwise thereto. The reaction mixture was stirred at room temperature for 2 hr, water was added, and the mixture was extracted with ethyl acetate. The organic layer was washed with saturated brine, and dried over magnesium sulfate, and the solvent was evaporated under reduced pressure. The obtained residue was subjected to silica gel column chromatography (hexane-ethyl acetate 9:1-1:2) to give the title compound (0.026 g, 6%) as a colorless solid.
1H NMR (300 MHz, CDCl₃) δ ppm 1.27 (s, 3 H), 1.47-1.79 (m, 8 H), 2.44 (br, 1 H), 3.21-3.33 (m, 4 H), 3.74-3.81 (m, 1 H), 4.46 (s, 2 H), 7.20-7.24 (dd, J=8.1, 2.4 Hz, 1 H), 7.27-7.32 (m, 1 H), 7.37-7.38 (d, J=2.4 Hz, 1 H).

### Example 59B 1-[1-(1-adamantyl)ethyl]-3-(2,4-dichlorobenzyl)imidazolidin-2-one

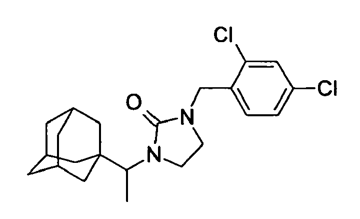

In the same manner as in Reference Example 1B, the title compound was obtained from 1-[1-(1-adamantyl)ethyl]imidazolidin-2-one and α,2,4-trichlorotoluene.
1H NMR (300 MHz, CDCl₃) δ ppm 1.06-1.09 (d, J=7.2 Hz, 3 H), 1.57-1. 72 (m, 13 H), 1.98 (m, 3 H), 3.19-3.24 (m, 2 H), 3.37-3.43 (m, 2 H), 3.62-3.69 (q, J=7.5 Hz, 1 H), 4.39-4.55 (q, J=15.6 Hz, 2 H), 7.20-7.23 (m, 1 H), 7.31-7.37 (m, 2 H).

### Example 60B 1-(2,6-dichlorobenzyl)-3-(1,4-dioxaspiro[4.5]dec-8-yl)imidazolidin-2-one

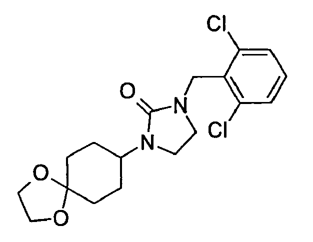

In the same manner as in Example 56B, the title compound was obtained from 1,4-dioxaspiro[4.5]decan-8-amine, 1-chloro-2-isocyanatoethane, and α,2,6-trichlorotoluene.
1H NMR (300 MHz, CDCl₃) δ ppm 1.68-1.80 (m, 8 H), 3.12-3.18 (m, 2 H), 3.20-3.26 (m, 2 H), 3.88-3.91 (m, 1 H), 3.94 (s, 4 H), 4.70 (s, 2 H), 7.15-7.20 (m, 1 H), 7.29-7.33 (m, 2 H).

### Example 61B 1-(2,6-dichlorobenzyl)-3-(4-oxocyclohexyl) imidazolidin-2-one

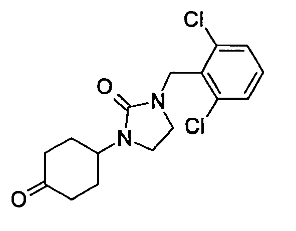

In the same manner as in Example 57B, the title compound was obtained from 1-(2,6-dichlorobenzyl)-3-(1,4-dioxaspiro[4.5]dec-8-yl)imidazolidin-2-one obtained in Example 60B.
1H NMR (300 MHz, CDCl₃) δ ppm 1.80-1.95 (m, 4 H), 2.40-2.62 (m, 4 H), 3.15-3.20 (m, 4 H), 4.23-4.34 (m, 1 H), 4.73 (s, 2 H), 7.15-7.35 (m, 3 H).

### Example 62B 1-(2,6-dichlorobenzyl)-3-(4-hydroxy-4-methylcyclohexyl)imidazolidin-2-one

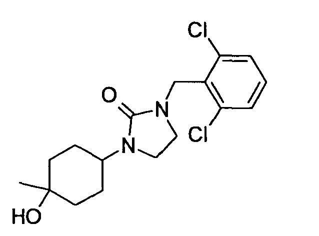

In the same manner as in Example 58B, the title compound was obtained from 1-(2,6-dichlorobenzyl)-3-(4-oxocyclohexyl)imidazolidin-2-one obtained in Example 61B.
1H NMR (300 MHz, CDCl₃) δ ppm 1.26 (s, 3 H), 1.43-1.78 (m, 8 H), 2.20 (br, 1 H), 3.10-3.29 (m, 4 H), 3.72-3.84 (m, 1 H), 4.70 (s, 2 H), 7.14-7.22 (m, 1 H), 7.31-7.35 (m, 2 H).

### Example 1C 1-benzyl-3-(2,6-dichlorobenzyl)piperidin-2-one

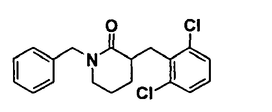

A mixture of 3-(2,6-dichlorobenzyl)piperidin-2-one obtained in Reference Example 2C (258 mg), 60% sodium hydride (80 mg) and N,N-dimethylformamide (5 mL) was stirred at room temperature for 30 min, benzyl bromide (257 mg) was added, and the mixture was further stirred at room temperature for 16 hr. The reaction mixture was poured into water, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 9:1) to give the title compound (270 mg, 78%) as a colorless powder.
1H NMR (300 MHz, CDCl₃) δ ppm 1.60-1.70 (m, 3 H),1.85-1.92 (m, 1 H), 2.85-2.95 (m, 1 H), 3.12 (dd, J=13.4, 11.2 Hz, 1 H), 3.20-3.28 (m, 2 H), 3.79 (dd, J=13.4, 4.4 Hz, 1 H), 4.51 (d, J=14.8 Hz, 1 H), 4.74 (d, J=14.8 Hz, 1 H), 7.05-7.12 (m, 1 H), 7.25-7.36 (m, 7 H).

### Example 2C 1-(2-chlorobenzyl)-3-(2,6-dichlorobenzyl)piperidin-2-one

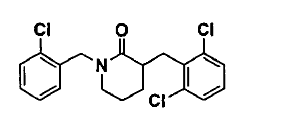

In the same manner as in Example 1C, the title compound was obtained from 3-(2,6-dichlorobenzyl)piperidin-2-one obtained in Reference Example 2C and 2-chlorobenzyl bromide.
1H NMR (300 MHz, CDCl₃) δ ppm 1.59-1.80 (m, 3 H), 1.84-2.00 (m, 1 H), 2.84-3.02 (m, 1 H), 3.13 (dd, J=13.6, 11.3 Hz, 1 H), 3.20-3. 39 (m, 2 H), 3. 78 (dd, J=13.6, 4 . 5 Hz, 1 H), 4 . 61-4 . 77 (m, 1 H), 4.77-4.94 (m, 1 H), 7.05-7.15 (m, 1 H), 7.16-7.42 (m, 6 H). Example 3C 3-(2,6-dichlorobenzyl)-1-(4-methylbenzyl)piperidin-2-one

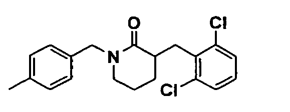

In the same manner as in Example 1C, the title compound was obtained from 3-(2,6-dichlorobenzyl)piperidin-2-one obtained in Reference Example 2C and a-bromo-p-xylene.
1H NMR (300 MHz, CDCl₃) δ ppm 1.52-1.74 (m, 3 H), 1.77-1.94 (m, 1 H), 2.34 (s, 3 H), 2.77-2.99 (m, 1 H), 3.03-3.18 (m, 1 H), 3.17-3.37 (m, 2 H), 3.78 (dd, J=13.6, 4.3 Hz, 1 H), 4.46 (d, J=14.5 Hz, 1 H), 4.70 (d, J=14.5 Hz, 1 H), 7.03-7.23 (m, 5 H), 7.24-7.38 (m, 2 H).

### Example 4C 3-(2,6-dichlorobenzyl)-1-(3-methylbenzyl)piperidin-2-one

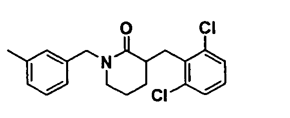

In the same manner as in Example 1C, the title compound was obtained from 3-(2,6-dichlorobenzyl)piperidin-2-one obtained in Reference Example 2C and α-bromo-m-xylene.
1H NMR (300 MHz, CDCl₃) δ ppm 1.49-1.76 (m, 3 H), 1.79-1.98 (m, 1 H), 2.35 (s, 3 H), 2.78-3.01 (m, 1 H), 3.12 (dd, J=13.6, 11.3 Hz, 1 H), 3.18-3.32 (m, 2 H), 3.79 (dd, J=13.6, 4.5 Hz, 1 H), 4.46 (d, J=14.5 Hz, 1 H), 4.72 (d, J=14.5 Hz, 1 H), 7.01-7.16 (m, 4 H), 7.16-7.38 (m, 3 H).

### Example 5C 3-(2,6-dichlorobenzyl)-1-(2-methylbenzyl)piperidin-2-one

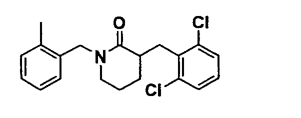

In the same manner as in Example 1C, the title compound was obtained from 3-(2,6-dichlorobenzyl)piperidin-2-one obtained in Reference Example 2C and.α-bromo-o-xylene.
1H NMR (300 MHz, CDCl₃) δ ppm 0.74-0.98 (m, 1 H), 1.60-1.76 (m, 3 H), 1.79-1.97 (m, 1 H), 2.31 (s, 3 H), 2.83-3.00 (m, 1 H), 3.11-3.23 (m, 2 H), 3.79 (dd, J=13.6, 4.3 Hz, 1 H), 4.59 (d, J=15.0 Hz, 1 H), 4.59 (d, J=15.0 Hz, 1 H), 7.02-7.23 (m, 5 H), 7.27-7.38 (m, 2 H).

### Example 6C 1-(1-adamantyl)-3-(2,6-dichlorobenzyl)piperidin-2-one

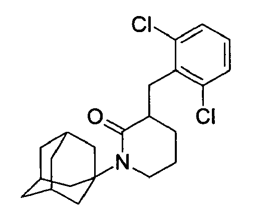

To a solution of 1-(1-adamantyl)piperidin-2-one (0.55 g) in tetrahydrofuran (10 mL) was added lithium diisopropylamide (1.8 M tetrahydrofuran solution, 1.31 mL) at -78°C under nitrogen atmosphere, and the mixture was stirred for 10 min. A solution of α,2,6-trichlorotoluene (0.46 g) in tetrahydrofuran (2 mL) was added to the obtained solution at the same temperature, the mixture was further stirred at -78°C for 10 min, and the temperature was gradually raised to room temperature. Water was added to the reaction mixture, and the mixture was extracted with ethyl acetate. The ethyl acetate layer was washed with saturated brine, and dried over anhydrous magnesium sulfate. The solvent was evaporated under reduced pressure, and the residue was subjected to silica gel column chromatography (hexane-ethyl acetate 19:1-4:1) to give the title compound (0.14 g, 15%) as a colorless solid.
1H NMR (300 MHz, CDCl₃) δ ppm 1.48-1.63 (m, 2 H), 1.67-1.85 (m, 8 H), 2.10 (br, 3 H), 2.24 (br, 6 H), 2.27-2.78 (m, 1 H), 3.06 (t, J=13.5 Hz, 1 H), 3.10-3.39 (m, 2 H), 3.58-3.64 (dd, J=13.5, 3.9 Hz, 1 H), 7.04-7.09 (m, 1 H), 7.25-7.28 (m, 2 H).

### Example 7C 1-(1-adamantyl)-3-(2,6-dichlorobenzyl)azepan-2-one

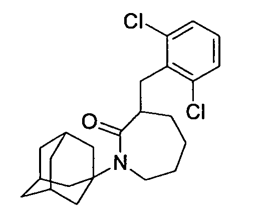

In the same manner as in Example 6C, the title compound was obtained from 1-(1-adamantyl)azepan-2-one and α, 2, 6-trichlorotoluene.
1H NMR (300 MHz, CDCl₃) δ ppm 1.32-1.42 (m, 2 H), 1.49-1.54 (m, 2 H), 1.65-1.76 (m, 8 H), 2.12 (br, 3 H), 2.24 (br, 6 H), 3.11-3.33 (m, 4 H), 3.62 -3.69 (m, 1 H), 7.08 (t, J=7.8 Hz, 1 H), 7.27-7.33 (m, 2 H).

### Example 1D 1,3-bis(2-methylbenzyl)tetrahydropyrimidin-2(1H)-one

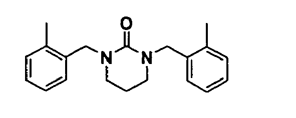

In the same manner as in Reference Example 1D, the title compound was obtained from tetrahydro-2-pyrimidinone and α-bromo-o-xylene.
1H NMR (300 MHz, CDCl₃) δ ppm 1.81-1.98 (m, 2 H), 2.33 (s, 6 H), 3.04-3.25 (m, 4 H), 4.66 (s, 4 H), 7.08-7.24 (m, 8 H).

### Example 2D 1-(2,6-dichlorobenzyl)-3-(2-methylbenzyl)tetrahydropyrimidin-2(1H)-one

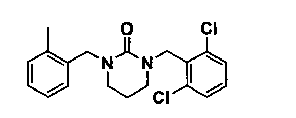

In the same manner as in Reference Example 1D, the title compound was obtained from 1-(2-methylbenzyl)tetrahydropyrimidin-2(1H)-one obtained in Reference Example 1D and α,2,6-trichlorotoluene.
1H NMR (300 MHz, CDCl₃) δ ppm 1.77-1.92 (m, 2 H), 2.32 (s, 3 H), 2.99-3.15 (m, 4 H), 4.65 (s, 2 H), 5.03 (s, 2 H), 7.12-7.22 (m, 5 H), 7.30-7.36 (m, 2 H).

### Experimental Example 1

### Determination of 11βHSD1 inhibitory activity

The enzyme reaction was performed in a polypropylene 96-well plate (Corning). The microsome fraction obtained in Reference Example 2a was diluted with a buffer (30 mM Tris-HCl, 1 mM EDTA, 0.1% BSA, pH 7.2) to a concentration of 100 µg/ml. A solution (2.5 µl) of the test compound in dimethyl sulfoxide was added to the obtained diluted solution (22.5 µl) and the mixture was incubated at room temperature for 5 min. A substrate solution (500 nM cortisone, 400 µM NADPH) (25 µl) was added to the obtained suspension, allowed to react at room temperature for 60 min and the reaction was quenched by the addition of 2 mM 18-β-glycyrrhetinic acid (Sigma Ltd.) solution (5 µl). The produced cortisole was quantified by the following competitive ELISA.

Anti-mouse IgG antibody (Chemicon) was suspended in 100 mM sodium phosphate buffer (pH 9.0) to a concentration of 5 µg/ml.

The obtained suspension (100 µl) was added to a 96-well black plate (Maxisorp, Nunc), and incubated overnight at 4°C to immobilize a first antibody. The plate was washed with PBS containing 0.05% Tween20 (Bio-Rad), 30% block ace (Yukijirushi) (300 µl) diluted with PBS was added. After blocking at room temperature for 1 hr or more, the plate was washed with PBS containing 0.05% Tween20. Anti-cortisole antibody (assay design) (100 µl) diluted 4-fold with a buffer (30 mM Tris-HCl, 1 mM EDTA, pH 7.2) was added to the plate, and the plate was incubated at room temperature for 90 min. The plate was washed 4 times with PBS containing 0.05% Tween20, the enzyme reaction mixture (25 µl) and alkaline phosphatase conjugated cortisole (assay design) (25 µl) were added, and the plate was incubated at room temperature for 120 min. The plate was washed 4 times with PBS containing 0.05% Tween20, a luminescence substrate for alkaline phosphatase, Immun star (Enhancer plus, Bio-Rad) (50 µl) was added. The plate was incubated at room temperature for 10 min and the level of luminescence was measured by Arvo malti label counter (Wallac). The concentration (IC₅₀ value) of the test compound necessary for inhibiting the 11βHSD1 activity by 50% was calculated by PRISM2.01 (Graphpad Software). The results are shown in Table 1.

**[Table 1]**

| test compound | IC₅₀ value |
|---|---|
| (Example No.) | (nM) |
| 9A | 7.9 |
| 38A | 26 |
| 66A | 56 |
| 94A | 85 |
| 412A | 430 |
| 413A | 29 |
| 468A | 19 |
| 470A | 37 |
| 510A | 9.9 |
| 602A | 26 |
| 603A | 98 |
| 619A | 46 |
| 654A | 150 |
| 32B | 53 |
| 5C | 110 |
| 2D | 130 |

| Formulation Example 1 (production of capsules) | |
|---|---|
| 1) compound of Example 1A | 30 mg |
| 2) fine cellulose powder | 10 mg |
| 3) lactose | 19 mg |
| 4) magnesium stearate | 1 mg |
| total | 60 mg |

| Formulation Example 2 (production of tablets) | |
|---|---|
| 1) compound of Example 1A | 30 g |
| 2) lactose | 50 g |
| 3) corn starch | 15 g |
| 4) carboxymethylcellulose calcium | 44 g |
| 5) magnesium stearate | 1 g |
| total of 1000 tablets | 140 g |

| | |
|---|---|
| 1), 2), 3) and 4) are mixed and filled in gelatin capsules. | |

The entire amounts of 1), 2) and 3), and 30 g of 4) are kneaded with water, dried in vacuo and granulated.

The granules are mixed with 14 g of 4) and 1 g of 5) and the mixture is compressed with a tableting machine, whereby 1000 tablets containing 30 mg of compound of Example 1A per tablet are obtained.

### Industrial Applicability

The 11β-hydroxysteroid dehydrogenase 1 inhibitor of the present invention has a superior activity, and is useful as a pharmaceutical agent such as agents for the prophylaxis or treatment of diabetes, insulin resistance, obesity, abnormal lipid metabolism, hypertension and the like, and the like.

This application is based on patent application Nos. 2005-102913 and 2005-306397 filed in Japan, the contents of which are incorporated in full herein by this reference.

## Claims

1. A 11β-hydroxysteroid dehydrogenase 1 inhibitor comprising a compound represented by the formula (1): 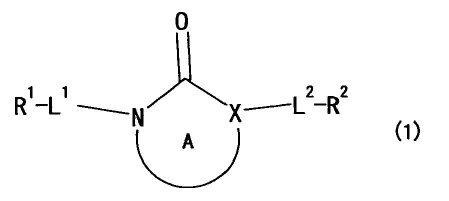 wherein
R¹ is an optionally substituted cyclic group;
R² is a hydrogen atom or an optionally substituted cyclic group;
X is N or CR³ (R³ is a hydrogen atom or a substituent);
L¹ and L² are the same or different and each is a bond, an optionally substituted divalent aliphatic hydrocarbon group, or a group represented by the formula : - (akn¹)ₘY-(akn²)ₙ- (akn¹ and akn² are the same or different and each is an optionally substituted C₁₋₆ alkylene group; m and n are the same or different and each is 0 or 1; and Y is -O-, -S-, -SO-, -SO₂-, -NR⁴-, -SO₂NR⁴- or -NR⁴SO₂- (R⁴ is a hydrogen atom or an optionally substituted C₁₋₆ alkyl group); and
ring A is an optionally substituted 4- to 7-membered nitrogen-containing non-aromatic heterocycle wherein the non-aromatic heterocycle is optionally condensed with an optionally substituted ring,
or a salt thereof, or a prodrug thereof.

2. The agent of claim 1, wherein the cyclic group for R¹ is a C₃₋₆ cycloalkyl group.

3. Use of a compound represented by the formula (1): 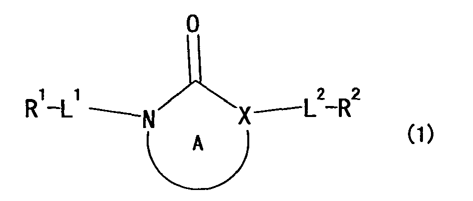 wherein each symbol is as defined in claim 1,
or a salt thereof, or a prodrug thereof, for the production of a 11β-hydroxysteroid dehydrogenase 1 inhibitor.

4. A method of inhibiting 11β-hydroxysteroid dehydrogenase 1 in a mammal, which comprises administering a compound represented by the formula (1): 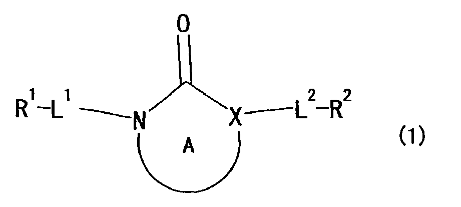 wherein each symbol is as defined in claim 1,
or a salt thereof, or a prodrug thereof, to the mammal.

5. A compound represented by the formula (2a): 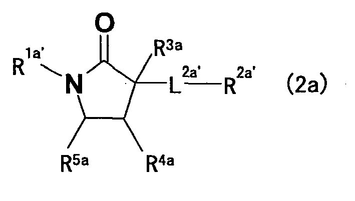 wherein
R^{1a'} is an optionally substituted non-aromatic cyclic group provided that the non-aromatic cyclic group should be selected from a C₃₋₆ cycloalkyl group optionally condensed with a benzene ring, a 6-membered non-aromatic heterocyclic group, a 5- or 6-membered non-aromatic heterocyclic group condensed with a benzene ring, and a C₇₋₁₀ cross-linked hydrocarbon group; and the non-aromatic cyclic group should not have, as a substituent, a group represented by R'-E- (E is -S(O)ₜ-CHR^{e}-, -CHR^{e}NR^{e}-, -C(O)-NR^{e}-, -NR^{e}-C(O)NR^{e}-, -SO₂-NR^{e}- or -NR^{e}-SO₂NR^{e}-(t is an integer of 0 to 2; and R^{e} is a hydrogen atom or a C₁₋₃ alkyl group); and R' is an optionally substituted C₆₋₁₀ aryl group or an optionally substituted 5- to 10-membered aromatic heterocyclic group);
R^{2a'} is an optionally substituted cyclic group (provided that the cyclic group should not be a non-aromatic heterocyclic group); L^{2a'} is a C₁₋₆ alkylene group;
R^{3a} is a hydrogen atom, an optionally substituted hydroxy group, an optionally substituted mercapto group, an optionally substituted amino group or an acyl group;
R^{4a} and R^{5a} are the same or different and each is a hydrogen atom or a substituent,
or a salt thereof.

6. The compound of claim 5, wherein the non-aromatic cyclic group for R^{1a'} is a C₃₋₆ cycloalkyl group.

7. The compound of claim 5, wherein R^{1a'} is a C₃₋₆ cycloalkyl group optionally condensed with a benzene ring, a 6-membered non-aromatic heterocyclic group, a 5- or 6-membered non-aromatic heterocyclic group condensed with a benzene ring, or a C₇₋₁₀ cross-linked hydrocarbon group, each of which is optionally substituted by 1 to 5 substituents selected from
(1) a halogen atom;
(2) a hydroxy group;
(3) a cyano group;
(4) a nitro group;
(5) a carboxyl group;
(6) a carbamoyl group;
(7) an oxo group;
(8) a C₁₋₃ alkylenedioxy group;
(9) a C₁₋₆ alkyl group optionally substituted by 1 to 3 substituents selected from the following (i) to (vii)
(i) a halogen atom,
(ii) a carboxyl group,
(iii) a hydroxy group,
(iv) a C₁₋₆ alkoxy-carbonyl group,
(v) a carbamoyl group optionally mono- or di-substituted by substituent (s) selected from a C₆₋₁₄ aryl group and a C₇₋₁₃ aralkyl group,
(vi) an aromatic heterocyclic group optionally substituted by C₆₋₁₄ aryl group (s) optionally substituted by 1 to 3 halogen atoms, and
(vii) a non-aromatic heterocyclyl-carbonyl group;
(10) a C₂₋₆ alkenyl group optionally substituted by 1 to 3 substituents selected from a carboxyl group and a C₁₋₆ alkoxy-carbonyl group;
(11) a C₂₋₆ alkynyl group;
(12) a C₆₋₁₄ aryl group optionally substituted by 1 to 3 halogen atoms;
(13) a C₇₋₁₃ aralkyl group;
(14) a C₁₋₆ alkoxy group optionally substituted by 1 to 3 halogen atoms;
(15) an amino group optionally mono- or di-substituted by C₁₋₆ alkyl group(s) ;
(16) a C₁₋₆ alkyl-carbonyl group;
(17) a C₁₋₆ alkoxy-carbonyl group;
(18) a C₇₋₁₃ aralkyloxy-carbonyl group;
(19) a C₁₋₆ alkylsulfinyl group;
(20) a C₁₋₆ alkylsulfonyl group;
(21) a non-aromatic heterocyclic group; and
(22) a formyl group.

8. The compound of claim 5, wherein the cyclic group for R^{2a'} is a C₆₋₁₄ aryl group.

9. The compound of claim 5, wherein the non-aromatic cyclic group for R^{1a'} is a C₇₋₁₀ cross-linked hydrocarbon group, and R^{2a'} is a phenyl group having a substituent at the para-position.

10. The compound of claim 9, wherein the substituent which the phenyl group has at the para-position,
(1) a nitro group;
(2) a carboxyl group;
(3) a C₁₋₃ alkylenedioxy group;
(4) a C₁₋₆ alkyl group substituted by 1 to 3 substituents selected from the following (i) to (viii)
(i) a carboxyl group,
(ii) a hydroxy group,
(iii) a C₁₋₆ alkoxy-carbonyl group,
(iv) a carbamoyl group optionally mono- or di-substituted by substituent (s) selected from a C₆₋₁₄ aryl group and a C₇₋₁₃ aralkyl group,
(v) an amino group optionally mono- or di-substituted by C₁₋₆ alkyl-carbonyl group(s),
(vi) an aromatic heterocyclic group optionally substituted by C₆₋₁₄ aryl group(s) optionally substituted by 1 to 3 halogen atoms,
(vii) a non-aromatic heterocyclic group, and
(viii) a non-aromatic heterocyclyl-carbonyl group optionally substituted by 1 to 3 C₁₋₆ alkoxy-carbonyl groups;
(5) a C₂₋₆ alkenyl group optionally substituted by 1 to 3 substituents selected from a carboxyl group and a C₁₋₆ alkoxy-carbonyl group;
(6) a C₂₋₆ alkynyl group optionally substituted by 1 to 3 hydroxy groups;
(7) a C₆₋₁₄ aryl group optionally substituted by 1 to 3 substituents selected from the following (i) to (ix)
(i) a C₁₋₆ alkyl group optionally substituted by 1 to 3 substituents selected from a hydroxy group and a halogen atom,
(ii) a formyl group,
(iii) a cyano group,
(iv) a carboxyl group,
(v) a carbamoyl group,
(vi) a halogen atom,
(vii) a C₁₋₆ alkyl-carbonyl group,
(viii) a C₁₋₆ alkylsulfonyl group, and
(ix) an amino group optionally mono- or di-substituted by substituent(s) selected from a C₁₋₆ alkyl-carbonyl group and a C₁₋₆ alkylsulfonyl group;
(8) a C₇₋₁₃ aralkyl group;
(9) an aromatic heterocyclic group optionally substituted by 1 to 3 substituents selected from the following (i) to (v)
(i) a C₁₋₆ alkyl group optionally substituted by 1 to 3 hydroxy groups,
(ii) a carboxyl group,
(iii) a C₃₋₁₀ cycloalkyl group,
(iv) a halogen atom, and
(v) a formyl group;
(10) a non-aromatic heterocyclic group optionally substituted by 1 to 3 substituents selected from the following (i) to (iii)
(i) a C₁₋₆ alkyl group optionally substituted by 1 to 3 substituents selected from
(a) a hydroxy group,
(b) a C₆₋₁₄ aryl group optionally substituted by 1 to 3 substituents selected from a C₁₋₆ alkyl group optionally substituted by 1 to 3 halogen atoms and a halogen atom, and
(c) an aromatic heterocyclic group,
(ii) an oxo group, and
(iii) a C₁₋₆ alkyl-carbonyl group;
(11) a C₁₋₆ alkoxy group substituted by 1 to 3 substituents selected from the following (i) to (xii)
(i) a cyano group,
(ii) a carboxyl group,
(iii) an amino group optionally mono- or di-substituted by substituent(s) selected from
(a) a C₁₋₆ alkyl group,
(b) a C₁₋₆ alkoxy-carbonyl group,
(c) a C₆₋₁₄ aryl-carbonyl group optionally substituted by C₁₋₆ alkyl group(s) optionally substituted by 1 to 3 halogen atoms, and
(d) a C₆₋₁₄ arylsulfonyl group,
(iv) a C₆₋₁₄ aryloxy group,
(v) a C₇₋₁₃ aralkyloxy group optionally substituted by 1 to 3 halogen atoms,
(vi) a C₁₋₆ alkyl-carbonyl group,
(vii) a C₁₋₆ alkoxy-carbonyl group,
(viii) a carbamoyl group optionally mono- or di-substituted by substituent(s) selected from
(a) a C₁₋₆ alkyl group optionally substituted by 1 to 3 substituents selected from
(a-1) a C₆₋₁₄ aryl group optionally substituted by 1 to 3 substituents selected from a C₁₋₆ alkyl group optionally substituted by 1 to 3 halogen atoms, a halogen atom and a C₁₋₆ alkoxy group,
(a-2) an aromatic heterocyclic group, and
(a-3) a C₃₋₁₀ cycloalkyl group,
(b) a C₃₋₁₀ cycloalkyl group,
(c) a C₆₋₁₄ aryl group optionally substituted by 1 to 3 substituents selected from
(c-1) a halogen atom, and
(c-2) a C₁₋₆ alkyl group optionally substituted by 1 to 3 halogen atoms, and
(d) a C₆₋₁₄ arylsulfonyl group optionally substituted by C₁₋₆ alkyl group(s) optionally substituted by 1 to 3 halogen atoms,
(ix) an aromatic heterocyclic group optionally substituted by 1 to 3 substituents selected from
(a) a C₁₋₆ alkyl group optionally substituted by 1 to 3 substituents selected from a hydroxy group and a halogen atom,
(b) a C₆₋₁₄ aryl group optionally substituted by 1 to 3 halogen atoms,
(c) a carboxyl group, and
(d) a C₁₋₆ alkoxy-carbonyl group,
(x) an aromatic heterocyclyl-oxy group optionally substituted by C₁₋₆ alkyl group(s) optionally substituted by 1 to 3 halogen atoms,
(xi) a non-aromatic heterocyclic group optionally substituted by 1 to 3 substituents selected from
(a) a C₃₋₁₀ cycloalkyl group, and
(b) an oxo group, and
(xii) a C₁₋₆ alkoxy group;
(12) a C₂₋₆ alkynyloxy group;
(13) a C₃₋₁₀ cycloalkyl-C₁₋₆ alkyloxy group;
(14) a C₁₋₆ alkylthio group;
(15) an amino group optionally mono- or di-substituted by substituent(s) selected from the following (i) to (xvii)
(i) a C₁₋₆ alkyl group optionally substituted by 1 to 3 substituents selected from a hydroxy group and a C₁₋₆ alkoxy-carbonyl group,
(ii) a C₆₋₁₄ aryl group,
(iii) a C₇₋₁₃ aralkyl group,
(iv) a C₁₋₆ alkyl-carbonyl group optionally substituted by 1 to 3 substituents selected from
(a) a hydroxy group,
(b) a carboxyl group,
(c) a C₆₋₁₄ aryl group,
(d) a C₁₋₆ alkoxy-carbonyl group,
(e) a C₁₋₆ alkyl-carbonyloxy group,
(f) a C₆₋₁₄ arylsulfonyl group,
(g) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
(h) a halogen atom, and
(i) an aromatic heterocyclic group,
(v) a C₁₋₆ alkoxy-carbonyl group,
(vi) a C₆₋₁₄ aryl-carbonyl group optionally substituted by 1 to 3 substituents selected from
(a) a C₁₋₆ alkyl group optionally substituted by 1 to 3 halogen atoms, and
(b) a sulfamoyl group,
(vii) a C₇₋₁₃ aralkyl-carbonyl group,
(viii) a C₃₋₁₀ cycloalkyl-carbonyl group optionally substituted by 1 to 3 substituents selected from
(a) a hydroxy group,
(b) a carboxyl group,
(c) a C₁₋₆ alkoxy-carbonyl group,
(d) an amino group optionally mono- or di-substituted by substituent(s) selected from a C₁₋₆ alkoxy-carbonyl group, a C₆₋₁₄ aryl-carbonyl group and a C₆₋₁₄ arylsulfonyl group,
(e) an oxo group, and
(f) a C₁₋₆ alkyl group optionally substituted by 1 to 3 hydroxy groups,
(ix) an aromatic heterocyclyl-carbonyl group optionally substituted by C₁₋₆ alkyl group(s) optionally substituted by 1 to 3 hydroxy groups,
(x) a C₁₋₆ alkylsulfonyl group optionally substituted by 1 to 3 substituents selected from a non-aromatic heterocyclic group optionally substituted by oxo group(s) and a halogen atom,
(xi) a C₆₋₁₄ arylsulfonyl group optionally substituted by 1 to 3 substituents selected from a C₁₋₆ alkyl group optionally substituted by 1 to 3 halogen atoms and a halogen atom,
(xii) a C₇₋₁₃ aralkylsulfonyl group,
(xiii) a C₃₋₁₀ cycloalkylsulfonyl group,
(xiv) an aromatic heterocyclyl-sulfonyl group optionally substituted by 1 to 3 C₁₋₆ alkyl group(s),
(xv) -CONR⁶R⁷
wherein
R⁶ and R⁷ are the same or different and each is selected
from the following (a) to (f),
(a) a hydrogen atom,
(b) a C₁₋₆ alkyl group optionally substituted by 1 to 3 substituents selected from the following (b-1) to (b-4)
(b-1) a hydroxy group,
(b-2) a carboxyl group,
(b-3) a C₁₋₆ alkoxy-carbonyl group, and
(b-4) a carbamoyl group optionally mono- or di-substituted by C₁₋₆ alkyl group(s),
(c) a C₆₋₁₄ aryl group optionally substituted by 1 to 3 substituents selected from the following (c-1) to (c-5)
(c-1) a C₁₋₆ alkyl group optionally substituted by 1 to 3 substituents selected from a hydroxy group and a halogen atom,
(c-2) a carboxyl group,
(c-3) a C₁₋₆ alkoxy-carbonyl group,
(c-4) a carbamoyl group, and
(c-5) a C₁₋₆ alkoxy group optionally substituted by 1 to 3 halogen atoms,
(d) a C₇₋₁₃ aralkyl group,
(e) a C₁₋₆ alkoxy group, and
(f) a C₇₋₁₃ aralkyloxy group, or
R⁶ and R⁷ form, together with the adjacent nitrogen atom, an optionally substituted nitrogen-containing heterocycle,
(xvi) a sulfamoyl group optionally mono- or di-substituted by C₁₋₆ alkyl group(s) optionally substituted by 1 to 3 C₃₋₁₀ cycloalkyl groups, and
(xvii) a non-aromatic heterocyclyl-carbonyl group optionally substituted by 1 to 3 substituents selected from
(a) a carboxyl group,
(b) a C₁₋₆ alkoxy-carbonyl group, and
(c) a C₇₋₁₃ aralkyl group optionally substituted by C₁₋₆ alkyl group(s) optionally substituted by 1 to 3 halogen atoms;
(16) a C₁₋₆ alkyl-carbonyl group;
(17) a C₁₋₆ alkoxy-carbonyl group;
(18) a C₇₋₁₃ aralkyloxy-carbonyl group;
(19) a C₁₋₆ alkylsulfinyl group;
(20) a C₁₋₆ alkylsulfonyl group;
(21) a carbamoyl group optionally mono- or di-substituted by substituent(s) selected from a C₁₋₆ alkyl group, a C₆₋₁₄ aryl group, a C₇₋₁₃ aralkyl group and an aromatic heterocyclyl-C₁₋₆ alkyl group;
(22) a C₆₋₁₄ aryloxy group;
(23) a C₇₋₁₃ aralkyloxy group substituted by 1 to 3 substituents selected from a C₁₋₆ alkyl group substituted by 1 to 3 hydroxy groups, a carboxyl group and a C₁₋₆ alkoxy-carbonyl group;
(24) an aromatic heterocyclyl-oxy group optionally substituted by 1 to 3 substituents selected from a C₁₋₆ alkyl group optionally substituted by 1 to 3 halogen atoms and a halogen atom;
(25) a tri-C₁₋₆ alkyl-silyloxy group;
(26) a formyl group; and
(27) a C₁₋₆ alkylsulfonyloxy group optionally substituted by 1 to 3 halogen atoms.

11. The compound of claim 5, wherein R^{3a} is a hydrogen atom.

12. The compound of claim 5, wherein R^{4a} is a hydrogen atom.

13. The compound of claim 5, wherein R^{5a} is a hydrogen atom.

14. The compound of claim 5, which is
3-(2,4-dichlorobenzyl)-1-(4-hydroxy-4-methylcyclohexyl)pyrrolidin-2-one;
N-(3-chloro-4-{[1-(5-hydroxy-2-adamantyl)-2-oxopyrrolidin-3-yl]methyl}phenyl)-N'-[4-(hydroxymethyl)phenyl]urea;
N-(3'-chloro-4'-{[1-(5-hydroxy-2-adamantyl)-2-oxopyrrolidin-3-yl]methyl}biphenyl-3-yl)methanesulfonamide;
1-(3-chloro-4-{[1-(5-hydroxy-2-adamantyl)-2-oxopyrrolidin-3-yl]methyl}phenyl)-3-[4-(trifluoromethyl)benzyl]imidazolidin-2-one;
2-(3-chloro-4-{[1-(4-hydroxy-4-methylcyclohexyl)-2-oxopyrrolidin-3-yl]methyl}phenoxy)-N-[4-(trifluoromethyl)benzyl]acetamide;
3-(2-chloro-4-{[3-(4-fluorophenyl)-1,2,4-oxadiazol-5-yl]methoxy}benzyl)-1-(4-hydroxy-4-methylcyclohexyl)pyrrolidin-2-one;
3-{[3-chloro-3'-(hydroxymethyl)biphenyl-4-yl]methyl}-1-(4-hydroxy-4-methylcyclohexyl)pyrrolidin-2-one;
or a salt thereof.

15. A pharmaceutical agent comprising the compound of claim 5.

16. the pharmaceutical agent of claim 15, which is an agent for the prophylaxis or treatment of diabetes, obesity or arteriosclerosis.
